# EUROPEAN PATENT APPLICATION

(11) **EP 2 022 800 A2**
(43) Date of publication of application: **11.02.2009**
(21) Application number: 08165934.4
(22) Date of filing: 17.07.2001
(51) Int. Cl.: C07K 16/30, A61K 39/395, C07K 14/705, C12N 15/12, A61K 39/00, C07K 19/00, A61P 35/00

(54) **Compositions for the therapy and diagnosis of ovarian cancer**

(30) Priority: 17.07.2000 US 617747; 10.08.2000 US 636801; 20.09.2000 US 667857; 04.04.2001 US 827271; 18.06.2001 US 884441
(62) Divisional of application: 01954748.8
(71) Applicant: CORIXA CORPORATION, Wilmington, DE 19808 (US)
(72) Inventor: Mitcham, Jennifer L., Redmone, PA 98052 (US); King, Gordon E., Shoreline, WA 98177 (US); Algate, Paul A., Issaquah, WA 98027 (US); Fling, Steven P., Bainbridge, WA 98110 (US); Retter, Marc W., Carnation, WA 98014 (US); Fanger, Gary Richard, Mill Creek, WA 98012 (US); Reed, Steven G., Bellevue, WA 98005 (US); Vedvick, Thomas S., Federal Way, WA 98003 (US); Carter, Darrick, Seattle, WA 98102 (US); Hill, Paul, Everett, WA 98201 (US); Albone, Earl, Plymouth Meeting, PA 19462 (US)
(74) Representative: Walker, Ross Thomson

(57) **Abstract**

Compositions and methods for the therapy and diagnosis of cancer, such as ovarian cancer, are disclosed. Compositions may comprise one or more ovarian carcinoma proteins, immunogenic portions thereof, polynucleotides that encode such portions or antibodies or immune system cells specific for such proteins. Such compositions may be used, for example, for the prevention and treatment of diseases such as ovarian cancer. Methods are further provided for identifying tumor antigens that are secreted from ovarian carcinomas and/or other tumors. Polypeptides and polynucleotides as provided herein may further be used for the diagnosis and monitoring of ovarian cancer.

## Description

### Technical Field

The present invention relates generally to ovarian cancer therapy. The invention is more specifically related to polypeptides comprising at least a portion of an ovarian carcinoma protein, and to polynucleotides encoding such polypeptides, as well as antibodies and immune system cells that specifically recognize such polypeptides. Such polypeptides, polynucleotides, antibodies and cells may be used in vaccines and pharmaceutical compositions for treatment of ovarian cancer.

### Background of the Invention

Ovarian cancer is a significant health problem for women in the United States and throughout the world. Although advances have been made in detection and therapy of this cancer, no vaccine or other universally successful method for prevention or treatment is currently available. Management of the disease currently relies on a combination of early diagnosis and aggressive treatment, which may include one or more of a variety of treatments such as surgery, radiotherapy, chemotherapy and hormone therapy. The course of treatment for a particular cancer is often selected based on a variety of prognostic parameters, including an analysis of specific tumor markers. However, the use of established markers often leads to a result that is difficult to interpret, and high mortality continues to be observed in many cancer patients.

Immunotherapies have the potential to substantially improve cancer treatment and survival. Such therapies may involve the generation or enhancement of an immune response to an ovarian carcinoma antigen. However, to date, relatively few ovarian carcinoma antigens are known and the generation of an immune response against such antigens has not been shown to be therapeutically beneficial.

Accordingly, there is a need in the art for improved methods for identifying ovarian tumor antigens and for using such antigens in the therapy of ovarian cancer. The present invention fulfills these needs and further provides other related advantages.

### SUMMARY OF THE INVENTION

Briefly stated, this invention provides compositions and methods for the therapy of cancer, such as ovarian cancer. In one aspect, the present invention provides polypeptides comprising an immunogenic portion of an ovarian carcinoma protein, or a variant thereof that differs in one or more substitutions, deletions, additions and/or insertions such that the ability of the variant to react with ovarian carcinoma protein-specific antisera is not substantially diminished. Within certain embodiments, the ovarian carcinoma protein comprises a sequence that is encoded by a polynucleotide sequence selected from the group consisting of SEQ ID NO:456-457, 460-477 and 512-570 and complements of such polynucleotides.

The present invention further provides polynucleotides that encode a polypeptide as described above or a portion thereof, expression vectors comprising such polynucleotides and host cells transformed or transfected with such expression vectors.

The present invention further provides polypeptide compositions comprising an amino acid sequence selected from the group consisting of sequences recited in SEQ ID Nos:394-455, 458-459, 478-511, and 571-596.

Within other aspects, the present invention provides pharmaceutical compositions and vaccines. Pharmaceutical compositions may comprise a physiologically acceptable carrier or excipient in combination with one or more of: (i) a polypeptide comprising an immunogenic portion of an ovarian carcinoma protein, or a variant thereof that differs in one or more substitutions, deletions, additions and/or insertions such that the ability of the variant to react with ovarian carcinoma protein-specific antisera is not substantially diminished, wherein the ovarian carcinoma protein comprises an amino acid sequence encoded by a polynucleotide that comprises a sequence recited in any one of SEQ ID NO: 456-457, 460-477 and 512-570 or (ii) a polynucleotide encoding such a polypeptide; (iii) an antibody that specifically binds to such a polypeptide; (iv) an antigen-presenting cell that expresses such a polypeptide and/or (v) a T cell that specifically reacts with such a polypeptide. Vaccines may comprise a non-specific immune response enhancer in combination with one or more of: (i) a polypeptide comprising an immunogenic portion of an ovarian carcinoma protein, or a variant thereof that differs in one or more substitutions, deletions, additions and/or insertions such that the ability of the variant to react with ovarian carcinoma protein-specific antisera is not substantially diminished, wherein the ovarian carcinoma protein comprises an amino acid sequence set forth in SEQ ID Nos:394-455, 458-459, 478-511, and 571-596 or an amino acid sequence encoded by a polynucleotide that comprises a sequence recited in any one of SEQ ID NO: 456-457, 460-477 and 512-570 or (ii) a polynucleotide encoding such a polypeptide; (iii) an anti-idiotypic antibody that is specifically bound by an antibody that specifically binds to such a polypeptide; (iv) an antigen-presenting cell that expresses such a polypeptide and/or (v) a T cell that specifically reacts with such a polypeptide.

The present invention further provides, in other aspects, fusion proteins that comprise at least one polypeptide as described above, as well as polynucleotides encoding such fusion proteins.

Within related aspects, pharmaceutical compositions comprising a fusion protein or polynucleotide encoding a fusion protein in combination with a physiologically acceptable carrier are provided.

Vaccines are further provided, within other aspects, comprising a fusion protein or polynucleotide encoding a fusion protein in combination with a non-specific immune response enhancer.

Within further aspects, the present invention provides methods for inhibiting the development of a cancer in a patient, comprising administering to a patient a pharmaceutical composition or vaccine as recited above.

The present invention further provides, within other aspects, methods for stimulating and/or expanding T cells, comprising contacting T cells with (a) a polypeptide comprising an immunogenic portion of an ovarian carcinoma protein, or a variant thereof that differs in one or more substitutions, deletions, additions and/or insertions such that the ability of the variant to react with ovarian carcinoma protein-specific antisera is not substantially diminished, wherein the ovarian carcinoma protein comprises an amino acid sequence set forth in SEQ ID Nos:394-455, 458-459, 478-511, and 571-596 or an amino acid sequence encoded by a polynucleotide that comprises a sequence recited in any one of SEQ ID NO: 456-457, 460-477 and 512-570; (b) a polynucleotide encoding such a polypeptide and/or (c) an antigen presenting cell that expresses such a polypeptide under conditions and for a time sufficient to permit the stimulation and/or expansion of T cells. Such polypeptide, polynucleotide and/or antigen presenting cell(s) may be present within a pharmaceutical composition or vaccine, for use in stimulating and/or expanding T cells in a mammal.

Within other aspects, the present invention provides methods for inhibiting the development of ovarian cancer in a patient, comprising administering to a patient T cells prepared as described above.

Within further aspects, the present invention provides methods for inhibiting the development of ovarian cancer in a patient, comprising the steps of: (a) incubating CD4⁺ and/or CD8⁺ T cells isolated from a patient with one or more of: (i) a polypeptide comprising an immunogenic portion of an ovarian carcinoma protein, or a variant thereof that differs in one or more substitutions, deletions, additions and/or insertions such that the ability of the variant to react with ovarian carcinoma protein-specific antisera is not substantially diminished, wherein the ovarian carcinoma protein comprises an amino acid sequence encoded by a polynucleotide that comprises a sequence recited in any one of SEQ ID NO: 456-457, 460-477 and 512-570; (ii) a polynucleotide encoding such a polypeptide; or (iii) an antigen-presenting cell that expresses such a polypeptide; such that T cells proliferate; and (b) administering to the patient an effective amount of the proliferated T cells, and thereby inhibiting the development of ovarian cancer in the patient. The proliferated cells may be cloned prior to administration to the patient.

The present invention also provides, within other aspects, methods for identifying secreted tumor antigens. Such methods comprise the steps of: (a) implanting tumor cells in an immunodeficient mammal; (b) obtaining serum from the immunodeficient mammal after a time sufficient to permit secretion of tumor antigens into the serum; (c) immunizing an immunocompetent mammal with the serum; (d) obtaining antiserum from the immunocompetent mammal; and (e) screening a tumor expression library with the antiserum, and therefrom identifying a secreted tumor antigen. A preferred method for identifying a secreted ovarian carcinoma antigen comprises the steps of: (a) implanting ovarian carcinoma cells in a SCID mouse; (b) obtaining serum from the SCID mouse after a time sufficient to permit secretion of ovarian carcinoma antigens into the serum; (c) immunizing an immunocompetent mouse with the serum; (d) obtaining antiserum from the immunocompetent mouse; and (e) screening an ovarian carcinoma expression library with the antiserum, and therefrom identifying a secreted ovarian carcinoma antigen.

The present invention also discloses antibody epitopes recognized by the 08E polyclonal anti-sera which epitopes are presented herein as SEQ ID NO: 394-415.

Further disclosed by the present invention are 10-mer and 9-mer peptides predicted to bind HLA-0201 which peptides are disclosed herein as SEQ ID NO:416-435 and SEQ ID NO:436-455, respectively.

These and other aspects of the present invention will become apparent upon reference to the following detailed description and attached drawings. All references disclosed herein are hereby incorporated by reference in their entirety as if each was incorporated individually.

In another aspect of the present invention, the applicants have unexpectedly identified a series of novel repeating sequence elements in the 5' end of the gene encoding 0772P. Therefore, the present invention provides 0772P polypeptides having structures represented by Xₙ-Y, wherein X comprises a sequence having at least 50% identity, preferably at least 70% identity, and more preferably at least 90% identity with an 0772P repeat sequence set forth in SEQ ID NO: 596. Y will typically comprise a sequence having at least 80% identity, preferably at least 90% identity and more preferably at least 95% identity with the 0772P constant region sequence set forth in SEQ ID NO: 594. According to this embodiment, n will generally be an integer from 1 to 35, preferably an integer from 15 to 25, and X can be the same or different.

In one preferred embodiment, X comprises a sequence selected from the group consisting of any one of SEQ ID NOs: 574-593 and Y comprises the sequence set forth in SEQ ID NO: 594.

In another preferred embodiment, an illustrative O772P polypeptide comprises the sequence set forth in SEQ ID NO: 595, containing 20 repeating sequence elements (i.e., X₂₀) wherein the X elements are arranged in the following order (moving from N-terminal to C-terminal in the 0772P repeat region): SEQ ID NO: 574 - SEQ ID NO: 575 - SEQ ID NO: 576 - SEQ ID NO: 577 - SEQ ID NO: 578 - SEQ ID NO: 579 - SEQ ID NO: 580 - SEQ ID NO: 581 - SEQ ID NO: 582 - SEQ ID NO: 583 - SEQ ID NO: 584 - SEQ ID NO: 585 - SEQ ID NO: 586 - SEQ ID NO: 587 - SEQ ID NO: 588 - SEQ ID NO: 589 - SEQ ID NO: 590 - SEQ ID NO: 591 - SEQ ID NO: 592 - SEQ ID NO: 593.

According to another aspect of the present invention, an 0772P polynucleotide is provided having the structure Xₙ-Y, wherein X comprises an 0772P repeat sequence element selected from the group consisting of any one of SEQ ID NOs: 512-540, 542-546 and 548-567. Y will generally comprise a sequence having at least 80% identity, preferably at least 90% identity, and more preferably at least 95% identity with the 0772P constant region sequence set forth in SEQ ID NO: 568. In this embodiment, n is typically an integer from 1 to 35, preferably from 15 to 25 and X can be the same or different.

In another embodiment, an illustrative 0772P polynucleotide comprises the sequence set forth in SEQ ID NO: 569, containing 20 repeating sequence elements (i.e., X₂₀).

According to another aspect of the present invention, 0772 polypeptides are provided comprising at least an antibody epitope sequence set forth in any one of SEQ ID NOs: 490-511.

According to another aspect of the present invention, O8E polypeptides are provided comprising at least an antibody epitope sequence set forth in any one of SEQ ID NOs: 394-415.

### BRIEF DESCRIPTION OF THE SEQUENCE IDENTIFIERS AND DRAWINGS

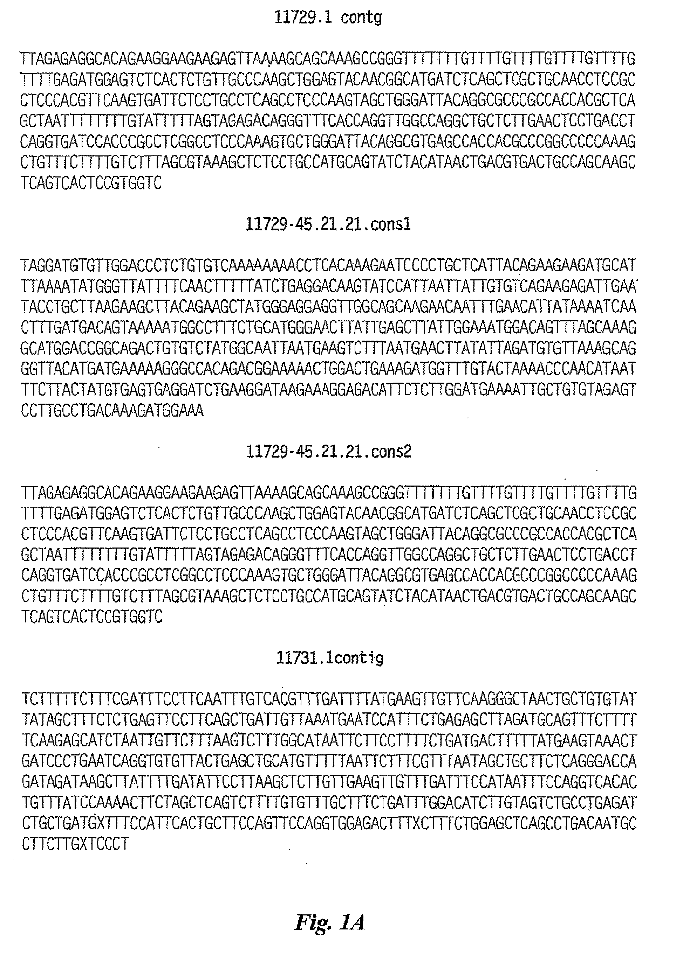

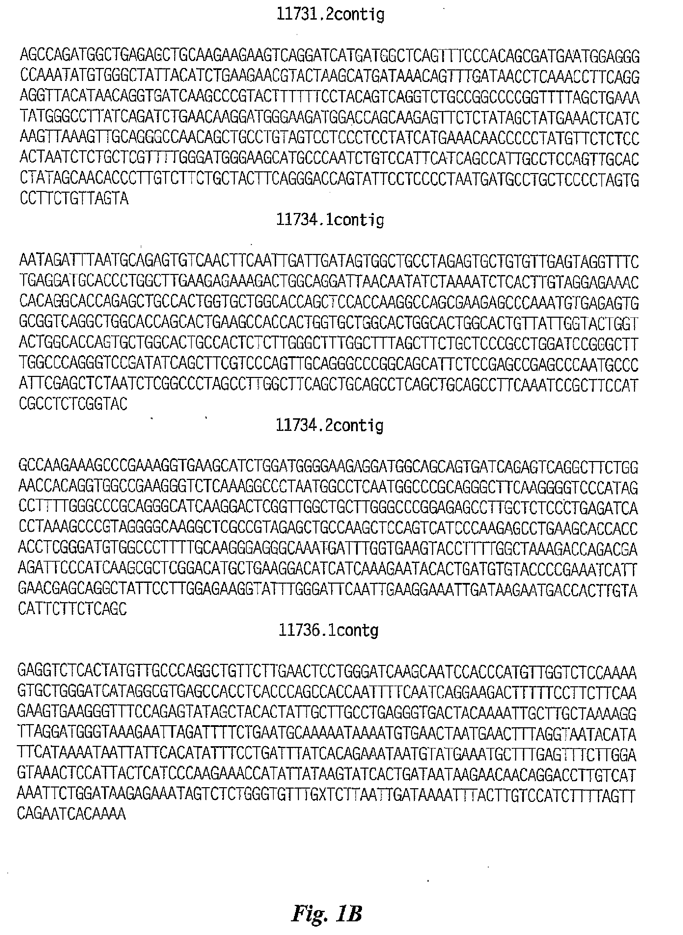

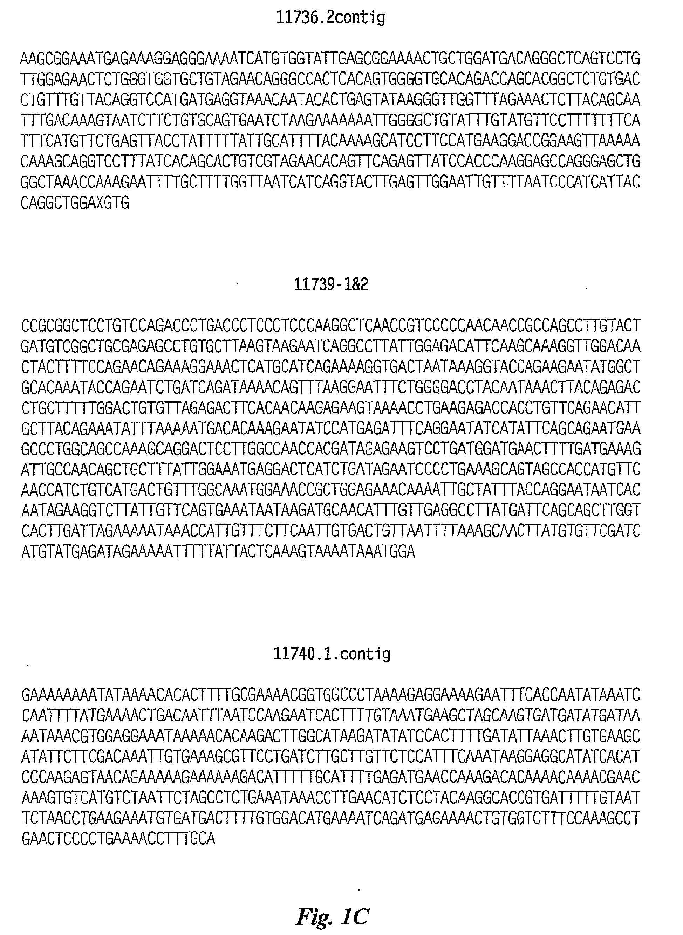

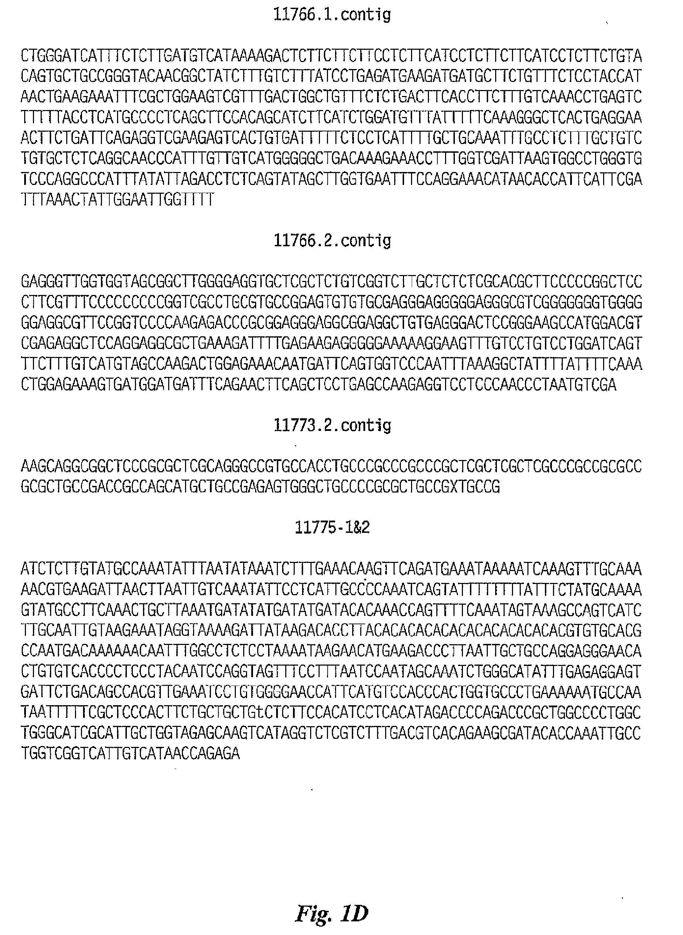

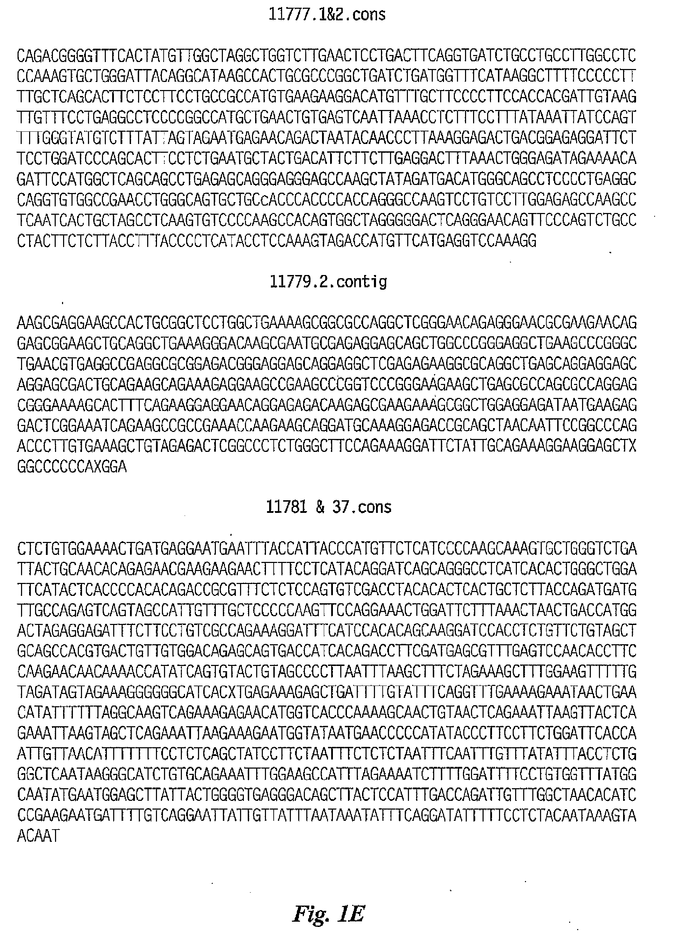

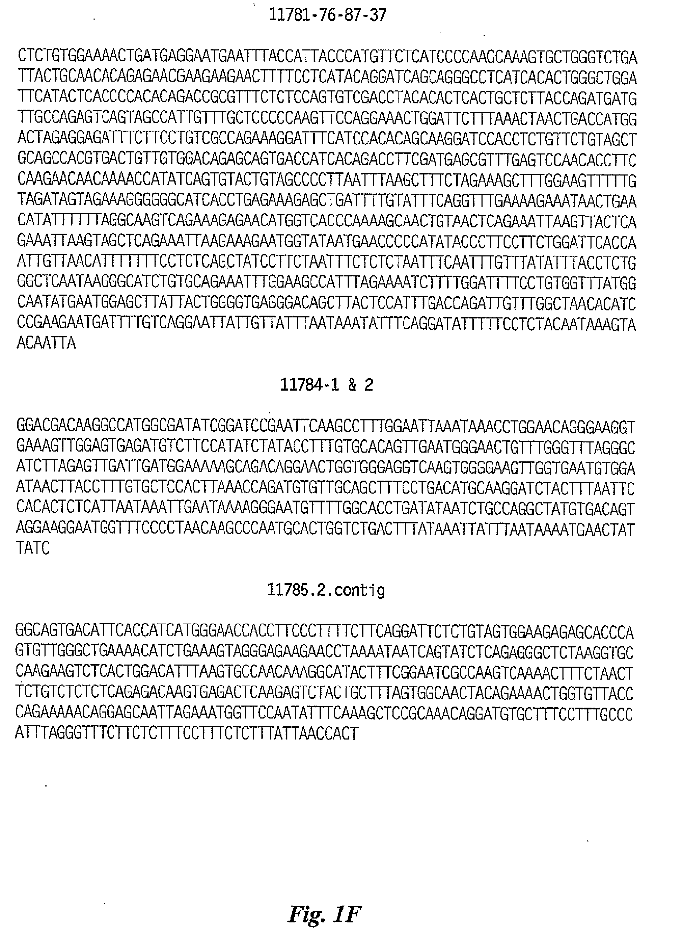

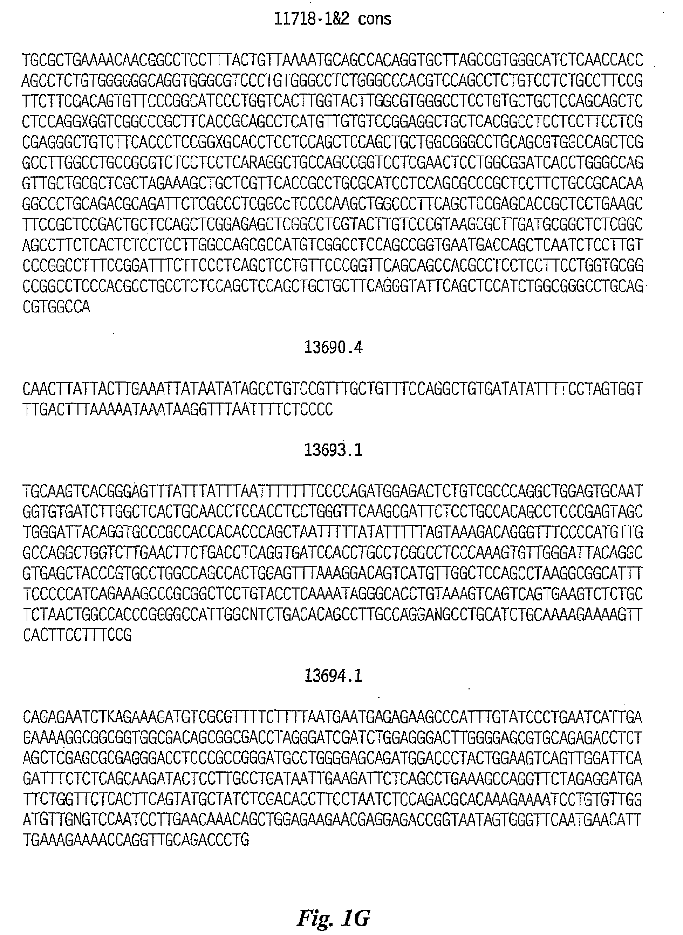

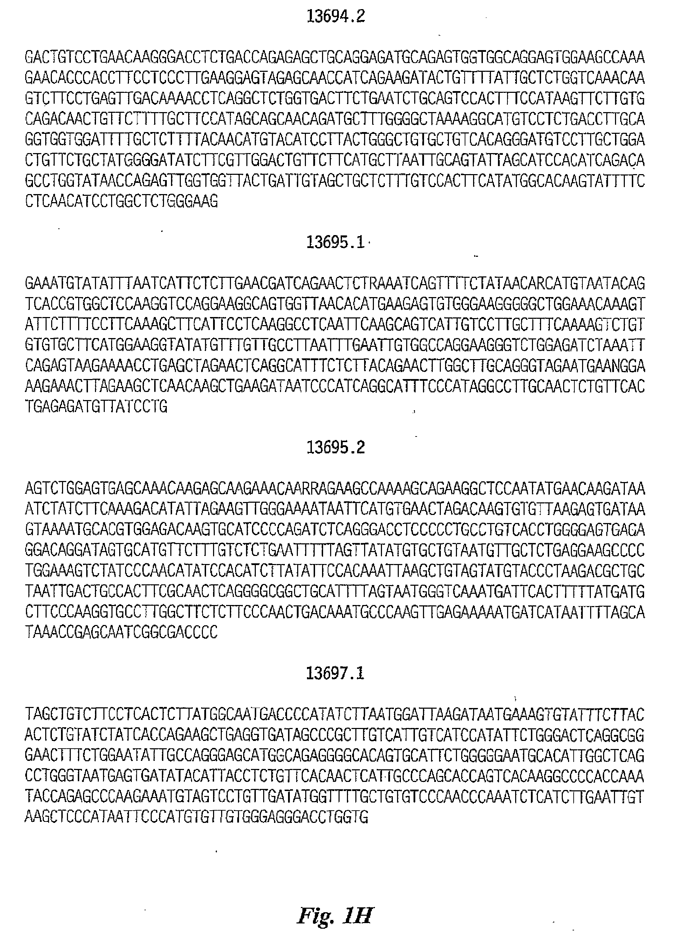

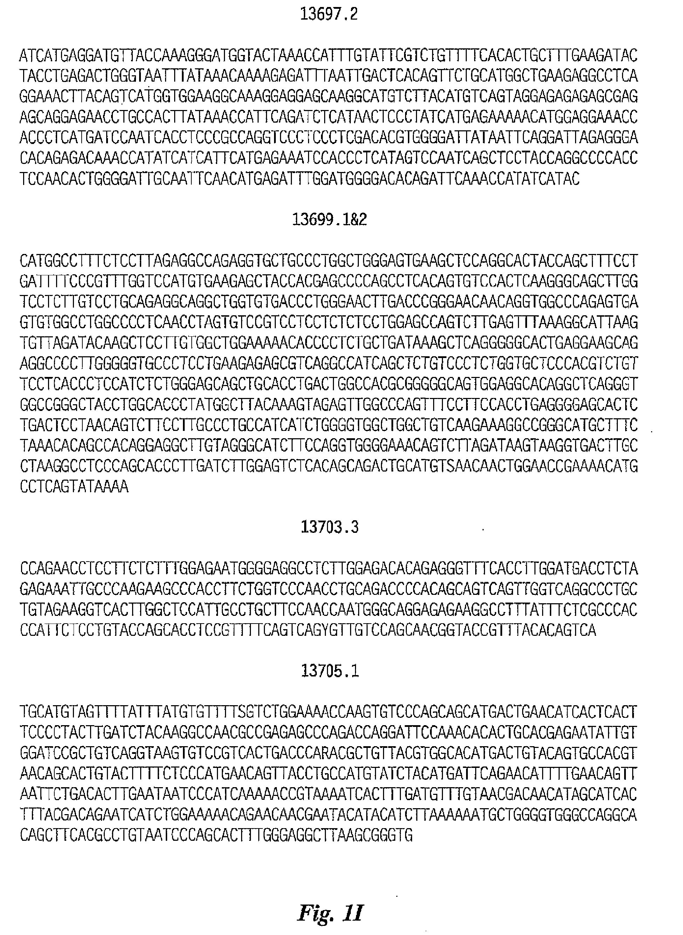

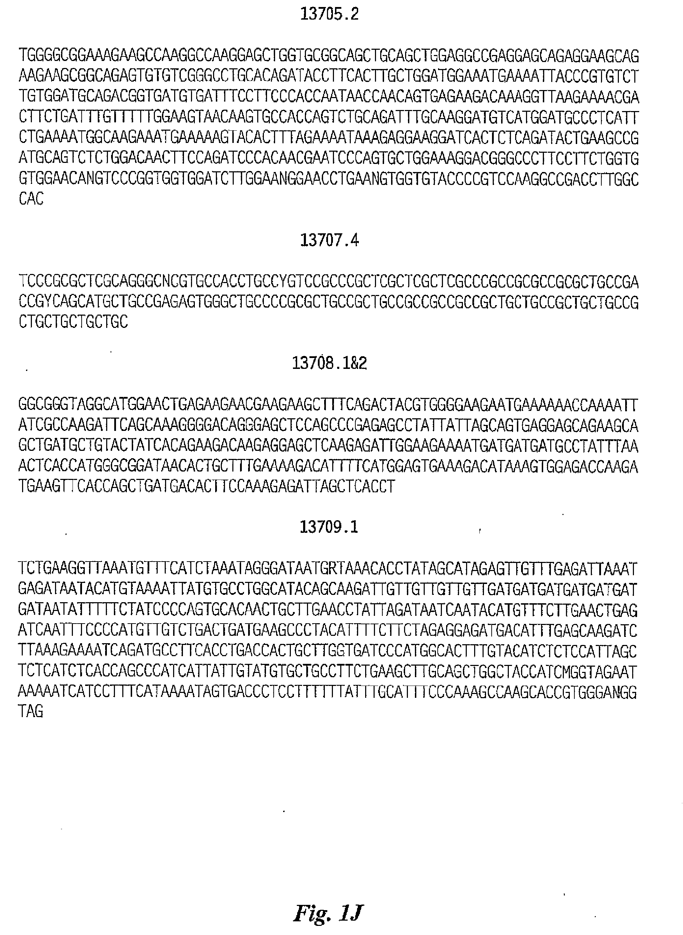

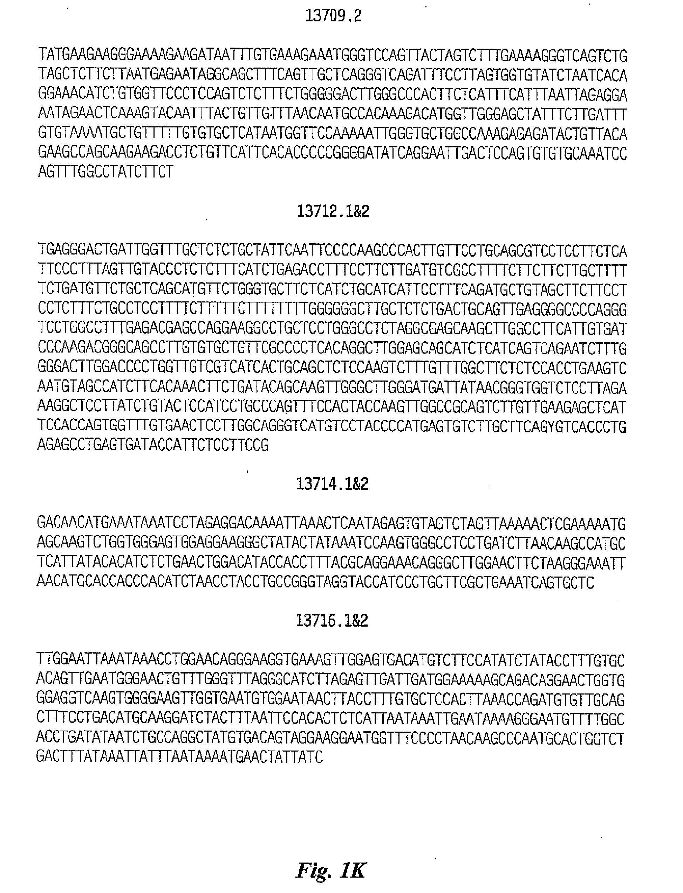

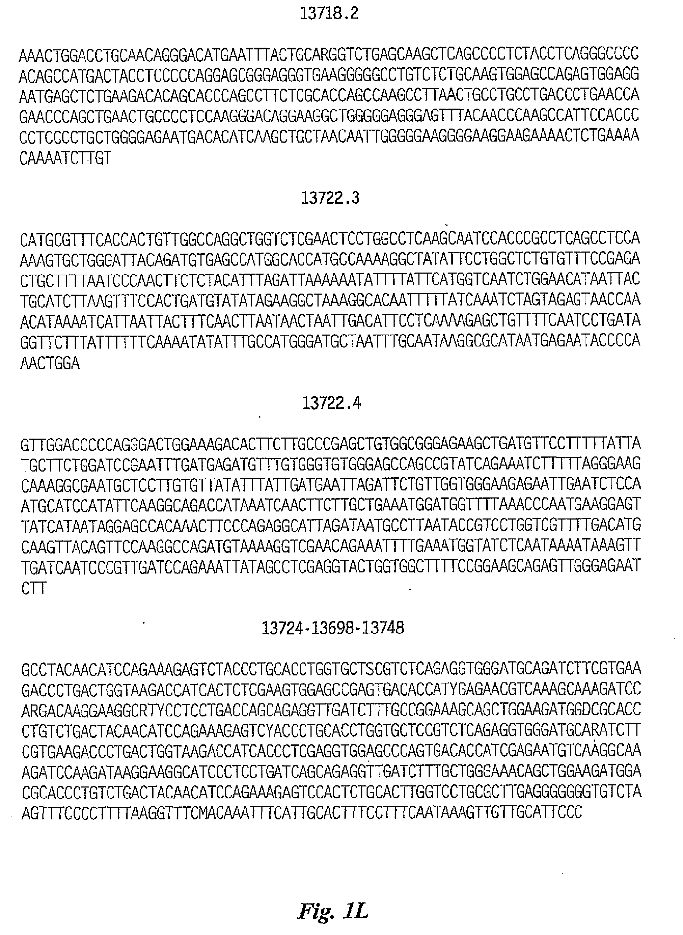

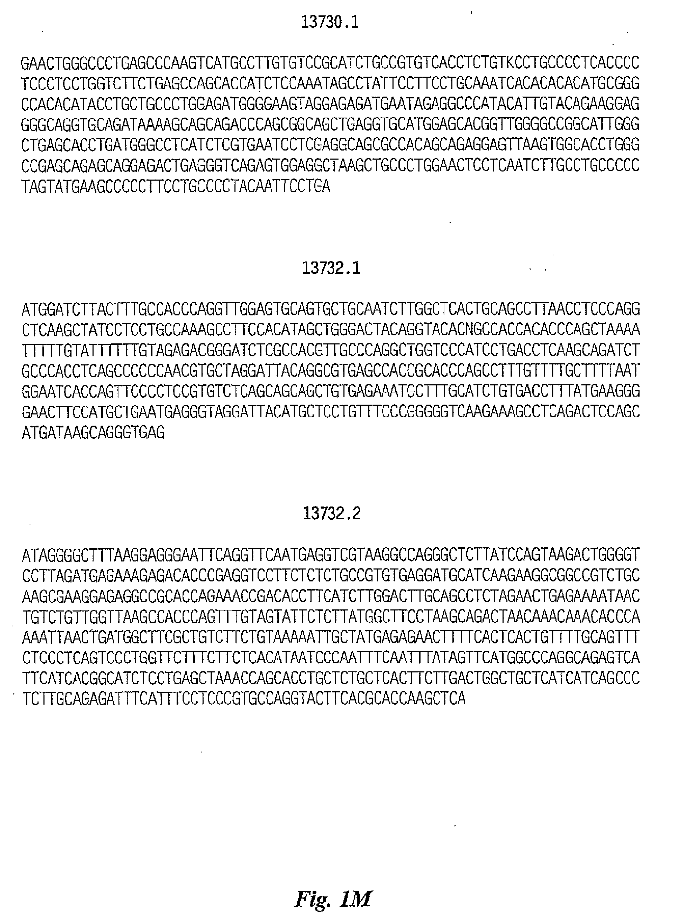

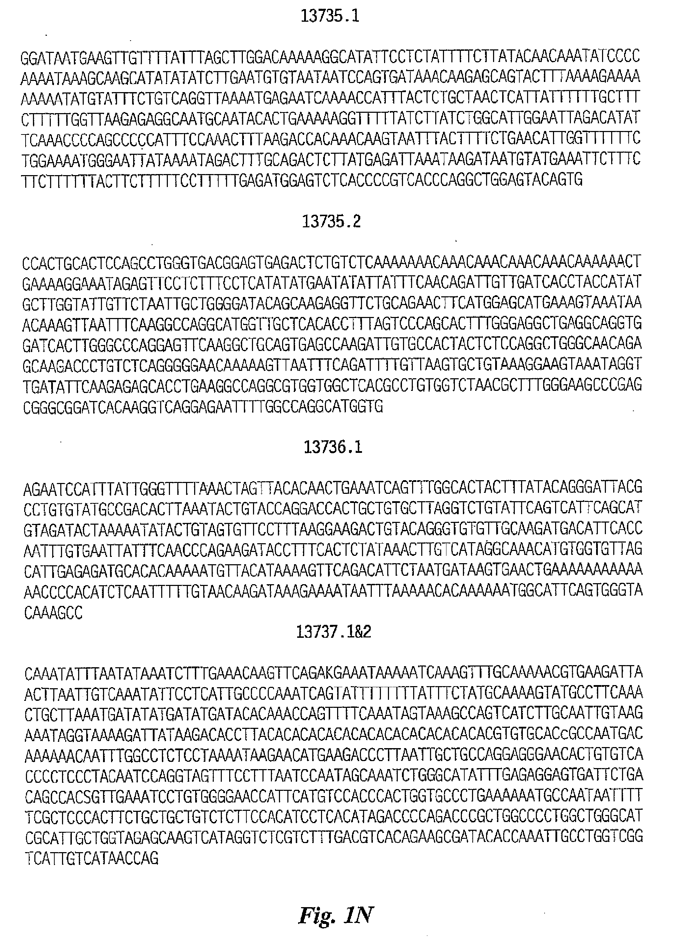

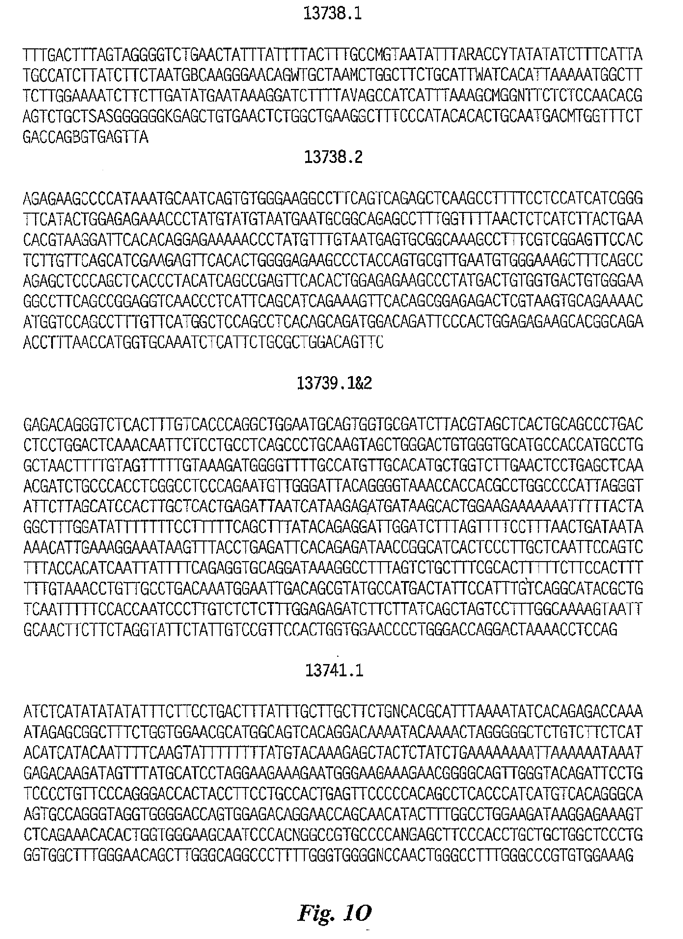

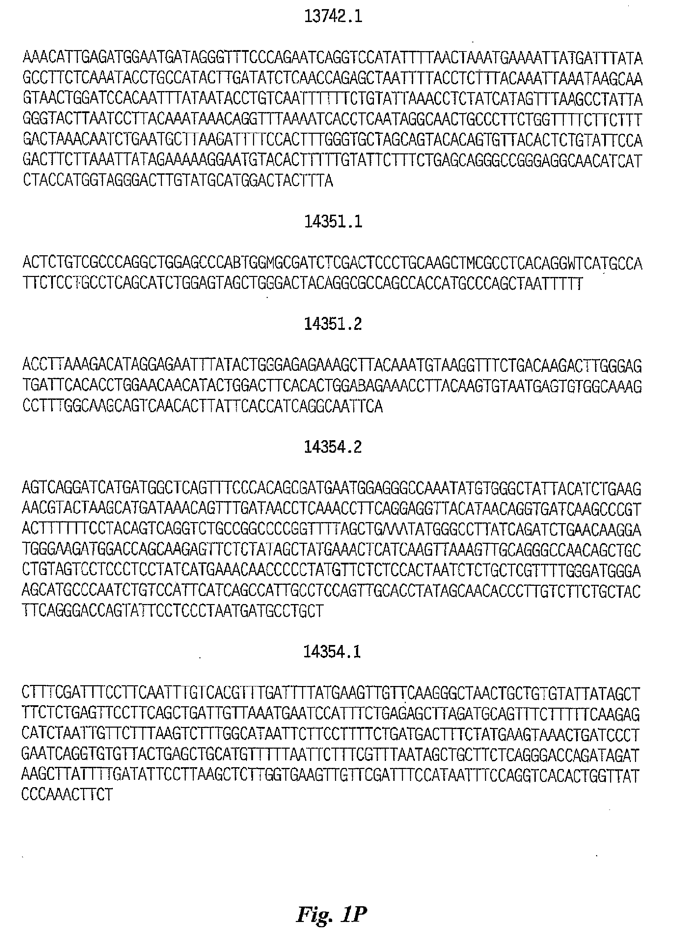

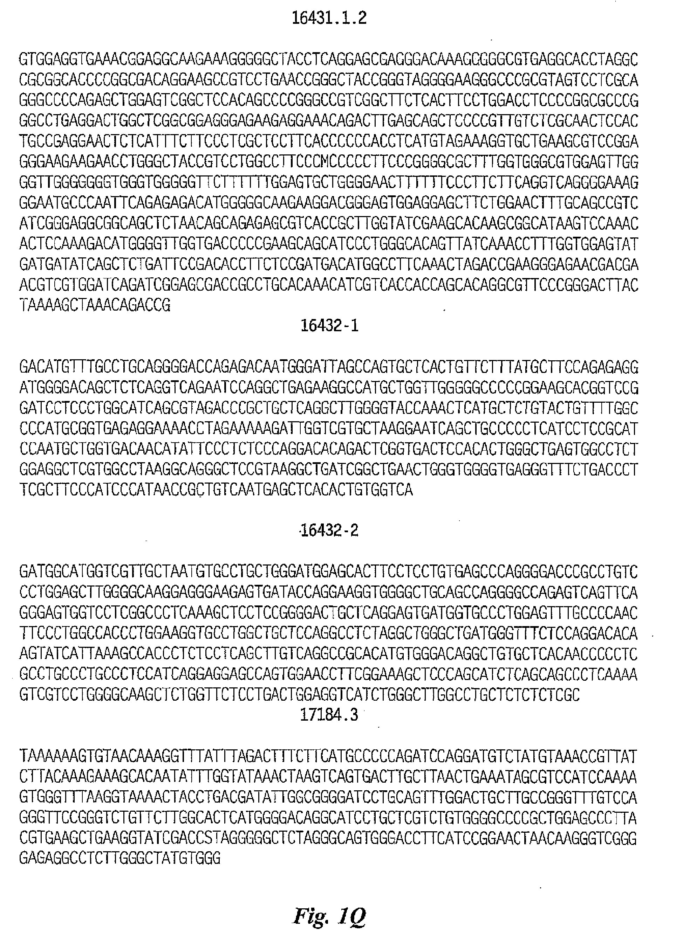

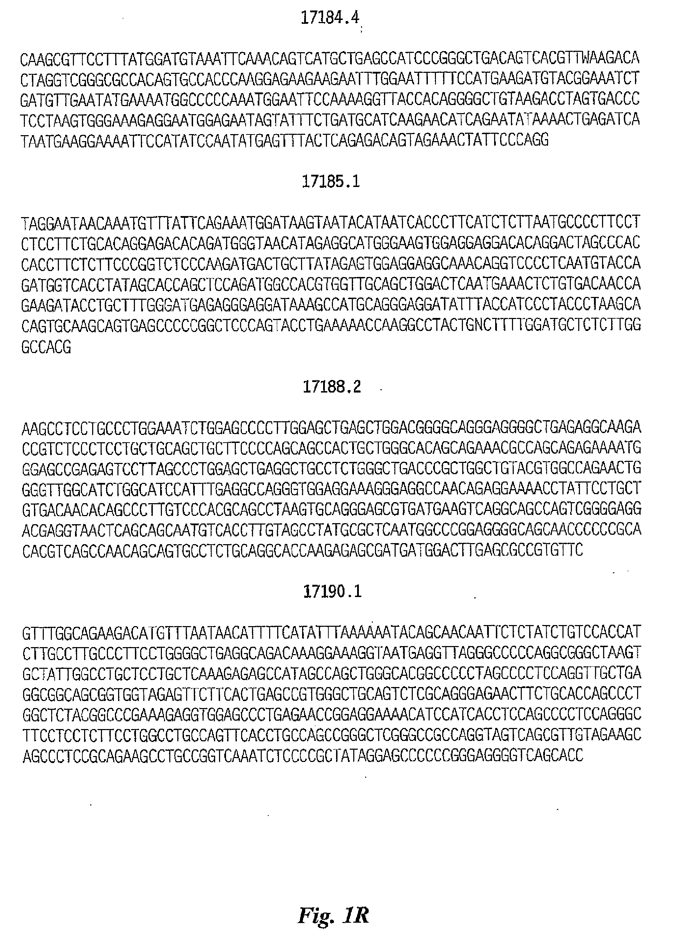

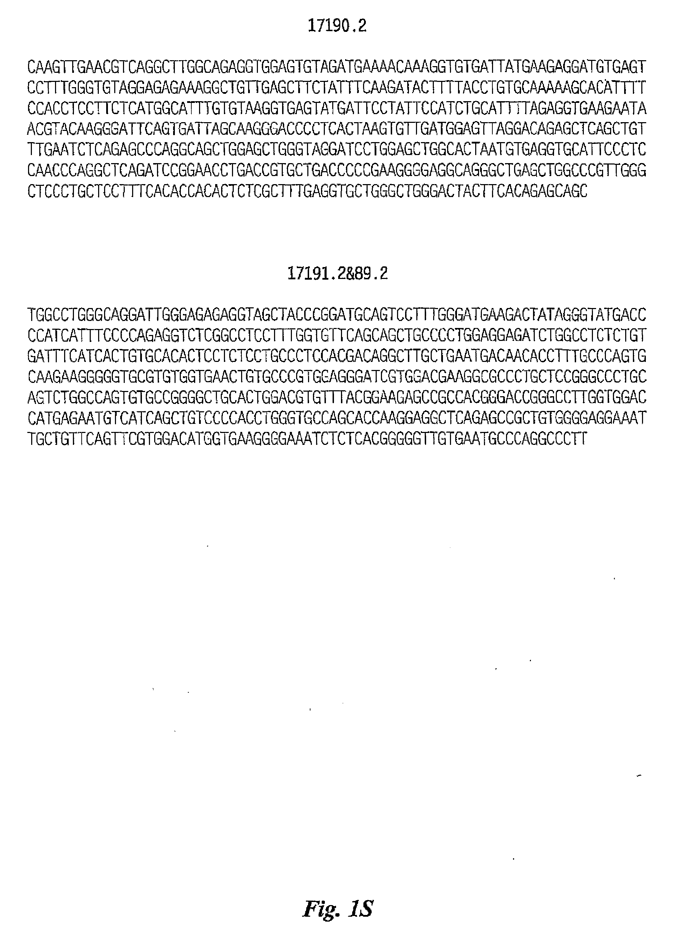

SEQ ID NO:1-71 are ovarian carcinoma antigen polynucleotides shown in Figures 1A-1S.

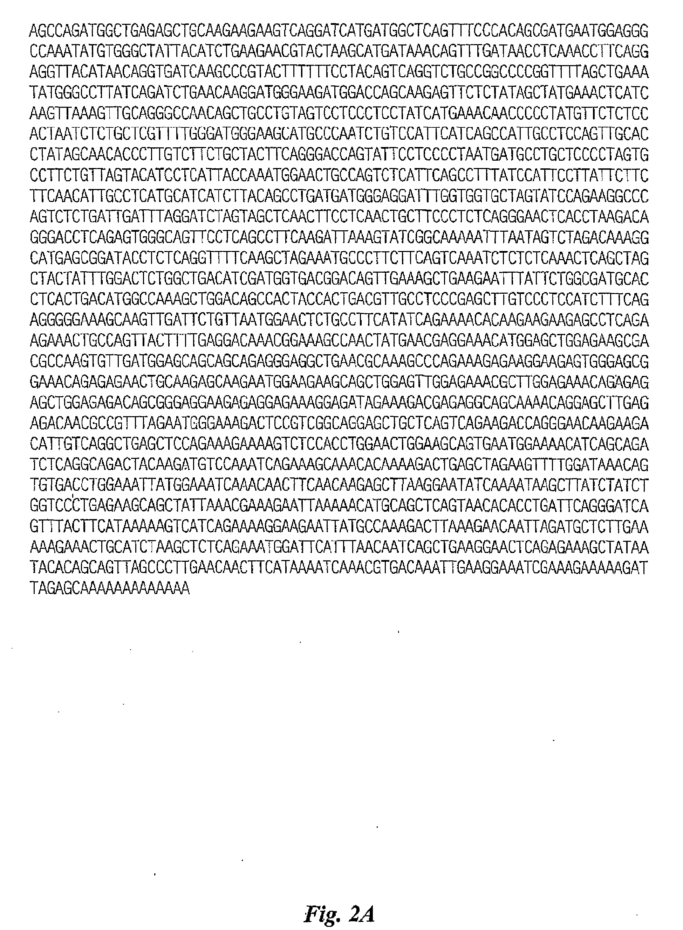

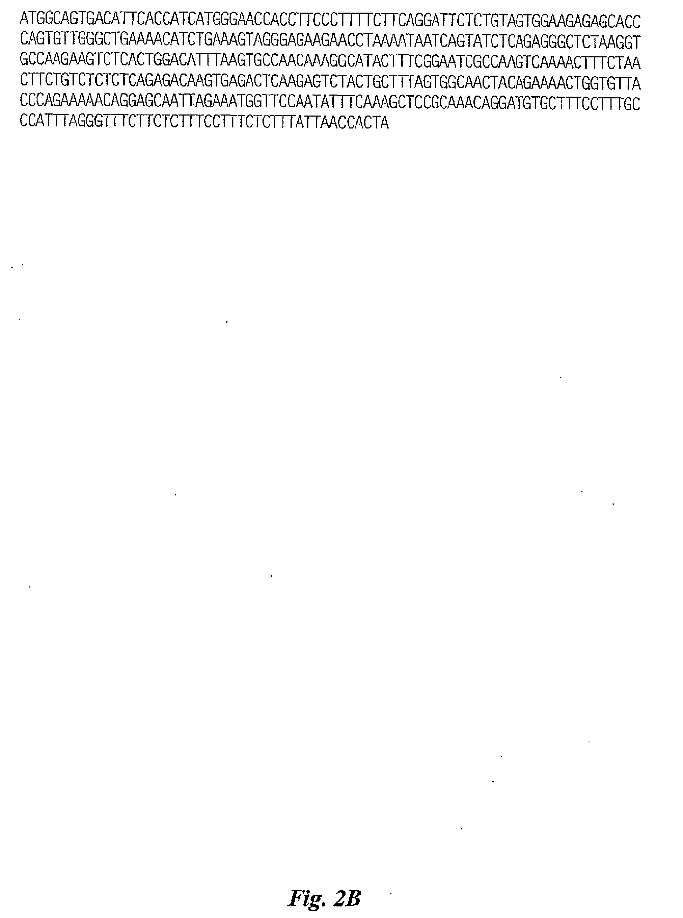

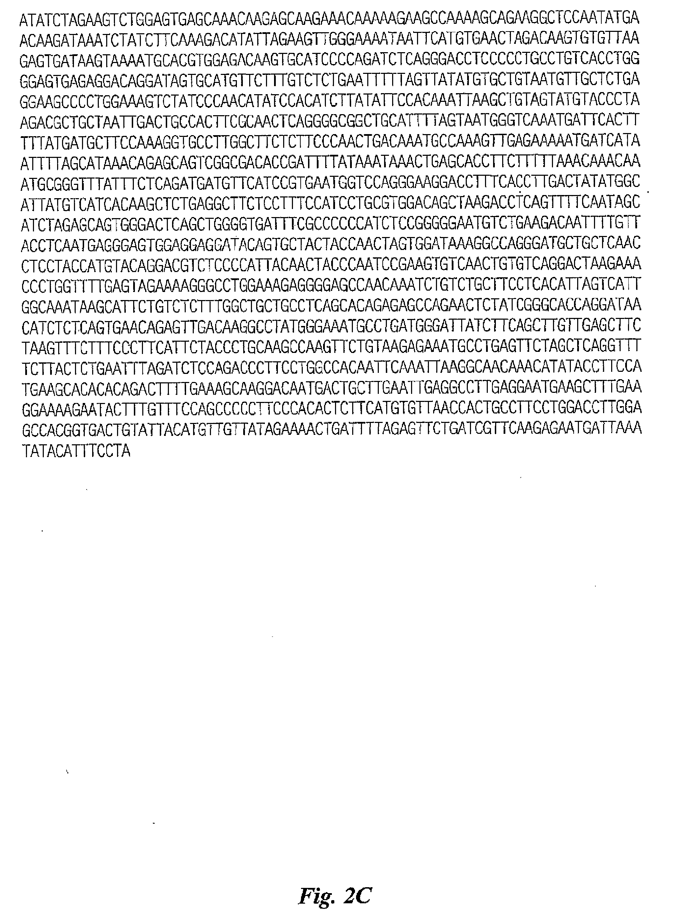

SEQ ID NO:72-74 are ovarian carcinoma antigen polynucleotides shown in Figures 2A-2C.

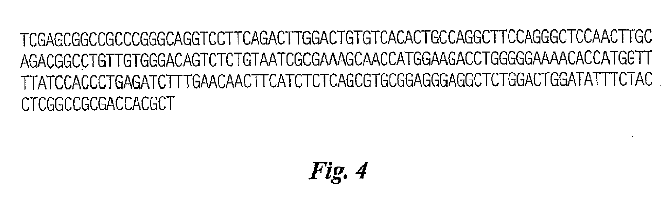

SEQ ID NO:75 is the ovarian carcinoma polynucleotide 3g (Figure 4).

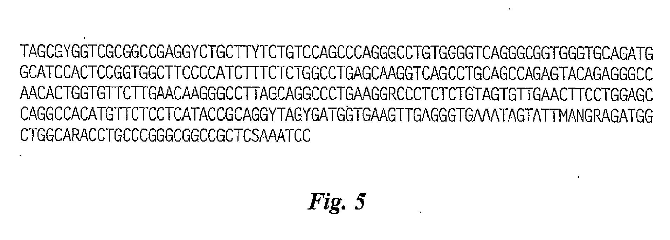

SEQ ID NO:76 is the ovarian carcinoma polynucleotide 3f (Figure 5).

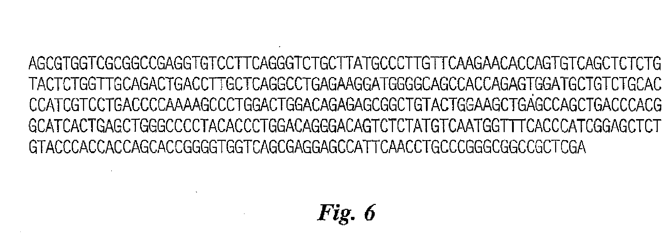

SEQ ID NO:77 is the ovarian carcinoma polynucleotide 6b (Figure 6).

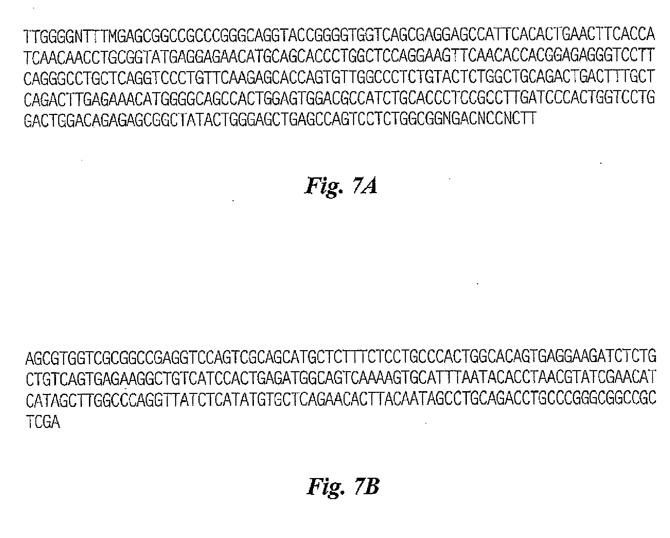

SEQ ID NO:78 is the ovarian carcinoma polynucleotide 8e (Figure 7A).

SEQ ID NO:79 is the ovarian carcinoma polynucleotide 8h (Figure 7B).

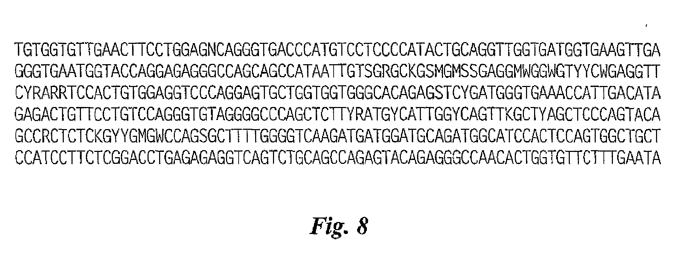

SEQ ID NO:80 is the ovarian carcinoma polynucleotide 12e (Figure 8).

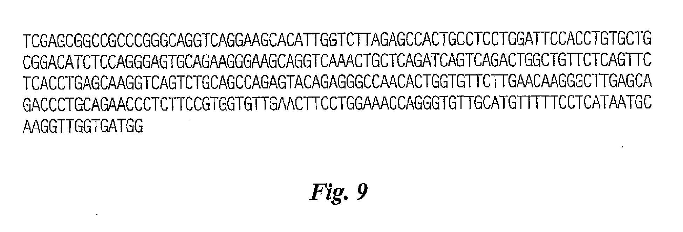

SEQ ID NO:81 is the ovarian carcinoma polynucleotide 12h (Figure 9).

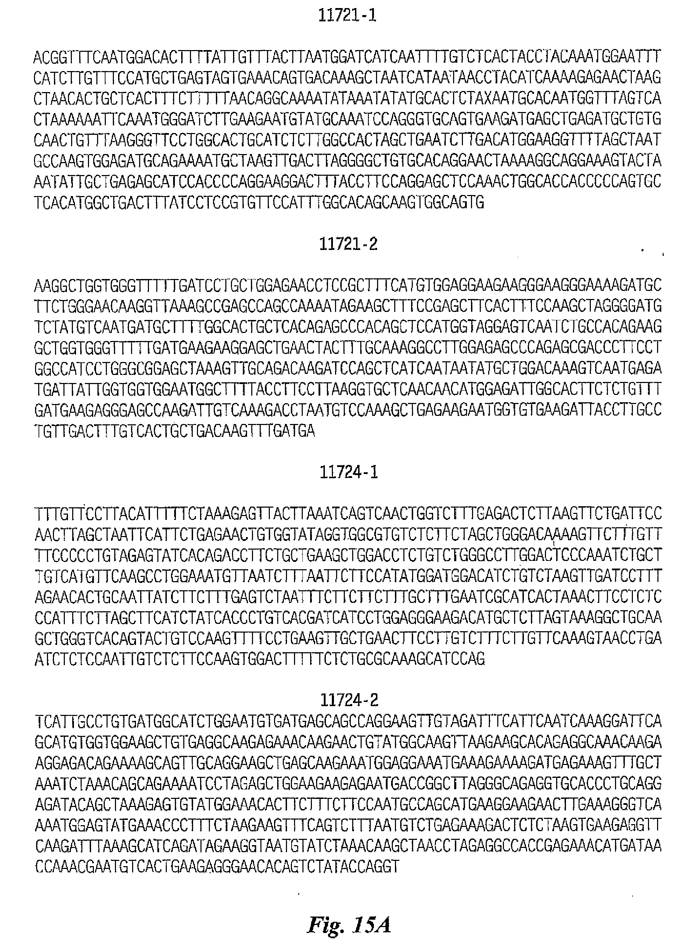

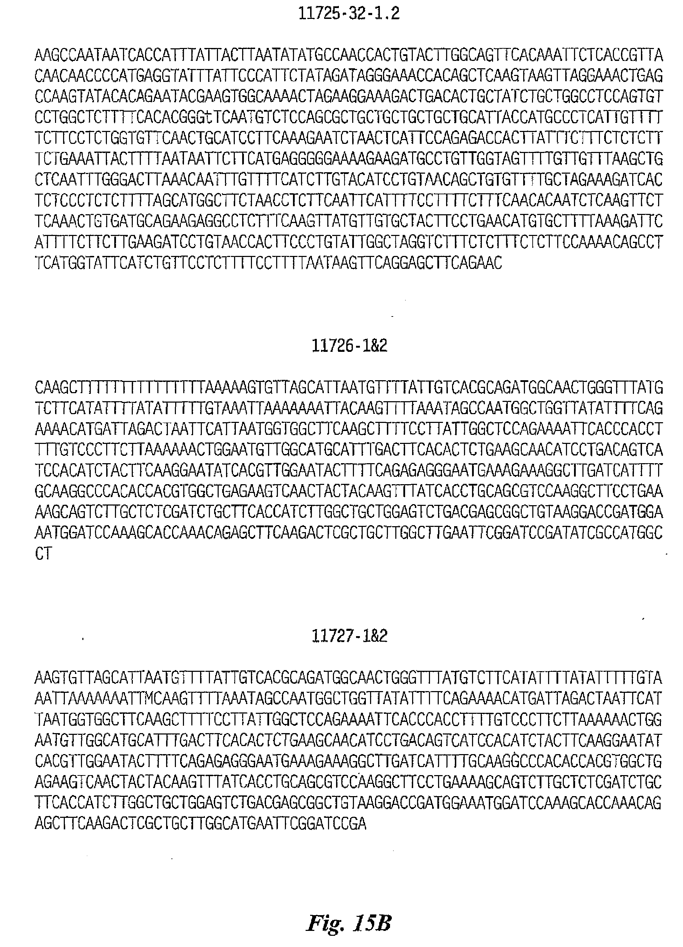

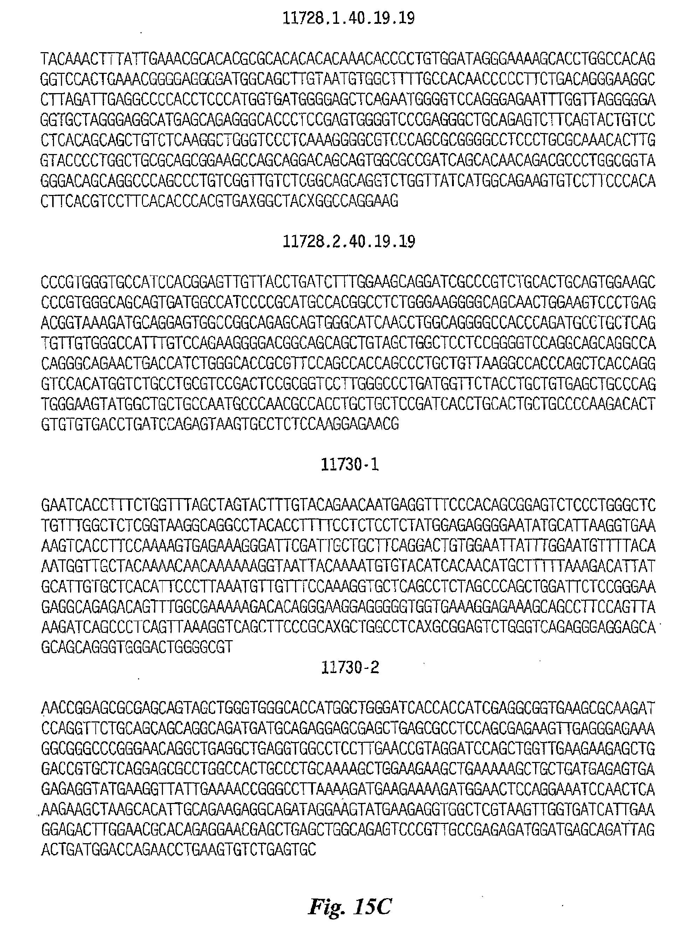

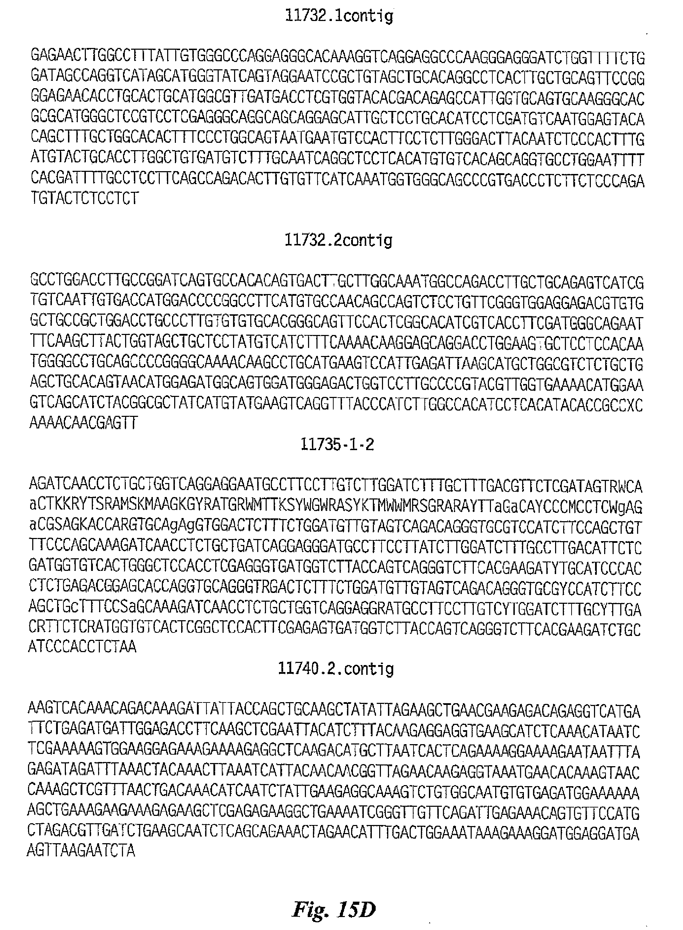

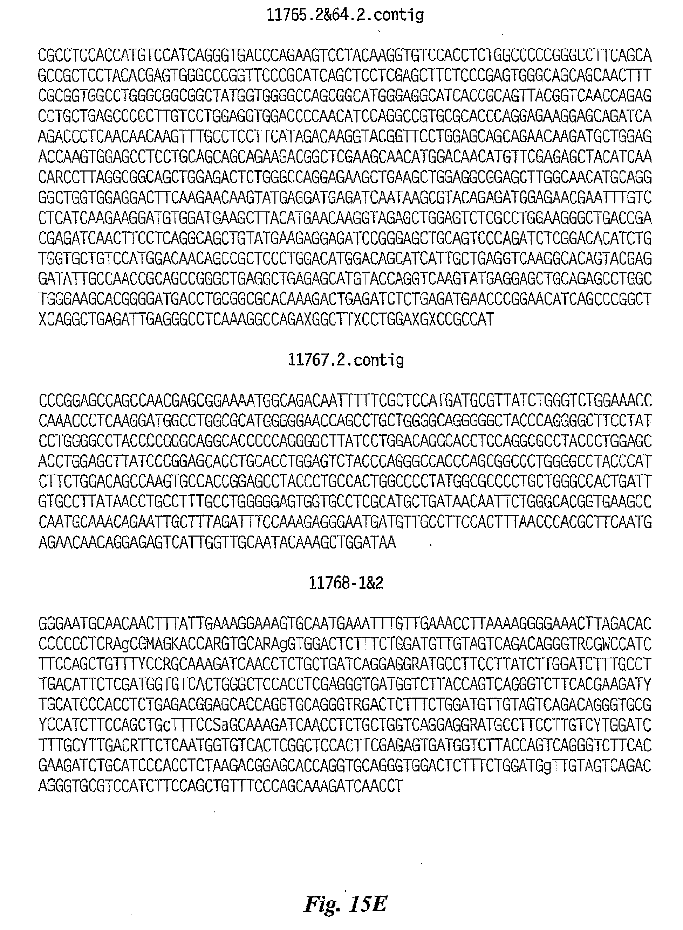

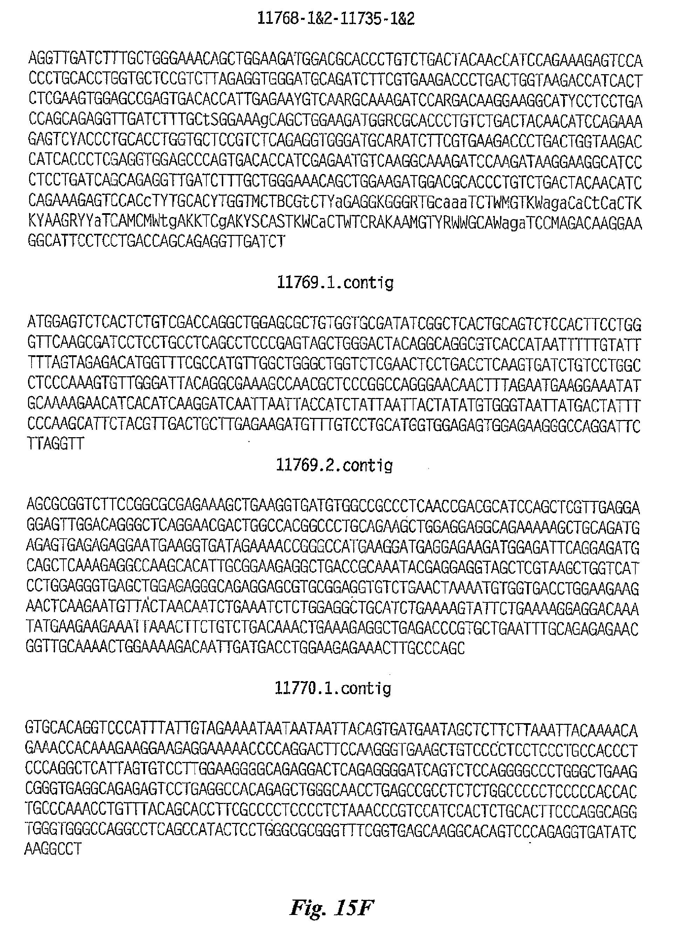

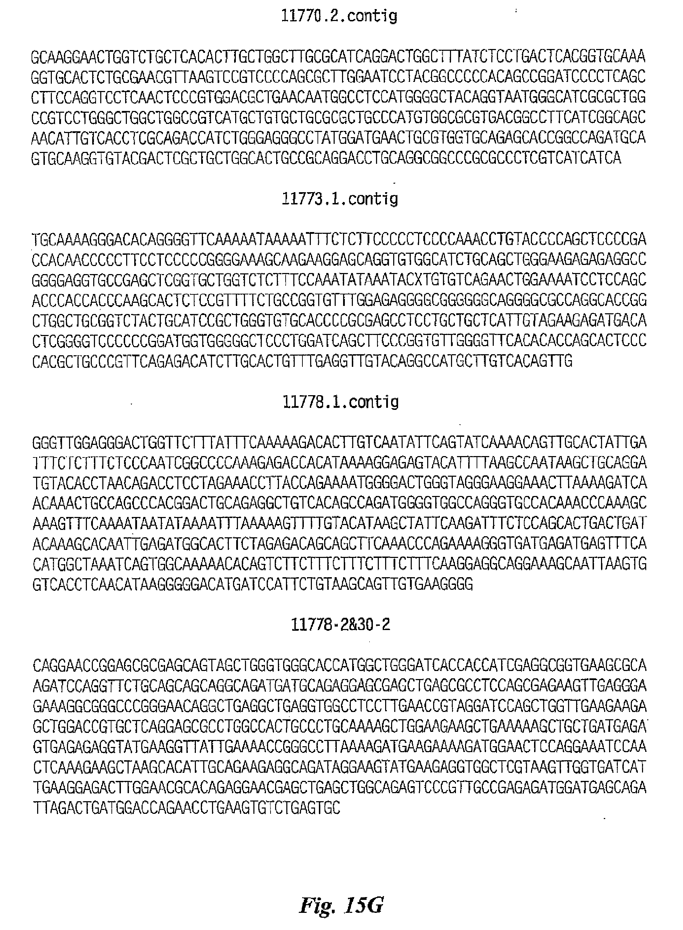

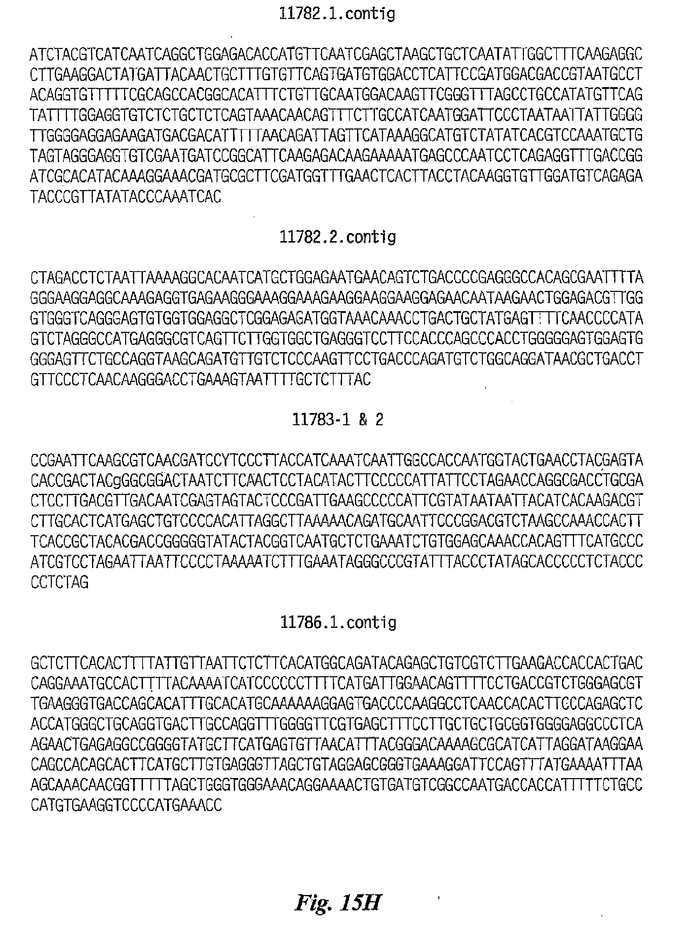

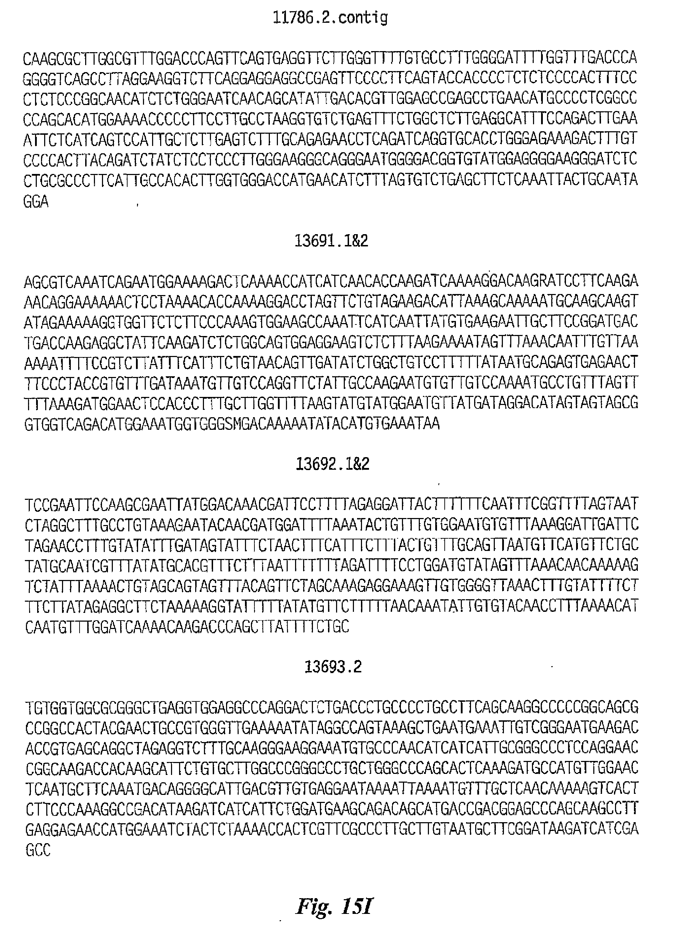

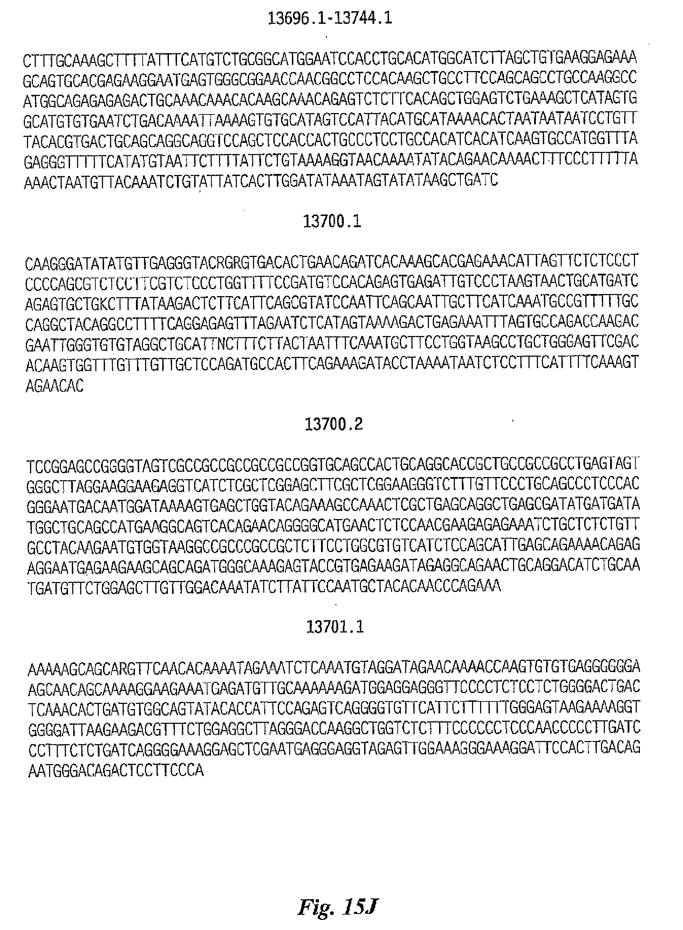

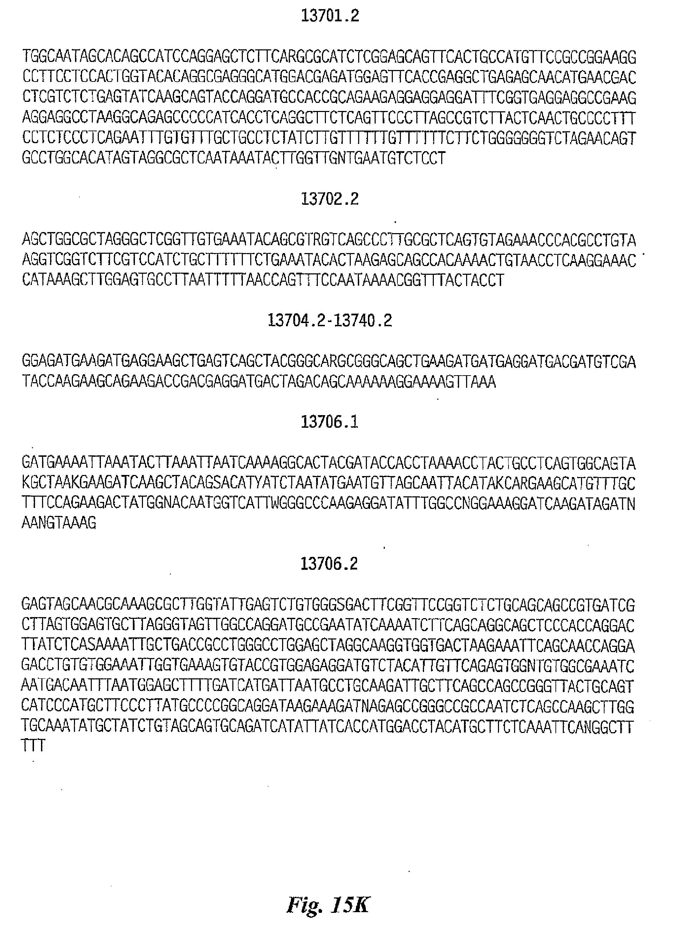

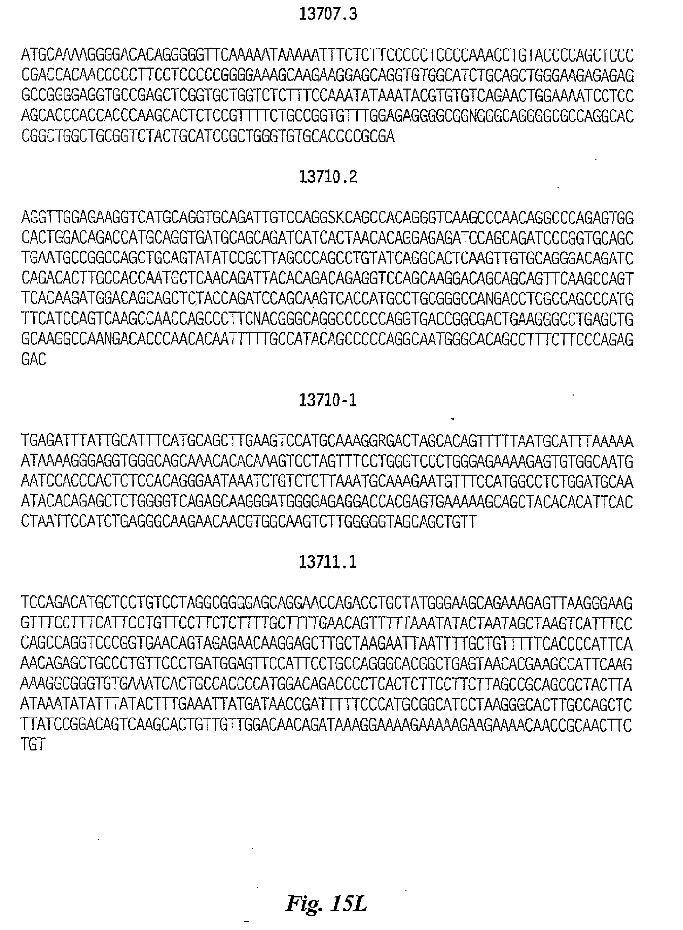

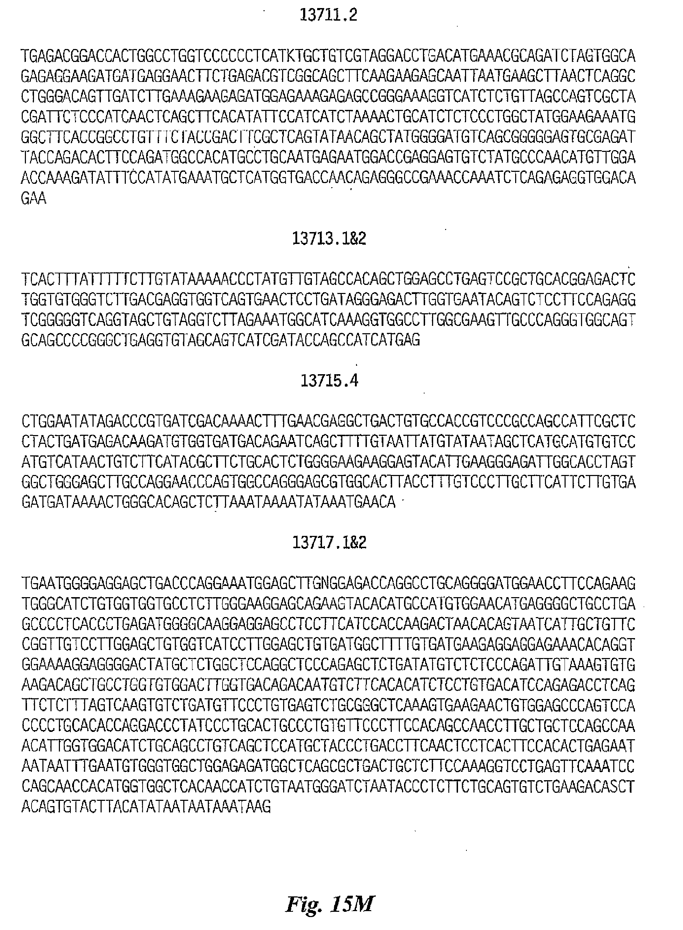

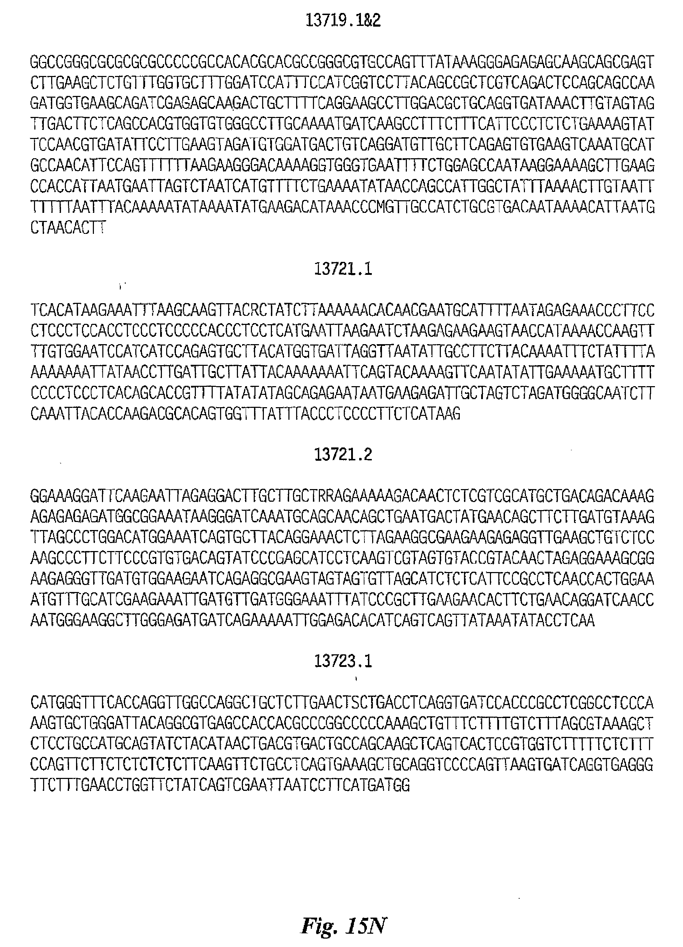

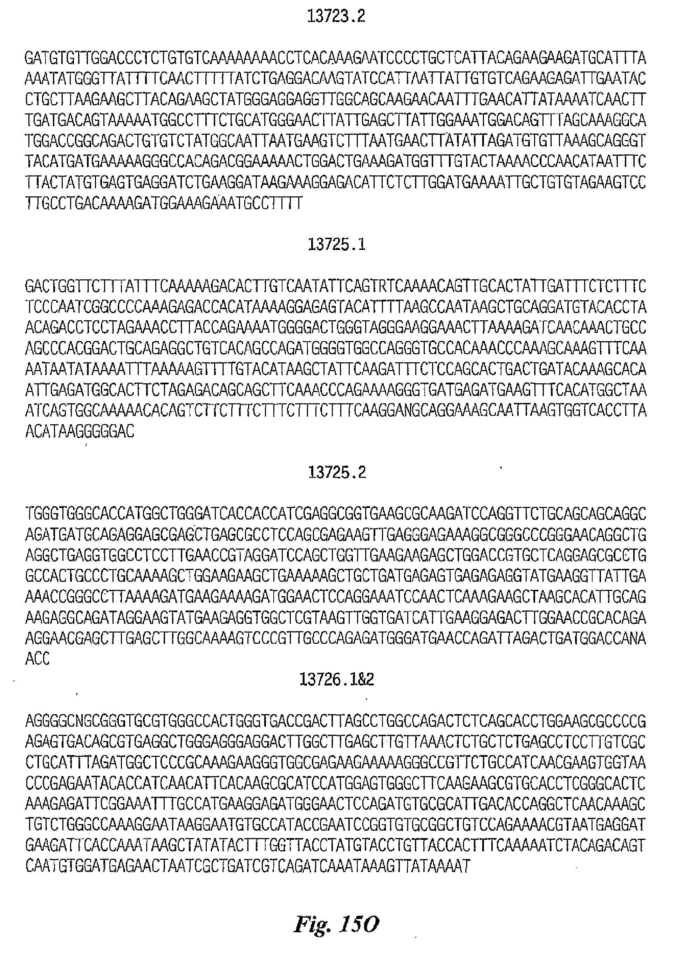

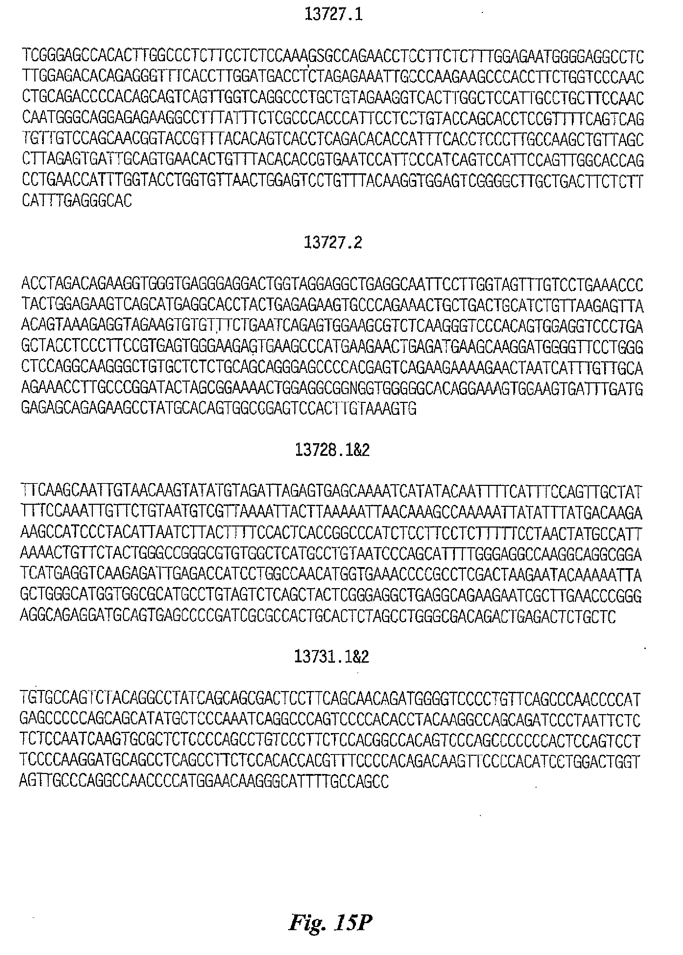

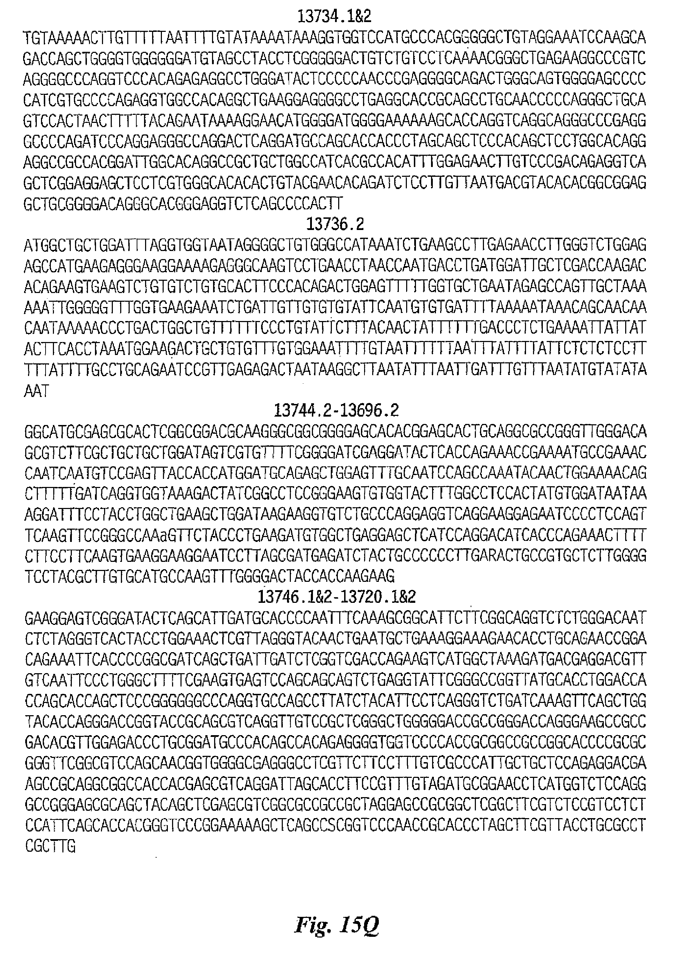

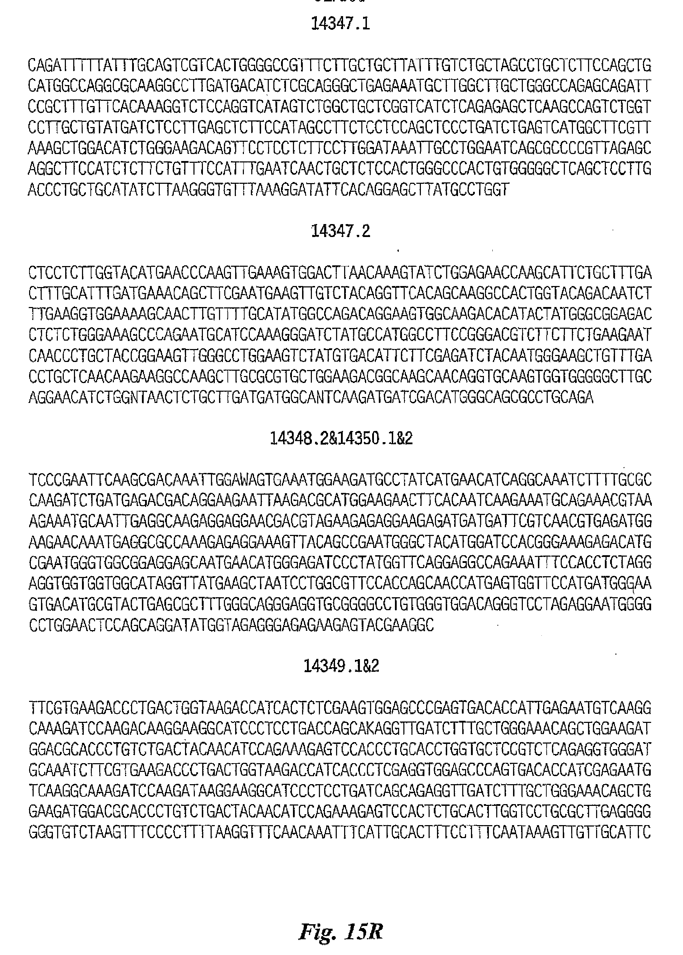

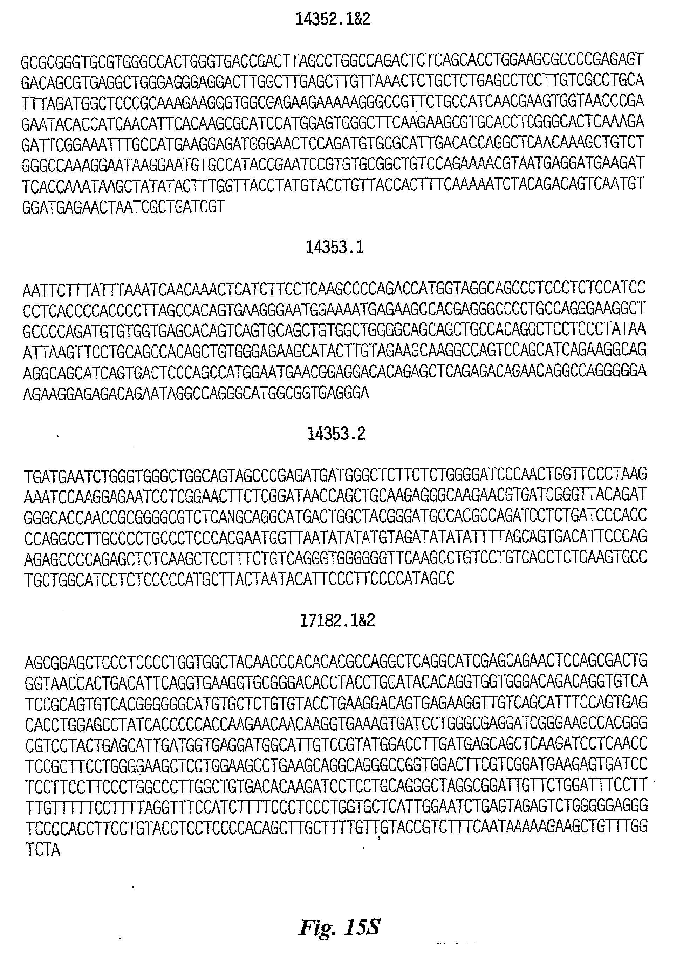

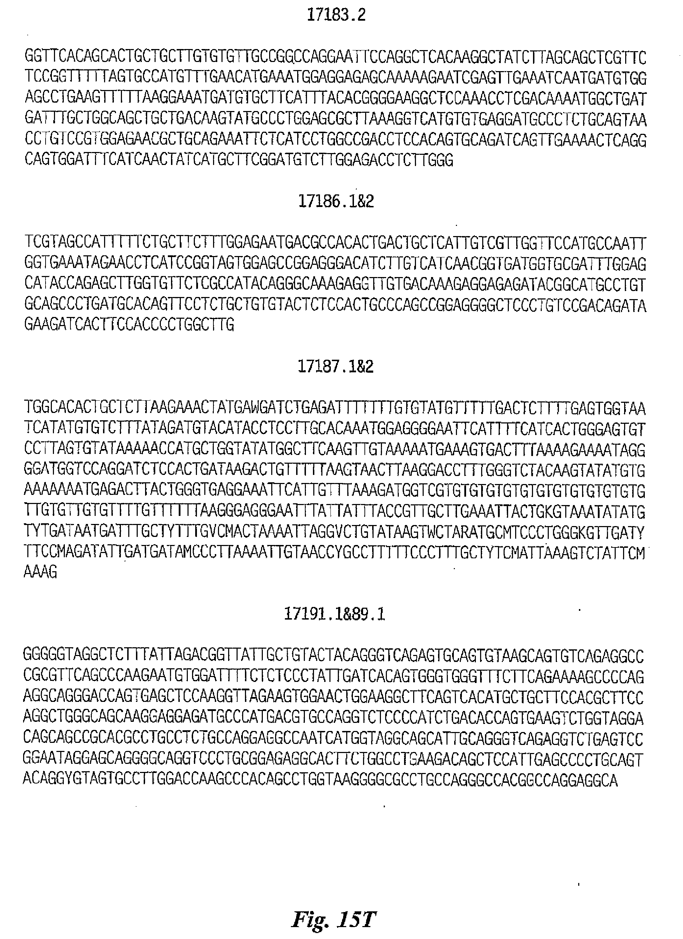

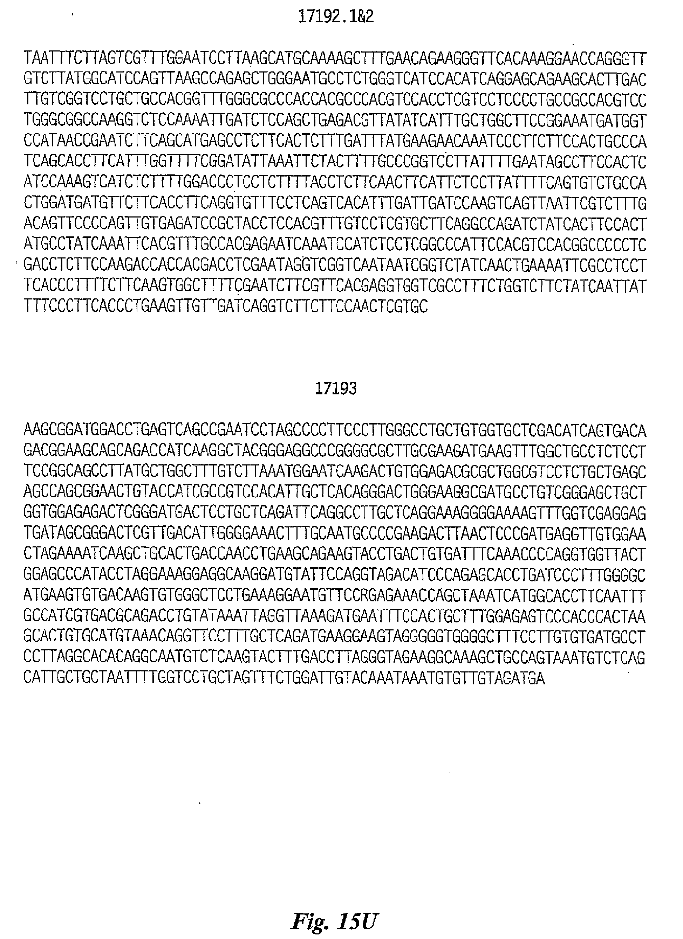

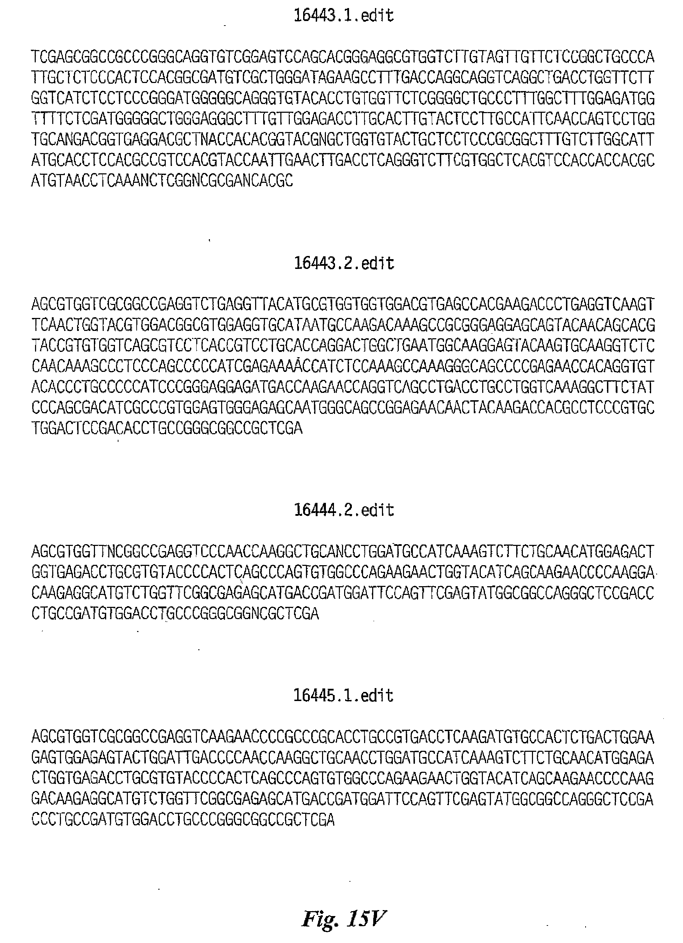

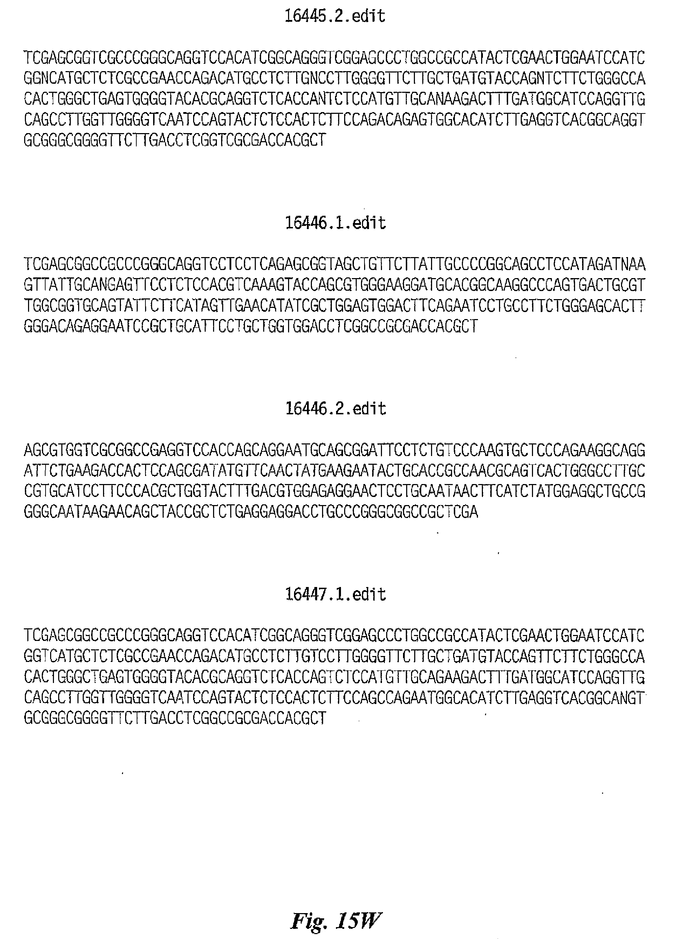

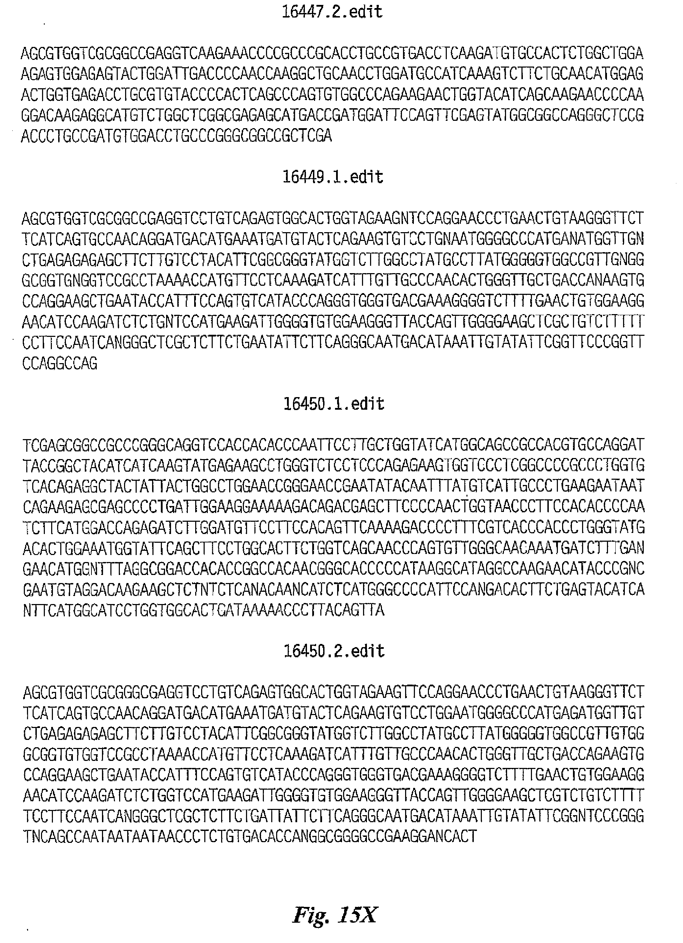

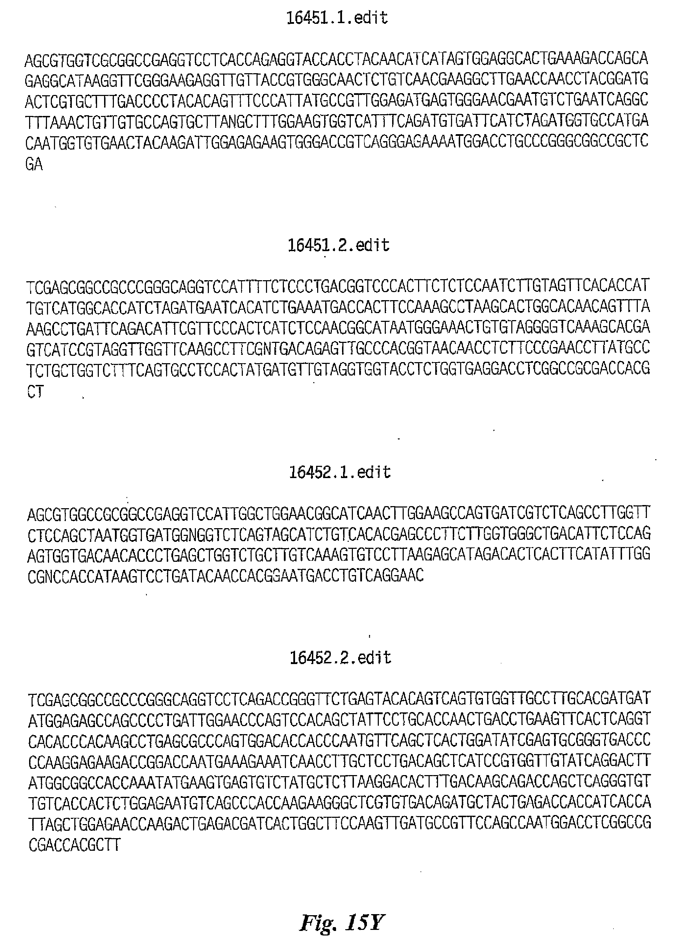

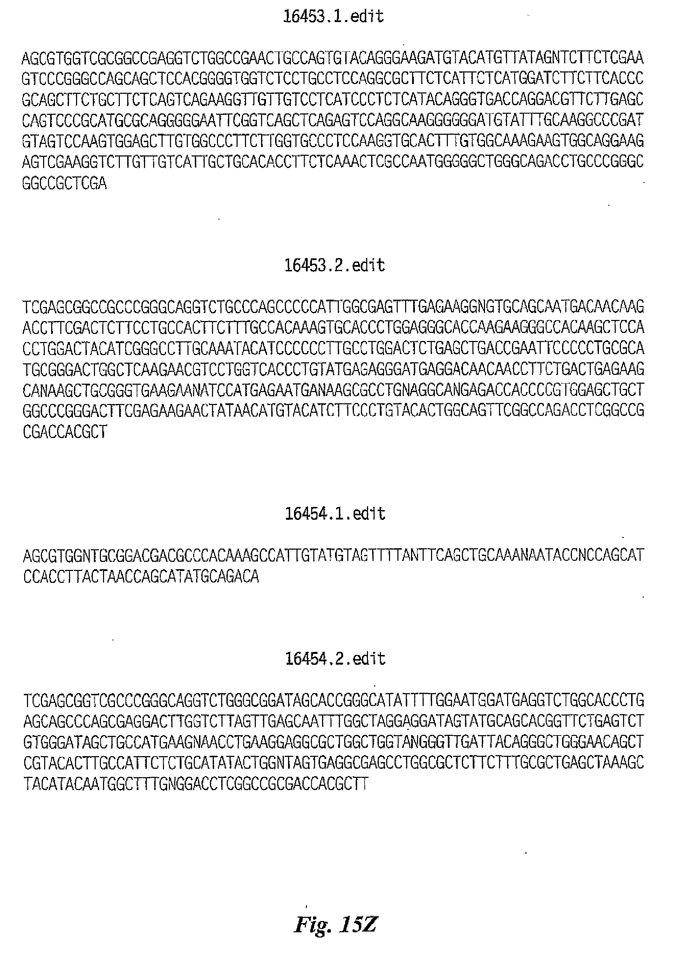

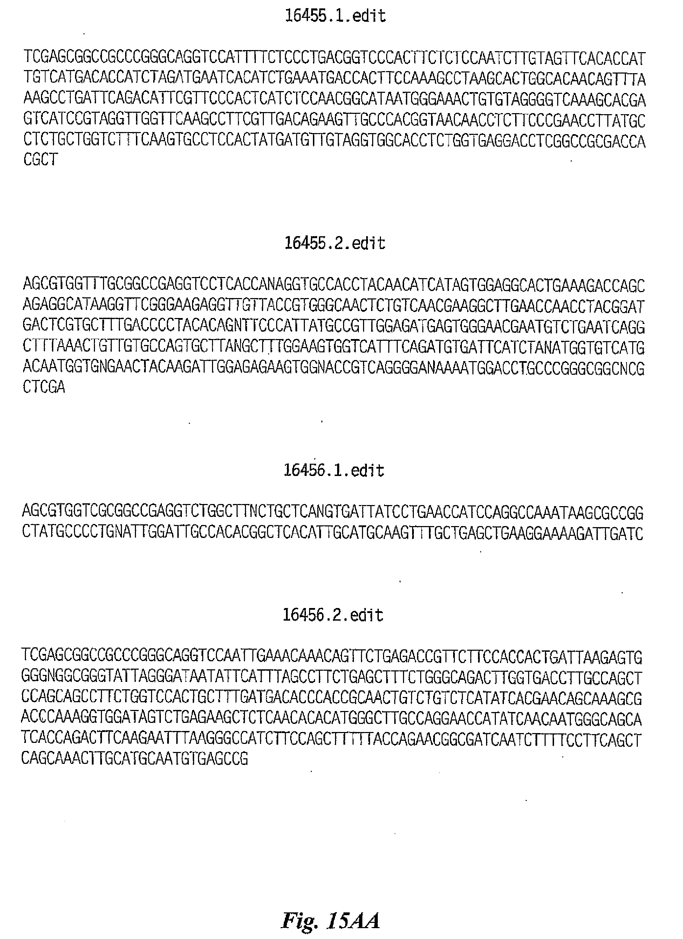

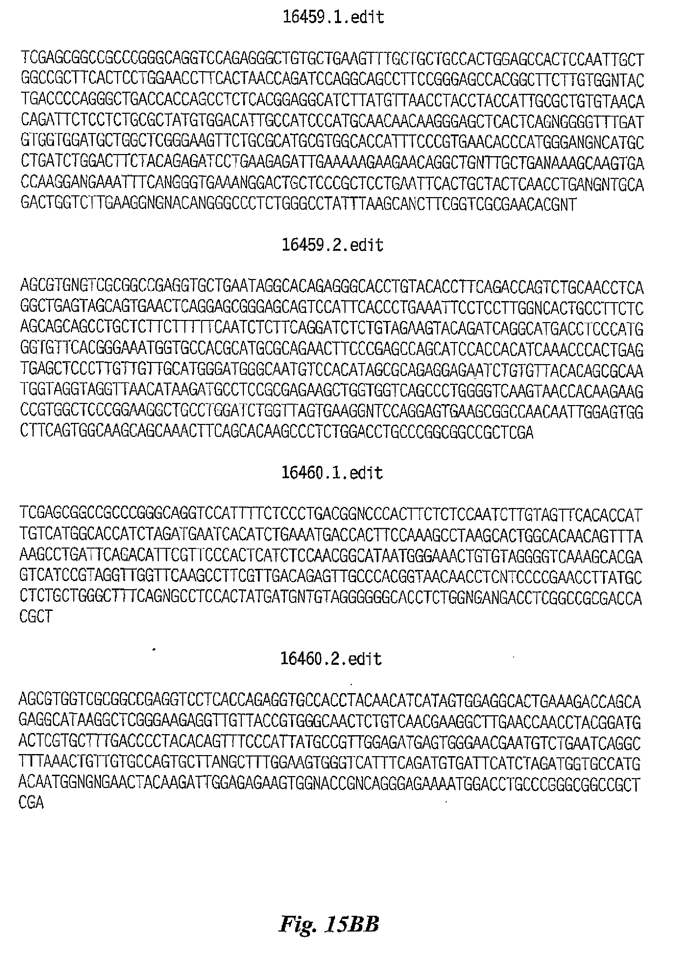

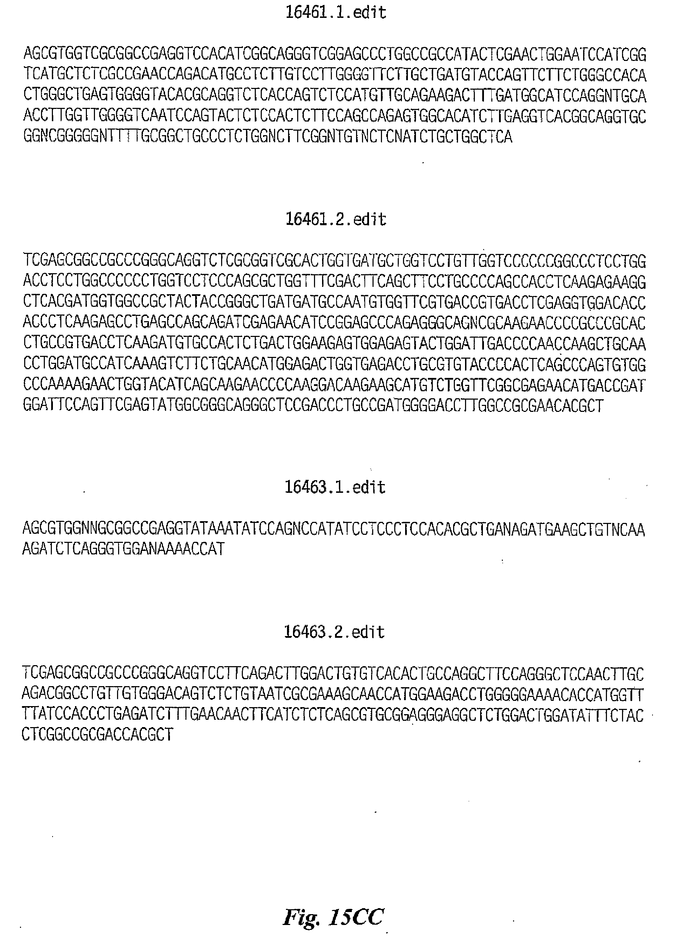

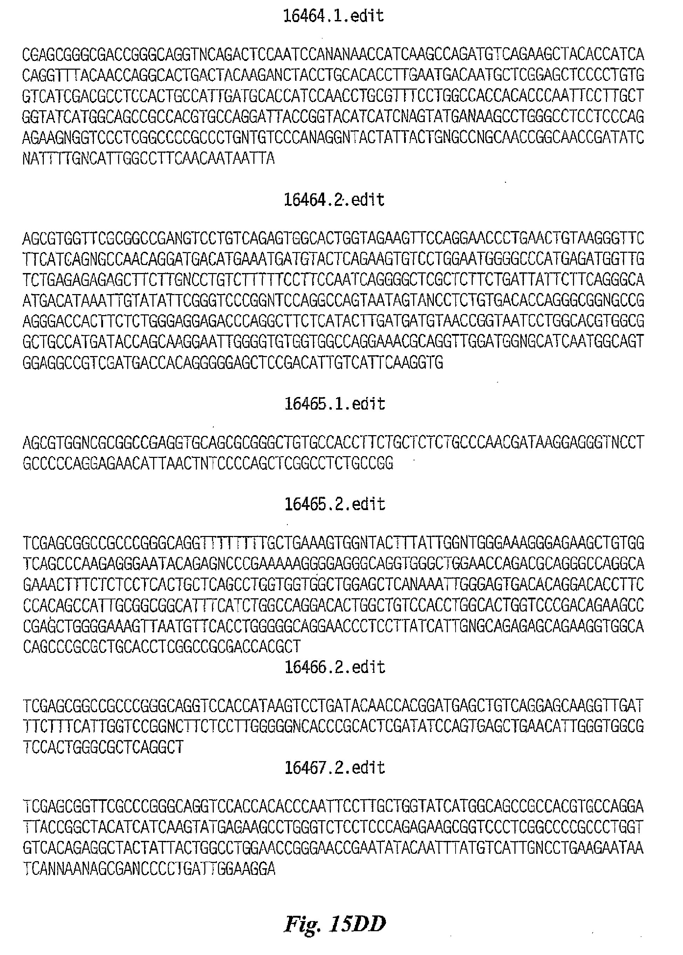

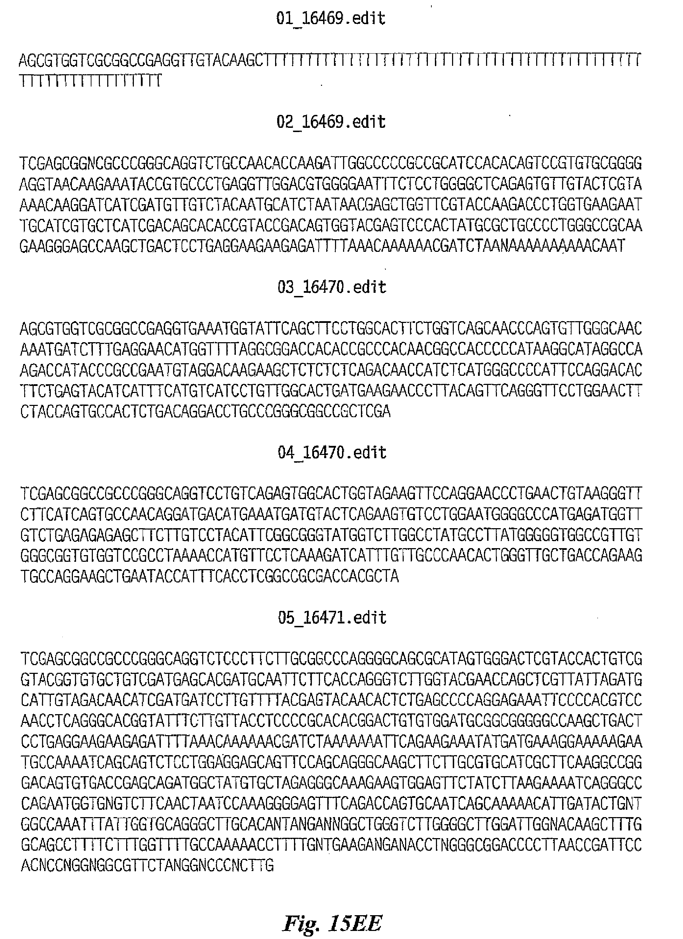

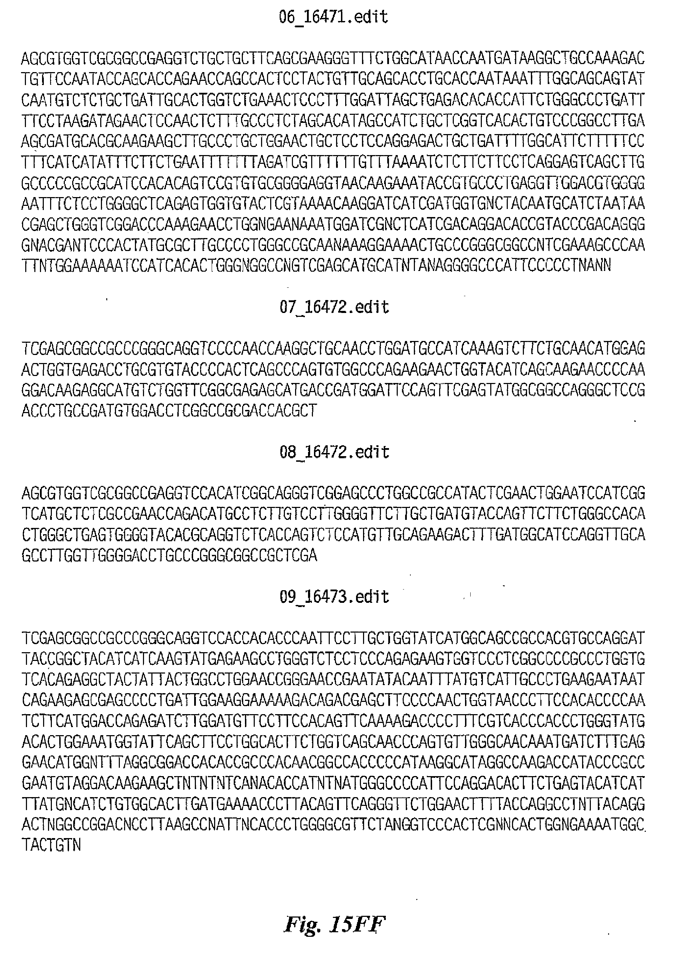

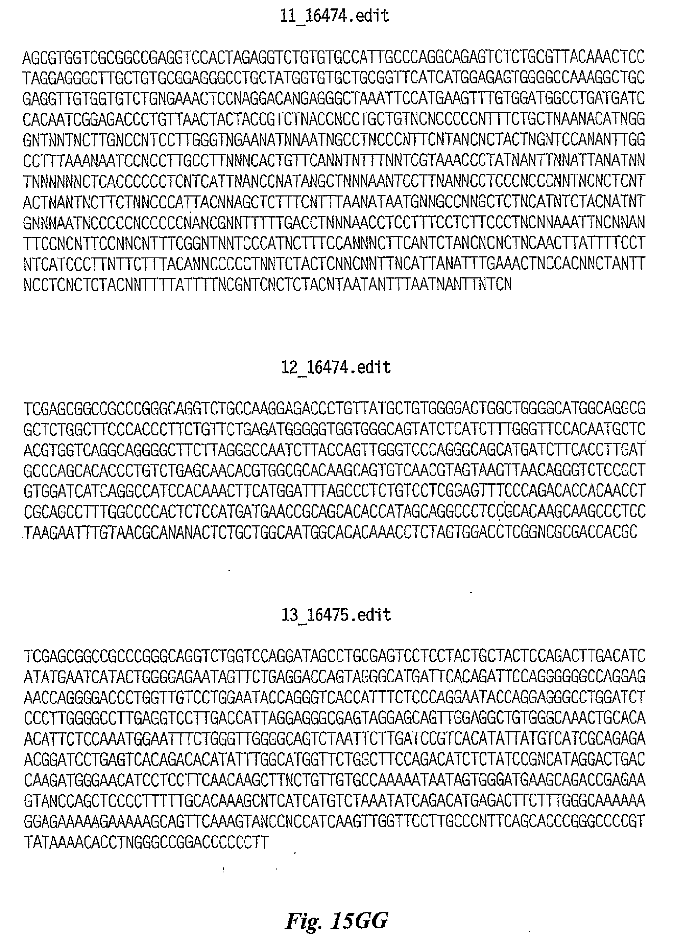

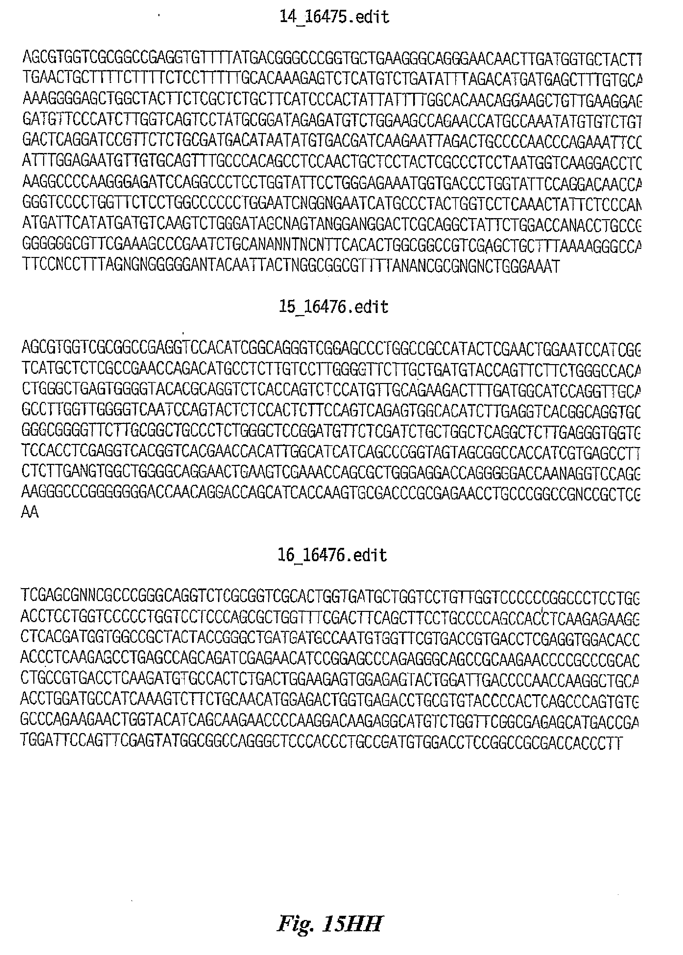

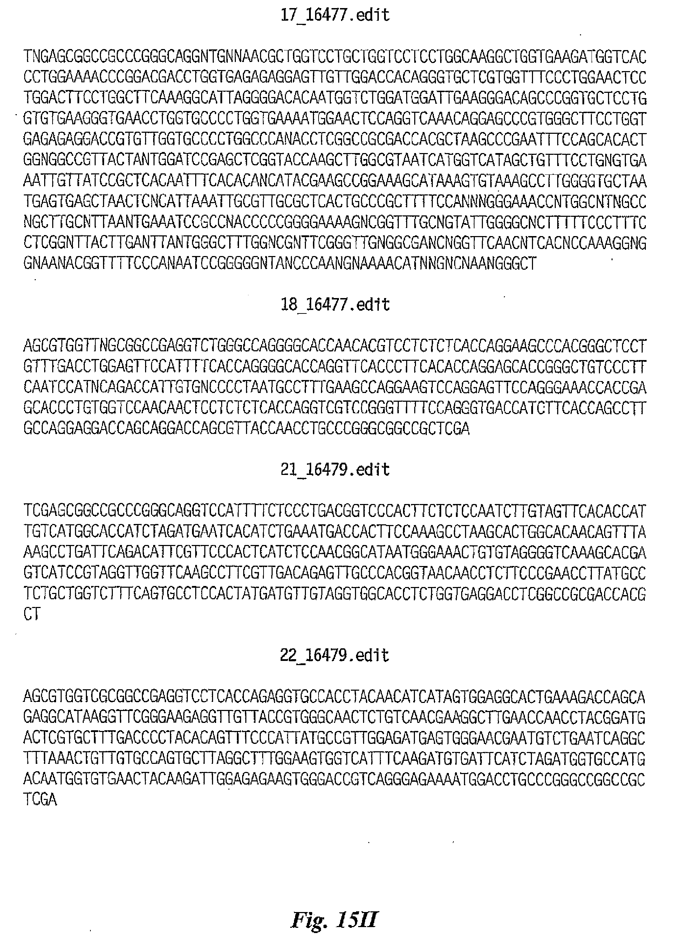

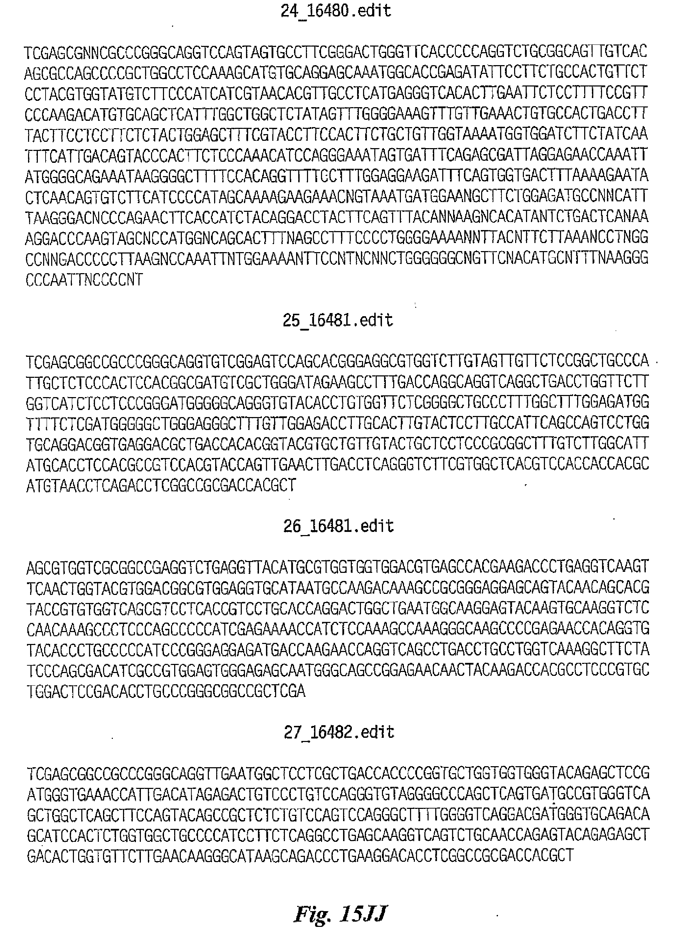

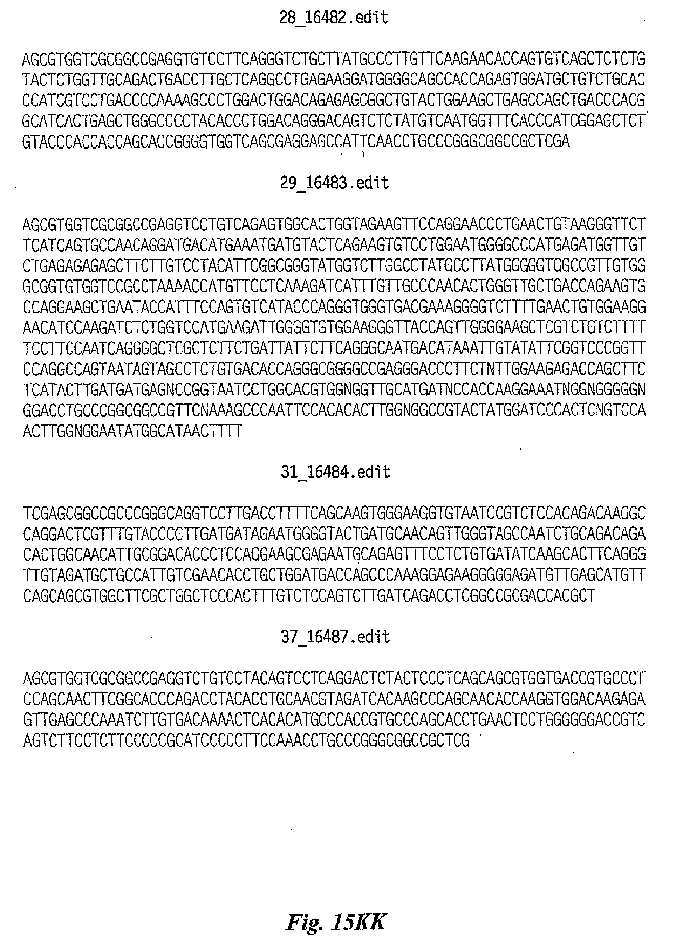

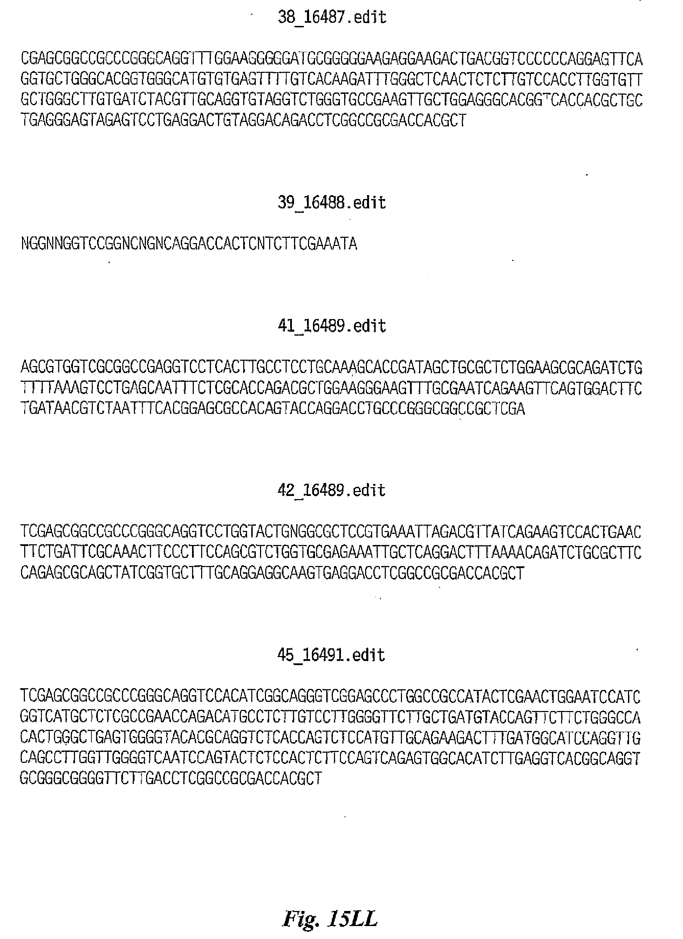

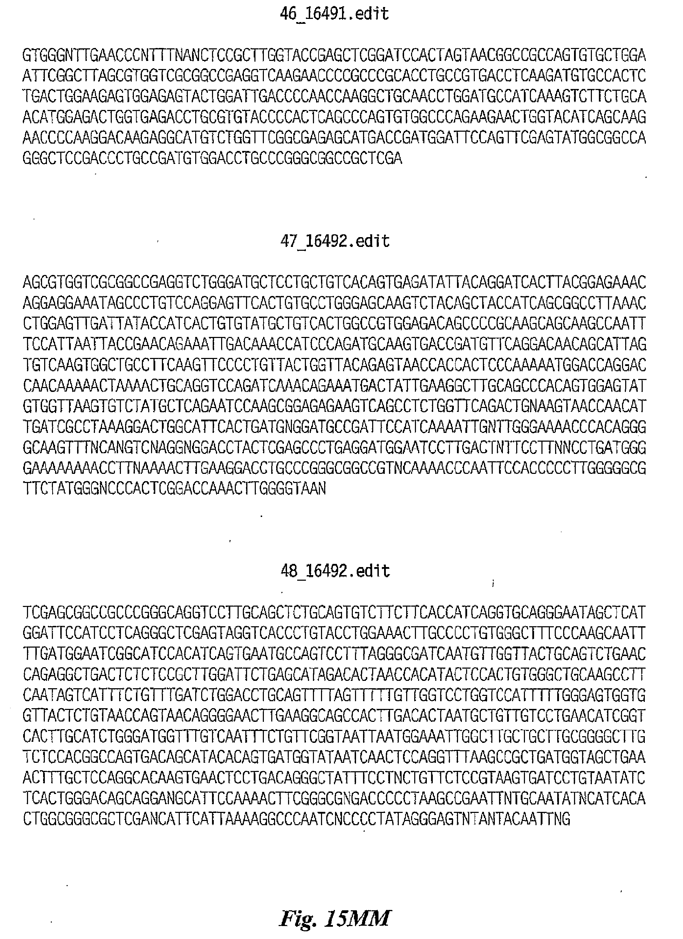

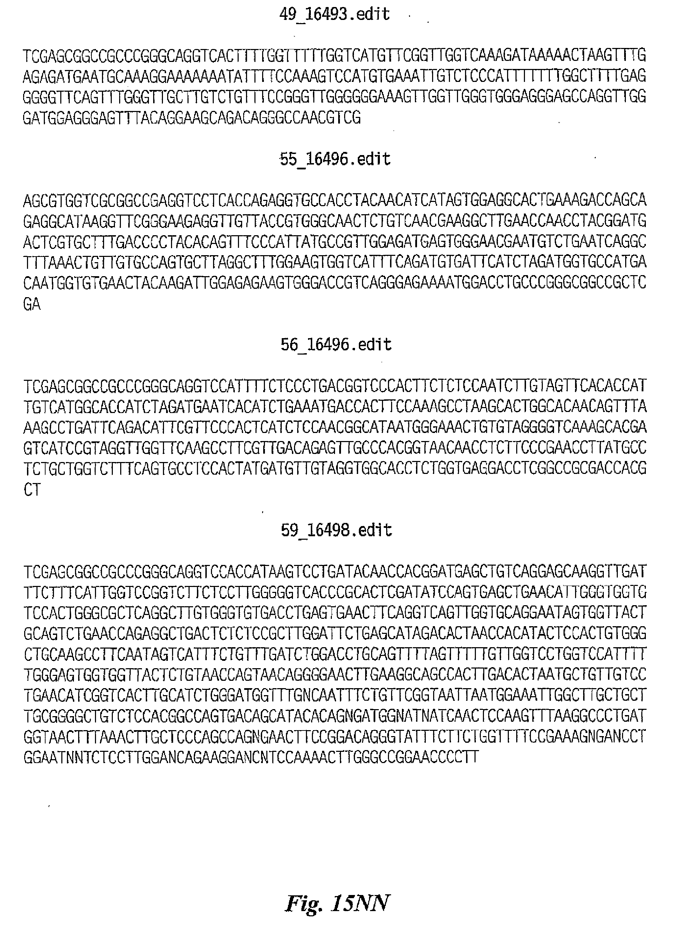

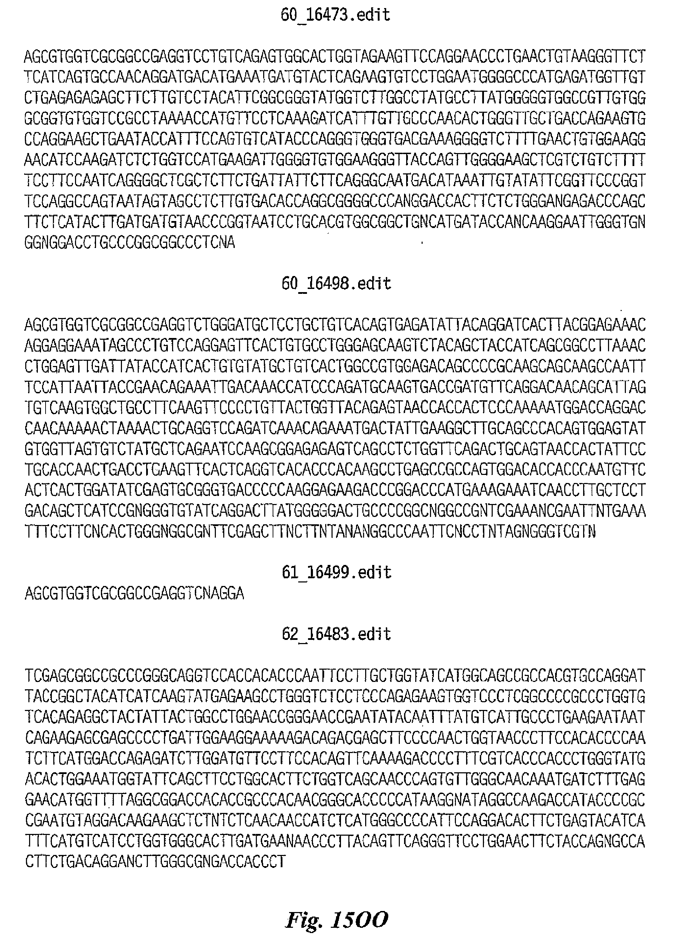

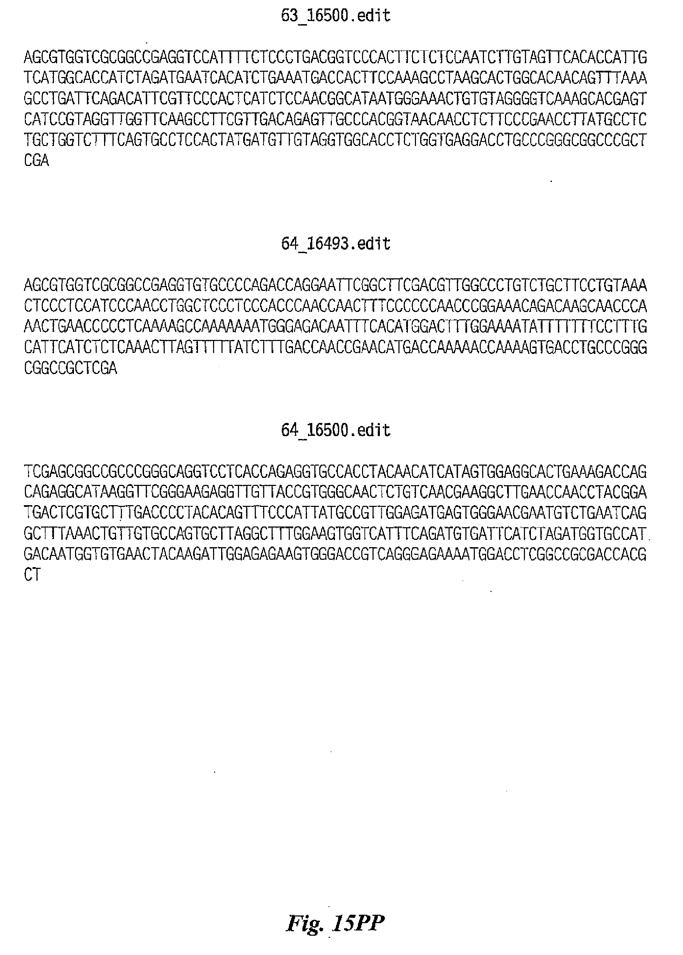

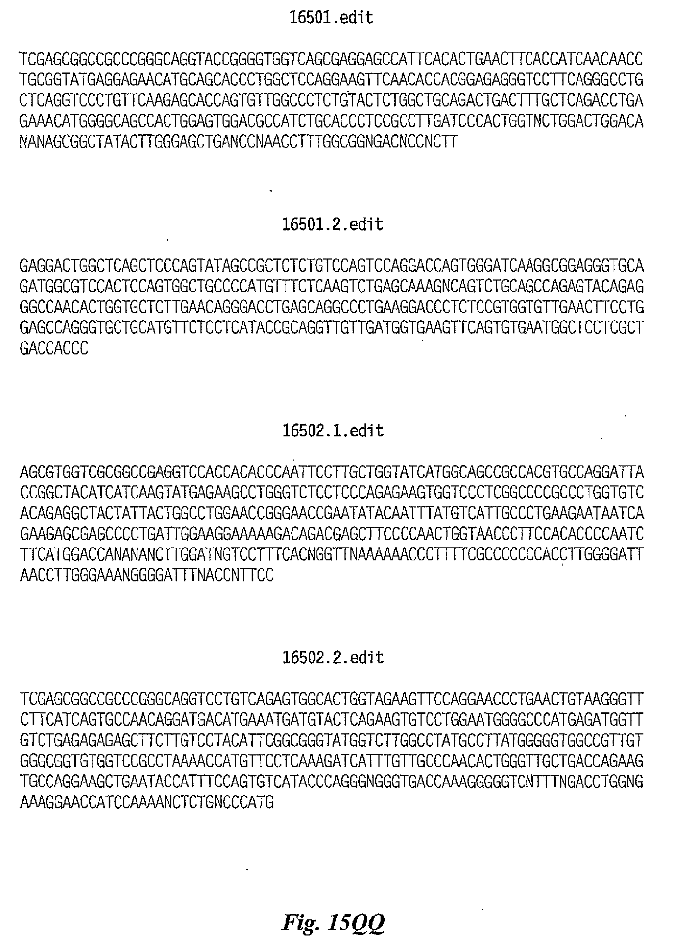

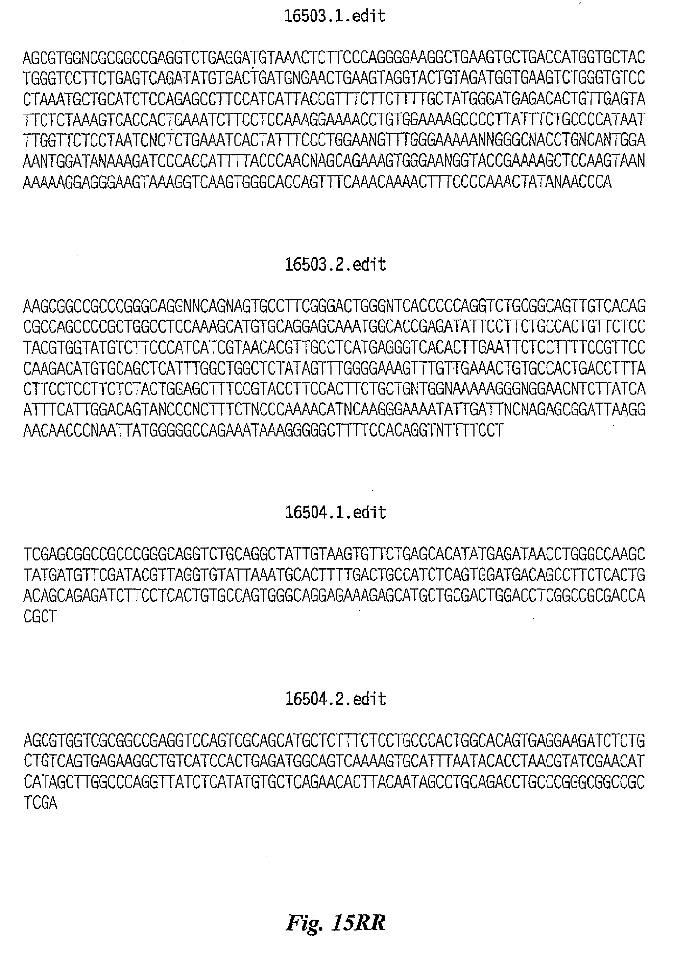

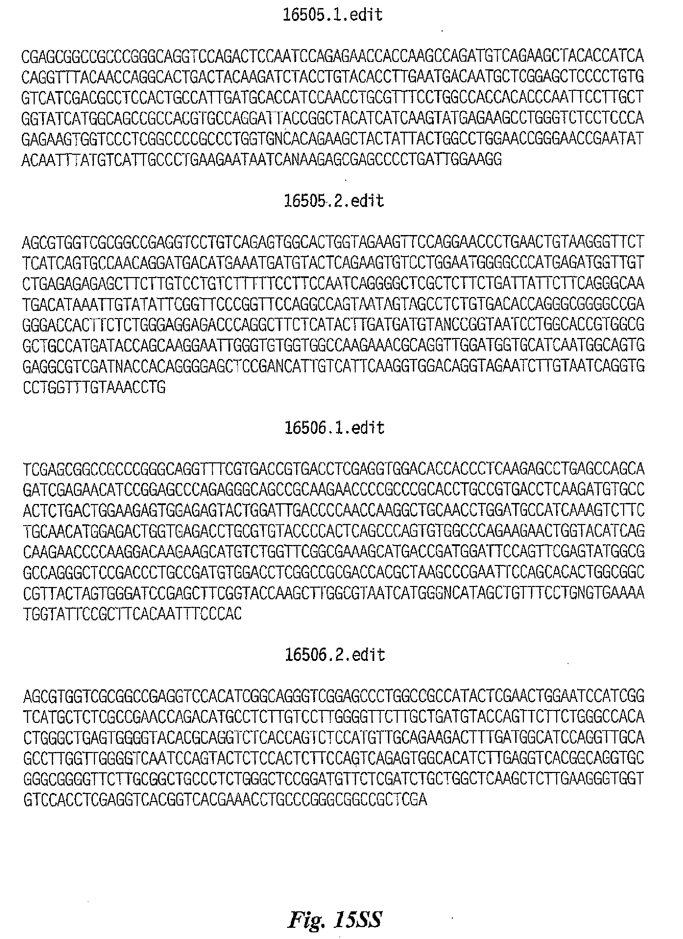

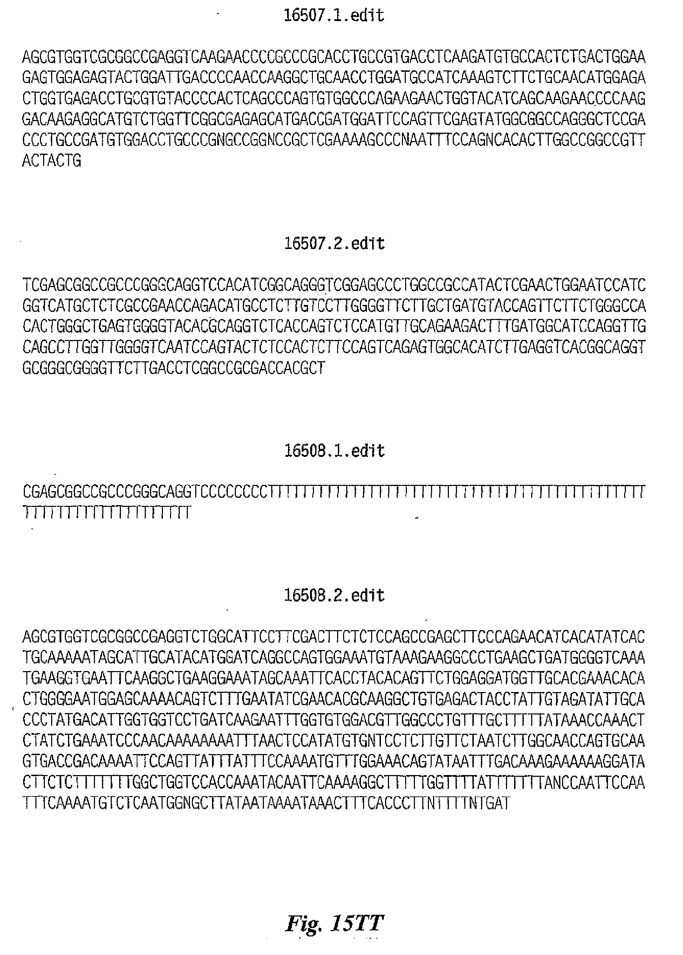

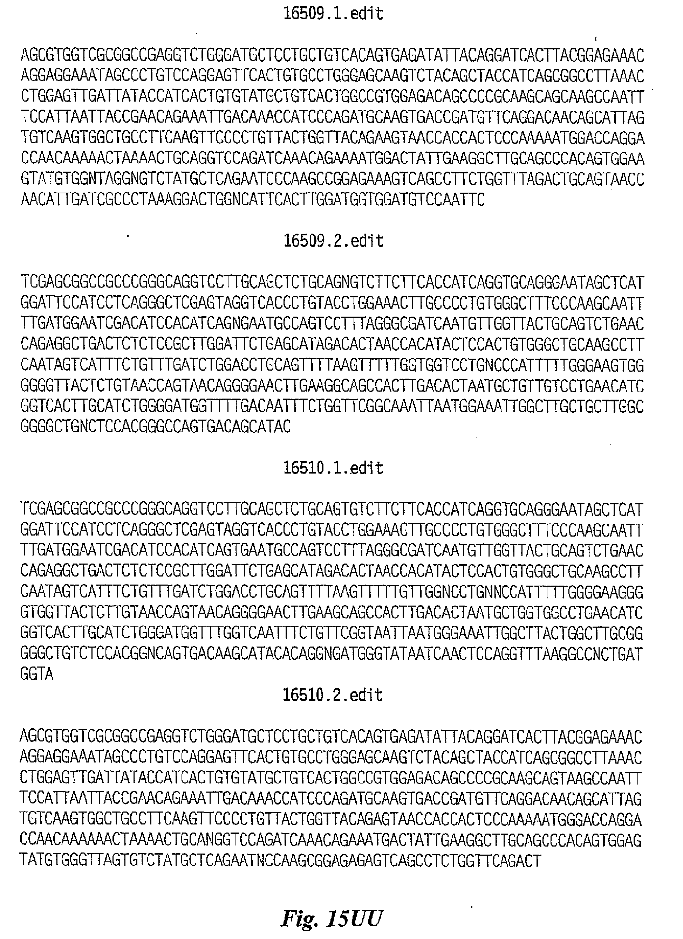

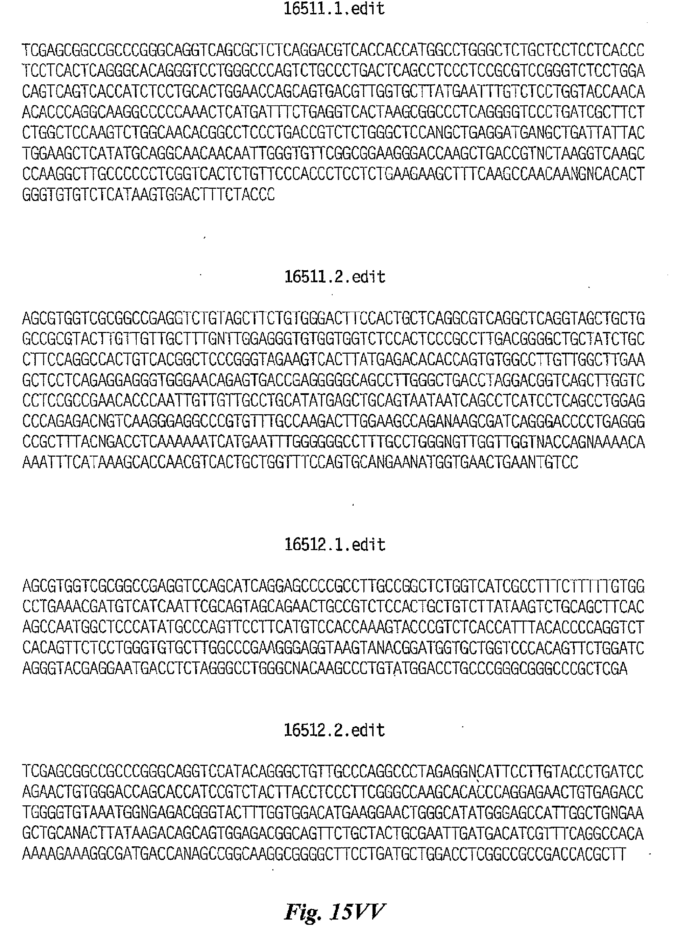

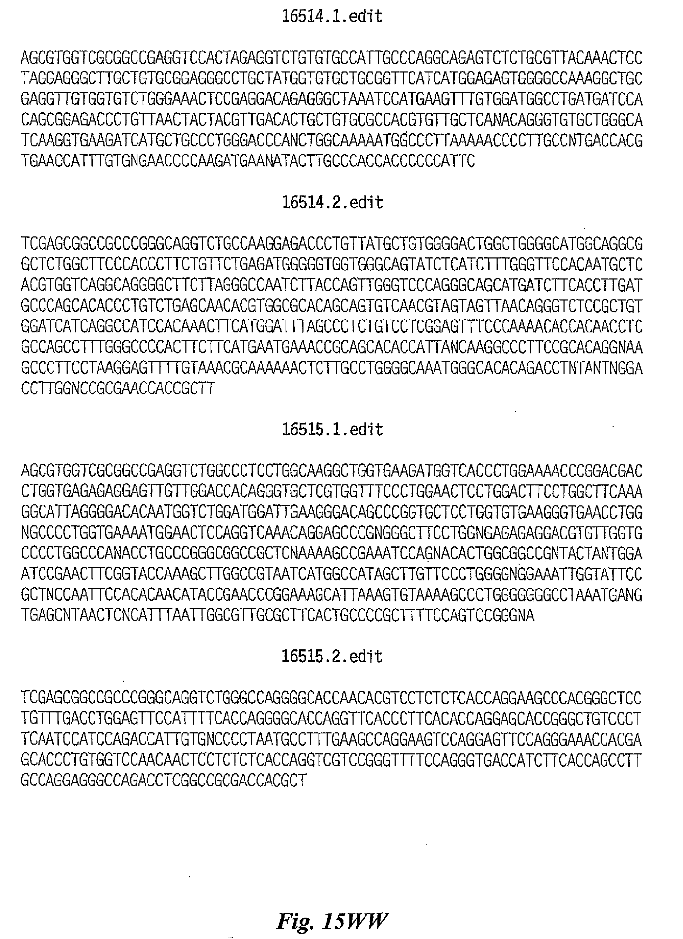

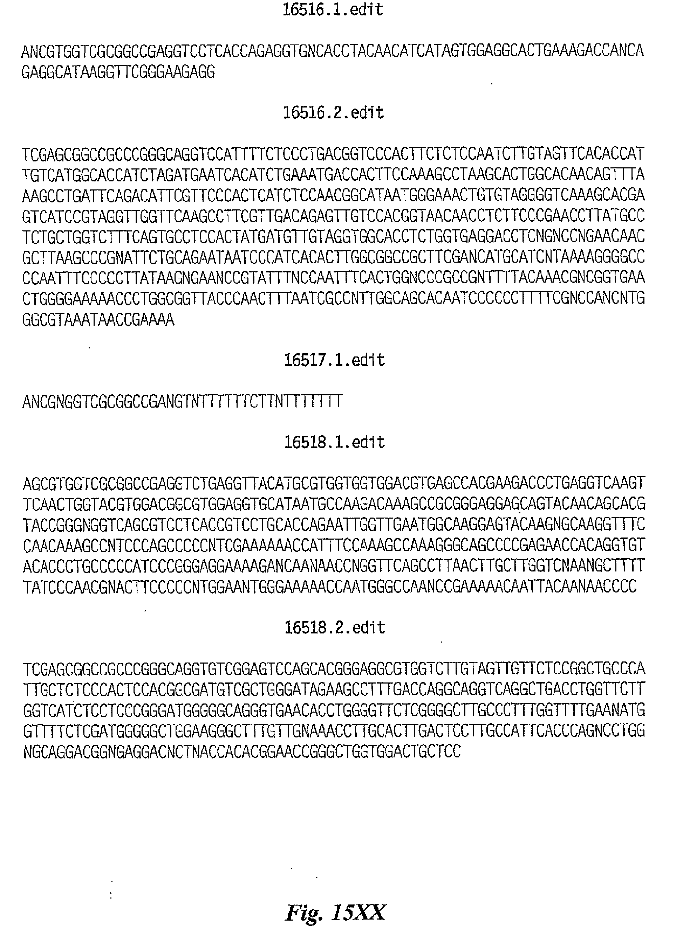

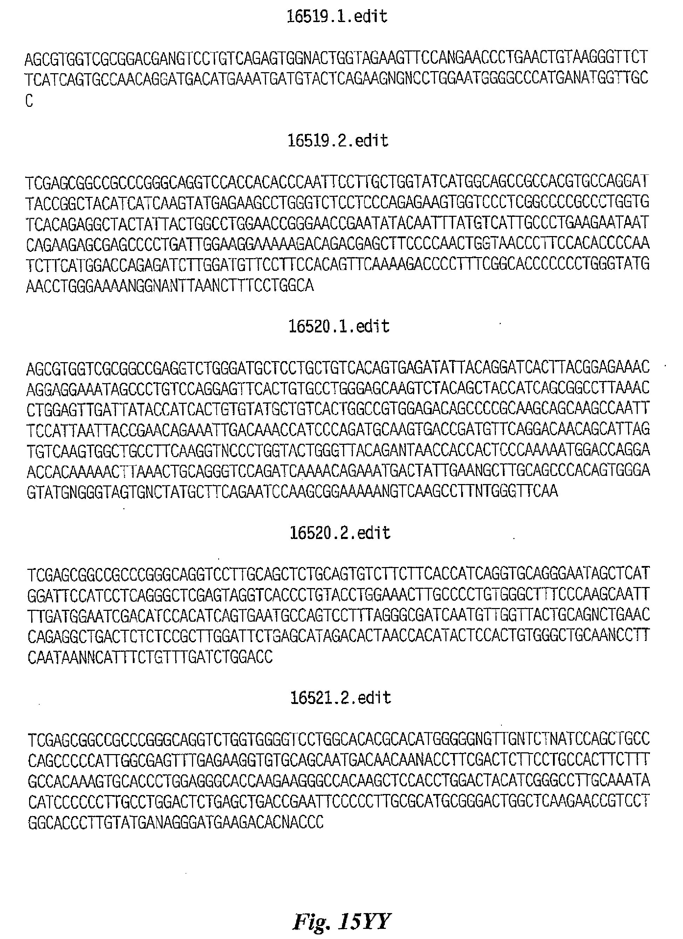

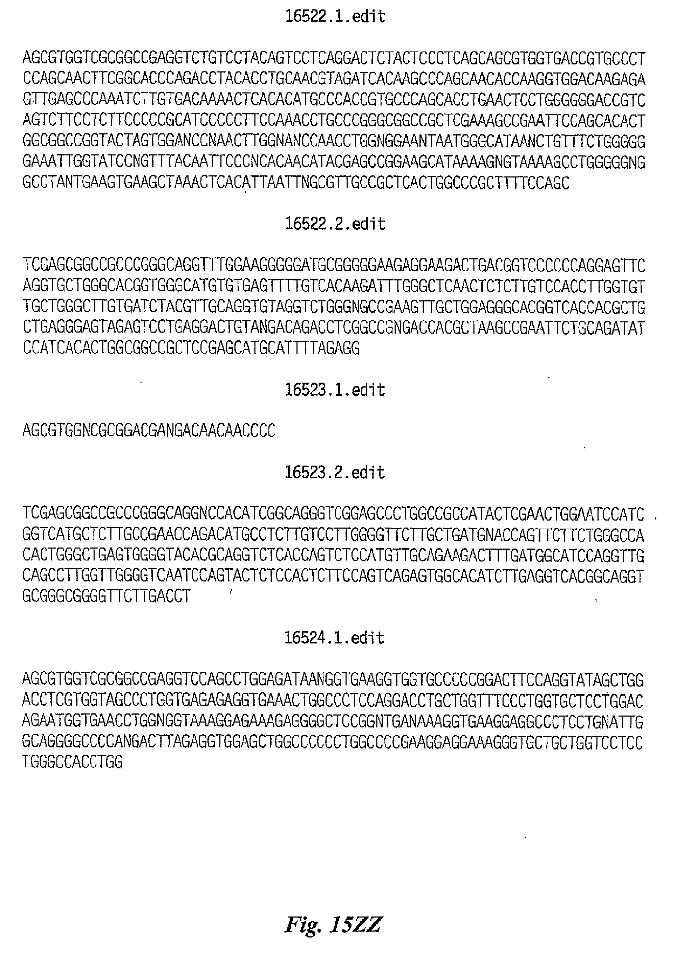

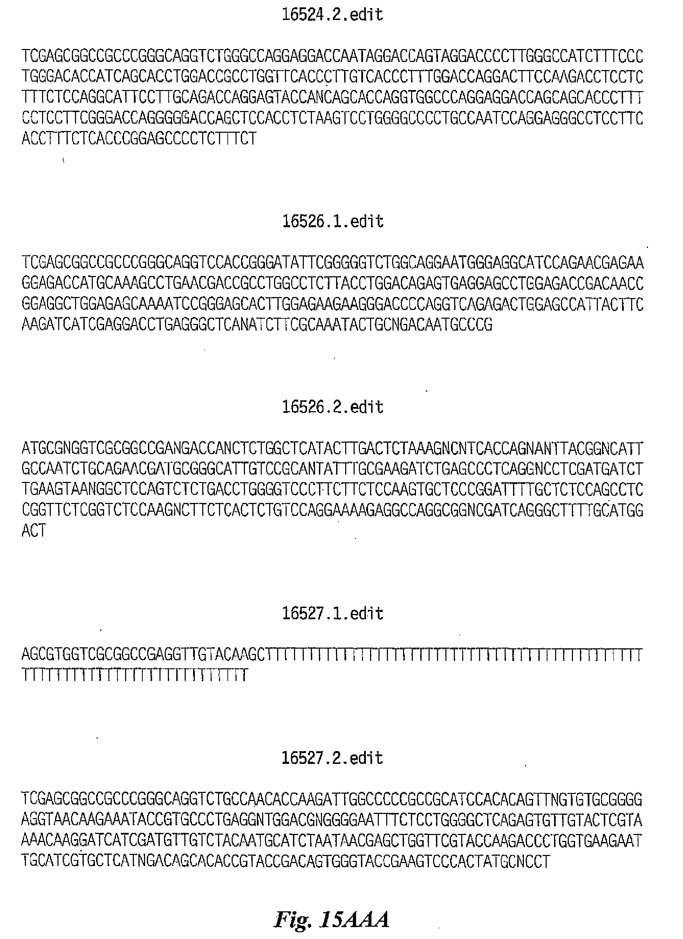

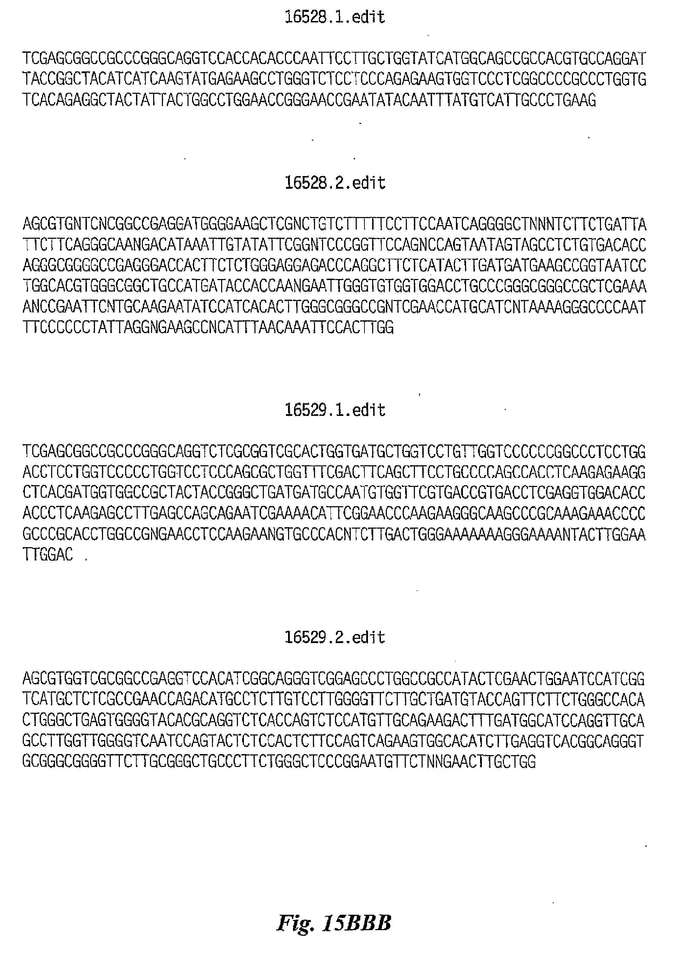

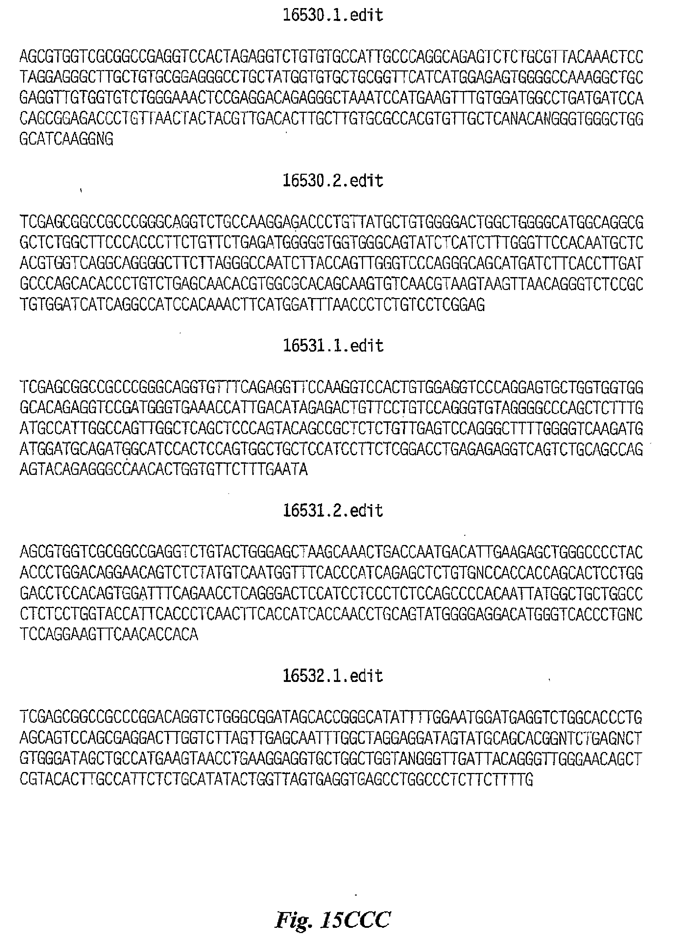

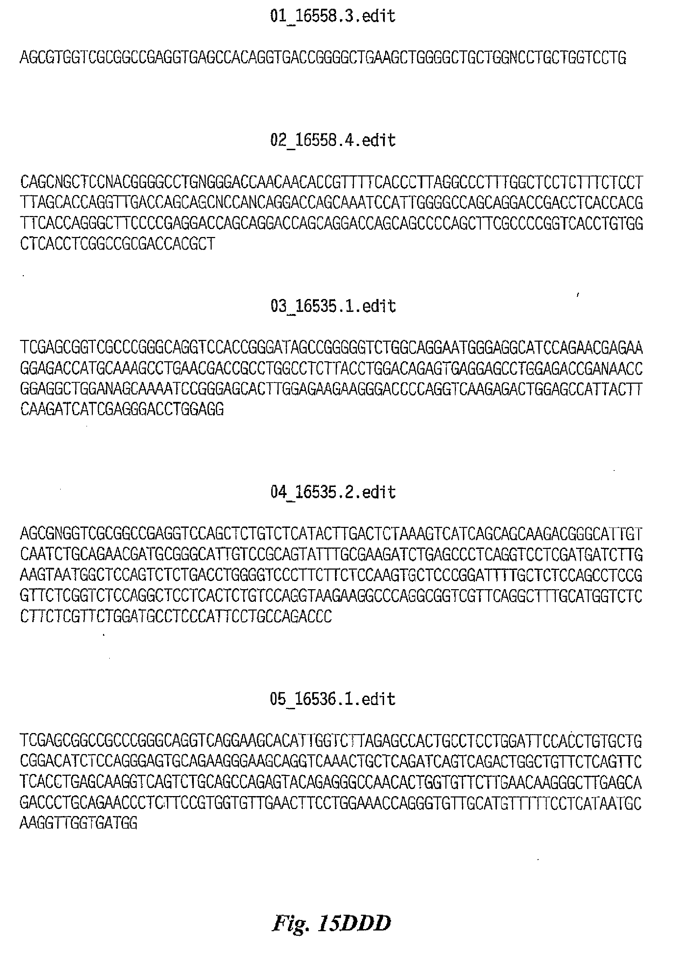

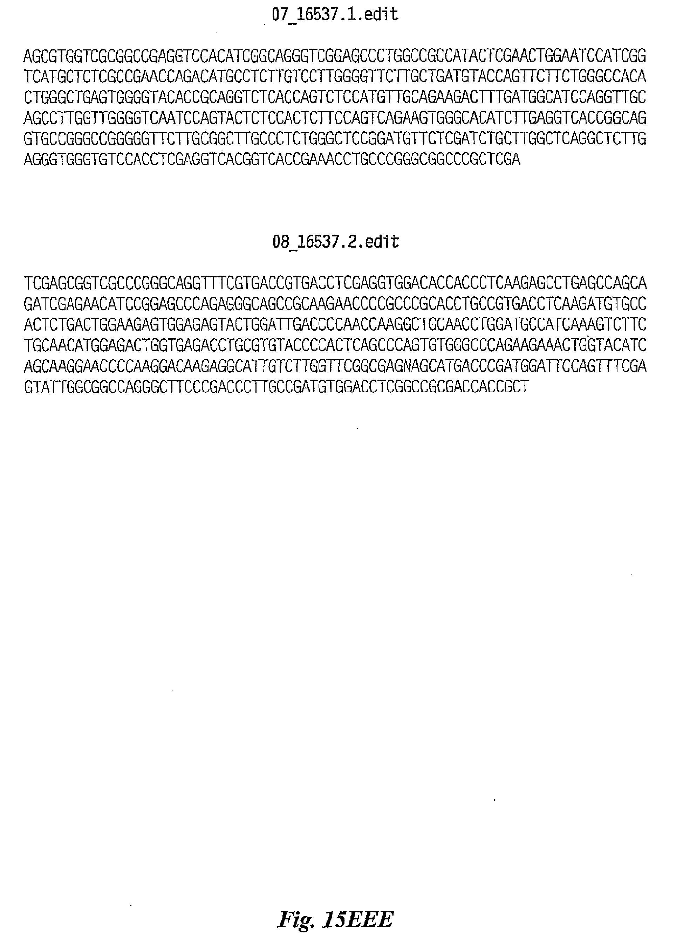

SEQ ID NO:82-310 are ovarian carcinoma antigen polynucleotides shown in Figures 15A-15EEE.

SEQ ID NO:311 is a full length sequence of ovarian carcinoma polynucleotide 0772P.

SEQ ID NO:312 is the 0772P amino acid sequence.

SEQ ID NO:313-384 are ovarian carcinoma antigen polynucleotides.

SEQ ID NO:385 represents the cDNA sequence of a form of the clone 0772P, designated 21013.

SEQ ID NO:386 represents the cDNA sequence of a form of the clone 0772P, designated 21003.

SEQ ID NO:387 represents the cDNA sequence of a form of the clone 0772P, designated 21008.

SEQ ID NOs:388 is the amino acid sequence corresponding to SEQ ID NO:385.

SEQ ID NOs:389 is the amino acid sequence corresponding to SEQ ID NO:386.SEQ ID NOs:390 is the amino acid sequence corresponding to SEQ ID NO:387.

SEQ ID NO:391 is a full length sequence of ovarian carcinoma polynucleotide 08E.

SEQ ID NO:392-393 are protein sequences encoded by O8E.

SEQ ID NO:394-415 are peptide sequences corresponding to the OE8 antibody epitopes.

SEQ ID NO:416-435 are potential HLA-A2 10-mer binding peptides predicted using the full length open-reading frame from OE8.

SEQ ID NO:436-455 are potential HLA-A2 9-mer binding peptides predicted using the full length open-reading frame from OE8.

SEQ ID NO:456 is a truncated nucleotide sequence of the full length Genbank sequence showing homology to 0772P

SEQ ID NO:457 is the full length Genbank sequence showing significant homology to 0772P

SEQ ID NO:458 is a protein encoding a truncated version of the full length Genbank sequence showing homology to 0772P

SEQ ID NO:459 is the full length protein sequence from Genbank showing significant homology to the protein sequence for 0772P

SEQ ID NO:460 encodes a unique N-terminal portion of 0772P contained in residues 1-70.

SEQ ID NO:461 contains unique sequence and encodes residues 1-313 of SEQ ID NO:456.

SEQ ID NO:462 is the hypothetical sequence for clone 0772P.

SEQ ID NO:463 is the cDNA sequence for clone FLJ14303.

SEQ ID NO:464 is a partial cDNA sequence for clone 0772P.

SEQ ID NO:465 is a partial cDNA sequence for clone 0772P.

SEQ ID NO:466 is a partial cDNA sequence for clone O772P.

SEQ ID NO:467 is a partial cDNA sequence for clone 0772P.

SEQ ID NO:468 is a partial cDNA sequence for clone 0772P.

SEQ ID NO:469 is a partial cDNA sequence for clone 0772P.

SEQ ID NO:470 is a partial cDNA sequence for clone 0772P.

SEQ ID NO:471 is a partial cDNA sequence for clone 0772P.

SEQ ID NO:472 is a partial cDNA sequence for clone 0772P.

SEQ ID NO:473 is a partial cDNA sequence for clone 0772P.

SEQ ID NO:474 is a partial cDNA sequence for clone 0772P.

SEQ ID NO:475 is a partial cDNA sequence for clone 0772P.

SEQ ID NO:476 is a partial cDNA sequence for clone 0772P.

SEQ ID NO:477 represents the novel 5'-end of the ovarian tumor antigen 0772P.

SEQ ID NO:478 is the amino acid sequence encoded by SEQ ID NO:462.

SEQ ID NO:479 is the amino acid sequence encoded by SEQ ID NO:463.

SEQ ID NO:480 is a partial amino acid sequence encoded by SEQ ID NO:472.

SEQ ID NO:481 is a partial amino acid sequence encoded by a possible open reading frame of SEQ ID NO:471.

SEQ ID NO:482 is a partial amino acid sequence encoded by a second possible open reading frame of SEQ ID NO:471.

SEQ ID NO:483 is a partial amino acid sequence encoded by SEQ ID NO:467.

SEQ ID NO:484 is a partial amino acid sequence encoded by a possible open reading frame of SEQ ID NO:466.

SEQ ID NO:485 is a partial amino acid sequence encoded by a second possible open reading frame of SEQ ID NO:466.

SEQ ID NO:486 is a partial amino acid sequence encoded by SEQ ID NO:465.

SEQ ID NO:487 is a partial amino acid sequence encoded by SEQ ID NO:464.

SEQ ID NO:488 represents the extracellular, transmembrane and cytoplasmic regions of 0772P.

SEQ ID NO:489 represents the predicted extracellular domain of 0772P.

SEQ ID NO:490 represents the amino acid sequence of peptide #2 which corresponds to an 0772P specific antibody epitope.

SEQ ID NO:491 represents the amino acid sequence of peptide #6 which corresponds to an 0772P specific antibody epitope.

SEQ ID NO:492 represents the amino acid sequence of peptide #7 which corresponds to an 0772P specific antibody epitope.

SEQ ID NO:493 represents the amino acid sequence of peptide #8 which corresponds to an 0772P specific antibody epitope.

SEQ ID NO:494 represents the amino acid sequence of peptide #9 which corresponds to an 0772P specific antibody epitope.

SEQ ID NO:495 represents the amino acid sequence of peptide #11 which corresponds to an 0772P specific antibody epitope.

SEQ ID NO:496 represents the amino acid sequence of peptide #13 which corresponds to an 0772P specific antibody epitope.

SEQ ID NO:497 represents the amino acid sequence of peptide #22 which corresponds to an 0772P specific antibody epitope.

SEQ ID NO:498 represents the amino acid sequence of peptide #24 which corresponds to an 0772P specific antibody epitope.

SEQ ID NO:499 represents the amino acid sequence of peptide #27 which corresponds to an 0772P specific antibody epitope.

SEQ ID NO:500 represents the amino acid sequence of peptide #40 which corresponds to an 0772P specific antibody epitope.

SEQ ID NO:501 represents the amino acid sequence of peptide #41 which corresponds to an 0772P specific antibody epitope.

SEQ ID NO:502 represents the amino acid sequence of peptide #47 which corresponds to an 0772P specific antibody epitope.

SEQ ID NO:503 represents the amino acid sequence of peptide #50 which corresponds to an 0772P specific antibody epitope.

SEQ ID NO:504 represents the amino acid sequence of peptide #51 which corresponds to an O772P specific antibody epitope.

SEQ ID NO:505 represents the amino acid sequence of peptide #52 which corresponds to an 0772P specific antibody epitope.

SEQ ID NO:506 represents the amino acid sequence of peptide #53 which corresponds to an 0772P specific antibody epitope.

SEQ ID NO:507 represents the amino acid sequence of peptide #58 which corresponds to an 0772P specific antibody epitope.

SEQ ID NO:508 represents the amino acid sequence of peptide #59 which corresponds to an 0772P specific antibody epitope.

SEQ ID NO:509 represents the amino acid sequence of peptide #60 which corresponds to an 0772P specific antibody epitope.

SEQ ID NO:510 represents the amino acid sequence of peptide #61 which corresponds to an 0772P specific antibody epitope.

SEQ ID NO:511 represents the amino acid sequence of peptide #71 which corresponds to an 0772P specific antibody epitope.

SEQ ID NO:512 (0772P repeat1) represents an example of a cDNA sequence corresponding to repeat number 21 from the 5' variable region of 0772P.

SEQ ID NO:513 (0772P repeat2) represents an example of a cDNA sequence corresponding to repeat number 20 from the 5' variable region of 0772P.

SEQ ID NO:514 (0772P repeat3) represents an example of a cDNA sequence corresponding to repeat number 19 from the 5' variable region of 0772P.

SEQ ID NO:515 (0772P repeat4) represents an example of a cDNA sequence corresponding to repeat number 18 from the 5' variable region of 0772P.

SEQ ID NO:516 (0772P repeat5) represents an example of a cDNA sequence corresponding to repeat number 17 from the 5' variable region of O772P.

SEQ ID NO:517 (HB repeat1) represents an example of a cDNA sequence corresponding to repeat number 21 from the 5' variable region of 0772P.

SEQ ID NO:518 (HB repeat2) represents an example of a cDNA sequence corresponding to repeat number 20 from the 5' variable region of 0772P.

SEQ ID NO:519 (HB repeat3) represents an example of a cDNA sequence corresponding to repeat number 19 from the 5' variable region of 0772P.

SEQ ID NO:520 (HB repeat4) represents an example of a cDNA sequence corresponding to repeat number 18 from the 5' variable region of 0772P.

SEQ ID NO:521 (HB repeat5) represents an example of a cDNA sequence corresponding to repeat number 17 from the 5' variable region of 0772P.

SEQ ID NO:522 (HB repeat6 5'-end) represents an example of a cDNA sequence corresponding to repeat number 16 from the 5' variable region of 0772P.

SEQ ID NO:523 (1043400.1 repeat1) represents an example of a cDNA sequence corresponding to repeat number 9 from the 5' variable region of 0772P.

SEQ ID NO:524 (1043400.1 repeat2) represents an example of a cDNA sequence corresponding to repeat number 10 from the 5' variable region of 0772P.

SEQ ID NO:525 (1043400.1 repeat3) represents an example of a cDNA sequence corresponding to repeat number 10/11 from the 5' variable region of 0772P.

SEQ ID NO:526 (1043400.1 repeat4) represents an example of a cDNA sequence corresponding to repeat number 11 from the 5' variable region of 0772P.

SEQ ID NO:527 (1043400.1 repeat5) represents an example of a cDNA sequence corresponding to repeat number 14 from the 5' variable region of 0772P.

SEQ ID NO:528 (1043400.1 repeat6) represents an example of a cDNA sequence corresponding to repeat number 17 from the 5' variable region of 0772P.

SEQ ID NO:529 (1043400.3 repeat1) represents an example of a cDNA sequence corresponding to repeat number 20 from the 5' variable region of 0772P.

SEQ ID NO:530 (1043400.3 repeat2) represents an example of a cDNA sequence corresponding to repeat number 21 from the 5' variable region of 0772P.

SEQ ID NO:531 (1043400.5 repeat1) represents an example of a cDNA sequence corresponding to repeat number 8 from the 5' variable region of O772P.

SEQ ID NO:532 (1043400.5 repeat2) represents an example of a cDNA sequence corresponding to repeat number 9 from the 5' variable region of 0772P, in addition containing intron sequence.

SEQ ID NO:533 (1043400.5 repeat2) represents an example of a cDNA sequence corresponding to repeat number 9 from the 5' variable region of 0772P.

SEQ ID NO:534 (1043400.8 repeat1) represents an example of a cDNA sequence corresponding to repeat number 17 from the 5' variable region of 0772P.

SEQ ID NO:535 (1043400.8 repeat2) represents an example of a cDNA sequence corresponding to repeat number 18 from the 5' variable region of 0772P.

SEQ ID NO:536 (1043400.8 repeat3) represents an example of a cDNA sequence corresponding to repeat number 19 from the 5' variable region of 0772P.

SEQ ID NO:537 (1043400.9 repeat1) represents an example of a cDNA sequence corresponding to repeat number 4 from the 5' variable region of 0772P.

SEQ ID NO:538 (1043400.9 repeat2) represents an example of a cDNA sequence corresponding to repeat number 5 from the 5' variable region of 0772P.

SEQ ID NO:539 (1043400.9 repeat3) represents an example of a cDNA sequence corresponding to repeat number 7 from the 5' variable region of 0772P.

SEQ ID NO:540 (1043400.9 repeat4) represents an example of a cDNA sequence corresponding to repeat number 8 from the 5' variable region of 0772P.

SEQ ID NO:541 (1043400.11 repeat1) represents an example of a cDNA sequence corresponding to repeat number 1 from the 5' variable region of 0772P.

SEQ ID NO:542 (1043400.11 repeat2) represents an example of a cDNA sequence corresponding to repeat number 2 from the 5' variable region of 0772P.

SEQ ID NO:543 (1043400.11 repeat3) represents an example of a cDNA sequence corresponding to repeat number 3 from the 5' variable region of 0772P.

SEQ ID NO:544 (1043400.11 repeat4) represents an example of a cDNA sequence corresponding to repeat number 11 from the 5' variable region of 0772P.

SEQ ID NO:545 (1043400.11 repeat5) represents an example of a cDNA sequence corresponding to repeat number 12 from the 5' variable region of 0772P.

SEQ ID NO:546 (1043400.12 repeat1) represents an example of a cDNA sequence corresponding to repeat number 20 from the 5' variable region of 0772P.

SEQ ID NO:547 (PB repeatA) represents an example of a cDNA sequence corresponding to repeat number 1 from the 5' variable region of 0772P.

SEQ ID NO:548 (PB repeatB) represents an example of a cDNA sequence corresponding to repeat number 2 from the 5' variable region of 0772P.

SEQ ID NO:549 (PB repeatE) represents an example of a cDNA sequence corresponding to repeat number 3 from the 5' variable region of 0772P.

SEQ ID NO:550 (PB repeatG) represents an example of a cDNA sequence corresponding to repeat number 4 from the 5' variable region of 0772P.

SEQ ID NO:551 (PB repeatC) represents an example of a cDNA sequence corresponding to repeat number 4 from the 5' variable region of 0772P.

SEQ ID NO:552 (PB repeatH) represents an example of a cDNA sequence corresponding to repeat number 6 from the 5' variable region of 0772P.

SEQ ID NO:553 (PB repeatJ) represents an example of a cDNA sequence corresponding to repeat number 7 from the 5' variable region of 0772P.

SEQ ID NO:554 (PB repeatK) represents an example of a cDNA sequence corresponding to repeat number 8 from the 5' variable region of O772P.

SEQ ID NO:555 (PB repeatD) represents an example of a cDNA sequence corresponding to repeat number 9 from the 5' variable region of 0772P.

SEQ ID NO:556 (PB repeatI) represents an example of a cDNA sequence corresponding to repeat number 10 from the 5' variable region of 0772P.

SEQ ID NO:557 (PB repeatM) represents an example of a cDNA sequence corresponding to repeat number 11 from the 5' variable region of 0772P.

SEQ ID NO:558 (PB repeat9) represents an example of a cDNA sequence corresponding to repeat number 12 from the 5' variable region of 0772P.

SEQ ID NO:559 (PB repeat8.5) represents an example of a cDNA sequence corresponding to repeat number 13 from the 5' variable region of 0772P.

SEQ ID NO:560 (PB repeat8) represents an example of a cDNA sequence corresponding to repeat number 14 from the 5' variable region of 0772P.

SEQ ID NO:561 (PB repeat7) represents an example of a cDNA sequence corresponding to repeat number 15 from the 5' variable region of 0772P.

SEQ ID NO:562 (PB repeat6) represents an example of a cDNA sequence corresponding to repeat number 16 from the 5' variable region of 0772P.

SEQ ID NO:563 (PB repeat5) represents an example of a cDNA sequence corresponding to repeat number 17 from the 5' variable region of 0772P.

SEQ ID NO:564 (PB repeat4) represents an example of a cDNA sequence corresponding to repeat number 18 from the 5' variable region of 0772P.

SEQ ID NO:565 (PB repeat3) represents an example of a cDNA sequence corresponding to repeat number 19 from the 5' variable region of 0772P.

SEQ ID NO:566 (PB repeat2) represents an example of a cDNA sequence corresponding to repeat number 20 from the 5' variable region of O772P.

SEQ ID NO:567 (PB repeat1) represents an example of a cDNA sequence corresponding to repeat number 21 from the 5' variable region of 0772P.

SEQ ID NO:568 represents the cDNA sequence form the 3' constant region.

SEQ ID NO:569 represents a cDNA sequence containing the consensus sequences of the 21 repeats, the 3' constant region and the 3' untranslated region.

SEQ ID NO:570 represents the cDNA sequence of the consensus repeat sequence.

SEQ ID NO:571 represents the consensus amino acid sequence of one potential open reading frame of repeat number 1 from the 5' variable region of 0772P.

SEQ ID NO:572 represents the consensus amino acid sequence of a second potential open reading frame of repeat number 1 from the 5' variable region of 0772P.

SEQ ID NO:573 represents the consensus amino acid sequence of a third potential open reading frame of repeat number 1 from the 5' variable region of 0772P.

SEQ ID NO:574 represents the consensus amino acid sequence of repeat number 2 from the 5' variable region of O772P.

SEQ ID NO:575 represents the consensus amino acid sequence of repeat number 3 from the 5' variable region of 0772P.

SEQ ID NO:576 represents the consensus amino acid sequence of repeat number 4 from the 5' variable region of 0772P.

SEQ ID NO:577 represents the consensus amino acid sequence of repeat number 5 from the 5' variable region of O772P.

SEQ ID NO:578 represents the consensus amino acid sequence of repeat number 6 from the 5' variable region of O772P.

SEQ ID NO:579 represents the consensus amino acid sequence of repeat number 7 from the 5' variable region of O772P.

SEQ ID NO:580 represents the consensus amino acid sequence of repeat number 8 from the 5' variable region of 0772P.

SEQ ID NO:581 represents the consensus amino acid sequence of repeat number 9 from the 5' variable region of O772P.

SEQ ID NO:582 represents the consensus amino acid sequence of repeat number 10 from the 5' variable region of 0772P.

SEQ ID NO:583 represents the consensus amino acid sequence of repeat number 11 from the 5' variable region of 0772P.

SEQ ID NO:584 represents the consensus amino acid sequence of repeat number 12 from the 5' variable region of 0772P.

SEQ ID NO:585 represents the consensus amino acid sequence of repeat number 13 from the 5' variable region of 0772P.

SEQ ID NO:586 represents the consensus amino acid sequence of repeat number 14 from the 5' variable region of 0772P.

SEQ ID NO:587 represents the consensus amino acid sequence of repeat number 15 from the 5' variable region of O772P.

SEQ ID NO:588 represents the consensus amino acid sequence of repeat number 16 from the 5' variable region of O772P.

SEQ ID NO:589 represents the consensus amino acid sequence of repeat number 17 from the 5' variable region of 0772P.

SEQ ID NO:590 represents the consensus amino acid sequence of repeat number 18 from the 5' variable region of 0772P.

SEQ ID NO:591 represents the consensus amino acid sequence of repeat number 19 from the 5' variable region of 0772P.

SEQ ID NO:592 represents the consensus amino acid sequence of repeat number 20 from the 5' variable region of 0772P.

SEQ ID NO:593 represents the consensus amino acid sequence of repeat number 21 from the 5' variable region of 0772P.

SEQ ID NO:594 represents the amino acid sequence of the 3' constant region.

SEQ ID NO:595 represents an amino acid sequence containing the consensus sequences of the 21 repeats and the 3' constant region.

SEQ ID NO:596 represents the amino acid sequence of the consensus repeat sequence.

Figures 1A-1S (SEQ ID NO:1-71) depict partial sequences of polynucleotides encoding representative secreted ovarian carcinoma antigens.

Figures 2A-2C depict full insert sequences for three of the clones of Figure 1. Figure 2A shows the sequence designated 07E (11731; SEQ ID NO:72), Figure 2B shows the sequence designated 09E (11785; SEQ ID NO:73) and Figure 2C shows the sequence designated 08E (13695; SEQ ID NO:74).

Figure 3 presents results of microarray expression analysis of the ovarian carcinoma sequence designated 08E.

Figure 4 presents a partial sequence of a polynucleotide (designated 3g; SEQ ID NO:75) encoding an ovarian carcinoma sequence that is a splice fusion between the human T-cell leukemia virus type I oncoprotein TAX and osteonectin.

Figure 5 presents the ovarian carcinoma polynucleotide designated 3f (SEQ ID NO:76).

Figure 6 presents the ovarian carcinoma polynucleotide designated 6b (SEQ ID NO:77).

Figures 7A and 7B present the ovarian carcinoma polynucleotides designated 8e (SEQ ID NO:78) and 8h (SEQ ID NO:79).

Figure 8 presents the ovarian carcinoma polynucleotide designated 12c (SEQ ID NO:80).

Figure 9 presents the ovarian carcinoma polynucleotide designated 12h (SEQ ID NO:81).

Figure 10 depicts results of microarray expression analysis of the ovarian carcinoma sequence designated 3f.

Figure 11 depicts results of microarray expression analysis of the ovarian carcinoma sequence designated 6b.

Figure 12 depicts results of microarray expression analysis of the ovarian carcinoma sequence designated 8e.

Figure 13 depicts results of microarray expression analysis of the ovarian carcinoma sequence designated 12c.

Figure 14 depicts results of microarray expression analysis of the ovarian carcinoma sequence designated 12h.

Figures 15A-15EEE depict partial sequences of additional polynucleotides encoding representative secreted ovarian carcinoma antigens (SEQ ID NO:82-310).

Figure 16 is a diagram illustrating the location of various partial O8E sequences within the full length sequence.

Figure 17 is a graph illustrating the results of epitope mapping studies on O8E protein.

Figure 18 is graph of a fluorescence activated cell sorting (FACS) analysis of O8E cell surface expression.

Figure 19 is graph of a FACS analysis of 08E cell surface expression.

Figure 20 shows FACS analysis results for 08E transfected HEK293 cells demonstrating cell surface expression of 08E.

Figure 21 shows FACS analysis results for SKBR3 breast tumor cells demonstrating cell surface expression of 08E.

Figure 22 shows 08E expression in HEK 293 cells. The cells were probed with anti-08E rabbit polyclonal antisera #2333L.

Figure 23 shows the ELISA analysis of anti-08E rabbit sera.

Figure 24 shows the ELISA analysis of affinity purified rabbit anti-08E polyclonal antibody.

Figure 25 is a graph determining antibody internalization of anti-O8E mAb showing that mAbs against amino acids 61-80 induces ligand internalization.

### DETAILED DESCRIPTION OF THE INVENTION

As noted above, the present invention is generally directed to compositions and methods for the therapy of cancer, such as ovarian cancer. The compositions described herein may include immunogenic polypeptides, polynucleotides encoding such polypeptides, binding agents such as antibodies that bind to a polypeptide, antigen presenting cells (APCs) and/or immune system cells (e.g., T cells).

Polypeptides of the present invention generally comprise at least an immunogenic portion of an ovarian carcinoma protein or a variant thereof. Certain ovarian carcinoma proteins have been identified using an immunoassay technique, and are referred to herein as ovarian carcinoma antigens. An "ovarian carcinoma antigen" is a protein that is expressed by ovarian tumor cells (preferably human cells) at a level that is at least two fold higher than the level in normal ovarian cells. Certain ovarian carcinoma antigens react detectably (within an immunoassay, such as an ELISA or Western blot) with antisera generated against serum from an immunodeficient animal implanted with a human ovarian tumor. Such ovarian carcinoma antigens are shed or secreted from an ovarian tumor into the sera of the immunodeficient animal. Accordingly, certain ovarian carcinoma antigens provided herein are secreted antigens. Certain nucleic acid sequences of the subject invention generally comprise a DNA or RNA sequence that encodes all or a portion of such a polypeptide, or that is complementary to such a sequence.

The present invention further provides ovarian carcinoma sequences that are identified using techniques to evaluate altered expression within an ovarian tumor. Such sequences may be polynucleotide or protein sequences. Ovarian carcinoma sequences are generally expressed in an ovarian tumor at a level that is at least two fold, and preferably at least five fold, greater than the level of expression in normal ovarian tissue, as determined using a representative assay provided herein. Certain partial ovarian carcinoma polynucleotide sequences are presented herein. Proteins encoded by genes comprising such polynucleotide sequences (or complements thereof) are also considered ovarian carcinoma proteins.

Antibodies are generally immune system proteins, or antigen-binding fragments thereof, that are capable of binding to at least a portion of an ovarian carcinoma polypeptide as described herein. T cells that may be employed within the compositions provided herein are generally T cells (e.g., CD4⁺ and/or CD8⁺) that are specific for such a polypeptide. Certain methods described herein further employ antigen-presenting cells (such as dendritic cells or macrophages) that express an ovarian carcinoma polypeptide as provided herein.

### Ovarian Carcinoma Polynucleotides

Any polynucleotide that encodes an ovarian carcinoma protein or a portion or other variant thereof as described herein is encompassed by the present invention. Preferred polynucleotides comprise at least 15 consecutive nucleotides, preferably at least 30 consecutive nucleotides, and more preferably at least 45 consecutive nucleotides, that encode a portion of an ovarian carcinoma protein. More preferably, a polynucleotide encodes an immunogenic portion of an ovarian carcinoma protein, such as an ovarian carcinoma antigen. Polynucleotides complementary to any such sequences are also encompassed by the present invention. Polynucleotides may be single-stranded (coding or antisense) or double-stranded, and may be DNA (genomic, cDNA or synthetic) or RNA molecules. Additional coding or non-coding sequences may, but need not, be present within a polynucleotide of the present invention, and a polynucleotide may, but need not, be linked to other molecules and/or support materials.

Polynucleotides may comprise a native sequence (*i.e.*, an endogenous sequence that encodes an ovarian carcinoma protein or a portion thereof) or may comprise a variant of such a sequence. Polynucleotide variants may contain one or more substitutions, additions, deletions and/or insertions such that the immunogenicity of the encoded polypeptide is not diminished, relative to a native ovarian carcinoma protein. The effect on the immunogenicity of the encoded polypeptide may generally be assessed as described herein. Variants preferably exhibit at least about 70% identity, more preferably at least about 80% identity and most preferably at least about 90% identity to a polynucleotide sequence that encodes a native ovarian carcinoma protein or a portion thereof.

The percent identity for two polynucleotide or polypeptide sequences may be readily determined by comparing sequences using computer algorithms well known to those of ordinary skill in the art, such as Megalign, using default parameters. Comparisons between two sequences are typically performed by comparing the sequences over a comparison window to identify and compare local regions of sequence similarity. A "comparison window" as used herein, refers to a segment of at least about 20 contiguous positions, usually 30 to about 75, or 40 to about 50, in which a sequence may be compared to a reference sequence of the same number of contiguous positions after the two sequences are optimally aligned. Optimal alignment of sequences for comparison may be conducted, for example, using the Megalign program in the Lasergene suite of bioinformatics software (DNASTAR, Inc., Madison, WI), using default parameters. Preferably, the percentage of sequence identity is determined by comparing two optimally aligned sequences over a window of comparison of at least 20 positions, wherein the portion of the polynucleotide or polypeptide sequence in the window may comprise additions or deletions (*i.e.,* gaps) of 20 % or less, usually 5 to 15 %, or 10 to 12%, relative to the reference sequence (which does not contain additions or deletions). The percent identity may be calculated by determining the number of positions at which the identical nucleic acid bases or amino acid residue occurs in both sequences to yield the number of matched positions, dividing the number of matched positions by the total number of positions in the reference sequence (*i.e.,* the window size) and multiplying the results by 100 to yield the percentage of sequence identity.

Variants may also, or alternatively, be substantially homologous to a native gene, or a portion or complement thereof. Such polynucleotide variants are capable of hybridizing under moderately stringent conditions to a naturally occurring DNA sequence encoding a native ovarian carcinoma protein (or a complementary sequence). Suitable moderately stringent conditions include prewashing in a solution of 5 X SSC, 0.5% SDS, 1.0 mM EDTA (pH 8.0); hybridizing at 50°C-65°C, 5 X SSC, overnight; followed by washing twice at 65°C for 20 minutes with each of 2X, 0.5X and 0.2X SSC containing 0.1% SDS.

It will be appreciated by those of ordinary skill in the art that, as a result of the degeneracy of the genetic code, there are many nucleotide sequences that encode a polypeptide as described herein. Some of these polynucleotides bear minimal homology to the nucleotide sequence of any native gene. Nonetheless, polynucleotides that vary due to differences in codon usage are specifically contemplated by the present invention. Further, alleles of the genes comprising the polynucleotide sequences provided herein are within the scope of the present invention. Alleles are endogenous genes that are altered as a result of one or more mutations, such as deletions, additions and/or substitutions of nucleotides. The resulting mRNA and protein may, but need not, have an altered structure or function. Alleles may be identified using standard techniques (such as hybridization, amplification and/or database sequence comparison).

Polynucleotides may be prepared using any of a variety of techniques. For example, an ovarian carcinoma polynucleotide may be identified, as described in more detail below, by screening a late passage ovarian tumor expression library with antisera generated against sera of immunocompetent mice after injection of such mice with sera from SCID mice implanted with late passage ovarian tumors. Ovarian carcinoma polynucleotides may also be identified using any of a variety of techniques designed to evaluate differential gene expression. Alternatively, polynucleotides may be amplified from cDNA prepared from ovarian tumor cells. Such polynucleotides may be amplified via polymerase chain reaction (PCR). For this approach, sequence-specific primers may be designed based on the sequences provided herein, and may be purchased or synthesized.

An amplified portion may be used to isolate a full length gene from a suitable library (e.g., an ovarian carcinoma cDNA library) using well known techniques. Within such techniques, a library (cDNA or genomic) is screened using one or more polynucleotide probes or primers suitable for amplification. Preferably, a library is size-selected to include larger molecules. Random primed libraries may also be preferred for identifying 5' and upstream regions of genes. Genomic libraries are preferred for obtaining introns and extending 5' sequences.

For hybridization techniques, a partial sequence may be labeled (e.g., by nick-translation or end-labeling with ³²P) using well known techniques. A bacterial or bacteriophage library is then screened by hybridizing filters containing denatured bacterial colonies (or lawns containing phage plaques) with the labeled probe (*see* Sambrook et al., Malecular Cloning: A Laboratory Manual, Cold Spring Harbor Laboratories, Cold Spring Harbor, NY, 1989). Hybridizing colonies or plaques are selected and expanded, and the DNA is isolated for further analysis. cDNA clones may be analyzed to determine the amount of additional sequence by, for example, PCR using a primer from the partial sequence and a primer from the vector. Restriction maps and partial sequences may be generated to identify one or more overlapping clones. The complete sequence may then be determined using standard techniques, which may involve generating a series of deletion clones. The resulting overlapping sequences are then assembled into a single contiguous sequence. A full length cDNA molecule can be generated by ligating suitable fragments, using well known techniques.

Alternatively, there are numerous amplification techniques for obtaining a full length coding sequence from a partial cDNA sequence. Within such techniques, amplification is generally performed via PCR. Any of a variety of commercially available kits may be used to perform the amplification step. Primers may be designed using, for example, software well known in the art. Primers are preferably 22-30 nucleotides in length, have a GC content of at least 50% and anneal to the target sequence at temperatures of about 68°C to 72°C. The amplified region may be sequenced as described above, and overlapping sequences assembled into a contiguous sequence.

One such amplification technique is inverse PCR (*see* Triglia et al., Nucl. Acids Res. 16:8186, 1988), which uses restriction enzymes to generate a fragment in the known region of the gene. The fragment is then circularized by intramolecular ligation and used as a template for PCR with divergent primers derived from the known region. Within an alternative approach, sequences adjacent to a partial sequence may be retrieved by amplification with a primer to a linker sequence and a primer specific to a known region. The amplified sequences are typically subjected to a second round of amplification with the same linker primer and a second primer specific to the known region. A variation on this procedure, which employs two primers that initiate extension in opposite directions from the known sequence, is described in WO 96/38591. Additional techniques include capture PCR (Lagerstrom et al., PCR Methods Applic. 1:111-19, 1991) and walking PCR (Parker et al., Nucl. Acids. Res. 19:3055-60, 1991). Other methods employing amplification may also be employed to obtain a full length cDNA sequence.

In certain instances, it is possible to obtain a full length cDNA sequence by analysis of sequences provided in an expressed sequence tag (EST) database, such as that available from GenBank. Searches for overlapping ESTs may generally be performed using well known programs (*e.g.,* NCBI BLAST searches), and such ESTs may be used to generate a contiguous full length sequence.

Certain nucleic acid sequences of cDNA molecules encoding portions of ovarian carcinoma antigens are provided in Figures 1A-1S (SEQ ID NO:1 to 71) and Figures 15A to 15EEE (SEQ ID NO:82 to 310). The sequences provided in Figures 1A-1S appear to be novel. For sequences in Figures 15A-15EEE, database searches revealed matches having substantial identity. These polynucleotides were isolated by serological screening of an ovarian tumor cDNA expression library, using a technique designed to identify secreted tumor antigens. Briefly, a late passage ovarian tumor expression library was prepared from a SCID-derived human ovarian tumor (OV9334) in the vector λ-screen (Novagen). The sera used for screening were obtained by injecting immunocompetent mice with sera from SCID mice implanted with one late passage ovarian tumors. This technique permits the identification of cDNA molecules that encode immunogenic portions of secreted tumor antigens.

The polynucleotides recited herein, as well as full length polynucleotides comprising such sequences, other portions of such full length polynucleotides, and sequences complementary to all or a portion of such full length molecules, are specifically encompassed by the present invention. It will be apparent to those of ordinary skill in the art that this technique can also be applied to the identification of antigens that are secreted from other types of tumors.

Other nucleic acid sequences of cDNA molecules encoding portions of ovarian carcinoma proteins are provided in Figures 4-9 (SEQ ID NO:75-81), as well as SEQ ID NO:313-384. These sequences were identified by screening a microarray of cDNAs for tumor-associated expression. (*i*.*e*., expression that is at least five fold greater in an ovarian tumor than in normal ovarian tissue, as determined using a representative assay provided herein). Such screens were performed using a Synteni microarray (Palo Alto, CA) according to the manufacturer's instructions (and essentially as described by Schena et al., Proc. Natal. Acad. Sci. USA 93:10614-10619, 1996 and Heller et al., Proc. Natl. Acad. Sci. USA 94:2150-2155, 1997). SEQ ID NO:311 and 391 provide full length sequences incorporating certain of these nucleic acid sequences.

Any of a variety of well known techniques may be used to evaluate tumor-associated expression of a cDNA. For example, hybridization techniques using labeled polynucleotide probes may be employed. Alternatively, or in addition, amplification techniques such as real-time PCR may be used *(see* Gibson et al., Genome Research 6:995-1001, 1996; Heid et al., Genome Research 6:986-994, 1996). Real-time PCR is a technique that evaluates the level of PCR product accumulation during amplification. This technique permits quantitative evaluation of mRNA levels in multiple samples. Briefly, mRNA is extracted from tumor and normal tissue and cDNA is prepared using standard techniques. Real-time PCR may be performed, for example, using a Perkin Elmer/Applied Biosystems (Foster City, CA) 7700 Prism instrument. Matching primers and fluorescent probes may be designed for genes of interest using, for example, the primer express program provided by Perkin Elmer/Applied Biosystems (Foster City, CA). Optimal concentrations of primers and probes may be initially determined by those of ordinary skill in the art, and control (*e*.*g*., β-actin) primers and probes may be obtained commercially from, for example, Perkin Elmer/Applied Biosystems (Foster City, CA). To quantitate the amount of specific RNA in a sample, a standard curve is generated alongside using a plasmid containing the gene of interest. Standard curves may be generated using the Ct values determined in the real-time PCR, which are related to the initial cDNA concentration used in the assay. Standard dilutions ranging from 10-10⁶ copies of the gene of interest are generally sufficient. In addition, a standard curve is generated for the control sequence. This permits standardization of initial RNA content of a tissue sample to the amount of control for comparison purposes.

Polynucleotide variants may generally be prepared by any method known in the art, including chemical synthesis by, for example, solid phase phosphoramidite chemical synthesis. Modifications in a polynucleotide sequence may also be introduced using standard mutagenesis techniques, such as oligonucleotide-directed site-specific mutagenesis (*see* Adelman et al., DNA 2:183, 1983). Alternatively, RNA molecules may be generated by *in vitro* or *in vivo* transcription of DNA sequences encoding an ovarian carcinoma antigen, or portion thereof, provided that the DNA is incorporated into a vector with a suitable RNA polymerase promoter (such as T7 or SP6). Certain portions may be used to prepare an encoded polypeptide, as described herein. In addition, or alternatively, a portion may be administered to a patient such that the encoded polypeptide is generated *in vivo*.

A portion of a sequence complementary to a coding sequence (*i*.*e*., an antisense polynucleotide) may also be used as a probe or to modulate gene expression. cDNA constructs that can be transcribed into antisense RNA may also be introduced into cells or tissues to facilitate the production of antisense RNA. An antisense polynucleotide may be used, as described herein, to inhibit expression of an ovarian carcinoma protein. Antisense technology can be used to control gene expression through triple-helix formation, which compromises the ability of the double helix to open sufficiently for the binding of polymerases, transcription factors or regulatory molecules (*see* Gee et al., In Huber and Carr, Molecular and Immunologic Approaches, Futura Publishing Co. (Mt. Kisco, NY; 1994). Alternatively, an antisense molecule may be designed to hybridize with a control region of a gene (*e*.*g*., promoter, enhancer or transcription initiation site), and block transcription of the gene; or to block translation by inhibiting binding of a transcript to ribosomes.

Any polynucleotide may be further modified to increase stability *in vivo*. Possible modifications include, but are not limited to, the addition of flanking sequences at the 5' and/or 3' ends; the use of phosphorothioate or 2' O-methyl rather than phosphodiesterase linkages in the backbone; and/or the inclusion of nontraditional bases such as inosine, queosine and wybutosine, as well as acetyl- methyl-, thio- and other modified forms of adenine, cytidine, guanine, thymine and uridine.

Nucleotide sequences as described herein may be joined to a variety of other nucleotide sequences using established recombinant DNA techniques. For example, a polynucleotide may be cloned into any of a variety of cloning vectors, including plasmids, phagemids, lambda phage derivatives and cosmids. Vectors of particular interest include expression vectors, replication vectors, probe generation vectors and sequencing vectors. In general, a vector will contain an origin of replication functional in at least one organism, convenient restriction endonuclease sites and one or more selectable markers. Other elements will depend upon the desired use, and will be apparent to those of ordinary sldll in the art.

Within certain embodiments, polynucleotides may be formulated so as to permit entry into a cell of a mammal, and expression therein. Such formulations are particularly useful for therapeutic purposes, as described below. Those of ordinary skill in the art will appreciate that there are many ways to achieve expression of a polynucleotide in a target cell, and any suitable method may be employed. For example, a polynucleotide may be incorporated into a viral vector such as, but not limited to, adenovirus, adeno-associated virus, retrovirus, or vaccinia or other pox virus (e.g., avian pox virus). Techniques for incorporating DNA into such vectors are well known to those of ordinary skill in the art. A retroviral vector may additionally transfer or incorporate a gene for a selectable marker (to aid in the identification or selection of transduced cells) and/or a targeting moiety, such as a gene that encodes a ligand for a receptor on a specific target cell, to render the vector target specific. Targeting may also be accomplished using an antibody, by methods known to those of ordinary skill in the art.

Other formulations for therapeutic purposes include colloidal dispersion systems, such as macromolecule complexes, nanocapsules, microspheres, beads, and lipid-based systems including oil-in-water emulsions, micelles, mixed micelles, and liposomes. A preferred colloidal system for use as a delivery vehicle *in vitra* and *in vivo* is a liposome (*i*.*e*., an artificial membrane vesicle). The preparation and use of such systems is well known in the art.

### Ovarian Carcinoma Polypeptides

Within the context of the present invention, polypeptides may comprise at least an immunogenic portion of an ovarian carcinoma protein or a variant thereof, as described herein. As noted above, certain ovarian carcinoma proteins are ovarian carcinoma antigens that are expressed by ovarian tumor cells and react detectably within an immunoassay (such as an ELISA) with antisera generated against serum from an immunodeficient animal implanted with an ovarian tumor. Other ovarian carcinoma proteins are encoded by ovarian carcinoma polynucleotides recited herein. Polypeptides as described herein may be of any length. Additional sequences derived from the native protein and/or heterologous sequences may be present, and such sequences may (but need not) possess further immunogenic or antigenic properties.

An "immunogenic portion," as used herein is a portion of an antigen that is recognized (*i*.*e*., specifically bound) by a B-cell and/or T-cell surface antigen receptor. Such immunogenic portions generally comprise at least 5 amino acid residues, more preferably at least 10, and still more preferably at least 20 amino acid residues of an ovarian carcinoma protein or a variant thereof. Preferred immunogenic portions are encoded by cDNA molecules isolated as described herein. Further immunogenic portions may generally be identified using well known techniques, such as those summarized in Paul, Fundamental Immunology, 3rd ed., 243-247 (Raven Press, 1993) and references cited therein: Such techniques include screening polypeptides for the ability to react with ovarian carcinoma protein-specific antibodies, antisera and/or T-eell lines or clones. As used herein, antisera and antibodies are "ovarian carcinoma protein-specific" if they specifically bind to an ovarian carcinoma protein (*i*.*e*., they react with the ovarian carcinoma protein in an ELISA or other immunoassay, and do not react detectably with unrelated proteins). Such antisera, antibodies and T cells may be prepared as described herein, and using well known techniques. An immunogenic portion of a native ovarian carcinoma protein is a portion that reacts with such antisera, antibodies and/or T-cells at a level that is not substantially less than the reactivity of the full length polypeptide (e.g., in an ELISA and/or T-cell reactivity assay). Such immunogenic portions may react within such assays at a level that is similar to or greater than the reactivity of the full length protein. Such screens may generally be performed using methods well known to those of ordinary skill in the art, such as those described in Harlow and Lane, Antibodies: A Laboratory Manual, Cold Spring Harbor Laboratory, 1988. For example, a polypeptide may be immobilized on a solid support and contacted with patient sera to allow binding of antibodies within the sera to the immobilized polypeptide. Unbound sera may then be removed and bound antibodies detected using, for example, ¹²⁵I-labeled Protein A.

As noted above, a composition may comprise a variant of a native ovarian carcinoma protein. A polypeptide "variant," as used herein, is a polypeptide that differs from a native ovarian carcinoma protein in one or more substitutions, deletions, additions and/or insertions, such that the immunogenicity of the polypeptide is not substantially diminished. In other words, the ability of a variant to react with ovarian carcinoma protein-specific antisera may be enhanced or unchanged, relative to the native ovarian carcinoma protein, or may be diminished by less than 50%, and preferably less than 20%, relative to the native ovarian carcinoma protein. Such variants may generally be identified by modifying one of the above polypeptide sequences and evaluating the reactivity of the modified polypeptide with ovarian carcinoma protein-specific antibodies or antisera as described herein. Preferred variants include those in which one or more portions, such as an N-terminal leader sequence or transmembrane domain, have been removed. Other preferred variants include variants in which a small portion (e.g., 1-30 amino acids, preferably 5-15 amino acids) has been removed from the N- and/or C-teiminal of the mature protein.

Polypeptide variants preferably exhibit at least about 70%, more preferably at least about 90% and most preferably at least about 95% identity to the native polypeptide. Preferably, a variant contains conservative substitutions. A "conservative substitution" is one in which an amino acid is substituted for another amino acid that has similar properties, such that one skilled in the art of peptide chemistry would expect the secondary structure and hydropathic nature of the polypeptide to be substantially unchanged. Amino acid substitutions may generally be made on the basis of similarity in polarity, charge, solubility, hydrophobicity, hydrophilicity and/or the amphipathic nature of the residues. For example, negatively charged amino acids include aspartic acid and glutamic acid; positively charged amino acids include lysine and arginine; and amino acids with uncharged polar head groups having similar hydrophilicity values include leucine, isoleucine and valine; glycine and alanine; asparagine and glutamine; and serine, threonine, phenylalanine and tyrosine. Other groups of amino acids that may represent conservative changes include: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his. A variant may also, or alternatively, contain nonconservative changes. Variants may also (or alternatively) be modified by, for example, the deletion or addition of amino acids that have minimal influence on the immunogenicity, secondary structure and hydropathic nature of the polypeptide.

As noted above, polypeptides may comprise a signal (or leader) sequence at the N-terminal end of the protein which co-translationally or post-translationally directs transfer of the protein. The polypeptide may also be conjugated to a linker or other sequence for ease of synthesis, purification or identification of the polypeptide (e.g., poly-His), or to enhance binding of the polypeptide to a solid support. For example, a polypeptide may be conjugated to an immunoglobulin Fc region.

Polypeptides may be prepared using any of a variety of well known techniques. Recombinant polypeptides encoded by DNA sequences as described above may be readily prepared from the DNA sequences using any of a variety of expression vectors known to those of ordinary skill in the art. Expression may be achieved in any appropriate host cell that has been transformed or transfected with an expression vector containing a DNA molecule that encodes a recombinant polypeptide. Suitable host cells include prokaryotes, yeast and higher eukaryotic cells. Preferably, the host cells employed are *E. coli*, yeast or a mammalian cell line such as COS or CHO. Supernatants from suitable host/vector systems which secrete recombinant protein or polypeptide into culture media may be first concentrated using a commercially available filter. Following concentration, the concentrate may be applied to a suitable purification matrix such as an affinity matrix or an ion exchange resin. Finally, one or more reverse phase HPLC steps can be employed to further purify a recombinant polypeptide.

Portions and other variants having fewer than about 100 amino acids, and generally fewer than about 50 amino acids, may also be generated by synthetic means, using techniques well known to those of ordinary skill in the art. For example, such polypeptides may be synthesized using any of the commercially available solid-phase techniques, such as the Merrifield solid-phase synthesis method, where amino acids are sequentially added to a growing amino acid chain. *See* Merrifield, J. Am. Chem. Soc. 85:2149-2146, 1963. Equipment for automated synthesis of polypeptides is commercially available from suppliers such as Applied BioSystems, Inc. (Foster City, CA), and may be operated according to the manufacturer's instructions.

Within certain specific embodiments, a polypeptide may be a fusion protein that comprises multiple polypeptides as described herein, or that comprises one polypeptide as described herein and a known tumor antigen, such as an ovarian carcinoma protein or a variant of such a protein. A fusion partner may, for example, assist in providing T helper epitopes (an immunological fusion partner), preferably T helper epitopes recognized by humans, or may assist in expressing the protein (an expression enhancer) at higher yields than the native recombinant protein. Certain preferred fusion partners are both immunological and expression enhancing fusion partners. Other fusion partners may be selected so as to increase the solubility of the protein or to enable the protein to be targeted to desired intracellular compartments. Still further fusion partners include affinity tags, which facilitate purification of the protein.

Fusion proteins may generally be prepared using standard techniques, including chemical conjugation. Preferably, a fusion protein is expressed as a recombinant protein, allowing the production of increased levels, relative to a non-fused protein, in an expression systems. Briefly, DNA sequences encoding the polypeptide components may be assembled separately, and ligated into an appropriate expression vector. The 3' end of the DNA sequence encoding one polypeptide component is ligated, with or without a peptide linker, to the 5' end of a DNA sequence encoding the second polypeptide component so that the reading frames of the sequences are in phase. This permits translation into a single fusion protein that retains the biological activity of both component polypeptides.

A peptide linker sequence may be employed to separate the first and the second polypeptide components by a distance sufficient to ensure that each polypeptide folds into its secondary and tertiary structures. Such a peptide linker sequence is incorporated into the fusion protein using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., Gene 40:39-46, 1985; Murphy et al., Proc. Natl. Acad Sci. USA 83:8258-8262, 1986; U.S. Patent No. 4,935,233 and U.S. Patent No. 4,751,180. The linker sequence may generally be from 1 to about 50 amino acids in length. Linker sequences are not required when the first and second polypeptides have non-essential N-terminal amino acid regions that can be used to separate the functional domains and prevent steric interference.

The ligated DNA sequences are operably linked to suitable transcriptional or translational regulatory elements. The regulatory elements responsible for expression of DNA are located only 5' to the DNA sequence encoding the first polypeptides. Similarly, stop codons required to end translation and transcription termination signals are only present 3' to the DNA sequence encoding the second polypeptide.

Fusion proteins are also provided that comprise a polypeptide of the present invention together with an unrelated immunogenic protein. Preferably the immunogenic protein is capable of eliciting a recall response. Examples of such proteins include tetanus, tuberculosis and hepatitis proteins *(see,* for example, Stoute et al. New Engl. J. Med., 336:86-91, 1997).

Within preferred embodiments, an immunological fusion partner is derived from protein D, a surface protein of the gram-negative bacterium Haemophilus influenza B (WO 91/18926). Preferably, a protein D derivative comprises approximately the first third of the protein (*e*.*g*., the first N-terminal 100-110 amino acids), and a protein D derivative may be lipidated. Within certain preferred embodiments, the first 109 residues of a Lipoprotein D fusion partner is included on the N-ternoanus to provide the polypeptide with additional exogenous T-cell epitopes and to increase the expression level in *E. coli* (thus functioning as an expression enhance). The lipid tail ensures optimal presentation of the antigen to antigen present cells. Other fusion partners include the non-structural protein from influenzae virus, NS1 (hemaglutinin). Typically, the N-terminal 81 amino acids are used, although different fragments that include T-helper epitopes may be used.

In another embodiment, the immunological fusion partner is the protein known as LYTA, or a portion thereof (preferably a C-terminal portion). LYTA is derived from *Streptococcus pneumoniae*, which synthesizes an N-acetyl-L-alanine amidase known as amidase LYTA (encoded by the LytA gene; Gene 43:265-292, 1985). LYTA is an autolysin that specifically degrades certain bonds in the peptidoglycan backbone. The C-terminal domain of the LYTA protein is responsible for the affinity to the choline or to some choline analogues such as DEAE. This property has been exploited for the development of *E. coli* C-LYTA expressing plasmids useful for expression of fusion proteins. Purification of hybrid proteins containing the C-LYTA fragment at the amino terminus has been described (*see* Biotechnology 10:795-798, 1992). Within a preferred embodiment, a repeat portion of LYTA may be incorporated into a fusion protein. A repeat portion is found in the C-terzninal region starting at residue 178. A particularly preferred repeat portion incorporates residues 188-305.

In general, polypeptides (including fusion proteins) and polynucleotides as described herein are isolated. An "isolated" polypeptide or polynucleotide is one that is removed from its original environment. For example, a naturally-occurring protein is isolated if it is separated from some or all of the coexisting materials in the natural system. Preferably, such polypeptides are at least about 90% pure, more preferably at least about 95% pure and most preferably at least about 99% pure. A polynucleotide is considered to be isolated if, for example, it is cloned into a vector that is not a part of the natural environment.

### Binding Agents

The present invention further provides agents, such as antibodies and antigen-binding fragments thereof, that specifically bind to an ovarian carcinoma protein. As used herein, an antibody, or antigen-binding fragment thereof, is said to "specifically bind" to an ovarian carcinoma protein if it reacts at a detectable level (within, for example, an ELISA) with an ovarian carcinoma protein, and does not react detectably with unrelated proteins under similar conditions. As used herein, "binding" refers to a noncovalent association between two separate molecules such that a "complex" is formed. The ability to bind may be evaluated by, for example, determining a binding constant for the formation of the complex. The binding constant is the value obtained when the concentration of the complex is divided by the product of the component concentrations. In general, two compounds are said to "bind," in the context of the present invention, when the binding constant for complex formation exceeds about 10³ L/mol. The binding constant maybe determined using methods well known in the art.

Binding agents may be further capable of differentiating between patients with and without a cancer, such as ovarian cancer, using the representative assays provided herein. In other words, antibodies or other binding agents that bind to a ovarian carcinoma antigen will generate a signal indicating the presence of a cancer in at least about 20% of patients with the disease, and will generate a negative signal indicating the absence of the disease in at least about 90% of individuals without the cancer. To determine whether a binding agent satisfies this requirement, biological samples (e.g., blood, sera, leukophoresis, urine and/or tumor biopsies) from patients with and without a cancer (as determined using standard clinical tests) may be assayed as described herein for the presence of polypeptides that bind to the binding agent. It will be apparent that a statistically significant number of samples with and without the disease should be assayed. Each binding agent should satisfy the above criteria; however, those of ordinary skill in the art will recognize that binding agents may be used in combination to improve sensitivity.

Any agent that satisfies the above requirements may be a binding agent. For example, a binding agent may be a ribosome, with or without a peptide component, an RNA molecule or a polypeptide. In a preferred embodiment, a binding agent is an antibody or an antigen-binding fragment thereof. Antibodies may be prepared by any of a variety of techniques known to those of ordinary skill in the art. *See, e.g*., Harlow and Lane, Antibodies: A Laboratory Manual, Cold Spring Harbor Laboratory, 1988. In general, antibodies can be produced by cell culture techniques, including the generation of monoclonal antibodies as described herein, or via transfection of antibody genes into suitable bacterial or mammalian cell hosts, in order to allow for the production of recombinant antibodies. In one technique, an immunogen comprising the polypeptide is initially injected into any of a wide variety of mammals (e.g., mice, rats, rabbits, sheep or goats). In this step, the polypeptides of this invention may serve as the immunogen without modification. Alternatively, particularly for relatively short polypeptides, a superior immune response may be elicited if the polypeptide is joined to a carrier protein, such as bovine serum albumin or keyhole limpet hemocyanin. The immunogen is injected into the animal host, preferably according to a predetermined schedule incorporating one or more booster immunizations, and the animals are bled periodically. Polyclonal antibodies specific for the polypeptide may then be purified from such antisera by, for example, affinity chromatography using the polypeptide coupled to a suitable solid support.

Monoclonal antibodies specific for an antigenic polypeptide of interest may be prepared, for example, using the technique of Kohler and Milstein, Eu. J. Immunol. 6:511-519, 1976, and improvements thereto. Briefly, these methods involve the preparation of immortal cell lines capable of producing antibodies having the desired specificity (*i*.*e*., reactivity with the polypeptide of interest). Such cell lines may be produced, for example, from spleen cells obtained from an animal immunized as described above. The spleen cells are then immortalized by, for example, fusion with a myeloma cell fusion partner, preferably one that is syngeneic with the immunized animal. A variety of fusion techniques may be employed. For example, the spleen cells and myeloma cells may be combined with a nonionic detergent for a few minutes and then plated at low density on a selective medium that supports the growth of hybrid cells, but not myeloma cells. A preferred selection technique uses HAT (hypoxanthine, aminopterin, thymidine) selection. After a sufficient time, usually about 1 to 2 weeks, colonies of hybrids are observed. Single colonies are selected and their culture supernatants tested for binding activity against the polypeptide. Hybridomas having high reactivity and specificity are preferred.

Monoclonal antibodies may be isolated from the supernatants of growing hybridoma colonies. In addition, various techniques may be employed to enhance the yield, such as injection of the hybridoma cell line into the peritoneal cavity of a suitable vertebrate host, such as a mouse. Monoclonal antibodies may then be harvested from the ascites fluid or the blood. Contaminants may be removed from the antibodies by conventional techniques, such as chromatography, gel filtration, precipitation, and extraction. The polypeptides of this invention may be used in the purification process in, for example, an affinity chromatography step.

Within certain embodiments, the use of antigen-binding fragments of antibodies may be preferred. Such fragments include Fab fragments, which may be prepared using standard techniques. Briefly, immunoglobulins may be purified from rabbit serum by affinity chromatography on Protein A bead columns (Harlow and Lane, Antibodies: A Laboratory Manual, Cold Spring Harbor Laboratory, 1988) and digested by papain to yield Fab and Fc fragments. The Fab and Fc fragments may be separated by affinity chromatography on protein A bead columns.

Monoclonal antibodies of the present invention may be coupled to one or more therapeutic agents. Suitable agents in this regard include radionuclides, differentiation inducers, drugs, toxins, and derivatives thereon Preferred radionuclides include ⁹⁰Y, ¹²³I, ¹²⁵I, ¹³¹I, ¹⁸⁶Re, ¹⁸⁸Re, ²¹¹At, and ²¹²Bi. Preferred drugs include methotrexate, and pyrimidine and purine analogs. Preferred differentiation inducers include phorbol esters and butyric acid. Preferred toxins include ricin, abrin, diptheria toxin, cholera toxin, gelonin, Pseudomonas exotoxin, Shigella toxin, and pokeweed antiviral protein.

A therapeutic agent may be coupled (*e*.*g*., covalently bonded) to a suitable monoclonal antibody either directly or indirectly (*e*.*g*., via a linker group). A direct reaction between an agent and an antibody is possible when each possesses a substituent capable of reacting with the other. For example, a nucleophilic group, such as an amino or sulfhydryl group, on one may be capable of reacting with a carbonyl-containing group, such as an anhydride or an acid halide, or with an alkyl group containing a good leaving group (*e*.*g*., a halide) on the other.

Alternatively, it may be desirable to couple a therapeutic agent and an antibody via a linker group. A linker group can function as a spacer to distance an antibody from an agent in order to avoid interference with binding capabilities. A linker group can also serve to increase the chemical reactivity of a substituent on an agent or an antibody, and thus increase the coupling efficiency. An increase in chemical reactivity may also facilitate the use of agents, or functional groups on agents, which otherwise would not be possible.

It will be evident to those skilled in the art that a variety of bifunctional or polyfunctional reagents, both homo- and hetero-functional (such as those described in the catalog of the Pierce Chemical Co., Rockford, IL), may be employed as the linker group. Coupling may be effected, for example, through amino groups, carboxyl groups, sullhydryl groups or oxidized carbohydrate residues. There are numerous references describing such methodology, e.g., U.S. Patent No. 4,671,958, to Rodwell et al.

Where a therapeutic agent is more potent when free from the antibody portion of the immunoconjugates of the present invention, it may be desirable to use a linker group which is cleavable during or upon internalization into a cell. A number of different cleavable linker groups have been described. The mechanisms for the intracellular release of an agent from these linker groups include cleavage by reduction of a disulfide bond (e.g., U.S. Patent No. 4,489,710, to Spitler), by irradiation of a photolabile bond (e.g., U.S. Patent No. 4,625,014, to Senter et al.), by hydrolysis of derivatized amino acid side chains (*e*.*g*., U.S. Patent No. 4,638,045, to Kohn et al.), by serum complement-mediated hydrolysis (e.g., U.S. Patent No. 4,671,958, to Rodwell et al.), and acid-catalyzed hydrolysis (e.g., U.S. Patent No. 4,569,789, to Blattler et al.).

It may be desirable to couple more than one agent to an antibody. In one embodiment, multiple molecules of an agent are coupled to one antibody molecule. In another embodiment, more than one type of agent may be coupled to one antibody. Regardless of the particular embodiment, immunoconjugates with more than one agent may be prepared in a variety of ways. For example, more than one agent may be coupled directly to an antibody molecule, or linkers which provide multiple sites for attachment can be used. Alternatively, a carrier can be used.

A carrier may bear the agents in a variety of ways, including covalent bonding either directly or via a linker group. Suitable carriers include proteins such as albumins (*e*.*g*., U.S. Patent No. 4,507,234, to Kato et al.), peptides and polysaccharides such as aminodextran (*e*.*g*., U.S. Patent No. 4,699,784, to Shih et al.). A carrier may also bear an agent by noncovalent bonding or by encapsulation, such as within a liposome vesicle (e.g., U.S. Patent Nos. 4,429,008 and 4,873,088). Carriers specific for radionuclide agents include radiohalogenated small molecules and chelating compounds. For example, U.S. Patent No. 4,735,792 discloses representative radiohalogenated small molecules and their synthesis. A radionuclide chelate may be formed from chelating compounds that include those containing nitrogen and sulfur atoms as the donor atoms for binding the metal, or metal oxide, radionuclide. For example, U.S. Patent No. 4,673,562, to Davison et al. discloses representative chelating compounds and their synthesis.

A variety of routes of administration for the antibodies and immunoconjugates may be used. Typically, administration will be intravenous, intramuscular, subcutaneous or in the bed of a resected tumor. It will be evident that the precise dose of the antibody/immunoconjugate will vary depending upon the antibody used, the antigen density on the tumor, and the rate of clearance of the antibody.

Also provided herein are anti-idiotypic antibodies that mimic an immunogenic portion of an ovarian carcinoma protein. Such antibodies may be raised against an antibody, or antigen-binding fragment thereof, that specifically binds to an immunogenic portion of an ovarian carcinoma protein, using well known techniques. Anti-idiotypic antibodies that mimic an immunogenic portion of an ovarian carcinoma protein are those antibodies that bind to an antibody, or antigen-binding fragment thereof, that specifically binds to an immunogenic portion of an ovarian carcinoma protein, as described herein.

### T Cells

Immunotherapeutic compositions may also, or alternatively, comprise T cells specific for an ovarian carcinoma protein. Such cells may generally be prepared in *vitro* or *ex vivo*, using standard procedures. For example, T cells may be present within (or isolated from) bone marrow, peripheral blood or a fraction of bone marrow or peripheral blood of a mammal, such as a patient, using a commercially available cell separation system, such as the CEPRATE™ system, available from CellPro Inc., Bothell WA (see also U.S. Patent No. 5,240,856; U.S. Patent No. 5,215,926; WO 89/06280; WO 91/16116 and WO 92/07243). Alternatively, T cells may be derived from related or unrelated humans, non-human animals, cell lines or cultures.

T cells may be stimulated with an ovarian carcinoma polypeptide, polynucleotide encoding an ovarian carcinoma polypeptide and/or an antigen presenting cell (APC) that expresses such a polypeptide. Such stimulation is performed under conditions and for a time sufficient to permit the generation of T cells that are specific for the polypeptide. Preferably, an ovarian carcinoma polypeptide or polynucleotide is present within a delivery vehicle, such as a microsphere, to facilitate the generation of specific T cells.

T cells are considered to be specific for an ovarian carcinoma polypeptide if the T cells kill target cells coated with an ovarian carcinoma polypeptide or expressing a gene encoding such a polypeptide. T cell specificity may be evaluated using any of a variety of standard techniques. For example, within a chromium release assay or proliferation assay, a stimulation index of more than two fold increase in lysis and/or proliferation, compared to negative controls, indicates T cell specificity. Such assays may be performed, for example, as described in Chen et al., Cancer Res. 54:1065-1070, 1994. Alternatively, detection of the proliferation of T cells may be accomplished by a variety of known techniques. For example, T cell proliferation can be detected by measuring an increased rate of DNA synthesis (*e*.*g*., by pulse-labeling cultures of T cells with tritiated thymidine and measuring the amount of tritiated thymidine incorporated into DNA). Contact with an ovarian carcinoma polypeptide (200 ng/ml - 100 µg/ml, preferably 100 µg/ml - 25 µg/ml) for 3 - 7 days should result in at least a two fold increase in proliferation of the T cells and/or contact as described above for 2-3 hours should result in activation of the T cells, as measured using standard cytokine assays in which a two fold increase in the level of cytokine release *(e.g.,* TNF or IFN-γ) is indicative of T cell activation (*see* Coligan et al., Current Protocols in Immunology, vol. 1, Wiley Interscience (Greene 1998). T cells that have been activated in response to an ovarian carcinoma polypeptide, polynucleotide or ovarian carcinoma polypeptide-expressing APC may be CD4⁺ and/or CD8⁺. Ovarian carcinoma polypeptide-specific T cells may be expanded using standard techniques. Within preferred embodiments, the T cells are derived from a patient or a related or unrelated donor and are administered to the patient following stimulation and expansion.

For therapeutic purposes, CD4⁺ or CD8⁺ T cells that proliferate in response to an ovarian carcinoma polypeptide, polynucleotide or APC can be expanded in number either *in vitro or in vivo*. Proliferation of such T cells *in vitro* may be accomplished in a variety of ways. For example, the T cells can be re-exposed to an ovarian carcinoma polypeptide, with or without the addition of T cell growth factors, such as interleukin-2, and/or stimulator cells that synthesize an ovarian carcinoma polypeptide. Alternatively, one or more T cells that proliferate in the presence of an ovarian carcinoma polypeptide can be expanded in number by cloning. Methods for cloning cells are well known in the art, and include limiting dilution. Following expansion, the cells may be administered back to the patient as described, for example, by Chang et al., Crit. Rev. Oncol. Hematol. 22:213, 1996.

### Pharmaceutical Compositions and Vaccines

Within certain aspects, polypeptides, polynucleotides, binding agents and/or immune system cells as described herein may be incorporated into pharmaceutical compositions or vaccines. Pharmaceutical compositions comprise one or more such compounds or cells and a physiologically acceptable carrier. Vaccines may comprise one or more such compounds or cells and a non-specific immune response enhancer. A non-specific immune response enhancer may be any substance that enhances an immune response to an exogenous antigen. Examples of non-specific immune response enhancers include adjuvants, biodegradable microspheres (*e*.*g*., polylactic galactide) and liposomes (into which the compound is incorporated; *see e.g.,* Fullerton, U.S. Patent No. 4,235,877). Vaccine preparation is generally described in, for example, M.F. Powell and M.J. Newman, eds., "Vaccine Design (the subunit and adjuvant approach)," Plenum Press (NY, 1995). Pharmaceutical compositions and vaccines within the scope of the present invention may also contain other compounds, which may be biologically active or inactive. For example, one or more immunogenic portions of other tumor antigens may be present, either incorporated into a fusion polypeptide or as a separate compound within the composition or vaccine.

A pharmaceutical composition or vaccine may contain DNA encoding one or more of the polypeptides as described above, such that the polypeptide is generated *in situ.* As noted above, the DNA may be present within any of a variety of delivery systems known to those of ordinary skill in the art, including nucleic acid expression systems, bacteria and viral expression systems. Appropriate nucleic acid expression systems contain the necessary DNA sequences for expression in the patient (such as a suitable promoter and terminating signal). Bacterial delivery systems involve the administration of a bacterium (such as *Bacillus-Calmette-Guerrin*) that expresses an immunogenic portion of the polypeptide on its cell surface. In a preferred embodiment, the DNA may be introduced using a viral expression system (*e*.*g*., vaccinia or other pox virus, retrovirus, or adenovirus), which may involve the use of a non-pathogenic (defective), replication competent virus. Suitable systems are disclosed, for example, in Fisher-Hoch et al., PNAS 86:317-321, 1989; Flexner et al., Ann. N.Y. Acad. Sci. 569:86-103, 1989; Flexner et al., Vaccine 8:17-21, 1990; U.S. Patent Nos. 4,603,11.2, 4,769,330, and 5,017,487; WO 89/01973; U.S. Patent No. 4,777,127; GB 2,200,651; EP 0,345,242; WO 91/02805; Berkner, Biotechniques 6:616-627, 1988; Rosenfeld et al., Science 252:431-434, 1991; Kolls et al., PNAS 91:215-219, 1994; Kass-Eisler et al., PNAS 90:11498-11502, 1993; Guzman et al., Circulation 88:2838-2848, 1993; and Guzman et al., Cir. Res. 73:1202-1207, 1993. Techniques for incorporating DNA into such expression systems are well known to those of ordinary skill in the art. The DNA may also be "naked," as described, for example, in Ulmer et al., Science 259:1745-1749, 1993 and reviewed by Cohen, Science 259:1.691-1692, 1993. The uptake of naked DNA may be increased by coating the DNA onto biodegradable beads, which are efficiently transported into the cells.

While any suitable carrier known to those of ordinary skill in the art may be employed in the pharmaceutical compositions of this invention, the type of carrier will vary depending on the mode of administration. Compositions of the present invention may be formulated for any appropriate manner of administration, including for example, topical, oral, nasal, intravenous, intracranial, intraperitoneal, subcutaneous or intramuscular administration. For parenteral administration, such as subcutaneous injection, the carrier preferably comprises water, saline, alcohol, a fat, a wax or a buffer. For oral administration, any of the above carriers or a solid carrier, such as mannitol, lactose, starch, magnesium stearate, sodium saccharine, talcum, cellulose, glucose, sucrose, and magnesium carbonate, may be employed. Biodegradable microspheres (*e*.*g*., polylactate polyglycolate) may also be employed as carriers for the pharmaceutical compositions of this invention. Suitable biodegradable microspheres are disclosed, for example, in U.S. Patent Nos. 4,897,268 and 5,075,109.

Such compositions may also comprise buffers (e.g., neutral buffered saline or phosphate buffered saline), carbohydrates (*e*.*g*., glucose, mannose, sucrose or dextrans), mannitol, proteins, polypeptides or amino acids such as glycine, antioxidants, chelating agents such as EDTA or glutathione, adjuvants (e.g., aluminum hydroxide) and/or preservatives. Alternatively, compositions of the present invention may be formulate as a lyophilizate. Compounds may also be encapsulated within liposomes using well known technology.

Any of a variety of non-specific immune response enhancers may be employed in the vaccines of this invention. For example, an adjuvant may be included. Most adjuvants contain a substance designed to protect the antigen from rapid catabolism, such as aluminum hydroxide or mineral oil, and a stimulator of immune responses, such as lipid A, *Bortadella pertussis* or *Mycobacterium tuberculosis* derived proteins. Suitable adjuvants are commercially available as, for example, Freund's Incomplete Adjuvant and Complete Adjuvant (Difco Laboratories, Detroit, MI), Merck Adjuvant 65 (Merck and Company, Inc., Rahway, NJ), alum, biodegradable microspheres, monophosphoryl lipid A and quil A. Cytokines, such as GM-CSF or interleukin-2, -7, or -12, may also be used as adjuvants.

Within the vaccines provided herein, the adjuvant composition is preferably designed to induce an immune response predominantly of the Th1 type. High levels of Th1-type cytokines (e.g., IFN-γ, IL-2 and IL-12) tend to favor the induction of cell mediated immune responses to an administered antigen. In contrast, high levels of Th2-type cytokines (e.g., IL-4, IL-5, IL-6, IL-10 and TNF-β) tend to favor the induction of humoral immune responses. Following application of a vaccine as provided herein, a patient will support an immune response that includes Th1- and Th2-type responses. Within a preferred embodiment, in which a response is predominantly Th1-type, the level of Th1-type cytokines will increase to a greater extent than the level of Th2-type cytokines. The levels of these cytokines may be readily assessed using standard assays. For a review of the families of cytokines, see Mosmann and Coffman, Ann. Rev. Immunol. 7:145-173, 1989.

Preferred adjuvants for use in eliciting a predominantly Th1-type response include, for example, a combination of monaphasphoryl lipid A, preferably 3-de-O-acylated monophosphoryl lipid A (3D-MPL), together with an aluminum salt. MPL adjuvants are available from Ribi ImmunoChem Research Inc. (Hamilton, MT; *see* US Patent Nos. 4,436,727; 4,877,611; 4,866,034 and 4,912,094). Also preferred is AS-2 (SznithKline Beecham). CpG-cantaining oligonucleotides (in which the CpG dinucleotide is unmethylated) also induce a predominantly Th1 response. Such oligonucleotides are well known and are described, for example, in WO 96/02555. Another preferred adjuvant is a saponin, preferably QS21, which may be used alone or in combination with other adjuvants. For example, an enhanced system involves the combination of a monophosphoryl lipid A and saponin derivative, such as the combination of QS21 and 3D-MPL as described in WO 94/00153, or a less reactogenic composition where the QS21 is quenched with cholesterol, as described in WO 96/33739. Other preferred formulations comprises an oil-in-water emulsion and tocopherol. A particularly potent adjuvant formulation involving QS21, 3D-MPL and tocopherol in an oil-in-water emulsion is described in WO 95/17210. Any vaccine provided herein may be prepared using well known methods that result in a combination of antigen, immune response enhancer and a suitable carrier or excipient.

The compositions described herein may be administered as part of a sustained release formulation (*i*.*e*., a formulation such as a capsule or sponge that effects a slow release of compound following administration). Such formulations may generally be prepared using well known technology and administered by, for example, oral, rectal or subcutaneous implantation, or by implantation at the desired target site. Sustained-release formulations may contain a polypeptide, polynucleotide or antibody dispersed in a carrier matrix and/or contained within a reservoir surrounded by a rate controlling membrane. Carriers for use within such formulations are biocompatible, and may also be biodegradable; preferably the formulation provides a relatively constant level of active component release. The amount of active compound contained within a sustained release formulation depends upon the site of implantation, the rate and expected duration of release and the nature of the condition to be treated or prevented.

Any of a variety of delivery vehicles may be employed within pharmaceutical compositions and vaccines to facilitate production of an antigen-specific immune response that targets tumor cells. Delivery vehicles include antigen presenting cells (APCs), such as dendritic cells, macrophages, B cells, monocytes and other cells that may be engineered to be efficient APCs. Such cells may, but need not, be genetically modified to increase the capacity for presenting the antigen, to improve activation and/or maintenance of the T cell response, to have anti-tumor effects *per se* and/or to be immunologically compatible with the receiver (*i*.*e*., matched HLA haplotype). APCs may generally be isolated from any of a variety of biological fluids and organs, including tumor and peritumoral tissues, and may be autologous, allogeneic, syngeneic or xenogeneic cells.

Certain preferred embodiments of the present invention use dendritic cells or progenitors thereof as antigen-presenting cells. Dendritic cells are highly potent APCs (Banchereau and Steinman, Nature 392:245-251, 1998) and have been shown to be effective as a physiological adjuvant for eliciting prophylactic or therapeutic antitumor immunity (see Timmerman and Levy, Ann. Rev. Med. 50:507-529, 1999). In general, dendritic cells may be identified based on their typical shape (stellate *in situ*, with marked cytoplasmic processes (dendrites) visible *in vitro*) and based on the lack of differentiation markers of B cells (CD19 and CD20), T cells (CD3), monocytes (CD14) and natural killer cells (CD56), as determined using standard assays. Dendritic cells may, of course, be engineered to express specific cell-surface receptors or ligands that are not commonly found on dendritic cells in vivo or *ex vivo,* and such modified dendritic cells are contemplated by the present invention. As an alternative to dendritic cells, secreted vesicles antigen-loaded dendritic cells (called exosomes) may be used within a vaccine *(see* Zitvogel et al., Nature Med. 4:594-600, 1998).

Dendritic cells and progenitors may be obtained from peripheral blood, bone marrow, tumor-infiltrating cells, peritumoral tissues-infiltrating cells, lymph nodes, spleen, skin, umbilical cord blood or any other suitable tissue or fluid. For example, dendritic cells may be differentiated *ex vivo* by adding a combination of cytokines such as GM-CSF, IL-4, IL-13 and/or TNFα to cultures of monocytes harvested from peripheral blood. Alternatively, CD34 positive cells harvested from peripheral blood, umbilical cord blood or bone marrow may be differentiated into dendritic cells by adding to the culture medium combinations of GM-CSF, IL-3, TNFα, CD40 ligand, LPS, flt3 ligand and/or other compound(s) that induce maturation and proliferation of dendritic cells.

Dendritic cells are conveniently categorized as "immature" and "mature" cells, which allows a simple way to discriminate between two well characterized phenotypes. However, this nomenclature should not be construed to exclude all possible intermediate stages of differentiation. Immature dendritic cells are characterized as APC with a high capacity for antigen uptake and processing, which correlates with the high expression of Fcγ receptor, mannose receptor and DEC-205 marker. The mature phenotype is typically characterized by a lower expression of these markers, but a high expression of cell surface molecules responsible for T cell activation such as class I and class II MHC, adhesion molecules (e.g., CD54 and CD11) and costimulatory molecules (e.g., CD40, CD80 and CD86).

APCs may generally be transfected with a polynucleotide encoding a ovarian carcinoma antigen (or portion or other variant thereof) such that the antigen, or an immunogenic portion thereof, is expressed on the cell surface. Such transfection may take place *ex vivo,* and a composition or vaccine comprising such transfected cells may then be used for therapeutic purposes, as described herein. Alternatively, a gene delivery vehicle that targets a dendritic or other antigen presenting cell may be administered to a patient, resulting in transfection that occurs *in vivo. In vivo* and *ex vivo* transfection of dendritic cells, for example, may generally be performed using any methods known in the art, such as those described in WO 97/24447, or the gene gun approach described by Mahvi et al., Immunology and cell Biology 75:456-460, 1997. Antigen loading of dendritic cells may be achieved by incubating dendritic cells or progenitor cells with the polypeptide, DNA (naked or within a plasmid vector) or RNA; or with antigen-expressing recombinant bacterium or viruses (e.g., vaccinia, fawlpox, adenovirus or lentivirus vectors). Prior to loading, the polypeptide may be covalently conjugated to an immunological partner that provides T cell help (e.g., a carrier molecule). Alternatively, a dendritic cell may be pulsed with a non-conjugated immunological partner, separately or in the presence of the polypeptide.

### Cancer Therapy

In further aspects of the present invention, the compositions described herein may be used for immunotherapy of cancer, such as ovarian cancer. Within such methods, pharmaceutical compositions and vaccines are typically administered to a patient. As used herein, a "patient" refers to any warm-blooded animal, preferably a human. A patient may or may not be afflicted with cancer. Accordingly, the above pharmaceutical compositions and vaccines may be used to prevent the development of a cancer or to treat a patient afflicted with a cancer. Within certain preferred embodiments, a patient is afflicted with ovarian cancer. Such cancer may be diagnosed using criteria generally accepted in the art, including the presence of a malignant tumor. Pharmaceutical compositions and vaccines may be administered either prior to or following surgical removal of primary tumors and/or treatment such as administration of radiotherapy or conventional chemotherapeutic drugs.

Within certain embodiments, immunotherapy may be active immunotherapy, in which treatment relies on the *in vivo* stimulation of the endogenous host immune system to react against tumors with the administration of immuno response-modifying agents (such as tumor vaccines, bacterial adjuvants and/or cytokines).

Within other embodiments, immunotherapy may be passive immunotherapy, in which treatment involves the delivery of agents with established tumor-immune reactivity (such as effector cells or antibodies) that can directly or indirectly mediate antitumor effects and does not necessarily depend on an intact host immune system. Examples of effector cells include T lymphocytes (such as CD8⁺ cytotoxic T lymphocytes and CD4⁺ T-helper tumor-infiltrating lymphocytes), killer cells (such as Natural Killer cells and lymphokine-activated killer cells), B cells and antigen-presenting cells (such as dendritic cells and macrophages) expressing a polypeptide provided herein. T cell receptors and antibody receptors specific for the polypeptides recited herein may be cloned, expressed and transferred into other vectors or effector cells for adoptive immunotherapy. The polypeptides provided herein may also be used to generate antibodies or anti-idiotypic antibodies (as described above and in U.S. Patent No. 4,918,164) for passive immunotherapy.

Effector cells may generally be obtained in sufficient quantities for adoptive immunotherapy by growth *in vitro*, as described herein. Culture conditions for expanding single antigen-specific effector cells to several billion in number with retention of antigen recognition *in vivo* are well known in the art. Such *in vitro* culture conditions typically use intermittent stimulation with antigen, often in the presence of cytokines (such as IL-2) and non-dividing feeder cells. As noted above, immunoreactive polypeptides as provided herein may be used to rapidly expand antigen-specific T cell cultures in order to generate a sufficient number of cells for immunotherapy. In particular, antigen-presenting cells, such as dendritic, macrophage or B cells, may be pulsed with immunoreactive polypeptides or transfected with one or more polynucleotides using standard techniques well known in the art. For example, antigen-presenting cells can be transfected with a polynucleotide having a promoter appropriate for increasing expression in a recombinant virus or other expression system. Cultured effector cells for use in therapy must be able to grow and distribute widely, and to survive long term *in vivo*. Studies have shown that cultured effector cells can be induced to grow in vivo and to survive long term in substantial numbers by repeated stimulation with antigen supplemented with IL-2 *(see,* for example, Cheever et al., Immunological Reviews 157:177, 1997).

Alternatively, a vector expressing a polypeptide recited herein may be introduced into stem cells taken from a patient and clonally propagated *in vitro* for autologous transplant back into the same patient.

Routes and frequency of administration, as well as dosage, will vary from individual to individual, and may be readily established using standard techniques. In general, the pharmaceutical compositions and vaccines may be administered by injection (e.g., intracutaneous, intramuscular, intravenous or subcutaneous), intranasally (e.g., by aspiration), orally or in the bed of a resected tumor. Preferably, between 1 and 10 doses may be administered over a 52 week period. Preferably, 6 doses are administered, at intervals of 1 month, and booster vaccinations may be given periodically thereafter. Alternate protocols may be appropriate for individual patients. A suitable dose is an amount of a compound that, when administered as described above, is capable of promoting an anti-tumor immune response, and is at least 10-50% above the basal (*i*.*e*., untreated) level.. Such response can be monitored by measuring the anti-tumor antibodies in a patient or by vaccine-dependent generation of cytolytic effector cells capable of killing the patient's tumor cells *in vitro*. Such vaccines should also be capable of causing an immune response that leads to an improved clinical outcome (e.g., more frequent remissions, complete or partial or longer disease-free survival) in vaccinated patients as compared to non-vaccinated patients. In general, for pharmaceutical compositions and vaccines comprising one or more polypeptides, the amount of each polypeptide present in a dose ranges from about 100 µg to 5 mg per kg of host. Suitable dose sizes will vary with the size of the patient, but will typically range from about 0.1 mL to about 5 mL.

In general, an appropriate dosage and treatment regimen provides the active compound(s) in an amount sufficient to provide therapeutic and/or prophylactic benefit. Such a response can be monitored by establishing an improved clinical outcome (e.g., more frequent remissions, complete or partial, or longer disease-free survival) in treated patients as compared to non-treated patients. Increases in preexisting immune responses to an ovarian carcinoma antigen generally correlate with an improved clinical outcome. Such immune responses may generally be evaluated using standard proliferation, cytotoxicity or cytokine assays, which may be performed using samples obtained from a patient before and after treatment.

### Screens for Identifying Secreted Ovarian Carcinoma Antigens

The present invention provides methods for identifying secreted tumor antigens. Within such methods, tumors are implanted into immunodeficient animals such as SCID mice and maintained for a time sufficient to permit secretion of tumor antigens into serum. In general, tumors may be implanted subcutaneously or within the gonadal fat pad of an immunodeficient animal and maintained for 1-9 months, preferably 1-4 months. Implantation may generally be performed as described in WO 97/18300. The serum containing secreted antigens is then used to prepare antisera in immunocompetent mice, using standard techniques and as described herein. Briefly, 50-100 µL of sera (pooled from three sets of immunodeficient mice, each set bearing a different SCID-derived human ovarian tumor) may be mixed 1:1 (vol:vol) with an appropriate adjuvant, such as RIBI-MPL or MPL + TDM (Sigma Chemical Co., St. Louis, MO) and injected intraperitoneally into syngeneic immunocompetent animals at monthly intervals for a total of 5 months. Antisera from animals immunized in such a manner may be obtained by drawing blood after the third, fourth and fifth immunizations. The resulting antiserum is generally pre-cleared of *E. coli* and phage antigens and used (generally following dilution, such as 1:200) in a serological expression screen.

The library is typically an expression library containing cDNAs from one or more tumors of the type that was implanted into SCID mice. This expression library may be prepared in any suitable vector, such as γ-screen (Novagen). cDNAs that encode a polypeptide that reacts with the antiserum may be identified using standard techniques, and sequenced. Such cDNA molecules may be further characterized to evaluate expression in tumor and normal tissue, and to evaluate antigen secretion in patients.

The methods provided herein have advantages over other methods for tumor antigen discovery. In particular, all antigens identified by such methods should be secreted or released through necrosis of the tumor cells. Such antigens may be present on the surface of tumor cells for an amount of time sufficient to permit targeting and killing by the immune system, following vaccination.

### Methods for Detecting Cancer

In general, a cancer may be detected in a patient based on the presence of one or more ovarian carcinoma proteins and/or polynucleotides encoding such proteins in a biological sample (such as blood, sera, urine and/or tumor biopsies) obtained from the patient. In other words, such proteins may be used as markers to indicate the presence or absence of a cancer such as ovarian cancer. In addition, such proteins may be useful for the detection of other cancers. The binding agents provided herein generally permit detection of the level of protein that binds to the agent in the biological sample. Polynucleotide primers and probes may be used to detect the level of mRNA encoding a tumor protein, which is also indicative of the presence or absence of a cancer. In general, an ovarian carcinoma-associated sequence should be present at a level that is at least three fold higher in tumor tissue than in normal tissue

There are a variety of assay formats known to those of ordinary skill in the art for using a binding agent to detect polypeptide markers in a sample. *See*, *e*.*g*., Harlow and Lane, Antibodies: A Laboratory Manual, Cold Spring Harbor Laboratory, 1988. In general, the presence or absence of a cancer in a patient may be determined by (a) contacting a biological sample obtained from a patient with a binding agent; (b) detecting in the sample a level of polypeptide that binds to the binding agent; and (c) comparing the level of polypeptide with a predetermined cut-off value.

In a preferred embodiment, the assay involves the use of binding agent immobilized on a solid support to bind to and remove the polypeptide from the remainder of the sample. The bound polypeptide may then be detected using a detection reagent that contains a reporter group and specifically binds to the binding agent/polypeptide complex. Such detection reagents may comprise, for example, a binding agent that specifically binds to the polypeptide or an antibody or other agent that specifically binds to the binding agent, such as an anti-immunoglobulin, protein G, protein A or a lectin. Alternatively, a competitive assay may be utilized, in which a polypeptide is labeled with a reporter group and allowed to bind to the immobilized binding agent after incubation of the binding agent with the sample. The extent to which components of the sample inhibit the binding of the labeled polypeptide to the binding agent is indicative of the reactivity of the sample with the immobilized binding agent. Suitable polypeptides for use within such assays include full length ovarian carcinoma proteins and portions thereof to which the binding agent binds, as described above.

The solid support may be any material known to those of ordinary skill in the art to which the tumor protein may be attached. For example, the solid support may be a test well in a microtiter plate or a nitrocellulose or other suitable membrane. Alternatively, the support may be a bead or disc, such as glass, fiberglass, latex or a plastic material such as polystyrene or polyvinylchloride. The support may also be a magnetic particle or a fiber optic sensor, such as those disclosed, for example, in U.S. Patent No. 5,359,681. The binding agent may be immobilized on the solid support using a variety of techniques known to those of skill in the art, which are amply described in the patent and scientific literature. In the context of the present invention, the term "immobilization" refers to both noncovalent association, such as adsorption, and covalent attachment (which may be a direct linkage between the agent and functional groups on the support or may be a linkage by way of a cross-linking agent). Immobilization by adsorption to a well in a microtiter plate or to a membrane is preferred. In such cases, adsorption may be achieved by contacting the binding agent, in a suitable buffer, with the solid support for a suitable amount of time. The contact time varies with temperature, but is typically between about 1 hour and about 1 day. In general, contacting a well of a plastic microtiter plate (such as polystyrene or polyvinylchloride) with an amount of binding agent ranging from about 10 ng to about 10 µg, and preferably about 100 ng to about 1 µg, is sufficient to immobilize an adequate amount of binding agent.

Covalent attachment of binding agent to a solid support may generally be achieved by first reacting the support with a bifunctional reagent that will react with both the support and a functional group, such as a hydroxyl or amino group, on the binding agent. For example, the binding agent may be covalently attached to supports having an appropriate polymer coating using benzoquinone or by condensation of an aldehyde group on the support with an amine and an active hydrogen on the binding partner *(see,* e.g., Pierce Immunotechnology Catalog and Handbook, 1991, at A12-A13).

In certain embodiments, the assay is a two-antibody sandwich assay. This assay may be performed by first contacting an antibody that has been immobilized on a solid support, commonly the well of a microtiter plate, with the sample, such that polypeptides within the sample are allowed to bind to the immobilized antibody. Unbound sample is then removed from the immobilized polypeptide-antibody complexes and a detection reagent (preferably a second antibody capable of binding to a different site on the polypeptide) containing a reporter group is added. The amount of detection reagent that remains bound to the solid support is then determined using a method appropriate for the specific reporter group.

More specifically, once the antibody is immobilized on the support as described above, the remaining protein binding sites on the support are typically blocked. Any suitable blocking agent known to those of ordinary skill in the art, such as bovine serum albumin or Tween 20™ (Sigma Chemical Co., St. Louis, MO). The immobilized antibody is then incubated with the sample, and polypeptide is allowed to bind to the antibody. The sample may be diluted with a suitable diluent, such as phosphate-buffered saline (PBS) prior to incubation. In general, an appropriate contact time (*i*.*e*., incubation time) is a period of time that is sufficient to detect the presence of polypeptide within a sample obtained from an individual with ovarian cancer. Preferably, the contact time is sufficient to achieve a level of binding that is at least about 95% of that achieved at equilibrium between bound and unbound polypeptide. Those of ordinary skill in the art will recognize that the time necessary to achieve equilibrium may be readily determined by assaying the level of binding that occurs over a period of time. At room temperature, an incubation time of about 30 minutes is generally sufficient.

Unbound sample may then be removed by washing the solid support with an appropriate buffer, such as PBS containing 0.1% Tween 20™. The second antibody, which contains a reporter group, may then be added to the solid support. Preferred reporter groups include those groups recited above.

The detection reagent is then incubated with the immobilized antibody-polypeptide complex for an amount of time sufficient to detect the bound polypeptide. An appropriate amount of time may generally be determined by assaying the level of binding that occurs over a period of time. Unbound detection reagent is then removed and bound detection reagent is detected using the reporter group. The method employed for detecting the reporter group depends upon the nature of the reporter group. For radioactive groups, scintillation counting or autoradiographic methods are generally appropriate. Spectroscopic methods may be used to detect dyes, luminescent groups and fluorescent groups. Biotin may be detected using avidin, coupled to a different reporter group (commonly a radioactive or fluorescent group or an enzyme). Enzyme reporter groups may generally be detected by the addition of substrate (generally for a specific period of time), followed by spectroscopic or other analysis of the reaction products.

To determine the presence or absence of a cancer, such as ovarian cancer, the signal detected from the reporter group that remains bound to the solid support is generally compared to a signal that corresponds to a predetermined cut-off value. In one preferred embodiment, the cut-off value for the detection of a cancer is the average mean signal obtained when the immobilized antibody is incubated with samples from patients without the cancer. In general, a sample generating a signal that is three standard deviations above the predetermined cut-off value is considered positive for the cancer. In an alternate preferred embodiment, the cut-off value is determined using a Receiver Operator Curve, according to the method of Sackett et al., Clinical Epidemiology: A Basic Science for Clinical Medicine, Little Brown and Co., 1985, p. 106-7. Briefly, in this embodiment, the cut-off value maybe determined from a plot of pairs of true positive rates (*i.e*., sensitivity) and false positive rates (100%-specificity) that correspond to each possible cut-off value for the diagnostic test result. The cut-off value on the plot that is the closest to the upper left-hand corner (*i*.*e*., the value that encloses the largest area) is the most accurate cut-off value, and a sample generating a signal that is higher than the cut-off value determined by this method may be considered positive. Alternatively, the cut-off value may be shifted to the left along the plot, to minimize the false positive rate, or to the right, to minimize the false negative rate. In general, a sample generating a signal that is higher than the cut-off value determined by this method is considered positive for a cancer.

In a related embodiment, the assay is performed in a flow-through or strip test format, wherein the binding agent is immobilized on a membrane, such as nitrocellulose. In the flow-through test, polypeptides within the sample bind to the immobilized binding agent as the sample passes through the membrane. A second, labeled binding agent then binds to the binding agent-polypeptide complex as a solution containing the second binding agent flows through the membrane. The detection of bound second binding agent may then be performed as described above. In the strip test format, one end of the membrane to which binding agent is bound is immersed in a solution containing the sample. The sample migrates along the membrane through a region containing second binding agent and to the area of immobilized binding agent. Concentration of second binding agent at the area of immobilized antibody indicates the presence of a cancer. Typically, the concentration of second binding agent at that site generates a pattern, such as a line, that can be read visually. The absence of such a pattern indicates a negative result. In general, the amount of binding agent immobilized on the membrane is selected to generate a visually discernible pattern when the biological sample contains a level of polypeptide that would be sufficient to generate a positive signal in the two-antibody sandwich assay, in the format discussed above. Preferred binding agents for use in such assays are antibodies and antigen-binding fragments thereof. Preferably, the amount of antibody immobilized on the membrane ranges from about 25 ng to about 1µg, and more preferably from about 50 ng to about 500 ng. Such tests can typically be performed with a very small amount of biological sample.

Of course, numerous other assay protocols exist that are suitable for use with the tumor proteins or binding agents of the present invention. The above descriptions are intended to be exemplary only. For example, it will be apparent to those of ordinary skill in the art that the above protocols may be readily modified to use ovarian carcinoma polypeptides to detect antibodies that bind to such polypeptides in a biological sample. The detection of such ovarian carcinoma protein specific antibodies may correlate with the presence of a cancer.

A cancer may also, or alternatively, be detected based on the presence of T cells that specifically react with an ovarian carcinoma protein in a biological sample. Within certain methods, a biological sample comprising CD4⁺ and/or CD8⁺ T cells isolated from a patient is incubated with an ovarian carcinoma protein, a polynucleotide encoding such a polypeptide and/or an APC that expresses at least an immunogenic portion of such a polypeptide, and the presence or absence of specific activation of the T cells is detected. Suitable biological samples include, but are not limited to, isolated T cells. For example, T cells may be isolated from a patient by routine techniques (such as by Ficoll/Hypaque density gradient centrifugation of peripheral blood lymphocytes). T cells may be incubated *in vitro* for 2-9 days (typically 4 days) at 37°C with an ovarian carcinoma protein (*e*.*g*., 5 - 25 µg/ml). It may be desirable to incubate another aliquot of a T cell sample in the absence of ovarian carcinoma protein to serve as a control. For CD4⁺ T cells, activation is preferably detected by evaluating proliferation of the T cells. For CD8⁺ T cells, activation is preferably detected by evaluating cytolytic activity. A level of proliferation that is at least two fold greater and/or a level of cytolytic activity that is at least 20% greater than in disease-free patients indicates the presence of a cancer in the patient.

As noted above, a cancer may also, or alternatively, be detected based on the level of mRNA encoding an ovarian carcinoma protein in a biological sample. For example, at least two oligonucleotide primers may be employed in a polymerase chain reaction (PCR) based assay to amplify a portion of an ovarian carcinoma protein cDNA derived from a biological sample, wherein at least one of the oligonucleotide primers is specific for (*i*.*e*., hybridizes to) a polynucleotide encoding the ovarian carcinoma protein. The amplified cDNA is then separated and detected using techniques well known in the art, such as gel electrophoresis. Similarly, oligonucleotide probes that specifically hybridize to a polynucleotide encoding an ovarian carcinoma protein may be used in a hybridization assay to detect the presence of polynucleotide encoding the tumor protein in a biological sample.

To permit hybridization under assay conditions, oligonucleotide primers and probes should comprise an oligonucleotide sequence that has at least about 60%, preferably at least about 75% and more preferably at least about 90%, identity to a portion of a polynucleotide encoding an ovarian carcinoma protein that is at least 10 nucleotides, and preferably at least 20 nucleotides, in length. Preferably, oligonucleotide primers and/or probes hybridize to a polynucleotide encoding a polypeptide described herein under moderately stringent conditions, as defined above. Oligonucleotide primers and/or probes which may be usefully employed in the diagnostic methods described herein preferably are at least 10-40 nucleotides in length. In a preferred embodiment, the oligonucleotide primers comprise at least 10 contiguous nucleotides, more preferably at least 15 contiguous nucleotides, of a DNA molecule having a sequence provided herein. Techniques for both PCR based assays and hybridization assays are well known in the art (*see*, for example, Mullis et al., Cold Spring Harbor Symp. Quant. Biol., 51:263, 1987; Erlich ed., PCR Technology, Stockton Press, NY, 1989).

One preferred assay employs RT-PCR, in which PCR is applied in conjunction with reverse transcription. Typically, RNA is extracted from a biological sample such as a biopsy tissue and is reverse transcribed to produce cDNA molecules. PCR amplification using at least one specific primer generates a cDNA molecule, which may be separated and visualized using, for example, gel electrophoresis. Amplification may be performed on biological samples taken from a test patient and from an individual who is not afflicted with a cancer. The amplification reaction may be performed on several dilutions of cDNA spanning two orders of magnitude. A two-fold or greater increase in expression in several dilutions of the test patient sample as compared to the same dilutions of the non-cancerous sample is typically considered positive.

In another embodiment, ovarian carcinoma proteins and polynucleotides encoding such proteins may be used as markers for monitoring the progression of cancer. In this embodiment, assays as described above for the diagnosis of a cancer may be performed over time, and the change in the level of reactive polypeptide(s) evaluated. For example, the assays may be performed every 24-72 hours for a period of 6 months to 1 year, and thereafter performed as needed. In general, a cancer is progressing in those patients in whom the level of polypeptide detected by the binding agent increases over time. In contrast, the cancer is not progressing when the level of reactive polypeptide either remains constant or decreases with time.

Certain *in vivo* diagnostic assays may be performed directly on a tumor. One such assay involves contacting tumor cells with a binding agent. The bound binding agent may then be detected directly or indirectly via a reporter group. Such binding agents may also be used in histological applications. Alternatively, polynucleotide probes may be used within such applications.

As noted above, to improve sensitivity, multiple ovarian carcinoma protein markers may be assayed within a given sample. It will be apparent that binding agents specific for different proteins provided herein may be combined within a single assay. Further, multiple primers or probes may be used concurrently. The selection of tumor protein markers may be based on routine experiments to determine combinations that results in optimal sensitivity. In addition, or alternatively, assays for tumor proteins provided herein may be combined with assays for other known tumor antigens.

### Diagnostic Kits

The present invention further provides kits for use within any of the above diagnostic methods. Such kits typically comprise two or more components necessary for performing a diagnostic assay. Components may be compounds, reagents, containers and/or equipment. For example, one container within a kit may contain a monoclonal antibody or fragment thereof that specifically binds to an ovarian carcinoma protein. Such antibodies or fragments may be provided attached to a support material, as described above. One or more additional containers may enclose elements, such as reagents or buffers, to be used in the assay. Such kits may also, or alternatively, contain a detection reagent as described above that contains a reporter group suitable for direct or indirect detection of antibody binding.

Alternatively, a kit may be designed to detect the level of mRNA encoding an ovarian carcinoma protein in a biological sample. Such kits generally comprise at least one oligonucleotide probe or primer, as described above, that hybridizes to a polynucleotides encoding an ovarian carcinoma protein. Such an oligonucleotide may be used, for example, within a PCR or hybridization assay. Additional components that may be present within such kits include a second oligonucleotide and/or a diagnostic reagent or container to facilitate the detection of a polynucleotide encoding an ovarian carcinoma protein.

The following Examples are offered by way of illustration and not by way of limitation.

### EXAMPLES

### EXAMPLE 1

### IDENTIFICATION OF REPRESENTATIVE OVARIAN CARCINOMA PROTEIN cDNAs

This Example illustrates the identification of cDNA molecules encoding ovarian carcinoma proteins.

Anti-SCID mouse sera (generated against sera from SCID mice carrying late passage ovarian carcinoma) was pre-cleared of E. coli and phage antigens and used at a 1:200 dilution in a serological expression screen. The library screened was made from a SCID-derived human ovarian tumor (OV9334) using a directional RH oligo(dT) priming cDNA library construction kit and the λSereen vector (Novagen). A bacteriophage lambda screen was employed. Approximately 400,000 pfu of the amplified OV9334 library were screened.

196 positive clones were isolated. Certain sequences that appear to be novel are provided in Figures 1A-1S and SEQ ID NO:1 to 71. Three complete insert sequences are shown in Figures 2A-2C (SEQ ID NO:72 to 74). Other clones having known sequences are presented in Figures 15A-15EEE (SEQ ID NO:82 to 310). Database searches identified the following sequences that were substantially identical to the sequences presented in Figures 15A-15EEE.

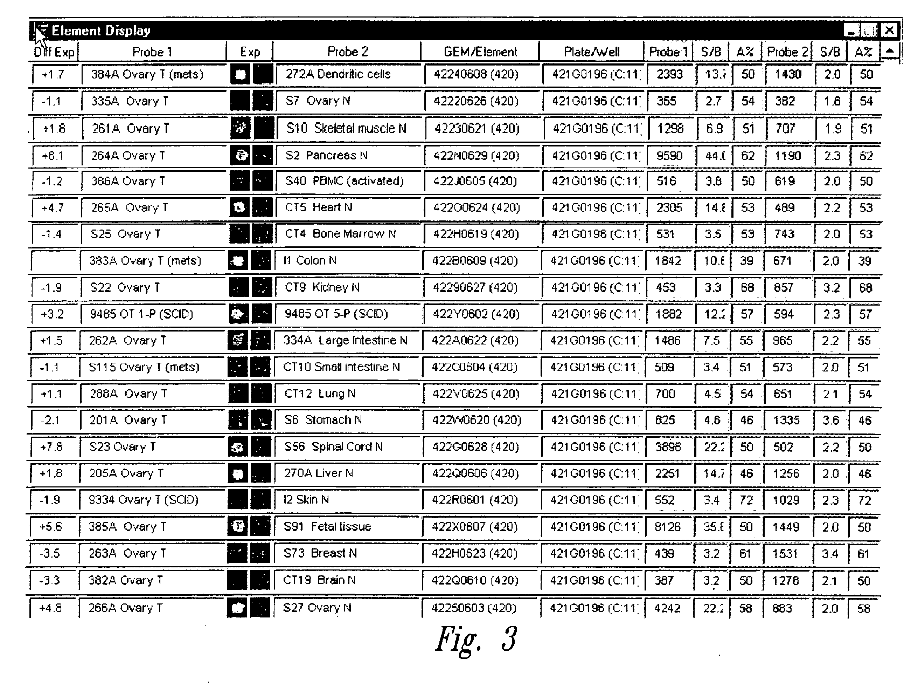

These clones were further characterized using microarray technology to determine mRNA expression levels in a variety of tumor and normal tissues. Such analyses were performed using a Synteni (Palo Alto, CA) microarray, according to the manufacturer's instructions. PCR amplification products were arrayed on slides, with each product occupying a unique location in the array. mRNA was extracted from the tissue sample to be tested, reverse transcribed and fluorescent-labeled cDNA probes were generated. The microarrays were probed with the labeled cDNA probes and the slides were scanned to measure fluorescence intensity. Data was analyzed using Synteni's provided GEMtools software. The results for one clone (13695, also referred to as 08E) are shown in Figure 3.

### EXAMPLE 2

### IDENTIFICATION OF OVARIAN CARCINOMA CDNAS USING MICROARRAY TECHNOLOGY

This Example illustrates the identification of ovarian carcinoma polynucleotides by PCR subtraction and microarray analysis. Microarrays of cDNAs were analyzed for ovarian tumor-specific expression using a Synteni (Palo Alto, CA) microarray, according to the manufacturer's instructions (and essentially as described by Schena et al., Proc. Natl. Acad. Sci. USA 93:10614-10619, 1996 and Heller et al., Proc. Natl. Acad. Sci. USA 94:2150-2155, 1997).

A PCR subtraction was performed using a tester comprising cDNA of four ovarian tumors (three of which were metastatic tumors) and a driver of cDNA form five normal tissues (adrenal gland, lung, pancreas, spleen and brain). cDNA fragments recovered from this subtraction were subjected to DNA microarray analysis where the fragments were PCR amplified, adhered to chips and hybridized with fluorescently labeled probes derived from mRNAs of human ovarian tumors and a variety of normal human tissues. In this analysis, the slides were scanned and the fluorescence intensity was measured, and the data were analyzed using Synteni's GEMtools software. In general, sequences showing at least a 5-fold increase in expression in tumor cells (relative to normal cells) were considered ovarian tumor antigens. The fluorescent results were analyzed and clones that displayed increased expression in ovarian tumors were further characterized by DNA sequencing and database searches to determine the novelty of the sequences.

Using such assays, an ovarian tumor antigen was identified that is a splice fusion between the human T-cell leukemia virus type I oncoprotein TAX (*see* Jin et al., Cell 93:81-91, 1998) and an extracellular matrix protein called osteonectin. A splice junction sequence exists at the fusion point. The sequence of this clone is presented in Figure 4 and SEQ ID NO:75. Osteonectin, unspliced and unaltered, was also identified from such assays independently.

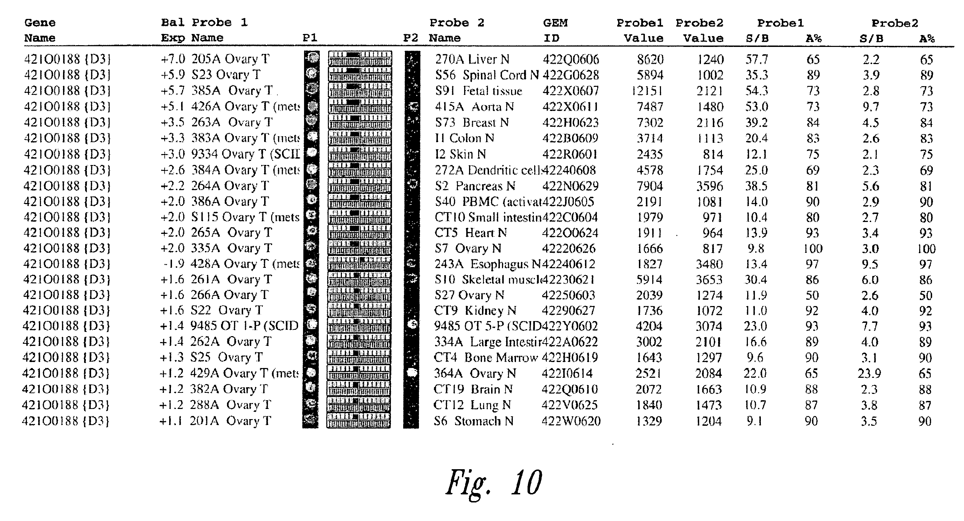

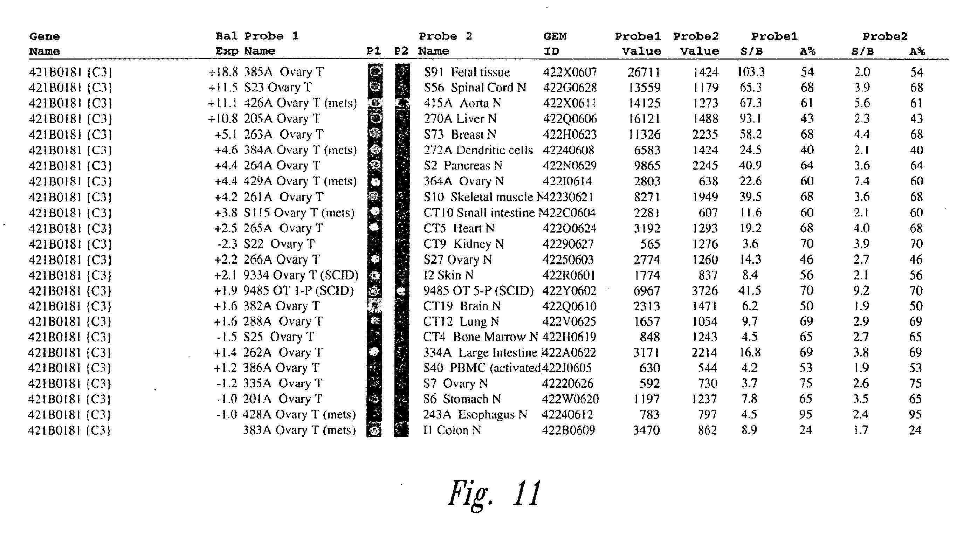

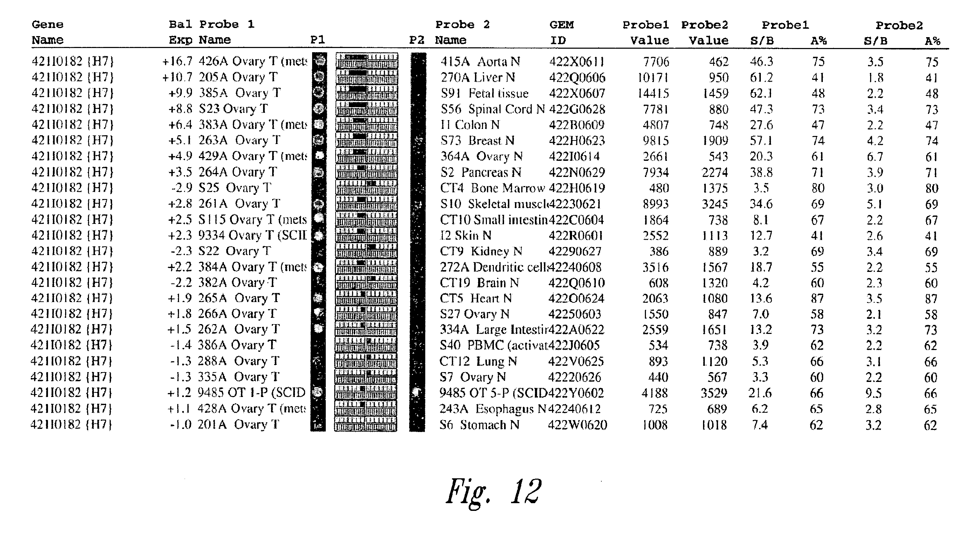

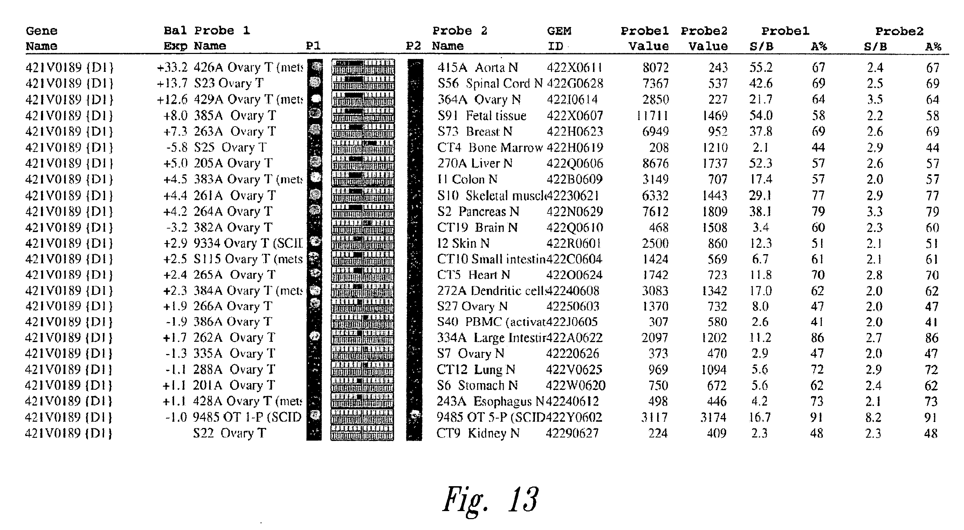

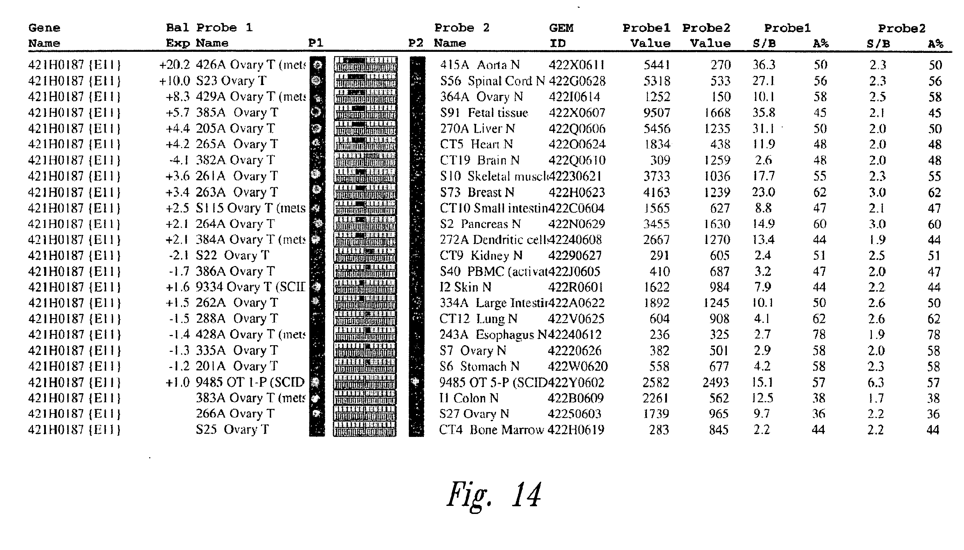

Further clones identified by this method are referred to herein as 3f, 6b, 8e, 8h, 12c and 12h. Sequences of these clones are shown in Figures 5 to 9 and SEQ ID NO:76 to 81. Microarray analyses were performed as described above, and are presented in Figures 10 to 14. A full length sequence encompassing clones 3f, 6b, 8e and 12h was obtained by screening an ovarian tumor (SCID-derived) cDNA library. This 2996 base pair sequence (designated 0772P) is presented in SEQ ID NO:311, and the encoded 914 amino acid protein sequence is shown in SEQ ID NO:312. PSORT analysis indicates a Type 1a transmembrane protein localized to the plasma membrane.

In addition to certain of the sequences described above, this screen identified the following sequences which are described in detail in Table 1:

**Table 1**

| Sequence | Comments |
|---|---|
| OV4vG11 (SEQ ID NO:313) | human clone 1119D9 on chromosome 20p12 |
| OV4vB11 (SEQ ID NO:314) | human UWGC:y14c094 from chromosome 6p21 |
| OV4vD9 (SEQ ID NO:315) | human clone 1049G16 chromosome 20q12-13.2 |
| OV4vD5 (SEQ ID NO:316) | human KIAA0014 gene |
| OV4vC2 (SEQ ID NO:317) | human KIAA0084 gene |
| OV4vF3 (SEQ ID NO:318) | human chromosome 19 cosmic R31167 |
| OV4VC1 (SEQ ID NO:319) | novel |
| OV4vH3 (SEQ ID NO:320) | novel |
| OV4vD2 (SEQ ID NO:321) | novel |
| O815P (SEQ ID NO:322) | novel |
| OV4vC12 (SEQ ID NO:323) | novel |
| OV4vA4 (SEQ ID NO:324) | novel |
| OV4vA3 (SEQ ID NO:325) | novel |
| OV4v2A5 (SEQ ID NO:326) | novel |
| 0819P (SEQ ID NO:327) | novel |
| 0818P (SEQ ID NO:328) | novel |
| 0817P (SEQ ID NO:329) | novel |
| O816P (SEQ ID NO:330) | novel |
| Ov4vC5 (SEQ ID NO:331) | novel |
| 21721 (SEQ ID NO:332) | human lumican |
| 21719 (SEQ ID NO:333) | human retinoic acid-binding protein II |
| 21717 (SEQ ID NO:334) | human26S proteasome ATPase subunit |
| 21654 (SEQ ID NO:335) | human copine I |
| 21627 (SEQ ID NO:336) | human neuron specific gamma-2 enolase |
| 21623 (SEQ ID NO:337) | human geranylgeranyl transferase II |
| 21621 (SEQ ID NO:338) | human cyclin-dependent protein kinase |
| 21616 (SEQ ID NO:339) | human prepro-megakaryocyte potentiating factor |
| 21612 (SEQ ID NO:340) | human UPH1 |
| 21558 (SEQ ID NO:341) | human RalGDS-like 2 (RGL2) |
| 21555 (SEQ ID NO:342) | human autoantigen P542 |
| 21548 (SEQ ID NO:343) | human actin-related protein (ARP2) |
| 21462 (SEQ ID NO:344) | human huntingtin interacting protein |
| 21441 (SEQ ID NO:345) | human 90K product (tumor associated antigen) |
| 21439 (SEQ ID NO:346) | human guanine nucleotide regulator protein (tim1) |
| 21438 (SEQ ID NO:347) | human Ku autoimmune (p70/p80) antigen |
| 21237 (SEQ ID NO:348) | human S-laminin |
| 21436 (SEQ ID NO:349) | human ribophorin I |
| 21435 (SEQ ID NO:350) | human cytoplasmic chaperonin hTRiC5 |
| 21425 (SEQ ID NO:351) | humanEMX2 |
| 21423 (SEQ ID NO:352) | human p87/p89 gene |
| 21419 (SEQ ID NO:353) | human HPBRII-7 |
| 21252 (SEQ ID NO:354) | human T1-227H |
| 21251 (SEQ ID NO:355) | human cullin I |
| 21247 (SEQ ID NO:356) | kunitz type protease inhibitor (KOP) |
| 21244-1 (SEQ ID NO:357) | human protein tyrosine phosphatase receptor F (PTPRF) |
| 21718 (SEQ ID NO:358) | human LTR repeat |
| OV2-90 (SEQ ID NO:359) | novel |
| Human zinc finger (SEQ ID NO:360) | |
| Human polyA binding protein (SEQ ID NO:361) | |
| Human pleitrophin (SEQ ID NO:362) | |
| Human PAC clone 278C19 (SEQ ID NO:363) | |
| Human LLRep3 (SEQ ID NO:364) | |
| Human Kunitz type protease inhib (SEQ ID NO:365) | |
| Human KIAA0106 gene (SEQ ID NO:366) | |
| Human keratin (SEQ ID NO:367) | |
| Human HIV-1TAR (SEQ ID NO:368) | |
| Human glia derived nexin (SEQ ID NO:369) | |
| Human fibronectin (SEQ ID NO:370) | |
| Human ECMproBM40 (SEQ ID NO:371) | |
| Human collagen (SEQ ID NO:372) | |
| Human alpha enolase (SEQ ID NO:373) | |
| Human aldolase (SEQ ID NO:374) | |
| Human transf growth factor BIG H3 (SEQ ID NO:375) | |
| Human SPARC osteonectin (SEQ ID NO:376) | |
| Human SLPI leucocyte protease (SEQ ID NO:377) | |
| Human mitochondrial ATP synth (SEQ ID NO:378) | |
| Human DNA seq clone 461P17 (SEQ ID NO:379) | |
| Human dbpB pro Y box (SEQ ID NO:380) | |
| Human 40 kDa keratin (SEQ ID NO:381) | |
| Human arginosuccinate synth (SEQ ID NO:382) | |
| Human acidic ribosomal phosphoprotein (SEQ ID NO:383) | |
| Human colon carcinoma laminin binding pro (SEQ ID NO:384) | |

This screen further identified multiple forms of the clone O772P, referred to herein as 21013, 21003 and 21008. PSORT analysis indicates that 21003 (SEQ ID NO:386; translated as SEQ ID NO:389) and 21008 (SEQ ID NO:387; translated as SEQ ID NO:390) represent Type 1a transmembrane protein forms of 0772P. 21013 (SEQ ID NO:385; translated as SEQ ID NO:388) appears to be a truncated form of the protein and is predicted by PSORT analysis to be a secreted protein.

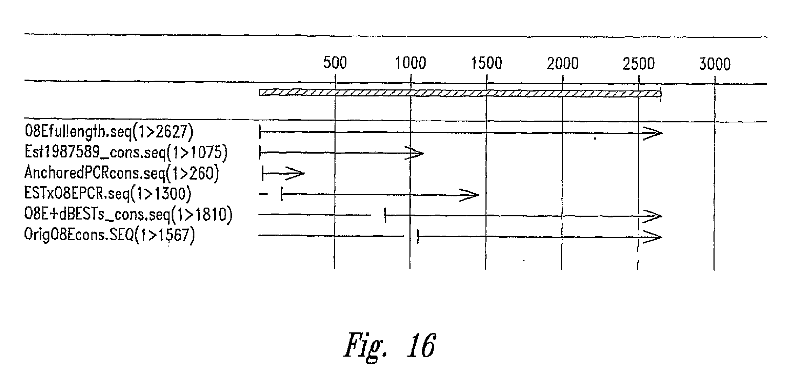

Additional sequence analysis resulted in a full length clone for O8E (2627 bp, which agrees with the message size observed by Northern analysis; SEQ ID NO:391). This nucleotide sequence was obtained as follows: the original O8E sequence (OrigO8Econs) was found to overlap by 33 nucleotides with a sequence from an EST clone (IMAGE#1987589). This clone provided 1042 additional nucleotides upstream of the original O8E sequence. The link between the EST and O8E was confirmed by sequencing multiple PCR fragments generated from an ovary primary tumor library using primers to the unique EST and the 08E sequence (ESTxO8EPCR). Full length status was further indicated when anchored PCR from the ovary tumor library gave several clones (AnchoredPCR cons) that all terminated upstream of the putative start methionine, but failed to yield any additional sequence information. Figure 16 presents a diagram that illustrates the location of each partial sequence within the full length O8E sequence.

Two protein sequences may be translated from the full length 08E. For "a" (SEQ ID NO:393) begins with a putative start methionine. A second form "b" (SEQ ID NO:392) includes 27 additional upstream residues to the 5' end of the nucleotide sequence.

### EXAMPLE 3

This example discloses the identification and characterization of antibody epitopes recognized by the 08E polyclonal anti-sera.

Rabbit anti-sera was raised against E. coli derived O8E recombinant protein and tested for antibody epitope recognition against 20 or 21 mer peptides that correspond to the 08E amino acid sequence. Peptides spanning amino acid regions 31 to 65, 76 to 110, 136 to 200 and 226 to 245 of the full length 08E protein were recognized by an acid eluted peak and/or a salt eluted peak from affinity purified anti-O8E sera. Thus, the corresponding amino acid sequences of the above peptides constitute the antibody epitopes recognized by affinity purified anti-O8E antibodies.

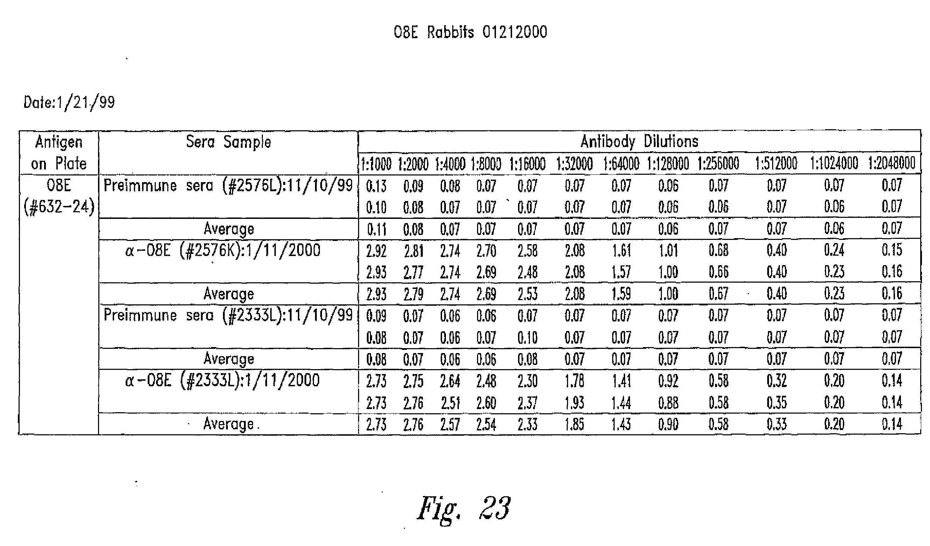

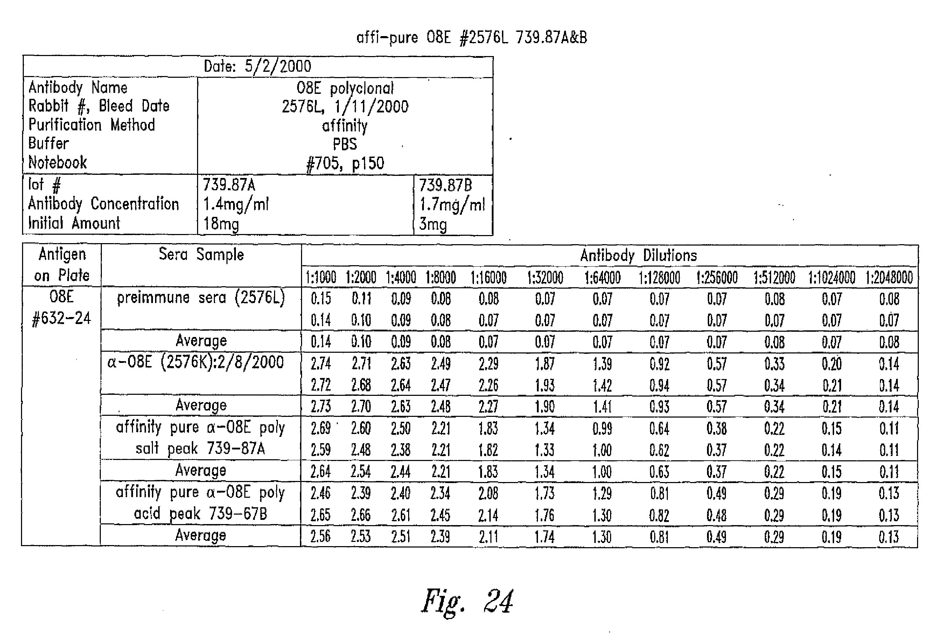

ELISA analysis of anti-08E rabbit sera is shown in Figure 23, and ELISA analysis of affinity purified rabbit anti-08E polyclonal antibody is shown in Figure 24.

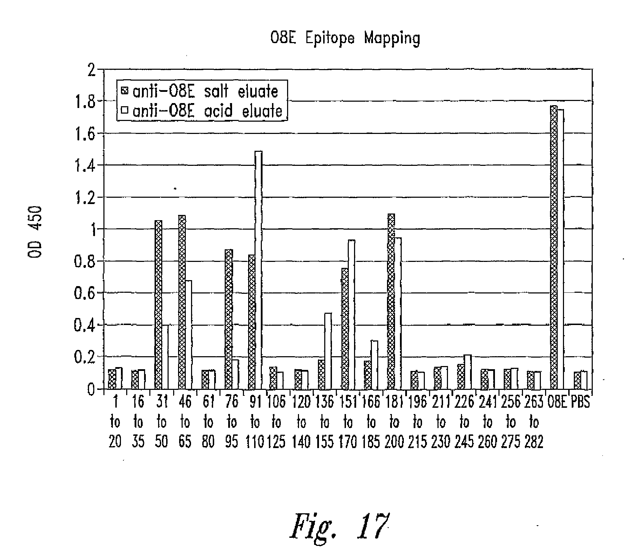

For epitope mapping, 20 or 21 mer peptides corresponding to the O8E protein were synthesized. For antibody affinity purification, rabbit anti-O8E sera was run over an O8E-sepharose column, then antibody was eluted with a salt buffer containing 0.5 M NaCl and 20 mM PO₄, followed by an acid elution step using 0.2 M Glycine, pH 2.3. Purified antibody was neutralized by the addition of 1M Tris, pH 8 and buffer exchanged into phosphate buffered saline (PBS). For enzyme linked immunosorbant assay (ELISA) analysis, 08E peptides and O8E recombinant protein were coated onto 96 well flat bottom plates at 2 µg/ml for 2 hours at room temperature (RT). Plates were then washed 5 times with PBS + 0.1 % Tween 20 and blocked with PBS + 1 % bovine serum albumin (BSA) for 1 hour. Affinity purified anti-08E antibody, either an acid or salt eluted fraction, was then added to the wells at 1 µg/ml and incubated at RT for 1 hr. Plates were again washed, followed by the addition of donkey anti-rabbit-Ig-horseradish peroxidase (HRP) antibody for 1 hour at RT. Plates were washed, then developed by the addition of the chromagenic substrate 3, 3', 5, 5'-tetramethylbenzidine (TMB) (described by Bos et al., J. of Immunoassay 2:187-204 (1981); available from Sigma (St. Louis, MO)). The reaction was incubated 15 minutes at RT and then stopped by the addition of 1 N H₂SO₄. Plates were read at an optical density of 450 (OD450) in an automated plate reader. The sequences of peptides corresponding to the OE8 antibody epitopes are disclosed herein as SEQ ID NO: 394-415. Antibody epitopes recognized by the O8E polyclonal anti-sera are disclosed herein in Figure 17.

### EXAMPLE 4

This example discloses IHC analysis of 08E expression in ovarian cancer tissue samples.

For immunohistochemistry studies, paraffin-embedded formalin fixed ovarian cancer tissue was sliced into 8 micron sections. Steam heat induced epitope retrieval (SHIER) in 0.1 M sodium citrate buffer (pH 6.0) was used for optimal staining conditions. Sections were incubated with 10% serum/PBS for 5 minutes. Primary antibody (anti-08E rabbit affinity purified polyclonal antibody) was added to each section for 25 min followed by a 25 min incubation with an anti-rabbit biotinylated antibody. Endogenous peroxidase activity was blocked by three 1.5 min incubations with hydrogen peroxidase. The avidin biotin complex/horse radish peroxidase system was used along with DAB chromogen to visualize antigen expression. Slides were counterstained with hematoxylin. One (papillary serous carcinoma) of six ovarian cancer tissue sections displayed 08E immunoreactivity. Upon optimization of the staining conditions, 4/5 ovarian cancer samples stained positive using the O8E polyclonal antibody. 08E expression was localized to the plasma membrane.

Six ovarian cancer tissues were analyzed with the anti-O8E rabbit polyclonal antibody. One (papillary serous carcinoma) of six ovarian cancer tissue samples stained positive for O8E expression. 08E expression was localized to the surface membrane.

### EXAMPLE 5

This example discloses O8E peptides that are predicted to bind HLA-A2 and to be immunogenic for CDS T cell responses in humans.

Potential HLA-A2 binding peptides of O8E were predicted by using the full-length open-reading frame (ORF) from 08E and running it through "Episeek," a program used to predict MHC binding peptides. The program used is based on the algorithm published by Porker, K.C. et al., J. Immunol. 152(1):163-175 (1994) (incorporated by reference herein in its entirety). 10-mer and 9-mer peptides predicted to bind HLA-0201 are disclosed herein as SEQ ID NO: 416-435 and SEQ ID NO: 436-455, respectively.

### EXAMPLE 6

This example discloses 08E cell surface expression measured by fluoresence activated cell sorting.

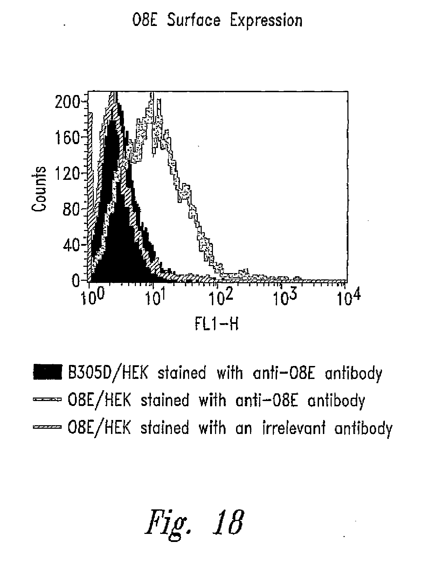

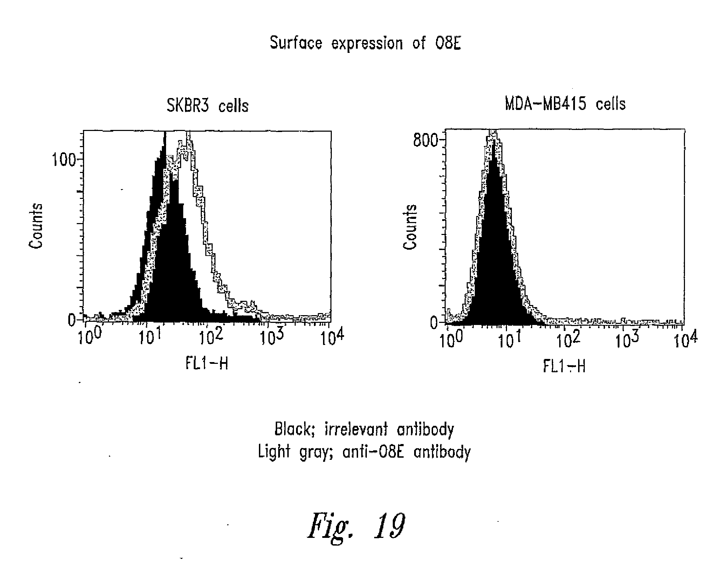

For FACS analysis, cells were washed with ice cold staining buffer (PBS/1% BSA/azide). Next, the cells were incubated for 30 minutes on ice with 10 micrograms/ml of affinity purified rabbit anti-B305D polyclonal antibody. The cells were washed 3 times with staining buffer and then incubated with a 1:100 dilution of a goat anti-rabbit lg (H+L)-FITC reagent (Southern Biotechnology) for 30 minutes on ice. Following 3 washes, the cells were resuspended in staining buffer containing prodium iodide, a vital stain that allows for identification of permeable cells, and analyzed by FACS. 08E surface expression was confirmed on SKBR3 breast cancer cells and HEK293 cells that stably overexpress the cDNA for O8E. Neither MB415 cells nor HEK293 cells stably transfected with a control irrelevant plasmid DNA showed surface expression of O8E (Figures 18 and 19).

### EXAMPLE 7

This example further evaluates the expression and surface localization of O8E.

For expression and purification of antigen used for immunization, O8E expressed in an E. coli recombinant expression system was grown overnight in LB Broth with the appropriate antibiotics at 37°C in a shaking incubator. The next morning, 10 ml of the overnight culture was added to 500 ml of 2x YT plus appropriate antibiotics in a 2L-baffled Erlenmeyer flask. When the Optical Density (at 560 nanometers) of the culture reached 0.4-0.6 the cells were induced with IPTG (1 mM). 4 hours after induction with IPTG the cells were harvested by centrifugation. The cells were then washed with phosphate buffered saline and centrifuged again. The supernatant was discarded and the cells were,either frozen for future use or immediately processed. Twenty milliliters of lysis buffer was added to the cell pellets and vortexed. To break open the E. coli cells, this mixture was then run through the French Press at a pressure of 16,000 psi. The cells were then centrifuged again and the supernatant and pellet were checked by SDS-PAGE for the partitioning of the recombinant protein. For protein that localized to the cell pellet, the pellet was resuspended in 10 mM Tris pH 8.0 , 1% CHAPS and the inclusion body pellet was washed and centrifuged again. This procedure was repeated twice more. The washed inclusion body pellet was solubilized with either 8 M urea or 6 M guanidine HCl containing 10 mM Tris pH 8.0 plus 10 mM imidazole. The solubilized protein was added to 5 ml of nickel-chelate resin (Qiagen) and incubated for 45 min to 1 hour at room temperature with continuous agitation. After incubation, the resin and protein mixture were poured through a disposable column and the flow through was collected. The column was then washed with 10-20 column volumes of the solubilization buffer. The antigen was then eluted from the column using 8M urea, 10 mM tris pH 8.0 and 300 mM imidazole and collected in 3 ml fractions. A SDS-PAGE gel was run to determine which fractions to pool for further purification. As a final purification step, a strong anion exchange resin such as Hi-Prep Q (Biorad) was equilibrated with the appropriate buffer and the pooled fractions from above were loaded onto the column. Each antigen was eluted off of the column with an increasing salt gradient. Fractions were collected as the column was run and another SDS-PAGE gel was run to determine which fractions from the column to pool. The pooled fractions were dialyzed against 10 mM Tris pH 8.0. This material was then evaluated for acceptable purity as determined by SDS-PAGE or HPLC, concentration as determined by Lowry assay or Amino Acid Analysis, identity as determined by amino terminal protein sequence, and endotoxin level as determined by the Limulus (LAL) assay. The proteins were then vialed after filtration through a 0.22 micron filter and the antigens were frozen until needed for immunization.

For generation of polyclonal anti-sera, 400 micrograms of each prostate antigen was combined with 100 micrograms of muramyldipeptide (MDP). Equal volume of Incomplete Freund's Adjuvant (IFA) was added and then mixed. Every four weeks animals were boosted with 100 micrograms of antigen mixed with an equal volume of IFA. Seven days following each boost the animal was bled. Sera was generated by incubating the blood at 4°C for 12-24 hours followed by centrifugation.

For characterization of polyclonal antisera, 96 well plates were coated with antigen by incubating with 50 microliters (typically 1 microgram) at 4°C for 20 hrs. 250 microliters of BSA blocking buffer was added to the wells and incubated at RT for 2 hrs. Plates were washed 6 times with PBS/0.01% tween. Anti-O8E rabbit sera or affinity purified anti-O8e antibody was diluted in PBS. Fifty microliters of diluted antibody was added to each well and incubated at RT for 30 min. Plates were washed as described above before 50 microliters of goat anti-rabbit horse radish peroxidase (HRP) at a 1:10000 dilution was added and incubated at RT for 30 min. Plates were washed as described above and 100 microliters of TMB microwell Peroxidase Substrate was added to each well. Following a 15 minute incubation in the dark at room temperature the colorimetric reaction was stopped with 100 microliters of 1N H2SO4 and read immediately at 450 nm. All polyclonal antibodies showed immunoreactivity to the O8E antigen.

For recombinant expression in mammalian HEK293 cells, full length 08E cDNA was subcloned into the mammalian expression vectors pcDNA3.1+ and pCEP4 (Invitrogen) which were modified to contain His and FLAG epitope tags, respectively. These constructs were transfected into HEK293 cells (ATCC) using Fugene 6 reagent (Roche). Briefly, HEK293 cells were plated at a density of 100,000 cells/ml in DMEM (Gibco) containing 10% FBS (Hyclone) and grown overnight. The following day, 2 ul of Fugene6 was added to 100 ul of DMEM containing no FBS and incubated for 15 minutes at room temperature. The Fugene6/DMEM mixture was then added to 1ug of O8E/pCEP4 or O8E/pcDNA3.1 plasmid DNA and incubated for 15 minutes at room temperature. The Fugene/DNA mix was then added to the HEK293 cells and incubated for 48-72 hrs at 37°C with 7% CO2. Cells were rinsed with PBS then collected and pelleted by centrifugation. For Western blot analysis, whole cell lysates were generated by incubating the cells in Triton-X100 containing lysis buffer for 30 minutes on ice. Lysates were then cleared by centrifugation at 10,000rpm for 5 minutes at 4 C. Samples were diluted with SDS-PAGE loading buffer containing betamercaptoethanol, then boiled for 10 minutes prior to loading the SDS-PAGE gel. Protein was transferred to nitrocellulose and probed using anti-O8E rabbit polyclonal sera #2333L at a dilution of 1:750. The blot was revealed with a goat anti-rabbit Ig coupled to HRP followed by incubation in ECL substrate.

For FACS analysis, cells were washed further with ice cold staining buffer (PBS+1%BSA+Azide). Next, the cells were incubated for 30 minutes on ice with 10ug/ml of Protein A purified anti-O8E polyclonal sera. The cells were washed 3 times with staining buffer and then incubated with a 1:100 dilution of a goat anti-rabbit Ig(H+L)-FITC reagent (Southern Biotechnology) for 30 minutes on ice. Following 3 washes, the cells were resuspended in staining buffer containing Propidium Iodide (PI), a vital stain that allows for the identification of permeable cells, and analyzed by FACS.

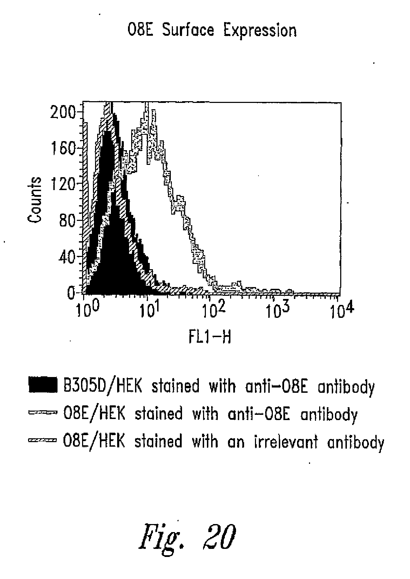

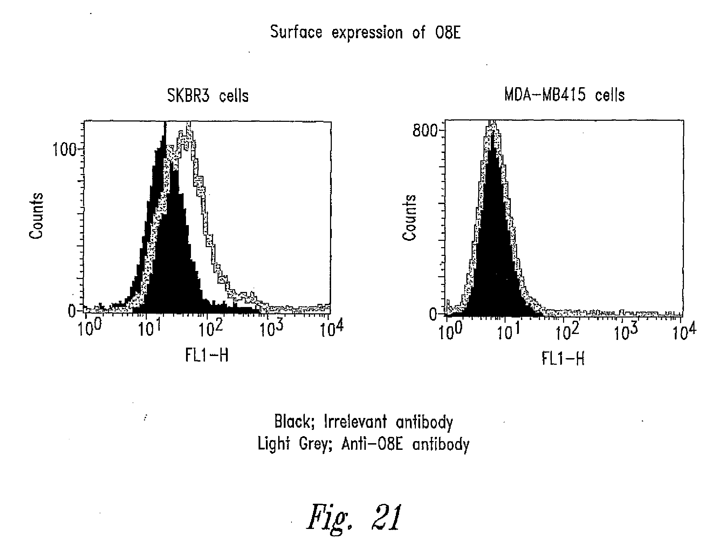

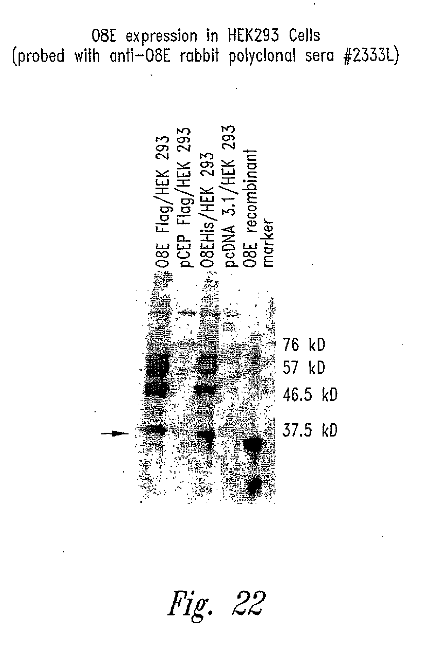

From these experiments, the results of which are illustrated in Figures 20-21, O8E expression was detected on the surface of transfected HEK293 cells and SKBR3 cells by FACS analysis using rabbit anti-O8E sera. Expression was also detected in transfected HEK293 cell lysates by Western blot analysis (Figure 22).

### EXAMPLE 8

### GENERATION AND CHARACTERIZATION OF ANTI-O8E MABS.

Mouse monoclonal antibodies were raised against E. coli derived 08E proteins as follows. A/J mice were immunized intraperitoneally (IP) with Complete Freund's Adjuvant (CFA) containing 50 µg recombinant O8E, followed by a subsequent IP boost with Incomplete Freund's Adjuvant (IFA) containing 10µg recombinant 08E protein. Three days prior to removal of the spleens, the mice were immunized intravenously with approximately 50µg of soluble O8E recombinant protein. The spleen of a mouse with a positive titer to O8E was removed, and a single-cell suspension made and used for fusion to SP2/0 myeloma cells to generate B cell hybridomas. The supernatants from the hybrid clones were tested by ELISA for specificity to recombinant 08E, and epitope mapped using peptides that spanned the entire O8E sequence. The mAbs were also tested by flow cytometry for their ability to detect O8E on the surface of cells stably transfected with O8E and on the surface of a breast tumor cell line.

For ELISA analysis, 96 well plates were coated with either recombinant O8E protein or overlapping 20-mer peptides spanning the entire O8E molecule at a concentration of either 1-2µg/ml or 10µg/ml, respectively. After coating, the plates were washed 5 times with washing buffer (PBS +0.1% Tween-20) and blocked with PBS containing 0.5% BSA, 0.4% Tween-20. Hybrid supernatants or purified mAbs were then added and the plates incubated for 60 minutes at room temperature. The plates were washed 5 times with washing buffer and the secondary antibody, donkey-anti mouse lg linked to horseradish peroxidase (HRP)(Jackson ImmunoResearch), was added for 60 minutes. The plates were again washed 5 times in washing buffer, followed by the addition of the peroxidase substrate. Of the hybridoma clones generated, 15 secreted mAbs that recognized the entire 08E protein. Epitope mapping revealed that of these 15 clones, 14 secreted mAbs that recognized the 08E amino acid residues 61-80 and one clone secreted a mAb that recognized amino acid residues 151-170.

For flow cytometric analysis, HEK293 cells which had been stably transfected with 08E and SKBR3 cells which express 08E mRNA, were harvested and washed in flow staining buffer (PBS+1%BSA+Azide). The cells were incubated with the supernatant from the mAb hybrids for 30 minutes on ice followed by 3 washes with staining buffer. The cells were incubated with goat-anti mouse Ig-FITC for 30 minutes on ice, followed by three washes with staining buffer before being resuspended in wash buffer containing propidium iodide. Flow cytometric analysis revealed that 15/15 mAbs were able to detect 08E protein expressed on the surface of 08E-transfected HEK293 cells. 6/6 mAbs tested on SKBR3 cells were able to recognize surface expressed O8E.

### EXAMPLE 9

### EXTENDED DNA AND PROTEIN SEQUENCE ANALYSIS OF SEQUENCE 0772P

A full-length sequence encompassing clones 3f, 6b, 8e, and 12 was obtained by screening an ovarian tumor (SCID-derived) cDNA library described in detail in Example 2. This 2996 base pair sequence, designated O772P, is presented in SEQ ID NO: 311, and the encoded 914 amino acid protein sequence is shown in SEQ ID NO: 312. The DNA sequence 0772P was searched against public databases including Genbank and showed a significant hit to Genbank Accession number AK024365 (SEQ ID NO: 457). This Genbank sequence was found to be 3557 base pairs in length and encodes a protein 1156 amino acids in length (SEQ ID NO: 459). A truncated version of this sequence, residues 25-3471, in which residue 25 corresponds to the first ATG initiation codon in the Genbank sequence, (SEQ ID NO: 456), encodes a protein that is 1148 amino acids in length (SEQ ID NO: 458). The published DNA sequence (SEQ ID NO: 457) differs from 0772P in that it has a 5 base pair insertion corresponding to bases 958-962 of SEQ ID NO: 457. This insertion results in a frame shift such that SEQ ID NO: 457 encodes an additional N-terminal protein sequence relative to 0772P (SEQ ID NO: 312). In addition, 0772P encodes a unique N-terminal portion contained in residues 1-79 (SEQ ID NO: 460). The N-terminal portion of SEQ ID NO: 456, residues 1-313, also contains unique sequence and is listed as SEQ ID NO: 461.

### EXAMPLE 10

### THE GENERATION OF POLYCLONAL ANTIBODIES FOR IMMUNOHISTOCHEMISTRY AND FLOW CYTOMETRIC ANALYSIS OF THE CELL ASSOCIATED EXPRESSION PATTERN OF MOLECULE 0772P

The 0772P molecule was identified in Examples 2 and 9 of this application. To evaluate the subcellular localization and specificity of antigen expression in various tissues, polyclonal antibodies were generated against 0772P. To produce these antibodies, O772P-1 (amino acids 44-772 of SEQ ID NO:312) and 0772P-2 (477-914 of SEQ ID NO:312) were expressed in an E. coli recombinant expression system and grown overnight at 37°C in LB Broth. The following day, 10ml of the overnight culture was added to 500ml of 2xYT containing the appropriate antibiotics. When the optical density of the cultures (560 nanometers) reached 0.4-0.6 the cells were induced with IPTG. Following induction, the cells were harvested, washed, lysed and run through a French Press at a pressure of 16000 psi. The cells were then centrifuged and the pellet checked by SDS-PAGE for the partitioning of the recombinant protein. For proteins that localize to the cell pellet, the pellet was resuspended in 10mM Tris, pH 8.0, 1% CHAPS and the inclusion body pellet washed and centrifuged. The washed inclusion body was solubilized with either 8M urea or 6M guanidine HCL containing 10mM Tris, pH 8.0, plus 10mM imidazole. The solubilized protein was then added to 5ml of nickel-chelate resin (Qiagen) and incubated for 45 minutes at room temperature.

Following the incubation, the resin and protein mixture was poured through a column and the flow through collected. The column was washed with 10-20 column volumes of buffer and the antigen eluted using 8M urea, 10mM Tris, pH 8.0, and 300 mM imidazole and collected in 3ml fractions. SDS-PAGE was run to determine which fractions to pool for further purification. As a final purification step, a strong anion exchange resin was equilibrated with the appropriate buffer and the pooled fractions were loaded onto the column. Each antigen was eluted from the column with an increasing salt gradient. Fractions were collected and analyzed by a SDS-PAGE to determine which fractions from the column to pool. The pooled fractions were dialyzed against 10mM Tris, pH 8.0, and the resulting protein was submitted for quality control for final release. The release criteria were: (a) purity as determined by SDS-PAGE or HPLC, (b) concentration as determined by Lowry assay or Amino Acid Analysis, (c) identity as determined by amino terminal protein, and (d) endotoxin levels as determined by the Limulus (LAL) assay. The proteins were then filtered through a 0.22µM filter and frozen until needed for immunizations.

To generate polyclonal antisera, 400µg of 0772P-1 or 0772P-2 was combined with 100µg of muramyldipeptide (MDP). The rabbits were immunized every 4 weeks with 100µg of antigen mixed with an equal volume of Incomplete Freund's Adjuvant (IFA). Seven days following each boost, the animals were bled and sera was generated by incubating the blood at 4°C for 12-24 hours followed by centrifugation.

To characterize the antisera, 96 well plates were coated with antigen followed by blocking with BSA. Rabbit sera was diluted in PBS and added to each well. The plates were then washed, and goat anti-rabbit horseradish peroxidase (HRP). The plates were again washed and TMB microwell Peroxidase Substrate was added. Following this incubation, the colormetric reaction was stopped and the plates read immediately at 450nm. All polyclonal antibodies showed immunoreactivity to the appropriate antigen.

Immunohistochemistry analysis of 0772P expression was performed on paraffin-embedded formalin fixed tissue. 0772P was found to be expressed in normal ovary and ovarian tumor, but not in normal heart, kidney, colon, lung or liver. Additionally, immunohistochemistry and flow cytometric analysis indicates that 0772P is a plasma membrane-associated molecule. 0772P contains 1 plasma transmembrane domain predicted to be encoded by amino acids 859-880. The N-terminus of 0772P is extracellular and is encoded by amino acids 1-859, while the C-terminus is intracellular. Sequence analysis shows that there are 17 potential N-linked glycosylation sites.

### EXAMPLE 11

### 0772P IS EXPRESSED ON THE SURFACE OF PRIMARY OVARIAN TUMOR CELLS

For recombinant expression in mammalian cells, the 0772P-21008 (SEQ ID NO:387) and 0772P full length cDNA (SEQ ID NO:311 encoding the protein of SEQ ID NO:312) were subcloned into mammalian expression vectors pBIB or pCEP4 respectively. These constructs were transfected into HEK293 cells using Fugene 6 (Roche). The HEK cells were then plated at a density of 100,000 cells/ml in DMEM containing fetal bovine serum (FBS) and grown overnight. The following day, 2µl of Fugene 6 was added to 100µl of DMEM, which contained no FBS, and incubated for 15 minutes at room temperature. The Fugene 6/DMEM mixture was then added to 1µg of 0772P/pBIB or 0772P/pCEP4 plasmid DNA and incubated for an additional 15 minutes at room temperature. The Fugene 6/DNA mix was then added to the HEK293 cells and incubated for 48-72 hours at 37°C with 7% CO₂. The cells were rinsed and pelleted by centrifugation.

For Western Blot analysis, whole cell lysates were generated by incubating the cells in lysis buffer followed by clarification by centrifugation. The samples were diluted and run on SDS-PAGE. The gel was then transferred to nitrocellulose and probed using purified anti-0772P-2 rabbit polyclonal antibody. The blot was revealed with a goat anti-rabbit Ig coupled to HRP followed by incubation in ECL substrate. Western Blot analysis revealed that 0772P-21008 could be detected in HEK293 cells that had been transfected with 0772P.

To determine the cell expression profile of 0772P in cells, primary ovarian tumor cells were grown in SCID mice. The cells were retrieved from the mice and analyzed by flow cytometry. Briefly, cells washed in cold staining buffer containing PBS, 1% BSA, and Na Azide. The cells were incubated for 30 minutes with 10µg/ml of purified anti-0772P-1 and 0772P-2 polyclonal sera. Following this incubation, the cells were washed three times in staining buffer and incubated with goat anti-rabbit Ig (H+L) conjugated to FITC (Southern Biotechnology). The cells were washed and resuspended in staining buffer containing Propidium Iodide (PI), a vital stain that identifies non-viable cells. The cells were then analyzed using Fluorescence Activated Cell Sorting (FACS). FACS analysis revealed that 0772P was present on the cells surface. Surface expression of O772P on tumor cells allows for immune targeting by therapeutic antibodies.

### EXAMPLE 12

### FUNCTIONAL CHARACTERIZATION OF ANTI-O8E MONOCLONAL ANTIBODIES

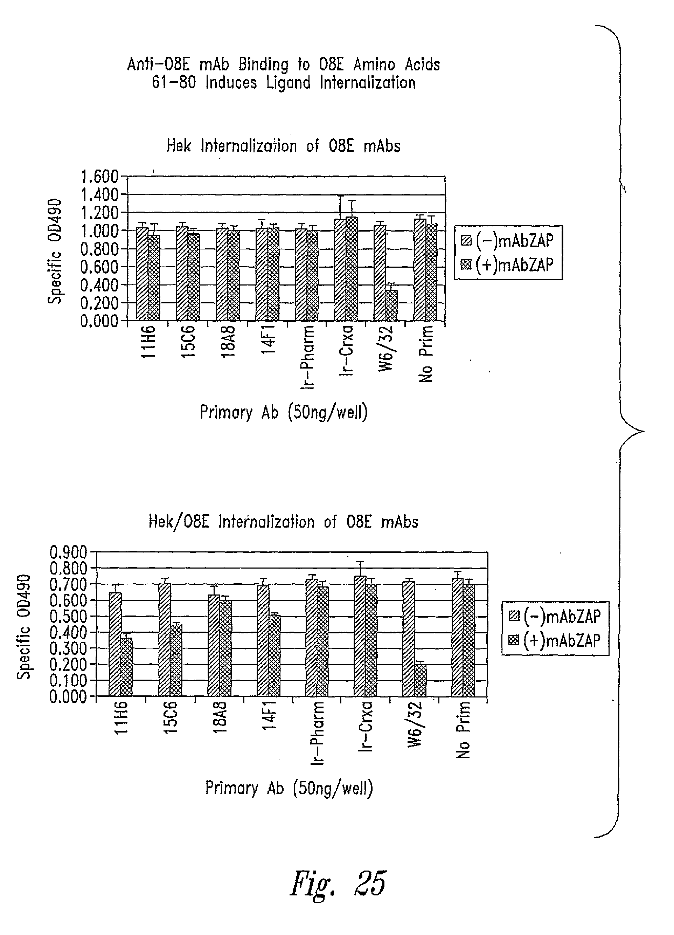

Mouse monoclonal antibodies (mAb) raised against E. coli derived O8E, as described in Example 8, were tested for their ability to promote 08E antigen internalization. Internalization of the antibody was determined using an in vitro cytotoxicity assay. Briefly, HEK293 and O8E/HEK transfected cells were plated into 96 well plates containing DME plus 10% heat-inactivated FBS in the presence of 50ng/well of purified anti-O8E or control antibodies. The isotype of the anti-O8E mAbs are as follows: 11A6-IgG1/kappa, 15C6-IgG2b/kappa, 18A8-IgG2b/kappa, and 14F1-IgG2a/kappa. W6/32 is a pan anti-human MHC class I mouse monoclonal antibody that serves as a positive control, and two irrelevant mAbs, Ir-Pharm and Ir-Crxa were included as negative controls. Following incubation with the O8E specific antibodies or the relevant controls antibodies, the mAb-zap, a goat anti-mouse Ig-saporin conjugated secondary antibody (Advanced Targeting Systems) was added at a concentration of 100ng/ml to half of the wells, and the plates were incubated for 48 to 72 hours at 37°C in a 7% CO₂ incubator. This assay takes advantage of the toxic nature of saporin, a ribozyme inactivating protein, which when internalized has a cytotoxic effect. Following incubation with the mAb-zap, internalization was quantitated by the addition of MTS reagent, followed by reading the OD490 of the plate on a microplate ELISA reader. Figure 25 depicts the results from these assays. The top panel represents HEK cells that have not been transfected with O8E and therefore 08E antibody should not bind and be internalized. Levels of proliferation were the same in all samples whether they were incubated with or without the mAb-zap, with the exception of the positive control Ab, W6/32. The lower panel represents cells that have been transfected with O8E and therefore should bind 08E specific antibodies. Antibodies from the hybridomas 11H6, 14F1, and 15C6, which recognize the amino acids 61-80 of 08E were able to promote internalization of the O8E surface protein as measured by decreased levels of proliferation due to the toxic nature of the mAb-zap (See Figure 25). The antibody generated by the hybridoma 18A8, which recognizes amino acids 151-170 of 08E, was unable to promote internalization as determined by normal levels of proliferation either in the absence or presence of the mAb-zap.

### EXAMPLE 13

### CHARACTERIZATION OF THE OVARIAN TUMOR ANTIGEN, O772P

The cDNA and protein sequences for multiple forms of the ovarian tumor antigen 0772P have been described in the above (e.g., Examples 2 and 9). A Genbank search indicated that 0772P has a high degree of similarity with FLJ14303 (Accession # AK024365; SEQ ID NO:457 and 463). Protein sequences corresponding to 0772P and FLJ14303 are disclosed in SEQ ID NO:478 and 479, respectively. FLJ14303 was identical to the majority of 0772P, with much of the 3'-end showing 100% homology. However, the 5'-end of FLJ14303 was found to extend further 5' than 0772P. In addition, FLJ14303 contained a 5 bp insert (SEQ ID NO:457) resulting in a frame shift of the amino-terminus protein sequence such that FLJ14303 utilizes a different starting methionine than 0772P and therefore encodes a different protein. This insertion was present in the genomic sequence and seen in all EST clones that showed identity to this region, suggesting that FLJ14303 (SEQ ID NO:457) represents a splice variant of 0772P, with an ORF that contains an extended and different amino-terminus. The additional 5'-nucleotide sequence included repeat sequences that were identified during the genomic mapping of O772P. The 5'-end of 0772P and the corresponding region of FLJ14303 showed between 90-100% homology. Taken together, this suggests that 0772P and FLJ14303 are different splice variants of the same gene, with different unique repeat sequences being spliced into the 5'-end of the gene.

The identification of an additional ten or more repeat sequences within the same region of chromosome 19, indicates that there may be many forms of 0772P, each with a different 5'-end, due to differential splicing of different repeat sequences. Northern blot analysis of 0772P demonstrated multiple O772P-hybridizing transcripts of different sizes, some in excess 10kb.

Upon further analysis, 13 additional 0772P-related sequences were identified, the cDNA and amino acid sequences of which are described in Table 2.

**Table 2**

| SEQ ID NO: | Description | Transmembrane Domains |
|---|---|---|
| 464 | LS #1043400.1 (cDNA) | nd |
| 465 | LS #1043400.10 (cDNA) | 0 |
| 466 | LS #1043400.11 (cDNA) | 2 |
| 467 | LS#1043400.12 (cDNA) | 2 |
| 468 | LS #1043400.2 (cDNA) | nd |
| 469 | LS #1043400.3 (cDNA) | |
| 470 | LS #1043400.5 (cDNA) | nd |
| 471 | LS #1043400.8 (cDNA) | 1 |
| 472 | LS #1043400.9 (cDNA) | 0 |
| 473 | LS#1043400.6 (cDNA) | nd |
| 474 | LS#1043400.7 (cDNA) | nd |
| 475 | LS #1043400.4 (cDNA) | nd |
| 476 | LS #1397610.1 (cDNA) | 0 |
| 477 | 1043400.10 Novel 5' (cDNA) | - |
| 480 | LS #1043400.9 (amino acid) | - |
| 481 | LS #1043400.8B (amino acid) Contains a transmembrane domain | - |
| 482 | LS#1043400.8A (amino acid) | - |
| 483 | LS #1043400.12 (amino acid) Contains a transmembrane domain | - |
| 484 | LS #1043400.11B (amino acid) Contains a transmembrane domain | - |
| 485 | LS #1043400.11A (amino acid) | - |
| 486 | LS #1043400.10 (amino acid) | - |
| 487 | LS #1043400.1 (amino acid) | - |

| | | |
|---|---|---|
| nd=not determined | | |

Initially it appeared that these sequences represented overlapping and/or discrete sequences of O772P splice forms that were capable of encoding polypeptides unique to the specific splice forms of 0772P. However, nucleotide alignment of these sequences failed to identify any identical regions within the repeat elements. This indicates that the sequences may represent different specific regions of a single 0772P gene, one that contains 16 or more repeat domains, all of which form a single linear transcript. The 5'-end of sequence LS #1043400.10 (Table 2; SEQ ID NO:465) is unique to both 0772P and FLJ14303 and contains no repeat elements, indicating that this sequence may represent the 5'-end of O772P.

Previously, transmembrane prediction analysis had indicated that 0772P contained between 1 and 3 transmembrane spanning domains. This was verified by the use of immunohistochemistry and flow cytometry, which demonstrated the existence of a plasma membrane-associated molecule representing 0772P. However, immunohistochemistry also indicated the presence of secreted form(s) of O772P, possibly resulting from an alternative splice form of 0772P or from a post-translational cleavage event. Analysis of several of the sequences presented in Table 2 showed that sequences 1043400B.12, 1043400.8B, and 1043400.11B all contained transmembrane regions, while 1043400.8A, 1043400.10, 1043400.1, 1043400.11A, and 1043400.9 were all lacking transmembrane sequences, suggesting that these proteins may be secreted.

Analysis indicates a part of 0772P is expressed and/or retained on the plasma membrane, making O772P an attractive target for directing specific immunotherapies, e.g., therapeutic antibodies, against this protein. The predicted extracellular domain of 0772P is disclosed in SEQ ID NO:489 and secretion of 0772P is likely to occur as a result of a cleavage event within the sequence:
SLVEQVFLD*K*TLNASFHWLGSTYQLVDIHVTEMESSVYQP.
Proteolytic cleavage is most likely to occur at the Lysine (K) at position 10 of SEQ ID NO:489. The extracellular, transmembrane, and cytoplasmic regions of 0772P are all disclosed in SEQ ID NO:488:
Extracellular: 
SLVEQVFLDKTLNASFHWLGSTYQLVDIHVTEMESSVYQPTSSSS
TQHFYLNFTITNLPYSQDKAQPGTTNYQRNKRNIEDALNQLFRNSSIKSYFSDCQ
VSTFRSVPNRHHTGVDSLCNFSPLARRVDRVAIYEEFLRMTRNGTQLQNFTLDR
SSVLVDGYFPNRNEPLTGNSDLPF
 Transmembrane:
WAVILIGLAGLLGLITCLICGVLVTT
Cytoplasmic:
RRRKKEGEYNVQQQCPGYYQSHLDLEDLQ

### EXAMPLE 14

### IMMUNOHISTOCHEMISTRY (IHC) ANALYSIS OF 08E EXPRESSION IN OVARIAN CANCER AND NORMAL TISSUES

In order to determine which tissues express the ovarian cancer antigen 08E, IHC analysis was performed on a diverse range of tissue sections using both polyclonal and monoclonal antibodies specific for O8E. The generation of O8E specific polyclonal antibodies is described in detail in Example 8. The Monoclonal antibodies used for staining were 11A6 and 14F1, both of which are specific for amino acids 61-80 of O8E and 18A8, which recognizes amino acids 151-170 of O8E (see Example 12 for details on generation).

To perform staining, tissue samples were fixed in formalin solution for 12-24 hours and embedded in paraffin before being sliced into 8 micron sections. Steam heat induced epitope retrieval (SHEIR) in 0.1M sodium citrate buffer (pH 6.0) was used for optimal staining conditions. Sections were incubated with 10% serum/PBS for 5 minutes. Primary antibody was then added to each section for 25 minutes followed by 25 minutes of incubation with either anti-rabbit or anti-mouse biotinylated antibody. Endogenous peroxidase activity was blocked by three 1.5 minute incubations with hydrogen peroxidase. The avidin biotin complex/horse radish peroxidase (ABC/HRP) system was used along with DAB chromogen to visualize the antigen expression. Slides were counterstained with hematoxylin to visualize the cell nuclei.

Results using rabbit affinity purified polyclonal antibody to O8E (a.a. 29-283; for details on the generation of this Ab, see Example 3) are presented in Table 3. Results using the three monoclonal antibodies are presented in Table 4.

**Table 3**

| Immunohistochemistry analysis of O8E using polyclonal antibodies | |
|---|---|
| **Tissue** | **O8E Expression** |
| Ovarian Cancer | Positive |
| Breast Cancer | Positive |
| Normal Ovary | Positive |
| Normal Breast | Positive |
| Blood Vessel | Positive |
| Kidney | Negative |
| Lung | Negative |
| Colon | Negative |
| Liver | Negative |
| Heart | Negative |

**Table 4**

| Immunohistochemistry analysis of O8E using monoclonal antibodies | | | | | | |
|---|---|---|---|---|---|---|
| Normal Tissue | 11A6 | | 18A8 | | 14F1 | |
| | Endothelial | Epithelial | Endothelial | Epithelial | Endothelial | Epithelial |
| Skin | 2 | 2 | 0 | 0 | 1 | 1 |
| Skin | 1 | 1 | 0 | 0 | 1 | 1 |
| Breast | 0 | 1 | n/a | n/a | 1 | 1 |
| Colon | 0 | 0 | 0 | 0 | 0 | 0 |
| Jejunum | 0 | 0 | 0 | 0 | 0 | 0 |
| Colon | 0 | 0 | 0 | 0 | 0 | 0 |
| Colon | 0 | 0 | 0 | 0 | 0 | 0 |
| Ovary | 0 | 0 | 0 | 0 | 1 | 0 |
| Colon | 0 | 0 | 0 | 0 | 0 | 1 |
| Liver | 0 | 0 | 0 | 0 | 1 | 2 |
| Skin | 0 | 0 | 0 | 0 | 1 | 0 |
| Duodenum and Pancreas | 0 | 0 | 0 | 0 | 0 | 0 |
| Appendix | 0 | 0 | 0 | 0 | 0 | 0 |
| Ileum | 0 | 0 | 0 | 0 | 0 | 0 |

| | | | | | | |
|---|---|---|---|---|---|---|
| 0=no staining, 1 =light staining, 2=moderate staining, n/a=not available | | | | | | |

### EXAMPLE 15

### EPITOPE MAPPING OF O772P POLYCLONAL ANTIBODIES

To perform epitope mapping of 0772P, peptides were generated, the sequences of which were derived from the sequence of 0772P. These peptides were 15 mers that overlapped by 5 amino acids and were generated via chemical synthesis on membrane supports. The peptides were covalently bound to Whatman 50 cellulose support by their C-terminus with the N-terminus unbound. In order to determine epitope specificity, the membranes were wet with 100% ethanol for 1 minute, and then blocked for 16 hours in TBS/Tween/Triton buffer (54mM Tris, 137 mM NaCl, 2.7 mM KCl, 0.5% BSA, 0.05% Tween 20, 0.05% Triton X-100, pH 7.5). The peptides were then probed with 2 0772P specific antibodies, O772P-1 (amino acids 44-772 of SEQ ID NO:312) and O772P-2 (477-914 of SEQ ID NO:312; see Example 10 for details of antibody generation), as well as irrelevant rabbit antibodies for controls. The antibodies were diluted to 1µg/ml and incubated with the membranes for 2 hours at room temperature. The membranes were then washed for 30 minutes in TBS/Tween/Triton buffer, prior to being incubated with a 1:10,000 dilution of HRP-conjugated anti-rabbit secondary antibody for 2 hours. The membranes were again washed for 30 minutes in TBS/Tween/Triton and anti-peptide reactivity was visualized using ECL. Specific epitope binding specificity for each of the O772P-polyclonal antibodies is described in Table 5.

**Table 5**

| **SEQ ID NO:** | **Peptide #** | **Anti-O772P1** | **Anti-O772P2** | **Peptide Sequence** |
|---|---|---|---|---|
| 490 | 2 | *** | - | TCGMRRTCSTLAPGS |
| 491 | 6 | * | */- | CRLTLLRPEKDGTAT |
| 492 | 7 | * | - | DGTATGVDAICTHHP |
| 493 | 8 | - | - | CTHHPDPKSPRLDRE |
| 494 | 9 | *** | *** | RLDREQLYWELSQLT |
| 495 | 11 | */- | - | LGPYALDNDSLFVNG |
| 496 | 13 | **** | - | SVSTTSTPGTPTYVL |
| 497 | 22 | - | - | LRPEKDGEATGVDAI |
| 498 | 24 | ** | */- | DPTGPGLDREQLYLE |
| 499 | 27 | */- | - | LDRDSLYVNGFTHRS |
| 500 | 40 | */- | - | GPYSLDKDSLYLNGY |
| 501 | 41 | - | - | YLNGYNEPGPDEPPT |
| 502 | 47 | *** | *** | ATFNSTEGVLQHLLR |
| 503 | 50 | - | *** | QLISLRPEKDGAATG |
| 504 | 51 | - | ** | GAATGVDTTCTYHPD |
| 505 | 52 | - | */- | TYHPDPVGPGLDIQQ |
| 506 | 53 | - | * | LDIQQLYWELSQLTH |
| 507 | 58 | - | * | HIVNWNLSNPDPTSS |
| 508 | 59 | - | * | DPTSSEYITLLRDIQ |
| 509 | 60 | - | * | LRDIQDKVTTLYKGS |
| 510 | 61 | - | *** | LYKGSQLHDTFRFCL |
| 511 | 71 | - | ** | DKAQPGTTNYQRNKR |

| | | | | |
|---|---|---|---|---|
| *= relative reactive level, -; no binding, ****; maximal binding | | | | |

### EXAMPLE 16

### IDENTIFICATION OF A NOVEL N-TERMINAL REPEAT STRUCTURE ASSOCIATED WITH 0772P

Various 0772P cDNA and protein forms have been identified and characterized as detailed above (e.g., Examples 1, 2, 9, and 14). Importantly, 0772P RNA and protein have been demonstrated to be over-expressed in ovarian cancer tissue relative to normal tissues and thus represents an attractive target for ovarian cancer diagnostic and therapeutic applications.

Using bioinformatic analysis of open reading frames (ORFs) from genomic nucleotide sequence identified previously as having homology with 0772P, multiple nucleotide repeat sequences were identified in the 5' region of the gene encoding the 0772P protein. A number of these repeat sequences were confirmed by RT-PCR using primers specific for the individual repeats. Fragments which contained multiple repeats were amplified from cDNA, thus confirming the presence of specific repeats and allowing an order of these repeats to be established.

Unexpectedly, when various sets of 0772P sequences derived from different database and laboratory sources were analyzed, at least 20 different repeat structures, each having substantial levels of identity with each other (see Table 6), were identified in the 5' region of the 0772P gene and the corresponding N-terminal region of the 0772P protein. Each repeat comprises a contiguous open reading frame encoding a polypeptide unit that is capable of being spliced to one or more other repeats such that concatomers of the repeats are formed in differing numbers and orders. Interestingly, other molecules have been described in the scientific literature that have repeating structural domains analogous to those described herein for 0772P. For example, the mucin family of proteins, which are the major glycoprotein component of the mucous which coats the surfaces of cells lining the respiratory, digestive and urogenital tracts, have been shown to be composed of tandemly repeated sequences that vary in number, length and amino acid sequence from one mucin to another (Perez-Vilar and Hill, J. Biol. Chem. 274(45):31751-31754,1999).

The various identified repeat structures set forth herein are expected to give rise to multiple forms of 0772P, most likely by alternative splicing. The cDNA sequences of the identified repeats are set forth in SEQ ID NOs:513-540, 542-546, and 548-567. The encoded amino acid sequences of the repeats are set forth in SEQ ID NOs:574-593. In many instances these amino acid sequences represent consensus sequences that were derived from the alignment of more than one experimentally derived sequence.

Each of these splice forms is capable of encoding a unique 0772P protein with multiple repeat domains attached to a constant carboxy terminal protein portion of 0772P that contains a trans membrane region. The cDNA sequence of the 0772P constant region is set forth in SEQ ID NO:568 and the encoded amino acid sequence is set forth in SEQ ID NO:594.

All of the available 0772P sequences that were obtained were broken down into their identifiable repeats and these sequences were compared using the Clustal method with weighted residue weight table (MegAlign software within DNASTAR sequence analysis package) to identify the relationship between the repeat sequences. Using this information, the ordering data provided by the RT-PCR, and sequence alignments (automatic and manual) using SeqMan (DNASTAR), one illustrative consensus full length 0772P contig was identified comprising 20 distinct repeat units. The cDNA for this 0772P cDNA contig is set forth in SEQ ID NO:569 and the encoded amino acid sequence is set forth in SEQ ID NO:595. This form of the O772P protein includes the following consensus repeat structures in the following order:
SEQ ID NO:572- SEQ ID NO:574- SEQ ID NO:575-SEQ ID NO:576-SEQ ID NO:577- SEQ ID NO:578- SEQ ID NO:579- SEQ ID NO:580- SEQ ID NO:581- SEQ ID NO:582- SEQ ID NO:583- SEQ ID NO:584- SEQ ID NO:585- SEQ ID NO:586- SEQ ID NO:587- SEQ ID NO:588- SEQ ID NO:589- SEQ ID NO:590-SEQ ID NO:591- SEQ ID NO:592- SEQ ID NO:593.
SEQ ID NO:595, therefore, represents one illustrative full-length consensus sequence for the 0772P protein. As discussed above, however, based on current knowledge of this protein and based upon scientific literature describing proteins containing analogous repeating structures, many other forms of 0772P are expected to exist with either more or less repeats. In addition, many forms of 0772P are expected to have differing arrangements, e.g., different orders, of these N-terminal repeat structures. The existence of multiple forms of 0772P having differing numbers of repeats is supported by Northern analysis of 0772P. In this study, Northern hybridization of a O772P-specific probe resulted in a smear of multiple O772P-hybridizing transcripts, some in excess 10kb.

Thus, the variable repeat region of the 0772 protein can be illustratively represented by the structure Xn - Y, wherein X comprises a repeat structure having at least 50% identity with the consensus repeat sequence set forth in SEQ ID NO:596; n is the number of repeats present in the protein and is expected to typically be a integer from 1 to about 35; Y comprise the 0772P constant region sequence set forth in SEQ ID NO:594 or sequences having at least 80% identity with SEQ ID NO:594. Each X present in the Xn repeat region of the 0772 molecule is different.

To determine the consensus sequences of each of the 20 repeat regions, sequences that were experimentally determined for a discrete repeat region were aligned and a consensus sequence determined. In addition to determining the consensus sequences for individual repeat regions, a consensus repeat sequence was also determined. This sequence was obtained by aligning the 20 individual consensus sequences. Variability of the repeats was determined by aligning the consensus amino acid sequences from each of the individual repeat regions with the over all repeat consensus sequence. Identity data is presented in Table 6.

**Table 6**

| Percent identities of Repeat Sequences with Reference to the Consensus Repeat Sequence | | |
|---|---|---|
| Repeat Number (amino acid) | SEQ ID NO: | Percent Identity to Consensus Repeat Sequence |
| 2 | 574 | 88 |
| 3 | 575 | 84 |
| 4 | 576 | 88 |
| 5 | 577 | 89 |
| 6 | 578 | 93 |
| 7 | 579 | 90 |
| 8 | 580 | 91 |
| 9 | 581 | 88 |
| 10 | 582 | 85 |
| 11 | 583 | 86 |
| 12 | 584 | 87 |
| 13 | 585 | 87 |
| 14 | 586 | 89 |
| 15 | 587 | 89 |
| 16 | 588 | 89 |
| 17 | 589 | 83 |
| 18 | 590 | 84 |
| 19 | 591 | 83 |
| 20 | 592 | 57 |
| 21 | 593 | 68 |

From the foregoing it will be appreciated that, although specific embodiments of the invention have been described herein for purposes of illustration, various modifications may be made without deviating from the spirit and scope of the invention. Accordingly, the invention is not limited except as by the appended claims.

The present invention will be further described by way of reference to the following clauses:
1. An 0772P polypeptide having the structure:
   Xₙ-Y
   wherein X comprises a sequence having at least 50% identity with the consensus O772P repeat sequence set forth in SEQ ID NO: 596;
   Y comprises a sequence having at least 80% identity with the O772P constant region sequence set forth in SEQ ID NO: 594;
   n is an integer from 1 to 35;
   wherein each X present in said polypeptide is different
2. The polypeptide of clause 1, wherein X comprises a sequence selected from the group consisting of any one of SEQ ID NOs: 574-593.
3. The polypeptide of clause 1, wherein Y comprises the sequence set forth in SEQ ID NO: 594.
4. The polypeptide of clause 1, wherein n is an integer from 15 to 25.
5. The polypeptide of clause 1, wherein n is 20.
6. The polypeptide of clause 1, wherein said polypeptide comprises SEQ ID NO: 595.
7. The polypeptide of clause 1, wherein said polypeptide is overexpressed in ovarian cancer cells compared with normal tissues.
8. An 0772P polypeptide having the structure:
   Xₙ-Y
   wherein X comprises an 0772P repeat sequence selected from the group consisting of any one of SEQ ID NOs: 574-593;
   Y comprises a sequence having at least 90% identity with the 0772P constant region sequence set forth in SEQ ID NO: 594;
   n is an integer from 15 to 25;
   wherein each X present in said polypeptide is different.
9. The polypeptide of clause 8, wherein n is 20.
10. The polypeptide of clause 8, wherein said polypeptide comprises SEQ ID NO: 595.
11. The polypeptide of clause 8, wherein said polypeptide is overexpressed in ovarian cancer cells compared with normal tissues.
12. An O772P polypeptide having the structure:
   Xₙ-Y
   wherein n is 20 and X comprises the following 0772P repeat sequences:
   SEQ ID NO: 574 - SEQ ID NO: 575 - SEQ ID NO: 576 - SEQ ID NO: 577 - SEQ ID NO: 578 - SEQ ID NO: 579 - SEQ ID NO: 580 - SEQ ID NO: 581 - SEQ ID NO: 582 - SEQ ID NO: 583 - SEQ ID NO: 584 - SEQ ID NO: 585 - SEQ ID NO: 586 - SEQ ID NO: 587 - SEQ ID NO: 588 - SEQ ID NO: 589 - SEQ ID NO: 590 - SEQ ID NO: 591 - SEQ ID NO: 592 - SEQ ID NO: 593; and
   Y comprises the sequence set forth in SEQ ID NO: 594.
13. The polypeptide of clause 12, wherein said polypeptide comprises SEQ ID NO: 595.
14. The polypeptide of clause 12, wherein said polypeptide is overexpressed in ovarian cancer cells compared with normal tissues.
15. An 0772P polynucleotide having the structure:
   Xₙ-Y
   wherein X comprises an 0772P repeat sequence selected from the group consisting of any one of SEQ ID NOs: 512-540, 542-546 and 548-567;
   Y comprises a sequence having at least 95% identity with the 0772P constant region sequence set forth in SEQ ID NO: 568;
   n is an integer from 1 to 35;
   wherein each X present in said polypeptide is different.
16. The polynucleotide of clause 15, wherein said polynucleotide comprises SEQ ID NO: 569.
17. The polynucleotide of clause 15, wherein n is from 15 to 25.
18. The polynucleotide of clause 15, wherein n is 20.
19. The polynucleotide of clause 15, wherein said polynucleotide is overexpressed in ovarian cancer cells compared with normal tissues.
20. An isolated polynucleotide comprising a sequence selected from the group consisting of:
   (a) sequences provided in SEQ ID NOs: 464-477 and 512-569;
   (b) complements of the sequences provided in SEQ ID NOs: 464-477 and 512-569;
   (c) sequences consisting of at least 20 contiguous residues of a sequence provided in SEQ ID NOs: 464-477 and 512-569;
   (d) sequences that hybridize to a sequence provided in SEQ ID NOs: 464-477 and 512-569, under highly stringent conditions;
   (e) sequences having at least 75% identity to a sequence of SEQ ID NOs: 464-477 and 512-569;
   (f) sequences having at least 90% identity to a sequence of SEQ ID NOs: 464-477 and 512-569; and
   (g) degenerate variants of a sequence provided in SEQ ID NOs: 464-477 and 512-569.
21. An isolated polypeptide comprising an amino acid sequence selected from the group consisting of:
   (a) sequences encoded by a polynucleotide of clause 20 and
   (b) sequences having at least 80% identity to a sequence encoded by a polynucleotide of clause 20; and
   (c) sequences having at least 90% identity to a sequence encoded by a polynucleotide of clause 20.
22. An expression vector comprising a polynucleotide of clause 20 operably linked to an expression control sequences.
23. A host cell transformed or transected with all expression vector according to clause 22.
24. An isolated antibody, or antigen-binding fragment thereof, that specifically binds to a polypeptide of clause 21.
25. A method for detecting the presence of a cancer in a patient, comprising the steps of:
   (a) obtaining a biological sample from the patient;
   (b) contacting the biological sample with a binding agent that binds to a polypeptide of clause 21 ;
   (c) detecting in the sample an amount of polypeptide that binds to the binding agent; and
   (d) comparing the amount of polypeptide to a predetermined cut-off value and therefrom determining the presence of a cancer in the patient.
26. A fusion protein comprising at least one polypeptide according to clause 21.
27. A method for stimulating and/or expanding T cells specific for a tumor protein, comprising contacting T cells with at least one component selected from the group consisting of:
   (a) polypeptides according to clause 21;
   (b) polynucleotides according to clause 20; and
   (c) antigen-presenting cells that express a polynucleotide according to clause 20,
   under conditions and for a time sufficient to permit the stimulation and/or expansion of T cells.
28. An isolated T cell population, comprising T cells prepared according to the method of clause 27.
29. A composition comprising a first component selected from the group consisting of physiologically acceptable carriers and immunostimulants, and a second component selected from the group consisting of:
   (a) polypeptides according to clause 21;
   (b) polynucleotides according to clause 20;
   (c) antibodies according to clause 24;
   (d) fusion proteins according to clause 26;
   (e) T cell populations according to clause 28; and
   (f) antigen presenting cells that express a polypeptide according to clause 21.
30. A method for stimulating an immune response in a patient, comprising administering to the patient a composition of clause 29.
31. A method for the treatment of a ovarian cancer in a patient, comprising administering to the patient a composition of clause 29.
32. A method for determining the presence of a cancer in a patient, comprising the steps of:
   (a) obtaining a biological sample from the patient;
   (b) contacting the biological sample with an oligonucleotide that hybridizes to a polynucelotide sequence according to clause 21. under moderately stringent conditions;
   (c) detecting in the sample an amount of said polynucleotide that hybridizes to the oligonucleotide; and
   (d) comparing the amount of said polynucleotide that hybridizes to the oligonucleotide to a predetermined cut-off value, and therefrom determining the presence of the cancer in the patient.
33. An 0772 polypeptide comprising at least an antibody epitope sequence set forth in any one of SEQ ID NOs: 490-511.
34. An O8E polypeptide comprising at least an antibody epitope sequence set forth in any one of SEQ ID NOs: 394-415.
35. An isolated antibody, or antigen-binding fragment thereof, that specifically binds to a polypeptide of clause 1.

## Claims

1. An isolated antibody, or antigen binding fragment thereof, which specifically binds to an 08E polypeptide at an amino acid sequence selected from the group consisting of SEQ ID NOs: 396-397, 399-400, 403-406, 409 and 413-415.

2. The isolated antibody or antigen binding fragment of claim 1, wherein the antibody or antigen binding fragment is conjugated to a toxin.

3. The isolated antibody, or antigen binding fragment of claim 2, wherein the toxin is selected from the group consisting of a ricin toxin, abrin toxin, diptheria toxin, cholera toxin, gelonin toxin, Pseudomonas exotoxin, Shigella toxin, and pokeweed antiviral protein.

4. The isolated antibody, or antigen binding fragment of claim 1, wherein the antibody or antigen binding fragment is conjugated to a radionuclide.

5. The isolated antibody, or antigen binding fragment of claim 4, wherein the radionuclide is selected from the group consisting of ⁹⁰Y, ¹²³I_{,} ¹²⁵I, ¹³¹I_{,} ¹⁸⁶Re, ¹⁸⁸Re, ²¹¹At and ²¹²Bi_{.}

6. The isolated antibody, or antigen binding fragment of claim 1, wherein the antibody or antigen binding fragment binds to a polypeptide encoded by SEQ ID NO: 391, and wherein said polypeptide is expressed on the cell surface of a ovarian tumor cell.

7. The isolated antibody, or antigen binding fragment of claim 1, wherein the antibody or antigen binding fragment induces internalization in an *in vitro* cytotoxicity assay upon binding a cell surface-expressed polypeptide encoded by SEQ ID NO: 391.

8. The isolated antibody, or antigen binding fragment as in any one of claims 1-7, wherein the antibody or antigen binding fragment is a human isolated antibody, or antigen binding fragment.

9. A pharmaceutical composition comprising at least one antibody or antigen binding fragment as in any one of claims 1-7, and a physiologically acceptable carrier, for use in treating ovarian cancer.

10. A diagnostic kit comprising at least one antibody or antigen binding fragment as in any one of claims 1-7, and a detection reagent, for use in diagnosing ovarian cancer.

11. The diagnostic kit of claim 10, wherein the detection reagent comprises a reporter group.

12. A pharmaceutical composition comprising a sufficient amount of an isolated antibody, or antigen binding fragment as in any one of claims 1-7, for killing or inhibiting the growth of a tumor cell which expresses a polypeptide encoded by SEQ ID NO: 391.

13. A pharmaceutical composition comprising a therapeutically effective amount of an isolated antibody, or antigen binding fragment as in any one of claims 1-7, for treating ovarian cancer in a patient, wherein said cancer expresses a polypeptide encoded by SEQ ID NO: 391.

14. A method for making an isolated antibody, comprising immunizing a mouse with a peptide having an amino acid sequence selected from any one of SEQ ID NOs: 396-397, 399-400, 403-406, 409, and 413-415.

15. An isolated polypeptide consisting of an amino acid sequence selected from the group consisting of: SEQ ID NOs: 396-397, 399-400, 403-406, 409, and 413-415.

16. An isolated antibody, or antigen binding fragment thereof that specifically binds a polypeptide of claim 15.

17. An isolated polynucleotide sequence encoding a polypeptide of claim 15.

18. A pharmaceutical composition comprising,
(a) a polypeptide of claim 15, or
(b) an isolated antibody or antigen binding fragment as in any one of claims 1-7; or
(c) a polynucleotide of claim 17; and
a physiologically acceptable carrier, for use in treating ovarian cancer.

19. A vaccine comprising,
(a) a polypeptide of claim 15, or
(b) an isolated antibody or antigen binding fragment as in any one of claims 1-7; or
(c) a polynucleotide of claim 17; and
a non-specific immune response enhancer, for use in preventing ovarian cancer.


SEQUENCE LISTING

<110> Corixa Corporation
 Mitcham, Jennifer L.
 King, Gordon E.
 Algate, Paul A.
 Fling, Steven P.
 Retter, Marc W.
 Fanger, Gary Richard
 Reed, Steven G.
 Vedvick, Thomas S.
 Carter, Darrick
 Hill, Paul
 Albone, Earl

<120> COMPOSITIONS AND METHODS FOR THE THERAPY
 AND DIAGNOSIS OF OVARIAN CANCER

<130> 210121.46201PC

<140> PCT
<141> 2001-07-17

<160> 596

<170> FastSEQ for Windows Version 4.0

<210> 1
<211> 461
<212> DNA
<213> Homo sapiens

<400> 1
ttagagaggc acagaaggaa gaagagttaa aagcagcaaa gccgggtttt tttgttttgt 60
tttgttttgt tttgttttga gatggagtct cactctgttg cccaagctgg agtacaacgg 120
catgatctca gctcgctgca acctccgcct cccacgttca agtgattctc ctgcctcagc 180
ctcccaagta gctgggatta caggcgcccg ccaccacgct cagctaattt tttttgtatt 240
tttagtagag acagggtttc accaggttgg ccaggctgct cttgaactcc tgacctcagg 300
tgatccaccc gcctcggcct cccaaagtgc tgggattaca ggcgtgagcc accacgcccg 360
gcccccaaag ctgtttcttt tgtctttagc gtaaagctct cctgccatgc agtatctaca 420
taactgacgt gactgccagc aagctcagtc actccgtggt c 461

<210> 2
<211> 540
<212> DNA
<213> Homo sapiens

<400> 2
taggatgtgt tggaccctct gtgtcaaaaa aaacctcaca aagaatcccc tgctcattac 60
agaagaagat gcatttaaaa tatgggttat tttcaacttt ttatctgagg acaagtatcc 120

attaattatt gtgtcagaag agattgaata cctgcttaag aagcttacag aagctatggg 180
aggaggttgg cagcaagaac aatttgaaca ttataaaatc aactttgatg acagtaaaaa 240
tggcctttct gcatgggaac ttattgagct tattggaaat ggacagttta gcaaaggcat 300
ggaccggcag actgtgtcta tggcaattaa tgaagtcttt aatgaactta tattagatgt 360
gttaaagcag ggttacatga tgaaaaaggg ccacagacgg aaaaactgga ctgaaagatg 420
gtttgtacta aaacccaaca taatttctta ctatgtgagt gaggatctga aggataagaa 480
aggagacatt ctcttggatg aaaattgctg tgtagagtcc ttgcctgaca aagatggaaa 540

<210> 3
<211> 461
<212> DNA
<213> Homo sapiens

<400> 3
ttagagaggc acagaaggaa gaagagttaa aagcagcaaa gccgggtttt tttgttttgt 60
tttgttttgt tttgttttga gatggagtct cactctgttg cccaagctgg agtacaacgg 120
catgatctca gctcgctgca acctccgcct cccacgttca agtgattctc ctgcctcagc 180
ctcccaagta gctgggatta caggcgcccg ccaccacgct cagctaattt tttttgtatt 240
tttagtagag acagggtttc accaggttgg ccaggctgct cttgaactcc tgacctcagg 300
tgatccaccc gcctcggcct cccaaagtgc tgggattaca ggcgtgagcc accacgcccg 360
gcccccaaag ctgtttcttt tgtctttagc gtaaagctct cctgccatgc agtatctaca 420
taactgacgt gactgccagc aagctcagtc actccgtggt c 461

<210> 4
<211> 531
<212> DNA
<213> Homo sapiens

<220>
<221> misc_feature
<222> 454, 492, 526
<223> n = A,T,C or G

<400> 4
tctttttctt tcgatttcct tcaatttgtc acgtttgatt ttatgaagtt gttcaagggc 60
taactgctgt gtattatagc tttctctgag ttccttcagc tgattgttaa atgaatccat 120
ttctgagagc ttagatgcag tttctttttc aagagcatct aattgttctt taagtctttg 180
gcataattct tccttttctg atgacttttt atgaagtaaa ctgatccctg aatcaggtgt 240
gttactgagc tgcatgtttt taattctttc gtttaatagc tgcttctcag ggaccagata 300
gataagctta ttttgatatt ccttaagctc ttgttgaagt tgtttgattt ccataatttc 360
caggtcacac tgtttatcca aaacttctag ctcagtcttt tgtgtttgct ttctgatttg 420
gacatcttgt agtctgcctg agatctgctg atgntttcca ttcactgctt ccagttccag 480
gtggagactt tnctttctgg agctcagcct gacaatgcct tcttgntccc t 531

<210> 5
<211> 531
<212> DNA
<213> Homo sapiens

<400> 5
agccagatgg ctgagagctg caagaagaag tcaggatcat gatggctcag tttcccacag 60
cgatgaatgg agggccaaat atgtgggcta ttacatctga agaacgtact aagcatgata 120
aacagtttga taacctcaaa ccttcaggag gttacataac aggtgatcaa gcccgtactt 180
ttttcctaca gtcaggtctg ccggccccgg ttttagctga aatatggccc ttatcagatc 240
tgaacaagga tgggaagatg gaccagcaag agttctctat agctatgaaa ctcatcaagt 300
taaagttgca gggccaacag ctgcctgtag tcctccctcc tatcatgaaa caacccccta 360

tgttctctcc actaatctct gctcgttttg ggatgggaag catgcccaat ctgtccattc 420
atcagccatt gcctccagtt gcacctatag caacaccctt gtcttctgct acttcaggga 480
ccagtattcc tcccctaatg atgcctgctc ccctagtgcc ttctgttagt a 531

<210> 6
<211> 531
<212> DNA
<213> Homo sapiens

<400> 6
aatagattta atgcagagtg tcaacttcaa ttgattgata gtggctgcct agagtgctgt 60
gttgagtagg tttctgagga tgcaccctgg cttgaagaga aagactggca ggattaacaa 120
tatctaaaat ctcacttgta ggagaaacca caggcaccag agctgccact ggtgctggca 180
ccagctccac caaggccagc gaagagccca aatgtgagag tggcggtcag gctggcacca 240
gcactgaagc caccactggt gctggcactg gcactggcac tgttattggt actggtactg 300
gcaccagtgc tggcactgcc actctcttgg gctttggctt tagcttctgc tcccgcctgg 360
atccgggctt tggcccaggg tccgatatca gcttcgtccc agttgcaggg cccggcagca 420
ttctccgagc cgagcccaat gcccattcga gctctaatct cggccctagc cttggcttca 480
gctgcagcct cagctgcagc cttcaaatcc gcttccatcg cctctcggta c 531

<210> 7
<211> 531
<212> DNA
<213> Homo sapiens

<400> 7
gccaagaaag cccgaaaggt gaagcatctg gatggggaag aggatggcag cagtgatcag 60
agtcaggctt ctggaaccac aggtggccga agggtctcaa aggccctaat ggcctcaatg 120
gcccgcaggg cttcaagggg tcccatagcc ttttgggccc gcagggcatc aaggactcgg 180
ttggctgctt gggcccggag agccttgctc tccctgagat cacctaaagc ccgtaggggc 240
aaggctcgcc gtagagctgc caagctccag tcatcccaag agcctgaagc accaccacct 300
cgggatgtgg cccttttgca agggagggca aatgatttgg tgaagtacct tttggctaaa 360
gaccagacga agattcccat caagcgctcg gacatgctga aggacatcat caaagaatac 420
actgatgtgt accccgaaat cattgaacga gcaggctatt ccttggagaa ggtatttggg 480
attcaattga aggaaattga taagaatgac cacttgtaca ttcttctcag c 531

<210> 8
<211> 531
<212> DNA
<213> Homo sapiens

<220>
<221> misc_feature
<222> 481
<223> n = A,T,C or G

<400> 8
gaggtctcac tatgttgccc aggctgttct tgaactcctg ggatcaagca atccacccat 60
gttggtctcc aaaagtgctg ggatcatagg cgtgagccac ctcacccagc caccaatttt 120
caatcaggaa gactttttcc ttcttcaaga agtgaagggt ttccagagta tagctacact 180
attgcttgcc tgagggtgac tacaaaattg cttgctaaaa ggttaggatg ggtaaagaat 240
tagattttct gaatgcaaaa ataaaatgtg aactaatgaa ctttaggtaa tacatattca 300
taaaataatt attcacatat ttcctgattt atcacagaaa taatgtatga aatgctttga 360
gtttcttgga gtaaactcca ttactcatcc caagaaacca tattataagt atcactgata 420
ataagaacaa caggaccttg tcataaattc tggataagag aaatagtctc tgggtgtttg 480
ntcttaattg ataaaattta cttgtccatc ttttagttca gaatcacaaa a 531

<210> 9
<211> 531
<212> DNA
<213> Homo sapiens

<220>
<221> misc_feature
<222> 528
<223> n = A,T,C or G

<400> 9
aagcggaaat gagaaaggag ggaaaatcat gtggtattga gcggaaaact gctggatgac 60
agggctcagt cctgttggag aactctgggt ggtgctgtag aacagggcca ctcacagtgg 120
ggtgcacaga ccagcacggc tctgtgacct gtttgttaca ggtccatgat gaggtaaaca 180
atacactgag tataagggtt ggtttagaaa ctcttacagc aatttgacaa agtaatcttc 240
tgtgcagtga atctaagaaa aaaattgggg ctgtatttgt atgttccttt ttttcatttc 300
atgttctgag ttacctattt ttattgcatt ttacaaaagc atccttccat gaaggaccgg 360
aagttaaaaa caaagcaggt cctttatcac agcactgtcg tagaacacag ttcagagtta 420
tccacccaag gagccaggga gctgggctaa accaaagaat tttgcttttg gttaatcatc 480
aggtacttga gttggaattg ttttaatccc atcattacca ggctggangt g 531

<210> 10
<211> 861
<212> DNA
<213> Homo sapiens

<400> 10
ccgcggctcc tgtccagacc ctgaccctcc ctcccaaggc tcaaccgtcc cccaacaacc 60
gccagccttg tactgatgtc ggctgcgaga gcctgtgctt aagtaagaat caggccttat 120
tggagacatt caagcaaagg ttggacaact acttttccag aacagaaagg aaactcatgc 180
atcagaaaag gtgactaata aaggtaccag aagaatatgg ctgcacaaat accagaatct 240
gatcagataa aacagtttaa ggaatttctg gggacctaca ataaacttac agagacctgc 300
tttttggact gtgttagaga cttcacaaca agagaagtaa aacctgaaga gaccacctgt 360
tcagaacatt gcttacagaa atatttaaaa atgacacaaa gaatatccat gagatttcag 420
gaatatcata ttcagcagaa tgaagccctg gcagccaaag caggactcct tggccaacca 480
cgatagagaa gtcctgatgg atgaactttt gatgaaagat tgccaacagc tgctttattg 540
gaaatgagga ctcatctgat agaatcccct gaaagcagta gccaccatgt tcaaccatct 600
gtcatgactg tttggcaaat ggaaaccgct ggagaaacaa aattgctatt taccaggaat 660
aatcacaata gaaggtctta ttgttcagtg aaataataag atgcaacatt tgttgaggcc 720
ttatgattca gcagcttggt cacttgatta gaaaaataaa ccattgtttc ttcaattgtg 780
actgttaatt ttaaagcaac ttatgtgttc gatcatgtat gagatagaaa aatttttatt 840
actcaaagta aaataaatgg a 861

<210> 11
<211> 541
<212> DNA
<213> Homo sapiens

<400> 11
gaaaaaaaat ataaaacaca cttttgcgaa aacggtggcc ctaaaagagg aaaagaattt 60
caccaatata aatccaattt tatgaaaact gacaatttaa tccaagaatc acttttgtaa 120
atgaagctag caagtgatga tatgataaaa taaacgtgga ggaaataaaa acacaagact 180
tggcataaga tatatccact tttgatatta aacttgtgaa gcatattctt cgacaaattg 240
tgaaagcgtt cctgatcttg cttgttctcc atttcaaata aggaggcata tcacatccca 300
agagtaacag aaaaagaaaa aagacatttt tgcattttga gatgaaccaa agacacaaaa 360
caaaacgaac aaagtgtcat gtctaattct agcctctgaa ataaaccttg aacatctcct 420
acaaggcacc gtgatttttg taattctaac ctgaagaaat gtgatgactt ttgtggacat 480

gaaaatcaga tgagaaaact gtggtctttc caaagcctga actcccctga aaacctttgc 540
a 541

<210> 12
<211> 541
<212> DNA
<213> Homo sapiens

<400> 12
ctgggatcat ttctcttgat gtcataaaag actcttcttc ttcctcttca tcctcttctt 60
catcctcttc tgtacagtgc tgccgggtac aacggctatc tttgtcttta tcctgagatg 120
aagatgatgc ttctgtttct cctaccataa ctgaagaaat ttcgctggaa gtcgtttgac 180
tggctgtttc tctgacttca ccttctttgt caaacctgag tctttttacc tcatgcccct 240
cagcttccac agcatcttca tctggatgtt tatttttcaa agggctcact gaggaaactt 300
ctgattcaga ggtcgaagag tcactgtgat ttttctcctc attttgctgc aaatttgcct 360
ctttgctgtc tgtgctctca ggcaacccat ttgttgtcat gggggctgac aaagaaacct 420
ttggtcgatt aagtggcctg ggtgtcccag gcccatttat attagacctc tcagtatagc 480
ttggtgaatt tccaggaaac ataacaccat tcattcgatt taaactattg gaattggttt 540
t 541

<210> 13
<211> 441
<212> DNA
<213> Homo sapiens

<400> 13
gagggttggt ggtagcggct tggggaggtg ctcgctctgt cggtcttgct ctctcgcacg 60
cttcccccgg ctcccttcgt ttcccccccc cggtcgcctg cgtgccggag tgtgtgcgag 120
ggagggggag ggcgtcgggg gggtgggggg aggcgttccg gtccccaaga gacccgcgga 180
gggaggcgga ggctgtgagg gactccggga agccatggac gtcgagaggc tccaggaggc 240
gctgaaagat tttgagaaga gggggaaaaa ggaagtttgt cctgtcctgg atcagtttct 300
ttgtcatgta gccaagactg gagaaacaat gattcagtgg tcccaattta aaggctattt 360
tattttcaaa ctggagaaag tgatggatga tttcagaact tcagctcctg agccaagagg 420
tcctcccaac cctaatgtcg a 441

<210> 14
<211> 131
<212> DNA
<213> Homo sapiens

<220>
<221> misc_feature
<222> 126
<223> n = A,T,C or G

<400> 14
aagcaggcgg ctcccgcgct cgcagggccg tgccacctgc ccgcccgccc gctcgctcgc 60
tcgcccgccg cgccgcgctg ccgaccgcca gcatgctgcc gagagtgggc tgccccgcgc 120
tgccgntgcc g 131

<210> 15
<211> 692
<212> DNA
<213> Homo sapiens

<400> 15
atctcttgta tgccaaatat ttaatataaa tctttgaaac aagttcagat gaaataaaaa 60

tcaaagtttg caaaaacgtg aagattaact taattgtcaa atattcctca ttgccccaaa 120
tcagtatttt ttttatttct atgcaaaagt atgccttcaa actgcttaaa tgatatatga 180
tatgatacac aaaccagttt tcaaatagta aagccagtca tcttgcaatt gtaagaaata 240
ggtaaaagat tataagacac cttacacaca cacacacaca cacacacgtg tgcacgccaa 300
tgacaaaaaa caatttggcc tctcctaaaa taagaacatg aagaccctta attgctgcca 360
ggagggaaca ctgtgtcacc cctccctaca atccaggtag tttcctttaa tccaatagca 420
aatctgggca tatttgagag gagtgattct gacagccacg ttgaaatcct gtggggaacc 480
attcatgtcc acccactggt gccctgaaaa aatgccaata atttttcgct cccacttctg 540
ctgctgtctc ttccacatcc tcacatagac cccagacccg ctggcccctg gctgggcatc 600
gcattgctgg tagagcaagt cataggtctc gtctttgacg tcacagaagc gatacaccaa 660
attgcctggt cggtcattgt cataaccaga ga 692

<210> 16
<211> 728
<212> DNA
<213> Homo sapiens

<400> 16
cagacggggt ttcactatgt tggctaggct ggtcttgaac tcctgacttc aggtgatctg 60
cctgccttgg cctcccaaag tgctgggatt acaggcataa gccactgcgc ccggctgatc 120
tgatggtttc ataaggcttt tccccctttt gctcagcact tctccttcct gccgccatgt 180
gaagaaggac atgtttgctt ccccttccac cacgattgta agttgtttcc tgaggcctcc 240
ccggccatgc tgaactgtga gtcaattaaa cctctttcct ttataaatta tccagttttg 300
ggtatgtctt tattagtaga atgagaacag actaatacaa cccttaaagg agactgacgg 360
agaggattct tcctggatcc cagcacttcc tctgaatgct actgacattc ttcttgagga 420
ctttaaactg ggagatagaa aacagattcc atggctcagc agcctgagag cagggaggga 480
gccaagctat agatgacatg ggcagcctcc cctgaggcca ggtgtggccg aacctgggca 540
gtgctgccac ccaccccacc agggccaagt cctgtccttg gagagccaag cctcaatcac 600
tgctagcctc aagtgtcccc aagccacagt ggctaggggg actcagggaa cagttcccag 660
tctgccctac ttctcttacc tttacccctc atacctccaa agtagaccat gttcatgagg 720
tccaaagg 728

<210> 17
<211> 531
<212> DNA
<213> Homo sapiens

<220>
<221> misc_feature
<222> 518, 528
<223> n = A,T,C or G

<400> 17
aagcgaggaa gccactgcgg ctcctggctg aaaagcggcg ccaggctcgg gaacagaggg 60
aacgcgaaga acaggagcgg aagctgcagg ctgaaaggga caagcgaatg cgagaggagc 120
agctggcccg ggaggctgaa gcccgggctg aacgtgaggc cgaggcgcgg agacgggagg 180
agcaggaggc tcgagagaag gcgcaggctg agcaggagga gcaggagcga ctgcagaagc 240
agaaagagga agccgaagcc cggtcccggg aagaagctga gcgccagcgc caggagcggg 300
aaaagcactt tcagaaggag gaacaggaga gacaagagcg aagaaagcgg ctggaggaga 360
taatgaagag gactcggaaa tcagaagccg ccgaaaccaa gaagcaggat gcaaaggaga 420
ccgcagctaa caattccggc ccagaccctt gtgaaagctg tagagactcg gccctctggg 480
cttccagaaa ggattctatt gcagaaagga aggagctngg ccccccangg a 531

<210> 18
<211> 1041
<212> DNA
<213> Homo sapiens

<220>
<221> misc_feature
<222> 544
<223> n = A,T,C or G

<400> 18
ctctgtggaa aactgatgag gaatgaattt accattaccc atgttctcat ccccaagcaa 60
agtgctgggt ctgattactg caacacagag aacgaagaag aacttttcct catacaggat 120
cagcagggcc tcatcacact gggctggatt catactcacc ccacacagac cgcgtttctc 180
tccagtgtcg acctacacac tcactgctct taccagatga tgttgccaga gtcagtagcc 240
attgtttgct cccccaagtt ccaggaaact ggattcttta aactaactga ccatggacta 300
gaggagattt cttcctgtcg ccagaaagga tttcatccac acagcaagga tccacctctg 360
ttctgtagct gcagccacgt gactgttgtg gacagagcag tgaccatcac agaccttcga 420
tgagcgtttg agtccaacac cttccaagaa caacaaaacc atatcagtgt actgtagccc 480
cttaatttaa gctttctaga aagctttgga agtttttgta gatagtagaa aggggggcat 540
cacntgagaa agagctgatt ttgtatttca ggtttgaaaa gaaataactg aacatatttt 600
ttaggcaagt cagaaagaga acatggtcac ccaaaagcaa ctgtaactca gaaattaagt 660
tactcagaaa ttaagtagct cagaaattaa gaaagaatgg tataatgaac ccccatatac 720
ccttccttct ggattcacca attgttaaca tttttttcct ctcagctatc cttctaattt 780
ctctctaatt tcaatttgtt tatatttacc tctgggctca ataagggcat ctgtgcagaa 840
atttggaagc catttagaaa atcttttgga ttttcctgtg gtttatggca atatgaatgg 900
agcttattac tggggtgagg gacagcttac tccatttgac cagattgttt ggctaacaca 960
tcccgaagaa tgattttgtc aggaattatt gttatttaat aaatatttca ggatattttt 1020
cctctacaat aaagtaacaa t 1041

<210> 19
<211> 1043
<212> DNA
<213> Homo sapiens

<400> 19
ctctgtggaa aactgatgag gaatgaattt accattaccc atgttctcat ccccaagcaa 60
agtgctgggt ctgattactg caacacagag aacgaagaag aacttttcct catacaggat 120
cagcagggcc tcatcacact gggctggatt catactcacc ccacacagac cgcgtttctc 180
tccagtgtcg acctacacac tcactgctct taccagatga tgttgccaga gtcagtagcc 240
attgtttgct cccccaagtt ccaggaaact ggattcttta aactaactga ccatggacta 300
gaggagattt cttcctgtcg ccagaaagga tttcatccac acagcaagga tccacctctg 360
ttctgtagct gcagccacgt gactgttgtg gacagagcag tgaccatcac agaccttcga 420
tgagcgtttg agtccaacac cttccaagaa caacaaaacc atatcagtgt actgtagccc 480
cttaatttaa gctttctaga aagctttgga agtttttgta gatagtagaa aggggggcat 540
cacctgagaa agagctgatt ttgtatttca ggtttgaaaa gaaataactg aacatatttt 600
ttaggcaagt cagaaagaga acatggtcac ccaaaagcaa ctgtaactca gaaattaagt 660
tactcagaaa ttaagtagct cagaaattaa gaaagaatgg tataatgaac ccccatatac 720
ccttccttct ggattcacca attgttaaca tttttttcct ctcagctatc cttctaattt 780
ctctctaatt tcaatttgtt tatatttacc tctgggctca ataagggcat ctgtgcagaa 840
atttggaagc catttagaaa atcttttgga ttttcctgtg gtttatggca atatgaatgg 900
agcttattac tggggtgagg gacagcttac tccatttgac cagattgttt ggctaacaca 960
tcccgaagaa tgattttgtc aggaattatt gttatttaat aaatatttca ggatattttt 1020
cctctacaat aaagtaacaa tta 1043

<210> 20
<211> 448
<212> DNA
<213> Homo sapiens

<400> 20

ggacgacaag gccatggcga tatcggatcc gaattcaagc ctttggaatt aaataaacct 60
ggaacaggga aggtgaaagt tggagtgaga tgtcttccat atctatacct ttgtgcacag 120
ttgaatggga actgtttggg tttagggcat cttagagttg attgatggaa aaagcagaca 180
ggaactggtg ggaggtcaag tggggaagtt ggtgaatgtg gaataactta cctttgtgct 240
ccacttaaac cagatgtgtt gcagctttcc tgacatgcaa ggatctactt taattccaca 300
ctctcattaa taaattgaat aaaagggaat gttttggcac ctgatataat ctgccaggct 360
atgtgacagt aggaaggaat ggtttcccct aacaagccca atgcactggt ctgactttat 420
aaattattta ataaaatgaa ctattatc 448

<210> 21
<211> 411
<212> DNA
<213> Homo sapiens

<400> 21
ggcagtgaca ttcaccatca tgggaaccac cttccctttt cttcaggatt ctctgtagtg 60
gaagagagca cccagtgttg ggctgaaaac atctgaaagt agggagaaga acctaaaata 120
atcagtatct cagagggctc taaggtgcca agaagtctca ctggacattt aagtgccaac 180
aaaggcatac tttcggaatc gccaagtcaa aactttctaa cttctgtctc tctcagagac 240
aagtgagact caagagtcta ctgctttagt ggcaactaca gaaaactggt gttacccaga 300
aaaacaggag caattagaaa tggttccaat atttcaaagc tccgcaaaca ggatgtgctt 360
tcctttgccc atttagggtt tcttctcttt cctttctctt tattaaccac t 411

<210> 22
<211> 896
<212> DNA
<213> Homo sapiens

<220>
<221> misc_feature
<222> 230, 320
<223> n = A,T,C or G

<400> 22
tgcgctgaaa acaacggcct cctttactgt taaaatgcag ccacaggtgc ttagccgtgg 60
gcatctcaac caccagcctc tgtggggggc aggtgggcgt ccctgtgggc ctctgggccc 120
acgtccagcc tctgtcctct gccttccgtt cttcgacagt gttcccggca tccctggtca 180
cttggtactt ggcgtgggcc tcctgtgctg ctccagcagc tcctccaggn ggtcggcccg 240
cttcaccgca gcctcatgtt gtgtccggag gctgctcacg gcctcctcct tcctcgcgag 300
ggctgtcttc accctccggn gcacctcctc cagctccagc tgctggcggg cctgcagcgt 360
ggccagctcg gccttggcct gccgcgtctc ctcctcarag gctgccagcc ggtcctcgaa 420
ctcctggcgg atcacctggg ccaggttgct gcgctcgcta gaaagctgct cgttcaccgc 480
ctgcgcatcc tccagcgccc gctccttctg ccgcacaagg ccctgcagac gcagattctc 540
gccctcggcc tccccaagct ggcccttcag ctccgagcac cgctcctgaa gcttccgctc 600
cgactgctcc agctcggaga gctcggcctc gtacttgtcc cgtaagcgct tgatgcggct 660
ctcggcagcc ttctcactct cctccttggc cagcgccatg tcggcctcca gccggtgaat 720
gaccagctca atctccttgt cccggccttt ccggatttct tccctcagct cctgttcccg 780
gttcagcagc cacgcctcct ccttcctggt gcggccggcc tcccacgcct gcctctccag 840
ctccagctgc tgcttcaggg tattcagctc catctggcgg gcctgcagcg tggcca 896

<210> 23
<211> 111
<212> DNA
<213> Homo sapiens

<400> 23
caacttatta cttgaaatta taatatagcc tgtccgtttg ctgtttccag gctgtgatat 60

attttcctag tggtttgact ttaaaaataa ataaggttta attttctccc c          111

<210> 24
<211> 531
<212> DNA
<213> Homo sapiens

<220>
<221> misc_feature
<222> 472, 494
<223> n = A,T,C or G

<400> 24
tgcaagtcac gggagtttat ttatttaatt tttttcccca gatggagact ctgtcgccca 60
ggctggagtg caatggtgtg atcttggctc actgcaacct ccacctcctg ggttcaagcg 120
attctcctgc cacagcctcc cgagtagctg ggattacagg tgcccgccac cacacccagc 180
taatttttat atttttagta aagacagggt ttccccatgt tggccaggct ggtcttgaac 240
ttctgacctc aggtgatcca cctgcctcgg cctcccaaag tgttgggatt acaggcgtga 300
gctacccgtg cctggccagc cactggagtt taaaggacag tcatgttggc tccagcctaa 360
ggcggcattt tcccccatca gaaagcccgc ggctcctgta cctcaaaata gggcacctgt 420
aaagtcagtc agtgaagtct ctgctctaac tggccacccg gggccattgg cntctgacac 480
agccttgcca ggangcctgc atctgcaaaa gaaaagttca cttcctttcc g          531

<210> 25
<211> 471
<212> DNA
<213> Homo sapiens

<220>
<221> misc_feature
<222> 377
<223> n = A,T,C or G

<400> 25
cagagaatct kagaaagatg tcgcgttttc ttttaatgaa tgagagaagc ccatttgtat 60
ccctgaatca ttgagaaaag gcggcggtgg cgacagcggc gacctaggga tcgatctgga 120
gggacttggg gagcgtgcag agacctctag ctcgagcgcg agggacctcc cgccgggatg 180
cctggggagc agatggaccc tactggaagt cagttggatt cagatttctc tcagcaagat 240
actccttgcc tgataattga agattctcag cctgaaagcc aggttctaga ggatgattct 300
ggttctcact tcagtatgct atctcgacac cttcctaatc tccagacgca caaagaaaat 360
cctgtgttgg atgttgngtc caatccttga acaaacagct ggagaagaac gaggagaccg 420
gtaatagtgg gttcaatgaa catttgaaag aaaaccaggt tgcagaccct g          471

<210> 26
<211> 541
<212> DNA
<213> Homo sapiens

<400> 26
gactgtcctg aacaagggac ctctgaccag agagctgcag gagatgcaga gtggtggcag 60
gagtggaagc caaagaacac ccaccttcct cccttgaagg agtagagcaa ccatcagaag 120
atactgtttt attgctctgg tcaaacaagt cttcctgagt tgacaaaacc tcaggctctg 180
gtgacttctg aatctgcagt ccactttcca taagttcttg tgcagacaac tgttcttttg 240
cttccatagc agcaacagat gctttggggc taaaaggcat gtcctctgac cttgcaggtg 300
gtggattttg ctcttttaca acatgtacat ccttactggg ctgtgctgtc acagggatgt 360
ccttgctgga ctgttctgct atggggatat cttcgttgga ctgttcttca tgcttaattg 420
cagtattagc atccacatca gacagcctgg tataaccaga gttggtggtt actgattgta 480

gctgctcttt gtccacttca tatggcacaa gtattttcct caacatcctg gctctgggaa 540
g 541

<210> 27
<211> 461
<212> DNA
<213> Homo sapiens

<220>
<221> misc_feature
<222> 367
<223> n = A,T,C or G

<400> 27
gaaatgtata tttaatcatt ctcttgaacg atcagaactc traaatcagt tttctataac 60
arcatgtaat acagtcaccg tggctccaag gtccaggaag gcagtggtta acacatgaag 120
agtgtgggaa gggggctgga aacaaagtat tcttttcctt caaagcttca ttcctcaagg 180
cctcaattca agcagtcatt gtccttgctt tcaaaagtct gtgtgtgctt catggaaggt 240
atatgtttgt tgccttaatt tgaattgtgg ccaggaaggg tctggagatc taaattcaga 300
gtaagaaaac ctgagctaga actcaggcat ttctcttaca gaacttggct tgcagggtag 360
aatgaangga aagaaactta gaagctcaac aagctgaaga taatcccatc aggcatttcc 420
cataggcctt gcaactctgt tcactgagag atgttatcct g 461

<210> 28
<211> 541
<212> DNA
<213> Homo sapiens

<400> 28
agtctggagt gagcaaacaa gagcaagaaa caarragaag ccaaaagcag aaggctccaa 60
tatgaacaag ataaatctat cttcaaagac atattagaag ttgggaaaat aattcatgtg 120
aactagacaa gtgtgttaag agtgataagt aaaatgcacg tggagacaag tgcatcccca 180
gatctcaggg acctccccct gcctgtcacc tggggagtga gaggacagga tagtgcatgt 240
tctttgtctc tgaattttta gttatatgtg ctgtaatgtt gctctgagga agcccctgga 300
aagtctatcc caacatatcc acatcttata ttccacaaat taagctgtag tatgtaccct 360
aagacgctgc taattgactg ccacttcgca actcaggggc ggctgcattt tagtaatggg 420
tcaaatgatt cactttttat gatgcttccc aaggtgcctt ggcttctctt cccaactgac 480
aaatgcccaa gttgagaaaa atgatcataa ttttagcata aaccgagcaa tcggcgaccc 540
c 541

<210> 29
<211> 411
<212> DNA
<213> Homo sapiens

<400> 29
tagctgtctt cctcactctt atggcaatga ccccatatct taatggatta agataatgaa 60
agtgtatttc ttacactctg tatctatcac cagaagctga ggtgatagcc cgcttgtcat 120
tgtcatccat attctgggac tcaggcggga actttctgga atattgccag ggagcatggc 180
agaggggcac agtgcattct gggggaatgc acattggctc agcctgggta atgagtgata 240
tacattacct ctgttcacaa ctcattgccc agcaccagtc acaaggcccc accaaatacc 300
agagcccaag aaatgtagtc ctgttgatat ggttttgctg tgtcccaacc caaatctcat 360
cttgaattgt aagctcccat aattcccatg tgttgtggga gggacctggt g 411

<210> 30
<211> 511
<212> DNA

<213> Homo sapiens

<400> 30
atcatgagga tgttaccaaa gggatggtac taaaccattt gtattcgtct gttttcacac 60
tgctttgaag atactacctg agactgggta atttataaac aaaagagatt taattgactc 120
acagttctgc atggctgaag aggcctcagg aaacttacag tcatggtgga aggcaaagga 180
ggagcaaggc atgtcttaca tgtcagtagg agagagagcg agagcaggag aacctgccac 240
ttataaacca ttcagatctc ataactccct atcatgagaa aaacatggag gaaaccaccc 300
tcatgatcca atcacctccc gccaggtccc tccctcgaca cgtggggatt ataattcagg 360
attagaggga cacagagaca aaccatatca tcattcatga gaaatccacc ctcatagtcc 420
aatcagctcc taccaggccc cacctccaac actggggatt gcaattcaac atgagatttg 480
gatggggaca cagattcaaa ccatatcata c 511

<210> 31
<211> 827
<212> DNA
<213> Homo sapiens

<400> 31
catggccttt ctccttagag gccagaggtg ctgccctggc tgggagtgaa gctccaggca 60
ctaccagctt tcctgatttt cccgtttggt ccatgtgaag agctaccacg agccccagcc 120
tcacagtgtc cactcaaggg cagcttggtc ctcttgtcct gcagaggcag gctggtgtga 180
ccctgggaac ttgacccggg aacaacaggt ggcccagagt gagtgtggcc tggcccctca 240
acctagtgtc cgtcctcctc tctcctggag ccagtcttga gtttaaaggc attaagtgtt 300
agatacaagc tccttgtggc tggaaaaaca cccctctgct gataaagctc agggggcact 360
gaggaagcag aggccccttg ggggtgccct cctgaagaga gcgtcaggcc atcagctctg 420
tccctctggt gctcccacgt ctgttcctca ccctccatct ctgggagcag ctgcacctga 480
ctggccacgc gggggcagtg gaggcacagg ctcagggtgg ccgggctacc tggcacccta 540
tggcttacaa agtagagttg gcccagtttc cttccacctg aggggagcac tctgactcct 600
aacagtcttc cttgccctgc catcatctgg ggtggctggc tgtcaagaaa ggccgggcat 660
gctttctaaa cacagccaca ggaggcttgt agggcatctt ccaggtgggg aaacagtctt 720
agataagtaa ggtgacttgc ctaaggcctc ccagcaccct tgatcttgga gtctcacagc 780
agactgcatg tsaacaactg gaaccgaaaa catgcctcag tataaaa 827

<210> 32
<211> 291
<212> DNA
<213> Homo sapiens

<400> 32
ccagaacctc cttctctttg gagaatgggg aggcctcttg gagacacaga gggtttcacc 60
ttggatgacc tctagagaaa ttgcccaaga agcccacctt ctggtcccaa cctgcagacc 120
ccacagcagt cagttggtca ggccctgctg tagaaggtca cttggctcca ttgcctgctt 180
ccaaccaatg ggcaggagag aaggccttta tttctcgccc acccattctc ctgtaccagc 240
acctccgttt tcagtcagyg ttgtccagca acggtaccgt ttacacagtc a 291

<210> 33
<211> 491
<212> DNA
<213> Homo sapiens

<400> 33
tgcatgtagt tttatttatg tgttttsgtc tggaaaacca agtgtcccag cagcatgact 60
gaacatcact cacttcccct acttgatcta caaggccaac gccgagagcc cagaccagga 120
ttccaaacac actgcacgag aatattgtgg atccgctgtc aggtaagtgt ccgtcactga 180
cccaracgct gttacgtggc acatgactgt acagtgccac gtaacagcac tgtacttttc 240
tcccatgaac agttacctgc catgtatcta catgattcag aacattttga acagttaatt 300

ctgacacttg aataatccca tcaaaaaccg taaaatcact ttgatgtttg taacgacaac 360
atagcatcac tttacgacag aatcatctgg aaaaacagaa caacgaatac atacatctta 420
aaaaatgctg gggtgggcca ggcacagctt cacgcctgta atcccagcac tttgggaggc 480
ttaagcgggt g 491

<210> 34
<211> 521
<212> DNA
<213> Homo sapiens

<220>
<221> misc_feature
<222> 453, 476, 487
<223> n = A,T,C or G

<400> 34
tggggcggaa agaagccaag gccaaggagc tggtgcggca gctgcagctg gaggccgagg 60
agcagaggaa gcagaagaag cggcagagtg tgtcgggcct gcacagatac cttcacttgc 120
tggatggaaa tgaaaattac ccgtgtcttg tggatgcaga cggtgatgtg atttccttcc 180
caccaataac caacagtgag aagacaaagg ttaagaaaac gacttctgat ttgtttttgg 240
aagtaacaag tgccaccagt ctgcagattt gcaaggatgt catggatgcc ctcattctga 300
aaatggcaag aaatgaaaaa gtacacttta gaaaataaag aggaaggatc actctcagat 360
actgaagccg atgcagtctc tggacaactt ccagatccca caacgaatcc cagtgctgga 420
aaggacgggc ccttccttct ggtggtggaa cangtcccgg tggtggatct tggaanggaa 480
cctgaangtg gtgtaccccg tccaaggccg accttggcca c 521

<210> 35
<211> 161
<212> DNA
<213> Homo sapiens

<220>
<221> misc_feature
<222> 18
<223> n = A,T,C or G

<400> 35
tcccgcgctc gcagggcncg tgccacctgc cygtccgccc gctcgctcgc tcgcccgccg 60
cgccgcgctg ccgaccgyca gcatgctgcc gagagtgggc tgccccgcgc tgccgctgcc 120
gccgccgccg ctgctgccgc tgctgccgct gctgctgctg c 161

<210> 36
<211> 341
<212> DNA
<213> Homo sapiens

<400> 36
ggcgggtagg catggaactg agaagaacga agaagctttc agactacgtg gggaagaatg 60
aaaaaaccaa aattatcgcc aagattcagc aaaggggaca gggagctcca gcccgagagc 120
ctattattag cagtgaggag cagaagcagc tgatgctgta ctatcacaga agacaagagg 180
agctcaagag attggaagaa aatgatgatg atgcctattt aaactcacca tgggcggata 240
acactgcttt gaaaagacat tttcatggag tgaaagacat aaagtggaga ccaagatgaa 300
gttcaccagc tgatgacact tccaaagaga ttagctcacc t 341

<210> 37
<211> 521
<212> DNA

<213> Homo sapiens

<220>
<221> misc_feature
<222> 516
<223> n = A,T,C or G

<400> 37
tctgaaggtt aaatgtttca tctaaatagg gataatgrta aacacctata gcatagagtt 60
gtttgagatt aaatgagata atacatgtaa aattatgtgc ctggcataca gcaagattgt 120
tgttgttgtt gatgatgatg atgatgatga taatattttt ctatccccag tgcacaactg 180
cttgaaccta ttagataatc aatacatgtt tcttgaactg agatcaattt ccccatgttg 240
tctgactgat gaagccctac attttcttct agaggagatg acatttgagc aagatcttaa 300
agaaaatcag atgccttcac ctgaccactg cttggtgatc ccatggcact ttgtacatct 360
ctccattagc tctcatctca ccagcccatc attattgtat gtgctgcctt ctgaagcttg 420
cagctggcta ccatcmggta gaataaaaat catcctttca taaaatagtg accctccttt 480
tttatttgca tttcccaaag ccaagcaccg tggganggta g 521

<210> 38
<211> 461
<212> DNA
<213> Homo sapiens

<400> 38
tatgaagaag ggaaaagaag ataatttgtg aaagaaatgg gtccagttac tagtctttga 60
aaagggtcag tctgtagctc ttcttaatga gaataggcag ctttcagttg ctcagggtca 120
gatttcctta gtggtgtatc taatcacagg aaacatctgt ggttccctcc agtctctttc 180
tgggggactt gggcccactt ctcatttcat ttaattagag gaaatagaac tcaaagtaca 240
atttactgtt gtttaacaat gccacaaaga catggttggg agctatttct tgatttgtgt 300
aaaatgctgt ttttgtgtgc tcataatggt tccaaaaatt gggtgctggc caaagagaga 360
tactgttaca gaagccagca agaagacctc tgttcattca cacccccggg gatatcagga 420
attgactcca gtgtgtgcaa atccagtttg gcctatcttc t 461

<210> 39
<211> 769
<212> DNA
<213> Homo sapiens

<400> 39
tgagggactg attggtttgc tctctgctat tcaattcccc aagcccactt gttcctgcag 60
cgtcctcctt ctcattccct ttagttgtac cctctctttc atctgagacc tttccttctt 120
gatgtcgcct tttcttcttc ttgctttttc tgatgttctg ctcagcatgt tctgggtgct 180
tctcatctgc atcattcctt tcagatgctg tagcttcttc ctcctctttc tgcctccttt 240
tctttttctt ttttttgggg ggcttgctct ctgactgcag ttgaggggcc ccagggtcct 300
ggcctttgag acgagccagg aaggcctgct cctgggcctc taggcgagca agcttggcct 360
tcattgtgat cccaagacgg gcagccttgt gtgctgttcg cccctcacag gcttggagca 420
gcatctcatc agtcagaatc tttggggact tggacccctg gttgtcgtca tcactgcagc 480
tctccaagtc tttgtttggc ttctctccac ctgaagtcaa tgtagccatc ttcacaaact 540
tctgatacag caagttgggc ttgggatgat tataacgggt ggtctcctta gaaaggctcc 600
ttatctgtac tccatcctgc ccagtttcca ctaccaagtt ggccgcagtc ttgttgaaga 660
gctcattcca ccagtggttt gtgaactcct tggcagggtc atgtcctacc ccatgagtgt 720
cttgcttcag ygtcaccctg agagcctgag tgataccatt ctccttccg 769

<210> 40
<211> 292
<212> DNA
<213> Homo sapiens

<400> 40
gacaacatga aataaatcct agaggacaaa attaaactca atagagtgta gtctagttaa 60
aaactcgaaa aatgagcaag tctggtggga gtggaggaag ggctatacta taaatccaag 120
tgggcctcct gatcttaaca agccatgctc attatacaca tctctgaact ggacatacca 180
cctttacgca ggaaacaggg cttggaactt ctaagggaaa ttaacatgca ccacccacat 240
ctaacctacc tgccgggtag gtaccatccc tgcttcgctg aaatcagtgc tc 292

<210> 41
<211> 406
<212> DNA
<213> Homo sapiens

<400> 41
ttggaattaa ataaacctgg aacagggaag gtgaaagttg gagtgagatg tcttccatat 60
ctataccttt gtgcacagtt gaatgggaac tgtttgggtt tagggcatct tagagttgat 120
tgatggaaaa agcagacagg aactggtggg aggtcaagtg ggaagttggg tgaatgtgga 180
ataacttacc tttgtgctcc acttaaacca gatgtgttgc agctttcctg acatgcaagg 240
atctacttta attccacact ctcattaata aattgaataa aagggaatgt tttggcacct 300
gatataatct gccaggctat gtgacagtag gaaggaatgg tttcccctaa caagcccaat 360
gcactggtct gactttataa attatttaat aaaatgaact attatc 406

<210> 42
<211> 381
<212> DNA
<213> Homo sapiens

<400> 42
aaactggacc tgcaacaggg acatgaattt actgcarggt ctgagcaagc tcagcccctc 60
tacctcaggg ccccacagcc atgactacct cccccaggag cgggagggtg aagggggcct 120
gtctctgcaa gtggagccag agtggaggaa tgagctctga agacacagca cccagccttc 180
tcgcaccagc caagccttaa ctgcctgcct gaccctgaac cagaacccag ctgaactgcc 240
cctccaaggg acaggaaggc tgggggaggg agtttacaac ccaagccatt ccaccccctc 300
ccctgctggg gagaatgaca catcaagctg ctaacaattg ggggaagggg aaggaagaaa 360
actctgaaaa caaaatcttg t 381

<210> 43
<211> 451
<212> DNA
<213> Homo sapiens

<400> 43
catgcgtttc accactgttg gccaggctgg tctcgaactc ctggcctcaa gcaatccacc 60
cgcctcagcc tccaaaagtg ctgggattac agatgtgagc catggcacca tgccaaaagg 120
ctatattcct ggctctgtgt ttccgagact gcttttaatc ccaacttctc tacatttaga 180
ttaaaaaata ttttattcat ggtcaatctg gaacataatt actgcatctt aagtttccac 240
tgatgtatat agaaggctaa aggcacaatt tttatcaaat ctagtagagt aaccaaacat 300
aaaatcatta attactttca acttaataac taattgacat tcctcaaaag agctgttttc 360
aatcctgata ggttctttat tttttcaaaa tatatttgcc atgggatgct aatttgcaat 420
aaggcgcata atgagaatac cccaaactgg a 451

<210> 44
<211> 521
<212> DNA
<213> Homo sapiens

<400> 44

gttggacccc cagggactgg aaagacactt cttgcccgag ctgtggcggg agaagctgat 60
gttccttttt attatgcttc tggatccgaa tttgatgaga tgtttgtggg tgtgggagcc 120
agccgtatca gaaatctttt tagggaagca aaggcgaatg ctccttgtgt tatatttatt 180
gatgaattag attctgttgg tgggaagaga attgaatctc caatgcatcc atattcaagg 240
cagaccataa atcaacttct tgctgaaatg gatggtttta aacccaatga aggagttatc 300
ataataggag ccacaaactt cccagaggca ttagataatg ccttaatacc gtcctggtcg 360
ttttgacatg caagttacag ttccaaggcc agatgtaaaa ggtcgaacag aaattttgaa 420
atggtatctc aataaaataa agtttgatca atcccgttga tccagaaatt atagcctcga 480
ggtactggtg gcttttccgg aagcagagtt gggagaatct t 521

<210> 45
<211> 585
<212> DNA
<213> Homo sapiens

<400> 45
gcctacaaca tccagaaaga gtctaccctg cacctggtgc tscgtctcag aggtgggatg 60
cagatcttcg tgaagaccct gactggtaag accatcactc tcgaagtgga gccgagtgac 120
accatygaga acgtcaaagc aaagatccar gacaaggaag gcrtycctcc tgaccagcag 180
aggttgatct ttgccggaaa gcagctggaa gatggdcgca ccctgtctga ctacaacatc 240
cagaaagagt cyaccctgca cctggtgctc cgtctcagag gtgggatgca ratcttcgtg 300
aagaccctga ctggtaagac catcaccctc gaggtggagc ccagtgacac catcgagaat 360
gtcaaggcaa agatccaaga taaggaaggc atccctcctg atcagcagag gttgatcttt 420
gctgggaaac agctggaaga tggacgcacc ctgtctgact acaacatcca gaaagagtcc 480
actctgcact tggtcctgcg cttgaggggg ggtgtctaag tttccccttt taaggtttcm 540
acaaatttca ttgcactttc ctttcaataa agttgttgca ttccc 585

<210> 46
<211> 481
<212> DNA
<213> Homo sapiens

<400> 46
gaactgggcc ctgagcccaa gtcatgcctt gtgtccgcat ctgccgtgtc acctctgtkc 60
ctgcccctca cccctccctc ctggtcttct gagccagcac catctccaaa tagcctattc 120
cttcctgcaa atcacacaca catgcgggcc acacatacct gctgccctgg agatggggaa 180
gtaggagaga tgaatagagg cccatacatt gtacagaagg aggggcaggt gcagataaaa 240
gcagcagacc cagcggcagc tgaggtgcat ggagcacggt tggggccggc attgggctga 300
gcacctgatg ggcctcatct cgtgaatcct cgaggcagcg ccacagcaga ggagttaagt 360
ggcacctggg ccgagcagag caggagactg agggtcagag tggaggctaa gctgccctgg 420
aactcctcaa tcttgcctgc cccctagtat gaagccccct tcctgcccct acaattcctg 480
a 481

<210> 47
<211> 461
<212> DNA
<213> Homo sapiens

<220>
<221> misc_feature
<222> 128
<223> n = A,T,C or G

<400> 47
atggatctta ctttgccacc caggttggag tgcagtgctg caatcttggc tcactgcagc 60
cttaacctcc caggctcaag ctatcctcct gccaaagcct tccacatagc tgggactaca 120
ggtacacngc caccacaccc agctaaaatt tttgtatttt ttgtagagac gggatctcgc 180

cacgttgccc aggctggtcc catcctgacc tcaagcagat ctgcccacct cagcccccca 240
acgtgctagg attacaggcg tgagccaccg cacccagcct ttgttttgct tttaatggaa 300
tcaccagttc ccctccgtgt ctcagcagca gctgtgagaa atgctttgca tctgtgacct 360
ttatgaaggg gaacttccat gctgaatgag ggtaggatta catgctcctg tttcccgggg 420
gtcaagaaag cctcagactc cagcatgata agcagggtga g 461

<210> 48
<211> 571
<212> DNA
<213> Homo sapiens

<400> 48
ataggggctt taaggaggga attcaggttc aatgaggtcg taaggccagg gctcttatcc 60
agtaagactg gggtccttag atgagaaaga gacacccgag gtccttctct ctgccgtgtg 120
aggatgcatc aagaaggcgg ccgtctgcaa gcgaaggaga ggccgcacca gaaaccgaca 180
ccttcatctt ggacttgcag cctctagaac tgagaaaata actgtctgtt ggttaagcca 240
cccagtttgt agtattctct tatggcttcc taagcagact aacaaacaaa cacccaaaat 300
taactgatgg cttcgctgtc ttctgtaaaa attgctatga gagaactttt cactcactgt 360
tttgcagttt ctccctcagt ccctggttct ttcttctcac ataatcccaa tttcaattta 420
tagttcatgg cccaggcaga gtcattcatc acggcatctc ctgagctaaa ccagcacctg 480
ctctgctcac ttcttgactg gctgctcatc atcagccctc ttgcagagat ttcatttcct 540
cccgtgccag gtacttcacg caccaagctc a 571

<210> 49
<211> 511
<212> DNA
<213> Homo sapiens

<400> 49
ggataatgaa gttgttttat ttagcttgga caaaaaggca tattcctcta ttttcttata 60
caacaaatat ccccaaaata aagcaagcat atatatcttg aatgtgtaat aatccagtga 120
taaacaagag cagtacttta aaagaaaaaa aaatatgtat ttctgtcagg ttaaaatgag 180
aatcaaaacc atttactctg ctaactcatt attttttgct ttctttttgg ttaagagagg 240
caatgcaata cactgaaaaa ggtttttatc ttatctggca ttggaattag acatattcaa 300
accccagccc ccatttccaa actttaagac cacaaacaag taatttactt ttctgaacat 360
tggttttttc tggaaaatgg gaattataaa atagactttg cagactctta tgagattaaa 420
taagataatg tatgaaattc tttcttcttt tttacttctt tttccttttt gagatggagt 480
ctcaccccgt cacccaggct ggagtacagt g 511

<210> 50
<211> 561
<212> DNA
<213> Homo sapiens

<400> 50
ccactgcact ccagcctggg tgacggagtg agactctgtc tcaaaaaaac aaacaaacaa 60
acaaacaaaa aactgaaaag gaaatagagt tcctctttcc tcatatatga atatattatt 120
tcaacagatt gttgatcacc taccatatgc ttggtattgt tctaattgct ggggatacag 180
caagaggttc tgcagaactt catggagcat gaaagtaaat aaacaaagtt aatttcaagg 240
ccaggcatgg ttgctcacac ctttagtccc agcactttgg gaggctgagg caggtggatc 300
acttgggccc aggagttcaa ggctgcagtg agccaagatt gtgccactac tctccaggct 360
gggcaacaga gcaagaccct gtctcagggg gaacaaaaag ttaatttcag attttgttaa 420
gtgctgtaaa ggaagtaaat aggttgatat tcaagagagc acctgaaggc caggcgtggt 480
ggctcacgcc tgtggtctaa cgctttggga agcccgagcg ggcggatcac aaggtcagga 540
gaattttggc caggcatggt g 561

<210> 51

<211> 451
<212> DNA
<213> Homo sapiens

<400> 51
agaatccatt tattgggttt taaactagtt acacaactga aatcagtttg gcactacttt 60
atacagggat tacgcctgtg tatgccgaca cttaaatact gtaccaggac cactgctgtg 120
cttaggtctg tattcagtca ttcagcatgt agatactaaa aatatactgt agtgttcctt 180
taaggaagac tgtacagggt gtgttgcaag atgacattca ccaatttgtg aattatttca 240
acccagaaga tacctttcac tctataaact tgtcataggc aaacatgtgg tgttagcatt 300
gagagatgca cacaaaaatg ttacataaaa gttcagacat tctaatgata agtgaactga 360
aaaaaaaaaa aaccccacat ctcaattttt gtaacaagat aaagaaaata atttaaaaac 420
acaaaaaatg gcattcagtg ggtacaaagc c 451

<210> 52
<211> 682
<212> DNA
<213> Homo sapiens

<400> 52
caaatattta atataaatct ttgaaacaag ttcagakgaa ataaaaatca aagtttgcaa 60
aaacgtgaag attaacttaa ttgtcaaata ttcctcattg ccccaaatca gtattttttt 120
tatttctatg caaaagtatg ccttcaaact gcttaaatga tatatgatat gatacacaaa 180
ccagttttca aatagtaaag ccagtcatct tgcaattgta agaaataggt aaaagattat 240
aagacacctt acacacacac acacacacac acacacacgt gtgcaccgcc aatgacaaaa 300
aacaatttgg cctctcctaa aataagaaca tgaagaccct taattgctgc caggagggaa 360
cactgtgtca cccctcccta caatccaggt agtttccttt aatccaatag caaatctggg 420
catatttgag aggagtgatt ctgacagcca csgttgaaat cctgtgggga accattcatg 480
tccacccact ggtgccctga aaaaatgcca ataatttttc gctcccactt ctgctgctgt 540
ctcttccaca tcctcacata gaccccagac ccgctggccc ctggctgggc atcgcattgc 600
tggtagagca agtcataggt ctcgtctttg acgtcacaga agcgatacac caaattgcct 660
ggtcggtcat tgtcataacc ag 682

<210> 53
<211> 311
<212> DNA
<213> Homo sapiens

<220>
<221> misc_feature
<222> 208
<223> n = A,T,C or G

<400> 53
tttgacttta gtaggggtct gaactattta ttttactttg ccmgtaatat ttaraccyta 60
tatatctttc attatgccat cttatcttct aatgbcaagg gaacagwtgc taamctggct 120
tctgcattwa tcacattaaa aatggctttc ttggaaaatc ttcttgatat gaataaagga 180
tcttttavag ccatcattta aagcmggntt ctctccaaca cgagtctgct sasggggggk 240
gagctgtgaa ctctggctga aggctttccc atacacactg caatgacmtg gtttctgacc 300
agbgtgagtt a 311

<210> 54
<211> 561
<212> DNA
<213> Homo sapiens

<400> 54

agagaagccc cataaatgca atcagtgtgg gaaggccttc agtcagagct caagcctttt 60
cctccatcat cgggttcata ctggagagaa accctatgta tgtaatgaat gcggcagagc 120
ctttggtttt aactctcatc ttactgaaca cgtaaggatt cacacaggag aaaaacccta 180
tgtttgtaat gagtgcggca aagcctttcg tcggagttcc actcttgttc agcatcgaag 240
agttcacact ggggagaagc cctaccagtg cgttgaatgt gggaaagctt tcagccagag 300
ctcccagctc accctacatc agccgagttc acactggaga gaagccctat gactgtggtg 360
actgtgggaa ggccttcagc cggaggtcaa ccctcattca gcatcagaaa gttcacagcg 420
gagagactcg taagtgcaga aaacatggtc cagcctttgt tcatggctcc agcctcacag 480
cagatggaca gattcccact ggagagaagc acggcagaac ctttaaccat ggtgcaaatc 540
tcattctgcg ctggacagtt c 561

<210> 55
<211> 811
<212> DNA
<213> Homo sapiens

<400> 55
gagacagggt ctcactttgt cacccaggct ggaatgcagt ggtgcgatct tacgtagctc 60
actgcagccc tgacctcctg gactcaaaca attctcctgc ctcagccctg caagtagctg 120
ggactgtggg tgcatgccac catgcctggc taacttttgt agtttttgta aagatggggt 180
tttgccatgt tgcacatgct ggtcttgaac tcctgagctc aaacgatctg cccacctcgg 240
cctcccagaa tgttgggatt acaggggtaa accaccacgc ctggccccat tagggtattc 300
ttagcatcca cttgctcact gagattaatc ataagagatg ataagcactg gaagaaaaaa 360
atttttacta ggctttggat atttttttcc tttttcagct ttatacagag gattggatct 420
ttagttttcc tttaactgat aataaaacat tgaaaggaaa taagtttacc tgagattcac 480
agagataacc ggcatcactc ccttgctcaa ttccagtctt taccacatca attattttca 540
gaggtgcagg ataaaggcct ttagtctgct ttcgcacttt ttcttccact tttttgtaaa 600
cctgttgcct gacaaatgga attgacagcg tatgccatga ctattccatt tgtcaggcat 660
acgctgtcaa tttttccacc aatcccttgt ctctctttgg agagatcttc ttatcagcta 720
gtcctttggc aaaagtaatt gcaacttctt ctaggtattc tattgtccgt tccactggtg 780
gaacccctgg gaccaggact aaaacctcca g 811

<210> 56
<211> 591
<212> DNA
<213> Homo sapiens

<220>
<221> misc_feature
<222> 45, 477, 490, 561
<223> n = A,T,C or G

<400> 56
atctcatata tatatttctt cctgacttta tttgcttgct tctgncacgc atttaaaata 60
tcacagagac caaaatagag cggctttctg gtggaacgca tggcagtcac aggacaaaat 120
acaaaactag ggggctctgt cttctcatac atcatacaat tttcaagtat tttttttatg 180
tacaaagagc tactctatct gaaaaaaaat taaaaaataa atgagacaag atagtttatg 240
catcctagga agaaagaatg ggaagaaaga acggggcagt tgggtacaga ttcctgtccc 300
ctgttcccag ggaccactac cttcctgcca ctgagttccc ccacagcctc acccatcatg 360
tcacagggca agtgccaggg taggtgggga ccagtggaga caggaaccag caacatactt 420
tggcctggaa gataaggaga aagtctcaga aacacactgg tgggaagcaa tcccacnggc 480
cgtgccccan gagcttccca cctgctgctg gctccctggg tggctttggg aacagcttgg 540
gcaggccctt ttgggtgggg nccaactggg cctttgggcc cgtgtggaaa g 591

<210> 57
<211> 481
<212> DNA

<213> Homo sapiens

<400> 57
   aaacattgag atggaatgat agggtttccc agaatcaggt ccatatttta actaaatgaa 60
   aattatgatt tatagccttc tcaaatacct gccatacttg atatctcaac cagagctaat 120
   tttacctctt tacaaattaa ataagcaagt aactggatcc acaatttata atacctgtca 180
   attttttctg tattaaacct ctatcatagt ttaagcctat tagggtactt aatccttaca 240
   aataaacagg tttaaaatca cctcaatagg caactgccct tctggttttc ttctttgact 300
   aaacaatctg aatgcttaag attttccact ttgggtgcta gcagtacaca gtgttacact 360
   ctgtattcca gacttcttaa attatagaaa aaggaatgta cactttttgt attctttctg 420
   agcagggccg ggaggcaaca tcatctacca tggtagggac ttgtatgcat ggactacttt 480
   a 481

<210> 58
<211> 141
<212> DNA
<213> Homo sapiens

<400> 58
actctgtcgc ccaggctgga gcccabtggm gcgatctcga ctccctgcaa gctmcgcctc 60
acaggwtcat gccattctcc tgcctcagca tctggagtag ctgggactac aggcgccagc 120
caccatgccc agctaatttt t 141

<210> 59
<211> 191
<212> DNA
<213> Homo sapiens

<400> 59
accttaaaga cataggagaa tttatactgg gagagaaagc ttacaaatgt aaggtttctg 60
acaagacttg ggagtgattc acacctggaa caacatactg gacttcacac tggabagaaa 120
ccttacaagt gtaatgagtg tggcaaagcc tttggcaagc agtcaacact tattcaccat 180
caggcaattc a 191

<210> 60
<211> 480
<212> DNA
<213> Homo sapiens

<400> 60
agtcaggatc atgatggctc agtttcccac agcgatgaat ggagggccaa atatgtgggc 60
tattacatct gaagaacgta ctaagcatga taaacagttt gataacctca aaccttcagg 120
aggttacata acaggtgatc aagcccgtac tttttcccta cagtcaggtc tgccggcccc 180
ggttttagct gaaatatggg ccttatcaga tctgaacaag gatgggaaga tggaccagca 240
agagttctct atagctatga aactcatcaa gttaaagttg cagggccaac agctgcctgt 300
agtcctccct cctatcatga aacaaccccc tatgttctct ccactaatct ctgctcgttt 360
tgggatggga agcatgccca atctgtccat tcatcagcca ttgcctccag ttgcacctat 420
agcaacaccc ttgtcttctg ctacttcagg gaccagtatt cctccctaat gatgcctgct 480

<210> 61
<211> 381
<212> DNA
<213> Homo sapiens

<400> 61
ctttcgattt ccttcaattt gtcacgtttg attttatgaa gttgttcaag ggctaactgc 60

tgtgtattat agctttctct gagttccttc agctgattgt taaatgaatc catttctgag 120
agcttagatg cagtttcttt ttcaagagca tctaattgtt ctttaagtct ttggcataat 180
tcttcctttt ctgatgactt tctatgaagt aaactgatcc ctgaatcagg tgtgttactg 240
agctgcatgt ttttaattct ttcgtttaat agctgcttct cagggaccag atagataagc 300
ttattttgat attccttaag ctcttggtga agttgttcga tttccataat ttccaggtca 360
cactggttat cccaaacttc t 381

<210> 62
<211> 906
<212> DNA
<213> Homo sapiens

<400> 62
gtggaggtga aacggaggca agaaaggggg ctacctcagg agcgagggac aaagggggcg 60
tgaggcacct aggccgcggc accccggcga caggaagccg tcctgaaccg ggctaccggg 120
taggggaagg gcccgcgtag tcctcgcagg gccccagagc tggagtcggc tccacagccc 180
cgggccgtcg gcttctcact tcctggacct ccccggcgcc cgggcctgag gactggctcg 240
gcggagggag aagaggaaac agacttgagc agctccccgt tgtctcgcaa ctccactgcc 300
gaggaactct catttcttcc ctcgctcctt caccccccac ctcatgtaga aaggtgctga 360
agcgtccgga gggaagaaga acctgggcta ccgtcctggc cttcccmccc ccttcccggg 420
gcgctttggt gggcgtggag ttggggttgg gggggtgggt gggggttctt ttttggagtg 480
ctggggaact tttttccctt cttcaggtca ggggaaaggg aatgcccaat tcagagagac 540
atgggggcaa gaaggacggg agtggaggag cttctggaac tttgcagccg tcatcgggag 600
gcggcagctc taacagcaga gagcgtcacc gcttggtatc gaagcacaag cggcataagt 660
ccaaacactc caaagacatg gggttggtga cccccgaagc agcatccctg ggcacagtta 720
tcaaaccttt ggtggagtat gatgatatca gctctgattc cgacaccttc tccgatgaca 780
tggccttcaa actagaccga agggagaacg acgaacgtcg tggatcagat cggagcgacc 840
gcctgcacaa acatcgtcac caccagcaca ggcgttcccg ggacttacta aaagctaaac 900
agaccg 906

<210> 63
<211> 491
<212> DNA
<213> Homo sapiens

<400> 63
gacatgtttg cctgcagggg accagagaca atgggattag ccagtgctca ctgttcttta 60
tgcttccaga gaggatgggg acagctctca ggtcagaatc caggctgaga aggccatgct 120
ggttgggggc ccccggaagc acggtccgga tcctccctgg catcagcgta gacccgctgc 180
tcaggcttgg ggtaccaaac tcatgctctg tactgttttg gccccatgcg gtgagaggaa 240
aacctagaaa aagattggtc gtgctaagga atcagctgcc ccctcatcct ccgcatccaa 300
tgctggtgac aacatattcc ctctcccagg acacagactc ggtgactcca cactgggctg 360
agtggcctct ggaggctcgt ggcctaaggc agggctccgt aaggctgatc ggctgaactg 420
ggtggggtga gggtttctga cccttcgctt cccatcccat aaccgctgtc aatgagctca 480
cactgtggtc a 491

<210> 64
<211> 511
<212> DNA
<213> Homo sapiens

<400> 64
gatggcatgg tcgttgctaa tgtgcctgct gggatggagc acttcctcct gtgagcccag 60
gggacccgcc tgtccctgga gcttggggca aggagggaag agtgatacca ggaaggtggg 120
gctgcagcca ggggccagag tcagttcagg gagtggtcct cggccctcaa agctcctccg 180
gggactgctc aggagtgatg gtgccctgga gtttgcccca acttccctgg ccaccctgga 240
aggtgcctgg ctgctccagg cctctaggct gggctgatgg gtttctccag gacacaagta 300

tcattaaagc caccctctcc tcagcttgtc aggccgcaca tgtgggacag gctgtgctca 360
caaccccctc gcctgccctg ccctccatca ggaggagcca gtggaacctt cggaaagctc 420
ccagcatctc agcagccctc aaaagtcgtc ctggggcaag ctctggttct cctgactgga 480
ggtcatctgg gcttggcctg ctctctctcg c 511

<210> 65
<211> 394
<212> DNA
<213> Homo sapiens

<400> 65
taaaaaagtg taacaaaggt ttatttagac tttcttcatg cccccagatc caggatgtct 60
atgtaaaccg ttatcttaca aagaaagcac aatatttggt ataaactaag tcagtgactt 120
gcttaactga aatagcgtcc atccaaaagt gggtttaagg taaaactacc tgacgatatt 180
ggcggggatc ctgcagtttg gactgcttgc cgggtttgtc cagggttccg ggtctgttct 240
tggcactcat ggggacaggc atcctgctcg tctgtggggc cccgctggag cccttacgtg 300
aagctgaagg tatcgaccst agggggctct agggcagtgg gaccttcatc cggaactaac 360
aagggtcggg gagaggcctc ttgggctatg tggg 394

<210> 66
<211> 359
<212> DNA
<213> Homo sapiens

<400> 66
caagcgttcc tttatggatg taaattcaaa cagtcatgct gagccatccc gggctgacag 60
tcacgttwaa gacactaggt cgggcgccac agtgccaccc aaggagaaga agaatttgga 120
atttttccat gaagatgtac ggaaatctga tgttgaatat gaaaatggcc cccaaatgga 180
attccaaaag gttaccacag gggctgtaag acctagtgac cctcctaagt gggaaagagg 240
aatggagaat agtatttctg atgcatcaag aacatcagaa tataaaactg agatcataat 300
gaaggaaaat tccatatcca atatgagttt actcagagac agtagaaact attcccagg 359

<210> 67
<211> 450
<212> DNA
<213> Homo sapiens

<220>
<221> misc_feature
<222> 425
<223> n = A,T,C or G

<400> 67
taggaataac aaatgtttat tcagaaatgg ataagtaata cataatcacc cttcatctct 60
taatgcccct tcctctcctt ctgcacagga gacacagatg ggtaacatag aggcatggga 120
agtggaggag gacacaggac tagcccacca ccttctcttc ccggtctccc aagatgactg 180
cttatagagt ggaggaggca aacaggtccc ctcaatgtac cagatggtca cctatagcac 240
cagctccaga tggccacgtg gttgcagctg gactcaatga aactctgtga caaccagaag 300
atacctgctt tgggatgaga gggaggataa agccatgcag ggaggatatt taccatccct 360
accctaagca cagtgcaagc agtgagcccc cggctcccag tacctgaaaa accaaggcct 420
actgnctttt ggatgctctc ttgggccacg 450

<210> 68
<211> 511
<212> DNA
<213> Homo sapiens

<400> 68
aagcctcctg ccctggaaat ctggagcccc ttggagctga gctggacggg gcagggaggg 60
gctgagaggc aagaccgtct ccctcctgct gcagctgctt ccccagcagc cactgctggg 120
cacagcagaa acgccagcag agaaaatggg agccgagagt ccttagccct ggagctgagg 180
ctgcctctgg gctgacccgc tggctgtacg tggccagaac tggggttggc atctggcatc 240
catttgaggc cagggtggag gaaagggagg ccaacagagg aaaacctatt cctgctgtga 300
caacacagcc cttgtcccac gcagcctaag tgcagggagc gtgatgaagt caggcagcca 360
gtcggggagg acgaggtaac tcagcagcaa tgtcaccttg tagcctatgc gctcaatggc 420
ccggaggggc agcaaccccc cgcacacgtc agccaacagc agtgcctctg caggcaccaa 480
gagagcgatg atggacttga gcgccgtgtt c 511

<210> 69
<211> 511
<212> DNA
<213> Homo sapiens

<400> 69
gtttggcaga agacatgttt aataacattt tcatatttaa aaaatacagc aacaattctc 60
tatctgtcca ccatcttgcc ttgcccttcc tggggctgag gcagacaaag gaaaggtaat 120
gaggttaggg cccccaggcg ggctaagtgc tattggcctg ctcctgctca aagagagcca 180
tagccagctg ggcacggccc cctagcccct ccaggttgct gaggcggcag cggtggtaga 240
gttcttcact gagccgtggg ctgcagtctc gcagggagaa cttctgcacc agccctggct 300
ctacggcccg aaagaggtgg agccctgaga accggaggaa aacatccatc acctccagcc 360
cctccagggc ttcctcctct tcctggcctg ccagttcacc tgccagccgg gctcggccgg 420
ccaggtagtc agcgttgtag aagcagccct ccgcagaagc ctgccggtca aatctccccg 480
ctataggagc cccccgggag gggtcagcac c 511

<210> 70
<211> 511
<212> DNA
<213> Homo sapiens

<400> 70
caagttgaac gtcaggcttg gcagaggtgg agtgtagatg aaaacaaagg tgtgattatg 60
aagaggatgt gagtcctttg ggtgtaggag agaaaggctg ttgagcttct atttcaagat 120
acttttacct gtgcaaaaag cacattttcc acctccttct catggcattt gtgtaaggtg 180
agtatgattc ctattccatc tgcattttag aggtgaagaa taacgtacaa gggattcagt 240
gattagcaag ggacccctca ctaagtgttg atggagttag gacagagctc agctgtttga 300
atctcagagc ccaggcagct ggagctgggt aggatcctgg agctggcact aatgtgaggt 360
gcattccctc caacccaggc tcagatccgg aacctgaccg tgctgacccc cgaaggggag 420
gcagggctga gctggcccgt tgggctccct gctcctttca caccacactc tcgctttgag 480
gtgctgggct gggactactt cacagagcag c 511

<210> 71
<211> 511
<212> DNA
<213> Homo sapiens

<400> 71
tggcctgggc aggattggga gagaggtagc tacccggatg cagtcctttg ggatgaagac 60
tatagggtat gaccccatca tttccccaga ggtctcggcc tcctttggtg ttcagcagct 120
gcccctggag gagatctggc ctctctgtga tttcatcact gtgcacactc ctctcctgcc 180
ctccacgaca ggcttgctga atgacaacac ctttgcccag tgcaagaagg gggtgcgtgt 240
ggtgaactgt gcccgtggag ggatcgtgga cgaaggcgcc ctgctccggg ccctgcagtc 300
tggccagtgt gccggggctg cactggacgt gtttacggaa gagccgccac gggaccgggc 360
cttggtggac catgagaatg tcatcagctg tccccacctg ggtgccagca ccaaggaggc 420
tcagagccgc tgtggggagg aaattgctgt tcagttcgtg gacatggtga aggggaaatc 480

tctcacgggg gttgtgaatg cccaggccct t 511

<210> 72
<211> 2017
<212> DNA
<213> Homo sapiens

<400> 72
agccagatgg ctgagagctg caagaagaag tcaggatcat gatggctcag tttcccacag 60
cgatgaatgg agggccaaat atgtgggcta ttacatctga agaacgtact aagcatgata 120
aacagtttga taacctcaaa ccttcaggag gttacataac aggtgatcaa gcccgtactt 180
ttttcctaca gtcaggtctg ccggccccgg ttttagctga aatatggccc ttatcagatc 240
tgaacaagga tgggaagatg gaccagcaag agttctctat agctatgaaa ctcatcaagt 300
taaagttgca gggccaacag ctgcctgtag tcctccctcc tatcatgaaa caacccccta 360
tgttctctcc actaatctct gctcgttttg ggatgggaag catgcccaat ctgtccattc 420
atcagccatt gcctccagtt gcacctatag caacaccctt gtcttctgct acttcaggga 480
ccagtattcc tcccctaatg atgcctgctc ccctagtgcc ttctgttagt acatcctcat 540
taccaaatgg aactgccagt ctcattcagc ctttatccat tccttattct tcttcaacat 600
tgcctcatgc atcatcttac agcctgatga tgggaggatt tggtggtgct agtatccaga 660
aggcccagtc tctgattgat ttaggatcta gtagctcaac ttcctcaact gcttccctct 720
cagggaactc acctaagaca gggacctcag agtgggcagt tcctcagcct tcaagattaa 780
agtatcggca aaaatttaat agtctagaca aaggcatgag cggatacctc tcaggttttc 840
aagctagaaa tgcccttctt cagtcaaatc tctctcaaac tcagctagct actatttgga 900
ctctggctga catcgatggt gacggacagt tgaaagctga agaatttatt ctggcgatgc 960
acctcactga catggccaaa gctggacagc cactaccact gacgttgcct cccgagcttg 1020
tccctccatc tttcagaggg ggaaagcaag ttgattctgt taatggaact ctgccttcat 1080
atcagaaaac acaagaagaa gagcctcaga agaaactgcc agttactttt gaggacaaac 1140
ggaaagccaa ctatgaacga ggaaacatgg agctggagaa gcgacgccaa gtgttgatgg 1200
agcagcagca gagggaggct gaacgcaaag cccagaaaga gaaggaagag tgggagcgga 1260
aacagagaga actgcaagag caagaatgga agaagcagct ggagttggag aaacgcttgg 1320
agaaacagag agagctggag agacagcggg aggaagagag gagaaaggag atagaaagac 1380
gagaggcagc aaaacaggag cttgagagac aacgccgttt agaatgggaa agactccgtc 1440
ggcaggagct gctcagtcag aagaccaggg aacaagaaga cattgtcagg ctgagctcca 1500
gaaagaaaag tctccacctg gaactggaag cagtgaatgg aaaacatcag cagatctcag 1560
gcagactaca agatgtccaa atcagaaagc aaacacaaaa gactgagcta gaagttttgg 1620
ataaacagtg tgacctggaa attatggaaa tcaaacaact tcaacaagag cttaaggaat 1680
atcaaaataa gcttatctat ctggtccctg agaagcagct attaaacgaa agaattaaaa 1740
acatgcagct cagtaacaca cctgattcag ggatcagttt acttcataaa aagtcatcag 1800
aaaaggaaga attatgccaa agacttaaag aacaattaga tgctcttgaa aaagaaactg 1860
catctaagct ctcagaaatg gattcattta acaatcagct gaaggaactc agagaaagct 1920
ataatacaca gcagttagcc cttgaacaac ttcataaaat caaacgtgac aaattgaagg 1980
aaatcgaaag aaaaagatta gagcaaaaaa aaaaaaa 2017

<210> 73
<211> 414
<212> DNA
<213> Homo sapiens

<400> 73
atggcagtga cattcaccat catgggaacc accttccctt ttcttcagga ttctctgtag 60
tggaagagag cacccagtgt tgggctgaaa acatctgaaa gtagggagaa gaacctaaaa 120
taatcagtat ctcagagggc tctaaggtgc caagaagtct cactggacat ttaagtgcca 180
acaaaggcat actttcggaa tcgccaagtc aaaactttct aacttctgtc tctctcagag 240
acaagtgaga ctcaagagtc tactgcttta gtggcaacta cagaaaactg gtgttaccca 300
gaaaaacagg agcaattaga aatggttcca atatttcaaa gctccgcaaa caggatgtgc 360
tttcctttgc ccatttaggg tttcttctct ttcctttctc tttattaacc acta 414

<210> 74
<211> 1567
<212> DNA
<213> Homo sapiens

<400> 74
atatctagaa gtctggagtg agcaaacaag agcaagaaac aaaaagaagc caaaagcaga 60
aggctccaat atgaacaaga taaatctatc ttcaaagaca tattagaagt tgggaaaata 120
attcatgtga actagacaag tgtgttaaga gtgataagta aaatgcacgt ggagacaagt 180
gcatccccag atctcaggga cctccccctg cctgtcacct ggggagtgag aggacaggat 240
agtgcatgtt ctttgtctct gaatttttag ttatatgtgc tgtaatgttg ctctgaggaa 300
gcccctggaa agtctatccc aacatatcca catcttatat tccacaaatt aagctgtagt 360
atgtacccta agacgctgct aattgactgc cacttcgcaa ctcaggggcg gctgcatttt 420
agtaatgggt caaatgattc actttttatg atgcttccaa aggtgccttg gcttctcttc 480
ccaactgaca aatgccaaag ttgagaaaaa tgatcataat tttagcataa acagagcagt 540
cggcgacacc gattttataa ataaactgag caccttcttt ttaaacaaac aaatgcgggt 600
ttatttctca gatgatgttc atccgtgaat ggtccaggga aggacctttc accttgacta 660
tatggcatta tgtcatcaca agctctgagg cttctccttt ccatcctgcg tggacagcta 720
agacctcagt tttcaatagc atctagagca gtgggactca gctggggtga tttcgccccc 780
catctccggg ggaatgtctg aagacaattt tgttacctca atgagggagt ggaggaggat 840
acagtgctac taccaactag tggataaagg ccagggatgc tgctcaacct cctaccatgt 900
acaggacgtc tccccattac aactacccaa tccgaagtgt caactgtgtc aggactaaga 960
aaccctggtt ttgagtagaa aagggcctgg aaagagggga gccaacaaat ctgtctgctt 1020
cctcacatta gtcattggca aataagcatt ctgtctcttt ggctgctgcc tcagcacaga 1080
gagccagaac tctatcgggc accaggataa catctctcag tgaacagagt tgacaaggcc 1140
tatgggaaat gcctgatggg attatcttca gcttgttgag cttctaagtt tctttccctt 1200
cattctaccc tgcaagccaa gttctgtaag agaaatgcct gagttctagc tcaggttttc 1260
ttactctgaa tttagatctc cagacccttc ctggccacaa ttcaaattaa ggcaacaaac 1320
atataccttc catgaagcac acacagactt ttgaaagcaa ggacaatgac tgcttgaatt 1380
gaggccttga ggaatgaagc tttgaaggaa aagaatactt tgtttccagc cccttcccca 1440
cactcttcat gtgttaacca ctgccttcct ggaccttgga gccacggtga ctgtattaca 1500
tgttgttata gaaaactgat tttagagttc tgatcgttca agagaatgat taaatataca 1560
tttccta 1567

<210> 75
<211> 240
<212> DNA
<213> Homo sapiens

<400> 75
tcgagcggcc gcccgggcag gtccttcaga cttggactgt gtcacactgc caggcttcca 60
gggctccaac ttgcagacgg cctgttgtgg gacagtctct gtaatcgcga aagcaaccat 120
ggaagacctg ggggaaaaca ccatggtttt atccaccctg agatctttga acaacttcat 180
ctctcagcgt gcggagggag gctctggact ggatatttct acctcggccg cgaccacgct 240

<210> 76
<211> 330
<212> DNA
<213> Homo sapiens

<220>
<221> misc_feature
<222> 288
<223> n = A,T,C or G

<400> 76

tagcgyggtc gcggccgagg yctgcttytc tgtccagccc agggcctgtg gggtcagggc 60
ggtgggtgca gatggcatcc actccggtgg cttccccatc tttctctggc ctgagcaagg 120
tcagcctgca gccagagtac agagggccaa cactggtgtt cttgaacaag ggccttagca 180
ggccctgaag grccctctct gtagtgttga acttcctgga gccaggccac atgttctcct 240
cataccgcag gytagygatg gtgaagttga gggtgaaata gtattmangr agatggctgg 300
caracctgcc cgggcggccg ctcsaaatcc 330

<210> 77
<211> 361
<212> DNA
<213> Homo sapiens

<400> 77
agcgtggtcg cggccgaggt gtccttcagg gtctgcttat gcccttgttc aagaacacca 60
gtgtcagctc tctgtactct ggttgcagac tgaccttgct caggcctgag aaggatgggg 120
cagccaccag agtggatgct gtctgcaccc atcgtcctga ccccaaaagc cctggactgg 180
acagagagcg gctgtactgg aagctgagcc agctgaccca cggcatcact gagctgggcc 240
cctacaccct ggacagggac agtctctatg tcaatggttt cacccatcgg agctctgtac 300
ccaccaccag caccggggtg gtcagcgagg agccattcaa cctgcccggg cggccgctcg 360
a 361

<210> 78
<211> 356
<212> DNA
<213> Homo sapiens

<220>
<221> misc_feature
<222> 7, 346, 350, 353
<223> n = A,T,C or G

<400> 78
ttggggnttt mgagcggccg cccgggcagg taccggggtg gtcagcgagg agccattcac 60
actgaacttc accatcaaca acctgcggta tgaggagaac atgcagcacc ctggctccag 120
gaagttcaac accacggaga gggtccttca gggcctgctc aggtccctgt tcaagagcac 180
cagtgttggc cctctgtact ctggctgcag actgactttg ctcagacttg agaaacatgg 240
ggcagccact ggagtggacg ccatctgcac cctccgcctt gatcccactg gtcctggact 300
ggacagagag cggctatact gggagctgag ccagtcctct ggcggngacn ccnctt 356

<210> 79
<211> 226
<212> DNA
<213> Homo sapiens

<400> 79
agcgtggtcg cggccgaggt ccagtcgcag catgctcttt ctcctgccca ctggcacagt 60
gaggaagatc tctgctgtca gtgagaaggc tgtcatccac tgagatggca gtcaaaagtg 120
catttaatac acctaacgta tcgaacatca tagcttggcc caggttatct catatgtgct 180
cagaacactt acaatagcct gcagacctgc ccgggcggcc gctcga 226

<210> 80
<211> 444
<212> DNA
<213> Homo sapiens

<220>
<221> misc_feature

<222> 23
<223> n = A,T,C or G

<400> 80
tgtggtgttg aacttcctgg agncagggtg acccatgtcc tccccatact gcaggttggt 60
gatggtgaag ttgagggtga atggtaccag gagagggcca gcagccataa ttgtsgrgck 120
gsmgmssgag gmwggwgtyy cwgaggttcy rarrtccact gtggaggtcc caggagtgct 180
ggtggtgggc acagagstcy gatgggtgaa accattgaca tagagactgt tcctgtccag 240
ggtgtagggg cccagctctt yratgycatt ggycagttkg ctyagctccc agtacagccr 300
ctctckgyyg mgwccagsgc ttttggggtc aagatgatgg atgcagatgg catccactcc 360
agtggctgct ccatccttct cggacctgag agaggtcagt ctgcagccag agtacagagg 420
gccaacactg gtgttctttg aata 444

<210> 81
<211> 310
<212> DNA
<213> Homo sapiens

<400> 81
tcgagcggcc gcccgggcag gtcaggaagc acattggtct tagagccact gcctcctgga 60
ttccacctgt gctgcggaca tctccaggga gtgcagaagg gaagcaggtc aaactgctca 120
gatcagtcag actggctgtt ctcagttctc acctgagcaa ggtcagtctg cagccagagt 180
acagagggcc aacactggtg ttcttgaaca agggcttgag cagaccctgc agaaccctct 240
tccgtggtgt tgaacttcct ggaaaccagg gtgttgcatg tttttcctca taatgcaagg 300
ttggtgatgg 310

<210> 82
<211> 571
<212> DNA
<213> Homo sapiens

<220>
<221> misc_feature
<222> 202
<223> n = A,T,C or G

<400> 82
acggtttcaa tggacacttt tattgtttac ttaatggatc atcaattttg tctcactacc 60
tacaaatgga atttcatctt gtttccatgc tgagtagtga aacagtgaca aagctaatca 120
taataaccta catcaaaaga gaactaagct aacactgctc actttctttt taacaggcaa 180
aatataaata tatgcactct anaatgcaca atggtttagt cactaaaaaa ttcaaatggg 240
atcttgaaga atgtatgcaa atccagggtg cagtgaagat gagctgagat gctgtgcaac 300
tgtttaaggg ttcctggcac tgcatctctt ggccactagc tgaatcttga catggaaggt 360
tttagctaat gccaagtgga gatgcagaaa atgctaagtt gacttagggg ctgtgcacag 420
gaactaaaag gcaggaaagt actaaatatt gctgagagca tccaccccag gaaggacttt 480
accttccagg agctccaaac tggcaccacc cccagtgctc acatggctga ctttatcctc 540
cgtgttccat ttggcacagc aagtggcagt g 571

<210> 83
<211> 551
<212> DNA
<213> Homo sapiens

<400> 83
aaggctggtg ggtttttgat cctgctggag aacctccgct ttcatgtgga ggaagaaggg 60
aagggaaaag atgcttctgg gaacaaggtt aaagccgagc cagccaaaat agaagctttc 120
cgagcttcac tttccaagct aggggatgtc tatgtcaatg atgcttttgg cactgctcac 180

agagcccaca gctccatggt aggagtcaat ctgccacaga aggctggtgg gtttttgatg 240
aagaaggagc tgaactactt tgcaaaggcc ttggagagcc cagagcgacc cttcctggcc 300
atcctgggcg gagctaaagt tgcagacaag atccagctca tcaataatat gctggacaaa 360
gtcaatgaga tgattattgg tggtggaatg gcttttacct tccttaaggt gctcaacaac 420
atggagattg gcacttctct gtttgatgaa gagggagcca agattgtcaa agacctaatg 480
tccaaagctg agaagaatgg tgtgaagatt accttgcctg ttgactttgt cactgctgac 540
aagtttgatg a 551

<210> 84
<211> 571
<212> DNA
<213> Homo sapiens

<400> 84
tttgttcctt acatttttct aaagagttac ttaaatcagt caactggtct ttgagactct 60
taagttctga ttccaactta gctaattcat tctgagaact gtggtatagg tggcgtgtct 120
cttctagctg ggacaaaagt tctttgtttt ccccctgtag agtatcacag accttctgct 180
gaagctggac ctctgtctgg gccttggact cccaaatctg cttgtcatgt tcaagcctgg 240
aaatgttaat ctttaattct tccatatgga tggacatctg tctaagttga tcctttagaa 300
cactgcaatt atcttctttg agtctaattt cttcttcttt gctttgaatc gcatcactaa 360
acttcctctc ccatttctta gcttcatcta tcaccctgtc acgatcatcc tggagggaag 420
acatgctctt agtaaaggct gcaagctggg tcacagtact gtccaagttt tcctgaagtt 480
gctgaacttc cttgtctttc ttgttcaaag taacctgaat ctctccaatt gtctcttcca 540
agtggacttt ttctctgcgc aaagcatcca g 571

<210> 85
<211> 561
<212> DNA
<213> Homo sapiens

<400> 85
tcattgcctg tgatggcatc tggaatgtga tgagcagcca ggaagttgta gatttcattc 60
aatcaaagga ttcagcatgt ggtggaagct gtgaggcaag agaaacaaga actgtatggc 120
aagttaagaa gcacagaggc aaacaagaag gagacagaaa agcagttgca ggaagctgag 180
caagaaatgg aggaaatgaa agaaaagatg agaaagtttg ctaaatctaa acagcagaaa 240
atcctagagc tggaagaaga gaatgaccgg cttagggcag aggtgcaccc tgcaggagat 300
acagctaaag agtgtatgga aacacttctt tcttccaatg ccagcatgaa ggaagaactt 360
gaaagggtca aaatggagta tgaaaccctt tctaagaagt ttcagtcttt aatgtctgag 420
aaagactctc taagtgaaga ggttcaagat ttaaagcatc agatagaagg taatgtatct 480
aaacaagcta acctagaggc caccgagaaa catgataacc aaacgaatgt cactgaagag 540
ggaacacagt ctataccagg t 561

<210> 86
<211> 795
<212> DNA
<213> Homo sapiens

<400> 86
aagccaataa tcaccattta ttacttaata tatgccaacc actgtacttg gcagttcaca 60
aattctcacc gttacaacaa ccccatgagg tatttattcc cattctatag atagggaaac 120
cacagctcaa gtaagttagg aaactgagcc aagtatacac agaatacgaa gtggcaaaac 180
tagaaggaaa gactgacact gctatctgct ggcctccagt gtcctggctc ttttcacacg 240
ggttcaatgt ctccagcgct gctgctgctg ctgcattacc atgccctcat tgtttttctt 300
cctctggtgt tcaactgcat ccttcaaaga atctaactca ttccagagac cacttatttc 360
tttctctctt tctgaaatta cttttaataa ttcttcatga gggggaaaag aagatgcctg 420
ttggtagttt tgttgtttaa gctgctcaat ttgggactta aacaatttgt tttcatcttg 480
tacatcctgt aacagctgtg ttttgctaga aagatcactc tccctctctt ttagcatggc 540

ttctaacctc ttcaattcat tttccttttc tttcaacaca atctcaagtt cttcaaactg 600
tgatgcagaa gaggcctctt tcaagttatg ttgtgctact tcctgaacat gtgcttttaa 660
agattcattt tcttcttgaa gatcctgtaa ccacttccct gtattggcta ggtctttctc 720
tttctcttcc aaaacagcct tcatggtatt catctgttcc tcttttcctt ttaataagtt 780
caggagcttc agaac 795

<210> 87
<211> 594
<212> DNA
<213> Homo sapiens

<400> 87
caagcttttt tttttttttt aaaaagtgtt agcattaatg ttttattgtc acgcagatgg 60
caactgggtt tatgtcttca tattttatat ttttgtaaat taaaaaaatt acaagtttta 120
aatagccaat ggctggttat attttcagaa aacatgatta gactaattca ttaatggtgg 180
cttcaagctt ttccttattg gctccagaaa attcacccac cttttgtccc ttcttaaaaa 240
actggaatgt tggcatgcat ttgacttcac actctgaagc aacatcctga cagtcatcca 300
catctacttc aaggaatatc acgttggaat acttttcaga gagggaatga aagaaaggct 360
tgatcatttt gcaaggccca caccacgtgg ctgagaagtc aactactaca agtttatcac 420
ctgcagcgtc caaggcttcc tgaaaagcag tcttgctctc gatctgcttc accatcttgg 480
ctgctggagt ctgacgagcg gctgtaagga ccgatggaaa tggatccaaa gcaccaaaca 540
gagcttcaag actcgctgct tggcttgaat tcggatccga tatcgccatg gcct 594

<210> 88
<211> 557
<212> DNA
<213> Homo sapiens

<400> 88
aagtgttagc attaatgttt tattgtcacg cagatggcaa ctgggtttat gtcttcatat 60
tttatatttt tgtaaattaa aaaaattmca agttttaaat agccaatggc tggttatatt 120
ttcagaaaac atgattagac taattcatta atggtggctt caagcttttc cttattggct 180
ccagaaaatt cacccacctt ttgtcccttc ttaaaaaact ggaatgttgg catgcatttg 240
acttcacact ctgaagcaac atcctgacag tcatccacat ctacttcaag gaatatcacg 300
ttggaatact tttcagagag ggaatgaaag aaaggcttga tcattttgca aggcccacac 360
cacgtggctg agaagtcaac tactacaagt ttatcacctg cagcgtccaa ggcttcctga 420
aaagcagtct tgctctcgat ctgcttcacc atcttggctg ctggagtctg acgagcggct 480
gtaaggaccg atggaaatgg atccaaagca ccaaacagag cttcaagact cgctgcttgg 540
catgaattcg gatccga 557

<210> 89
<211> 561
<212> DNA
<213> Homo sapiens

<220>
<221> misc_feature
<222> 544, 551
<223> n = A,T,C or G

<400> 89
tacaaacttt attgaaacgc acacgcgcac acacacaaac acccctgtgg atagggaaaa 60
gcacctggcc acagggtcca ctgaaacggg gaggggatgg cagcttgtaa tgtggctttt 120
gccacaaccc ccttctgaca gggaaggcct tagattgagg ccccacctcc catggtgatg 180
gggagctcag aatggggtcc agggagaatt tggttagggg gaggtgctag ggaggcatga 240
gcagagggca ccctccgagt ggggtcccga gggctgcaga gtcttcagta ctgtccctca 300
cagcagctgt ctcaaggctg ggtccctcaa aggggcgtcc cagcgcgggg cctccctgcg 360

caaacacttg gtacccctgg ctgcgcagcg gaagccagca ggacagcagt ggcgccgatc 420
agcacaacag acgccctggc ggtagggaca gcaggcccag ccctgtcggt tgtctcggca 480
gcaggtctgg ttatcatggc agaagtgtcc ttcccacact tcacgtcctt cacacccacg 540
tganggctac nggccaggaa g 561

<210> 90
<211> 561
<212> DNA
<213> Homo sapiens

<400> 90
cccgtgggtg ccatccacgg agttgttacc tgatctttgg aagcaggatc gcccgtctgc 60
actgcagtgg aagccccgtg ggcagcagtg atggccatcc ccgcatgcca cggcctctgg 120
gaaggggcag caactggaag tccctgagac ggtaaagatg caggagtggc cggcagagca 180
gtgggcatca acctggcagg ggccacccag atgcctgctc agtgttgtgg gccatttgtc 240
cagaagggga cggcagcagc tgtagctggc tcctccgggg tccaggcagc aggccacagg 300
gcagaactga ccatctgggc accgcgttcc agccaccagc cctgctgtta aggccaccca 360
gctcaccagg gtccacatgg tctgcctgcg tccgactccg cggtccttgg gccctgatgg 420
ttctacctgc tgtgagctgc ccagtgggaa gtatggctgc tgccaatgcc caacgccacc 480
tgctgctccg atcacctgca ctgctgcccc aagacactgt gtgtgacctg atccagagta 540
agtgcctctc caaggagaac g 561

<210> 91
<211> 541
<212> DNA
<213> Homo sapiens

<220>
<221> misc_feature
<222> 480, 491
<223> n = A,T,C or G

<400> 91
gaatcacctt tctggtttag ctagtacttt gtacagaaca atgaggtttc ccacagcgga 60
gtctccctgg gctctgtttg gctctcggta aggcaggcct acaccttttc ctctcctcta 120
tggagagggg aatatgcatt aaggtgaaaa gtcaccttcc aaaagtgaga aagggattcg 180
attgctgctt caggactgtg gaattatttg gaatgtttta caaatggttg ctacaaaaca 240
acaaaaaagg taattacaaa atgtgtacat cacaacatgc tttttaaaga cattatgcat 300
tgtgctcaca ttcccttaaa tgttgtttcc aaaggtgctc agcctctagc ccagctggat 360
tctccgggaa gaggcagaga cagtttggcg aaaaagacac agggaaggag ggggtggtga 420
aaggagaaag cagccttcca gttaaagatc agccctcagt taaaggtcag cttcccgcan 480
gctggcctca ngcggagtct gggtcagagg gaggagcagc agcaggtggg gactgggcgg 540
t 541

<210> 92
<211> 551
<212> DNA
<213> Homo sapiens

<400> 92
aaccggagcg cgagcagtag ctgggtgggc accatggctg ggatcaccac catcgaggcg 60
gtgaagcgca agatccaggt tctgcagcag caggcagatg atgcagagga gcgagctgag 120
cgcctccagc gagaagttga gggagaaagg cgggcccggg aacaggctga ggctgaggtg 180
gcctccttga accgtaggat ccagctggtt gaagaagagc tggaccgtgc tcaggagcgc 240
ctggccactg ccctgcaaaa gctggaagaa gctgaaaaag ctgctgatga gagtgagaga 300
ggtatgaagg ttattgaaaa ccgggcctta aaagatgaag aaaagatgga actccaggaa 360
atccaactca aagaagctaa gcacattgca gaagaggcag ataggaagta tgaagaggtg 420

gctcgtaagt tggtgatcat tgaaggagac ttggaacgca cagaggaacg agctgagctg 480
gcagagtccc gttgccgaga gatggatgag cagattagac tgatggacca gaacctgaag 540
tgtctgagtg c 551

<210> 93
<211> 531
<212> DNA
<213> Homo sapiens

<400> 93
gagaacttgg cctttattgt gggcccagga gggcacaaag gtcaggaggc ccaagggagg 60
gatctggttt tctggatagc caggtcatag catgggtatc agtaggaatc cgctgtagct 120
gcacaggcct cacttgctgc agttccgggg agaacacctg cactgcatgg cgttgatgac 180
ctcgtggtac acgacagagc cattggtgca gtgcaagggc acgcgcatgg gctccgtcct 240
cgagggcagg cagcaggagc attgctcctg cacatcctcg atgtcaatgg agtacacagc 300
tttgctggca cactttccct ggcagtaatg aatgtccact tcctcttggg acttacaatc 360
tcccactttg atgtactgca ccttggctgt gatgtctttg caatcaggct cctcacatgt 420
gtcacagcag gtgcctggaa ttttcacgat tttgcctcct tcagccagac acttgtgttc 480
atcaaatggt gggcagcccg tgaccctctt ctcccagatg tactctcctc t 531

<210> 94
<211> 531
<212> DNA
<213> Homo sapiens

<220>
<221> misc_feature
<222> 517
<223> n = A,T,C or G

<400> 94
gcctggacct tgccggatca gtgccacaca gtgacttgct tggcaaatgg ccagaccttg 60
ctgcagagtc atcgtgtcaa ttgtgaccat ggaccccggc cttcatgtgc caacagccag 120
tctcctgttc gggtggagga gacgtgtggc tgccgctgga cctgcccttg tgtgtgcacg 180
ggcagttcca ctcggcacat cgtcaccttc gatgggcaga atttcaagct tactggtagc 240
tgctcctatg tcatctttca aaacaaggag caggacctgg aagtgctcct ccacaatggg 300
gcctgcagcc ccggggcaaa acaagcctgc atgaagtcca ttgagattaa gcatgctggc 360
gtctctgctg agctgcacag taacatggag atggcagtgg atgggagact ggtccttgcc 420
ccgtacgttg gtgaaaacat ggaagtcagc atctacggcg ctatcatgta tgaagtcagg 480
tttacccatc ttggccacat cctcacatac accgccncaa aacaacgagt t 531

<210> 95
<211> 605
<212> DNA
<213> Homo sapiens

<400> 95
agatcaacct ctgctggtca ggaggaatgc cttccttgtc ttggatcttt gctttgacgt 60
tctcgatagt rwcaactkkr ytsramskma agkgyratgr wmttksywgw rasyktmwwm 120
rsgraraytt agacaycccm cctcwgagac gsagkaccar gtgcagaggt ggactctttc 180
tggatgttgt agtcagacag ggtgcgtcca tcttccagct gtttcccagc aaagatcaac 240
ctctgctgat caggagggat gccttcctta tcttggatct ttgccttgac attctcgatg 300
gtgtcactgg gctccacctc gagggtgatg gtcttaccag tcagggtctt cacgaagaty 360
tgcatcccac ctctgagacg gagcaccagg tgcagggtrg actctttctg gatgttgtag 420
tcagacaggg tgcgyccatc ttccagctgc tttccsagca aagatcaacc tctgctggtc 480
aggaggratg ccttccttgt cytggatctt tgcyttgacr ttctcratgg tgtcactcgg 540
ctccacttcg agagtgatgg tcttaccagt cagggtcttc acgaagatct gcatcccacc 600

tctaa 605

<210> 96
<211> 531
<212> DNA
<213> Homo sapiens

<400> 96
aagtcacaaa cagacaaaga ttattaccag ctgcaagcta tattagaagc tgaacgaaga 60
gacagaggtc atgattctga gatgattgga gaccttcaag ctcgaattac atctttacaa 120
gaggaggtga agcatctcaa acataatctc gaaaaagtgg aaggagaaag aaaagaggct 180
caagacatgc ttaatcactc agaaaaggaa aagaataatt tagagataga tttaaactac 240
aaacttaaat cattacaaca acggttagaa caagaggtaa atgaacacaa agtaaccaaa 300
gctcgtttaa ctgacaaaca tcaatctatt gaagaggcaa agtctgtggc aatgtgtgag 360
atggaaaaaa agctgaaaga agaaagagaa gctcgagaga aggctgaaaa tcgggttgtt 420
cagattgaga aacagtgttc catgctagac gttgatctga agcaatctca gcagaaacta 480
gaacatttga ctggaaataa agaaaggatg gaggatgaag ttaagaatct a 531

<210> 97
<211> 1017
<212> DNA
<213> Homo sapiens

<220>
<221> misc_feature
<222> 963, 995, 1001, 1008, 1010
<223> n = A,T,C or G

<400> 97
cgcctccacc atgtccatca gggtgaccca gaagtcctac aaggtgtcca cctctggccc 60
ccgggccttc agcagccgct cctacacgag tgggcccggt tcccgcatca gctcctcgag 120
cttctcccga gtgggcagca gcaactttcg cggtggcctg ggcggcggct atggtggggc 180
cagcggcatg ggaggcatca ccgcagttac ggtcaaccag agcctgctga gcccccttgt 240
cctggaggtg gaccccaaca tccaggccgt gcgcacccag gagaaggagc agatcaagac 300
cctcaacaac aagtttgcct ccttcataga caaggtacgg ttcctggagc agcagaacaa 360
gatgctggag accaagtgga gcctcctgca gcagcagaag acggctcgaa gcaacatgga 420
caacatgttc gagagctaca tcaacarcct taggcggcag ctggagactc tgggccagga 480
gaagctgaag ctggaggcgg agcttggcaa catgcagggg ctggtggagg acttcaagaa 540
caagtatgag gatgagatca ataagcgtac agagatggag aacgaatttg tcctcatcaa 600
gaaggatgtg gatgaagctt acatgaacaa ggtagagctg gagtctcgcc tggaagggct 660
gaccgacgag atcaacttcc tcaggcagct gtatgaagag gagatccggg agctgcagtc 720
ccagatctcg gacacatctg tggtgctgtc catggacaac agccgctccc tggacatgga 780
cagcatcatt gctgaggtca aggcacagta cgaggatatt gccaaccgca gccgggctga 840
ggctgagagc atgtaccagg tcaagtatga ggagctgcag agcctggctg ggaagcacgg 900
ggatgacctg cggcgcacaa agactgagat ctctgagatg aacccggaac atcagcccgg 960
ctncaggctg agattgaggg cctcaaaggc caganggctt ncctggangn ccgccat 1017

<210> 98
<211> 561
<212> DNA
<213> Homo sapiens

<400> 98
cccggagcca gccaacgagc ggaaaatggc agacaatttt tcgctccatg atgcgttatc 60
tgggtctgga aacccaaacc ctcaaggatg gcctggcgca tgggggaacc agcctgctgg 120
ggcagggggc tacccagggg cttcctatcc tggggcctac cccgggcagg cacccccagg 180
ggcttatcct ggacaggcac ctccaggcgc ctaccctgga gcacctggag cttatcccgg 240

agcacctgca cctggagtct acccagggcc acccagcggc cctggggcct acccatcttc 300
tggacagcca agtgccaccg gagcctaccc tgccactggc ccctatggcg cccctgctgg 360
gccactgatt gtgccttata acctgccttt gcctggggga gtggtgcctc gcatgctgat 420
aacaattctg ggcacggtga agcccaatgc aaacagaatt gctttagatt tccaaagagg 480
gaatgatgtt gccttccact ttaacccacg cttcaatgag aacaacagga gagtcattgg 540
ttgcaataca aagctggata a 561

<210> 99
<211> 636
<212> DNA
<213> Homo sapiens

<400> 99
gggaatgcaa caactttatt gaaaggaaag tgcaatgaaa tttgttgaaa ccttaaaagg 60
ggaaacttag acaccccccc tcragcgmag kaccargtgc araggtggac tctttctgga 120
tgttgtagtc agacagggtr cgwccatctt ccagctgttt yccrgcaaag atcaacctct 180
gctgatcagg aggratgcct tccttatctt ggatctttgc cttgacattc tcgatggtgt 240
cactgggctc cacctcgagg gtgatggtct taccagtcag ggtcttcacg aagatytgca 300
tcccacctct gagacggagc accaggtgca gggtrgactc tttctggatg ttgtagtcag 360
acagggtgcg yccatcttcc agctgctttc csagcaaaga tcaacctctg ctggtcagga 420
ggratgcctt ccttgtcytg gatctttgcy ttgacrttct caatggtgtc actcggctcc 480
acttcgagag tgatggtctt accagtcagg gtcttcacga agatctgcat cccacctcta 540
agacggagca ccaggtgcag ggtggactct ttctggatgg ttgtagtcag acagggtgcg 600
tccatcttcc agctgtttcc cagcaaagat caacct 636

<210> 100
<211> 697
<212> DNA
<213> Homo sapiens

<400> 100
aggttgatct ttgctgggaa acagctggaa gatggacgca ccctgtctga ctacaaccat 60
ccagaaagag tccaccctgc acctggtgct ccgtcttaga ggtgggatgc agatcttcgt 120
gaagaccctg actggtaaga ccatcactct cgaagtggag ccgagtgaca ccattgagaa 180
ygtcaargca aagatccarg acaaggaagg catycctcct gaccagcaga ggttgatctt 240
tgctsggaaa gcagctggaa gatggrcgca ccctgtctga ctacaacatc cagaaagagt 300
cyaccctgca cctggtgctc cgtctcagag gtgggatgca ratcttcgtg aagaccctga 360
ctggtaagac catcaccctc gaggtggagc ccagtgacac catcgagaat gtcaaggcaa 420
agatccaaga taaggaaggc atccctcctg atcagcagag gttgatcttt gctgggaaac 480
agctggaaga tggacgcacc ctgtctgact acaacatcca gaaagagtcc acctytgcac 540
ytggtmctbc gtctyagagg kgggrtgcaa atctwmgtkw agacactcac tkkyaagryy 600
atcamcmwtg akktcgakys castkwcact wtcrakaamg tyrwwgcawa gatccmagac 660
aaggaaggca ttcctcctga ccagcagagg ttgatct 697

<210> 101
<211> 451
<212> DNA
<213> Homo sapiens

<400> 101
atggagtctc actctgtcga ccaggctgga gcgctgtggt gcgatatcgg ctcactgcag 60
tctccacttc ctgggttcaa gcgatcctcc tgcctcagcc tcccgagtag ctgggactac 120
aggcaggcgt caccataatt tttgtatttt tagtagagac atggtttcgc catgttggct 180
gggctggtct cgaactcctg acctcaagtg atctgtcctg gcctcccaaa gtgttgggat 240
tacaggcgaa agccaacgct cccggccagg gaacaacttt agaatgaagg aaatatgcaa 300
aagaacatca catcaaggat caattaatta ccatctatta attactatat gtgggtaatt 360
atgactattt cccaagcatt ctacgttgac tgcttgagaa gatgtttgtc ctgcatggtg 420

gagagtggag aagggccagg attcttaggt t                                451

<210> 102
<211> 571
<212> DNA
<213> Homo sapiens

<400> 102
agcgcggtct tccggcgcga gaaagctgaa ggtgatgtgg ccgccctcaa ccgacgcatc 60
cagctcgttg aggaggagtt ggacagggct caggaacgac tggccacggc cctgcagaag 120
ctggaggagg cagaaaaagc tgcagatgag agtgagagag gaatgaaggt gatagaaaac 180
cgggccatga aggatgagga gaagatggag attcaggaga tgcagctcaa agaggccaag 240
cacattgcgg aagaggctga ccgcaaatac gaggaggtag ctcgtaagct ggtcatcctg 300
gagggtgagc tggagagggc agaggagcgt gcggaggtgt ctgaactaaa atgtggtgac 360
ctggaagaag aactcaagaa tgttactaac aatctgaaat ctctggaggc tgcatctgaa 420
aagtattctg aaaaggagga caaatatgaa gaagaaatta aacttctgtc tgacaaactg 480
aaagaggctg agacccgtgc tgaatttgca gagagaacgg ttgcaaaact ggaaaagaca 540
attgatgacc tggaagagaa acttgcccag c                                571

<210> 103
<211> 451
<212> DNA
<213> Homo sapiens

<400> 103
gtgcacaggt cccatttatt gtagaaaata ataataatta cagtgatgaa tagctcttct 60
taaattacaa aacagaaacc acaaagaagg aagaggaaaa accccaggac ttccaagggt 120
gaagctgtcc cctcctccct gccaccctcc caggctcatt agtgtccttg gaaggggcag 180
aggactcaga ggggatcagt ctccaggggc cctgggctga agcgggtgag gcagagagtc 240
ctgaggccac agagctgggc aacctgagcc gcctctctgg ccccctcccc caccactgcc 300
caaacctgtt tacagcacct tcgcccctcc cctctaaacc cgtccatcca ctctgcactt 360
cccaggcagg tgggtgggcc aggcctcagc catactcctg ggcgcgggtt tcggtgagca 420
aggcacagtc ccagaggtga tatcaaggcc t                                451

<210> 104
<211> 441
<212> DNA
<213> Homo sapiens

<400> 104
gcaaggaact ggtctgctca cacttgctgg cttgcgcatc aggactggct ttatctcctg 60
actcacggtg caaaggtgca ctctgcgaac gttaagtccg tccccagcgc ttggaatcct 120
acggccccca cagccggatc ccctcagcct tccaggtcct caactcccgt ggacgctgaa 180
caatggcctc catggggcta caggtaatgg gcatcgcgct ggccgtcctg ggctggctgg 240
ccgtcatgct gtgctgcgcg ctgcccatgt ggcgcgtgac ggccttcatc ggcagcaaca 300
ttgtcacctc gcagaccatc tgggagggcc tatggatgaa ctgcgtggtg cagagcaccg 360
gccagatgca gtgcaaggtg tacgactcgc tgctggcact gccgcaggac ctgcaggcgg 420
cccgcgccct cgtcatcatc a                                           441

<210> 105
<211> 509
<212> DNA
<213> Homo sapiens

<220>
<221> misc_feature
<222> 195

<223> n = A,T,C or G

<400> 105
tgcaaaaggg acacaggggt tcaaaaataa aaatttctct tccccctccc caaacctgta 60
ccccagctcc ccgaccacaa cccccttcct cccccgggga aagcaagaag gagcaggtgt 120
ggcatctgca gctgggaaga gagaggccgg ggaggtgccg agctcggtgc tggtctcttt 180
ccaaatataa atacntgtgt cagaactgga aaatcctcca gcacccacca cccaagcact 240
ctccgttttc tgccggtgtt tggagagggg cggggggcag gggcgccagg caccggctgg 300
ctgcggtcta ctgcatccgc tgggtgtgca ccccgcgagc ctcctgctgc tcattgtaga 360
agagatgaca ctcggggtcc ccccggatgg tgggggctcc ctggatcagc ttcccggtgt 420
tggggttcac acaccagcac tccccacgct gcccgttcag agacatcttg cactgtttga 480
ggttgtacag gccatgcttg tcacagttg 509

<210> 106
<211> 571
<212> DNA
<213> Homo sapiens

<400> 106
gggttggagg gactggttct ttatttcaaa aagacacttg tcaatattca gtatcaaaac 60
agttgcacta ttgatttctc tttctcccaa tcggccccaa agagaccaca taaaaggaga 120
gtacatttta agccaataag ctgcaggatg tacacctaac agacctccta gaaaccttac 180
cagaaaatgg ggactgggta gggaaggaaa cttaaaagat caacaaactg ccagcccacg 240
gactgcagag gctgtcacag ccagatgggg tggccagggt gccacaaacc caaagcaaag 300
tttcaaaata atataaaatt taaaaagttt tgtacataag ctattcaaga tttctccagc 360
actgactgat acaaagcaca attgagatgg cacttctaga gacagcagct tcaaacccag 420
aaaagggtga tgagatgagt ttcacatggc taaatcagtg gcaaaaacac agtcttcttt 480
ctttctttct ttcaaggagg caggaaagca attaagtggt cacctcaaca taagggggac 540
atgatccatt ctgtaagcag ttgtgaaggg g 571

<210> 107
<211> 555
<212> DNA
<213> Homo sapiens

<400> 107
caggaaccgg agcgcgagca gtagctgggt gggcaccatg gctgggatca ccaccatcga 60
ggcggtgaag cgcaagatcc aggttctgca gcagcaggca gatgatgcag aggagcgagc 120
tgagcgcctc cagcgagaag ttgagggaga aaggcgggcc cgggaacagg ctgaggctga 180
ggtggcctcc ttgaaccgta ggatccagct ggttgaagaa gagctggacc gtgctcagga 240
gcgcctggcc actgccctgc aaaagctgga agaagctgaa aaagctgctg atgagagtga 300
gagaggtatg aaggttattg aaaaccgggc cttaaaagat gaagaaaaga tggaactcca 360
ggaaatccaa ctcaaagaag ctaagcacat tgcagaagag gcagatagga agtatgaaga 420
ggtggctcgt aagttggtga tcattgaagg agacttggaa cgcacagagg aacgagctga 480
gctggcagag tcccgttgcc gagagatgga tgagcagatt agactgatgg accagaacct 540
gaagtgtctg agtgc 555

<210> 108
<211> 541
<212> DNA
<213> Homo sapiens

<400> 108
   atctacgtca tcaatcaggc tggagacacc atgttcaatc gagctaagct gctcaatatt 60
   ggctttcaag aggccttgaa ggactatgat tacaactgct ttgtgttcag tgatgtggac 120
   ctcattccga tggacgaccg taatgcctac aggtgttttt cgcagccacg gcacatttct 180
   gttgcaatgg acaagttcgg gtttagcctg ccatatgttc agtattttgg aggtgtctct 240
   gctctcagta aacaacagtt tcttgccatc aatggattcc ctaataatta ttggggttgg 300
   ggaggagaag atgacgacat ttttaacaga ttagttcata aaggcatgtc tatatcacgt 360
   ccaaatgctg tagtagggag gtgtcgaatg atccggcatt caagagacaa gaaaaatgag 420
   cccaatcctc agaggtttga ccggatcgca catacaaagg aaacgatgcg cttcgatggt 480
   ttgaactcac ttacctacaa ggtgttggat gtcagagata cccgttatat acccaaatca 540
   c 541

<210> 109
<211> 411
<212> DNA
<213> Homo sapiens

<400> 109
ctagacctct aattaaaagg cacaatcatg ctggagaatg aacagtctga ccccgagggc 60
cacagcgaat tttagggaag gaggcaaaga ggtgagaagg gaaaggaaag aaggaaggaa 120
ggagaacaat aagaactgga gacgttgggt gggtcaggga gtgtggtgga ggctcggaga 180
gatggtaaac aaacctgact gctatgagtt ttcaacccca tagtctaggg ccatgagggc 240
gtcagttctt ggtggctgag ggtccttcca cccagcccac ctgggggagt ggagtgggga 300
gttctgccag gtaagcagat gttgtctccc aagttcctga cccagatgtc tggcaggata 360
acgctgacct gttccctcaa caagggacct gaaagtaatt ttgctcttta c 411

<210> 110
<211> 451
<212> DNA
<213> Homo sapiens

<400> 110
ccgaattcaa gcgtcaacga tccytccctt accatcaaat caattggcca ccaatggtac 60
tgaacctacg agtacaccga ctacgggcgg actaatcttc aactcctaca tacttccccc 120
attattccta gaaccaggcg acctgcgact ccttgacgtt gacaatcgag tagtactccc 180
gattgaagcc cccattcgta taataattac atcacaagac gtcttgcact catgagctgt 240
ccccacatta ggcttaaaaa cagatgcaat tcccggacgt ctaagccaaa ccactttcac 300
cgctacacga ccgggggtat actacggtca atgctctgaa atctgtggag caaaccacag 360
tttcatgccc atcgtcctag aattaattcc cctaaaaatc tttgaaatag ggcccgtatt 420
taccctatag caccccctct accccctcta g 451

<210> 111
<211> 541
<212> DNA
<213> Homo sapiens

<400> 111
gctcttcaca cttttattgt taattctctt cacatggcag atacagagct gtcgtcttga 60
agaccaccac tgaccaggaa atgccacttt tacaaaatca tccccccttt tcatgattgg 120
aacagttttc ctgaccgtct gggagcgttg aagggtgacc agcacatttg cacatgcaaa 180
aaaggagtga ccccaaggcc tcaaccacac ttcccagagc tcaccatggg ctgcaggtga 240
cttgccaggt ttggggttcg tgagctttcc ttgctgctgc ggtggggagg ccctcaagaa 300
ctgagaggcc ggggtatgct tcatgagtgt taacatttac gggacaaaag cgcatcatta 360
ggataaggaa cagccacagc acttcatgct tgtgagggtt agctgtagga gcgggtgaaa 420
ggattccagt ttatgaaaat ttaaagcaaa caacggtttt tagctgggtg ggaaacagga 480
aaactgtgat gtcggccaat gaccaccatt tttctgccca tgtgaaggtc cccatgaaac 540
c 541

<210> 112
<211> 521
<212> DNA
<213> Homo sapiens

<400> 112
caagcgcttg gcgtttggac ccagttcagt gaggttcttg ggttttgtgc ctttggggat 60
tttggtttga cccaggggtc agccttagga aggtcttcag gaggaggccg agttcccctt 120
cagtaccacc cctctctccc cactttccct ctcccggcaa catctctggg aatcaacagc 180
atattgacac gttggagccg agcctgaaca tgcccctcgg ccccagcaca tggaaaaccc 240
ccttccttgc ctaaggtgtc tgagtttctg gctcttgagg catttccaga cttgaaattc 300
tcatcagtcc attgctcttg agtctttgca gagaacctca gatcaggtgc acctgggaga 360
aagactttgt ccccacttac agatctatct cctcccttgg gaagggcagg gaatggggac 420
ggtgtatgga ggggaaggga tctcctgcgc ccttcattgc cacacttggt gggaccatga 480
acatctttag tgtctgagct tctcaaatta ctgcaatagg a 521

<210> 113
<211> 568
<212> DNA
<213> Homo sapiens

<400> 113
agcgtcaaat cagaatggaa aagactcaaa accatcatca acaccaagat caaaaggaca 60
agratccttc aagaaacagg aaaaaactcc taaaacacca aaaggaccta gttctgtaga 120
agacattaaa gcaaaaatgc aagcaagtat agaaaaaggt ggttctcttc ccaaagtgga 180
agccaaattc atcaattatg tgaagaattg cttccggatg actgaccaag aggctattca 240
agatctctgg cagtggagga agtctcttta agaaaatagt ttaaacaatt tgttaaaaaa 300
ttttccgtct tatttcattt ctgtaacagt tgatatctgg ctgtcctttt tataatgcag 360
agtgagaact ttccctaccg tgtttgataa atgttgtcca ggttctattg ccaagaatgt 420
gttgtccaaa atgcctgttt agtttttaaa gatggaactc caccctttgc ttggttttaa 480
gtatgtatgg aatgttatga taggacatag tagtagcggt ggtcagacat ggaaatggtg 540
ggsmgacaaa aatatacatg tgaaataa 568

<210> 114
<211> 483
<212> DNA
<213> Homo sapiens

<400> 114
tccgaattcc aagcgaatta tggacaaacg attcctttta gaggattact tttttcaatt 60
tcggttttag taatctaggc tttgcctgta aagaatacaa cgatggattt taaatactgt 120
ttgtggaatg tgtttaaagg attgattcta gaacctttgt atatttgata gtatttctaa 180
ctttcatttc tttactgttt gcagttaatg ttcatgttct gctatgcaat cgtttatatg 240
cacgtttctt taattttttt agattttcct ggatgtatag tttaaacaac aaaaagtcta 300
tttaaaactg tagcagtagt ttacagttct agcaaagagg aaagttgtgg ggttaaactt 360
tgtattttct ttcttataga ggcttctaaa aaggtatttt tatatgttct ttttaacaaa 420
tattgtgtac aacctttaaa acatcaatgt ttggatcaaa acaagaccca gcttattttc 480
tgc 483

<210> 115
<211> 521
<212> DNA
<213> Homo sapiens

<400> 115
tgtggtggcg cgggctgagg tggaggccca ggactctgac cctgcccctg ccttcagcaa 60
ggcccccggc agcgccggcc actacgaact gccgtgggtt gaaaaatata ggccagtaaa 120
gctgaatgaa attgtcggga atgaagacac cgtgagcagg ctagaggtct ttgcaaggga 180
aggaaatgtg cccaacatca tcattgcggg ccctccagga accggcaaga ccacaagcat 240
tctgtgcttg gcccgggccc tgctgggccc agcactcaaa gatgccatgt tggaactcaa 300
tgcttcaaat gacaggggca ttgacgttgt gaggaataaa attaaaatgt ttgctcaaca 360

aaaagtcact cttcccaaag gccgacataa gatcatcatt ctggatgaag cagacagcat 420
gaccgacgga gcccagcaag ccttgaggag aaccatggaa atctactcta aaaccactcg 480
ttcgcccttg cttgtaatgc ttcggataag atcatcgagc c 521

<210> 116
<211> 501
<212> DNA
<213> Homo sapiens

<400> 116
ctttgcaaag cttttatttc atgtctgcgg catggaatcc acctgcacat ggcatcttag 60
ctgtgaagga gaaagcagtg cacgagaagg aatgagtggg cggaaccaac ggcctccaca 120
agctgccttc cagcagcctg ccaaggccat ggcagagaga gactgcaaac aaacacaagc 180
aaacagagtc tcttcacagc tggagtctga aagctcatag tggcatgtgt gaatctgaca 240
aaattaaaag tgtgcatagt ccattacatg cataaaacac taataataat cctgtttaca 300
cgtgactgca gcaggcaggt ccagctccac cactgccctc ctgccacatc acatcaagtg 360
ccatggttta gagggttttt catatgtaat tcttttattc tgtaaaaggt aacaaaatat 420
acagaacaaa actttccctt tttaaaacta atgttacaaa tctgtattat cacttggata 480
taaatagtat ataagctgat c 501

<210> 117
<211> 451
<212> DNA
<213> Homo sapiens

<220>
<221> misc_feature
<222> 320
<223> n = A,T,C or G

<400> 117
caaggatatt atgttgaggg tacrgrgtga cactgaacag atcacaaagc acgagaaaca 60
ttagttctct ccctccccag cgtctccttc gtctccctgg ttttccgatg tccacagagt 120
gagattgtcc ctaagtaact gcatgatcag agtgctgkct ttataagact cttcattcag 180
cgtatccaat tcagcaattg cttcatcaaa tgccgttttt gccaggctac aggccttttc 240
aggagagttt agaatctcat agtaaaagac tgagaaattt agtgccagac caagacgaat 300
tgggtgtgta ggctgcattn ctttcttact aatttcaaat gcttcctggt aagcctgctg 360
ggagttcgac acaagtggtt tgtttgttgc tccagatgcc acttcagaaa gatacctaaa 420
ataatctcct ttcattttca aagtagaaca c 451

<210> 118
<211> 501
<212> DNA
<213> Homo sapiens

<400> 118
tccggagccg gggtagtcgc cgccgccgcc gccggtgcag ccactgcagg caccgctgcc 60
gccgcctgag tagtgggctt aggaaggaag aggtcatctc gctcggagct tcgctcggaa 120
gggtctttgt tccctgcagc cctcccacgg gaatgacaat ggataaaagt gagctggtac 180
agaaagccaa actcgctgag caggctgagc gatatgatga tatggctgca gccatgaagg 240
cagtcacaga acaggggcat gaactctcca acgaagagag aaatctgctc tctgttgcct 300
acaagaatgt ggtaaggccg cccgccgctc ttcctggcgt gtcatctcca gcattgagca 360
gaaaacagag aggaatgaga agaagcagca gatgggcaaa gagtaccgtg agaagataga 420
ggcagaactg caggacatct gcaatgatgt tctggagctt gttggacaaa tatcttattc 480
caatgctaca caacccagaa a 501

<210> 119

<211> 391
<212> DNA
<213> Homo sapiens

<400> 119
aaaaagcagc argttcaaca caaaatagaa atctcaaatg taggatagaa caaaaccaag 60
tgtgtgaggg gggaagcaac agcaaaagga agaaatgaga tgttgcaaaa aagatggagg 120
agggttcccc tctcctctgg ggactgactc aaacactgat gtggcagtat acaccattcc 180
agagtcaggg gtgttcattc ttttttggga gtaagaaaag gtggggatta agaagacgtt 240
tctggaggct tagggaccaa ggctggtctc tttcccccct cccaaccccc ttgatccctt 300
tctctgatca ggggaaagga gctcgaatga gggaggtaga gttggaaagg gaaaggattc 360
cacttgacag aatgggacag actccttccc a 391

<210> 120
<211> 421
<212> DNA
<213> Homo sapiens

<220>
<221> misc_feature
<222> 409
<223> n = A,T,C or G

<400> 120
   tggcaatagc acagccatcc aggagctctt cargcgcatc tcggagcagt tcactgccat 60
   gttccgccgg aaggccttcc tccactggta cacaggcgag ggcatggacg agatggagtt 120
   caccgaggct gagagcaaca tgaacgacct cgtctctgag tatcaagcag taccaggatg 180
   ccaccgcaga agaggaggag gatttcggtg aggaggccga agaggaggcc taaggcagag 240
   cccccatcac ctcaggcttc tcagttccct tagccgtctt actcaactgc ccctttcctc 300
   tccctcagaa tttgtgtttg ctgcctctat cttgtttttt gttttttctt ctgggggggt 360
   ctagaacagt gcctggcaca tagtaggcgc tcaataaata cttggttgnt gaatgtctcc 420
   t 421

<210> 121
<211> 206
<212> DNA
<213> Homo sapiens

<400> 121
agctggcgct agggctcggt tgtgaaatac agcgtrgtca gcccttgcgc tcagtgtaga 60
aacccacgcc tgtaaggtcg gtcttcgtcc atctgctttt ttctgaaata cactaagagc 120
agccacaaaa ctgtaacctc aaggaaacca taaagcttgg agtgccttaa tttttaacca 180
gtttccaata aaacggttta ctacct 206

<210> 122
<211> 131
<212> DNA
<213> Homo sapiens

<400> 122
ggagatgaag atgaggaagc tgagtcagct acgggcargc gggcagctga agatgatgag 60
gatgacgatg tcgataccaa gaagcagaag accgacgagg atgactagac agcaaaaaag 120
gaaaagttaa a 131

<210> 123
<211> 231
<212> DNA

<213> Homo sapiens

<220>
<221> misc_feature
<222> 166, 202, 222, 225
<223> n = A,T,C or G

<400> 123
gatgaaaatt aaatacttaa attaatcaaa aggcactacg ataccaccta aaacctactg 60
cctcagtggc agtakgctaa kgaagatcaa gctacagsac atyatctaat atgaatgtta 120
gcaattacat akcargaagc atgtttgctt tccagaagac tatggnacaa tggtcattwg 180
ggcccaagag gatatttggc cnggaaagga tcaagataga tnaangtaaa g 231

<210> 124
<211> 521
<212> DNA
<213> Homo sapiens

<220>
<221> misc_feature
<222> 284, 412, 513
<223> n = A,T,C or G

<400> 124
gagtagcaac gcaaagcgct tggtattgag tctgtgggsg acttcggttc cggtctctgc 60
agcagccgtg atcgcttagt ggagtgctta gggtagttgg ccaggatgcc gaatatcaaa 120
atcttcagca ggcagctccc accaggactt atctcasaaa attgctgacc gcctgggcct 180
ggagctaggc aaggtggtga ctaagaaatt cagcaaccag gagacctgtg tggaaattgg 240
tgaaagtgta ccgtggagag gatgtctaca ttgttcagag tggntgtggc gaaatcaatg 300
acaatttaat ggagcttttg atcatgatta atgcctgcaa gattgcttca gccagccggg 360
ttactgcagt catcccatgc ttcccttatg ccccggcagg ataagaaaga tnagagccgg 420
gccgccaatc tcagccaagc ttggtgcaaa tatgctatct gtagcagtgc agatcatatt 480
atcaccatgg acctacatgc ttctcaaatt canggctttt t 521

<210> 125
<211> 341
<212> DNA
<213> Homo sapiens

<220>
<221> misc_feature
<222> 277
<223> n = A,T,C or G

<400> 125
atgcaaaagg ggacacaggg ggttcaaaaa taaaaatttc tcttccccct ccccaaacct 60
gtaccccagc tccccgacca caaccccctt cctcccccgg ggaaagcaag aaggagcagg 120
tgtggcatct gcagctggga agagagaggc cggggaggtg ccgagctcgg tgctggtctc 180
tttccaaata taaatacgtg tgtcagaact ggaaaatcct ccagcaccca ccacccaagc 240
actctccgtt ttctgccggt gtttggagag gggcggnggg caggggcgcc aggcaccggc 300
tggctgcggt ctactgcatc cgctgggtgt gcaccccgcg a 341

<210> 126
<211> 521
<212> DNA
<213> Homo sapiens

<220>
<221> misc_feature
<222> 353, 399, 455
<223> n = A,T,C or G

<400> 126
aggttggaga aggtcatgca ggtgcagatt gtccaggskc agccacaggg tcaagcccaa 60
caggcccaga gtggcactgg acagaccatg caggtgatgc agcagatcat cactaacaca 120
ggagagatcc agcagatccc ggtgcagctg aatgccggcc agctgcagta tatccgctta 180
gcccagcctg tatcaggcac tcaagttgtg cagggacaga tccagacact tgccaccaat 240
gctcaacaga ttacacagac agaggtccag caaggacagc agcagttcaa gccagttcac 300
aagatggaca gcagctctac cagatccagc aagtcaccat gcctgcgggc cangacctcg 360
ccagcccatg ttcatccagt caagccaacc agcccttcna cgggcaggcc ccccaggtga 420
ccggcgactg aagggcctga gctggcaagg ccaangacac ccaacacaat ttttgccata 480
cagcccccag gcaatgggca cagcctttct tcccagagga c 521

<210> 127
<211> 351
<212> DNA
<213> Homo sapiens

<400> 127
tgagatttat tgcatttcat gcagcttgaa gtccatgcaa aggrgactag cacagttttt 60
aatgcattta aaaaataaaa gggaggtggg cagcaaacac acaaagtcct agtttcctgg 120
gtccctggga gaaaagagtg tggcaatgaa tccacccact ctccacaggg aataaatctg 180
tctcttaaat gcaaagaatg tttccatggc ctctggatgc aaatacacag agctctgggg 240
tcagagcaag ggatggggag aggaccacga gtgaaaaagc agctacacac attcacctaa 300
ttccatctga gggcaagaac aacgtggcaa gtcttggggg tagcagctgt t 351

<210> 128
<211> 521
<212> DNA
<213> Homo sapiens

<400> 128
tccagacatg ctcctgtcct aggcggggag caggaaccag acctgctatg ggaagcagaa 60
agagttaagg gaaggtttcc tttcattcct gttccttctc ttttgctttt gaacagtttt 120
taaatatact aatagctaag tcatttgcca gccaggtccc ggtgaacagt agagaacaag 180
gagcttgcta agaattaatt ttgctgtttt tcaccccatt caaacagagc tgccctgttc 240
cctgatggag ttccattcct gccagggcac ggctgagtaa cacgaagcca ttcaagaaag 300
gcgggtgtga aatcactgcc accccatgga cagacccctc actcttcctt cttagccgca 360
gcgctactta ataaatatat ttatactttg aaattatgat aaccgatttt tcccatgcgg 420
catcctaagg gcacttgcca gctcttatcc ggacagtcaa gcactgttgt tggacaacag 480
ataaaggaaa agaaaaagaa gaaaacaacc gcaacttctg t 521

<210> 129
<211> 521
<212> DNA
<213> Homo sapiens

<400> 129
   tgagacggac cactggcctg gtcccccctc atktgctgtc gtaggacctg acatgaaacg 60
   cagatctagt ggcagagagg aagatgatga ggaacttctg agacgtcggc agcttcaaga 120
   agagcaatta atgaagctta actcaggcct gggacagttg atcttgaaag aagagatgga 180
   gaaagagagc cgggaaaggt catctctgtt agccagtcgc tacgattctc ccatcaactc 240
   agcttcacat attccatcat ctaaaactgc atctctccct ggctatggaa gaaatgggct 300
   tcaccggcct gtttctaccg acttcgctca gtataacagc tatggggatg tcagcggggg 360
   agtgcgagat taccagacac ttccagatgg ccacatgcct gcaatgagaa tggaccgagg 420
   agtgtctatg cccaacatgt tggaaccaaa gatatttcca tatgaaatgc tcatggtgac 480
   caacagaggg ccgaaaccaa atctcagaga ggtggacaga a 521

<210> 130
<211> 270
<212> DNA
<213> Homo sapiens

<400> 130
tcactttatt tttcttgtat aaaaacccta tgttgtagcc acagctggag cctgagtccg 60
ctgcacggag actctggtgt gggtcttgac gaggtggtca gtgaactcct gatagggaga 120
cttggtgaat acagtctcct tccagaggtc gggggtcagg tagctgtagg tcttagaaat 180
ggcatcaaag gtggccttgg cgaagttgcc cagggtggca gtgcagcccc gggctgaggt 240
gtagcagtca tcgataccag ccatcatgag 270

<210> 131
<211> 341
<212> DNA
<213> Homo sapiens

<400> 131
ctggaatata gacccgtgat cgacaaaact ttgaacgagg ctgactgtgc caccgtcccg 60
ccagccattc gctcctactg atgagacaag atgtggtgat gacagaatca gcttttgtaa 120
ttatgtataa tagctcatgc atgtgtccat gtcataactg tcttcatacg cttctgcact 180
ctggggaaga aggagtacat tgaagggaga ttggcaccta gtggctggga gcttgccagg 240
aacccagtgg ccagggagcg tggcacttac ctttgtccct tgcttcattc ttgtgagatg 300
ataaaactgg gcacagctct taaataaaat ataaatgaac a 341

<210> 132
<211> 844
<212> DNA
<213> Homo sapiens

<220>
<221> misc_feature
<222> 37
<223> n = A,T,C or G

<400> 132
tgaatgggga ggagctgacc caggaaatgg agcttgngga gaccaggcct gcaggggatg 60
gaaccttcca gaagtgggca tctgtggtgg tgcctcttgg gaaggagcag aagtacacat 120
gccatgtgga acatgagggg ctgcctgagc ccctcaccct gagatggggc aaggaggagc 180
ctccttcatc caccaagact aacacagtaa tcattgctgt tccggttgtc cttggagctg 240
tggtcatcct tggagctgtg atggcttttg tgatgaagag gaggagaaac acaggtggaa 300
aaggagggga ctatgctctg gctccaggct cccagagctc tgatatgtct ctcccagatt 360
gtaaagtgtg aagacagctg cctggtgtgg acttggtgac agacaatgtc ttcacacatc 420
tcctgtgaca tccagagacc tcagttctct ttagtcaagt gtctgatgtt ccctgtgagt 480
ctgcgggctc aaagtgaaga actgtggagc ccagtccacc cctgcacacc aggaccctat 540
ccctgcactg ccctgtgttc ccttccacag ccaaccttgc tgctccagcc aaacattggt 600
ggacatctgc agcctgtcag ctccatgcta ccctgacctt caactcctca cttccacact 660
gagaataata atttgaatgt gggtggctgg agagatggct cagcgctgac tgctcttcca 720
aaggtcctga gttcaaatcc cagcaaccac atggtggctc acaaccatct gtaatgggat 780
ctaataccct cttctgcagt gtctgaagac asctacagtg tacttacata taataataaa 840
taag 844

<210> 133

<211> 601
<212> DNA
<213> Homo sapiens

<400> 133
ggccgggcgc gcgcgccccc gccacacgca cgccgggcgt gccagtttat aaagggagag 60
agcaagcagc gagtcttgaa gctctgtttg gtgctttgga tccatttcca tcggtcctta 120
cagccgctcg tcagactcca gcagccaaga tggtgaagca gatcgagagc aagactgctt 180
ttcaggaagc cttggacgct gcaggtgata aacttgtagt agttgacttc tcagccacgt 240
ggtgtgggcc ttgcaaaatg atcaagcctt tctttcattc cctctctgaa aagtattcca 300
acgtgatatt ccttgaagta gatgtggatg actgtcagga tgttgcttca gagtgtgaag 360
tcaaatgcat gccaacattc cagtttttta agaagggaca aaaggtgggt gaattttctg 420
gagccaataa ggaaaagctt gaagccacca ttaatgaatt agtctaatca tgttttctga 480
aaatataacc agccattggc tatttaaaac ttgtaatttt tttaatttac aaaaatataa 540
aatatgaaga cataaacccm gttgccatct gcgtgacaat aaaacattaa tgctaacact 600
t 601

<210> 134
<211> 421
<212> DNA
<213> Homo sapiens

<400> 134
tcacataaga aatttaagca agttacrcta tcttaaaaaa cacaacgaat gcattttaat 60
agagaaaccc ttccctccct ccacctccct cccccaccct cctcatgaat taagaatcta 120
agagaagaag taaccataaa accaagtttt gtggaatcca tcatccagag tgcttacatg 180
gtgattaggt taatattgcc ttcttacaaa atttctattt taaaaaaaat tataaccttg 240
attgcttatt acaaaaaaat tcagtacaaa agttcaatat attgaaaaat gcttttcccc 300
tccctcacag caccgtttta tatatagcag agaataatga agagattgct agtctagatg 360
gggcaatctt caaattacac caagacgcac agtggtttat ttaccctccc cttctcataa 420
g 421

<210> 135
<211> 511
<212> DNA
<213> Homo sapiens

<400> 135
ggaaaggatt caagaattag aggacttgct tgctrragaa aaagacaact ctcgtcgcat 60
gctgacagac aaagagagag agatggcgga aataagggat caaatgcagc aacagctgaa 120
tgactatgaa cagcttcttg atgtaaagtt agccctggac atggaaatca gtgcttacag 180
gaaactctta gaaggcgaag aagagaggtt gaagctgtct ccaagccctt cttcccgtgt 240
gacagtatcc cgagcatcct caagtcgtag tgtaccgtac aactagagga aagcggaaga 300
gggttgatgt ggaagaatca gaggcgaagt agtagtgtta gcatctctca ttccgcctca 360
accactggaa atgtttgcat cgaagaaatt gatgttgatg ggaaatttat cccgcttgaa 420
gaacacttct gaacaggatc aaccaatggg aaggcttggg agatgatcag aaaaattgga 480
gacacatcag tcagttataa atatacctca a 511

<210> 136
<211> 341
<212> DNA
<213> Homo sapiens

<400> 136
catgggtttc accaggttgg ccaggctgct cttgaactsc tgacctcagg tgatccaccc 60
gcctcggcct cccaaagtgc tgggattaca ggcgtgagcc accacgcccg gcccccaaag 120
ctgtttcttt tgtctttagc gtaaagctct cctgccatgc agtatctaca taactgacgt 180

gactgccagc aagctcagtc actccgtggt ctttttctct ttccagttct tctctctctc 240
ttcaagttct gcctcagtga aagctgcagg tccccagtta agtgatcagg tgagggttct 300
ttgaacctgg ttctatcagt cgaattaatc cttcatgatg g 341

<210> 137
<211> 551
<212> DNA
<213> Homo sapiens

<400> 137
gatgtgttgg accctctgtg tcaaaaaaaa cctcacaaag aatcccctgc tcattacaga 60
agaagatgca tttaaaatat gggttatttt caacttttta tctgaggaca agtatccatt 120
aattattgtg tcagaagaga ttgaatacct gcttaagaag cttacagaag ctatgggagg 180
aggttggcag caagaacaat ttgaacatta taaaatcaac tttgatgaca gtaaaaatgg 240
cctttctgca tgggaactta ttgagcttat tggaaatgga cagtttagca aaggcatgga 300
ccggcagact gtgtctatgg caattaatga agtctttaat gaacttatat tagatgtgtt 360
aaagcagggt tacatgatga aaaagggcca cagacggaaa aactggactg aaagatggtt 420
tgtactaaaa cccaacataa tttcttacta tgtgagtgag gatctgaagg ataagaaagg 480
agacattctc ttggatgaaa attgctgtgt agaagtcctt gcctgacaaa agatggaaag 540
aaatgccttt t 551

<210> 138
<211> 531
<212> DNA
<213> Homo sapiens

<220>
<221> misc_feature
<222> 490
<223> n = A,T,C or G

<400> 138
gactggttct ttatttcaaa aagacacttg tcaatattca gtrtcaaaac agttgcacta 60
ttgatttctc tttctcccaa tcggccccaa agagaccaca taaaaggaga gtacatttta 120
agccaataag ctgcaggatg tacacctaac agacctccta gaaaccttac cagaaaatgg 180
ggactgggta gggaaggaaa cttaaaagat caacaaactg ccagcccacg gactgcagag 240
gctgtcacag ccagatgggg tggccagggt gccacaaacc caaagcaaag tttcaaaata 300
atataaaatt taaaaagttt tgtacataag ctattcaaga tttctccagc actgactgat 360
acaaagcaca attgagatgg cacttctaga gacagcagct tcaaacccag aaaagggtga 420
tgagatgaag tttcacatgg ctaaatcagt ggcaaaaaca cagtcttctt tctttctttc 480
tttcaaggan gcaggaaagc aattaagtgg tcaccttaac ataaggggga c 531

<210> 139
<211> 521
<212> DNA
<213> Homo sapiens

<220>
<221> misc_feature
<222> 517
<223> n = A,T,C or G

<400> 139
tgggtgggca ccatggctgg gatcaccacc atcgaggcgg tgaagcgcaa gatccaggtt 60
ctgcagcagc aggcagatga tgcagaggag cgagctgagc gcctccagcg agaagttgag 120
ggagaaaggc gggcccggga acaggctgag gctgaggtgg cctccttgaa ccgtaggatc 180
cagctggttg aagaagagct ggaccgtgct caggagcgcc tggccactgc cctgcaaaag 240

ctggaagaag ctgaaaaagc tgctgatgag agtgagagag gtatgaaggt tattgaaaac 300
cgggccttaa aagatgaaga aaagatggaa ctccaggaaa tccaactcaa agaagctaag 360
cacattgcag aagaggcaga taggaagtat gaagaggtgg ctcgtaagtt ggtgatcatt 420
gaaggagact tggaaccgca cagaaggaac gagcttgagc ttggcaaaag tcccgttgcc 480
cagagatggg atgaaccaga ttagactgat ggaccanaac c 521

<210> 140
<211> 571
<212> DNA
<213> Homo sapiens

<220>
<221> misc_feature
<222> 7
<223> n = A,T,C or G

<400> 140
aggggcngcg ggtgcgtggg ccactgggtg accgacttag cctggccaga ctctcagcac 60
ctggaagcgc cccgagagtg acagcgtgag gctgggaggg aggacttggc ttgagcttgt 120
taaactctgc tctgagcctc cttgtcgcct gcatttagat ggctcccgca aagaagggtg 180
gcgagaagaa aaagggccgt tctgccatca acgaagtggt aacccgagaa tacaccatca 240
acattcacaa gcgcatccat ggagtgggct tcaagaagcg tgcacctcgg gcactcaaag 300
agattcggaa atttgccatg aaggagatgg gaactccaga tgtgcgcatt gacaccaggc 360
tcaacaaagc tgtctgggcc aaaggaataa ggaatgtgcc ataccgaatc cggtgtgcgg 420
ctgtccagaa aacgtaatga ggatgaagat tcaccaaata agctatatac tttggttacc 480
tatgtacctg ttaccacttt caaaaatcta cagacagtca atgtggatga gaactaatcg 540
ctgatcgtca gatcaaataa agttataaaa t 571

<210> 141
<211> 531
<212> DNA
<213> Homo sapiens

<400> 141
tcgggagcca cacttggccc tcttcctctc caaagsgcca gaacctcctt ctctttggag 60
aatggggagg cctcttggag acacagaggg tttcaccttg gatgacctct agagaaattg 120
cccaagaagc ccaccttctg gtcccaacct gcagacccca cagcagtcag ttggtcaggc 180
cctgctgtag aaggtcactt ggctccattg cctgcttcca accaatgggc aggagagaag 240
gcctttattt ctcgcccacc cattcctcct gtaccagcac ctccgttttc agtcagtgtt 300
gtccagcaac ggtaccgttt acacagtcac ctcagacaca ccatttcacc tcccttgcca 360
agctgttagc cttagagtga ttgcagtgaa cactgtttac acaccgtgaa tccattccca 420
tcagtccatt ccagttggca ccagcctgaa ccatttggta cctggtgtta actggagtcc 480
tgtttacaag gtggagtcgg ggcttgctga cttctcttca tttgaggcca c 531

<210> 142
<211> 491
<212> DNA
<213> Homo sapiens

<220>
<221> misc_feature
<222> 410
<223> n = A,T,C or G

<400> 142
acctagacag aaggtgggtg agggaggact ggtaggaggc tgaggcaatt ccttggtagt 60
ttgtcctgaa accctactgg agaagtcagc atgaggcacc tactgagaga agtgcccaga 120

aactgctgac tgcatctgtt aagagttaac agtaaagagg tagaagtgtg tttctgaatc 180
agagtggaag cgtctcaagg gtcccacagt ggaggtccct gagctacctc ccttccgtga 240
gtgggaagag tgaagcccat gaagaactga gatgaagcaa ggatggggtt cctgggctcc 300
aggcaagggc tgtgctctct gcagcaggga gccccacgag tcagaagaaa agaactaatc 360
atttgttgca agaaaccttg cccggatact agcggaaaac tggaggcggn ggtgggggca 420
caggaaagtg gaagtgattt gatggagagc agagaagcct atgcacagtg gccgagtcca 480
cttgtaaagt g 491

<210> 143
<211> 515
<212> DNA
<213> Homo sapiens

<400> 143
ttcaagcaat tgtaacaagt atatgtagat tagagtgagc aaaatcatat acaattttca 60
tttccagttg ctattttcca aattgttctg taatgtcgtt aaaattactt aaaaattaac 120
aaagccaaaa attatattta tgacaagaaa gccatcccta cattaatctt acttttccac 180
tcaccggccc atctccttcc tctttttcct aactatgcca ttaaaactgt tctactgggc 240
cgggcgtgtg gctcatgcct gtaatcccag cattttggga ggccaaggca ggcggatcat 300
gaggtcaaga gattgagacc atcctggcca acatggtgaa accccgcctc gactaagaat 360
acaaaaatta gctgggcatg gtggcgcatg cctgtagtct cagctactcg ggaggctgag 420
gcagaagaat cgcttgaacc cgggaggcag aggatgcagt gagccccgat cgcgccactg 480
cactctagcc tgggcgacag actgagactc tgctc 515

<210> 144
<211> 340
<212> DNA
<213> Homo sapiens

<400> 144
tgtgccagtc tacaggccta tcagcagcga ctccttcagc aacagatggg gtcccctgtt 60
cagcccaacc ccatgagccc ccagcagcat atgctcccaa atcaggccca gtccccacac 120
ctacaaggcc agcagatccc taattctctc tccaatcaag tgcgctctcc ccagcctgtc 180
ccttctccac ggccacagtc ccagcccccc cactccagtc cttccccaag gatgcagcct 240
cagccttctc cacaccacgt ttccccacag acaagttccc cacatcctgg actggtagtt 300
gcccaggcca accccatgga acaagggcat tttgccagcc 340

<210> 145
<211> 630
<212> DNA
<213> Homo sapiens

<400> 145
tgtaaaaact tgtttttaat tttgtataaa ataaaggtgg tccatgccca cgggggctgt 60
aggaaatcca agcagaccag ctggggtggg ggatgtagcc ctacctcggg ggactgtctg 120
tcctcaaaac gggctgagaa ggcccgtcag gggcccaggt cccacagaga ggcctgggat 180
actcccccaa cccgaggggc agactgggca gtggggagcc cccatcgtgc cccagaggtg 240
gccacaggct gaaggagggg cctgaggcac cgcagcctgc aacccccagg gctgcagtcc 300
actaactttt tacagaataa aaggaacatg gggatgggga aaaaagcacc aggtcaggca 360
gggcccgagg gccccagatc ccaggagggc caggactcag gatgccagca ccaccctagc 420
agctcccaca gctcctggca caggaggccg ccacggattg gcacaggccg ctgctggcca 480
tcacgccaca tttggagaac ttgtcccgac agaggtcagc tcggaggagc tcctcgtggg 540
cacacactgt acgaacacag atctccttgt taatgacgta cacacggcgg aggctgcggg 600
gacagggcac gggaggtctc agccccactt 630

<210> 146
<211> 521

<212> DNA
<213> Homo sapiens

<400> 146
atggctgctg gatttaggtg gtaatagggg ctgtgggcca taaatctgaa gccttgagaa 60
ccttgggtct ggagagccat gaagagggaa ggaaaagagg gcaagtcctg aacctaacca 120
atgacctgat ggattgctcg accaagacac agaagtgaag tctgtgtctg tgcacttccc 180
acagactgga gtttttggtg ctgaatagag ccagttgcta aaaaattggg ggtttggtga 240
agaaatctga ttgttgtgtg tattcaatgt gtgattttaa aaataaacag caacaacaat 300
aaaaaccctg actggctgtt ttttccctgt attctttaca actatttttt gaccctctga 360
aaattattat acttcaccta aatggaagac tgctgtgttt gtggaaattt tgtaattttt 420
taatttattt tattctctct cctttttatt ttgcctgcag aatccgttga gagactaata 480
aggcttaata tttaattgat ttgtttaata tgtatataaa t 521

<210> 147
<211> 562
<212> DNA
<213> Homo sapiens

<400> 147
ggcatgcgag cgcactcggc ggacgcaagg gcggcgggga gcacacggag cactgcaggc 60
gccgggttgg gacagcgtct tcgctgctgc tggatagtcg tgttttcggg gatcgaggat 120
actcaccaga aaccgaaaat gccgaaacca atcaatgtcc gagttaccac catggatgca 180
gagctggagt ttgcaatcca gccaaataca actggaaaac agctttttga tcaggtggta 240
aagactatcg gcctccggga agtgtggtac tttggcctcc actatgtgga taataaagga 300
tttcctacct ggctgaagct ggataagaag gtgtctgccc aggaggtcag gaaggagaat 360
cccctccagt tcaagttccg ggccaaagtt ctaccctgaa gatgtggctg aggagctcat 420
ccaggacatc acccagaaac ttttcttcct tcaagtgaag gaaggaatcc ttagcgatga 480
gatctactgc cccccttgar actgccgtgc tcttggggtc ctacgcttgt gcatgccaag 540
tttggggact accaccaaga ag 562

<210> 148
<211> 820
<212> DNA
<213> Homo sapiens

<400> 148
gaaggagtcg ggatactcag cattgatgca ccccaatttc aaagcggcat tcttcggcag 60
gtctctggga caatctctag ggtcactacc tggaaactcg ttagggtaca actgaatgct 120
gaaaggaaag aacacctgca gaaccggaca gaaattcacc ccggcgatca gctgattgat 180
ctcggtcgac cagaagtcat ggctaaagat gacgaggacg ttgtcaattc cctgggcttt 240
tcgaagtgag tccagcagca gtctgaggta ttcgggccgg ttatgcacct ggaccaccag 300
caccagctcc cggggggccc aggtgccagc cttatctaca ttcctcaggg tctgatcaaa 360
gttcagctgg tacaccaggg accggtaccg cagcgtcagg ttgtccgctc gggctggggg 420
accgccggga ccagggaagc cgccgacacg ttggagaccc tgcggatgcc cacagccaca 480
gaggggtggt ccccaccgcg gccgccggca ccccgcgcgg gttcggcgtc cagcaacggt 540
ggggcgaggg cctcgttctt cctttgtcgc ccattgctgc tccagaggac gaagccgcag 600
gcggccacca cgagcgtcag gattagcacc ttccgtttgt agatgcggaa cctcatggtc 660
tccagggccg ggagcgcagc tacagctcga gcgtcggcgc cgccgctagg agccgcggct 720
cggcttcgtc tccgtcctct ccattcagca ccacgggtcc cggaaaaagc tcagccscgg 780
tcccaaccgc accctagctt cgttacctgc gcctcgcttg 820

<210> 149
<211> 501
<212> DNA
<213> Homo sapiens

<400> 149
cagattttta tttgcagtcg tcactggggc cgtttcttgc tgcttatttg tctgctagcc 60
tgctcttcca gctgcatggc caggcgcaag gccttgatga catctcgcag ggctgagaaa 120
tgcttggctt gctgggccag agcagattcc gctttgttca caaaggtctc caggtcatag 180
tctggctgct cggtcatctc agagagctca agccagtctg gtccttgctg tatgatctcc 240
ttgagctctt ccatagcctt ctcctccagc tccctgatct gagtcatggc ttcgttaaag 300
ctggacatct gggaagacag ttcctcctct tccttggata aattgcctgg aatcagcgcc 360
ccgttagagc aggcttccat ctcttctgtt tccatttgaa tcaactgctc tccactgggc 420
ccactgtggg ggctcagctc cttgaccctg ctgcatatct taagggtgtt taaaggatat 480
tcacaggagc ttatgcctgg t 501

<210> 150
<211> 511
<212> DNA
<213> Homo sapiens

<220>
<221> misc_feature
<222> 457, 479
<223> n = A,T,C or G

<400> 150
ctcctcttgg tacatgaacc caagttgaaa gtggacttaa caaagtatct ggagaaccaa 60
gcattctgct ttgactttgc atttgatgaa acagcttcga atgaagttgt ctacaggttc 120
acagcaaggc cactggtaca gacaatcttt gaaggtggaa aagcaacttg ttttgcatat 180
ggccagacag gaagtggcaa gacacatact atgggcggag acctctctgg gaaagcccag 240
aatgcatcca aagggatcta tgccatggcc ttccgggacg tcttcttctg aagaatcaac 300
cctgctaccg gaagttgggc ctggaagtct atgtgacatt cttcgagatc tacaatggga 360
agctgtttga cctgctcaac aagaaggcca agcttgcgcg tgctggaaga cggcaagcaa 420
caggtgcaag tggtgggggc ttgcaggaac atctggntaa ctctgcttga tgatggcant 480
caagatgatc gacatgggca gcgcctgcag a 511

<210> 151
<211> 566
<212> DNA
<213> Homo sapiens

<400> 151
tcccgaattc aagcgacaaa ttggawagtg aaatggaaga tgcctatcat gaacatcagg 60
caaatctttt gcgccaagat ctgatgagac gacaggaaga attaagacgc atggaagaac 120
ttcacaatca agaaatgcag aaacgtaaag aaatgcaatt gaggcaagag gaggaacgac 180
gtagaagaga ggaagagatg atgattcgtc aacgtgagat ggaagaacaa atgaggcgcc 240
aaagagagga aagttacagc cgaatgggct acatggatcc acgggaaaga gacatgcgaa 300
tgggtggcgg aggagcaatg aacatgggag atccctatgg ttcaggaggc cagaaatttc 360
cacctctagg aggtggtggt ggcataggtt atgaagctaa tcctggcgtt ccaccagcaa 420
ccatgagtgg ttccatgatg ggaagtgaca tgcgtactga gcgctttggg cagggaggtg 480
cggggcctgt gggtggacag ggtcctagag gaatggggcc tggaactcca gcaggatatg 540
gtagagggag agaagagtac gaaggc 566

<210> 152
<211> 518
<212> DNA
<213> Homo sapiens

<400> 152
ttcgtgaaga ccctgactgg taagaccatc actctcgaag tggagcccga gtgacaccat 60
tgagaatgtc aaggcaaaga tccaagacaa ggaaggcatc cctcctgacc agcakaggtt 120

gatctttgct gggaaacagc tggaagatgg acgcaccctg tctgactaca acatccagaa 180
agagtccacc ctgcacctgg tgctccgtct cagaggtggg atgcaaatct tcgtgaagac 240
cctgactggt aagaccatca ccctcgaggt ggagcccagt gacaccatcg agaatgtcaa 300
ggcaaagatc caagataagg aaggcatccc tcctgatcag cagaggttga tctttgctgg 360
gaaacagctg gaagatggac gcaccctgtc tgactacaac atccagaaag agtccactct 420
gcacttggtc ctgcgcttga gggggggtgt ctaagtttcc ccttttaagg tttcaacaaa 480
tttcattgca ctttcctttc aataaagttg ttgcattc 518

<210> 153
<211> 542
<212> DNA
<213> Homo sapiens

<400> 153
gcgcgggtgc gtgggccact gggtgaccga cttagcctgg ccagactctc agcacctgga 60
agcgccccga gagtgacagc gtgaggctgg gagggaggac ttggcttgag cttgttaaac 120
tctgctctga gcctccttgt cgcctgcatt tagatggctc ccgcaaagaa gggtggcgag 180
aagaaaaagg gccgttctgc catcaacgaa gtggtaaccc gagaatacac catcaacatt 240
cacaagcgca tccatggagt gggcttcaag aagcgtgcac ctcgggcact caaagagatt 300
cggaaatttg ccatgaagga gatgggaact ccagatgtgc gcattgacac caggctcaac 360
aaagctgtct gggccaaagg aataaggaat gtgccatacc gaatccgtgt gcggctgtcc 420
agaaaacgta atgaggatga agattcacca aataagctat atactttggt tacctatgta 480
cctgttacca ctttcaaaaa tctacagaca gtcaatgtgg atgagaacta atcgctgatc 540
gt 542

<210> 154
<211> 411
<212> DNA
<213> Homo sapiens

<400> 154
aattctttat ttaaatcaac aaactcatct tcctcaagcc ccagaccatg gtaggcagcc 60
ctccctctcc atcccctcac cccacccctt agccacagtg aagggaatgg aaaatgagaa 120
gccacgaggg cccctgccag ggaaggctgc cccagatgtg tggtgagcac agtcagtgca 180
gctgtggctg gggcagcagc tgccacaggc tcctccctat aaattaagtt cctgcagcca 240
cagctgtggg agaagcatac ttgtagaagc aaggccagtc cagcatcaga aggcagaggc 300
agcatcagtg actcccagcc atggaatgaa cggaggacac agagctcaga gacagaacag 360
gccaggggga agaaggagag acagaatagg ccagggcatg gcggtgaggg a 411

<210> 155
<211> 421
<212> DNA
<213> Homo sapiens

<220>
<221> misc_feature
<222> 173
<223> n = A,T,C or G

<400> 155
tgatgaatct gggtgggctg gcagtagccc gagatgatgg gctcttctct ggggatccca 60
actggttccc taagaaatcc aaggagaatc ctcggaactt ctcggataac cagctgcaag 120
agggcaagaa cgtgatcggg ttacagatgg gcaccaaccg cggggcgtct cangcaggca 180
tgactggcta cgggatgcca cgccagatcc tctgatccca ccccaggcct tgcccctgcc 240
ctcccacgaa tggttaatat atatgtagat atatatttta gcagtgacat tcccagagag 300
ccccagagct ctcaagctcc tttctgtcag ggtggggggt tcaagcctgt cctgtcacct 360
ctgaagtgcc tgctggcatc ctctccccca tgcttactaa tacattccct tccccatagc 420

c                                                                   421

<210> 156
<211> 670
<212> DNA
<213> Homo sapiens

<400> 156
agcggagctc cctcccctgg tggctacaac ccacacacgc caggctcagg catcgagcag 60
aactccagcg actgggtaac cactgacatt caggtgaagg tgcgggacac ctacctggat 120
acacaggtgg tgggacagac aggtgtcatc cgcagtgtca cggggggcat gtgctctgtg 180
tacctgaagg acagtgagaa ggttgtcagc atttccagtg agcacctgga gcctatcacc 240
cccaccaaga acaacaaggt gaaagtgatc ctgggcgagg atcgggaagc cacgggcgtc 300
ctactgagca ttgatggtga ggatggcatt gtccgtatgg accttgatga gcagctcaag 360
atcctcaacc tccgcttcct ggggaagctc ctggaagcct gaagcaggca gggccggtgg 420
acttcgtcgg atgaagagtg atcctccttc cttccctggc ccttggctgt gacacaagat 480
cctcctgcag ggctaggcgg attgttctgg atttcctttt gtttttcctt ttaggtttcc 540
atcttttccc tccctggtgc tcattggaat ctgagtagag tctgggggag ggtccccacc 600
ttcctgtacc tcctccccac agcttgcttt tgttgtaccg tctttcaata aaaagaagct 660
gtttggtcta                                                          670

<210> 157
<211> 421
<212> DNA
<213> Homo sapiens

<400> 157
ggttcacagc actgctgctt gtgtgttgcc ggccaggaat tccaggctca caaggctatc 60
ttagcagctc gttctccggt ttttagtgcc atgtttgaac atgaaatgga ggagagcaaa 120
aagaatcgag ttgaaatcaa tgatgtggag cctgaagttt ttaaggaaat gatgtgcttc 180
atttacacgg ggaaggctcc aaacctcgac aaaatggctg atgatttgct ggcagctgct 240
gacaagtatg ccctggagcg cttaaaggtc atgtgtgagg atgccctctg cagtaacctg 300
tccgtggaga acgctgcaga aattctcatc ctggccgacc tccacagtgc agatcagttg 360
aaaactcagg cagtggattt catcaactat catgcttcgg atgtcttgga gacctcttgg 420
g                                                                   421

<210> 158
<211> 321
<212> DNA
<213> Homo sapiens

<400> 158
tcgtagccat tttctgcttc tttggagaa tgacgccaca ctgactgctc attgtcgttg 60
gttccatgcc aattggtgaa atagaacctc atccggtagt ggagccggag ggacatcttg 120
tcatcaacgg tgatggtgcg atttggagca taccagagct tggtgttctc gccatacagg 180
gcaaagaggt tgtgacaaag aggagagata cggcatgcct gtgcagccct gatgcacagt 240
tcctctgctg tgtactctcc actgcccagc cggaggggct ccctgtccga cagatagaag 300
atcacttcca cccctggctt g                                             321

<210> 159
<211> 596
<212> DNA
<213> Homo sapiens

<400> 159
tggcacactg ctcttaagaa actatgawga tctgagattt ttttgtgtat gtttttgact 60
cttttgagtg gtaatcatat gtgtctttat agatgtacat acctccttgc acaaatggag 120

gggaattcat tttcatcact gggagtgtcc ttagtgtata aaaaccatgc tggtatatgg 180
cttcaagttg taaaaatgaa agtgacttta aaagaaaata ggggatggtc caggatctcc 240
actgataaga ctgtttttaa gtaacttaag gacctttggg tctacaagta tatgtgaaaa 300
aaatgagact tactgggtga ggaaattcat tgtttaaaga tggtcgtgtg tgtgtgtgtg 360
tgtgtgtgtg ttgtgttgtg ttttgttttt taagggaggg aatttattat ttaccgttgc 420
ttgaaattac tgkgtaaata tatgtytgat aatgatttgc tytttgvcma ctaaaattag 480
gvctgtataa gtwctaratg cmtccctggg kgttgatytt ccmagatatt gatgatamcc 540
cttaaaattg taaccygcct tttccctttt gctytcmatt aaagtctatt cmaaag 596

<210> 160
<211> 515
<212> DNA
<213> Homo sapiens

<400> 160
gggggtaggc tctttattag acggttattg ctgtactaca gggtcagagt gcagtgtaag 60
cagtgtcaga ggcccgcgtt cagcccaaga atgtggattt tctctcccta ttgatcacag 120
tgggtgggtt tcttcagaaa agccccagag gcagggacca gtgagctcca aggttagaag 180
tggaactgga aggcttcagt cacatgctgc ttccacgctt ccaggctggg cagcaaggag 240
gagatgccca tgacgtgcca ggtctcccca tctgacacca gtgaagtctg gtaggacagc 300
agccgcacgc ctgcctctgc caggaggcca atcatggtag gcagcattgc agggtcagag 360
gtctgagtcc ggaataggag caggggcagg tccctgcgga gaggcacttc tggcctgaag 420
acagctccat tgagcccctg cagtacaggy gtagtgcctt ggaccaagcc cacagcctgg 480
taaggggcgc ctgccagggc cacggccagg aggca 515

<210> 161
<211> 936
<212> DNA
<213> Homo sapiens

<400> 161
taatttctta gtcgtttgga atccttaagc atgcaaaagc tttgaacaga agggttcaca 60
aaggaaccag ggttgtctta tggcatccag ttaagccaga gctgggaatg cctctgggtc 120
atccacatca ggagcagaag cacttgactt gtcggtcctg ctgccacggt ttgggcgccc 180
accacgccca cgtccacctc gtcctcccct gccgccacgt cctgggcggc caaggtctcc 240
aaaattgatc tccagctgag acgttatatc atttgctggc ttccggaaat gatggtccat 300
aaccgaatct tcagcatgag cctcttcact ctttgattta tgaagaacaa atcccttctt 360
ccactgccca tcagcacctt catttggttt tcggatatta aattctactt ttgcccggtc 420
cttattttga atagccttcc actcatccaa agtcatctct tttggaccct cctcttttac 480
ctcttcaact tcattctcct tattttcagt gtctgccact ggatgatgtt cttcaccttc 540
aggtgtttcc tcagtcacat ttgattgatc caagtcagtt aattcgtctt tgacagttcc 600
ccagttgtga gatccgctac ctccacgttt gtcctcgtgc ttcaggccag atctatcact 660
tccactatgc ctatcaaatt cacgtttgcc acgagaatca aatccatctc ctcggcccat 720
tccacgtcca cggccccctc gacctcttcc aagaccacca cgacctcgaa taggtcggtc 780
aataatcggt ctatcaactg aaaattcgcc tccttcaccc ttttcttcaa gtggcttttc 840
gaatcttcgt tcacgaggtg gtcgcctttc tggtcttcta tcaattattt tcccttcacc 900
ctgaagttgt tgatcaggtc ttcttccaac tcgtgc 936

<210> 162
<211> 950
<212> DNA
<213> Homo sapiens

<400> 162
aagcggatgg acctgagtca gccgaatcct agccccttcc cttgggcctg ctgtggtgct 60
cgacatcagt gacagacgga agcagcagac catcaaggct acggaggccc cggggcgctt 120
gcgaagatga agtttggctg cctctccttc cggcagcctt atgctggctt tgtcttaaat 180

ggaatcaaga ctgtggagac gcgctggcgt cctctgctga gcagccagcg gaactgtacc 240
atcgccgtcc acattgctca cagggactgg gaaggcgatg cctgtcggga gctgctggtg 300
gagagactcg ggatgactcc tgctcagatt caggccttgc tcaggaaagg ggaaaagttt 360
ggtcgaggag tgatagcggg actcgttgac attggggaaa ctttgcaatg ccccgaagac 420
ttaactcccg atgaggttgt ggaactagaa aatcaagctg cactgaccaa cctgaagcag 480
aagtacctga ctgtgatttc aaaccccagg tggttactgg agcccatacc taggaaagga 540
ggcaaggatg tattccaggt agacatccca gagcacctga tccctttggg gcatgaagtg 600
tgacaagtgt gggctcctga aaggaatgtt ccrgagaaac cagctaaatc atggcacctt 660
caatttgcca tcgtgacgca gacctgtata aattaggtta aagatgaatt tccactgctt 720
tggagagtcc cacccactaa gcactgtgca tgtaaacagg ttcctttgct cagatgaagg 780
aagtaggggg tggggctttc cttgtgtgat gcctccttag gcacacaggc aatgtctcaa 840
gtactttgac cttagggtag aaggcaaagc tgccagtaaa tgtctcagca ttgctgctaa 900
ttttggtcct gctagtttct ggattgtaca aataaatgtg ttgtagatga 950

<210> 163
<211> 475
<212> DNA
<213> Homo sapiens

<220>
<221> misc_feature
<222> 301, 317, 331, 458, 464, 470
<223> n = A,T,C or G

<400> 163
tcgagcggcc gcccgggcag gtgtcggagt ccagcacggg aggcgtggtc ttgtagttgt 60
tctccggctg cccattgctc tcccactcca cggcgatgtc gctgggatag aagcctttga 120
ccaggcaggt caggctgacc tggttcttgg tcatctcctc ccgggatggg ggcagggtgt 180
acacctgtgg ttctcggggc tgccctttgg ctttggagat ggttttctcg atgggggctg 240
ggagggcttt gttggagacc ttgcacttgt actccttgcc attcaaccag tcctggtgca 300
ngacggtgag gacgctnacc acacggtacg ngctggtgta ctgctcctcc cgcggctttg 360
tcttggcatt atgcacctcc acgccgtcca cgtaccaatt gaacttgacc tcagggtctt 420
cgtggctcac gtccaccacc acgcatgtaa cctcaaanct cggncgcgan cacgc 475

<210> 164
<211> 476
<212> DNA
<213> Homo sapiens

<400> 164
agcgtggtcg cggccgaggt ctgaggttac atgcgtggtg gtggacgtga gccacgaaga 60
ccctgaggtc aagttcaact ggtacgtgga cggcgtggag gtgcataatg ccaagacaaa 120
gccgcgggag gagcagtaca acagcacgta ccgtgtggtc agcgtcctca ccgtcctgca 180
ccaggactgg ctgaatggca aggagtacaa gtgcaaggtc tccaacaaag ccctcccagc 240
ccccatcgag aaaaccatct ccaaagccaa agggcagccc cgagaaccac aggtgtacac 300
cctgccccca tcccgggagg agatgaccaa gaaccaggtc agcctgacct gcctggtcaa 360
aggcttctat cccagcgaca tcgcccgtgg agtgggagag caatgggcag ccggagaaca 420
actacaagac cacgcctccc gtgctggact ccgacacctg ccgggcggcc gctcga 476

<210> 165
<211> 256
<212> DNA
<213> Homo sapiens

<220>
<221> misc_feature
<222> 10, 37, 249

<223> n = A,T,C or G

<400> 165
agcgtggttn cggccgaggt cccaaccaag gctgcancct ggatgccatc aaagtcttct 60
gcaacatgga gactggtgag acctgcgtgt accccactca gcccagtgtg gcccagaaga 120
actggtacat cagcaagaac cccaaggaca agaggcatgt ctggttcggc gagagcatga 180
ccgatggatt ccagttcgag tatggcggcc agggctccga ccctgccgat gtggacctgc 240
ccgggcggnc gctcga 256

<210> 166
<211> 332
<212> DNA
<213> Homo sapiens

<400> 166
agcgtggtcg cggccgaggt caagaacccc gcccgcacct gccgtgacct caagatgtgc 60
cactctgact ggaagagtgg agagtactgg attgacccca accaaggctg caacctggat 120
gccatcaaag tcttctgcaa catggagact ggtgagacct gcgtgtaccc cactcagccc 180
agtgtggccc agaagaactg gtacatcagc aagaacccca aggacaagag gcatgtctgg 240
ttcggcgaga gcatgaccga tggattccag ttcgagtatg gcggccaggg ctccgaccct 300
gccgatgtgg acctgcccgg gcggccgctc ga 332

<210> 167
<211> 332
<212> DNA
<213> Homo sapiens

<220>
<221> misc_feature
<222> 77, 109, 136, 184, 198
<223> n = A,T,C or G

<400> 167
tcgagcggtc gcccgggcag gtccacatcg gcagggtcgg agccctggcc gccatactcg 60
aactggaatc catcggncat gctctcgccg aaccagacat gcctcttgnc cttggggttc 120
ttgctgatgt accagntctt ctgggccaca ctgggctgag tggggtacac gcaggtctca 180
ccantctcca tgttgcanaa gactttgatg gcatccaggt tgcagccttg gttggggtca 240
atccagtact ctccactctt ccagacagag tggcacatct tgaggtcacg gcaggtgcgg 300
gcggggttct tgacctcggt cgcgaccacg ct 332

<210> 168
<211> 276
<212> DNA
<213> Homo sapiens

<220>
<221> misc_feature
<222> 72, 84
<223> n = A,T,C or G

<400> 168
tcgagcggcc gcccgggcag gtcctcctca gagcggtagc tgttcttatt gccccggcag 60
cctccataga tnaagttatt gcangagttc ctctccacgt caaagtacca gcgtgggaag 120
gatgcacggc aaggcccagt gactgcgttg gcggtgcagt attcttcata gttgaacata 180
tcgctggagt ggacttcaga atcctgcctt ctgggagcac ttgggacaga ggaatccgct 240
gcattcctgc tggtggacct cggccgcgac cacgct 276

<210> 169
<211> 276
<212> DNA
<213> Homo sapiens

<400> 169
agcgtggtcg cggccgaggt ccaccagcag gaatgcagcg gattcctctg tcccaagtgc 60
tcccagaagg caggattctg aagaccactc cagcgatatg ttcaactatg aagaatactg 120
caccgccaac gcagtcactg ggccttgccg tgcatccttc ccacgctggt actttgacgt 180
ggagaggaac tcctgcaata acttcatcta tggaggctgc cggggcaata agaacagcta 240
ccgctctgag gaggacctgc ccgggcggcc gctcga 276

<210> 170
<211> 332
<212> DNA
<213> Homo sapiens

<220>
<221> misc_feature
<222> 294
<223> n = A,T,C or G

<400> 170
tcgagcggcc gcccgggcag gtccacatcg gcagggtcgg agccctggcc gccatactcg 60
aactggaatc catcggtcat gctctcgccg aaccagacat gcctcttgtc cttggggttc 120
ttgctgatgt accagttctt ctgggccaca ctgggctgag tggggtacac gcaggtctca 180
ccagtctcca tgttgcagaa gactttgatg gcatccaggt tgcagccttg gttggggtca 240
atccagtact ctccactctt ccagccagaa tggcacatct tgaggtcacg gcangtgcgg 300
gcggggttct tgacctcggc cgcgaccacg ct 332

<210> 171
<211> 333
<212> DNA
<213> Homo sapiens

<400> 171
agcgtggtcg cggccgaggt caagaaaccc cgcccgcacc tgccgtgacc tcaagatgtg 60
ccactctggc tggaagagtg gagagtactg gattgacccc aaccaaggct gcaacctgga 120
tgccatcaaa gtcttctgca acatggagac tggtgagacc tgcgtgtacc ccactcagcc 180
cagtgtggcc cagaagaact ggtacatcag caagaacccc aaggacaaga ggcatgtctg 240
gctcggcgag agcatgaccg atggattcca gttcgagtat ggcggccagg gctccgaccc 300
tgccgatgtg gacctgcccg ggcggccgct cga 333

<210> 172
<211> 527
<212> DNA
<213> Homo sapiens

<220>
<221> misc_feature
<222> 46, 125, 140, 148, 220, 229, 291, 388, 456
<223> n = A,T,C or G

<400> 172
agcgtggtcg cggccgaggt cctgtcagag tggcactggt agaagntcca ggaaccctga 60
actgtaaggg ttcttcatca gtgccaacag gatgacatga aatgatgtac tcagaagtgt 120
cctgnaatgg ggcccatgan atggttgnct gagagagagc ttcttgtcct acattcggcg 180

ggtatggtct tggcctatgc cttatggggg tggccgttgn gggcggtgng gtccgcctaa 240
aaccatgttc ctcaaagatc atttgttgcc caacactggg ttgctgacca naagtgccag 300
gaagctgaat accatttcca gtgtcatacc cagggtgggt gacgaaaggg gtcttttgaa 360
ctgtggaagg aacatccaag atctctgntc catgaagatt ggggtgtgga agggttacca 420
gttggggaag ctcgctgtct ttttccttcc aatcangggc tcgctcttct gaatattctt 480
cagggcaatg acataaattg tatattcggt tcccggttcc aggccag 527

<210> 173
<211> 635
<212> DNA
<213> Homo sapiens

<220>
<221> misc_feature
<222> 444, 453, 517, 540, 546, 551, 573, 593
<223> n = A,T,C or G

<400> 173
tcgagcggcc gcccgggcag gtccaccaca cccaattcct tgctggtatc atggcagccg 60
ccacgtgcca ggattaccgg ctacatcatc aagtatgaga agcctgggtc tcctcccaga 120
gaagtggtcc ctcggccccg ccctggtgtc acagaggcta ctattactgg cctggaaccg 180
ggaaccgaat atacaattta tgtcattgcc ctgaagaata atcagaagag cgagcccctg 240
attggaagga aaaagacaga cgagcttccc caactggtaa cccttccaca ccccaatctt 300
catggaccag agatcttgga tgttccttcc acagttcaaa agaccccttt cgtcacccac 360
cctgggtatg acactggaaa tggtattcag cttcctggca cttctggtca gcaacccagt 420
gttgggcaac aaatgatctt tgangaacat ggntttaggc ggaccacacc ggccacaacg 480
ggcaccccca taaggcatag gccaagaaca tacccgncga atgtaggaca agaagctctn 540
tctcanacaa ncatctcatg ggccccattc cangacactt ctgagtacat canttcatgg 600
catcctggtg gcactgataa aaacccttac agtta 635

<210> 174
<211> 572
<212> DNA
<213> Homo sapiens

<220>
<221> misc_feature
<222> 457, 511, 520, 552, 568
<223> n = A,T,C or G

<400> 174
agcgtggtcg cgggcgaggt cctgtcagag tggcactggt agaagttcca ggaaccctga 60
actgtaaggg ttcttcatca gtgccaacag gatgacatga aatgatgtac tcagaagtgt 120
cctggaatgg ggcccatgag atggttgtct gagagagagc ttcttgtcct acattcggcg 180
ggtatggtct tggcctatgc cttatggggg tggccgttgt gggcggtgtg gtccgcctaa 240
aaccatgttc ctcaaagatc atttgttgcc caacactggg ttgctgacca gaagtgccag 300
gaagctgaat accatttcca gtgtcatacc cagggtgggt gacgaaaggg gtcttttgaa 360
ctgtggaagg aacatccaag atctctggtc catgaagatt ggggtgtgga agggttacca 420
gttggggaag ctcgtctgtc tttttccttc caatcanggg ctcgctcttc tgattattct 480
tcagggcaat gacataaatt gtatattcgg ntcccgggtn cagccaataa taataaccct 540
ctgtgacacc anggcggggc cgaagganca ct 572

<210> 175
<211> 372
<212> DNA
<213> Homo sapiens

<220>
<221> misc_feature
<222> 247
<223> n = A,T,C or G

<400> 175
agcgtggtcg cggccgaggt cctcaccaga ggtaccacct acaacatcat agtggaggca 60
ctgaaagacc agcagaggca taaggttcgg gaagaggttg ttaccgtggg caactctgtc 120
aacgaaggct tgaaccaacc tacggatgac tcgtgctttg acccctacac agtttcccat 180
tatgccgttg gagatgagtg ggaacgaatg tctgaatcag gctttaaact gttgtgccag 240
tgcttangct ttggaagtgg tcatttcaga tgtgattcat ctagatggtg ccatgacaat 300
ggtgtgaact acaagattgg agagaagtgg gaccgtcagg gagaaaatgg acctgcccgg 360
gcggccgctc ga 372

<210> 176
<211> 372
<212> DNA
<213> Homo sapiens

<220>
<221> misc_feature
<222> 251
<223> n = A,T,C or G

<400> 176
tcgagcggcc gcccgggcag gtccattttc tccctgacgg tcccacttct ctccaatctt 60
gtagttcaca ccattgtcat ggcaccatct agatgaatca catctgaaat gaccacttcc 120
aaagcctaag cactggcaca acagtttaaa gcctgattca gacattcgtt cccactcatc 180
tccaacggca taatgggaaa ctgtgtaggg gtcaaagcac gagtcatccg taggttggtt 240
caagccttcg ntgacagagt tgcccacggt aacaacctct tcccgaacct tatgcctctg 300
ctggtctttc agtgcctcca ctatgatgtt gtaggtggta cctctggtga ggacctcggc 360
cgcgaccacg ct 372

<210> 177
<211> 269
<212> DNA
<213> Homo sapiens

<220>
<221> misc_feature
<222> 94, 225
<223> n = A,T,C or G

<400> 177
agcgtggccg cggccgaggt ccattggctg gaacggcatc aacttggaag ccagtgatcg 60
tctcagcctt ggttctccag ctaatggtga tggnggtctc agtagcatct gtcacacgag 120
cccttcttgg tgggctgaca ttctccagag tggtgacaac accctgagct ggtctgcttg 180
tcaaagtgtc cttaagagca tagacactca cttcatattt ggcgnccacc ataagtcctg 240
atacaaccac ggaatgacct gtcaggaac 269

<210> 178
<211> 529
<212> DNA
<213> Homo sapiens

<400> 178
tcgagcggcc gcccgggcag gtcctcagac cgggttctga gtacacagtc agtgtggttg 60

ccttgcacga tgatatggag agccagcccc tgattggaac ccagtccaca gctattcctg 120
caccaactga cctgaagttc actcaggtca cacccacaag cctgagcgcc cagtggacac 180
cacccaatgt tcagctcact ggatatcgag tgcgggtgac ccccaaggag aagaccggac 240
caatgaaaga aatcaacctt gctcctgaca gctcatccgt ggttgtatca ggacttatgg 300
cggccaccaa atatgaagtg agtgtctatg ctcttaagga cactttgaca agcagaccag 360
ctcagggtgt tgtcaccact ctggagaatg tcagcccacc aagaagggct cgtgtgacag 420
atgctactga gaccaccatc accattagct ggagaaccaa gactgagacg atcactggct 480
tccaagttga tgccgttcca gccaatggac ctcggccgcg accacgctt 529

<210> 179
<211> 454
<212> DNA
<213> Homo sapiens

<220>
<221> misc_feature
<222> 64
<223> n = A,T,C or G

<400> 179
agcgtggtcg cggccgaggt ctggccgaac tgccagtgta cagggaagat gtacatgtta 60
tagntcttct cgaagtcccg ggccagcagc tccacggggt ggtctcctgc ctccaggcgc 120
ttctcattct catggatctt cttcacccgc agcttctgct tctcagtcag aaggttgttg 180
tcctcatccc tctcatacag ggtgaccagg acgttcttga gccagtcccg catgcgcagg 240
gggaattcgg tcagctcaga gtccaggcaa ggggggatgt atttgcaagg cccgatgtag 300
tccaagtgga gcttgtggcc cttcttggtg ccctccaagg tgcactttgt ggcaaagaag 360
tggcaggaag agtcgaaggt cttgttgtca ttgctgcaca ccttctcaaa ctcgccaatg 420
ggggctgggc agacctgccc gggcggccgc tcga 454

<210> 180
<211> 454
<212> DNA
<213> Homo sapiens

<220>
<221> misc_feature
<222> 55, 299, 317, 332, 342, 348
<223> n = A,T,C or G

<400> 180
tcgagcggcc gcccgggcag gtctgcccag cccccattgg cgagtttgag aaggngtgca 60
gcaatgacaa caagaccttc gactcttcct gccacttctt tgccacaaag tgcaccctgg 120
agggcaccaa gaagggccac aagctccacc tggactacat cgggccttgc aaatacatcc 180
ccccttgcct ggactctgag ctgaccgaat tccccctgcg catgcgggac tggctcaaga 240
acgtcctggt caccctgtat gagagggatg aggacaacaa ccttctgact gagaagcana 300
agctgcgggt gaagaanatc catgagaatg anaagcgcct gnaggcanga gaccaccccg 360
tggagctgct ggcccgggac ttcgagaaga actataacat gtacatcttc cctgtacact 420
ggcagttcgg ccagacctcg gccgcgacca cgct 454

<210> 181
<211> 102
<212> DNA
<213> Homo sapiens

<220>
<221> misc_feature
<222> 8, 47, 60, 67

<223> n = A,T,C or G

<400> 181
agcgtggntg cggacgacgc ccacaaagcc attgtatgta gttttanttc agctgcaaan 60
aataccncca gcatccacct tactaaccag catatgcaga ca 102

<210> 182
<211> 337
<212> DNA
<213> Homo sapiens

<220>
<221> misc_feature
<222> 169, 195, 253, 314
<223> n = A,T,C or G

<400> 182
tcgagcggtc gcccgggcag gtctgggcgg atagcaccgg gcatattttg gaatggatga 60
ggtctggcac cctgagcagc ccagcgagga cttggtctta gttgagcaat ttggctagga 120
ggatagtatg cagcacggtt ctgagtctgt gggatagctg ccatgaagna acctgaagga 180
ggcgctggct ggtangggtt gattacaggg ctgggaacag ctcgtacact tgccattctc 240
tgcatatact ggntagtgag gcgagcctgg cgctcttctt tgcgctgagc taaagctaca 300
tacaatggct ttgnggacct cggccgcgac cacgctt 337

<210> 183
<211> 374
<212> DNA
<213> Homo sapiens

<400> 183
tcgagcggcc gcccgggcag gtccattttc tccctgacgg tcccacttct ctccaatctt 60
gtagttcaca ccattgtcat gacaccatct agatgaatca catctgaaat gaccacttcc 120
aaagcctaag cactggcaca acagtttaaa gcctgattca gacattcgtt cccactcatc 180
tccaacggca taatgggaaa ctgtgtaggg gtcaaagcac gagtcatccg taggttggtt 240
caagccttcg ttgacagaag ttgcccacgg taacaacctc ttcccgaacc ttatgcctct 300
gctggtcttt caagtgcctc cactatgatg ttgtaggtgg cacctctggt gaggacctcg 360
gccgcgacca cgct 374

<210> 184
<211> 375
<212> DNA
<213> Homo sapiens

<220>
<221> misc_feature
<222> 30, 174, 248, 285, 306, 332, 345, 368
<223> n = A,T,C or G

<400> 184
agcgtggttt gcggccgagg tcctcaccan aggtgccacc tacaacatca tagtggaggc 60
actgaaagac cagcagaggc ataaggttcg ggaagaggtt gttaccgtgg gcaactctgt 120
caacgaaggc ttgaaccaac ctacggatga ctcgtgcttt gacccctaca cagnttccca 180
ttatgccgtt ggagatgagt gggaacgaat gtctgaatca ggctttaaac tgttgtgcca 240
gtgcttangc tttggaagtg gtcatttcag atgtgattca tctanatggt gtcatgacaa 300
tggtgngaac tacaagattg gagagaagtg gnaccgtcag ggganaaaat ggacctgccc 360
gggcggcncg ctcga 375

<210> 185
<211> 148
<212> DNA
<213> Homo sapiens

<220>
<221> misc_feature
<222> 28, 36, 86
<223> n = A,T,C or G

<400> 185
agcgtggtcg cggccgaggt ctggcttnct gctcangtga ttatcctgaa ccatccaggc 60
caaataagcg ccggctatgc ccctgnattg gattgccaca cggctcacat tgcatgcaag 120
tttgctgagc tgaaggaaaa gattgatc 148

<210> 186
<211> 397
<212> DNA
<213> Homo sapiens

<220>
<221> misc_feature
<222> 78
<223> n = A,T,C or G

<400> 186
tcgagcggcc gcccgggcag gtccaattga aacaaacagt tctgagaccg ttcttccacc 60
actgattaag agtggggngg cgggtattag ggataatatt catttagcct tctgagcttt 120
ctgggcagac ttggtgacct tgccagctcc agcagccttc tggtccactg ctttgatgac 180
acccaccgca actgtctgtc tcatatcacg aacagcaaag cgacccaaag gtggatagtc 240
tgagaagctc tcaacacaca tgggcttgcc aggaaccata tcaacaatgg gcagcatcac 300
cagacttcaa gaatttaagg gccatcttcc agctttttac cagaacggcg atcaatcttt 360
tccttcagct cagcaaactt gcatgcaatg tgagccg 397

<210> 187
<211> 584
<212> DNA
<213> Homo sapiens

<220>
<221> misc_feature
<222> 145, 286, 363, 365, 425, 433, 452, 462, 471, 512, 514, 534,
536, 540, 565, 583
<223> n = A,T,C or G

<400> 187
tcgagcggcc gcccgggcag gtccagaggg ctgtgctgaa gtttgctgct gccactggag 60
ccactccaat tgctggccgc ttcactcctg gaaccttcac taaccagatc caggcagcct 120
tccgggagcc acggcttctt gtggntactg accccagggc tgaccaccag cctctcacgg 180
aggcatctta tgttaaccta cctaccattg cgctgtgtaa cacagattct cctctgcgct 240
atgtggacat tgccatccca tgcaacaaca agggagctca ctcagngggg tttgatgtgg 300
tggatgctgg ctcgggaagt tctgcgcatg cgtggcacca tttcccgtga acacccatgg 360
gangncatgc ctgatctgga cttctacaga gatcctgaag agattgaaaa agaagaacag 420
gctgnttgct ganaaagcaa gtgaccaagg angaaatttc angggtgaaa nggactgctc 480
ccgctcctga attcactgct actcaacctg angntgcaga ctggtcttga aggngnacan 540
gggccctctg ggcctattta agcancttcg gtcgcgaaca cgnt 584

<210> 188
<211> 579
<212> DNA
<213> Homo sapiens

<220>
<221> misc_feature
<222> 7, 136, 486
<223> n = A,T,C or G

<400> 188
agcgtgngtc gcggccgagg tgctgaatag gcacagaggg cacctgtaca ccttcagacc 60
agtctgcaac ctcaggctga gtagcagtga actcaggagc gggagcagtc cattcaccct 120
gaaattcctc cttggncact gccttctcag cagcagcctg ctcttctttt tcaatctctt 180
caggatctct gtagaagtac agatcaggca tgacctccca tgggtgttca cgggaaatgg 240
tgccacgcat gcgcagaact tcccgagcca gcatccacca catcaaaccc actgagtgag 300
ctcccttgtt gttgcatggg atgggcaatg tccacatagc gcagaggaga atctgtgtta 360
cacagcgcaa tggtaggtag gttaacataa gatgcctccg cgagaagctg gtggtcagcc 420
ctggggtcaa gtaaccacaa gaagccgtgg ctcccggaag gctgcctgga tctggttagt 480
gaaggntcca ggagtgaagc ggccaacaat tggagtggct tcagtggcaa gcagcaaact 540
tcagcacaag ccctctggac ctgcccggcg gccgctcga 579

<210> 189
<211> 374
<212> DNA
<213> Homo sapiens

<220>
<221> misc_feature
<222> 41, 280, 314, 330, 350, 353
<223> n = A,T,C or G

<400> 189
tcgagcggcc gcccgggcag gtccattttc tccctgacgg ncccacttct ctccaatctt 60
gtagttcaca ccattgtcat ggcaccatct agatgaatca catctgaaat gaccacttcc 120
aaagcctaag cactggcaca acagtttaaa gcctgattca gacattcgtt cccactcatc 180
tccaacggca taatgggaaa ctgtgtaggg gtcaaagcac gagtcatccg taggttggtt 240
caagccttcg ttgacagagt tgcccacggt aacaacctcn tccccgaacc ttatgcctct 300
gctgggcttt cagngcctcc actatgatgn tgtagggggg cacctctggn gangacctcg 360
gccgcgacca cgct 374

<210> 190
<211> 373
<212> DNA
<213> Homo sapiens

<220>
<221> misc_feature
<222> 247, 304, 306, 332, 337
<223> n = A,T,C or G

<400> 190
agcgtggtcg cggccgaggt cctcaccaga ggtgccacct acaacatcat agtggaggca 60
ctgaaagacc agcagaggca taaggctcgg gaagaggttg ttaccgtggg caactctgtc 120
aacgaaggct tgaaccaacc tacggatgac tcgtgctttg acccctacac agtttcccat 180
tatgccgttg gagatgagtg ggaacgaatg tctgaatcag gctttaaact gttgtgccag 240
tgcttangct ttggaagtgg gtcatttcag atgtgattca tctagatggt gccatgacaa 300

tggngngaac tacaagattg gagagaagtg gnaccgncag ggagaaaatg gacctgcccg 360
ggcggccgct cga 373

<210> 191
<211> 354
<212> DNA
<213> Homo sapiens

<220>
<221> misc_feature
<222> 218, 299, 306, 326, 333, 337, 341
<223> n = A,T,C or G

<400> 191
agcgtggtcg cggccgaggt ccacatcggc agggtcggag ccctggccgc catactcgaa 60
ctggaatcca tcggtcatgc tctcgccgaa ccagacatgc ctcttgtcct tggggttctt 120
gctgatgtac cagttcttct gggccacact gggctgagtg gggtacacgc aggtctcacc 180
agtctccatg ttgcagaaga ctttgatggc atccaggntg caaccttggt tggggtcaat 240
ccagtactct ccactcttcc agccagagtg gcacatcttg aggtcacggc aggtgcggnc 300
gggggntttt gcggctgccc tctggncttc ggntgtnctc natctgctgg ctca 354

<210> 192
<211> 587
<212> DNA
<213> Homo sapiens

<220>
<221> misc_feature
<222> 276
<223> n = A,T,C or G

<400> 192
tcgagcggcc gcccgggcag gtctcgcggt cgcactggtg atgctggtcc tgttggtccc 60
cccggccctc ctggacctcc tggcccccct ggtcctccca gcgctggttt cgacttcagc 120
ttcctgcccc agccacctca agagaaggct cacgatggtg gccgctacta ccgggctgat 180
gatgccaatg tggttcgtga ccgtgacctc gaggtggaca ccaccctcaa gagcctgagc 240
cagcagatcg agaacatccg gagcccagag ggcagncgca agaaccccgc ccgcacctgc 300
cgtgacctca agatgtgcca ctctgactgg aagagtggag agtactggat tgaccccaac 360
caagctgcaa cctggatgcc atcaaagtct tctgcaacat ggagactggt gagacctgcg 420
tgtaccccac tcagcccagt gtggcccaaa agaactggta catcagcaag aaccccaagg 480
acaagaagca tgtctggttc ggcgagaaca tgaccgatgg attccagttc gagtatggcg 540
ggcagggctc cgaccctgcc gatggggacc ttggccgcga acacgct 587

<210> 193
<211> 98
<212> DNA
<213> Homo sapiens

<220>
<221> misc_feature
<222> 8, 9, 33, 58, 71, 90
<223> n = A,T,C or G

<400> 193
agcgtggnng cggccgaggt ataaatatcc agnccatatc ctccctccac acgctganag 60
atgaagctgt ncaaagatct cagggtggan aaaaccat 98

<210> 194
<211> 240
<212> DNA
<213> Homo sapiens

<400> 194
tcgagcggcc gcccgggcag gtccttcaga cttggactgt gtcacactgc caggcttcca 60
gggctccaac ttgcagacgg cctgttgtgg gacagtctct gtaatcgcga aagcaaccat 120
ggaagacctg ggggaaaaca ccatggtttt atccaccctg agatctttga acaacttcat 180
ctctcagcgt gcggagggag gctctggact ggatatttct acctcggccg cgaccacgct 240


<210> 195
<211> 400
<212> DNA
<213> Homo sapiens

<220>
<221> misc_feature
<222> 22, 37, 39, 105, 268, 276, 302, 323, 331, 335, 347, 351,
371, 378
<223> n = A,T,C or G

<400> 195
cgagcgggcg accgggcagg tncagactcc aatccanana accatcaagc cagatgtcag 60
aagctacacc atcacaggtt tacaaccagg cactgactac aaganctacc tgcacacctt 120
gaatgacaat gctcggagct cccctgtggt catcgacgcc tccactgcca ttgatgcacc 180
atccaacctg cgtttcctgg ccaccacacc caattccttg ctggtatcat ggcagccgcc 240
acgtgccagg attaccggta catcatcnag tatganaagc ctgggcctcc tcccagagaa 300
gnggtccctc ggccccgccc tgntgtccca naggntacta ttactgngcc ngcaaccggc 360
aaccgatatc nattttgnca ttggccttca acaataatta 400

<210> 196
<211> 494
<212> DNA
<213> Homo sapiens

<220>
<221> misc_feature
<222> 19, 83, 168, 252, 271, 292, 430
<223> n = A,T,C or G

<400> 196
agcgtggttc gcggccgang tcctgtcaga gtggcactgg tagaagttcc aggaaccctg 60
aactgtaagg gttcttcatc agngccaaca ggatgacatg aaatgatgta ctcagaagtg 120
tcctggaatg gggcccatga gatggttgtc tgagagagag cttcttgncc tgtctttttc 180
cttccaatca ggggctcgct cttctgatta ttcttcaggg caatgacata aattgtatat 240
tcgggtcccg gntccaggcc agtaatagta ncctctgtga caccagggcg gngccgaggg 300
accacttctc tgggaggaga cccaggcttc tcatacttga tgatgtaacc ggtaatcctg 360
gcacgtggcg gctgccatga taccagcaag gaattggggt gtggtggcca ggaaacgcag 420
gttggatggn gcatcaatgg cagtggaggc cgtcgatgac cacaggggga gctccgacat 480
tgtcattcaa ggtg 494

<210> 197
<211> 118
<212> DNA
<213> Homo sapiens

<220>
<221> misc_feature
<222> 8, 71, 96
<223> n = A,T,C or G

<400> 197
agcgtggncg cggccgaggt gcagcgcggg ctgtgccacc ttctgctctc tgcccaacga 60
taaggagggt ncctgccccc aggagaacat taactntccc cagctcggcc tctgccgg 118

<210> 198
<211> 403
<212> DNA
<213> Homo sapiens

<220>
<221> misc_feature
<222> 41, 53, 98, 195, 350
<223> n = A,T,C or G

<400> 198
tcgagcggcc gcccgggcag gttttttttg ctgaaagtgg ntactttatt ggntgggaaa 60
gggagaagct gtggtcagcc caagagggaa tacagagncc cgaaaaaggg gagggcaggt 120
gggctggaac cagacgcagg gccaggcaga aactttctct cctcactgct cagcctggtg 180
gtggctggag ctcanaaatt gggagtgaca caggacacct tcccacagcc attgcggcgg 240
catttcatct ggccaggaca ctggctgtcc acctggcact ggtcccgaca gaagcccgag 300
ctggggaaag ttaatgttca cctgggggca ggaaccctcc ttatcattgn gcagagagca 360
gaaggtggca cagcccgcgc tgcacctcgg ccgcgaccac gct 403

<210> 199
<211> 167
<212> DNA
<213> Homo sapiens

<220>
<221> misc_feature
<222> 92, 107
<223> n = A,T,C or G

<400> 199
tcgagcggcc gcccgggcag gtccaccata agtcctgata caaccacgga tgagctgtca 60
ggagcaaggt tgatttcttt cattggtccg gncttctcct tgggggncac ccgcactcga 120
tatccagtga gctgaacatt gggtggcgtc cactgggcgc tcaggct 167

<210> 200
<211> 252
<212> DNA
<213> Homo sapiens

<220>
<221> misc_feature
<222> 210, 226, 227, 230, 236
<223> n = A,T,C or G

<400> 200
tcgagcggtt cgcccgggca ggtccaccac acccaattcc ttgctggtat catggcagcc 60
gccacgtgcc aggattaccg gctacatcat caagtatgag aagcctgggt ctcctcccag 120

agaagcggtc cctcggcccc gccctggtgt cacagaggct actattactg gcctggaacc 180
gggaaccgaa tatacaattt atgtcattgn cctgaagaat aatcannaan agcgancccc 240
tgattggaag ga 252

<210> 201
<211> 91
<212> DNA
<213> Homo sapiens

<400> 201
agcgtggtcg cggccgaggt tgtacaagct tttttttttt tttttttttt tttttttttt 60
tttttttttt tttttttttt tttttttttt t 91

<210> 202
<211> 368
<212> DNA
<213> Homo sapiens

<220>
<221> misc_feature
<222> 9, 354
<223> n = A,T,C or G

<400> 202
tcgagcggnc gcccgggcag gtctgccaac accaagattg gcccccgccg catccacaca 60
gtccgtgtgc ggggaggtaa caagaaatac cgtgccctga ggttggacgt ggggaatttc 120
tcctggggct cagagtgttg tactcgtaaa acaaggatca tcgatgttgt ctacaatgca 180
tctaataacg agctggttcg taccaagacc ctggtgaaga attgcatcgt gctcatcgac 240
agcacaccgt accgacagtg gtacgagtcc cactatgcgc tgcccctggg ccgcaagaag 300
ggagccaagc tgactcctga ggaagaagag attttaaaca aaaaacgatc taanaaaaaa 360
aaaacaat 368

<210> 203
<211> 340
<212> DNA
<213> Homo sapiens

<400> 203
agcgtggtcg cggccgaggt gaaatggtat tcagcttcct ggcacttctg gtcagcaacc 60
cagtgttggg caacaaatga tctttgagga acatggtttt aggcggacca caccgcccac 120
aacggccacc cccataaggc ataggccaag accatacccg ccgaatgtag gacaagaagc 180
tctctctcag acaaccatct catgggcccc attccaggac acttctgagt acatcatttc 240
atgtcatcct gttggcactg atgaagaacc cttacagttc agggttcctg gaacttctac 300
cagtgccact ctgacaggac ctgcccgggc ggccgctcga 340

<210> 204
<211> 341
<212> DNA
<213> Homo sapiens

<400> 204
tcgagcggcc gcccgggcag gtcctgtcag agtggcactg gtagaagttc caggaaccct 60
gaactgtaag ggttcttcat cagtgccaac aggatgacat gaaatgatgt actcagaagt 120
gtcctggaat ggggcccatg agatggttgt ctgagagaga gcttcttgtc ctacattcgg 180
cgggtatggt cttggcctat gccttatggg ggtggccgtt gtgggcggtg tggtccgcct 240
aaaaccatgt tcctcaaaga tcatttgttg cccaacactg ggttgctgac cagaagtgcc 300
aggaagctga ataccatttc acctcggccg cgaccacgct a 341

<210> 205
<211> 770
<212> DNA
<213> Homo sapiens

<220>
<221> misc_feature
<222> 529, 591, 623, 626, 629, 630, 656, 702, 709, 712, 717, 743,
746, 749, 759, 762, 766
<223> n = A,T,C or G

<400> 205
tcgagcggcc gcccgggcag gtctcccttc ttgcggccca ggggcagcgc atagtgggac 60
tcgtaccact gtcggtacgg tgtgctgtcg atgagcacga tgcaattctt caccagggtc 120
ttggtacgaa ccagctcgtt attagatgca ttgtagacaa catcgatgat ccttgtttta 180
cgagtacaac actctgagcc ccaggagaaa ttccccacgt ccaacctcag ggcacggtat 240
ttcttgttac ctccccgcac acggactgtg tggatgcggc gggggccaag ctgactcctg 300
aggaagaaga gattttaaac aaaaaacgat ctaaaaaaat tcagaagaaa tatgatgaaa 360
ggaaaaagaa tgccaaaatc agcagtctcc tggaggagca gttccagcag ggcaagcttc 420
ttgcgtgcat cgcttcaagg ccgggacagt gtgaccgagc agatggctat gtgctagagg 480
gcaaagaagt ggagttctat cttaagaaaa tcagggccca gaatggtgng tcttcaacta 540
atccaaaggg gagtttcaga ccagtgcaat cagcaaaaac attgatactg ntggccaaat 600
ttattggtgc agggcttgca cantangann ggctgggtct tggggcttgg attggnacaa 660
gctttggcag ccttttcttt ggttttgcca aaaacctttt gntgaagang anacctnggg 720
cggacccctt aaccgattcc acnccnggng gcgttctang gncccncttg 770

<210> 206
<211> 810
<212> DNA
<213> Homo sapiens

<220>
<221> misc_feature
<222> 574, 621, 625, 636, 668, 673, 704, 728, 743, 767, 772, 786,
789, 807, 809, 810
<223> n = A,T,C or G

<400> 206
agcgtggtcg cggccgaggt ctgctgcttc agcgaagggt ttctggcata accaatgata 60
aggctgccaa agactgttcc aataccagca ccagaaccag ccactcctac tgttgcagca 120
cctgcaccaa taaatttggc agcagtatca atgtctctgc tgattgcact ggtctgaaac 180
tccctttgga ttagctgaga cacaccattc tgggccctga ttttcctaag atagaactcc 240
aactctttgc cctctagcac atagccatct gctcggtcac actgtcccgg ccttgaagcg 300
atgcacgcaa gaagcttgcc ctgctggaac tgctcctcca ggagactgct gattttggca 360
ttctttttcc tttcatcata tttcttctga atttttttag atcgtttttt gtttaaaatc 420
tcttcttcct caggagtcag cttggccccc gccgcatcca cacagtccgt gtgcggggag 480
gtaacaagaa ataccgtgcc ctgaggttgg acgtggggaa tttctcctgg ggctcagagt 540
ggtgtactcg taaaacaagg atcatcgatg gtgnctacaa tgcatctaat aacgagctgg 600
gtcggaccca aagaacctgg ngaanaaatg gatcgnctca tcgacaggac accgtacccg 660
acaggggnac gantcccact atgcgcttgc ccctgggccg caanaaagga aaactgcccg 720
ggcggccntc gaaagcccaa ttntggaaaa aatccatcac actgggnggc cngtcgagca 780
tgcatntana ggggcccatt ccccctnann 810

<210> 207
<211> 257
<212> DNA

<213> Homo sapiens

<400> 207
tcgagcggcc gcccgggcag gtccccaacc aaggctgcaa cctggatgcc atcaaagtct 60
tctgcaacat ggagactggt gagacctgcg tgtaccccac tcagcccagt gtggcccaga 120
agaactggta catcagcaag aaccccaagg acaagaggca tgtctggttc ggcgagagca 180
tgaccgatgg attccagttc gagtatggcg gccagggctc cgaccctgcc gatgtggacc 240
tcggccgcga ccacgct 257

<210> 208
<211> 257
<212> DNA
<213> Homo sapiens

<400> 208
agcgtggtcg cggccgaggt ccacatcggc agggtcggag ccctggccgc catactcgaa 60
ctggaatcca tcggtcatgc tctcgccgaa ccagacatgc ctcttgtcct tggggttctt 120
gctgatgtac cagttcttct gggccacact gggctgagtg gggtacacgc aggtctcacc 180
agtctccatg ttgcagaaga ctttgatggc atccaggttg cagccttggt tggggacctg 240
cccgggcggc cgctcga 257

<210> 209
<211> 747
<212> DNA
<213> Homo sapiens

<220>
<221> misc_feature
<222> 453, 538, 540, 542, 546, 554, 556, 598, 659, 670, 679, 689,
693, 711, 723, 724, 731, 747
<223> n = A,T,C or G

<400> 209
tcgagcggcc gcccgggcag gtccaccaca cccaattcct tgctggtatc atggcagccg 60
ccacgtgcca ggattaccgg ctacatcatc aagtatgaga agcctgggtc tcctcccaga 120
gaagtggtcc ctcggccccg ccctggtgtc acagaggcta ctattactgg cctggaaccg 180
ggaaccgaat atacaattta tgtcattgcc ctgaagaata atcagaagag cgagcccctg 240
attggaagga aaaagacaga cgagcttccc caactggtaa cccttccaca ccccaatctt 300
catggaccag agatcttgga tgttccttcc acagttcaaa agaccccttt cgtcacccac 360
cctgggtatg acactggaaa tggtattcag cttcctggca cttctggtca gcaacccagt 420
gttgggcaac aaatgatctt tgaggaacat ggntttaggc ggaccacacc gcccacaacg 480
gccaccccca taaggcatag gccaagacca tacccgccga atgtaggaca agaagctntn 540
tntcanacac catntnatgg gccccattcc aggacacttc tgagtacatc atttatgnca 600
tctgtggcac ttgatgaaaa cccttacagt tcagggttct ggaactttta ccaggcctnt 660
tacaggactn ggccggacnc cttaagccna ttncaccctg gggcgttcta nggtcccact 720
cgnncactgg ngaaaatggc tactgtn 747

<210> 210
<211> 872
<212> DNA
<213> Homo sapiens

<220>
<221> misc_feature
<222> 165, 174, 181, 256, 260, 269, 271, 277, 286, 289, 294, 298,
300, 301, 303, 308, 311, 321, 325, 328, 329, 333, 338, 342,
346, 349, 351, 357, 359, 364, 366, 379, 385, 395, 396, 397,

407, 408, 410, 414, 415, 429, 431, 434, 435, 440, 443
<223> n = A,T,C or G

<220>
<221> misc_feature
<222> 444, 446, 447, 448, 449, 450, 451, 464, 470, 472, 475, 479,
483, 484, 485, 488, 494, 496, 497, 504, 508, 509, 511, 513,
517, 522, 524, 526, 532, 533, 542, 543, 553, 559, 566, 567,
571, 572, 578, 582, 588, 591, 594, 595, 596, 600, 606
<223> n = A,T,C or G

<220>
<221> misc_feature
<222> 612, 614, 617, 618, 629, 630, 631, 652, 654, 655, 661, 663,
664, 666, 671, 673, 678, 679, 681, 688, 690, 691, 698, 706,
707, 708, 714, 719, 721, 723, 726, 741, 751, 761, 762, 769,
770, 778, 779, 781, 782, 785, 791, 802, 807, 808, 812
<223> n = A,T,C or G

<220>
<221> misc_feature
<222> 815, 820, 827, 828, 838, 841, 844, 851, 857, 864, 866, 869,
872
<223> n = A,T,C or G

<400> 210
agcgtggtcg cggccgaggt ccactagagg tctgtgtgcc attgcccagg cagagtctct 60
gcgttacaaa ctcctaggag ggcttgctgt gcggagggcc tgctatggtg tgctgcggtt 120
catcatggag agtggggcca aaggctgcga ggttgtggtg tctgngaaac tccnaggaca 180
ngagggctaa attccatgaa gtttgtggat ggcctgatga tccacaatcg gagaccctgt 240
taactactac cgtctnaccn cctgctgtnc ncccccnttt ctgctnaana catngggntn 300
ntncttgncc ntccttgggt ngaanatnna atngcctncc cnttcntanc nctactngnt 360
ccananttgg cctttaaana atccnccttg ccttnnncac tgttcanntn tttnntcgta 420
aaccctatna nttnnattan atnntnnnnn nctcaccccc ctcntcattn anccnatang 480
ctnnnaantc cttnanncct cccncccnnt ncnctcntac tnantncttc tnncccatta 540
cnnagctctt tcntttaana taatgnngcc nngctctnca tntctacnat ntgnnnaatn 600
cccccncccc cnancgnntt tttgacctnn naacctcctt tcctcttccc tncnnaaatt 660
ncnnanttcc ncnttccnnc ntttcggntn ntcccatnct ttccannnct tcantctanc 720
ncnctncaac ttattttcct ntcatccctt nttctttaca nnccccctnn tctactcnnc 780
nnttncatta natttgaaac tnccacnnct anttncctcn ctctacnntt ttattttncg 840
ntcnctctac ntaatanttt aatnanttnt cn 872

<210> 211
<211> 517
<212> DNA
<213> Homo sapiens

<220>
<221> misc_feature
<222> 462, 464, 506
<223> n = A,T,C or G

<400> 211
tcgagcggcc gcccgggcag gtctgccaag gagaccctgt tatgctgtgg ggactggctg 60
gggcatggca ggcggctctg gcttcccacc cttctgttct gagatggggg tggtgggcag 120
tatctcatct ttgggttcca caatgctcac gtggtcaggc aggggcttct tagggccaat 180
cttaccagtt gggtcccagg gcagcatgat cttcaccttg atgcccagca caccctgtct 240

gagcaacacg tggcgcacaa gcagtgtcaa cgtagtaagt taacagggtc tccgctgtgg 300
atcatcaggc catccacaaa cttcatggat ttagccctct gtcctcggag tttcccagac 360
accacaacct cgcagccttt ggccccactc tccatgatga accgcagcac accatagcag 420
gccctccgca caagcaagcc ctcctaagaa tttgtaacgc ananactctg ctggcaatgg 480
cacacaaacc tctagtggac ctcggncgcg accacgc 517

<210> 212
<211> 695
<212> DNA
<213> Homo sapiens

<220>
<221> misc_feature
<222> 432, 476, 522, 547, 621, 624, 647, 679
<223> n = A,T,C or G

<400> 212
tcgagcggcc gcccgggcag gtctggtcca ggatagcctg cgagtcctcc tactgctact 60
ccagacttga catcatatga atcatactgg ggagaatagt tctgaggacc agtagggcat 120
gattcacaga ttccaggggg gccaggagaa ccaggggacc ctggttgtcc tggaatacca 180
gggtcaccat ttctcccagg aataccagga gggcctggat ctcccttggg gccttgaggt 240
ccttgaccat taggagggcg agtaggagca gttggaggct gtgggcaaac tgcacaacat 300
tctccaaatg gaatttctgg gttggggcag tctaattctt gatccgtcac atattatgtc 360
atcgcagaga acggatcctg agtcacagac acatatttgg catggttctg gcttccagac 420
atctctatcc gncataggac tgaccaagat gggaacatcc tccttcaaca agcttnctgt 480
tgtgccaaaa ataatagtgg gatgaagcag accgagaagt anccagctcc cctttttgca 540
caaagcntca tcatgtctaa atatcagaca tgagacttct ttgggcaaaa aaggagaaaa 600
agaaaaagca gttcaaagta nccnccatca agttggttcc ttgcccnttc agcacccggg 660
ccccgttata aaacacctng ggccggaccc ccctt 695

<210> 213
<211> 804
<212> DNA
<213> Homo sapiens

<220>
<221> misc_feature
<222> 552, 555, 592, 624, 629, 633, 658, 695, 697, 698, 700, 702,
745, 753, 755, 762, 773, 786, 788, 793, 795
<223> n = A,T,C or G

<400> 213
agcgtggtcg cggccgaggt gttttatgac gggcccggtg ctgaagggca gggaacaact 60
tgatggtgct actttgaact gcttttcttt tctccttttt gcacaaagag tctcatgtct 120
gatatttaga catgatgagc tttgtgcaaa aggggagctg gctacttctc gctctgcttc 180
atcccactat tattttggca caacaggaag ctgttgaagg aggatgttcc catcttggtc 240
agtcctatgc ggatagagat gtctggaagc cagaaccatg ccaaatatgt gtctgtgact 300
caggatccgt tctctgcgat gacataatat gtgacgatca agaattagac tgccccaacc 360
cagaaattcc atttggagaa tgttgtgcag tttgcccaca gcctccaact gctcctactc 420
gccctcctaa tggtcaagga cctcaaggcc ccaagggaga tccaggccct cctggtattc 480
ctgggagaaa tggtgaccct ggtattccag gacaaccagg gtcccctggt tctcctggcc 540
cccctggaat cnggngaatc atgccctact ggtcctcaaa ctattctccc anatgattca 600
tatgatgtca agtctgggat agcnagtang ganggactcg caggctattc tggaccanac 660
ctgccggggg ggcgttcgaa agcccgaatc tgcanannt n cnttcacact ggcggccgtc 720
gagctgcttt aaaagggcca ttccnccttt agngnggggg antacaatta ctnggcggcg 780
ttttanancg cgngnctggg aaat 804

<210> 214
<211> 594
<212> DNA
<213> Homo sapiens

<220>
<221> misc_feature
<222> 452, 509, 585
<223> n = A,T,C or G

<400> 214
agcgtggtcg cggccgaggt ccacatcggc agggtcggag ccctggccgc catactcgaa 60
ctggaatcca tcggtcatgc tctcgccgaa ccagacatgc ctcttgtcct tggggttctt 120
gctgatgtac cagttcttct gggccacact gggctgagtg gggtacacgc aggtctcacc 180
agtctccatg ttgcagaaga ctttgatggc atccaggttg cagccttggt tggggtcaat 240
ccagtactct ccactcttcc agtcagagtg gcacatcttg aggtcacggc aggtgcgggc 300
ggggttcttg cggctgccct ctgggctccg gatgttctcg atctgctggc tcaggctctt 360
gagggtggtg tccacctcga ggtcacggtc acgaaccaca ttggcatcat cagcccggta 420
gtagcggcca ccatcgtgag ccttctcttg angtggctgg ggcaggaact gaagtcgaaa 480
ccagcgctgg gaggaccagg gggaccaana ggtccaggaa gggcccgggg gggaccaaca 540
ggaccagcat caccaagtgc gacccgcgag aacctgcccg gccgnccgct cgaa 594

<210> 215
<211> 590
<212> DNA
<213> Homo sapiens

<220>
<221> misc_feature
<222> 8, 9
<223> n = A,T,C or G

<400> 215
tcgagcgnnc gcccgggcag gtctcgcggt cgcactggtg atgctggtcc tgttggtccc 60
cccggccctc ctggacctcc tggtccccct ggtcctccca gcgctggttt cgacttcagc 120
ttcctgcccc agccacctca agagaaggct cacgatggtg gccgctacta ccgggctgat 180
gatgccaatg tggttcgtga ccgtgacctc gaggtggaca ccaccctcaa gagcctgagc 240
cagcagatcg agaacatccg gagcccagag ggcagccgca agaaccccgc ccgcacctgc 300
cgtgacctca agatgtgcca ctctgactgg aagagtggag agtactggat tgaccccaac 360
caaggctgca acctggatgc catcaaagtc ttctgcaaca tggagactgg tgagacctgc 420
gtgtacccca ctcagcccag tgtggcccag aagaactggt acatcagcaa gaaccccaag 480
gacaagaggc atgtctggtt cggcgagagc atgaccgatg gattccagtt cgagtatggc 540
ggccagggct cccaccctgc cgatgtggac ctccggccgc gaccaccctt 590

<210> 216
<211> 801
<212> DNA
<213> Homo sapiens

<220>
<221> misc_feature
<222> 2, 22, 25, 26, 328, 373, 385, 440, 473, 534, 571, 572, 573,
582, 587, 589, 593, 600, 605, 617, 633, 642, 653, 672, 681,
685, 696, 699, 709, 715, 717, 726, 731, 739, 742, 745, 758,
769, 772, 778, 780, 788, 789, 791, 793, 796
<223> n = A,T,C or G

<400> 216
tngagcggcc gcccgggcag gntgnnaacg ctggtcctgc tggtcctcct ggcaaggctg 60
gtgaagatgg tcaccctgga aaacccggac gacctggtga gagaggagtt gttggaccac 120
agggtgctcg tggtttccct ggaactcctg gacttcctgg cttcaaaggc attaggggac 180
acaatggtct ggatggattg aagggacagc ccggtgctcc tggtgtgaag ggtgaacctg 240
gtgcccctgg tgaaaatgga actccaggtc aaacaggagc ccgtgggctt cctggtgaga 300
gaggaccgtg ttggtgcccc tggcccanac ctcggccgcg accacgctaa gcccgaattt 360
ccagcacact ggnggccgtt actantggat ccgagctcgg taccaagctt ggcgtaatca 420
tggtcatagc tgtttcctgn gtgaaattgt tatccgctca caatttcaca cancatacga 480
agccggaaag cataaagtgt aaagccttgg ggtgctaatg agtgagctaa ctcncattaa 540
attgcgttgc gctcactgcc cgcttttcca nnngggaaac cntggcntng ccngcttgcn 600
ttaantgaaa tccgccnacc cccggggaaa agncggtttg cngtattggg gcnctttttc 660
cctttcctcg gnttacttga nttantgggc tttggncgnt tcgggttgng gcgancnggt 720
tcaacntcac nccaaaggng gnaanacggt tttcccanaa tccgggggnt ancccaangn 780
aaaacatnng ncnaangggc t 801

<210> 217
<211> 349
<212> DNA
<213> Homo sapiens

<220>
<221> misc_feature
<222> 10, 157, 170
<223> n = A,T,C or G

<400> 217
agcgtggttn gcggccgagg tctgggccag gggcaccaac acgtcctctc tcaccaggaa 60
gcccacgggc tcctgtttga cctggagttc cattttcacc aggggcacca ggttcaccct 120
tcacaccagg agcaccgggc tgtcccttca atccatncag accattgtgn cccctaatgc 180
ctttgaagcc aggaagtcca ggagttccag ggaaaccacc gagcaccctg tggtccaaca 240
actcctctct caccaggtcg tccgggtttt ccagggtgac catcttcacc agccttgcca 300
ggaggaccag caggaccagc gttaccaacc tgcccgggcg gccgctcga 349

<210> 218
<211> 372
<212> DNA
<213> Homo sapiens

<400> 218
tcgagcggcc gcccgggcag gtccattttc tccctgacgg tcccacttct ctccaatctt 60
gtagttcaca ccattgtcat ggcaccatct agatgaatca catctgaaat gaccacttcc 120
aaagcctaag cactggcaca acagtttaaa gcctgattca gacattcgtt cccactcatc 180
tccaacggca taatgggaaa ctgtgtaggg gtcaaagcac gagtcatccg taggttggtt 240
caagccttcg ttgacagagt tgcccacggt aacaacctct tcccgaacct tatgcctctg 300
ctggtctttc agtgcctcca ctatgatgtt gtaggtggca cctctggtga ggacctcggc 360
cgcgaccacg ct 372

<210> 219
<211> 374
<212> DNA
<213> Homo sapiens

<400> 219
agcgtggtcg cggccgaggt cctcaccaga ggtgccacct acaacatcat agtggaggca 60
ctgaaagacc agcagaggca taaggttcgg gaagaggttg ttaccgtggg caactctgtc 120
aacgaaggct tgaaccaacc tacggatgac tcgtgctttg acccctacac agtttcccat 180

tatgccgttg gagatgagtg ggaacgaatg tctgaatcag gctttaaact gttgtgccag 240
tgcttaggct ttggaagtgg tcatttcaag atgtgattca tctagatggt gccatgacaa 300
tggtgtgaac tacaagattg gagagaagtg ggaccgtcag ggagaaaatg gacctgcccg 360
ggccggccgc tcga 374

<210> 220
<211> 828
<212> DNA
<213> Homo sapiens

<220>
<221> misc_feature
<222> 8, 9, 557, 571, 587, 588, 601, 642, 643, 647, 654, 664, 681,
688, 698, 719, 720, 725, 734, 738, 743, 744, 757, 765, 773,
778, 780, 782, 783, 793, 798, 805, 809, 822, 827
<223> n = A,T,C or G

<400> 220
tcgagcgnnc gcccgggcag gtccagtagt gccttcggga ctgggttcac ccccaggtct 60
gcggcagttg tcacagcgcc agccccgctg gcctccaaag catgtgcagg agcaaatggc 120
accgagatat tccttctgcc actgttctcc tacgtggtat gtcttcccat catcgtaaca 180
cgttgcctca tgagggtcac acttgaattc tccttttccg ttcccaagac atgtgcagct 240
catttggctg gctctatagt ttggggaaag tttgttgaaa ctgtgccact gacctttact 300
tcctccttct ctactggagc tttcgtacct tccacttctg ctgttggtaa aatggtggat 360
cttctatcaa tttcattgac agtacccact tctcccaaac atccagggaa atagtgattt 420
cagagcgatt aggagaacca aattatgggg cagaaataag gggcttttcc acaggttttc 480
ctttggagga agatttcagt ggtgacttta aaagaatact caacagtgtc ttcatcccca 540
tagcaaaaga agaaacngta aatgatggaa ngcttctgga gatgccnnca tttaagggac 600
ncccagaact tcaccatcta caggacctac ttcagtttac annaagncac atantctgac 660
tcanaaagga cccaagtagc nccatggnca gcactttnag cctttcccct ggggaaaann 720
ttacnttctt aaancctngg ccnngacccc cttaagncca aattntggaa aanttccntn 780
cnnctggggg gcngttcnac atgcntttna agggcccaat tnccccnt 828

<210> 221
<211> 476
<212> DNA
<213> Homo sapiens

<400> 221
tcgagcggcc gcccgggcag gtgtcggagt ccagcacggg aggcgtggtc ttgtagttgt 60
tctccggctg cccattgctc tcccactcca cggcgatgtc gctgggatag aagcctttga 120
ccaggcaggt caggctgacc tggttcttgg tcatctcctc ccgggatggg ggcagggtgt 180
acacctgtgg ttctcggggc tgccctttgg ctttggagat ggttttctcg atgggggctg 240
ggagggcttt gttggagacc ttgcacttgt actccttgcc attcagccag tcctggtgca 300
ggacggtgag gacgctgacc acacggtacg tgctgttgta ctgctcctcc cgcggctttg 360
tcttggcatt atgcacctcc acgccgtcca cgtaccagtt gaacttgacc tcagggtctt 420
cgtggctcac gtccaccacc acgcatgtaa cctcagacct cggccgcgac cacgct 476

<210> 222
<211> 477
<212> DNA
<213> Homo sapiens

<400> 222
   agcgtggtcg cggccgaggt ctgaggttac atgcgtggtg gtggacgtga gccacgaaga 60
   ccctgaggtc aagttcaact ggtacgtgga cggcgtggag gtgcataatg ccaagacaaa 120
   gccgcgggag gagcagtaca acagcacgta ccgtgtggtc agcgtcctca ccgtcctgca 180
   ccaggactgg ctgaatggca aggagtacaa gtgcaaggtc tccaacaaag ccctcccagc 240
   ccccatcgag aaaaccatct ccaaagccaa agggcagccc cgagaaccac aggtgtacac 300
   cctgccccca tcccgggagg agatgaccaa gaaccaggtc agcctgacct gcctggtcaa 360
   aggcttctat cccagcgaca tcgccgtgga gtgggagagc aatgggcagc cggagaacaa 420
   ctacaagacc acgcctcccg tgctggactc cgacacctgc ccgggcggcc cgctcga 477

<210> 223
<211> 361
<212> DNA
<213> Homo sapiens

<400> 223
   tcgagcggcc gcccgggcag gttgaatggc tcctcgctga ccaccccggt gctggtggtg 60
   ggtacagagc tccgatgggt gaaaccattg acatagagac tgtccctgtc cagggtgtag 120
   gggcccagct cagtgatgcc gtgggtcagc tggctcagct tccagtacag ccgctctctg 180
   tccagtccag ggcttttggg gtcaggacga tgggtgcaga cagcatccac tctggtggct 240
   gccccatcct tctcaggcct gagcaaggtc agtctgcaac cagagtacag agagctgaca 300
   ctggtgttct tgaacaaggg cataagcaga ccctgaagga cacctcggcc gcgaccacgc 360
   t 361

<210> 224
<211> 361
<212> DNA
<213> Homo sapiens

<400> 224
   agcgtggtcg cggccgaggt gtccttcagg gtctgcttat gcccttgttc aagaacacca 60
   gtgtcagctc tctgtactct ggttgcagac tgaccttgct caggcctgag aaggatgggg 120
   cagccaccag agtggatgct gtctgcaccc atcgtcctga ccccaaaagc cctggactgg 180
   acagagagcg gctgtactgg aagctgagcc agctgaccca cggcatcact gagctgggcc 240
   cctacaccct ggacagggac agtctctatg tcaatggttt cacccatcgg agctctgtac 300
   ccaccaccag caccggggtg gtcagcgagg agccattcaa cctgcccggg cggccgctcg 360
   a 361

<210> 225
<211> 766
<212> DNA
<213> Homo sapiens

<220>
<221> misc_feature
<222> 574, 610, 631, 643, 657, 660, 666, 688, 712, 735, 747
<223> n = A,T,C or G

<400> 225
agcgtggtcg cggccgaggt cctgtcagag tggcactggt agaagttcca ggaaccctga 60
actgtaaggg ttcttcatca gtgccaacag gatgacatga aatgatgtac tcagaagtgt 120
cctggaatgg ggcccatgag atggttgtct gagagagagc ttcttgtcct acattcggcg 180
ggtatggtct tggcctatgc cttatggggg tggccgttgt gggcggtgtg gtccgcctaa 240
aaccatgttc ctcaaagatc atttgttgcc caacactggg ttgctgacca gaagtgccag 300
gaagctgaat accatttcca gtgtcatacc cagggtgggt gacgaaaggg gtcttttgaa 360
ctgtggaagg aacatccaag atctctggtc catgaagatt ggggtgtgga agggttacca 420
gttggggaag ctcgtctgtc tttttccttc caatcagggg ctcgctcttc tgattattct 480
tcagggcaat gacataaatt gtatattcgg tcccggttcc aggccagtaa tagtagcctc 540
tgtgacacca gggcggggcc gagggaccct tctnttggaa gagaccagct tctcatactt 600
gatgatgagn ccggtaatcc tggcacgtgg nggttgcatg atnccaccaa ggaaatnggn 660
gggggnggac ctgcccggcg gccgttcnaa agcccaattc cacacacttg gnggccgtac 720

tatggatccc actcngtcca acttggngga atatggcata actttt 766

<210> 226
<211> 364
<212> DNA
<213> Homo sapiens

<400> 226
tcgagcggcc gcccgggcag gtccttgacc ttttcagcaa gtgggaaggt gtaatccgtc 60
tccacagaca aggccaggac tcgtttgtac ccgttgatga tagaatgggg tactgatgca 120
acagttgggt agccaatctg cagacagaca ctggcaacat tgcggacacc ctccaggaag 180
cgagaatgca gagtttcctc tgtgatatca agcacttcag ggttgtagat gctgccattg 240
tcgaacacct gctggatgac cagcccaaag gagaaggggg agatgttgag catgttcagc 300
agcgtggctt cgctggctcc cactttgtct ccagtcttga tcagacctcg gccgcgacca 360
cgct 364

<210> 227
<211> 275
<212> DNA
<213> Homo sapiens

<400> 227
agcgtggtcg cggccgaggt ctgtcctaca gtcctcagga ctctactccc tcagcagcgt 60
ggtgaccgtg ccctccagca acttcggcac ccagacctac acctgcaacg tagatcacaa 120
gcccagcaac accaaggtgg acaagagagt tgagcccaaa tcttgtgaca aaactcacac 180
atgcccaccg tgcccagcac ctgaactcct ggggggaccg tcagtcttcc tcttcccccg 240
catccccctt ccaaacctgc ccgggcggcc gctcg 275

<210> 228
<211> 275
<212> DNA
<213> Homo sapiens

<400> 228
cgagcggccg cccgggcagg tttggaaggg ggatgcgggg gaagaggaag actgacggtc 60
cccccaggag ttcaggtgct gggcacggtg ggcatgtgtg agttttgtca caagatttgg 120
gctcaactct cttgtccacc ttggtgttgc tgggcttgtg atctacgttg caggtgtagg 180
tctgggtgcc gaagttgctg gagggcacgg tcaccacgct gctgagggag tagagtcctg 240
aggactgtag gacagacctc ggccgcgacc acgct 275

<210> 229
<211> 40
<212> DNA
<213> Homo sapiens

<220>
<221> misc_feature
<222> 1, 4, 5, 13, 15, 17, 29
<223> n = A,T,C or G

<400> 229
nggnnggtcc ggncngncag gaccactcnt cttcgaaata 40

<210> 230
<211> 208
<212> DNA
<213> Homo sapiens

<400> 230
agcgtggtcg cggccgaggt cctcacttgc ctcctgcaaa gcaccgatag ctgcgctctg 60
gaagcgcaga tctgttttaa agtcctgagc aatttctcgc accagacgct ggaagggaag 120
tttgcgaatc agaagttcag tggacttctg ataacgtcta atttcacgga gcgccacagt 180
accaggacct gcccgggcgg ccgctcga 208

<210> 231
<211> 208
<212> DNA
<213> Homo sapiens

<220>
<221> misc_feature
<222> 33
<223> n = A,T,C or G

<400> 231
tcgagcggcc gcccgggcag gtcctggtac tgnggcgctc cgtgaaatta gacgttatca 60
gaagtccact gaacttctga ttcgcaaact tcccttccag cgtctggtgc gagaaattgc 120
tcaggacttt aaaacagatc tgcgcttcca gagcgcagct atcggtgctt tgcaggaggc 180
aagtgaggac ctcggccgcg accacgct 208

<210> 232
<211> 332
<212> DNA
<213> Homo sapiens

<400> 232
tcgagcggcc gcccgggcag gtccacatcg gcagggtcgg agccctggcc gccatactcg 60
aactggaatc catcggtcat gctctcgccg aaccagacat gcctcttgtc cttggggttc 120
ttgctgatgt accagttctt ctgggccaca ctgggctgag tggggtacac gcaggtctca 180
ccagtctcca tgttgcagaa gactttgatg gcatccaggt tgcagccttg gttggggtca 240
atccagtact ctccactctt ccagtcagag tggcacatct tgaggtcacg gcaggtgcgg 300
gcggggttct tgacctcggc cgcgaccacg ct 332

<210> 233
<211> 415
<212> DNA
<213> Homo sapiens

<220>
<221> misc_feature
<222> 6, 15, 19, 21
<223> n = A,T,C or G

<400> 233
gtgggnttga acccntttna nctccgcttg gtaccgagct cggatccact agtaacggcc 60
gccagtgtgc tggaattcgg cttagcgtgg tcgcggccga ggtcaagaac cccgcccgca 120
cctgccgtga cctcaagatg tgccactctg actggaagag tggagagtac tggattgacc 180
ccaaccaagg ctgcaacctg gatgccatca aagtcttctg caacatggag actggtgaga 240
cctgcgtgta ccccactcag cccagtgtgg cccagaagaa ctggtacatc agcaagaacc 300
ccaaggacaa gaggcatgtc tggttcggcg agagcatgac cgatggattc cagttcgagt 360
atggcggcca gggctccgac cctgccgatg tggacctgcc cgggcggccg ctcga 415

<210> 234
<211> 776

<212> DNA
<213> Homo sapiens

<220>
<221> misc_feature
<222> 505, 550, 574, 601, 604, 608, 612, 649, 656, 657, 680, 711,
750, 776
<223> n = A,T,C or G

<400> 234
agcgtggtcg cggccgaggt ctgggatgct cctgctgtca cagtgagata ttacaggatc 60
acttacggag aaacaggagg aaatagccct gtccaggagt tcactgtgcc tgggagcaag 120
tctacagcta ccatcagcgg ccttaaacct ggagttgatt ataccatcac tgtgtatgct 180
gtcactggcc gtggagacag ccccgcaagc agcaagccaa tttccattaa ttaccgaaca 240
gaaattgaca aaccatccca gatgcaagtg accgatgttc aggacaacag cattagtgtc 300
aagtggctgc cttcaagttc ccctgttact ggttacagag taaccaccac tcccaaaaat 360
ggaccaggac caacaaaaac taaaactgca ggtccagatc aaacagaaat gactattgaa 420
ggcttgcagc ccacagtgga gtatgtggtt aagtgtctat gctcagaatc caagcggaga 480
gaagtcagcc tctggttcag actgnaagta accaacattg atcgcctaaa ggactggcat 540
tcactgatgn ggatgccgat tccatcaaaa ttgnttggga aaacccacag gggcaagttt 600
ncangtcnag gnggacctac tcgagccctg aggatggaat ccttgactnt tccttnncct 660
gatggggaaa aaaaaccttn aaaacttgaa ggacctgccc gggcggccgt ncaaaaccca 720
attccacccc cttgggggcg ttctatgggn cccactcgga ccaaacttgg ggtaan 776

<210> 235
<211> 805
<212> DNA
<213> Homo sapiens

<220>
<221> misc_feature
<222> 637, 684, 705, 724, 733, 756, 778, 793, 796, 804
<223> n = A,T,C or G

<400> 235
tcgagcggcc gcccgggcag gtccttgcag ctctgcagtg tcttcttcac catcaggtgc 60
agggaatagc tcatggattc catcctcagg gctcgagtag gtcaccctgt acctggaaac 120
ttgcccctgt gggctttccc aagcaatttt gatggaatcg gcatccacat cagtgaatgc 180
cagtccttta gggcgatcaa tgttggttac tgcagtctga accagaggct gactctctcc 240
gcttggattc tgagcataga cactaaccac atactccact gtgggctgca agccttcaat 300
agtcatttct gtttgatctg gacctgcagt tttagttttt gttggtcctg gtccattttt 360
gggagtggtg gttactctgt aaccagtaac aggggaactt gaaggcagcc acttgacact 420
aatgctgttg tcctgaacat cggtcacttg catctgggat ggtttgtcaa tttctgttcg 480
gtaattaatg gaaattggct tgctgcttgc ggggcttgtc tccacggcca gtgacagcat 540
acacagtgat ggtataatca actccaggtt taagccgctg atggtagctg aaactttgct 600
ccaggcacaa gtgaactcct gacagggcta tttcctnctg ttctccgtaa gtgatcctgt 660
aatatctcac tgggacagca ggangcattc caaaacttcg ggcgngaccc cctaagccga 720
attntgcaat atncatcaca ctggcgggcg ctcgancatt cattaaaagg cccaatcncc 780
cctataggga gtntantaca attng 805

<210> 236
<211> 262
<212> DNA
<213> Homo sapiens

<400> 236
tcgagcggcc gcccgggcag gtcacttttg gtttttggtc atgttcggtt ggtcaaagat 60

aaaaactaag tttgagagat gaatgcaaag gaaaaaaata ttttccaaag tccatgtgaa 120
attgtctccc atttttttgg cttttgaggg ggttcagttt gggttgcttg tctgtttccg 180
ggttgggggg aaagttggtt gggtgggagg gagccaggtt gggatggagg gagtttacag 240
gaagcagaca gggccaacgt cg 262

<210> 237
<211> 372
<212> DNA
<213> Homo sapiens

<400> 237
agcgtggtcg cggccgaggt cctcaccaga ggtgccacct acaacatcat agtggaggca 60
ctgaaagacc agcagaggca taaggttcgg gaagaggttg ttaccgtggg caactctgtc 120
aacgaaggct tgaaccaacc tacggatgac tcgtgctttg acccctacac agtttcccat 180
tatgccgttg gagatgagtg ggaacgaatg tctgaatcag gctttaaact gttgtgccag 240
tgcttaggct ttggaagtgg tcatttcaga tgtgattcat ctagatggtg ccatgacaat 300
ggtgtgaact acaagattgg agagaagtgg gaccgtcagg gagaaaatgg acctgcccgg 360
gcggccgctc ga 372

<210> 238
<211> 372
<212> DNA
<213> Homo sapiens

<400> 238
tcgagcggcc gcccgggcag gtccattttc tccctgacgg tcccacttct ctccaatctt 60
gtagttcaca ccattgtcat ggcaccatct agatgaatca catctgaaat gaccacttcc 120
aaagcctaag cactggcaca acagtttaaa gcctgattca gacattcgtt cccactcatc 180
tccaacggca taatgggaaa ctgtgtaggg gtcaaagcac gagtcatccg taggttggtt 240
caagccttcg ttgacagagt tgcccacggt aacaacctct tcccgaacct tatgcctctg 300
ctggtctttc agtgcctcca ctatgatgtt gtaggtggca cctctggtga ggacctcggc 360
cgcgaccacg ct 372

<210> 239
<211> 720
<212> DNA
<213> Homo sapiens

<220>
<221> misc_feature
<222> 478, 557, 563, 566, 620, 660, 663, 672, 673, 684, 693, 695
<223> n = A,T,C or G

<400> 239
tcgagcggcc gcccgggcag gtccaccata agtcctgata caaccacgga tgagctgtca 60
ggagcaaggt tgatttcttt cattggtccg gtcttctcct tgggggtcac ccgcactcga 120
tatccagtga gctgaacatt gggtggtgtc cactgggcgc tcaggcttgt gggtgtgacc 180
tgagtgaact tcaggtcagt tggtgcagga atagtggtta ctgcagtctg aaccagaggc 240
tgactctctc cgcttggatt ctgagcatag acactaacca catactccac tgtgggctgc 300
aagccttcaa tagtcatttc tgtttgatct ggacctgcag ttttagtttt tgttggtcct 360
ggtccatttt tgggagtggt ggttactctg taaccagtaa caggggaact tgaaggcagc 420
cacttgacac taatgctgtt gtcctgaaca tcggtcactt gcatctggga tggtttgnca 480
atttctgttc ggtaattaat ggaaattggc ttgctgcttg cggggctgtc tccacggcca 540
gtgacagcat acacagngat ggnatnatca actccaagtt taaggccctg atggtaactt 600
taaacttgct cccagccagn gaacttccgg acagggtatt tcttctggtt ttccgaaagn 660
gancctggaa tnntctcctt ggancagaag gancntccaa aacttgggcc ggaacccctt 720

<210> 240
<211> 691
<212> DNA
<213> Homo sapiens

<220>
<221> misc_feature
<222> 564, 582, 640, 651, 666, 669, 690
<223> n = A,T,C or G

<400> 240
agcgtggtcg cggccgaggt cctgtcagag tggcactggt agaagttcca ggaaccctga 60
actgtaaggg ttcttcatca gtgccaacag gatgacatga aatgatgtac tcagaagtgt 120
cctggaatgg ggcccatgag atggttgtct gagagagagc ttcttgtcct acattcggcg 180
ggtatggtct tggcctatgc cttatggggg tggccgttgt gggcggtgtg gtccgcctaa 240
aaccatgttc ctcaaagatc atttgttgcc caacactggg ttgctgacca gaagtgccag 300
gaagctgaat accatttcca gtgtcatacc cagggtgggt gacgaaaggg gtcttttgaa 360
ctgtggaagg aacatccaag atctctggtc catgaagatt ggggtgtgga agggttacca 420
gttggggaag ctcgtctgtc tttttccttc caatcagggg ctcgctcttc tgattattct 480
tcagggcaat gacataaatt gtatattcgg ttcccggttc caggccagta atagtagcct 540
cttgtgacac caggcggggc ccanggacca cttctctggg angagaccca gcttctcata 600
cttgatgatg taacccggta atcctgcacg tggcggctgn catgatacca ncaaggaatt 660
gggtgnggng gacctgcccg gcggccctcn a 691

<210> 241
<211> 808
<212> DNA
<213> Homo sapiens

<220>
<221> misc_feature
<222> 680, 715, 721, 728, 735, 749, 757, 762, 772, 776, 779, 781,
792, 796, 800, 808
<223> n = A,T,C or G

<400> 241
agcgtggtcg cggccgaggt ctgggatgct cctgctgtca cagtgagata ttacaggatc 60
acttacggag aaacaggagg aaatagccct gtccaggagt tcactgtgcc tgggagcaag 120
tctacagcta ccatcagcgg ccttaaacct ggagttgatt ataccatcac tgtgtatgct 180
gtcactggcc gtggagacag ccccgcaagc agcaagccaa tttccattaa ttaccgaaca 240
gaaattgaca aaccatccca gatgcaagtg accgatgttc aggacaacag cattagtgtc 300
aagtggctgc cttcaagttc ccctgttact ggttacagag taaccaccac tcccaaaaat 360
ggaccaggac caacaaaaac taaaactgca ggtccagatc aaacagaaat gactattgaa 420
ggcttgcagc ccacagtgga gtatgtggtt agtgtctatg ctcagaatcc aagcggagag 480
agtcagcctc tggttcagac tgcagtaacc actattcctg caccaactga cctgaagttc 540
actcaggtca cacccacaag cctgagccgc cagtggacac cacccaatgt tcactcactg 600
gatatcgagt gcgggtgacc cccaaggaga agacccggac ccatgaaaga aatcaacctt 660
gctcctgaca gctcatccgn gggtgtatca ggacttatgg gggactgccc cggcnggccg 720
ntcgaaancg aattntgaaa tttccttcnc actgggnggc gnttcgagct tncttntana 780
nggcccaatt cncctntagn gggtcgtn 808

<210> 242
<211> 26
<212> DNA
<213> Homo sapiens

<220>
<221> misc_feature
<222> 22
<223> n = A,T,C or G

<400> 242
agcgtggtcg cggccgaggt cnagga                                            26

<210> 243
<211> 697
<212> DNA
<213> Homo sapiens

<220>
<221> misc_feature
<222> 496, 541, 624, 662, 679, 688
<223> n = A,T,C or G

<400> 243
   tcgagcggcc gcccgggcag gtccaccaca cccaattcct tgctggtatc atggcagccg 60
   ccacgtgcca ggattaccgg ctacatcatc aagtatgaga agcctgggtc tcctcccaga 120
   gaagtggtcc ctcggccccg ccctggtgtc acagaggcta ctattactgg cctggaaccg 180
   ggaaccgaat atacaattta tgtcattgcc ctgaagaata atcagaagag cgagcccctg 240
   attggaagga aaaagacaga cgagcttccc caactggtaa cccttccaca ccccaatctt 300
   catggaccag agatcttgga tgttccttcc acagttcaaa agaccccttt cgtcacccac 360
   cctgggtatg acactggaaa tggtattcag cttcctggca cttctggtca gcaacccagt 420
   gttgggcaac aaatgatctt tgaggaacat ggttttaggc ggaccacacc gcccacaacg 480
   ggcaccccca taaggnatag gccaagacca taccccgccg aatgtaggac aagaagctct 540
   ntctcaacaa ccatctcatg ggccccattc caggacactt ctgagtacat catttcatgt 600
   catcctggtg ggcacttgat gaanaaccct tacagttcag ggttcctgga acttctacca 660
   gngccacttc tgacagganc ttgggcgnga ccaccct                               697

<210> 244
<211> 373
<212> DNA
<213> Homo sapiens

<400> 244
agcgtggtcg cggccgaggt ccattttctc cctgacggtc ccacttctct ccaatcttgt 60
agttcacacc attgtcatgg caccatctag atgaatcaca tctgaaatga ccacttccaa 120
agcctaagca ctggcacaac agtttaaagc ctgattcaga cattcgttcc cactcatctc 180
caacggcata atgggaaact gtgtaggggt caaagcacga gtcatccgta ggttggttca 240
agccttcgtt gacagagttg cccacggtaa caacctcttc ccgaacctta tgcctctgct 300
ggtctttcag tgcctccact atgatgttgt aggtggcacc tctggtgagg acctgcccgg 360
gcggcccgct cga                                                          373

<210> 245
<211> 307
<212> DNA
<213> Homo sapiens

<400> 245
agcgtggtcg cggccgaggt gtgccccaga ccaggaattc ggcttcgacg ttggccctgt 60
ctgcttcctg taaactccct ccatcccaac ctggctccct cccacccaac caactttccc 120
cccaacccgg aaacagacaa gcaacccaaa ctgaaccccc tcaaaagcca aaaaaatggg 180
agacaatttc acatggactt tggaaaatat ttttttcctt tgcattcatc tctcaaactt 240
agtttttatc tttgaccaac cgaacatgac caaaaaccaa aagtgacctg cccgggcggc 300

cgctcga                                                              307

<210> 246
<211> 372
<212> DNA
<213> Homo sapiens

<400> 246
tcgagcggcc gcccgggcag gtcctcacca gaggtgccac ctacaacatc atagtggagg 60
cactgaaaga ccagcagagg cataaggttc gggaagaggt tgttaccgtg ggcaactctg 120
tcaacgaagg cttgaaccaa cctacggatg actcgtgctt tgacccctac acagtttccc 180
attatgccgt tggagatgag tgggaacgaa tgtctgaatc aggctttaaa ctgttgtgcc 240
agtgcttagg ctttggaagt ggtcatttca gatgtgattc atctagatgg tgccatgaca 300
atggtgtgaa ctacaagatt ggagagaagt gggaccgtca gggagaaaat ggacctcggc 360
cgcgaccacg ct                                                        372

<210> 247
<211> 348
<212> DNA
<213> Homo sapiens

<220>
<221> misc_feature
<222> 284, 297, 299, 322, 325, 338, 342, 345
<223> n = A,T,C or G

<400> 247
tcgagcggcc gcccgggcag gtaccggggt ggtcagcgag gagccattca cactgaactt 60
caccatcaac aacctgcggt atgaggagaa catgcagcac cctggctcca ggaagttcaa 120
caccacggag agggtccttc agggcctgct caggtccctg ttcaagagca ccagtgttgg 180
ccctctgtac tctggctgca gactgacttt gctcagacct gagaaacatg gggcagccac 240
tggagtggac gccatctgca ccctccgcct tgatcccact ggtnctggac tggacanana 300
gcggctatac ttgggagctg anccnaacct ttggcggnga cnccnctt                 348

<210> 248
<211> 304
<212> DNA
<213> Homo sapiens

<220>
<221> misc_feature
<222> 125
<223> n = A,T,C or G

<400> 248
gaggactggc tcagctccca gtatagccgc tctctgtcca gtccaggacc agtgggatca 60
aggcggaggg tgcagatggc gtccactcca gtggctgccc catgtttctc aagtctgagc 120
aaagncagtc tgcagccaga gtacagaggg ccaacactgg tgctcttgaa cagggacctg 180
agcaggccct gaaggaccct ctccgtggtg ttgaacttcc tggagccagg gtgctgcatg 240
ttctcctcat accgcaggtt gttgatggtg aagttcagtg tgaatggctc ctcgctgacc 300
accc                                                                 304

<210> 249
<211> 400
<212> DNA
<213> Homo sapiens

<220>
<221> misc_feature
<222> 308, 310, 312, 320, 331, 336, 383, 392, 396
<223> n = A,T,C or G

<400> 249
   agcgtggtcg cggccgaggt ccaccacacc caattccttg ctggtatcat ggcagccgcc 60
   acgtgccagg attaccggct acatcatcaa gtatgagaag cctgggtctc ctcccagaga 120
   agtggtccct cggccccgcc ctggtgtcac agaggctact attactggcc tggaaccggg 180
   aaccgaatat acaatttatg tcattgccct gaagaataat cagaagagcg agcccctgat 240
   tggaaggaaa aagacagacg agcttcccca actggtaacc cttccacacc ccaatcttca 300
   tggaccanan ancttggatn gtcctttcac nggttnaaaa aacccttttc gcccccccac 360
   cttggggatt aaccttggga aanggggatt tnaccnttcc 400

<210> 250
<211> 400
<212> DNA
<213> Homo sapiens

<220>
<221> misc_feature
<222> 338, 357, 361, 369, 388, 394
<223> n = A,T,C or G

<400> 250
tcgagcggcc gcccgggcag gtcctgtcag agtggcactg gtagaagttc caggaaccct 60
gaactgtaag ggttcttcat cagtgccaac aggatgacat gaaatgatgt actcagaagt 120
gtcctggaat ggggcccatg agatggttgt ctgagagaga gcttcttgtc ctacattcgg 180
cgggtatggt cttggcctat gccttatggg ggtggccgtt gtgggcggtg tggtccgcct 240
aaaaccatgt tcctcaaaga tcatttgttg cccaacactg ggttgctgac cagaagtgcc 300
aggaagctga ataccatttc cagtgtcata cccagggngg gtgaccaaag ggggtcnttt 360
ngacctggng aaaggaacca tccaaaanct ctgncccatg 400

<210> 251
<211> 514
<212> DNA
<213> Homo sapiens

<220>
<221> misc_feature
<222> 8, 107, 312, 338, 351, 352, 357, 363, 366, 373, 380, 405,
421, 444, 508
<223> n = A,T,C or G

<400> 251
agcgtggncg cggccgaggt ctgaggatgt aaactcttcc caggggaagg ctgaagtgct 60
gaccatggtg ctactgggtc cttctgagtc agatatgtga ctgatgngaa ctgaagtagg 120
tactgtagat ggtgaagtct gggtgtccct aaatgctgca tctccagagc cttccatcat 180
taccgtttct tcttttgcta tgggatgaga cactgttgag tattctctaa agtcaccact 240
gaaatcttcc tccaaaggaa aacctgtgga aaagcccctt atttctgccc cataatttgg 300
ttctcctaat cnctctgaaa tcactatttc cctggaangt ttgggaaaaa nngggcnacc 360
tgncantgga aantggatan aaagatccca ccattttacc caacnagcag aaagtgggaa 420
nggtaccgaa aagctccaag taanaaaaag gagggaagta aaggtcaagt gggcaccagt 480
ttcaaacaaa actttcccca aactatanaa ccca 514

<210> 252
<211> 501

<212> DNA
<213> Homo sapiens

<220>
<221> misc_feature
<222> 20, 21, 25, 44, 343, 347, 356, 362, 387, 391, 398, 409, 428,
430, 453, 494
<223> n = A,T,C or G

<400> 252
aagcggccgc ccgggcaggn ncagnagtgc cttcgggact gggntcaccc ccaggtctgc 60
ggcagttgtc acagcgccag ccccgctggc ctccaaagca tgtgcaggag caaatggcac 120
cgagatattc cttctgccac tgttctccta cgtggtatgt cttcccatca tcgtaacacg 180
ttgcctcatg agggtcacac ttgaattctc cttttccgtt cccaagacat gtgcagctca 240
tttggctggc tctatagttt ggggaaagtt tgttgaaact gtgccactga cctttacttc 300
ctccttctct actggagctt tccgtacctt ccacttctgc tgntggnaaa aagggnggaa 360
cntcttatca atttcattgg acagtanccc nctttctncc caaaacatnc aagggaaaat 420
attgattncn agagcggatt aaggaacaac ccnaattatg ggggccagaa ataaaggggg 480
cttttccaca ggtnttttcc t 501

<210> 253
<211> 226
<212> DNA
<213> Homo sapiens

<400> 253
tcgagcggcc gcccgggcag gtctgcaggc tattgtaagt gttctgagca catatgagat 60
aacctgggcc aagctatgat gttcgatacg ttaggtgtat taaatgcact tttgactgcc 120
atctcagtgg atgacagcct tctcactgac agcagagatc ttcctcactg tgccagtggg 180
caggagaaag agcatgctgc gactggacct cggccgcgac cacgct 226

<210> 254
<211> 226
<212> DNA
<213> Homo sapiens

<400> 254
agcgtggtcg cggccgaggt ccagtcgcag catgctcttt ctcctgccca ctggcacagt 60
gaggaagatc tctgctgtca gtgagaaggc tgtcatccac tgagatggca gtcaaaagtg 120
catttaatac acctaacgta tcgaacatca tagcttggcc caggttatct catatgtgct 180
cagaacactt acaatagcct gcagacctgc ccgggcggcc gctcga 226

<210> 255
<211> 427
<212> DNA
<213> Homo sapiens

<220>
<221> misc_feature
<222> 327, 403
<223> n = A,T,C or G

<400> 255
cgagcggccg cccgggcagg tccagactcc aatccagaga accaccaagc cagatgtcag 60
aagctacacc atcacaggtt tacaaccagg cactgactac aagatctacc tgtacacctt 120
gaatgacaat gctcggagct cccctgtggt catcgacgcc tccactgcca ttgatgcacc 180
atccaacctg cgtttcctgg ccaccacacc caattccttg ctggtatcat ggcagccgcc 240

acgtgccagg attaccggct acatcatcaa gtatgagaag cctgggtctc ctcccagaga 300
agtggtccct cggccccgcc ctggtgncac agaagctact attactggcc tggaaccggg 360
aaccgaatat acaatttatg tcattgccct gaagaataat canaagagcg agcccctgat 420
tggaagg 427

<210> 256
<211> 535
<212> DNA
<213> Homo sapiens

<220>
<221> misc_feature
<222> 347, 456, 475
<223> n = A,T,C or G

<400> 256
agcgtggtcg cggccgaggt cctgtcagag tggcactggt agaagttcca ggaaccctga 60
actgtaaggg ttcttcatca gtgccaacag gatgacatga aatgatgtac tcagaagtgt 120
cctggaatgg ggcccatgag atggttgtct gagagagagc ttcttgtcct gtctttttcc 180
ttccaatcag gggctcgctc ttctgattat tcttcagggc aatgacataa attgtatatt 240
cggttcccgg ttccaggcca gtaatagtag cctctgtgac accagggcgg ggccgaggga 300
ccacttctct gggaggagac ccaggcttct catacttgat gatgtanccg gtaatcctgg 360
caccgtggcg gctgccatga taccagcaag gaattgggtg tggtggccaa gaaacgcagg 420
ttggatggtg catcaatggc agtggaggcg tcgatnacca caggggagct ccgancattg 480
tcattcaagg tggacaggta gaatcttgta atcaggtgcc tggtttgtaa acctg 535

<210> 257
<211> 544
<212> DNA
<213> Homo sapiens

<220>
<221> misc_feature
<222> 495, 511
<223> n = A,T,C or G

<400> 257
tcgagcggcc gcccgggcag gtttcgtgac cgtgacctcg aggtggacac caccctcaag 60
agcctgagcc agcagatcga gaacatccgg agcccagagg gcagccgcaa gaaccccgcc 120
cgcacctgcc gtgacctcaa gatgtgccac tctgactgga agagtggaga gtactggatt 180
gaccccaacc aaggctgcaa cctggatgcc atcaaagtct tctgcaacat ggagactggt 240
gagacctgcg tgtaccccac tcagcccagt gtggcccaga agaactggta catcagcaag 300
aaccccaagg acaagaagca tgtctggttc ggcgaaagca tgaccgatgg attccagttc 360
gagtatggcg gccagggctc cgaccctgcc gatgtggacc tcggccgcga ccacgctaag 420
cccgaattcc agcacactgg cggccgttac tagtgggatc cgagcttcgg taccaagctt 480
ggcgtaatca tgggncatag ctgtttcctg ngtgaaaatg gtattccgct tcacaatttc 540
ccac 544

<210> 258
<211> 418
<212> DNA
<213> Homo sapiens

<400> 258
agcgtggtcg cggccgaggt ccacatcggc agggtcggag ccctggccgc catactcgaa 60
ctggaatcca tcggtcatgc tctcgccgaa ccagacatgc ctcttgtcct tggggttctt 120
gctgatgtac cagttcttct gggccacact gggctgagtg gggtacacgc aggtctcacc 180

agtctccatg ttgcagaaga ctttgatggc atccaggttg cagccttggt tggggtcaat 240
ccagtactct ccactcttcc agtcagagtg gcacatcttg aggtcacggc aggtgcgggc 300
ggggttcttg cggctgccct ctgggctccg gatgttctcg atctgctggc tcaagctctt 360
gaagggtggt gtccacctcg aggtcacggt cacgaaacct gcccgggcgg ccgctcga 418

<210> 259
<211> 377
<212> DNA
<213> Homo sapiens

<220>
<221> misc_feature
<222> 320, 326, 342, 352
<223> n = A,T,C or G

<400> 259
agcgtggtcg cggccgaggt caagaacccc gcccgcacct gccgtgacct caagatgtgc 60
cactctgact ggaagagtgg agagtactgg attgacccca accaaggctg caacctggat 120
gccatcaaag tcttctgcaa catggagact ggtgagacct gcgtgtaccc cactcagccc 180
agtgtggccc agaagaactg gtacatcagc aagaacccca aggacaagag gcatgtctgg 240
ttcggcgaga gcatgaccga tggattccag ttcgagtatg gcggccaggg ctccgaccct 300
gccgatgtgg acctgcccgn gccggnccgc tcgaaaagcc cnaatttcca gncacacttg 360
gccggccgtt actactg 377

<210> 260
<211> 332
<212> DNA
<213> Homo sapiens

<400> 260
tcgagcggcc gcccgggcag gtccacatcg gcagggtcgg agccctggcc gccatactcg 60
aactggaatc catcggtcat gctctcgccg aaccagacat gcctcttgtc cttggggttc 120
ttgctgatgt accagttctt ctgggccaca ctgggctgag tggggtacac gcaggtctca 180
ccagtctcca tgttgcagaa gactttgatg gcatccaggt tgcagccttg gttggggtca 240
atccagtact ctccactctt ccagtcagag tggcacatct tgaggtcacg gcaggtgcgg 300
gcggggttct tgacctcggc cgcgaccacg ct 332

<210> 261
<211> 94
<212> DNA
<213> Homo sapiens

<400> 261
cgagcggccg cccgggcagg tcccccccct tttttttttt tttttttttt tttttttttt 60
tttttttttt tttttttttt tttttttttt tttt 94

<210> 262
<211> 650
<212> DNA
<213> Homo sapiens

<220>
<221> misc_feature
<222> 412, 582, 612, 641, 646
<223> n = A,T,C or G

<400> 262

agcgtggtcg cggccgaggt ctggcattcc ttcgacttct ctccagccga gcttcccaga 60
acatcacata tcactgcaaa aatagcattg catacatgga tcaggccagt ggaaatgtaa 120
agaaggccct gaagctgatg gggtcaaatg aaggtgaatt caaggctgaa ggaaatagca 180
aattcaccta cacagttctg gaggatggtt gcacgaaaca cactggggaa tggagcaaaa 240
cagtctttga atatcgaaca cgcaaggctg tgagactacc tattgtagat attgcaccct 300
atgacattgg tggtcctgat caagaatttg gtgtggacgt tggccctgtt tgctttttat 360
aaaccaaact ctatctgaaa tcccaacaaa aaaaatttaa ctccatatgt gntcctcttg 420
ttctaatctt ggcaaccagt gcaagtgacc gacaaaattc cagttattta tttccaaaat 480
gtttggaaac agtataattt gacaaagaaa aaaggatact tctctttttt tggctggtcc 540
accaaataca attcaaaagg ctttttggtt ttattttttt anccaattcc aatttcaaaa 600
tgtctcaatg gngcttataa taaaataaac tttcaccctt nttttntgat 650

<210> 263
<211> 573
<212> DNA
<213> Homo sapiens

<220>
<221> misc_feature
<222> 453, 458, 544
<223> n = A,T,C or G

<400> 263
agcgtggtcg cggccgaggt ctgggatgct cctgctgtca cagtgagata ttacaggatc 60
acttacggag aaacaggagg aaatagccct gtccaggagt tcactgtgcc tgggagcaag 120
tctacagcta ccatcagcgg ccttaaacct ggagttgatt ataccatcac tgtgtatgct 180
gtcactggcc gtggagacag ccccgcaagc agcaagccaa tttccattaa ttaccgaaca 240
gaaattgaca aaccatccca gatgcaagtg accgatgttc aggacaacag cattagtgtc 300
aagtggctgc cttcaagttc ccctgttact ggttacagaa gtaaccacca ctcccaaaaa 360
tggaccagga ccaacaaaaa ctaaaactgc aggtccagat caaacagaaa atggactatt 420
gaaggcttgc agcccacagt ggaagtatgt ggntaggngt ctatgctcag aatcccaagc 480
cggagaaagt cagccttctg gtttagactg cagtaaccaa cattgatcgc cctaaaggac 540
tggncattca cttggatggt ggatgtccaa ttc 573

<210> 264
<211> 550
<212> DNA
<213> Homo sapiens

<220>
<221> misc_feature
<222> 39, 174, 352, 526
<223> n = A,T,C or G

<400> 264
tcgagcggcc gcccgggcag gtccttgcag ctctgcagng tcttcttcac catcaggtgc 60
agggaatagc tcatggattc catcctcagg gctcgagtag gtcaccctgt acctggaaac 120
ttgcccctgt gggctttccc aagcaatttt gatggaatcg acatccacat cagngaatgc 180
cagtccttta gggcgatcaa tgttggttac tgcagtctga accagaggct gactctctcc 240
gcttggattc tgagcataga cactaaccac atactccact gtgggctgca agccttcaat 300
agtcatttct gtttgatctg gacctgcagt tttaagtttt tggtggtcct gncccatttt 360
tgggaagtgg ggggttactc tgtaaccagt aacaggggaa cttgaaggca gccacttgac 420
actaatgctg ttgtcctgaa catcggtcac ttgcatctgg ggatggtttt gacaatttct 480
ggttcggcaa attaatggaa attggcttgc tgcttggcgg ggctgnctcc acgggccagt 540
gacagcatac 550

<210> 265

<211> 596
<212> DNA
<213> Homo sapiens

<220>
<221> misc_feature
<222> 347, 352, 353, 534, 555, 587
<223> n = A,T,C or G

<400> 265
tcgagcggcc gcccgggcag gtccttgcag ctctgcagtg tcttcttcac catcaggtgc 60
agggaatagc tcatggattc catcctcagg gctcgagtag gtcaccctgt acctggaaac 120
ttgcccctgt gggctttccc aagcaatttt gatggaatcg acatccacat cagtgaatgc 180
cagtccttta gggcgatcaa tgttggttac tgcagtctga accagaggct gactctctcc 240
gcttggattc tgagcataga cactaaccac atactccact gtgggctgca agccttcaat 300
agtcatttct gtttgatctg gacctgcagt tttaagtttt tgttggncct gnnccatttt 360
tggggaaggg gtggttactc ttgtaaccag taacagggga acttgaagca gccacttgac 420
actaatgctg gtggcctgaa catcggtcac ttgcatctgg gatggtttgg tcaatttctg 480
ttcggtaatt aatgggaaat tggcttactg gcttgcgggg gctgtctcca cggncagtga 540
caagcataca caggngatgg gtataatcaa ctccaggttt aaggccnctg atggta 596

<210> 266
<211> 506
<212> DNA
<213> Homo sapiens

<220>
<221> misc_feature
<222> 393, 473
<223> n = A,T,C or G

<400> 266
agcgtggtcg cggccgaggt ctgggatgct cctgctgtca cagtgagata ttacaggatc 60
acttacggag aaacaggagg aaatagccct gtccaggagt tcactgtgcc tgggagcaag 120
tctacagcta ccatcagcgg ccttaaacct ggagttgatt ataccatcac tgtgtatgct 180
gtcactggcc gtggagacag ccccgcaagc agtaagccaa tttccattaa ttaccgaaca 240
gaaattgaca aaccatccca gatgcaagtg accgatgttc aggacaacag cattagtgtc 300
aagtggctgc cttcaagttc ccctgttact ggttacagag taaccaccac tcccaaaaat 360
gggaccagga ccaacaaaaa actaaaactg canggtccag atcaaacaga aatgactatt 420
gaaggcttgc agcccacagt ggagtatgtg ggttagtgtc tatgctcaga atnccaagcg 480
gagagagtca gcctctggtt cagact 506

<210> 267
<211> 548
<212> DNA
<213> Homo sapiens

<220>
<221> misc_feature
<222> 346, 358, 432, 510, 512
<223> n = A,T,C or G

<400> 267
tcgagcggcc gcccgggcag gtcagcgctc tcaggacgtc accaccatgg cctgggctct 60
gctcctcctc accctcctca ctcagggcac agggtcctgg gcccagtctg ccctgactca 120
gcctccctcc gcgtccgggt ctcctggaca gtcagtcacc atctcctgca ctggaaccag 180
cagtgacgtt ggtgcttatg aatttgtctc ctggtaccaa caacacccag gcaaggcccc 240

caaactcatg atttctgagg tcactaagcg gccctcaggg gtccctgatc gcttctctgg 300
ctccaagtct ggcaacacgg cctccctgac cgtctctggg ctccangctg aggatgangc 360
tgattattac tggaagctca tatgcaggca acaacaattg ggtgttcggc ggaagggacc 420
aagctgaccg tnctaaggtc aagcccaagg cttgcccccc tcggtcactc tgttcccacc 480
ctcctctgaa gaagctttca agccaacaan gncacactgg gtgtgtctca taagtggact 540
ttctaccc 548

<210> 268
<211> 584
<212> DNA
<213> Homo sapiens

<220>
<221> misc_feature
<222> 98, 380, 421, 454, 495, 506, 512, 561, 565, 579
<223> n = A,T,C or G

<400> 268
agcgtggtcg cggccgaggt ctgtagcttc tgtgggactt ccactgctca ggcgtcaggc 60
tcaggtagct gctggccgcg tacttgttgt tgctttgntt ggagggtgtg gtggtctcca 120
ctcccgcctt gacggggctg ctatctgcct tccaggccac tgtcacggct cccgggtaga 180
agtcacttat gagacacacc agtgtggcct tgttggcttg aagctcctca gaggagggtg 240
ggaacagagt gaccgagggg gcagccttgg gctgacctag gacggtcagc ttggtccctc 300
cgccgaacac ccaattgttg ttgcctgcat atgagctgca gtaataatca gcctcatcct 360
cagcctggag cccagagacn gtcaagggag gcccgtgttt gccaagactt ggaagccaga 420
naagcgatca gggacccctg agggccgctt tacngacctc aaaaaatcat gaatttgggg 480
ggcctttgcc tgggngttgg ttggtnacca gnaaaacaaa atttcataaa gcaccaacgt 540
cactgctggt ttccagtgca ngaanatggt gaactgaant gtcc 584

<210> 269
<211> 368
<212> DNA
<213> Homo sapiens

<220>
<221> misc_feature
<222> 265, 329
<223> n = A,T,C or G

<400> 269
agcgtggtcg cggccgaggt ccagcatcag gagccccgcc ttgccggctc tggtcatcgc 60
ctttcttttt gtggcctgaa acgatgtcat caattcgcag tagcagaact gccgtctcca 120
ctgctgtctt ataagtctgc agcttcacag ccaatggctc ccatatgccc agttccttca 180
tgtccaccaa agtacccgtc tcaccattta caccccaggt ctcacagttc tcctgggtgt 240
gcttggcccg aagggaggta agtanacgga tggtgctggt cccacagttc tggatcaggg 300
tacgaggaat gacctctagg gcctgggcna caagccctgt atggacctgc ccgggcgggc 360
ccgctcga 368

<210> 270
<211> 368
<212> DNA
<213> Homo sapiens

<220>
<221> misc_feature
<222> 54, 163, 219, 229, 316
<223> n = A,T,C or G

<400> 270
tcgagcggcc gcccgggcag gtccatacag ggctgttgcc caggccctag aggncattcc 60
ttgtaccctg atccagaact gtgggaccag caccatccgt ctacttacct cccttcgggc 120
caagcacacc caggagaact gtgagacctg gggtgtaaat ggngagacgg gtactttggt 180
ggacatgaag gaactgggca tatgggagcc attggctgng aagctgcana cttataagac 240
agcagtggag acggcagttc tgctactgcg aattgatgac atcgtttcag gccacaaaaa 300
gaaaggcgat gaccanagcc ggcaaggcgg ggcttcctga tgctggacct cggccgccga 360
ccacgctt 368

<210> 271
<211> 424
<212> DNA
<213> Homo sapiens

<220>
<221> misc_feature
<222> 279, 329, 362, 384, 400
<223> n = A,T,C or G

<400> 271
agcgtggtcg cggccgaggt ccactagagg tctgtgtgcc attgcccagg cagagtctct 60
gcgttacaaa ctcctaggag ggcttgctgt gcggagggcc tgctatggtg tgctgcggtt 120
catcatggag agtggggcca aaggctgcga ggttgtggtg tctgggaaac tccgaggaca 180
gagggctaaa tccatgaagt ttgtggatgg cctgatgatc cacagcggag accctgttaa 240
ctactacgtt gacactgctg tgcgccacgt gttgctcana cagggtgtgc tgggcatcaa 300
ggtgaagatc atgctgccct gggacccanc tggcaaaaat ggcccttaaa aaccccttgc 360
cntgaccacg tgaaccattt gtgngaaccc caagatgaan atacttgccc accacccccc 420
attc 424

<210> 272
<211> 541
<212> DNA
<213> Homo sapiens

<220>
<221> misc_feature
<222> 422, 442, 510, 513, 515, 525
<223> n = A,T,C or G

<400> 272
tcgagcggcc gcccgggcag gtctgccaag gagaccctgt tatgctgtgg ggactggctg 60
gggcatggca ggcggctctg gcttcccacc cttctgttct gagatggggg tggtgggcag 120
tatctcatct ttgggttcca caatgctcac gtggtcaggc aggggcttct tagggccaat 180
cttaccagtt gggtcccagg gcagcatgat cttcaccttg atgcccagca caccctgtct 240
gagcaacacg tggcgcacag cagtgtcaac gtagtagtta acagggtctc cgctgtggat 300
catcaggcca tccacaaact tcatggattt agccctctgt cctcggagtt tcccaaaaca 360
ccacaacctc gccagccttt gggccccact tcttcatgaa tgaaaccgca gcacaccatt 420
ancaaggccc ttccgcacag gnaagccctt cctaaggagt tttgtaaacg caaaaaactc 480
ttgcctgggg caaatgggca cacagacctn tantnggacc ttggnccgcg aaccaccgct 540
t 541

<210> 273
<211> 579
<212> DNA
<213> Homo sapiens

<220>
<221> misc_feature
<222> 223, 265, 277, 308, 329, 346, 360, 366, 429, 448, 517, 524,
531, 578
<223> n = A,T,C or G

<400> 273
agcgtggtcg cggccgaggt ctggccctcc tggcaaggct ggtgaagatg gtcaccctgg 60
aaaacccgga cgacctggtg agagaggagt tgttggacca cagggtgctc gtggtttccc 120
tggaactcct ggacttcctg gcttcaaagg cattagggga cacaatggtc tggatggatt 180
gaagggacag cccggtgctc ctggtgtgaa gggtgaacct ggngcccctg gtgaaaatgg 240
aactccaggt caaacaggag cccgngggct tcctggngag agaggacgtg ttggtgcccc 300
tggcccanac ctgcccgggc ggccgctcna aaagccgaaa tccagnacac tggcggccgn 360
tactantgga atccgaactt cggtaccaaa gcttggccgt aatcatggcc atagcttgtt 420
ccctggggng gaaattggta ttccgctncc aattccacac aacataccga acccggaaag 480
cattaaagtg taaaagccct gggggggcct aaatgangtg agcntaactc ncatttaatt 540
ggcgttgcgc ttcactgccc cgcttttcca gtccgggna 579

<210> 274
<211> 330
<212> DNA
<213> Homo sapiens

<220>
<221> misc_feature
<222> 171
<223> n = A,T,C or G

<400> 274
tcgagcggcc gcccgggcag gtctgggcca ggggcaccaa cacgtcctct ctcaccagga 60
agcccacggg ctcctgtttg acctggagtt ccattttcac caggggcacc aggttcaccc 120
ttcacaccag gagcaccggg ctgtcccttc aatccatcca gaccattgtg ncccctaatg 180
cctttgaagc caggaagtcc aggagttcca gggaaaccac gagcaccctg tggtccaaca 240
actcctctct caccaggtcg tccgggtttt ccagggtgac catcttcacc agccttgcca 300
ggagggccag acctcggccg cgaccacgct 330

<210> 275
<211> 97
<212> DNA
<213> Homo sapiens

<220>
<221> misc_feature
<222> 2, 35, 72
<223> n = A,T,C or G

<400> 275
ancgtggtcg cggccgaggt cctcaccaga ggtgncacct acaacatcat agtggaggca 60
ctgaaagacc ancagaggca taaggttcgg gaagagg 97

<210> 276
<211> 610
<212> DNA
<213> Homo sapiens

<220>
<221> misc_feature

<222> 358, 360, 363, 382, 424, 433, 464, 468, 477, 491, 499, 511,
558, 584, 588, 590
<223> n = A,T,C or G

<400> 276
tcgagcggcc gcccgggcag gtccattttc tccctgacgg tcccacttct ctccaatctt 60
gtagttcaca ccattgtcat ggcaccatct agatgaatca catctgaaat gaccacttcc 120
aaagcctaag cactggcaca acagtttaaa gcctgattca gacattcgtt cccactcatc 180
tccaacggca taatgggaaa ctgtgtaggg gtcaaagcac gagtcatccg taggttggtt 240
caagccttcg ttgacagagt tgtccacggt aacaacctct tcccgaacct tatgcctctg 300
ctggtctttc agtgcctcca ctatgatgtt gtaggtggca cctctggtga ggacctcngn 360
ccngaacaac gcttaagccc gnattctgca gaataatccc atcacacttg gcggccgctt 420
cgancatgca tcntaaaagg ggccccaatt tcccccttat aagngaancc gtatttncca 480
atttcactgg ncccgccgnt tttacaaacg ncggtgaact ggggaaaaac cctggcggtt 540
acccaacttt aatcgccntt ggcagcacaa tccccccttt tcgnccancn tgggcgtaaa 600
taaccgaaaa 610

<210> 277
<211> 38
<212> DNA
<213> Homo sapiens

<220>
<221> misc_feature
<222> 2, 5, 18, 21, 31
<223> n = A,T,C or G

<400> 277
ancgnggtcg cggccgangt nttttttctt nttttttt 38

<210> 278
<211> 443
<212> DNA
<213> Homo sapiens

<220>
<221> misc_feature
<222> 156, 212, 233, 245, 327, 331, 336, 361, 364, 381, 391, 397,
419, 437
<223> n = A,T,C or G

<400> 278
agcgtggtcg cggccgaggt ctgaggttac atgcgtggtg gtggacgtga gccacgaaga 60
ccctgaggtc aagttcaact ggtacgtgga cggcgtggag gtgcataatg ccaagacaaa 120
gccgcgggag gagcagtaca acagcacgta ccgggnggtc agcgtcctca ccgtcctgca 180
ccagaattgg ttgaatggca aggagtacaa gngcaaggtt tccaacaaag ccntcccagc 240
ccccntcgaa aaaaccattt ccaaagccaa agggcagccc cgagaaccac aggtgtacac 300
cctgccccca tcccgggagg aaaagancaa naaccnggtt cagccttaac ttgcttggtc 360
naangctttt tatcccaacg nacttccccc ntggaantgg gaaaaaccaa tgggccaanc 420
cgaaaaacaa ttacaanaac ccc 443

<210> 279
<211> 348
<212> DNA
<213> Homo sapiens

<220>

<221> misc_feature
<222> 219, 256, 291, 297, 307, 314, 317
<223> n = A,T,C or G

<400> 279
tcgagcggcc gcccgggcag gtgtcggagt ccagcacggg aggcgtggtc ttgtagttgt 60
tctccggctg cccattgctc tcccactcca cggcgatgtc gctgggatag aagcctttga 120
ccaggcaggt caggctgacc tggttcttgg tcatctcctc ccgggatggg ggcagggtga 180
acacctgggg ttctcggggc ttgccctttg gttttgaana tggttttctc gatgggggct 240
ggaagggctt tgttgnaaac cttgcacttg actccttgcc attcacccag ncctggngca 300
ggacggngag gacnctnacc acacggaacc gggctggtgg actgctcc 348

<210> 280
<211> 149
<212> DNA
<213> Homo sapiens

<220>
<221> misc_feature
<222> 18, 34, 51, 118, 120, 140
<223> n = A,T,C or G

<400> 280
agcgtggtcg cggacgangt cctgtcagag tggnactggt agaagttcca ngaaccctga 60
actgtaaggg ttcttcatca gtgccaacag gatgacatga aatgatgtac tcagaagngn 120
cctggaatgg ggcccatgan atggttgcc 149

<210> 281
<211> 404
<212> DNA
<213> Homo sapiens

<220>
<221> misc_feature
<222> 383, 386, 388, 393
<223> n = A,T,C or G

<400> 281
tcgagcggcc gcccgggcag gtccaccaca cccaattcct tgctggtatc atggcagccg 60
ccacgtgcca ggattaccgg ctacatcatc aagtatgaga agcctgggtc tcctcccaga 120
gaagtggtcc ctcggccccg ccctggtgtc acagaggcta ctattactgg cctggaaccg 180
ggaaccgaat atacaattta tgtcattgcc ctgaagaata atcagaagag cgagcccctg 240
attggaagga aaaagacaga cgagcttccc caactggtaa cccttccaca ccccaatctt 300
catggaccag agatcttgga tgttccttcc acagttcaaa agaccccttt cggcaccccc 360
cctgggtatg aacctgggaa aanggnantt aanctttcct ggca 404

<210> 282
<211> 507
<212> DNA
<213> Homo sapiens

<220>
<221> misc_feature
<222> 320, 341, 424, 450, 459, 487, 498
<223> n = A,T,C or G

<400> 282

agcgtggtcg cggccgaggt ctgggatgct cctgctgtca cagtgagata ttacaggatc 60
acttacggag aaacaggagg aaatagccct gtccaggagt tcactgtgcc tgggagcaag 120
tctacagcta ccatcagcgg ccttaaacct ggagttgatt ataccatcac tgtgtatgct 180
gtcactggcc gtggagacag ccccgcaagc agcaagccaa tttccattaa ttaccgaaca 240
gaaattgaca aaccatccca gatgcaagtg accgatgttc aggacaacag cattagtgtc 300
aagtggctgc cttcaaggtn ccctggtact gggttacaga ntaaccacca ctcccaaaaa 360
tggaccagga accacaaaaa cttaaactgc agggtccaga tcaaaacaga aatgactatt 420
gaangcttgc agcccacagt gggagtatgn gggtagtgnc tatgcttcag aatccaagcg 480
gaaaaangtc aagccttntg ggttcaa 507

<210> 283
<211> 325
<212> DNA
<213> Homo sapiens

<220>
<221> misc_feature
<222> 216, 292, 303, 304
<223> n = A,T,C or G

<400> 283
tcgagcggcc gcccgggcag gtccttgcag ctctgcagtg tcttcttcac catcaggtgc 60
agggaatagc tcatggattc catcctcagg gctcgagtag gtcaccctgt acctggaaac 120
ttgcccctgt gggctttccc aagcaatttt gatggaatcg acatccacat cagtgaatgc 180
cagtccttta gggcgatcaa tgttggttac tgcagnctga accagaggct gactctctcc 240
gcttggattc tgagcataga cactaaccac atactccact gtgggctgca anccttcaat 300
aanncatttc tgtttgatct ggacc 325

<210> 284
<211> 331
<212> DNA
<213> Homo sapiens

<220>
<221> misc_feature
<222> 54, 59, 63, 121, 312, 327
<223> n = A,T,C or G

<400> 284
tcgagcggcc gcccgggcag gtctggtggg gtcctggcac acgcacatgg gggngttgnt 60
ctnatccagc tgcccagccc ccattggcga gtttgagaag gtgtgcagca atgacaacaa 120
naccttcgac tcttcctgcc acttctttgc cacaaagtgc accctggagg gcaccaagaa 180
gggccacaag ctccacctgg actacatcgg gccttgcaaa tacatccccc cttgcctgga 240
ctctgagctg accgaattcc cccttgcgca tgcgggactg gctcaagaac cgtcctggca 300
cccttgtatg anagggatga agacacnacc c 331

<210> 285
<211> 509
<212> DNA
<213> Homo sapiens

<220>
<221> misc_feature
<222> 316, 319, 327, 329, 339, 344, 357, 384, 398, 427, 443, 450,
478
<223> n = A,T,C or G

<400> 285
agcgtggtcg cggccgaggt ctgtcctaca gtcctcagga ctctactccc tcagcagcgt 60
ggtgaccgtg ccctccagca acttcggcac ccagacctac acctgcaacg tagatcacaa 120
gcccagcaac accaaggtgg acaagagagt tgagcccaaa tcttgtgaca aaactcacac 180
atgcccaccg tgcccagcac ctgaactcct ggggggaccg tcagtcttcc tcttcccccg 240
catccccctt ccaaacctgc ccgggcggcc gctcgaaagc cgaattccag cacactggcg 300
gccggtacta gtgganccna acttggnanc caacctggng gaantaatgg gcataanctg 360
tttctggggg gaaattggta tccngtttac aattcccnca caacatacga gccggaagca 420
taaaagngta aaagcctggg ggnggcctan tgaagtgaag ctaaactcac attaattngc 480
gttgccgctc actggcccgc ttttccagc 509

<210> 286
<211> 336
<212> DNA
<213> Homo sapiens

<220>
<221> misc_feature
<222> 188, 251, 267
<223> n = A,T,C or G

<400> 286
tcgagcggcc gcccgggcag gtttggaagg gggatgcggg ggaagaggaa gactgacggt 60
ccccccagga gttcaggtgc tgggcacggt gggcatgtgt gagttttgtc acaagatttg 120
ggctcaactc tcttgtccac cttggtgttg ctgggcttgt gatctacgtt gcaggtgtag 180
gtctgggngc cgaagttgct ggagggcacg gtcaccacgc tgctgaggga gtagagtcct 240
gaggactgta ngacagacct cggccgngac cacgctaagc cgaattctgc agatatccat 300
cacactggcg gccgctccga gcatgcattt tagagg 336

<210> 287
<211> 30
<212> DNA
<213> Homo sapiens

<220>
<221> misc_feature
<222> 8, 18
<223> n = A,T,C or G

<400> 287
agcgtggncg cggacganga caacaacccc 30

<210> 288
<211> 316
<212> DNA
<213> Homo sapiens

<220>
<221> misc_feature
<222> 22, 130
<223> n = A,T,C or G

<400> 288
tcgagcggcc gcccgggcag gnccacatcg gcagggtcgg agccctggcc gccatactcg 60
aactggaatc catcggtcat gctcttgccg aaccagacat gcctcttgtc cttggggttc 120
ttgctgatgn accagttctt ctgggccaca ctgggctgag tggggtacac gcaggtctca 180
ccagtctcca tgttgcagaa gactttgatg gcatccaggt tgcagccttg gttggggtca 240

atccagtact ctccactctt ccagtcagag tggcacatct tgaggtcacg gcaggtgcgg 300
gcggggttct tgacct 316

<210> 289
<211> 308
<212> DNA
<213> Homo sapiens

<220>
<221> misc_feature
<222> 36, 165, 191, 195, 218, 235
<223> n = A,T,C or G

<400> 289
agcgtggtcg cggccgaggt ccagcctgga gataanggtg aaggtggtgc ccccggactt 60
ccaggtatag ctggacctcg tggtagccct ggtgagagag gtgaaactgg ccctccagga 120
cctgctggtt tccctggtgc tcctggacag aatggtgaac ctggnggtaa aggagaaaga 180
ggggctccgg ntganaaagg tgaaggaggc cctcctgnat tggcaggggc cccangactt 240
agaggtggag ctggcccccc tggccccgaa ggaggaaagg gtgctgctgg tcctcctggg 300
ccacctgg 308

<210> 290
<211> 324
<212> DNA
<213> Homo sapiens

<220>
<221> misc_feature
<222> 184
<223> n = A,T,C or G

<400> 290
tcgagcggcc gcccgggcag gtctgggcca ggaggaccaa taggaccagt aggacccctt 60
gggccatctt tccctgggac accatcagca cctggaccgc ctggttcacc cttgtcaccc 120
tttggaccag gacttccaag acctcctctt tctccaggca ttccttgcag accaggagta 180
ccancagcac caggtggccc aggaggacca gcagcaccct ttcctccttc gggaccaggg 240
ggaccagctc cacctctaag tcctggggcc cctgccaatc caggagggcc tccttcacct 300
ttctcacccg gagcccctct ttct 324

<210> 291
<211> 278
<212> DNA
<213> Homo sapiens

<220>
<221> misc_feature
<222> 249, 267
<223> n = A,T,C or G

<400> 291
   tcgagcggcc gcccgggcag gtccaccggg atattcgggg gtctggcagg aatgggaggc 60
   atccagaacg agaaggagac catgcaaagc ctgaacgacc gcctggcctc ttacctggac 120
   agagtgagga gcctggagac cgacaaccgg aggctggaga gcaaaatccg ggagcacttg 180
   gagaagaagg gaccccaggt cagagactgg agccattact tcaagatcat cgaggacctg 240
   agggctcana tcttcgcaaa tactgcngac aatgcccg 278

<210> 292

<211> 299
<212> DNA
<213> Homo sapiens

<220>
<221> misc_feature
<222> 6, 19, 25, 51, 53, 61, 63, 70, 109, 136, 157, 241, 276
<223> n = A,T,C or G

<400> 292
atgcgnggtc gcggccgang accanctctg gctcatactt gactctaaag ncntcaccag 60
nanttacggn cattgccaat ctgcagaacg atgcgggcat tgtccgcant atttgcgaag 120
atctgagccc tcaggncctc gatgatcttg aagtaanggc tccagtctct gacctggggt 180
cccttcttct ccaagtgctc ccggattttg ctctccagcc tccggttctc ggtctccaag 240
ncttctcact ctgtccagga aaagaggcca ggcggncgat cagggctttt gcatggact 299

<210> 293
<211> 101
<212> DNA
<213> Homo sapiens

<400> 293
agcgtggtcg cggccgaggt tgtacaagct tttttttttt tttttttttt tttttttttt 60
tttttttttt tttttttttt tttttttttt tttttttttt t 101

<210> 294
<211> 285
<212> DNA
<213> Homo sapiens

<220>
<221> misc_feature
<222> 64, 103, 110, 237, 282
<223> n = A,T,C or G

<400> 294
tcgagcggcc gcccgggcag gtctgccaac accaagattg gcccccgccg catccacaca 60
gttngtgtgc ggggaggtaa caagaaatac cgtgccctga ggntggacgn ggggaatttc 120
tcctggggct cagagtgttg tactcgtaaa acaaggatca tcgatgttgt ctacaatgca 180
tctaataacg agctggttcg taccaagacc ctggtgaaga attgcatcgt gctcatngac 240
agcacaccgt accgacagtg ggtaccgaag tcccactatg cncct 285

<210> 295
<211> 216
<212> DNA
<213> Homo sapiens

<400> 295
tcgagcggcc gcccgggcag gtccaccaca cccaattcct tgctggtatc atggcagccg 60
ccacgtgcca ggattaccgg ctacatcatc aagtatgaga agcctgggtc tcctcccaga 120
gaagtggtcc ctcggccccg ccctggtgtc acagaggcta ctattactgg cctggaaccg 180
ggaaccgaat atacaattta tgtcattgcc ctgaag 216

<210> 296
<211> 414
<212> DNA
<213> Homo sapiens

<220>
<221> misc_feature
<222> 7, 10, 33, 61, 62, 63, 88, 109, 122, 255, 298, 307, 340,
355, 386, 393
<223> n = A,T,C or G

<400> 296
agcgtgntcn cggccgagga tggggaagct cgnctgtctt tttccttcca atcaggggct 60
nnntcttctg attattcttc agggcaanga cataaattgt atattcggnt cccggttcca 120
gnccagtaat agtagcctct gtgacaccag ggcggggccg agggaccact tctctgggag 180
gagacccagg cttctcatac ttgatgatga agccggtaat cctggcacgt gggcggctgc 240
catgatacca ccaangaatt gggtgtggtg gacctgcccg ggcgggccgc tcgaaaancc 300
gaattcntgc aagaatatcc atcacacttg ggcgggccgn tcgaaccatg catcntaaaa 360
gggccccaat ttccccccta ttaggngaag ccncatttaa caaattccac ttgg 414

<210> 297
<211> 376
<212> DNA
<213> Homo sapiens

<220>
<221> misc_feature
<222> 312, 326, 335, 361
<223> n = A,T,C or G

<400> 297
tcgagcggcc gcccgggcag gtctcgcggt cgcactggtg atgctggtcc tgttggtccc 60
cccggccctc ctggacctcc tggtccccct ggtcctccca gcgctggttt cgacttcagc 120
ttcctgcccc agccacctca agagaaggct cacgatggtg gccgctacta ccgggctgat 180
gatgccaatg tggttcgtga ccgtgacctc gaggtggaca ccaccctcaa gagccttgag 240
ccagcagaat cgaaaacatt cggaacccaa gaagggcaag cccgcaaaga aaccccgccc 300
gcacctggcc gngaacctcc aagaangtgc ccacntcttg actgggaaaa aaagggaaaa 360
ntacttggaa ttggac 376

<210> 298
<211> 357
<212> DNA
<213> Homo sapiens

<220>
<221> misc_feature
<222> 345, 346
<223> n = A,T,C or G

<400> 298
agcgtggtcg cggccgaggt ccacatcggc agggtcggag ccctggccgc catactcgaa 60
ctggaatcca tcggtcatgc tctcgccgaa ccagacatgc ctcttgtcct tggggttctt 120
gctgatgtac cagttcttct gggccacact gggctgagtg gggtacacgc aggtctcacc 180
agtctccatg ttgcagaaga ctttgatggc atccaggttg cagccttggt tggggtcaat 240
ccagtactct ccactcttcc agtcagaagt ggcacatctt gaggtcacgg cagggtgcgg 300
gcggggttct tgcgggctgc ccttctgggc tcccggaatg ttctnngaac ttgctgg 357

<210> 299
<211> 307
<212> DNA
<213> Homo sapiens

<220>
<221> misc_feature
<222> 281, 285, 306
<223> n = A,T,C or G

<400> 299
agcgtggtcg cggccgaggt ccactagagg tctgtgtgcc attgcccagg cagagtctct 60
gcgttacaaa ctcctaggag ggcttgctgt gcggagggcc tgctatggtg tgctgcggtt 120
catcatggag agtggggcca aaggctgcga ggttgtggtg tctgggaaac tccgaggaca 180
gagggctaaa tccatgaagt ttgtggatgg cctgatgatc cacagcggag accctgttaa 240
ctactacgtt gacacttgct tgtgcgccac gtgttgctca nacangggtg ggctgggcat 300
caaggng 307

<210> 300
<211> 351
<212> DNA
<213> Homo sapiens

<400> 300
tcgagcggcc gcccgggcag gtctgccaag gagaccctgt tatgctgtgg ggactggctg 60
gggcatggca ggcggctctg gcttcccacc cttctgttct gagatggggg tggtgggcag 120
tatctcatct ttgggttcca caatgctcac gtggtcaggc aggggcttct tagggccaat 180
cttaccagtt gggtcccagg gcagcatgat cttcaccttg atgcccagca caccctgtct 240
gagcaacacg tggcgcacag caagtgtcaa cgtaagtaag ttaacagggt ctccgctgtg 300
gatcatcagg ccatccacaa acttcatgga tttaaccctc tgtcctcgga g 351

<210> 301
<211> 330
<212> DNA
<213> Homo sapiens

<400> 301
tcgagcggcc gcccgggcag gtgtttcaga ggttccaagg tccactgtgg aggtcccagg 60
agtgctggtg gtgggcacag aggtccgatg ggtgaaacca ttgacataga gactgttcct 120
gtccagggtg taggggccca gctctttgat gccattggcc agttggctca gctcccagta 180
cagccgctct ctgttgagtc cagggctttt ggggtcaaga tgatggatgc agatggcatc 240
cactccagtg gctgctccat ccttctcgga cctgagagag gtcagtctgc agccagagta 300
cagagggcca acactggtgt tctttgaata 330

<210> 302
<211> 317
<212> DNA
<213> Homo sapiens

<220>
<221> misc_feature
<222> 129, 295
<223> n = A,T,C or G

<400> 302
agcgtggtcg cggccgaggt ctgtactggg agctaagcaa actgaccaat gacattgaag 60
agctgggccc ctacaccctg gacaggaaca gtctctatgt caatggtttc acccatcaga 120
gctctgtgnc caccaccagc actcctggga cctccacagt ggatttcaga acctcaggga 180
ctccatcctc cctctccagc cccacaatta tggctgctgg ccctctcctg gtaccattca 240
ccctcaactt caccatcacc aacctgcagt atggggagga catgggtcac cctgnctcca 300
ggaagttcaa caccaca 317

<210> 303
<211> 283
<212> DNA
<213> Homo sapiens

<220>
<221> misc_feature
<222> 139, 146, 195
<223> n = A,T,C or G

<400> 303
tcgagcggcc gcccggacag gtctgggcgg atagcaccgg gcatattttg gaatggatga 60
ggtctggcac cctgagcagt ccagcgagga cttggtctta gttgagcaat ttggctagga 120
ggatagtatg cagcacggnt ctgagnctgt gggatagctg ccatgaagta acctgaagga 180
ggtgctggct ggtangggtt gattacaggg ttgggaacag ctcgtacact tgccattctc 240
tgcatatact ggttagtgag gtgagcctgg ccctcttctt ttg 283

<210> 304
<211> 72
<212> DNA
<213> Homo sapiens

<220>
<221> misc_feature
<222> 59
<223> n = A,T,C or G

<400> 304
agcgtggtcg cggccgaggt gagccacagg tgaccggggc tgaagctggg gctgctggnc 60
ctgctggtcc tg 72

<210> 305
<211> 245
<212> DNA
<213> Homo sapiens

<220>
<221> misc_feature
<222> 5, 11, 22, 98, 102
<223> n = A,T,C or G

<400> 305
cagcngctcc nacggggcct gngggaccaa caacaccgtt ttcaccctta ggccctttgg 60
ctcctctttc tcctttagca ccaggttgac cagcagcncc ancaggacca gcaaatccat 120
tggggccagc aggaccgacc tcaccacgtt caccagggct tccccgagga ccagcaggac 180
cagcaggacc agcagcccca gcttcgcccc ggtcacctgt ggctcacctc ggccgcgacc 240
acgct 245

<210> 306
<211> 246
<212> DNA
<213> Homo sapiens

<220>
<221> misc_feature
<222> 144, 159

<223> n = A,T,C or G

<400> 306
tcgagcggtc gcccgggcag gtccaccggg atagccgggg gtctggcagg aatgggaggc 60
atccagaacg agaaggagac catgcaaagc ctgaacgacc gcctggcctc ttacctggac 120
agagtgagga gcctggagac cganaaccgg aggctggana gcaaaatccg ggagcacttg 180
gagaagaagg gaccccaggt caagagactg gagccattac ttcaagatca tcgagggacc 240
tggagg 246

<210> 307
<211> 333
<212> DNA
<213> Homo sapiens

<220>
<221> misc_feature
<222> 5
<223> n = A,T,C or G

<400> 307
agcgnggtcg cggccgaggt ccagctctgt ctcatacttg actctaaagt catcagcagc 60
aagacgggca ttgtcaatct gcagaacgat gcgggcattg tccgcagtat ttgcgaagat 120
ctgagccctc aggtcctcga tgatcttgaa gtaatggctc cagtctctga cctggggtcc 180
cttcttctcc aagtgctccc ggattttgct ctccagcctc cggttctcgg tctccaggct 240
cctcactctg tccaggtaag aaggcccagg cggtcgttca ggctttgcat ggtctccttc 300
tcgttctgga tgcctcccat tcctgccaga ccc 333

<210> 308
<211> 310
<212> DNA
<213> Homo sapiens

<400> 308
tcgagcggcc gcccgggcag gtcaggaagc acattggtct tagagccact gcctcctgga 60
ttccacctgt gctgcggaca tctccaggga gtgcagaagg gaagcaggtc aaactgctca 120
gatcagtcag actggctgtt ctcagttctc acctgagcaa ggtcagtctg cagccagagt 180
acagagggcc aacactggtg ttcttgaaca agggcttgag cagaccctgc agaaccctct 240
tccgtggtgt tgaacttcct ggaaaccagg gtgttgcatg tttttcctca taatgcaagg 300
ttggtgatgg 310

<210> 309
<211> 429
<212> DNA
<213> Homo sapiens

<400> 309
agcgtggtcg cggccgaggt ccacatcggc agggtcggag ccctggccgc catactcgaa 60
ctggaatcca tcggtcatgc tctcgccgaa ccagacatgc ctcttgtcct tggggttctt 120
gctgatgtac cagttcttct gggccacact gggctgagtg gggtacaccg caggtctcac 180
cagtctccat gttgcagaag actttgatgg catccaggtt gcagccttgg ttggggtcaa 240
tccagtactc tccactcttc cagtcagaag tgggcacatc ttgaggtcac cggcaggtgc 300
cgggccgggg gttcttgcgg cttgccctct gggctccgga tgttctcgat ctgcttggct 360
caggctcttg agggtgggtg tccacctcga ggtcacggtc accgaaacct gcccgggcgg 420
cccgctcga 429

<210> 310
<211> 430

<212> DNA
<213> Homo sapiens

<220>
<221> misc_feature
<222> 342
<223> n = A,T,C or G

<400> 310
tcgagcggtc gcccgggcag gtttcgtgac cgtgacctcg aggtggacac caccctcaag 60
agcctgagcc agcagatcga gaacatccgg agcccagagg gcagccgcaa gaaccccgcc 120
cgcacctgcc gtgacctcaa gatgtgccac tctgactgga agagtggaga gtactggatt 180
gaccccaacc aaggctgcaa cctggatgcc atcaaagtct tctgcaacat ggagactggt 240
gagacctgcg tgtaccccac tcagcccagt gtgggcccag aagaaactgg tacatcagca 300
aggaacccca aggacaagag gcattgtctt ggttcggcga gnagcatgac ccgatggatt 360
ccagtttcga gtattggcgg ccagggcttc ccgacccttg ccgatgtgga cctcggccgc 420
gaccaccgct 430

<210> 311
<211> 2996
<212> DNA
<213> Homo sapiens

<400> 311
cagccaccgg agtggatgcc atctgcaccc accgccctga ccccacaggc cctgggctgg 60
acagagagca gctgtatttg gagctgagcc agctgaccca cagcatcact gagctgggcc 120
cctacaccct ggacagggac agtctctatg tcaatggttt cacacagcgg agctctgtgc 180
ccaccactag cattcctggg acccccacag tggacctggg aacatctggg actccagttt 240
ctaaacctgg tccctcggct gccagccctc tcctggtgct attcactctc aacttcacca 300
tcaccaacct gcggtatgag gagaacatgc agcaccctgg ctccaggaag ttcaacacca 360
cggagagggt ccttcagggc ctggtccctg ttcaagagca ccagtgttgg ccctctgtac 420
tctggctgca gactgacttt gctcaggcct gaaaaggatg ggacagccac tggagtggat 480
gccatctgca cccaccaccc tgaccccaaa agccctaggc tggacagaga gcagctgtat 540
tgggagctga gccagctgac ccacaatatc actgagctgg gcccctatgc cctggacaac 600
gacagcctct ttgtcaatgg tttcactcat cggagctctg tgtccaccac cagcactcct 660
gggaccccca cagtgtatct gggagcatct aagactccag cctcgatatt tggcccttca 720
gctgccagcc atctcctgat actattcacc ctcaacttca ccatcactaa cctgcggtat 780
gaggagaaca tgtggcctgg ctccaggaag ttcaacacta cagagagggt ccttcagggc 840
ctgctaaggc ccttgttcaa gaacaccagt gttggccctc tgtactctgg ctgcaggctg 900
accttgctca ggccagagaa agatggggaa gccaccggag tggatgccat ctgcacccac 960
cgccctgacc ccacaggccc tgggctggac agagagcagc tgtatttgga gctgagccag 1020
ctgacccaca gcatcactga gctgggcccc tacacactgg acagggacag tctctatgtc 1080
aatggtttca cccatcggag ctctgtaccc accaccagca ccggggtggt cagcgaggag 1140
ccattcacac tgaacttcac catcaacaac ctgcgctaca tggcggacat gggccaaccc 1200
ggctccctca agttcaacat cacagacaac gtcatgaagc acctgctcag tcctttgttc 1260
cagaggagca gcctgggtgc acggtacaca ggctgcaggg tcatcgcact aaggtctgtg 1320
aagaacggtg ctgagacacg ggtggacctc ctctgcacct acctgcagcc cctcagcggc 1380
ccaggtctgc ctatcaagca ggtgttccat gagctgagcc agcagaccca tggcatcacc 1440
cggctgggcc cctactctct ggacaaagac agcctctacc ttaacggtta caatgaacct 1500
ggtccagatg agcctcctac aactcccaag ccagccacca cattcctgcc tcctctgtca 1560
gaagccacaa cagccatggg gtaccacctg aagaccctca cactcaactt caccatctcc 1620
aatctccagt attcaccaga tatgggcaag ggctcagcta cattcaactc caccgagggg 1680
gtccttcagc acctgctcag acccttgttc cagaagagca gcatgggccc cttctacttg 1740
ggttgccaac tgatctccct caggcctgag aaggatgggg cagccactgg tgtggacacc 1800
acctgcacct accaccctga ccctgtgggc cccgggctgg acatacagca gctttactgg 1860
gagctgagtc agctgaccca tggtgtcacc caactgggct tctatgtcct ggacagggat 1920
agcctcttca tcaatggcta tgcaccccag aatttatcaa tccggggcga gtaccagata 1980

aatttccaca ttgtcaactg gaacctcagt aatccagacc ccacatcctc agagtacatc 2040
accctgctga gggacatcca ggacaaggtc accacactct acaaaggcag tcaactacat 2100
gacacattcc gcttctgcct ggtcaccaac ttgacgatgg actccgtgtt ggtcactgtc 2160
aaggcattgt tctcctccaa tttggacccc agcctggtgg agcaagtctt tctagataag 2220
accctgaatg cctcattcca ttggctgggc tccacctacc agttggtgga catccatgtg 2280
acagaaatgg agtcatcagt ttatcaacca acaagcagct ccagcaccca gcacttctac 2340
ctgaatttca ccatcaccaa cctaccatat tcccaggaca aagcccagcc aggcaccacc 2400
aattaccaga ggaacaaaag gaatattgag gatgcgctca accaactctt ccgaaacagc 2460
agcatcaaga gttatttttc tgactgtcaa gtttcaacat tcaggtctgt ccccaacagg 2520
caccacaccg gggtggactc cctgtgtaac ttctcgccac tggctcggag agtagacaga 2580
gttgccatct atgaggaatt tctgcggatg acccggaatg gtacccagct gcagaacttc 2640
accctggaca ggagcagtgt ccttgtggat gggtattttc ccaacagaaa tgagccctta 2700
actgggaatt ctgaccttcc cttctgggct gtcatcctca tcggcttggc aggactcctg 2760
ggactcatca catgcctgat ctgcggtgtc ctggtgacca cccgccggcg gaagaaggaa 2820
ggagaataca acgtccagca acagtgccca ggctactacc agtcacacct agacctggag 2880
gatctgcaat gactggaact tgccggtgcc tggggtgcct ttcccccagc cagggtccaa 2940
agaagcttgg ctggggcaga aataaaccat attggtcgga cacaaaaaaa aaaaaa 2996

<210> 312
<211> 914
<212> PRT
<213> Homo sapiens

<400> 312
Met Ser Met Val Ser His Ser Gly Ala Leu Cys Pro Pro Leu Ala Phe
1               5                   10                  15
Leu Gly Pro Pro Gln Trp Thr Trp Glu His Leu Gly Leu Gln Phe Leu
            20                  25                  30
Asn Leu Val Pro Arg Leu Pro Ala Leu Ser Trp Cys Tyr Ser Leu Ser
        35                  40                  45
Thr Ser Pro Ser Pro Thr Cys Gly Met Arg Arg Thr Cys Ser Thr Leu
    50                  55                  60
Ala Pro Gly Ser Ser Thr Pro Arg Arg Gly Ser Phe Arg Ala Trp Ser
65                  70                  75                  80
Leu Phe Lys Ser Thr Ser Val Gly Pro Leu Tyr Ser Gly Cys Arg Leu
                85                  90                  95
Thr Leu Leu Arg Pro Glu Lys Asp Gly Thr Ala Thr Gly Val Asp Ala
            100                 105                 110
Ile Cys Thr His His Pro Asp Pro Lys Ser Pro Arg Leu Asp Arg Glu
        115                 120                 125
Gln Leu Tyr Trp Glu Leu Ser Gln Leu Thr His Asn Ile Thr Glu Leu
    130                 135                 140
Gly Pro Tyr Ala Leu Asp Asn Asp Ser Leu Phe Val Asn Gly Phe Thr
145                 150                 155                 160
His Arg Ser Ser Val Ser Thr Thr Ser Thr Pro Gly Thr Pro Thr Val
                165                 170                 175
Tyr Leu Gly Ala Ser Lys Thr Pro Ala Ser Ile Phe Gly Pro Ser Ala
            180                 185                 190
Ala Ser His Leu Leu Ile Leu Phe Thr Leu Asn Phe Thr Ile Thr Asn
        195                 200                 205
Leu Arg Tyr Glu Glu Asn Met Trp Pro Gly Ser Arg Lys Phe Asn Thr
    210                 215                 220
Thr Glu Arg Val Leu Gln Gly Leu Leu Arg Pro Leu Phe Lys Asn Thr
225                 230                 235                 240
Ser Val Gly Pro Leu Tyr Ser Gly Cys Arg Leu Thr Leu Leu Arg Pro
                245                 250                 255
Glu Lys Asp Gly Glu Ala Thr Gly Val Asp Ala Ile Cys Thr His Arg

            260                 265                 270
Pro Asp Pro Thr Gly Pro Gly Leu Asp Arg Glu Gln Leu Tyr Leu Glu
        275                 280                 285
Leu Ser Gln Leu Thr His Ser Ile Thr Glu Leu Gly Pro Tyr Thr Leu
    290                 295                 300
Asp Arg Asp Ser Leu Tyr Val Asn Gly Phe Thr His Arg Ser Ser Val
305                 310                 315                 320
Pro Thr Thr Ser Thr Gly Val Val Ser Glu Glu Pro Phe Thr Leu Asn
                325                 330                 335
Phe Thr Ile Asn Asn Leu Arg Tyr Met Ala Asp Met Gly Gln Pro Gly
            340                 345                 350
Ser Leu Lys Phe Asn Ile Thr Asp Asn Val Met Lys His Leu Leu Ser
        355                 360                 365
Pro Leu Phe Gln Arg Ser Ser Leu Gly Ala Arg Tyr Thr Gly Cys Arg
    370                 375                 380
Val Ile Ala Leu Arg Ser Val Lys Asn Gly Ala Glu Thr Arg Val Asp
385                 390                 395                 400
Leu Leu Cys Thr Tyr Leu Gln Pro Leu Ser Gly Pro Gly Leu Pro Ile
                405                 410                 415
Lys Gln Val Phe His Glu Leu Ser Gln Gln Thr His Gly Ile Thr Arg
            420                 425                 430
Leu Gly Pro Tyr Ser Leu Asp Lys Asp Ser Leu Tyr Leu Asn Gly Tyr
        435                 440                 445
Asn Glu Pro Gly Pro Asp Glu Pro Pro Thr Thr Pro Lys Pro Ala Thr
    450                 455                 460
Thr Phe Leu Pro Pro Leu Ser Glu Ala Thr Thr Ala Met Gly Tyr His
465                 470                 475                 480
Leu Lys Thr Leu Thr Leu Asn Phe Thr Ile Ser Asn Leu Gln Tyr Ser
                485                 490                 495
Pro Asp Met Gly Lys Gly Ser Ala Thr Phe Asn Ser Thr Glu Gly Val
            500                 505                 510
Leu Gln His Leu Leu Arg Pro Leu Phe Gln Lys Ser Ser Met Gly Pro
        515                 520                 525
Phe Tyr Leu Gly Cys Gln Leu Ile Ser Leu Arg Pro Glu Lys Asp Gly
    530                 535                 540
Ala Ala Thr Gly Val Asp Thr Thr Cys Thr Tyr His Pro Asp Pro Val
545                 550                 555                 560
Gly Pro Gly Leu Asp Ile Gln Gln Leu Tyr Trp Glu Leu Ser Gln Leu
                565                 570                 575
Thr His Gly Val Thr Gln Leu Gly Phe Tyr Val Leu Asp Arg Asp Ser
            580                 585                 590
Leu Phe Ile Asn Gly Tyr Ala Pro Gln Asn Leu Ser Ile Arg Gly Glu
        595                 600                 605
Tyr Gln Ile Asn Phe His Ile Val Asn Trp Asn Leu Ser Asn Pro Asp
    610                 615                 620
Pro Thr Ser Ser Glu Tyr Ile Thr Leu Leu Arg Asp Ile Gln Asp Lys
625                 630                 635                 640
Val Thr Thr Leu Tyr Lys Gly Ser Gln Leu His Asp Thr Phe Arg Phe
                645                 650                 655
Cys Leu Val Thr Asn Leu Thr Met Asp Ser Val Leu Val Thr Val Lys
            660                 665                 670
Ala Leu Phe Ser Ser Asn Leu Asp Pro Ser Leu Val Glu Gln Val Phe
        675                 680                 685
Leu Asp Lys Thr Leu Asn Ala Ser Phe His Trp Leu Gly Ser Thr Tyr
    690                 695                 700
Gln Leu Val Asp Ile His Val Thr Glu Met Glu Ser Ser Val Tyr Gln
705                 710                 715                 720

Pro Thr Ser Ser Ser Ser Thr Gln His Phe Tyr Leu Asn Phe Thr Ile
                725                 730                 735
Thr Asn Leu Pro Tyr Ser Gln Asp Lys Ala Gln Pro Gly Thr Thr Asn
            740                 745                 750
Tyr Gln Arg Asn Lys Arg Asn Ile Glu Asp Ala Leu Asn Gln Leu Phe
        755                 760                 765
Arg Asn Ser Ser Ile Lys Ser Tyr Phe Ser Asp Cys Gln Val Ser Thr
    770                 775                 780
Phe Arg Ser Val Pro Asn Arg His His Thr Gly Val Asp Ser Leu Cys
785                 790                 795                 800
Asn Phe Ser Pro Leu Ala Arg Arg Val Asp Arg Val Ala Ile Tyr Glu
                805                 810                 815
Glu Phe Leu Arg Met Thr Arg Asn Gly Thr Gln Leu Gln Asn Phe Thr
            820                 825                 830
Leu Asp Arg Ser Ser Val Leu Val Asp Gly Tyr Phe Pro Asn Arg Asn
        835                 840                 845
Glu Pro Leu Thr Gly Asn Ser Asp Leu Pro Phe Trp Ala Val Ile Leu
    850                 855                 860
Ile Gly Leu Ala Gly Leu Leu Gly Leu Ile Thr Cys Leu Ile Cys Gly
865                 870                 875                 880
Val Leu Val Thr Thr Arg Arg Arg Lys Lys Glu Gly Glu Tyr Asn Val
                885                 890                 895
Gln Gln Gln Cys Pro Gly Tyr Tyr Gln Ser His Leu Asp Leu Glu Asp
            900                 905                 910
Leu Gln

<210> 313
<211> 656
<212> DNA
<213> Homo sapiens

<400> 313
acagccagtc ggagctgcaa gtgttctggg tggatcgcgy atatgcactc aaaatgctct 60
ttgtaaagga aagccacaac atgtccaagg gacctgaggc gacttggagg ctgagcaaag 120
tgcagtttgt ctacgactcc tcggagaaaa cccacttcaa agacgcagtc agtgctggga 180
agcacacagc caactcgcac cacctctctg ccttggtcac ccccgctggg aagtcctatg 240
agtgtcaagc tcaacaaacc atttcactgg cctctagtga tccgcagaag acggtcacca 300
tgatcctgtc tgcggtccac atccaacctt ttgacattat ctcagatttt gtcttcagtg 360
aagagcataa atgcccagtg gatgagcggg agcaactgga agaaaccttg cccctgattt 420
tggggctcat cttgggcctc gtcatcatgg taacactcgc gatttaccac gtccaccaca 480
aaatgactgc caaccaggtg cagatccctc gggacagatc ccagtataag cacatgggct 540
agaggccgtt aggcaggcac cccctattcc tgctccccca actggatcag gtagaacaac 600
aaaagcactt ttccatcttg tacacgagat acaccaacat agctacaatc aaacag     656

<210> 314
<211> 519
<212> DNA
<213> Homo sapiens

<400> 314
tgtgcgtgga ccagtcagct tccgggtgtg actggagcag ggcttgtcgt cttcttcaga 60
gtcactttgc aggggttggt gaagctgctc ccatccatgt acagctccca gtctactgat 120
gtttaaggat ggtctcggtg gttaggccca ctagaataaa ctgagtccaa tacctctaca 180
cagttatgtt taactgggct ctctgacacc gggaggaagg tggcggggtt taggtgttgc 240
aaacttcaat ggttatgcgg ggatgttcac agagcaagct ttggtatcta gctagtctag 300

cattcattag ctaatggtgt cctttggtat ttattaaaat caccacagca tagggggact 360
ttatgtttag gttttgtcta agagttagct tatctgcttc ttgtgctaac agggctattg 420
ctaccaggga ctttggacat gggggccagc gtttggaaac ctcatctagt ttttttgaga 480
gataggccac tggccttgga cctcggccgc gaccacgct 519

<210> 315
<211> 441
<212> DNA
<213> Homo sapiens

<400> 315
cacagagcgt ttattgacac caccactcct gaaaattggg atttcttatt aggttcccct 60
aaaagttccc atgttgatta catgtaaata gtcacatata tacaatgaag gcagtttctt 120
cagaggcaac cagggtttat agtgctaggt aaatgtcatc tcttttgtgc tactgactca 180
ttgtcaaacg tctctgcact gttttcagcc tctccacgtt gcctctgtcc tgcttcttag 240
ttccttcttt gtgacaaacc aaaagaataa gaggatttag aacaggactg cttttcccct 300
atgatttaaa aattccaatg actttcgccc ttgggagaaa tttccaagga aatctctctc 360
gctcgctctc tccgttttcc tttgtgagct tctgggggag ggttagtggt gactttttga 420
tacgaaaaaa tgcattttgt g 441

<210> 316
<211> 247
<212> DNA
<213> Homo sapiens

<400> 316
tggcgcggct gctggatttc accttcttgc acctgccggt gagcgcctgg ggtctaaagg 60
ggcgggatac tccattatgg cccctcgccc tgtagggctg gaatagttag aaaaggcaac 120
ccagtctagc ttggtaagaa gagagacatg cccccaacct cggcgccctt tttcctcacg 180
atctgctgtc cttacttcag cgactgcagg agcttcacct gcaagaaaac agcattgagc 240
tgctgac 247

<210> 317
<211> 409
<212> DNA
<213> Homo sapiens

<400> 317
tgacagggct cctggagttg ttaagtcacc aagtagctgc aggggatgga cactgcccca 60
cacgatgtgg gatgaacagc agccttggtt tgtagcccag ggtgtccatg gatttgaccc 120
gaatgctccc tggaggccct gtggcgagga caggcactgg atggtccaga ccctctggct 180
ggaggagtgg tggagccagg actgggcctt cagccatgag ggctagaata acctgacctc 240
ttgcattcta acactgggtc attaatgaca cctttccagt ggatgttgca aaaaccaaca 300
ctgtcaggaa cctggccctg ggagggctca ggtgagctca caaggagagg tcaagccaag 360
ccaaagggta ggkaacacac aacaccaggg gaaaccagcc cccaaacca 409

<210> 318
<211> 320
<212> DNA
<213> Homo sapiens

<220>
<221> misc_feature
<222> 6, 17, 24, 271
<223> n = A,T,C or G

<400> 318

caaggnagat cttaagnggg gtcntatgta agtgtgctcc tggctccagg gttcctggag 60
cctcacgagg tcaggggaac ccttgtagaa ctccaccagc agcatcatct cgtgaaggat 120
gtcattggtc aggaagctgt cctggacgta ggccatctcc acatccatgg ggatgccata 180
gtcactgggc ctttgctcgg gaggaggcat cacccagaaa ggcgagatct tggactcggg 240
gcctgggttg ccagaatagt aaggggagca nagcagggcg aggcagggct ggaagccatt 300
gctggagccc tgcagccgca 320

<210> 319
<211> 212
<212> DNA
<213> Homo sapiens

<220>
<221> misc_feature
<222> 172
<223> n = A,T,C or G

<400> 319
tgaagcaata gcgcccccat tttacaggcg gagcatggaa gccagagagg tgggtggggg 60
agggggtcct tccctggctc aggcagatgg gaagatgagg aagccgctga agacgctgtc 120
ggcctcagag ccctggtaaa tgtgaccctt tttggggtct ttttcaaccc anacctggtc 180
accctgctgc agacctcggc cgcgaccacg ct 212

<210> 320
<211> 769
<212> DNA
<213> Homo sapiens

<400> 320
tggaggtgta gcagtgagag gagatytcag gcaagagtgt cacagcagag ccctaaascc 60
tccaactcac cagtgagaga tgagactgcc cagtactcag ccttcatctc ctgggccacc 120
tggagggcgt ctttctccat cagcgcatac tgagcagggg tactcagatc cttcttggaa 180
cctacaagga agagaagcac actggaaggg tcattctcct tcagggcatc ggccagccac 240
tgcctgccat gggaggtgga aagtaaggga tgagtgagtc tgcagggccc ctcccactga 300
cattcatagg cccaattacc ccctctctgg tcctacatgc attcttcttc ttcctgacca 360
cccctctgtt ctgaaccctc tcttcccgga gcctcccatt atattgcagg atgctcactt 420
acttggtatg ttccagagat gccacatcat tcaggttgaa gacaatgatg atggcttgga 480
agagtggcag aaacagcccc aggttgacag ggaagacact actgctcatt tccccaatcc 540
ttccagctcc atatgagaaa gccatgtgca ctctgagacc cacctacccc acttcaccca 600
gccccttacc ttgagctcct ctatagtagg ttgatgcaat gcatttgaac ctctcctgcc 660
cagcggtatc ccaactggaa ggaaggaaga gtgaagcaca ggtatgtatc ttggggggtg 720
tgggtgctgg ggagaaggga tagctggaag gggtgtggaa gcactcaca 769

<210> 321
<211> 690
<212> DNA
<213> Homo sapiens

<220>
<221> misc_feature
<222> 633, 666
<223> n = A,T,C or G

<400> 321
tgggctgtgg gcggcacctg tgctctgcag gccagacagc gatagaagcc tttgtctgtg 60
cctactcccc cggaggcaac tgggaggtca acgggaagac aatcatcccc tataagaagg 120
gtgcctggtg ttcgctctgc acagccagtg tctcaggctg cttcaaagcc tgggaccatg 180

caggggggct ctgtgaggtc cccaggaatc cttgtcgcat gagctgccag aaccatggac 240
gtctcaacat cagcacctgc cactgccact gtccccctgg ctacacgggc agatactgcc 300
aagtgaggtg cagcctgcag tgtgtgcacg gccggttccg ggaggaggag tgctcgtgcg 360
tctgtgacat cggctacggg ggagcccagt gtgccaccaa ggtgcatttt cccttccaca 420
cctgtgacct gaggatcgac ggagactgct tcatggtgtc ttcagaggca gacacctatt 480
acagaagcca ggatgaaatg tcagaggaat ggcggggtgc tggcccagat caagagccag 540
aaagtgcagg acatcctcgc cttctatctg ggccgcctgg agaccaccaa cgaggtgact 600
gacagtgact ttgagaccag gaacttctgg atngggctca cctacaagac cgccaaggac 660
tccttncgct gggccacagg ggagcaccag 690

<210> 322
<211> 104
<212> DNA
<213> Homo sapiens

<400> 322
gtcgcaagcc ggagcaccac catgtagcct ttcccgaagt accggacctt ctcctcctcc 60
acgctcacat cacggacatc atggagcagg accaccacct ggtc 104

<210> 323
<211> 118
<212> DNA
<213> Homo sapiens

<400> 323
gggccctggg cgcttccaaa tgacccagga ggtggtctgc gacgaatgcc ctaatgtcaa 60
actagtgaat gaagaacgaa cactggaagt agaaatagag cctggggtga gagacgga 118

<210> 324
<211> 354
<212> DNA
<213> Homo sapiens

<400> 324
tgctctccgg gagcttgaag aagaaactgg ctacaaaggg gacattgccg aatgttctcc 60
agcggtctgt atggacccag gcttgtcaaa ctgtactata cacatcgtga cagtcaccat 120
taacggagat gatgccgaaa acgcaaggcc gaagccaaag ccaggggatg gagagtttgt 180
ggaagtcatt tctttaccca agaatgacct gctgcagaga cttgatgctc tggtagctga 240
agaacatctc acagtggacg ccagggtcta ttcctacgct ctagcgctga aacatgcaaa 300
tgcaaagcca tttgaagtgc ccttcttgaa attttaagcc caaatatgac actg 354

<210> 325
<211> 642
<212> DNA
<213> Homo sapiens

<220>
<221> misc_feature
<222> 1
<223> n = A,T,C or G

<400> 325
ncatgcttga atgggctcct ggtgagagat tgccccctgg tggtgaaaca atcgtgtgtg 60
cccactgata ccaagaccaa tgaaagagac acagttaagc agcaatccat ctcatttcca 120
ggcacttcaa taggtcgctg attggtcctt gcaccagcag tggtagtcgt acctatttca 180
gagaggtctg aaattcaggt tcttagtttg ccagggacag gccctacctt atattttttt 240
ccatcttcat catccacttc tgcttacagt ttgctgctta caataactta atgatggatt 300

gagttatctg ggtggtctct agccatctgg gcagtgtggt tctgtctaac caaagggcat 360
tggcctcaaa ccctgcattt ggtttagggg ctaacagagc tcctcagata atcttcacac 420
acatgtaact gctggagatc ttattctatt atgaataaga aacgagaagt ttttccaaag 480
tgttagtcag gatctgaagg ctgtcattca gataacccag cttttccttt tggcttttag 540
cccattcaga ctttgccaga gtcaagccaa ggattgcttt tttgctacag ttttctgcca 600
aatggcctag ttcctgagta cctggaaacc agagagaaag ag 642

<210> 326
<211> 455
<212> DNA
<213> Homo sapiens

<400> 326
tccgtgagga tgagcttcga gtccttcacc aggcactgca ggggcacagt cacgtcaatc 60
accttcacct tctcgctctt cctgctcttg tcattgacaa acttcccgta ccaggcattg 120
acgatgatga ggcccattct ggactcttct gcctcaatta tccttcggac agattcctgc 180
atcagccgga cagcggactc cgcctcttgc ttcttctgca gcacatcggt ggcggcgctt 240
tccctctgct tctccaattc cttctctttc tgagccctga ggtatggttt gatgatcaga 300
cggtgcatgg caaagtagac cactagaggc cccacggtgg catagaacat ggcgctgggc 360
agaagctggt ccgtcaagtg aatagggaag aagtatgtct gactggccct gttgagcttg 420
actttgagag aaacgccctg tggaactcca acgct 455

<210> 327
<211> 321
<212> DNA
<213> Homo sapiens

<400> 327
ttcactgtga actcgcagtc ctcgatgaac tcgcacagat gtgacagccc tgtctccttg 60
ctctctgagt tctcttcaat gatgctgatg atgcagtcca cgatagcgcg cttatactca 120
aagccaccct cttcccgcag catggtgaac aggaagttca taaggacggc gtgtttgcga 180
ggatatttct gacacagggc actgatggcc tggacaacca ccaccttgaa ttcatccgag 240
atttctgaca tgaaggagga gatctgcttc atgaggcggt cgatgctgct ctcgctgccc 300
gtcttaagga gggtggtgat g 321

<210> 328
<211> 476
<212> DNA
<213> Homo sapiens

<220>
<221> misc_feature
<222> 302, 311
<223> n = A,T,C or G

<400> 328
tgcaggaggg gccatggggg ctgtgaatgg gatgcagccc catggtgtcc ctgataaatc 60
cagtgtgcag tctgatgaag tctgggtggg tgtggtctac gggctggcag ctaccatgat 120
ccaagaggta atgcactcct tttcccatct ctccaccatc tgtatcctgg ccmagaaaaa 180
cttcccttca aaccaaccaa aatttccttt caaaggcata acccaaatgc catccttggt 240
ccggtctaat aaagcctccc ccatttttcc cctggtatgc attcccaggc tccctggcct 300
tncagggctt nctgtctgtg ggtcatagtt tatctcctcc cacttgctgg gagctccttg 360
aaggcaaaga ctctactgcc tccatctatc cagtggaagt ggctcttcag agggtgccaa 420
gttagtatgt atgactgtca tctctcccaa caggcctgga cttggsaggg cttcca 476

<210> 329
<211> 340

<212> DNA
<213> Homo sapiens

<400> 329
cgagggagat tgccagcacc ctgatggaga gtgagatgat ggagatcttg tcagtgctag 60
ctaagggtga ccacagccct gtcacaaggg ctgctgcagc ctgcctggac aaagcagtgg 120
aatatgggct tatccaaccc aaccaagatg gagagtgagg gggttgtccc tgggcccaag 180
gctcatgcac acgctaccta ttgtggcacg gagagtaagg acggaagcag ctttggctgg 240
tggtggctgg catgcccaat actcttgccc atcctcgctt gctgccctag gatgtcctct 300
gttctgagtc agcggccacg ttcagtcaca cagccctgct 340

<210> 330
<211> 277
<212> DNA
<213> Homo sapiens

<400> 330
tgtcaccatc acattggtgc caaataccca gaagacatcg tagatgaaga gtccgcccag 60
caggatgcag ccagtgctga cattgttgag gtgcaggagc tctactccat taagggagaa 120
ggccaggcca aaaaggttgt tggcaatcca gtgcttcctc agcaggtacc agacgccaac 180
gatgctgctc aggcccaggc acaccaggtc cttggtgtca aattcataat tgatgatctc 240
ctccttgttt tcccagaacc ctgtgtgaag agcagac 277

<210> 331
<211> 136
<212> DNA
<213> Homo sapiens

<400> 331
ttgcttccca cctcctttct ctgtcctctc ctgaggttct gccttacaat ggggacactg 60
atacaaacca cacacacaat gaggatgaaa acagataaca ggtaaaatga cctcacctgc 120
ccgggcggcc gctcga 136

<210> 332
<211> 184
<212> DNA
<213> Homo sapiens

<400> 332
ttgtgagata aacgcagata ctgcaatgca ttaaaacgct tgaaatactc atcagggatg 60
ttgctgatct tattgttgtc taagtagaga gttagaagag agacagggag accagaaggc 120
agtctggcta tctgattgaa gctcaagtca aggtattcga gtgatttaag acctttaaaa 180
gcag 184

<210> 333
<211> 384
<212> DNA
<213> Homo sapiens

<400> 333
cggaaaactt cgaggaattg ctcaaagtgc tgggggtgaa tgtgatgctg aggaagattg 60
ctgtggctgc agcgtccaag ccagcagtgg agatcaaaca ggagggagac actttctaca 120
tcaaaacctc caccaccgtg cgcaccacag agattaactt caaggttggg gaggagtttg 180
aggagcagac tgtggatggg aggccctgta agagcctggt gaaatgggag agtgagaata 240
aaatggtctg tgagcagaag ctcctgaagg gagagggccc caagacctcg tggaccagag 300
aactgaccaa cgatggggaa ctgatcctga ccatgacggc ggatgacgtt gtgtgcacca 360
gggtctacgt ccgagagtga gcgg 384

<210> 334
<211> 169
<212> DNA
<213> Homo sapiens

<220>
<221> misc_feature
<222> 2, 165
<223> n = A,T,C or G

<400> 334
cnacaaacag agcagacacc ctggatccgg tcctgctact ggccaggacg gctggaccgt 60
aaaattgaat ttccacttcc tgaccgccgc cagaagagat tgattttctc cactatcact 120
agcaagatga acctctctga ggaggttgac ttggaagact atgtngccc 169

<210> 335
<211> 185
<212> DNA
<213> Homo sapiens

<400> 335
ccaggtttgc agcccaggct gcacatcagg ggactgcctc gcaatacttc atgctgttgc 60
tgctgactga tggtgctgtg acggatgtgg aagccacacg tgaggctgtg gtgcgtgcct 120
cgaacctgcc catgtcagtg atcattgtgg gtgtgggtgg tgctgacttt gaggccatgg 180
agcag 185

<210> 336
<211> 358
<212> DNA
<213> Homo sapiens

<220>
<221> misc_feature
<222> 26
<223> n = A,T,C or G

<400> 336
ctgcccctgc cttacggcgg ccaganacac acccaggatg gcattggccc caaacttgga 60
tttgttctca gtcccatcca actccagcat caggttgtcc agtttctctt gctccaccac 120
agagagacct gagctgatga gggctggcgc gatggtggag ttgatgtggt ccactgcctt 180
caggacacct ttgcctaagt aacgctgttt gtctccatcc ctcagctcca gggcctcata 240
gatgcccgta gaggctccac tgggcactgc agcccggaaa agacctttgg cagtatagag 300
atccacctcc actgtggggt tcccgcggga gtccaggatc tcccgggccc agatcttc 358

<210> 337
<211> 271
<212> DNA
<213> Homo sapiens

<220>
<221> misc_feature
<222> 17
<223> n = A,T,C or G

<400> 337
cacaaagcca ccagccnggg aaatcagaat ttacttgatg caactgactt gtaatagcca 60

gaaatcctgc ccagcatggg attcagaacc tggtctgcaa ccaaatccac cgtcaaagtt 120
catacaggat aaaacaaatt caattgcctt ttccacatta atagcatcaa gcttccccaa 180
caaagccaaa gttgccaccg cacaaaaaga gaatcttgtg tcaatttctc cctactttat 240
aaaagtagat ttttcacatc ccatgaagca g 271

<210> 338
<211> 326
<212> DNA
<213> Homo sapiens

<220>
<221> misc_feature
<222> 15, 17, 18
<223> n = A,T,C or G

<400> 338
ctgtgctccc gactngnnca tctcaggtac caccgactgc actgggcggg gccctctggg 60
gggaaaggct ccacggggca gggatacatc tcgaggccag tcatcctctg gaggcagccc 120
aatcaggtca aagattttgc ccaactggtc ggcttcagag tttccacaga agagaggctt 180
tcgacgaaac atctctgcaa agatacagcc aacactccac atgtccacag gtgttgcata 240
tgtggactgc agaagaactt cgggagctcg gtaccagagt gtaacaacca cgggtgtaag 300
tgccatctgg tagctgtaga ttctgg 326

<210> 339
<211> 260
<212> DNA
<213> Homo sapiens

<220>
<221> misc_feature
<222> 47, 54, 60, 69, 90, 91, 96, 113, 117, 119, 195
<223> n = A,T,C or G

<400> 339
ttcacctgag gactcatttc gtgccctttg ttgacttcaa gcaaagncct tcanggtctn 60
caaggacgnc acatttccac ttgcgaatgn nctcanggct catcttgaag aanaagnanc 120
ccaagtgctg gatcccagac tcgggggtaa ccttgtgggt aagagctcat ccagtttatg 180
ctttaggacg tccanctact cgggggagct ggaagcctgc gtggatgcgg ccctgctgga 240
cctcggccgc gaccacgcta 260

<210> 340
<211> 220
<212> DNA
<213> Homo sapiens

<220>
<221> misc_feature
<222> 15, 18
<223> n = A,T,C or G

<400> 340
ctggaagccc ggctnggnct ggcagcggaa ggagccaggc aggttcacgc agcggtgctg 60
gcagtagcgg tagcggcact cgtctatgtc cacacactcg ggcccgatct tgcggtaacc 120
atcagggcag gtgcactgat aggagccagg caagttatgg cagtcctggc tggggcgaca 180
gtcgtgcagg gcctgggcac actcgtccac atccacacag 220

<210> 341

<211> 384
<212> DNA
<213> Homo sapiens

<400> 341
ctgctaccag gggagcgaga gctgactatc ccagcctcgg ctaatgtatt ctacgccatg 60
gatggagctt cacacgattt cctcctgcgg cagcggcgaa ggtcctctac tgctacaccg 120
ggcgtcacca gtggcccgtc tgcctcagga actcctccga gtgagggagg agggggctcc 180
tttcccagga tcaaggccac agggaggaag attgcacggg cactgttctg aggaggaagc 240
cccgttggct tacagaagtc atggtgttca taccagatgt gggtagccat cctgaatggt 300
ggcaattata tcacattgag acagaaattc agaaagggag ccagccaccc tggggcagtg 360
aagtgccact ggtttaccag acag 384

<210> 342
<211> 245
<212> DNA
<213> Homo sapiens

<400> 342
ctggctaagc tcatcattgt tactggtggg caccatgtcc ttgaagcttc aggcaagcaa 60
tgtaaccaac aagaatgacc ccaagtccat caactctcga gtcttcattg gaaacctcaa 120
cacagctctg gtgaagaaat cagatgtgga gaccatcttc tctaagtatg gccgtgtggc 180
cggctgttct gtgcacaagg gctatgcctt tgttcagtac tccaatgagc gccatgcccg 240
ggcag 245

<210> 343
<211> 611
<212> DNA
<213> Homo sapiens

<400> 343
ccaaaaaaat caagatttaa ttttttattt tgcactgaaa aactaatcat aactgttaat 60
tctcagccat ctttgaagct tgaaagaaga gtctttggta ttttgtaaac gttagcagac 120
tttcctgcca gtgtcagaaa atcctattta tgaatcctgt cggtattcct tggtatctga 180
aaaaaatacc aaatagtacc atacatgagt tatttctaag tttgaaaaat aaaaagaaat 240
tgcatcacac taattacaaa atacaagttc tggaaaaaat atttttcttc attttaaaac 300
tttttttaac taataatggc tttgaaagaa gaggcttaat ttgggggtgg taactaaaat 360
caaaagaaat gattgacttg agggtctctg tttggtaaga atacatcatt agcttaaata 420
agcagcagaa ggttagtttt aattatgtag cttctgttaa tattaagtgt tttttgtctg 480
ttttacctca atttgaacag ataagtttgc ctgcatgctg gacatgcctc agaaccatga 540
atagcccgta ctagatcttg ggaacatgga tcttagagtc ctttggaata agttcttata 600
taaatacccc c 611

<210> 344
<211> 311
<212> DNA
<213> Homo sapiens

<220>
<221> misc_feature
<222> 1, 275, 284, 296, 297, 300
<223> n = A,T,C or G

<400> 344
nctcgaaaaa gcccaagaca gcagaagcag acacctccag tgaactagca aagaaaagca 60
aagaagtatt cagaaaagag atgtcccagt tcatcgtcca gtgcctgaac ccttaccgga 120
aacctgactg caaagtggga agaattacca caactgaaga ctttaaacat ctggctcgca 180

agctgactca cggtgttatg aataaggagc tgaagtactg taagaatcct gaggacctgg 240
agtgcaatga gaatgtgaaa cacaaaacca aggantacat taanaagtac atgcannaan 300
tttggggctt g 311

<210> 345
<211> 201
<212> DNA
<213> Homo sapiens

<400> 345
cacacggtca tcccgactgc caacctggag gcccaggccc tgtggaagga gccgggcagc 60
aatgtcacca tgagtgtgga tgctgagtgt gtgcccatgg tcagggacct tctcaggtac 120
ttctactccc gaaggattga catcaccctg tcgtcagtca agtgcttcca caagctggcc 180
tctgcctatg gggccaggca g 201

<210> 346
<211> 370
<212> DNA
<213> Homo sapiens

<400> 346
ctgctccagg gcgtggtgtg ccttcgtggc ctctgcctcc tccgaggagc caggctgtgt 60
tctcttcaga atgttctgga gcagcagttt gaggcgggtg atgcgttgga agggcagaat 120
cagaaaggac ttgagggaaa ggcgctggca gacggggtcg ctctccagct tctccaagac 180
ctcccggaaa ttgctgttgc tattcatcag gctctggaag gtgcgttcct gataggtctg 240
gttggtgaca taaggcaggt agacccggcg gaagtctggg gcgtggttca ggactacgtc 300
acatacttgg aaggagaaga tattgttctc aaagttctct tccaggtctg aaaggaacgt 360
ggcgctgacg 370

<210> 347
<211> 416
<212> DNA
<213> Homo sapiens

<220>
<221> misc_feature
<222> 416
<223> n = A,T,C or G

<400> 347
ctgttgtgct gtgtatggac gtgggcttta ccatgagtaa ctccattcct ggtatagaat 60
ccccatttga acaagcaaag aaggtgataa ccatgtttgt acagcgacag gtgtttgctg 120
agaacaagga tgagattgct ttagtcctgt ttggtacaga tggcactgac aatccccttt 180
ctggtgggga tcagtatcag aacatcacag tgcacagaca tctgatgcta ccagattttg 240
atttgctgga ggacattgaa agcaaaatcc aaccaggttc tcaacaggct gacttcctgg 300
atgcactaat cgtgagcatg gatgtgattc aacatgaaac aataggaaag aagtttggag 360
aagaggcata ttgaaatatt cactgacctc aagcagcccg attcagcaaa agtcan 416

<210> 348
<211> 351
<212> DNA
<213> Homo sapiens

<400> 348
gtacaggaga ggatggcagg tgcagagcgg gcactgagct ctgcaggtga aagggctcgg 60
cagttggatg ctctcctgga ggctctgaaa ttgaaacggg caggaaatag tctggcagcc 120
tctacagcag aagaaacggc aggcagtgcc cagggacgag caggagacag atgccttcct 180

cttgtctcaa ctgcaaagag gcgttccttc ctctttcact aatcctcctc agcacagacc 240
ctttacgggt gtcaggctgg gggacagtaa ggtctttccc ttcccacaag gccatatctc 300
aggctgtctc agtgggggga aaccttggac aatacccggg ctttcttggg c 351

<210> 349
<211> 207
<212> DNA
<213> Homo sapiens

<220>
<221> misc_feature
<222> 1
<223> n = A,T,C or G

<400> 349
nccgggacat ctccaccctc aacagtggca agaagagcct ggagactgaa cacaaggcct 60
tgaccagtga gattgcactg ctgcagtcca ggctgaagac agagggctct gatctgtgcg 120
acagagtgag cgaaatgcag aagctggatg cacaggtcaa ggagctggtg ctgaagtcgg 180
cggtggaggc tgagcgcctg gtggctg 207

<210> 350
<211> 323
<212> DNA
<213> Homo sapiens

<400> 350
ccatacaggg ctgttgccca ggccctagag gtcattcctc gtaccctgat ccagaactgt 60
ggggccagca ccatccgtct acttacctcc cttcgggcca agcacaccca ggagaactgt 120
gagacctggg gtgtaaatgg tgagacgggt actttggtgg acatgaagga actgggcata 180
tgggagccat tggctgtgaa gctgcagact tataagacag cagtggagac ggcagttctg 240
ctactgcgaa ttgatgacat cgtttcaggc cacgaaaaga aaggcgatga ccagagccgg 300
caaggcgggg ctcctgatgc tgg 323

<210> 351
<211> 353
<212> DNA
<213> Homo sapiens

<220>
<221> misc_feature
<222> 12, 25, 39, 42
<223> n = A,T,C or G

<400> 351
cgccgcatcc cntggtccct tccantccct tttcctttnt cngggaacgt gtatgcggtt 60
tgtttttgtt ttgtagggtt tttttccttc tccacctctc cctgtctctt ttgctccatg 120
ttgtccgttt ctgtggggtt aggtttatgt ttttaatcat ctgaggtcac gtctatttcc 180
tccggactcg cctgcttggt ggcgattctc caccggttaa tatggtgcgt cccttttttc 240
ttttgttgcg aatctgagcc ttcttcctcc agcttctgcc ttttgaactt tgttcttcgg 300
ttctgaaacc atacttttac ctgagtttcc gtgaggctga ggctgtgtgc caa 353

<210> 352
<211> 467
<212> DNA
<213> Homo sapiens

<400> 352

ctgcccacac tgatcacttg cgagatgtcc ttagggtaca agaacaggaa ttgaagtctg 60
aatttgagca gaacctgtct gagaaactct ctgaacaaga attacaattt cgtcgtctca 120
gtcaagagca agttgacaac tttactctgg atataaatac tgcctatgcc agactcagag 180
gaatcgaaca ggctgttcag agccatgcag ttgctgaaga ggaagccaga aaagcccacc 240
aactctggct ttcagtggag gcattaaagt acagcatgaa gacctcatct gcagaaacac 300
ctactatccc gctgggtagt gcagttgagg ccatcaaagc caactgttct gataatgaat 360
tcacccaagc tttaaccgca gctatccctc cagagtccct gacccgtggg gtgtacagtg 420
aagagaccct tagagcccgt ttctatgctg ttcaaaaact ggcccga 467

<210> 353
<211> 350
<212> DNA
<213> Homo sapiens

<400> 353
ctgctgcagc cacagtagtt cctcccatgg tgggtggccc tcctggtcct gctggcccag 60
gaaatctgtc cccaccagga acagcccctg gaaaacggcc ccgtcctcta ccaccttgtg 120
gaaatgctgc acgggaactg cctcctggag gaccagcttt accttcccca gacatttgtc 180
ctgattgtgt agttttcctg gactgcattt caaattgact caggaactgt ttattgcatg 240
gagttacaac aggattctga ccatgaagtt ctcttttagg taacagatcc attaactttt 300
ttgaagatgc ttcagatcca acaccaacaa gggcaaaccc ctttgactgg 350

<210> 354
<211> 351
<212> DNA
<213> Homo sapiens

<400> 354
atttagatga gatctgaggc atggagacat ggagacagta tacagactcc tagatttaag 60
ttttaggttt tttgcttttc taatcaccaa ttcttatata caatgtatat tttagactcg 120
agcagatgat catcttcatc ttaagtcatt ccttttgact gagtatggca ggattagagg 180
gaatggcagt atagatcaat gtctttttct gtaaagtata ggaaaaacca gagaggaaaa 240
aaagagctga caattggaag gtagtagaaa attgacgata atttcttctt aacaaataat 300
agttgtatat acaaggaggc tagtcaacca gattttattt gttgagggcg a 351

<210> 355
<211> 308
<212> DNA
<213> Homo sapiens

<400> 355
ttttggcgca agttttacag attttattaa agtcgaagct attggtcttg gaagatgaaa 60
atgcaaatgt tgatgaggtg gaattgaagc cagatacctt aataaaatta tatcttggtt 120
ataaaaataa gaaattaagg gttaacatca atgtgccaat gaaaaccgaa cagaagcagg 180
aacaagaaac cacacacaaa aacatcgagg aagaccgcaa actactgatt caggcggcca 240
tcgtgagaat catgaagatg aggaaggttc tgaaacacca gcagttactt ggcgaggtcc 300
tcactcag 308

<210> 356
<211> 207
<212> DNA
<213> Homo sapiens

<400> 356
ctgtcccaag tgctcccaga aggcaggatt ctgaagacca ctccagcgat atgttcaact 60
atgaagaata ctgcaccgcc aacgcagtca ctgggccttg ccgtgcatcc ttcccacgct 120
ggtactttga cgtggagagg aactcctgca ataacttcat ctatggaggc tgccggggca 180

ataagaacag ctaccgctct gaggagg                                          207

<210> 357
<211> 188
<212> DNA
<213> Homo sapiens

<220>
<221> misc_feature
<222> 25, 29
<223> n = A,T,C or G

<400> 357
tcgaccacgc cctcgtagcg catgngctnc aggacgatgc tcagagtgat gaacaccccg 60
gtgcggccca cgccagcact gcagtgcacc gtgataggcc catcctgtcc aaactgctcc 120
ttggtcttat gcacctgccc gatgaagtca atgaatccct cgcctgtctt gggcacgccc 180
tgctctgg                                                          188

<210> 358
<211> 291
<212> DNA
<213> Homo sapiens

<400> 358
ctgggagcat cggcaagcta ctgccttaaa atccgatctc cccgagtgca caatttctgt 60
cccttttaag ggttcacaac actaaagatt tcacatgaaa gggttgtgat tgatttgagc 120
aggcaggcgg tacgtgacag gggctgcatg caccggtggt cagagagaaa cagaacaggg 180
cagggaattt cacaatgttc ttctatacaa tggctggaat ctatgaataa catcagtttc 240
taagttatgg gttgattttt aactactggg tttaggccag gcaggcccag g          291

<210> 359
<211> 117
<212> DNA
<213> Homo sapiens

<220>
<221> misc_feature
<222> 79, 98, 100
<223> n = A,T,C or G

<400> 359
gccaccacac tccagcctgg gcaatacagc aagactgtct caaaaaaaaa aaaaaaaaaa 60
cccaaaaaaa ctcaaaaang taatgaatga tacccaangn gccttttcta gaaaaag    117

<210> 360
<211> 394
<212> DNA
<213> Homo sapiens

<400> 360
ctgttcctct ggggtggtcc agttctagag tgggagaaag ggagtcaggc gcattgggaa 60
tcgtggttcc agtctggttg cagaatctgc acatttgcca agaaattttc cctgtttgga 120
aagtttgccc cagctttccc gggcacacca ccttttgtcc caagtgtctg ccggtcgacc 180
aatctgcctg ccacacattg accaagccag acccggttca cccagctcga ggatcccagg 240
ttgaagagtg gccccttgag gccctggaaa gaccaatcac tggacttctt cccttgagag 300
tcagaggtca cccgtgattc tgcctgcacc ttatcattga tctgcagtga tttctgcaaa 360
tcaagagaaa ctctgcaggg cactcccctg tttc                             394

<210> 361
<211> 394
<212> DNA
<213> Homo sapiens

<220>
<221> misc_feature
<222> 28, 31
<223> n = A,T,C or G

<400> 361
ctgggcggat agcaccgggc atattttntt natggatgag gtctggcacc ctgagcagtc 60
cagcgaggac ttggtcttag ttgagcaatt tggctaggag gatagtatgc agcacggttc 120
tgagtctgtg ggatagctgc catgaagtaa cctgaaggag gtgctggctg gtaggggttg 180
attacagggt tgggaacagc tcgtacactt gccattctct gcatatactg gttagtgagg 240
tgagcctggc gctcttcttt gcgctgagct aaagctacat acaatggctt tgtggacctc 300
ggccgcgacc acgctaagcc gaattccagc acactggcgg ccgttactag tggatccgag 360
ctcggtacca agcttggcgt aatcatggtc atag 394

<210> 362
<211> 268
<212> DNA
<213> Homo sapiens

<400> 362
ctgcgcgtgg accagtcagc ttccgggtgt gactggagca gggcttgtcg tcttcttcag 60
agtcactttg caggggttgg tgaagctgct cccatccatg tacagctccc agtctactga 120
tgtttaagga tggtctcggt ggttaggccc actagaataa actgagtcca atacctctac 180
acagttatgt ttaactgggc tctctgacac cgggaggaag gtggcggggt ttaggtgttg 240
caaacttcaa tggttatgcg gggatgtt 268

<210> 363
<211> 323
<212> DNA
<213> Homo sapiens

<400> 363
ccttgacctt ttcagcaagt gggaaggtgt aatccgtctc cacagacaag gccaggactc 60
gtttgtaccc gttgatgata gaatggggta ctgatgcaac agttgggtag ccaatctgca 120
gacagacact ggcaacattg cggacaccct ccaggaagcg agaatgcaga gtttcctctg 180
tgatatcaag cacttcaggg ttgtagatgc tgccattgtc gaacacctgc tggatgacca 240
gcccaaagga gaagggggag atgttgagca tgttcagcag cgtggcttcg ctggctccca 300
ctttgtctcc agtcttgatc aga 323

<210> 364
<211> 393
<212> DNA
<213> Homo sapiens

<220>
<221> misc_feature
<222> 29
<223> n = A,T,C or G

<400> 364
ccaagctctc catcgtcccc gtgcgcagng gctactgggg gaacaagatc ggcaagcccc 60

acactgtccc ttgcaaggtg acaggccgct gcggctctgt gctggtacgc ctcatcactg 120
cacccagggg cactggcatc gtctccgcac ctgtgcctaa gaagctgctc atgatggctg 180
gcatcgatga ctgctacacc tcagcccggg gctgcactgc caccctgggc aacttcgcca 240
aggccacctt tgatgccatt tctaagacct acagctacct gacccccgac ctctggaagg 300
agactgtatt caccaagtct ccctatcagg agttcactga ccacctcgtc aagacccaca 360
ccagagtctc cgtgcagcgg actcaggctc cag 393

<210> 365
<211> 371
<212> DNA
<213> Homo sapiens

<400> 365
cctcctcaga gcggtagctg ttcttattgc cccggcagcc tccatagatg aagttattgc 60
aggagttcct ctccacgtca aagtaccagc gtgggaagga tgcacggcaa ggcccagtga 120
ctgcgttggc ggtgcagtat tcttcatagt tgaacatatc gctggagtgg tcttcagaat 180
cctgccttct gggagcactt gggacagagg aatccgctgc attcctgctg gtggacctcg 240
gccgcgacca cgctaagccg aattccagca cactggcggc cgttactagt ggatccgagc 300
tcggtaccaa gcttggcgta atcatggtca tagctgtttc ctgtgtgaaa ttgttatccg 360
ctcacaattc c 371

<210> 366
<211> 393
<212> DNA
<213> Homo sapiens

<400> 366
atttcttgcc agatgggagc tctttggtga agactccttt cgggaaaagt tttttggctt 60
cttcttcagg gatggttgga aggaccatca cactatcccc atccttccaa tcaactgggg 120
tggcaaccct tttttctgct gtcagctgga gagagatgac taccctgaga atctcatcaa 180
agttcctgcc agtggtagct gggtagagga tagacagctt cagcttctta tcaggaccaa 240
aaacaaacac cacacgagct gccacaggca tgcccttttc atccttctct gctggatcca 300
gcatgcccaa caggatggca agctcccgat tcctatcatc gatgatggga aaaggtaact 360
tttctgtggg ctcttcacaa ttgtaagcat tga 393

<210> 367
<211> 327
<212> DNA
<213> Homo sapiens

<220>
<221> misc_feature
<222> 34, 54, 55
<223> n = A,T,C or G

<400> 367
ccagctctgt ctcatacttg actctaaagt cttnagcagc aagacgggca ttgnnaatct 60
gcagaacgat gcgggcattg tccacagtat ttgcgaagat ctgagccctc aggtcctcga 120
tgatcttgaa gtaatggctc cagtctctga cctggggtcc cttcttctcc aagtgctccc 180
ggatttgctc ctccagcctc cggttctcgg tctccaggct cctcactctg tccaggtaag 240
aggccaggcg gtcgttcagg ctttgcatgg tctccttctc gttctggatg cctcccattc 300
ctgccagacc cccggctatc ccggtgg 327

<210> 368
<211> 306
<212> DNA
<213> Homo sapiens

<220>
<221> misc_feature
<222> 24
<223> n = A,T,C or G

<400> 368
ctggagaagg acttcagcag tttnaagaag tactgccaag tcatccgtgt cattgcccac 60
acccagatgc gcctgcttcc tctgcgccag aagaaggccc acctgatgga gatccaggtg 120
aacggaggca ctgtggccga gaagctggac tgggcccgcg agaggcttga gcagcaggta 180
cctgtgaacc aagtgtttgg gcaggatgag atgatcgacg tcatcggggt gaccaagggc 240
aaaggctaca aaggggtcac cagtcgttgg cacaccaaga agctgccccg caagacccac 300
cgagga 306

<210> 369
<211> 394
<212> DNA
<213> Homo sapiens

<400> 369
tcgacccaca ccggaacacg gagagctggg ccagcattgg cacttgatag gatttcccgt 60
cggctgccac gaaagtgcgt ttctttgtgt tctcgggttg gaaccgtgat ttccacagac 120
ccttgaaata cactgcgttg acgaggacca gtctggtgag cacaccatca ataagatctg 180
gggacagcag attgtcaatc atatccctgg tttcattttt aacccatgca ttgatggaat 240
cacaggcaga ggctggatcc tcaaagttca cattccggac ctcacactgg aacacatctt 300
tgttccttgt aacaaaaggc acttcaattt cagaggcatt cttaacaaac acggcgttag 360
ccactgtcac aatgtcttta ttcttcttgg agac 394

<210> 370
<211> 653
<212> DNA
<213> Homo sapiens

<400> 370
ccaccacacc caattccttg ctggtatcat ggcagccgcc acgtgccagg attaccggct 60
acatcatcaa gtatgagaag cctgggtctc ctcccagaga agtggtccct cggccccgcc 120
ctggtgtcac agaggctact attactggcc tggaaccggg aaccgaatat acaatttatg 180
tcattgccct gaagaataat cagaagagcg agcccctgat tggaaggaaa aagacagacg 240
agcttcccca actggtaacc cttccacacc ccaatcttca tggaccagag atcttggatg 300
ttccttccac agttcaaaag acccctttcg tcacccaccc tgggtatgac actggaaatg 360
gtattcagct tcctggcact tctggtcagc aacccagtgt tgggcaacaa atgatctttg 420
aggaacatgg ttttaggcgg accacaccgc ccacaacggc cacccccata aggcataggc 480
caagaccata cccgccgaat gtaggacaag aagctctctc tcagacaacc atctcatggg 540
ccccattcca ggacacttct gagtacatca tttcatgtca tcctgttggc actgatgaag 600
aacccttaca gttcagggtt cctggaactt ctaccagtgc cactctgaca gga 653

<210> 371
<211> 268
<212> DNA
<213> Homo sapiens

<400> 371
ctgcccagcc cccattggcg agtttgagaa ggtgtgcagc aatgacaaca agaccttcga 60
ctcttcctgc cacttctttg ccacaaagtg caccctggag ggcaccaaga agggccacaa 120
gctccacctg gactacatcg ggccttgcaa atacatcccc ccttgcctgg actctgagct 180
gaccgaattc cccctgcgca tgcgggactg gctcaagaac gtcctggtca ccctgtatga 240
gagggatgag gacaacaacc ttctgact 268

<210> 372
<211> 392
<212> DNA
<213> Homo sapiens

<400> 372
gctggtgccc ctggtgaacg tggacctcct ggattggcag gggccccagg acttagaggt 60
ggaactggtc cccctggtcc cgaaggagga aagggtgctg ctggtcctcc tgggccacct 120
ggtgctgctg gtactcctgg tctgcaagga atgcctggag aaagaggagg tcttggaagt 180
cctggtccaa agggtgacaa gggtgaacca ggcggtccag gtgctgatgg tgtcccaggg 240
aaagatggcc caaggggtcc tactggtcct attggtcctc ctggcccagc tggccagcct 300
ggagataagg gtgaaggtgg tgcccccgga cttccaggta tagctggacc tcgtggtagc 360
cctggtgaga gaggtgaaac ctcggccgcg ac 392

<210> 373
<211> 388
<212> DNA
<213> Homo sapiens

<220>
<221> misc_feature
<222> 30
<223> n = A,T,C or G

<400> 373
ccaagcgctc agatcggcaa ggggcaccan ttttgatctg cccagtgcac agccccacaa 60
ccaggtcagc gatgaaggta tcttcagtct cccccgaacg atgagacacc atgacgcccc 120
aaccattggc ctgggccagc ttgcacgcct gaagagactc ggtcacggag ccaatctggt 180
tgactttgag caggaggcag ttgcaggact tctcgttcac ggccttggcg atcctctttg 240
ggttggtcac tgtgagatca tcccccacta cctggattcc tgcactggct gtgaacttct 300
gccaagctcc ccagtcatcc tggtcaaagg gatcttcgat agacaccact gggtagtcct 360
tgatgaagga cttgtacagg tcagccag 388

<210> 374
<211> 393
<212> DNA
<213> Homo sapiens

<400> 374
ctgacgaccg cgtgaacccc tgcattgggg gtgtcatcct cttccatgag acactctacc 60
agaaggcgga tgatgggcgt cccttccccc aagttatcaa atccaagggc ggtgttgtgg 120
gcatcaaggt agacaagggc gtggtccccc tggcagggac aaatggcgag actaccaccc 180
aagggttgga tgggctgtct gagcgctgtg cccagtacaa gaaggacgga gctgacttcg 240
ccaagtggcg ttgtgtgctg aagattgggg aacacacccc ctcagccctc gccatcatgg 300
aaaatgccaa tgttctggcc cgttatgcca gtatctgcca gcagaatggc attgtgccca 360
tcgtggagcc tgagatcctc cctgatgggg acc 393

<210> 375
<211> 394
<212> DNA
<213> Homo sapiens

<220>
<221> misc_feature
<222> 30, 33
<223> n = A,T,C or G

<400> 375
ccacaaatgg cgtggtccat gtcatcaccn ttnttctgca gcctccagcc aacagacctc 60
aggaaagagg ggatgaactt gcagactctg cgcttgagat cttcaaacaa gcatcagcgt 120
tttccagggc ttcccagagg tctgtgcgac tagcccctgt ctatcaaaag ttattagaga 180
ggatgaagca ttagcttgaa gcactacagg aggaatgcac cacggcagct ctccgccaat 240
ttctctcaga tttccacaga gactgtttga atgttttcaa aaccaagtat cacactttaa 300
tgtacatggg ccgcaccata atgagatgtg agccttgtgc atgtggggga ggagggagag 360
agatgtactt tttaaatcat gttcccccta aaca 394

<210> 376
<211> 392
<212> DNA
<213> Homo sapiens

<220>
<221> misc_feature
<222> 30
<223> n = A,T,C or G

<400> 376
ctgcccagcc cccattggcg agtttgattn ggtgtgcagc aatgacaaca agaccttcga 60
ctcttcctgc cacttctttg ccacaaagtg caccctggag ggcaccaaga agggccacaa 120
gctccacctg gactacatcg ggccttgcaa atacatcccc ccttgcctgg actctgagct 180
gaccgaattc cccctgcgca tgcgggactg gctcaagaac gtcctggtca ccctgtatga 240
gagggatgag gacaacaacc ttctgactga gaagcagaag ctgcgggtga agaagatcca 300
tgagaatgag aagcgcctgg aggcaggaga ccaccccgtg gagctgctgg cccgggactt 360
cgagaagaac tataacatgt acatcttccc tg 392

<210> 377
<211> 292
<212> DNA
<213> Homo sapiens

<400> 377
caatgtttga tgcttaaccc ccccaatttc tgtgagatgg atggccagtg caagcgtgac 60
ttgaagtgtt gcatgggcat gtgtgggaaa tcctgcgttt cccctgtgaa agcttgattc 120
ctgccatatg gaggaggctc tggagtcctg ctctgtgtgg tccaggtcct ttccaccctg 180
agacttggct ccaccactga tatcctcctt tggggaaagg cttggcacac agcaggcttt 240
caagaagtgc cagttgatca atgaataaat aaacgagcct atttctcttt gc 292

<210> 378
<211> 395
<212> DNA
<213> Homo sapiens

<400> 378
ctgctgcttc agcgaagggt ttctggcata tccaatgata aggctgccaa agactgttcc 60
aataccagca ccagaaccag ccactcctac tgttgcagca cctgcaccaa taaatttggc 120
agcagtatca atgtctctgc tgattgcact ggtctgaaac tccctttgga ttagctgaga 180
cacaccattc tgggccctga ttttcctaag atagaactcc aactctttgc cctctagcac 240
atagccatct gctcggccac actgtcccgg ccttgaagcg atgcacgcaa gaagcttgcc 300
ctgctggaac tgctcctcca ggagactgct gattttggca ttctttttcc tttcatcata 360
tttcttctga atttttaga tcgtttttttg tttaa 395

<210> 379
<211> 223

<212> DNA
<213> Homo sapiens

<400> 379
ccagatgaaa tgctgccgca atggctgtgg gaaggtgtcc tgtgtcactc ccaatttctg 60
agctccagcc accaccaggc tgagcagtga ggagagaaag tttctgcctg gccctgcatc 120
tggttccagc ccacctgccc tccccttttt cgggactctg tattccctct tgggctgacc 180
acagcttctc cctttcccaa ccaataaagt aaccactttc agc 223

<210> 380
<211> 317
<212> DNA
<213> Homo sapiens

<220>
<221> misc_feature
<222> 30, 32
<223> n = A,T,C or G

<400> 380
tcgaccacag tattccaacc ctcctgtgcn tngagaagtg atggagggtg ctgacaacca 60
gggtgcagga gaacaaggta gaccagtgag gcagaatatg tatcggggat atagaccacg 120
attccgcagg ggccctcctc gccaaagaca gcctagagag gacggcaatg aagaagataa 180
agaaaatcaa ggagatgaga cccaaggtca gcagccacct caacgtcggt accgccgcaa 240
cttcaattac cgacgcagac gcccagaaaa ccctaaacca caagatggca aagagacaaa 300
agcagccgat ccaccag 317

<210> 381
<211> 392
<212> DNA
<213> Homo sapiens

<220>
<221> misc_feature
<222> 29, 30, 31
<223> n = A,T,C or G

<400> 381
cctgaaggaa gagctggcct acctgaatnn naaccatgag gaggaaatca gtacgctgag 60
gggccaagtg ggaggccagg tcagtgtgga ggtggattcc gctccgggca ccgatctcgc 120
caagatcctg agtgacatgc gaagccaata tgaggtcatg gccgagcaga accggaagga 180
tgctgaagcc tggttcacca gccggactga agaattgaac cgggaggtcg ctggccacac 240
ggagcagctc cagatgagca ggtccgaggt tactgacctg cggcgcaccc ttcagggtct 300
tgagattgag ctgcagtcac agacctcggc cgcgaccacg ctaagccgaa ttccagcaca 360
ctggcggccg ttactagtgg atccgagctc gg 392

<210> 382
<211> 234
<212> DNA
<213> Homo sapiens

<400> 382
cctcgatgtc taaatgagcg tggtaaagga tggtgcctgc tggggtctcg tagatacctc 60
gggacttcat tccaatgaag cggttctcca cgatgtcaat acggcccacg ccatgcttgc 120
ccgcgacttc gttcaggtac atgaagagct ccaaggaggt ctggtgggtg gtgccatcct 180
tgacgttggt caccttcaca gggacccctt ttttgaactc catctccaga atgt 234

<210> 383
<211> 396
<212> DNA
<213> Homo sapiens

<220>
<221> misc_feature
<222> 66
<223> n = A,T,C or G

<400> 383
ccttgacctt ttcagcaagt gggaaggtgt tttccgtctc cacagacaag gccaggactc 60
gtttgnaccc gttgatgata gaatggggta ctgatgcaac agttgggtag ccaatctgca 120
gacagacact ggcaacattg cggacaccca ggatttcaat ggtgcccctg gagattttag 180
tggtgatacc taaagcctgg aaaaaggagg tcttctcggg cccgagacca gtgttctggg 240
ctggcacagt gacttcacat ggggcaatgg caccagcacg ggcagcagac ctgcccgggc 300
ggccgctcga aagccgaatt ccagcacact ggcggccgtt actagtggat ccgagctcgg 360
taccaagctt ggcgtaatca tggtcatagc tgtttc 396

<210> 384
<211> 396
<212> DNA
<213> Homo sapiens

<400> 384
gctgaatagg cacagagggc acctgtacac cttcagacca gtctgcaacc tcaggctgag 60
tagcagtgaa ctcaggagcg ggagcagtcc attcaccctg aaattcctcc ttggtcactg 120
ccttctcagc agcagcctgc tcttcttttt caatctcttc aggatctctg tagaagtaca 180
gatcaggcat gacctcccat gggtgttcac gggaaatggt gccacgcatg cgcagaactt 240
cccgagccag catccaccac atcaaaccca ctgagtgagc tcccttgttg ttgcatggga 300
tggcaatgtc cacatagcgc agaggagaat ctgtgttaca cagcgcaatg gtaggtaggt 360
taacataaga tgcctccgtg agaggctggt ggtcag 396

<210> 385
<211> 2943
<212> DNA
<213> Homo sapiens

<400> 385
cagccaccgg agtggatgcc atctgcaccc accgccctga ccccacaggc cctgggctgg 60
acagagagca gctgtatttg gagctgagcc agctgaccca cagcatcact gagctgggcc 120
cctacaccct ggacagggac agtctctatg tcaatggttt cacacagcgg agctctgtgc 180
ccaccactag cattcctggg acccccacag tggacctggg aacatctggg actccagttt 240
ctaaacctgg tccctcggct gccagccctc tcctggtgct attcactctc aacttcacca 300
tcaccaacct gcggtatgag gagaacatgc agcaccctgg ctccaggaag ttcaacacca 360
cggagagggt ccttcagggc ctggtccctg ttcaagagca ccagtgttgg ccctctgtac 420
tctggctgca gactgacttt gctcaggcct gaaaaggatg ggacagccac tggagtggat 480
gccatctgca cccaccaccc tgaccccaaa agccctaggc tggacagaga gcagctgtat 540
tgggagctga gccagctgac ccacaatatc actgagctgg gcccctatgc cctggacaac 600
gacagcctct ttgtcaatgg tttcactcat cggagctctg tgtccaccac cagcactcct 660
gggaccccca cagtgtatct gggagcatct aagactccag cctcgatatt tggcccttca 720
gctgccagcc atctcctgat actattcacc ctcaacttca ccatcactaa cctgcggtat 780
gaggagaaca tgtggcctgg ctccaggaag ttcaacacta cagagagggt ccttcagggc 840
ctgctaaggc ccttgttcaa gaacaccagt gttggccctc tgtactctgg ctgcaggctg 900
accttgctca ggccagagaa agatggggaa gccaccggag tggatgccat ctgcacccac 960
cgccctgacc ccacaggccc tgggctggac agagagcagc tgtatttgga gctgagccag 1020
ctgacccaca gcatcactga gctgggcccc tacacactgg acagggacag tctctatgtc 1080

aatggtttca cccatcggag ctctgtaccc accaccagca ccggggtggt cagcgaggag 1140
ccattcacac tgaacttcac catcaacaac ctgcgctaca tggcggacat gggccaaccc 1200
ggctccctca agttcaacat cacagacaac gtcatgaagc acctgctcag tcctttgttc 1260
cagaggagca gcctgggtgc acggtacaca ggctgcaggg tcatcgcact aaggtctgtg 1320
aagaacggtg ctgagacacg ggtggacctc ctctgcacct acctgcagcc cctcagcggc 1380
ccaggtctgc ctatcaagca ggtgttccat gagctgagcc agcagaccca tggcatcacc 1440
cggctgggcc cctactctct ggacaaagac agcctctacc ttaacggtta caatgaacct 1500
ggtccagatg agcctcctac aactcccaag ccagccacca cattcctgcc tcctctgtca 1560
gaagccacaa cagccatggg gtaccacctg aagaccctca cactcaactt caccatctcc 1620
aatctccagt attcaccaga tatgggcaag ggctcagcta cattcaactc caccgagggg 1680
gtccttcagc acctgctcag acccttgttc cagaagagca gcatgggccc cttctacttg 1740
ggttgccaac tgatctccct caggcctgag aaggatgggg cagccactgg tgtggacacc 1800
acctgcacct accaccctga ccctgtgggc cccgggctgg acatacagca gctttactgg 1860
gagctgagtc agctgaccca tggtgtcacc caactgggct tctatgtcct ggacagggat 1920
agcctcttca tcaatggcta tgcaccccag aatttatcaa tccggggcga gtaccagata 1980
aatttccaca ttgtcaactg gaacctcagt aatccagacc ccacatcctc agagtacatc 2040
accctgctga gggacatcca ggacaaggtc accacactct acaaaggcag tcaactacat 2100
gacacattcc gcttctgcct ggtcaccaac ttgacgatgg actccgtgtt ggtcactgtc 2160
aaggcattgt tctcctccaa tttggacccc agcctggtgg agcaagtctt tctagataag 2220
accctgaatg cctcattcca ttggctgggc tccacctacc agttggtgga catccatgtg 2280
acagaaatgg agtcatcagt ttatcaacca acaagcagct ccagcaccca gcacttctac 2340
ctgaatttca ccatcaccaa cctaccatat tcccaggaca aagcccagcc aggcaccacc 2400
aattaccaga ggaacaaaag gaatattgag gatgcggcac cacaccgggg tggactccct 2460
gtgtaacttc tcgccactgg ctcggagagt agacagagtt gccatctatg aggaatttct 2520
gcggatgacc cggaatggta cccagctgca gaacttcacc ctggacagga gcagtgtcct 2580
tgtggatggg tattttccca acagaaatga gcccttaact gggaattctg accttccctt 2640
ctgggctgtc atcctcatcg gcttggcagg actcctggga ctcatcacat gcctgatctg 2700
cggtgtcctg gtgaccaccc gccggcggaa gaaggaagga gaatacaacg tccagcaaca 2760
gtgcccaggc tactaccagt cacacctaga cctggaggat ctgcaatgac tggaacttgc 2820
cggtgcctgg ggtgcctttc ccccagccag ggtccaaaga agcttggctg gggcagaaat 2880
aaaccatatt ggtcggaaaa aaaaaaaaaa aaaaaaaaaa aaaaaaaaaa aaaaaaaaaa 2940
aaa 2943

<210> 386
<211> 2608
<212> DNA
<213> Homo sapiens

<400> 386
gttcaagagc accagtgttg gccctctgta ctctggctgc agactgactt tgctcaggcc 60
tgaaaaggat gggacagcca ctggagtgga tgccatctgc acccaccacc ctgaccccaa 120
aagccctagg ctggacagag agcagctgta ttgggagctg agccagctga cccacaatat 180
cactgagctg ggcccctatg ccctggacaa cgacagcctc tttgtcaatg gtttcactca 240
tcggagctct gtgtccacca ccagcactcc tgggaccccc acagtgtatc tgggagcatc 300
taagactcca gcctcgatat ttggcccttc agctgccagc catctcctga tactattcac 360
cctcaacttc accatcacta acctgcggta tgaggagaac atgtggcctg gctccaggaa 420
gttcaacact acagagaggg tccttcaggg cctgctaagg cccttgttca agaacaccag 480
tgttggccct ctgtactctg gctgcaggct gaccttgctc aggccagaga aagatgggga 540
agccaccgga gtggatgcca tctgcaccca ccgccctgac cccacaggcc ctgggctgga 600
cagagagcag ctgtatttgg agctgagcca gctgacccac agcatcactg agctgggccc 660
ctacacactg gacagggaca gtctctatgt caatggtttc acccatcgga gctctgtacc 720
caccaccagc accggggtgg tcagcgagga gccattcaca ctgaacttca ccatcaacaa 780
cctgcgctac atggcggaca tgggccaacc cggctccctc aagttcaaca tcacagacaa 840
cgtcatgaag cacctgctca gtcctttgtt ccagaggagc agcctgggtg cacggtacac 900
aggctgcagg gtcatcgcac taaggtctgt gaagaacggt gctgagacac gggtggacct 960
cctctgcacc tacctgcagc ccctcagcgg cccaggtctg cctatcaagc aggtgttcca 1020
tgagctgagc cagcagaccc atggcatcac ccggctgggc ccctactctc tggacaaaga 1080

cagcctctac cttaacggtt acaatgaacc tggtccagat gagcctccta caactcccaa 1140
gccagccacc acattcctgc ctcctctgtc agaagccaca acagccatgg ggtaccacct 1200
gaagaccctc acactcaact tcaccatctc caatctccag tattcaccag atatgggcaa 1260
gggctcagct acattcaact ccaccgaggg ggtccttcag cacctgctca gacccttgtt 1320
ccagaagagc agcatgggcc ccttctactt gggttgccaa ctgatctccc tcaggcctga 1380
gaaggatggg gcagccactg gtgtggacac cacctgcacc taccaccctg accctgtggg 1440
ccccgggctg gacatacagc agctttactg ggagctgagt cagctgaccc atggtgtcac 1500
ccaactgggc ttctatgtcc tggacaggga tagcctcttc atcaatggct atgcacccca 1560
gaatttatca atccggggcg agtaccagat aaatttccac attgtcaact ggaacctcag 1620
taatccagac cccacatcct cagagtacat caccctgctg agggacatcc aggacaaggt 1680
caccacactc tacaaaggca gtcaactaca tgacacattc cgcttctgcc tggtcaccaa 1740
cttgacgatg gactccgtgt tggtcactgt caaggcattg ttctcctcca atttggaccc 1800
cagcctggtg gagcaagtct ttctagataa gaccctgaat gcctcattcc attggctggg 1860
ctccacctac cagttggtgg acatccatgt gacagaaatg gagtcatcag tttatcaacc 1920
aacaagcagc tccagcaccc agcacttcta cctgaatttc accatcacca acctaccata 1980
ttcccaggac aaagcccagc caggcaccac caattaccag aggaacaaaa ggaatattga 2040
ggatgcgctc aaccaactct tccgaaacag cagcatcaag agttattttt ctgactgtca 2100
agtttcaaca ttcaggtctg tccccaacag gcaccacacc ggggtggact ccctgtgtaa 2160
cttctcgcca ctggctcgga gagtagacag agttgccatc tatgaggaat ttctgcggat 2220
gacccggaat ggtacccagc tgcagaactt caccctggac aggagcagtg tccttgtgga 2280
tgggtatttt cccaacagaa atgagccctt aactgggaat tctgaccttc ccttctgggc 2340
tgtcatcctc atcggcttgg caggactcct gggactcatc acatgcctga tctgcggtgt 2400
cctggtgacc acccgccggc ggaagaagga aggagaatac aacgtccagc aacagtgccc 2460
aggctactac cagtcacacc tagacctgga ggatctgcaa tgactggaac ttgccggtgc 2520
ctggggtgcc tttcccccag ccagggtcca aagaagcttg gctggggcag aaataaacca 2580
tattggtcgg acacaaaaaa aaaaaaaa 2608

<210> 387
<211> 1761
<212> DNA
<213> Homo sapiens

<400> 387
ctgaacttca ccatcaacaa cctgcgctac atggcggaca tgggccaacc cggctccctc 60
aagttcaaca tcacagacaa cgtcatgaag cacctgctca gtcctttgtt ccagaggagc 120
agcctgggtg cacggtacac aggctgcagg gtcatcgcac taaggtctgt gaagaacggt 180
gctgagacac gggtggacct cctctgcagg taggtgcaga ggaggtccac ggcatcaccc 240
ggctgggccc ctactctctg gacaaagaca gcctctacct taacgctccc aagccagcca 300
ccacattcct gcctcctctg tcagaagcca caacagccat ggggtaccac ctgaagaccc 360
tcacactcaa cttcaccatc tccaatctcc agtattcacc agatatgggc aagggctcag 420
ctacattcaa ctccaccgag ggggtccttc agcacctgct cagacccttg ttccagaaga 480
gcagcatggg ccccttctac ttgggttgcc aactgatctc cctcaggcct gagaaggatg 540
gggcagccac tggtgtggac accacctgca cctaccaccc tgaccctgtg ggccccgggc 600
tggacataca gcagctttac tgggagctga gtcagctgac ccatggtgtc acccaactgg 660
gcttctatgt cctggacagg gatagcctct tcatcaatgg ctatgcaccc cagaatttat 720
caatccgggg cgagtaccag ataaatttcc acattgtcaa ctggaacctc agtaatccag 780
accccacatc ctcagagtac atcaccctgc tgagggacat ccaggacaag gtcaccacac 840
tctacaaagg cagtcaacta catgacacat tccgcttctg cctggtcacc aacttgacga 900
tggactccgt gttggtcact gtcaaggcat tgttctcctc caatttggac cccagcctgg 960
tggagcaagt ctttctagat aagaccctga atgcctcatt ccattggctg ggctccacct 1020
accagttggt ggacatccat gtgacagaaa tggagtcatc agtttatcaa ccaacaagca 1080
gctccagcac ccagcacttc tacctgaatt tcaccatcac caacctacca tattcccagg 1140
acaaagccca gccaggcacc accaattacc agaggaacaa aaggaatatt gaggatgcgc 1200
tcaaccaact cttccgaaac agcagcatca agagttattt ttctgactgt caagtttcaa 1260
cattcaggtc tgtccccaac aggcaccaca ccggggtgga ctccctgtgt aacttctcgc 1320
cactggctcg gagagtagac agagttgcca tctatgagga atttctgcgg atgacccgga 1380
atggtaccca gctgcagaac ttcaccctgg acaggagcag tgtccttgtg gatgggtatt 1440

ttcccaacag aaatgagccc ttaactggga attctgacct tcccttctgg gctgtcatcc 1500
tcatcggctt ggcaggactc ctgggactca tcacatgcct gatctgcggt gtcctggtga 1560
ccacccgccg gcggaagaag gaaggagaat acaacgtcca gcaacagtgc ccaggctact 1620
accagtcaca cctagacctg gaggatctgc aatgactgga acttgccggt gcctggggtg 1680
cctttccccc agccagggtc caaagaagct tggctggggc agaaataaac catattggtc 1740
ggacacaaaa aaaaaaaaaa a 1761

<210> 388
<211> 772
<212> PRT
<213> Homo sapiens

<400> 388
Met Ser Met Val Ser His Ser Gly Ala Leu Cys Pro Pro Leu Ala Phe
1               5                   10                  15
Leu Gly Pro Pro Gln Trp Thr Trp Glu His Leu Gly Leu Gln Phe Leu
            20                  25                  30
Asn Leu Val Pro Arg Leu Pro Ala Leu Ser Trp Cys Tyr Ser Leu Ser
        35                  40                  45
Thr Ser Pro Ser Pro Thr Cys Gly Met Arg Arg Thr Cys Ser Thr Leu
    50                  55                  60
Ala Pro Gly Ser Ser Thr Pro Arg Arg Gly Ser Phe Arg Ala Trp Ser
65                  70                  75                  80
Leu Phe Lys Ser Thr Ser Val Gly Pro Leu Tyr Ser Gly Cys Arg Leu
                85                  90                  95
Thr Leu Leu Arg Pro Glu Lys Asp Gly Thr Ala Thr Gly Val Asp Ala
            100                 105                 110
Ile Cys Thr His His Pro Asp Pro Lys Ser Pro Arg Leu Asp Arg Glu
        115                 120                 125
Gln Leu Tyr Trp Glu Leu Ser Gln Leu Thr His Asn Ile Thr Glu Leu
    130                 135                 140
Gly Pro Tyr Ala Leu Asp Asn Asp Ser Leu Phe Val Asn Gly Phe Thr
145                 150                 155                 160
His Arg Ser Ser Val Ser Thr Thr Ser Thr Pro Gly Thr Pro Thr Val
                165                 170                 175
Tyr Leu Gly Ala Ser Lys Thr Pro Ala Ser Ile Phe Gly Pro Ser Ala
            180                 185                 190
Ala Ser His Leu Leu Ile Leu Phe Thr Leu Asn Phe Thr Ile Thr Asn
        195                 200                 205
Leu Arg Tyr Glu Glu Asn Met Trp Pro Gly Ser Arg Lys Phe Asn Thr
    210                 215                 220
Thr Glu Arg Val Leu Gln Gly Leu Leu Arg Pro Leu Phe Lys Asn Thr
225                 230                 235                 240
Ser Val Gly Pro Leu Tyr Ser Gly Cys Arg Leu Thr Leu Leu Arg Pro
                245                 250                 255
Glu Lys Asp Gly Glu Ala Thr Gly Val Asp Ala Ile Cys Thr His Arg
            260                 265                 270
Pro Asp Pro Thr Gly Pro Gly Leu Asp Arg Glu Gln Leu Tyr Leu Glu
        275                 280                 285
Leu Ser Gln Leu Thr His Ser Ile Thr Glu Leu Gly Pro Tyr Thr Leu
    290                 295                 300
Asp Arg Asp Ser Leu Tyr Val Asn Gly Phe Thr His Arg Ser Ser Val
305                 310                 315                 320
Pro Thr Thr Ser Thr Gly Val Val Ser Glu Glu Pro Phe Thr Leu Asn
                325                 330                 335
Phe Thr Ile Asn Asn Leu Arg Tyr Met Ala Asp Met Gly Gln Pro Gly
            340                 345                 350

Ser Leu Lys Phe Asn Ile Thr Asp Asn Val Met Lys His Leu Leu Ser
        355                 360                 365
Pro Leu Phe Gln Arg Ser Ser Leu Gly Ala Arg Tyr Thr Gly Cys Arg
    370                 375                 380
Val Ile Ala Leu Arg Ser Val Lys Asn Gly Ala Glu Thr Arg Val Asp
385                 390                 395                 400
Leu Leu Cys Thr Tyr Leu Gln Pro Leu Ser Gly Pro Gly Leu Pro Ile
                405                 410                 415
Lys Gln Val Phe His Glu Leu Ser Gln Gln Thr His Gly Ile Thr Arg
            420                 425                 430
Leu Gly Pro Tyr Ser Leu Asp Lys Asp Ser Leu Tyr Leu Asn Gly Tyr
        435                 440                 445
Asn Glu Pro Gly Pro Asp Glu Pro Pro Thr Thr Pro Lys Pro Ala Thr
    450                 455                 460
Thr Phe Leu Pro Pro Leu Ser Glu Ala Thr Thr Ala Met Gly Tyr His
465                 470                 475                 480
Leu Lys Thr Leu Thr Leu Asn Phe Thr Ile Ser Asn Leu Gln Tyr Ser
                485                 490                 495
Pro Asp Met Gly Lys Gly Ser Ala Thr Phe Asn Ser Thr Glu Gly Val
            500                 505                 510
Leu Gln His Leu Leu Arg Pro Leu Phe Gln Lys Ser Ser Met Gly Pro
        515                 520                 525
Phe Tyr Leu Gly Cys Gln Leu Ile Ser Leu Arg Pro Glu Lys Asp Gly
    530                 535                 540
Ala Ala Thr Gly Val Asp Thr Thr Cys Thr Tyr His Pro Asp Pro Val
545                 550                 555                 560
Gly Pro Gly Leu Asp Ile Gln Gln Leu Tyr Trp Glu Leu Ser Gln Leu
                565                 570                 575
Thr His Gly Val Thr Gln Leu Gly Phe Tyr Val Leu Asp Arg Asp Ser
            580                 585                 590
Leu Phe Ile Asn Gly Tyr Ala Pro Gln Asn Leu Ser Ile Arg Gly Glu
        595                 600                 605
Tyr Gln Ile Asn Phe His Ile Val Asn Trp Asn Leu Ser Asn Pro Asp
    610                 615                 620
Pro Thr Ser Ser Glu Tyr Ile Thr Leu Leu Arg Asp Ile Gln Asp Lys
625                 630                 635                 640
Val Thr Thr Leu Tyr Lys Gly Ser Gln Leu His Asp Thr Phe Arg Phe
                645                 650                 655
Cys Leu Val Thr Asn Leu Thr Met Asp Ser Val Leu Val Thr Val Lys
            660                 665                 670
Ala Leu Phe Ser Ser Asn Leu Asp Pro Ser Leu Val Glu Gln Val Phe
        675                 680                 685
Leu Asp Lys Thr Leu Asn Ala Ser Phe His Trp Leu Gly Ser Thr Tyr
    690                 695                 700
Gln Leu Val Asp Ile His Val Thr Glu Met Glu Ser Ser Val Tyr Gln
705                 710                 715                 720
Pro Thr Ser Ser Ser Ser Thr Gln His Phe Tyr Leu Asn Phe Thr Ile
                725                 730                 735
Thr Asn Leu Pro Tyr Ser Gln Asp Lys Ala Gln Pro Gly Thr Thr Asn
            740                 745                 750
Tyr Gln Arg Asn Lys Arg Asn Ile Glu Asp Ala Ala Pro His Arg Gly
        755                 760                 765
Gly Leu Pro Val
    770

<210> 389

<211> 833
<212> PRT
<213> Homo sapiens

<400> 389
Phe Lys Ser Thr Ser Val Gly Pro Leu Tyr Ser Gly Cys Arg Leu Thr
1               5                   10                  15
Leu Leu Arg Pro Glu Lys Asp Gly Thr Ala Thr Gly Val Asp Ala Ile
            20                  25                  30
Cys Thr His His Pro Asp Pro Lys Ser Pro Arg Leu Asp Arg Glu Gln
        35                  40                  45
Leu Tyr Trp Glu Leu Ser Gln Leu Thr His Asn Ile Thr Glu Leu Gly
    50                  55                  60
Pro Tyr Ala Leu Asp Asn Asp Ser Leu Phe Val Asn Gly Phe Thr His
65                  70                  75                  80
Arg Ser Ser Val Ser Thr Thr Ser Thr Pro Gly Thr Pro Thr Val Tyr
                85                  90                  95
Leu Gly Ala Ser Lys Thr Pro Ala Ser Ile Phe Gly Pro Ser Ala Ala
            100                 105                 110
Ser His Leu Leu Ile Leu Phe Thr Leu Asn Phe Thr Ile Thr Asn Leu
        115                 120                 125
Arg Tyr Glu Glu Asn Met Trp Pro Gly Ser Arg Lys Phe Asn Thr Thr
    130                 135                 140
Glu Arg Val Leu Gln Gly Leu Leu Arg Pro Leu Phe Lys Asn Thr Ser
145                 150                 155                 160
Val Gly Pro Leu Tyr Ser Gly Cys Arg Leu Thr Leu Leu Arg Pro Glu
                165                 170                 175
Lys Asp Gly Glu Ala Thr Gly Val Asp Ala Ile Cys Thr His Arg Pro
            180                 185                 190
Asp Pro Thr Gly Pro Gly Leu Asp Arg Glu Gln Leu Tyr Leu Glu Leu
        195                 200                 205
Ser Gln Leu Thr His Ser Ile Thr Glu Leu Gly Pro Tyr Thr Leu Asp
    210                 215                 220
Arg Asp Ser Leu Tyr Val Asn Gly Phe Thr His Arg Ser Ser Val Pro
225                 230                 235                 240
Thr Thr Ser Thr Gly Val Val Ser Glu Glu Pro Phe Thr Leu Asn Phe
                245                 250                 255
Thr Ile Asn Asn Leu Arg Tyr Met Ala Asp Met Gly Gln Pro Gly Ser
            260                 265                 270
Leu Lys Phe Asn Ile Thr Asp Asn Val Met Lys His Leu Leu Ser Pro
        275                 280                 285
Leu Phe Gln Arg Ser Ser Leu Gly Ala Arg Tyr Thr Gly Cys Arg Val
    290                 295                 300
Ile Ala Leu Arg Ser Val Lys Asn Gly Ala Glu Thr Arg Val Asp Leu
305                 310                 315                 320
Leu Cys Thr Tyr Leu Gln Pro Leu Ser Gly Pro Gly Leu Pro Ile Lys
                325                 330                 335
Gln Val Phe His Glu Leu Ser Gln Gln Thr His Gly Ile Thr Arg Leu
            340                 345                 350
Gly Pro Tyr Ser Leu Asp Lys Asp Ser Leu Tyr Leu Asn Gly Tyr Asn
        355                 360                 365
Glu Pro Gly Pro Asp Glu Pro Pro Thr Thr Pro Lys Pro Ala Thr Thr
    370                 375                 380
Phe Leu Pro Pro Leu Ser Glu Ala Thr Thr Ala Met Gly Tyr His Leu
385                 390                 395                 400
Lys Thr Leu Thr Leu Asn Phe Thr Ile Ser Asn Leu Gln Tyr Ser Pro
                405                 410                 415

Asp Met Gly Lys Gly Ser Ala Thr Phe Asn Ser Thr Glu Gly Val Leu
            420                 425                 430
Gln His Leu Leu Arg Pro Leu Phe Gln Lys Ser Ser Met Gly Pro Phe
        435                 440                 445
Tyr Leu Gly Cys Gln Leu Ile Ser Leu Arg Pro Glu Lys Asp Gly Ala
    450                 455                 460
Ala Thr Gly Val Asp Thr Thr Cys Thr Tyr His Pro Asp Pro Val Gly
465                 470                 475                 480
Pro Gly Leu Asp Ile Gln Gln Leu Tyr Trp Glu Leu Ser Gln Leu Thr
                485                 490                 495
His Gly Val Thr Gln Leu Gly Phe Tyr Val Leu Asp Arg Asp Ser Leu
            500                 505                 510
Phe Ile Asn Gly Tyr Ala Pro Gln Asn Leu Ser Ile Arg Gly Glu Tyr
        515                 520                 525
Gln Ile Asn Phe His Ile Val Asn Trp Asn Leu Ser Asn Pro Asp Pro
    530                 535                 540
Thr Ser Ser Glu Tyr Ile Thr Leu Leu Arg Asp Ile Gln Asp Lys Val
545                 550                 555                 560
Thr Thr Leu Tyr Lys Gly Ser Gln Leu His Asp Thr Phe Arg Phe Cys
                565                 570                 575
Leu Val Thr Asn Leu Thr Met Asp Ser Val Leu Val Thr Val Lys Ala
            580                 585                 590
Leu Phe Ser Ser Asn Leu Asp Pro Ser Leu Val Glu Gln Val Phe Leu
        595                 600                 605
Asp Lys Thr Leu Asn Ala Ser Phe His Trp Leu Gly Ser Thr Tyr Gln
    610                 615                 620
Leu Val Asp Ile His Val Thr Glu Met Glu Ser Ser Val Tyr Gln Pro
625                 630                 635                 640
Thr Ser Ser Ser Ser Thr Gln His Phe Tyr Leu Asn Phe Thr Ile Thr
                645                 650                 655
Asn Leu Pro Tyr Ser Gln Asp Lys Ala Gln Pro Gly Thr Thr Asn Tyr
            660                 665                 670
Gln Arg Asn Lys Arg Asn Ile Glu Asp Ala Leu Asn Gln Leu Phe Arg
        675                 680                 685
Asn Ser Ser Ile Lys Ser Tyr Phe Ser Asp Cys Gln Val Ser Thr Phe
    690                 695                 700
Arg Ser Val Pro Asn Arg His His Thr Gly Val Asp Ser Leu Cys Asn
705                 710                 715                 720
Phe Ser Pro Leu Ala Arg Arg Val Asp Arg Val Ala Ile Tyr Glu Glu
                725                 730                 735
Phe Leu Arg Met Thr Arg Asn Gly Thr Gln Leu Gln Asn Phe Thr Leu
            740                 745                 750
Asp Arg Ser Ser Val Leu Val Asp Gly Tyr Phe Pro Asn Arg Asn Glu
        755                 760                 765
Pro Leu Thr Gly Asn Ser Asp Leu Pro Phe Trp Ala Val Ile Leu Ile
    770                 775                 780
Gly Leu Ala Gly Leu Leu Gly Leu Ile Thr Cys Leu Ile Cys Gly Val
785                 790                 795                 800
Leu Val Thr Thr Arg Arg Arg Lys Lys Glu Gly Glu Tyr Asn Val Gln
                805                 810                 815
Gln Gln Cys Pro Gly Tyr Tyr Gln Ser His Leu Asp Leu Glu Asp Leu
            820                 825                 830
Gln

<210> 390

<211> 438
<212> PRT
<213> Homo sapiens

<400> 390
Met Gly Tyr His Leu Lys Thr Leu Thr Leu Asn Phe Thr Ile Ser Asn
1               5                   10                  15
Leu Gln Tyr Ser Pro Asp Met Gly Lys Gly Ser Ala Thr Phe Asn Ser
            20                  25                  30
Thr Glu Gly Val Leu Gln His Leu Leu Arg Pro Leu Phe Gln Lys Ser
        35                  40                  45
Ser Met Gly Pro Phe Tyr Leu Gly Cys Gln Leu Ile Ser Leu Arg Pro
    50                  55                  60
Glu Lys Asp Gly Ala Ala Thr Gly Val Asp Thr Thr Cys Thr Tyr His
65                  70                  75                  80
Pro Asp Pro Val Gly Pro Gly Leu Asp Ile Gln Gln Leu Tyr Trp Glu
                85                  90                  95
Leu Ser Gln Leu Thr His Gly Val Thr Gln Leu Gly Phe Tyr Val Leu
            100                 105                 110
Asp Arg Asp Ser Leu Phe Ile Asn Gly Tyr Ala Pro Gln Asn Leu Ser
        115                 120                 125
Ile Arg Gly Glu Tyr Gln Ile Asn Phe His Ile Val Asn Trp Asn Leu
    130                 135                 140
Ser Asn Pro Asp Pro Thr Ser Ser Glu Tyr Ile Thr Leu Leu Arg Asp
145                 150                 155                 160
Ile Gln Asp Lys Val Thr Thr Leu Tyr Lys Gly Ser Gln Leu His Asp
                165                 170                 175
Thr Phe Arg Phe Cys Leu Val Thr Asn Leu Thr Met Asp Ser Val Leu
            180                 185                 190
Val Thr Val Lys Ala Leu Phe Ser Ser Asn Leu Asp Pro Ser Leu Val
        195                 200                 205
Glu Gln Val Phe Leu Asp Lys Thr Leu Asn Ala Ser Phe His Trp Leu
    210                 215                 220
Gly Ser Thr Tyr Gln Leu Val Asp Ile His Val Thr Glu Met Glu Ser
225                 230                 235                 240
Ser Val Tyr Gln Pro Thr Ser Ser Ser Ser Thr Gln His Phe Tyr Leu
                245                 250                 255
Asn Phe Thr Ile Thr Asn Leu Pro Tyr Ser Gln Asp Lys Ala Gln Pro
            260                 265                 270
Gly Thr Thr Asn Tyr Gln Arg Asn Lys Arg Asn Ile Glu Asp Ala Leu
        275                 280                 285
Asn Gln Leu Phe Arg Asn Ser Ser Ile Lys Ser Tyr Phe Ser Asp Cys
    290                 295                 300
Gln Val Ser Thr Phe Arg Ser Val Pro Asn Arg His His Thr Gly Val
305                 310                 315                 320
Asp Ser Leu Cys Asn Phe Ser Pro Leu Ala Arg Arg Val Asp Arg Val
                325                 330                 335
Ala Ile Tyr Glu Glu Phe Leu Arg Met Thr Arg Asn Gly Thr Gln Leu
            340                 345                 350
Gln Asn Phe Thr Leu Asp Arg Ser Ser Val Leu Val Asp Gly Tyr Phe
        355                 360                 365
Pro Asn Arg Asn Glu Pro Leu Thr Gly Asn Ser Asp Leu Pro Phe Trp
    370                 375                 380
Ala Val Ile Leu Ile Gly Leu Ala Gly Leu Leu Gly Leu Ile Thr Cys
385                 390                 395                 400
Leu Ile Cys Gly Val Leu Val Thr Thr Arg Arg Arg Lys Lys Glu Gly
                405                 410                 415

Glu Tyr Asn Val Gln Gln Gln Cys Pro Gly Tyr Tyr Gln Ser His Leu
            420                 425                 430
Asp Leu Glu Asp Leu Gln
        435

<210> 391
<211> 2627
<212> DNA
<213> Homo sapiens

<400> 391
ccacgcgtcc gcccacgcgt ccggaaggca gcggcagctc cactcagcca gtacccagat 60
acgctgggaa ccttccccag ccatggcttc cctggggcag atcctcttct ggagcataat 120
tagcatcatc attattctgg ctggagcaat tgcactcatc attggctttg gtatttcagg 180
gagacactcc atcacagtca ctactgtcgc ctcagctggg aacattgggg aggatggaat 240
cctgagctgc acttttgaac ctgacatcaa actttctgat atcgtgatac aatggctgaa 300
ggaaggtgtt ttaggcttgg tccatgagtt caaagaaggc aaagatgagc tgtcggagca 360
ggatgaaatg ttcagaggcc ggacagcagt gtttgctgat caagtgatag ttggcaatgc 420
ctctttgcgg ctgaaaaacg tgcaactcac agatgctggc acctacaaat gttatatcat 480
cacttctaaa ggcaagggga atgctaacct tgagtataaa actggagcct tcagcatgcc 540
ggaagtgaat gtggactata atgccagctc agagaccttg cggtgtgagg ctccccgatg 600
gttcccccag cccacagtgg tctgggcatc ccaagttgac cagggagcca acttctcgga 660
agtctccaat accagctttg agctgaactc tgagaatgtg accatgaagg ttgtgtctgt 720
gctctacaat gttacgatca acaacacata ctcctgtatg attgaaaatg acattgccaa 780
agcaacaggg gatatcaaag tgacagaatc ggagatcaaa aggcggagtc acctacagct 840
gctaaactca aaggcttctc tgtgtgtctc ttctttcttt gccatcagct gggcacttct 900
gcctctcagc ccttacctga tgctaaaata atgtgccttg gccacaaaaa agcatgcaaa 960
gtcattgtta caacagggat ctacagaact atttcaccac cagatatgac ctagttttat 1020
atttctggga ggaaatgaat tcatatctag aagtctggag tgagcaaaca agagcaagaa 1080
acaaaaagaa gccaaaagca gaaggctcca atatgaacaa gataaatcta tcttcaaaga 1140
catattagaa gttgggaaaa taattcatgt gaactagaca agtgtgttaa gagtgataag 1200
taaaatgcac gtggagacaa gtgcatcccc agatctcagg gacctccccc tgcctgtcac 1260
ctggggagtg agaggacagg atagtgcatg ttctttgtct ctgaattttt agttatatgt 1320
gctgtaatgt tgctctgagg aagcccctgg aaagtctatc ccaacatatc cacatcttat 1380
attccacaaa ttaagctgta gtatgtaccc taagacgctg ctaattgact gccacttcgc 1440
aactcagggg cggctgcatt ttagtaatgg gtcaaatgat tcacttttta tgatgcttcc 1500
aaaggtgcct tggcttctct tcccaactga caaatgccaa agttgagaaa aatgatcata 1560
attttagcat aaacagagca gtcggcgaca ccgattttat aaataaactg agcaccttct 1620
ttttaaacaa acaaatgcgg gtttatttct cagatgatgt tcatccgtga atggtccagg 1680
gaaggacctt tcaccttgac tatatggcat tatgtcatca caagctctga ggcttctcct 1740
ttccatcctg cgtggacagc taagacctca gttttcaata gcatctagag cagtgggact 1800
cagctggggt gatttcgccc cccatctccg ggggaatgtc tgaagacaat tttggttacc 1860
tcaatgaggg agtggaggag gatacagtgc tactaccaac tagtggataa aggccaggga 1920
tgctgctcaa cctcctacca tgtacaggac gtctccccat tacaactacc caatccgaag 1980
tgtcaactgt gtcaggacta agaaaccctg gttttgagta gaaaagggcc tggaaagagg 2040
ggagccaaca aatctgtctg cttcctcaca ttagtcattg gcaaataagc attctgtctc 2100
tttggctgct gcctcagcac agagagccag aactctatcg ggcaccagga taacatctct 2160
cagtgaacag agttgacaag gcctatggga aatgcctgat gggattatct tcagcttgtt 2220
gagcttctaa gtttctttcc cttcattcta ccctgcaagc caagttctgt aagagaaatg 2280
cctgagttct agctcaggtt ttcttactct gaatttagat ctccagaccc ttcctggcca 2340
caattcaaat taaggcaaca aacatatacc ttccatgaag cacacacaga cttttgaaag 2400
caaggacaat gactgcttga attgaggcct tgaggaatga agctttgaag gaaaagaata 2460
ctttgtttcc agcccccttc ccacactctt catgtgttaa ccactgcctt cctggacctt 2520
ggagccacgg tgactgtatt acatgttgtt atagaaaact gattttagag ttctgatcgt 2580
tcaagagaat gattaaatat acatttccta caccaaaaaa aaaaaaa 2627

<210> 392
<211> 309
<212> PRT
<213> Homo sapiens

<400> 392
His Ala Ser Ala His Ala Ser Gly Arg Gln Arg Gln Leu His Ser Ala
1               5                   10                  15
Ser Thr Gln Ile Arg Trp Glu Pro Ser Pro Ala Met Ala Ser Leu Gly
            20                  25                  30
Gln Ile Leu Phe Trp Ser Ile Ile Ser Ile Ile Ile Ile Leu Ala Gly
        35                  40                  45
Ala Ile Ala Leu Ile Ile Gly Phe Gly Ile Ser Gly Arg His Ser Ile
    50                  55                  60
Thr Val Thr Thr Val Ala Ser Ala Gly Asn Ile Gly Glu Asp Gly Ile
65                  70                  75                  80
Leu Ser Cys Thr Phe Glu Pro Asp Ile Lys Leu Ser Asp Ile Val Ile
                85                  90                  95
Gln Trp Leu Lys Glu Gly Val Leu Gly Leu Val His Glu Phe Lys Glu
            100                 105                 110
Gly Lys Asp Glu Leu Ser Glu Gln Asp Glu Met Phe Arg Gly Arg Thr
        115                 120                 125
Ala Val Phe Ala Asp Gln Val Ile Val Gly Asn Ala Ser Leu Arg Leu
    130                 135                 140
Lys Asn Val Gln Leu Thr Asp Ala Gly Thr Tyr Lys Cys Tyr Ile Ile
145                 150                 155                 160
Thr Ser Lys Gly Lys Gly Asn Ala Asn Leu Glu Tyr Lys Thr Gly Ala
                165                 170                 175
Phe Ser Met Pro Glu Val Asn Val Asp Tyr Asn Ala Ser Ser Glu Thr
            180                 185                 190
Leu Arg Cys Glu Ala Pro Arg Trp Phe Pro Gln Pro Thr Val Val Trp
        195                 200                 205
Ala Ser Gln Val Asp Gln Gly Ala Asn Phe Ser Glu Val Ser Asn Thr
    210                 215                 220
Ser Phe Glu Leu Asn Ser Glu Asn Val Thr Met Lys Val Val Ser Val
225                 230                 235                 240
Leu Tyr Asn Val Thr Ile Asn Asn Thr Tyr Ser Cys Met Ile Glu Asn
                245                 250                 255
Asp Ile Ala Lys Ala Thr Gly Asp Ile Lys Val Thr Glu Ser Glu Ile
            260                 265                 270
Lys Arg Arg Ser His Leu Gln Leu Leu Asn Ser Lys Ala Ser Leu Cys
        275                 280                 285
Val Ser Ser Phe Phe Ala Ile Ser Trp Ala Leu Leu Pro Leu Ser Pro
    290                 295                 300
Tyr Leu Met Leu Lys
305


<210> 393
<211> 282
<212> PRT
<213> Homo sapiens

<400> 393
Met Ala Ser Leu Gly Gln Ile Leu Phe Trp Ser Ile Ile Ser Ile Ile
1               5                   10                  15
Ile Ile Leu Ala Gly Ala Ile Ala Leu Ile Ile Gly Phe Gly Ile Ser

20                  25                  30
Gly Arg His Ser Ile Thr Val Thr Thr Val Ala Ser Ala Gly Asn Ile
        35                  40                  45
Gly Glu Asp Gly Ile Leu Ser Cys Thr Phe Glu Pro Asp Ile Lys Leu
    50                  55                  60
Ser Asp Ile Val Ile Gln Trp Leu Lys Glu Gly Val Leu Gly Leu Val
65                  70                  75                  80
His Glu Phe Lys Glu Gly Lys Asp Glu Leu Ser Glu Gln Asp Glu Met
                85                  90                  95
Phe Arg Gly Arg Thr Ala Val Phe Ala Asp Gln Val Ile Val Gly Asn
            100                 105                 110
Ala Ser Leu Arg Leu Lys Asn Val Gln Leu Thr Asp Ala Gly Thr Tyr
        115                 120                 125
Lys Cys Tyr Ile Ile Thr Ser Lys Gly Lys Gly Asn Ala Asn Leu Glu
    130                 135                 140
Tyr Lys Thr Gly Ala Phe Ser Met Pro Glu Val Asn Val Asp Tyr Asn
145                 150                 155                 160
Ala Ser Ser Glu Thr Leu Arg Cys Glu Ala Pro Arg Trp Phe Pro Gln
                165                 170                 175
Pro Thr Val Val Trp Ala Ser Gln Val Asp Gln Gly Ala Asn Phe Ser
            180                 185                 190
Glu Val Ser Asn Thr Ser Phe Glu Leu Asn Ser Glu Asn Val Thr Met
        195                 200                 205
Lys Val Val Ser Val Leu Tyr Asn Val Thr Ile Asn Asn Thr Tyr Ser
    210                 215                 220
Cys Met Ile Glu Asn Asp Ile Ala Lys Ala Thr Gly Asp Ile Lys Val
225                 230                 235                 240
Thr Glu Ser Glu Ile Lys Arg Arg Ser His Leu Gln Leu Leu Asn Ser
                245                 250                 255
Lys Ala Ser Leu Cys Val Ser Ser Phe Phe Ala Ile Ser Trp Ala Leu
            260                 265                 270
Leu Pro Leu Ser Pro Tyr Leu Met Leu Lys
        275                 280


<210> 394
<211> 20
<212> PRT
<213> Homo sapiens

<400> 394
Met Ala Ser Leu Gly Gln Ile Leu Phe Trp Ser Ile Ile Ser Ile Ile
 1               5                  10                  15
Ile Ile Leu Ala
            20


<210> 395
<211> 20
<212> PRT
<213> Homo sapiens

<400> 395
Ile Ile Ile Leu Ala Gly Ala Ile Ala Leu Ile Ile Gly Phe Gly Ile
 1               5                  10                  15
Ser Gly Arg His
            20

<210> 396
<211> 20
<212> PRT
<213> Homo sapiens

<400> 396
Ile Ser Gly Arg His Ser Ile Thr Val Thr Thr Val Ala Ser Ala Gly
1               5                   10                  15
Asn Ile Gly Glu
            20

<210> 397
<211> 20
<212> PRT
<213> Homo sapiens

<400> 397
Gly Asn Ile Gly Glu Asp Gly Ile Leu Ser Cys Thr Phe Glu Pro Asp
1               5                   10                  15
Ile Lys Leu Ser
            20

<210> 398
<211> 20
<212> PRT
<213> Homo sapiens

<400> 398
Asp Ile Lys Leu Ser Asp Ile Val Ile Gln Trp Leu Lys Glu Gly Val
1               5                   10                  15
Leu Gly Leu Val
            20

<210> 399
<211> 20
<212> PRT
<213> Homo sapiens

<400> 399
Val Leu Gly Leu Val His Glu Phe Lys Glu Gly Lys Asp Glu Leu Ser
1               5                   10                  15
Glu Gln Asp Glu
            20

<210> 400
<211> 20
<212> PRT
<213> Homo sapiens

<400> 400
Ser Glu Gln Asp Glu Met Phe Arg Gly Arg Thr Ala Val Phe Ala Asp

 1               5                   10                  15
Gln Val Ile Val
            20

<210> 401
<211> 20
<212> PRT
<213> Homo sapiens

<400> 401
Asp Gln Val Ile Val Gly Asn Ala Ser Leu Arg Leu Lys Asn Val Gln
 1               5                   10                  15
Leu Thr Asp Ala
            20

<210> 402
<211> 21
<212> PRT
<213> Homo sapiens

<400> 402
Val Gln Leu Thr Asp Ala Gly Thr Tyr Lys Cys Tyr Ile Ile Thr Ser
 1               5                   10                  15
Lys Gly Lys Gly Asn
            20

<210> 403
<211> 20
<212> PRT
<213> Homo sapiens

<400> 403
Lys Gly Lys Gly Asn Ala Asn Leu Glu Tyr Lys Thr Gly Ala Phe Ser
 1               5                   10                  15
Met Pro Glu Val
            20

<210> 404
<211> 20
<212> PRT
<213> Homo sapiens

<400> 404
Ser Met Pro Glu Val Asn Val Asp Tyr Asn Ala Ser Ser Glu Thr Leu
 1               5                   10                  15
Arg Cys Glu Ala
            20

<210> 405
<211> 20
<212> PRT
<213> Homo sapiens

<400> 405
Leu Arg Cys Glu Ala Pro Arg Trp Phe Pro Gln Pro Thr Val Val Trp
1               5                   10                  15
Ala Ser Gln Val
            20

<210> 406
<211> 20
<212> PRT
<213> Homo sapiens

<400> 406
Trp Ala Ser Gln Val Asp Gln Gly Ala Asn Phe Ser Glu Val Ser Asn
1               5                   10                  15
Thr Ser Phe Glu
            20

<210> 407
<211> 20
<212> PRT
<213> Homo sapiens

<400> 407
Asn Thr Ser Phe Glu Leu Asn Ser Glu Asn Val Thr Met Lys Val Val
1               5                   10                  15
Ser Val Leu Tyr
            20

<210> 408
<211> 20
<212> PRT
<213> Homo sapiens

<400> 408
Val Ser Val Leu Tyr Asn Val Thr Ile Asn Asn Thr Tyr Ser Cys Met
1               5                   10                  15
Ile Glu Asn Asp
            20

<210> 409
<211> 20
<212> PRT
<213> Homo sapiens

<400> 409
Met Ile Glu Asn Asp Ile Ala Lys Ala Thr Gly Asp Ile Lys Val Thr
1               5                   10                  15
Glu Ser Glu Ile
            20

<210> 410

<211> 20
<212> PRT
<213> Homo sapiens

<400> 410
Thr Glu Ser Glu Ile Lys Arg Arg Ser His Leu Gln Leu Leu Asn Ser
1               5                   10                  15
Lys Ala Ser Leu
            20


<210> 411
<211> 20
<212> PRT
<213> Homo sapiens

<400> 411
Ser Lys Ala Ser Leu Cys Val Ser Ser Phe Phe Ala Ile Ser Trp Ala
1               5                   10                  15
Leu Leu Pro Leu
            20


<210> 412
<211> 20
<212> PRT
<213> Homo sapiens

<400> 412
Ser Ser Phe Phe Ala Ile Ser Trp Ala Leu Leu Pro Leu Ser Pro Tyr
1               5                   10                  15
Leu Met Leu Lys
            20


<210> 413
<211> 35
<212> PRT
<213> Homo sapiens

<400> 413
Ile Ser Gly Arg His Ser Ile Thr Val Thr Thr Val Ala Ser Ala Gly
1               5                   10                  15
Asn Ile Gly Glu Asp Gly Ile Leu Ser Cys Thr Phe Glu Pro Asp Ile
            20                  25                  30
Lys Leu Ser
        35


<210> 414
<211> 35
<212> PRT
<213> Homo sapiens

<400> 414
Val Leu Gly Leu Val His Glu Phe Lys Glu Gly Lys Asp Glu Leu Ser
1               5                   10                  15

Glu Gln Asp Glu Met Phe Arg Gly Arg Thr Ala Val Phe Ala Asp Gln
            20                  25                  30
Val Ile Val
        35

<210> 415
<211> 65
<212> PRT
<213> Homo sapiens

<400> 415
Lys Gly Lys Gly Asn Ala Asn Leu Glu Tyr Lys Thr Gly Ala Phe Ser
 1               5                  10                  15
Met Pro Glu Val Asn Val Asp Tyr Asn Ala Ser Ser Glu Thr Leu Arg
            20                  25                  30
Cys Glu Ala Pro Arg Trp Phe Pro Gln Pro Thr Val Val Trp Ala Ser
        35                  40                  45
Gln Val Asp Gln Gly Ala Asn Phe Ser Glu Val Ser Asn Thr Ser Phe
    50                  55                  60
Glu
65

<210> 416
<211> 10
<212> PRT
<213> Homo sapiens

<400> 416
Lys Leu Ser Asp Ile Val Ile Gln Trp Leu
 1               5                  10

<210> 417
<211> 10
<212> PRT
<213> Homo sapiens

<400> 417
Ser Leu Gly Gln Ile Leu Phe Trp Ser Ile
 1               5                  10

<210> 418
<211> 10
<212> PRT
<213> Homo sapiens

<400> 418
Leu Leu Asn Ser Lys Ala Ser Leu Cys Val
 1               5                  10

<210> 419
<211> 10
<212> PRT

<213> Homo sapiens

<400> 419
Ser Leu Cys Val Ser Ser Phe Phe Ala Ile
1               5                   10


<210> 420
<211> 10
<212> PRT
<213> Homo sapiens

<400> 420
Val Leu Tyr Asn Val Thr Ile Asn Asn Thr
1               5                   10


<210> 421
<211> 10
<212> PRT
<213> Homo sapiens

<400> 421
Ile Leu Phe Trp Ser Ile Ile Ser Ile Ile
1               5                   10


<210> 422
<211> 10
<212> PRT
<213> Homo sapiens

<400> 422
Leu Leu Pro Leu Ser Pro Tyr Leu Met Leu
1               5                   10


<210> 423
<211> 10
<212> PRT
<213> Homo sapiens

<400> 423
Cys Met Ile Glu Asn Asp Ile Ala Lys Ala
1               5                   10


<210> 424
<211> 10
<212> PRT
<213> Homo sapiens

<400> 424
Lys Thr Gly Ala Phe Ser Met Pro Glu Val
1               5                   10

<210> 425
<211> 10
<212> PRT
<213> Homo sapiens

<400> 425
Trp Ala Leu Leu Pro Leu Ser Pro Tyr Leu
1               5                   10


<210> 426
<211> 10
<212> PRT
<213> Homo sapiens

<400> 426
Ile Ile Leu Ala Gly Ala Ile Ala Leu Ile
1               5                   10


<210> 427
<211> 10
<212> PRT
<213> Homo sapiens

<400> 427
Gln Leu Thr Asp Ala Gly Thr Tyr Lys Cys
1               5                   10


<210> 428
<211> 10
<212> PRT
<213> Homo sapiens

<400> 428
Ala Leu Leu Pro Leu Ser Pro Tyr Leu Met
1               5                   10


<210> 429
<211> 10
<212> PRT
<213> Homo sapiens

<400> 429
Gln Leu Leu Asn Ser Lys Ala Ser Leu Cys
1               5                   10


<210> 430
<211> 10
<212> PRT
<213> Homo sapiens

<400> 430
Ile Leu Ser Cys Thr Phe Glu Pro Asp Ile

1               5                   10


<210> 431
<211> 10
<212> PRT
<213> Homo sapiens

<400> 431
Trp Leu Lys Glu Gly Val Leu Gly Leu Val
1               5                   10


<210> 432
<211> 10
<212> PRT
<213> Homo sapiens

<400> 432
Leu Gln Leu Leu Asn Ser Lys Ala Ser Leu
1               5                   10


<210> 433
<211> 10
<212> PRT
<213> Homo sapiens

<400> 433
Gln Ile Leu Phe Trp Ser Ile Ile Ser Ile
1               5                   10


<210> 434
<211> 10
<212> PRT
<213> Homo sapiens

<400> 434
Gly Ile Ser Gly Arg His Ser Ile Thr Val
1               5                   10


<210> 435
<211> 10
<212> PRT
<213> Homo sapiens

<400> 435
Phe Glu Pro Asp Ile Lys Leu Ser Asp Ile
1               5                   10


<210> 436
<211> 9
<212> PRT
<213> Homo sapiens

<400> 436
Ala Leu Leu Pro Leu Ser Pro Tyr Leu
1               5


<210> 437
<211> 9
<212> PRT
<213> Homo sapiens

<400> 437
Ser Leu Cys Val Ser Ser Phe Phe Ala
1               5


<210> 438
<211> 9
<212> PRT
<213> Homo sapiens

<400> 438
Ile Leu Phe Trp Ser Ile Ile Ser Ile
1               5


<210> 439
<211> 9
<212> PRT
<213> Homo sapiens

<400> 439
Gln Leu Leu Asn Ser Lys Ala Ser Leu
1               5


<210> 440
<211> 9
<212> PRT
<213> Homo sapiens

<400> 440
Lys Val Val Ser Val Leu Tyr Asn Val
1               5


<210> 441
<211> 9
<212> PRT
<213> Homo sapiens

<400> 441
Ile Leu Ala Gly Ala Ile Ala Leu Ile
1               5


<210> 442

<211> 9
<212> PRT
<213> Homo sapiens

<400> 442
Trp Leu Lys Glu Gly Val Leu Gly Leu
1               5


<210> 443
<211> 9
<212> PRT
<213> Homo sapiens

<400> 443
Ile Ile Leu Ala Gly Ala Ile Ala Leu
1               5


<210> 444
<211> 9
<212> PRT
<213> Homo sapiens

<400> 444
Asn Val Thr Met Lys Val Val Ser Val
1               5


<210> 445
<211> 9
<212> PRT
<213> Homo sapiens

<400> 445
Glu Met Phe Arg Gly Arg Thr Ala Val
1               5


<210> 446
<211> 9
<212> PRT
<213> Homo sapiens

<400> 446
Ala Val Phe Ala Asp Gln Val Ile Val
1               5


<210> 447
<211> 9
<212> PRT
<213> Homo sapiens

<400> 447
Leu Leu Pro Leu Ser Pro Tyr Leu Met
1               5

<210> 448
<211> 9
<212> PRT
<213> Homo sapiens

<400> 448
Leu Leu Asn Ser Lys Ala Ser Leu Cys
1               5


<210> 449
<211> 9
<212> PRT
<213> Homo sapiens

<400> 449
Val Ile Gln Trp Leu Lys Glu Gly Val
1               5


<210> 450
<211> 9
<212> PRT
<213> Homo sapiens

<400> 450
Ala Ile Ser Trp Ala Leu Leu Pro Leu
1               5


<210> 451
<211> 9
<212> PRT
<213> Homo sapiens

<400> 451
Ser Leu Gly Gln Ile Leu Phe Trp Ser
1               5


<210> 452
<211> 9
<212> PRT
<213> Homo sapiens

<400> 452
Ile Ala Leu Ile Ile Gly Phe Gly Ile
1               5


<210> 453
<211> 9
<212> PRT
<213> Homo sapiens

<400> 453
Cys Thr Phe Glu Pro Asp Ile Lys Leu
1               5


<210> 454
<211> 9
<212> PRT
<213> Homo sapiens

<400> 454
Ile Val Gly Asn Ala Ser Leu Arg Leu
1               5


<210> 455
<211> 9
<212> PRT
<213> Homo sapiens

<400> 455
Gly Gln Ile Leu Phe Trp Ser Ile Ile
1               5


<210> 456
<211> 3447
<212> DNA
<213> Homo sapiens

<400> 456
atgcccttgt tcaagaacac cagtgtcagc tctctgtact ctggttgcag actgaccttg 60
ctcaggcctg agaaggatgg ggcagccacc agagtggatg ctgtctgcac ccatcgtcct 120
gaccccaaaa gccctggact ggacagagag cggctgtact ggaagctgag ccagctgacc 180
cacggcatca ctgagctggg cccctacacc ctggacaggc acagtctcta tgtcaatggt 240
ttcacccatc agagctctat gacgaccacc agaactcctg atacctccac aatgcacctg 300
gcaacctcga gaactccagc ctccctgtct ggacctacga ccgccagccc tctcctggtg 360
ctattcacaa ttaacttcac catcactaac ctgcggtatg aggagaacat gcatcaccct 420
ggctctagaa agtttaacac cacggagaga gtccttcagg gtctgctcag gcctgtgttc 480
aagaacacca gtgttggccc tctgtactct ggctgcagac tgaccttgct caggcccaag 540
aaggatgggg cagccaccaa agtggatgcc atctgcacct accgccctga tcccaaaagc 600
cctggactgg acagagagca gctatactgg gagctgagcc agctaaccca cagcatcact 660
gagctgggcc cctacaccct ggacagggac agtctctatg tcaatggttt cacacagcgg 720
agctctgtgc ccaccactag cattcctggg acccccacag tggacctggg aacatctggg 780
actccagttt ctaaacctgg tccctcggct gccagccctc tcctggtgct attcactctc 840
aacttcacca tcaccaacct gcggtatgag gagaacatgc agcaccctgg ctccaggaag 900
ttcaacacca cggagagggt ccttcagggc ctgctcaggt ccctgttcaa gagcaccagt 960
gttggccctc tgtactctgg ctgcagactg actttgctca ggcctgaaaa ggatgggaca 1020
gccactggag tggatgccat ctgcacccac caccctgacc ccaaaagccc taggctggac 1080
agagagcagc tgtattggga gctgagccag ctgacccaca atatcactga gctgggccac 1140
tatgccctgg acaacgacag cctctttgtc aatggtttca ctcatcggag ctctgtgtcc 1200
accaccagca ctcctgggac ccccacagtg tatctgggag catctaagac tccagcctcg 1260
atatttggcc cttcagctgc cagccatctc ctgatactat tcaccctcaa cttcaccatc 1320
actaacctgc ggtatgagga gaacatgtgg cctggctcca ggaagttcaa cactacagag 1380
agggtccttc agggcctgct aaggcccttg ttcaagaaca ccagtgttgg ccctctgtac 1440
tctggctcca ggctgacctt gctcaggcca gagaaagatg gggaagccac cggagtggat 1500
gccatctgca cccaccgccc tgaccccaca ggccctgggc tggacagaga gcagctgtat 1560

ttggagctga gccagctgac ccacagcatc actgagctgg gcccctacac actggacagg 1620
gacagtctct atgtcaatgg tttcacccat cggagctctg tacccaccac cagcaccggg 1680
gtggtcagcg aggagccatt cacactgaac ttcaccatca acaacctgcg ctacatggcg 1740
gacatgggcc aacccggctc cctcaagttc aacatcacag acaacgtcat gaagcacctg 1800
ctcagtcctt tgttccagag gagcagcctg ggtgcacggt acacaggctg cagggtcatc 1860
gcactaaggt ctgtgaagaa cggtgctgag acacgggtgg acctcctctg cacctacctg 1920
cagcccctca gcggcccagg tctgcctatc aagcaggtgt tccatgagct gagccagcag 1980
acccatggca tcacccggct gggcccctac tctctggaca aagacagcct ctaccttaac 2040
ggttacaatg aacctggtct agatgagcct cctacaactc ccaagccagc caccacattc 2100
ctgcctcctc tgtcagaagc cacaacagcc atggggtacc acctgaagac cctcacactc 2160
aacttcacca tctccaatct ccagtattca ccagatatgg gcaagggctc agctacattc 2220
aactccaccg agggggtcct tcagcacctg ctcagaccct tgttccagaa gagcagcatg 2280
ggccccttct acttgggttg ccaactgatc tccctcaggc ctgagaagga tggggcagcc 2340
actggtgtgg acaccacctg cacctaccac cctgaccctg tgggccccgg gctggacata 2400
cagcagcttt actgggagct gagtcagctg acccatggtg tcacccaact gggcttctat 2460
gtcctggaca gggatagcct cttcatcaat ggctatgcac cccagaattt atcaatccgg 2520
ggcgagtacc agataaattt ccacattgtc aactggaacc tcagtaatcc agaccccaca 2580
tcctcagagt acatcaccct gctgagggac atccaggaca aggtcaccac actctacaaa 2640
ggcagtcaac tacatgacac attccgcttc tgcctggtca ccaacttgac gatggactcc 2700
gtgttggtca ctgtcaaggc attgttctcc tccaatttgg accccagcct ggtggagcaa 2760
gtctttctag ataagaccct gaatgcctca ttccattggc tgggctccac ctaccagttg 2820
gtggacatcc atgtgacaga aatggagtca tcagtttatc aaccaacaag cagctccagc 2880
acccagcact tctacccgaa tttcaccatc accaacctac catattccca ggacaaagcc 2940
cagccaggca ccaccaatta ccagaggaac aaaaggaata ttgaggatgc gctcaaccaa 3000
ctcttccgaa acagcagcat caagagttat ttttctgact gtcaagtttc aacattcagg 3060
tctgtcccca acaggcacca caccggggtg gactccctgt gtaacttctc gccactggct 3120
cggagagtag acagagttgc catctatgag gaatttctgc ggatgacccg gaatggtacc 3180
cagctgcaga acttcaccct ggacaggagc agtgtccttg tggatgggta ttctcccaac 3240
agaaatgagc ccttaactgg gaattctgac cttcccttct gggctgtcat cttcatcggc 3300
ttggcaggac tcctgggact catcacatgc ctgatctgcg gtgtcctggt gaccacccgc 3360
cggcggaaga aggaaggaga atacaacgtc cagcaacagt gcccaggcta ctaccagtca 3420
cacctagacc tggaggatct gcaatga 3447

<210> 457
<211> 3557
<212> DNA
<213> Homo sapiens

<400> 457
gagagggtcc ttcagggtct gcttatgccc ttgttcaaga acaccagtgt cagctctctg 60
tactctggtt gcagactgac cttgctcagg cctgagaagg atggggcagc caccagagtg 120
gatgctgtct gcacccatcg tcctgacccc aaaagccctg gactggacag agagcggctg 180
tactggaagc tgagccagct gacccacggc atcactgagc tgggccccta caccctggac 240
aggcacagtc tctatgtcaa tggtttcacc catcagagct ctatgacgac caccagaact 300
cctgatacct ccacaatgca cctggcaacc tcgagaactc cagcctccct gtctggacct 360
acgaccgcca gccctctcct ggtgctattc acaattaact tcaccatcac taacctgcgg 420
tatgaggaga acatgcatca ccctggctct agaaagttta acaccacgga gagagtcctt 480
cagggtctgc tcaggcctgt gttcaagaac accagtgttg gccctctgta ctctggctgc 540
agactgacct tgctcaggcc caagaaggat ggggcagcca ccaaagtgga tgccatctgc 600
acctaccgcc ctgatcccaa aagccctgga ctggacagag agcagctata ctgggagctg 660
agccagctaa cccacagcat cactgagctg ggcccctaca ccctggacag ggacagtctc 720
tatgtcaatg gtttcacaca gcggagctct gtgcccacca ctagcattcc tgggaccccc 780
acagtggacc tgggaacatc tgggactcca gtttctaaac ctggtccctc ggctgccagc 840
cctctcctgg tgctattcac tctcaacttc accatcacca acctgcggta tgaggagaac 900
atgcagcacc ctggctccag gaagttcaac accacggaga gggtccttca gggcctgctc 960
aggtccctgt tcaagagcac cagtgttggc cctctgtact ctggctgcag actgactttg 1020
ctcaggcctg aaaaggatgg gacagccact ggagtggatg ccatctgcac ccaccaccct 1080

gaccccaaaa gccctaggct ggacagagag cagctgtatt gggagctgag ccagctgacc 1140
cacaatatca ctgagctggg ccactatgcc ctggacaacg acagcctctt tgtcaatggt 1200
ttcactcatc ggagctctgt gtccaccacc agcactcctg ggacccccac agtgtatctg 1260
ggagcatcta agactccagc ctcgatattt ggcccttcag ctgccagcca tctcctgata 1320
ctattcaccc tcaacttcac catcactaac ctgcggtatg aggagaacat gtggcctggc 1380
tccaggaagt tcaacactac agagagggtc cttcagggcc tgctaaggcc cttgttcaag 1440
aacaccagtg ttggccctct gtactctggc tccaggctga ccttgctcag gccagagaaa 1500
gatggggaag ccaccggagt ggatgccatc tgcacccacc gccctgaccc cacaggccct 1560
gggctggaca gagagcagct gtatttggag ctgagccagc tgacccacag catcactgag 1620
ctgggcccct acacactgga cagggacagt ctctatgtca atggtttcac ccatcggagc 1680
tctgtaccca ccaccagcac cggggtggtc agcgaggagc cattcacact gaacttcacc 1740
atcaacaacc tgcgctacat ggcggacatg ggccaacccg gctccctcaa gttcaacatc 1800
acagacaacg tcatgaagca cctgctcagt cctttgttcc agaggagcag cctgggtgca 1860
cggtacacag gctgcagggt catcgcacta aggtctgtga agaacggtgc tgagacacgg 1920
gtggacctcc tctgcaccta cctgcagccc ctcagcggcc caggtctgcc tatcaagcag 1980
gtgttccatg agctgagcca gcagacccat ggcatcaccc ggctgggccc ctactctctg 2040
gacaaagaca gcctctacct taacggttac aatgaacctg gtctagatga gcctcctaca 2100
actcccaagc cagccaccac attcctgcct cctctgtcag aagccacaac agccatgggg 2160
taccacctga agaccctcac actcaacttc accatctcca atctccagta ttcaccagat 2220
atgggcaagg gctcagctac attcaactcc accgaggggg tccttcagca cctgctcaga 2280
cccttgttcc agaagagcag catgggcccc ttctacttgg gttgccaact gatctccctc 2340
aggcctgaga aggatggggc agccactggt gtggacacca cctgcaccta ccaccctgac 2400
cctgtgggcc ccgggctgga catacagcag ctttactggg agctgagtca gctgacccat 2460
ggtgtcaccc aactgggctt ctatgtcctg gacagggata gcctcttcat caatggctat 2520
gcaccccaga atttatcaat ccggggcgag taccagataa atttccacat tgtcaactgg 2580
aacctcagta atccagaccc cacatcctca gagtacatca ccctgctgag ggacatccag 2640
gacaaggtca ccacactcta caaaggcagt caactacatg acacattccg cttctgcctg 2700
gtcaccaact tgacgatgga ctccgtgttg gtcactgtca aggcattgtt ctcctccaat 2760
ttggacccca gcctggtgga gcaagtcttt ctagataaga ccctgaatgc ctcattccat 2820
tggctgggct ccacctacca gttggtggac atccatgtga cagaaatgga gtcatcagtt 2880
tatcaaccaa caagcagctc cagcacccag cacttctacc cgaatttcac catcaccaac 2940
ctaccatatt cccaggacaa agcccagcca ggcaccacca attaccagag gaacaaaagg 3000
aatattgagg atgcgctcaa ccaactcttc cgaaacagca gcatcaagag ttatttttct 3060
gactgtcaag tttcaacatt caggtctgtc cccaacaggc accacaccgg ggtggactcc 3120
ctgtgtaact tctcgccact ggctcggaga gtagacagag ttgccatcta tgaggaattt 3180
ctgcggatga cccggaatgg tacccagctg cagaacttca ccctggacag gagcagtgtc 3240
cttgtggatg ggtattctcc caacagaaat gagcccttaa ctgggaattc tgaccttccc 3300
ttctgggctg tcatcttcat cggcttggca ggactcctgg gactcatcac atgcctgatc 3360
tgcggtgtcc tggtgaccac ccgccggcgg aagaaggaag gagaatacaa cgtccagcaa 3420
cagtgcccag gctactacca gtcacaccta gacctggagg atctgcaatg actggaactt 3480
gccggtgcct ggggtgcctt tcccccagcc agggtccaaa gaagcttggc tggggcagaa 3540
ataaaccata ttggtcg 3557

<210> 458
<211> 1148
<212> PRT
<213> Homo sapiens

<400> 458
Met Pro Leu Phe Lys Asn Thr Ser Val Ser Ser Leu Tyr Ser Gly Cys
1               5                   10                  15
Arg Leu Thr Leu Leu Arg Pro Glu Lys Asp Gly Ala Ala Thr Arg Val
            20                  25                  30
Asp Ala Val Cys Thr His Arg Pro Asp Pro Lys Ser Pro Gly Leu Asp
        35                  40                  45
Arg Glu Arg Leu Tyr Trp Lys Leu Ser Gln Leu Thr His Gly Ile Thr
    50                  55                  60

Glu Leu Gly Pro Tyr Thr Leu Asp Arg His Ser Leu Tyr Val Asn Gly
65                  70                  75                  80
Phe Thr His Gln Ser Ser Met Thr Thr Thr Arg Thr Pro Asp Thr Ser
                85                  90                  95
Thr Met His Leu Ala Thr Ser Arg Thr Pro Ala Ser Leu Ser Gly Pro
            100                 105                 110
Thr Thr Ala Ser Pro Leu Leu Val Leu Phe Thr Ile Asn Phe Thr Ile
        115                 120                 125
Thr Asn Leu Arg Tyr Glu Glu Asn Met His His Pro Gly Ser Arg Lys
    130                 135                 140
Phe Asn Thr Thr Glu Arg Val Leu Gln Gly Leu Leu Arg Pro Val Phe
145                 150                 155                 160
Lys Asn Thr Ser Val Gly Pro Leu Tyr Ser Gly Cys Arg Leu Thr Leu
                165                 170                 175
Leu Arg Pro Lys Lys Asp Gly Ala Ala Thr Lys Val Asp Ala Ile Cys
            180                 185                 190
Thr Tyr Arg Pro Asp Pro Lys Ser Pro Gly Leu Asp Arg Glu Gln Leu
        195                 200                 205
Tyr Trp Glu Leu Ser Gln Leu Thr His Ser Ile Thr Glu Leu Gly Pro
    210                 215                 220
Tyr Thr Leu Asp Arg Asp Ser Leu Tyr Val Asn Gly Phe Thr Gln Arg
225                 230                 235                 240
Ser Ser Val Pro Thr Thr Ser Ile Pro Gly Thr Pro Thr Val Asp Leu
                245                 250                 255
Gly Thr Ser Gly Thr Pro Val Ser Lys Pro Gly Pro Ser Ala Ala Ser
            260                 265                 270
Pro Leu Leu Val Leu Phe Thr Leu Asn Phe Thr Ile Thr Asn Leu Arg
        275                 280                 285
Tyr Glu Glu Asn Met Gln His Pro Gly Ser Arg Lys Phe Asn Thr Thr
    290                 295                 300
Glu Arg Val Leu Gln Gly Leu Leu Arg Ser Leu Phe Lys Ser Thr Ser
305                 310                 315                 320
Val Gly Pro Leu Tyr Ser Gly Cys Arg Leu Thr Leu Leu Arg Pro Glu
                325                 330                 335
Lys Asp Gly Thr Ala Thr Gly Val Asp Ala Ile Cys Thr His His Pro
            340                 345                 350
Asp Pro Lys Ser Pro Arg Leu Asp Arg Glu Gln Leu Tyr Trp Glu Leu
        355                 360                 365
Ser Gln Leu Thr His Asn Ile Thr Glu Leu Gly His Tyr Ala Leu Asp
    370                 375                 380
Asn Asp Ser Leu Phe Val Asn Gly Phe Thr His Arg Ser Ser Val Ser
385                 390                 395                 400
Thr Thr Ser Thr Pro Gly Thr Pro Thr Val Tyr Leu Gly Ala Ser Lys
                405                 410                 415
Thr Pro Ala Ser Ile Phe Gly Pro Ser Ala Ala Ser His Leu Leu Ile
            420                 425                 430
Leu Phe Thr Leu Asn Phe Thr Ile Thr Asn Leu Arg Tyr Glu Glu Asn
        435                 440                 445
Met Trp Pro Gly Ser Arg Lys Phe Asn Thr Thr Glu Arg Val Leu Gln
    450                 455                 460
Gly Leu Leu Arg Pro Leu Phe Lys Asn Thr Ser Val Gly Pro Leu Tyr
465                 470                 475                 480
Ser Gly Ser Arg Leu Thr Leu Leu Arg Pro Glu Lys Asp Gly Glu Ala
                485                 490                 495
Thr Gly Val Asp Ala Ile Cys Thr His Arg Pro Asp Pro Thr Gly Pro
            500                 505                 510
Gly Leu Asp Arg Glu Gln Leu Tyr Leu Glu Leu Ser Gln Leu Thr His

515                 520                 525
Ser Ile Thr Glu Leu Gly Pro Tyr Thr Leu Asp Arg Asp Ser Leu Tyr
    530                 535                 540
Val Asn Gly Phe Thr His Arg Ser Ser Val Pro Thr Thr Ser Thr Gly
545                 550                 555                 560
Val Val Ser Glu Glu Pro Phe Thr Leu Asn Phe Thr Ile Asn Asn Leu
                565                 570                 575
Arg Tyr Met Ala Asp Met Gly Gln Pro Gly Ser Leu Lys Phe Asn Ile
                580                 585                 590
Thr Asp Asn Val Met Lys His Leu Leu Ser Pro Leu Phe Gln Arg Ser
            595                 600                 605
Ser Leu Gly Ala Arg Tyr Thr Gly Cys Arg Val Ile Ala Leu Arg Ser
    610                 615                 620
Val Lys Asn Gly Ala Glu Thr Arg Val Asp Leu Leu Cys Thr Tyr Leu
625                 630                 635                 640
Gln Pro Leu Ser Gly Pro Gly Leu Pro Ile Lys Gln Val Phe His Glu
                645                 650                 655
Leu Ser Gln Gln Thr His Gly Ile Thr Arg Leu Gly Pro Tyr Ser Leu
                660                 665                 670
Asp Lys Asp Ser Leu Tyr Leu Asn Gly Tyr Asn Glu Pro Gly Leu Asp
            675                 680                 685
Glu Pro Pro Thr Thr Pro Lys Pro Ala Thr Thr Phe Leu Pro Pro Leu
    690                 695                 700
Ser Glu Ala Thr Thr Ala Met Gly Tyr His Leu Lys Thr Leu Thr Leu
705                 710                 715                 720
Asn Phe Thr Ile Ser Asn Leu Gln Tyr Ser Pro Asp Met Gly Lys Gly
                725                 730                 735
Ser Ala Thr Phe Asn Ser Thr Glu Gly Val Leu Gln His Leu Leu Arg
                740                 745                 750
Pro Leu Phe Gln Lys Ser Ser Met Gly Pro Phe Tyr Leu Gly Cys Gln
            755                 760                 765
Leu Ile Ser Leu Arg Pro Glu Lys Asp Gly Ala Ala Thr Gly Val Asp
    770                 775                 780
Thr Thr Cys Thr Tyr His Pro Asp Pro Val Gly Pro Gly Leu Asp Ile
785                 790                 795                 800
Gln Gln Leu Tyr Trp Glu Leu Ser Gln Leu Thr His Gly Val Thr Gln
                805                 810                 815
Leu Gly Phe Tyr Val Leu Asp Arg Asp Ser Leu Phe Ile Asn Gly Tyr
                820                 825                 830
Ala Pro Gln Asn Leu Ser Ile Arg Gly Glu Tyr Gln Ile Asn Phe His
            835                 840                 845
Ile Val Asn Trp Asn Leu Ser Asn Pro Asp Pro Thr Ser Ser Glu Tyr
    850                 855                 860
Ile Thr Leu Leu Arg Asp Ile Gln Asp Lys Val Thr Thr Leu Tyr Lys
865                 870                 875                 880
Gly Ser Gln Leu His Asp Thr Phe Arg Phe Cys Leu Val Thr Asn Leu
                885                 890                 895
Thr Met Asp Ser Val Leu Val Thr Val Lys Ala Leu Phe Ser Ser Asn
                900                 905                 910
Leu Asp Pro Ser Leu Val Glu Gln Val Phe Leu Asp Lys Thr Leu Asn
            915                 920                 925
Ala Ser Phe His Trp Leu Gly Ser Thr Tyr Gln Leu Val Asp Ile His
    930                 935                 940
Val Thr Glu Met Glu Ser Ser Val Tyr Gln Pro Thr Ser Ser Ser Ser
945                 950                 955                 960
Thr Gln His Phe Tyr Pro Asn Phe Thr Ile Thr Asn Leu Pro Tyr Ser
                965                 970                 975

Gln Asp Lys Ala Gln Pro Gly Thr Thr Asn Tyr Gln Arg Asn Lys Arg
            980                 985                 990
Asn Ile Glu Asp Ala Leu Asn Gln Leu Phe Arg Asn Ser Ser Ile Lys
        995                 1000                1005
Ser Tyr Phe Ser Asp Cys Gln Val Ser Thr Phe Arg Ser Val Pro Asn
    1010                1015                1020
Arg His His Thr Gly Val Asp Ser Leu Cys Asn Phe Ser Pro Leu Ala
1025                1030                1035                1040
Arg Arg Val Asp Arg Val Ala Ile Tyr Glu Glu Phe Leu Arg Met Thr
                1045                1050                1055
Arg Asn Gly Thr Gln Leu Gln Asn Phe Thr Leu Asp Arg Ser Ser Val
            1060                1065                1070
Leu Val Asp Gly Tyr Ser Pro Asn Arg Asn Glu Pro Leu Thr Gly Asn
        1075                1080                1085
Ser Asp Leu Pro Phe Trp Ala Val Ile Phe Ile Gly Leu Ala Gly Leu
    1090                1095                1100
Leu Gly Leu Ile Thr Cys Leu Ile Cys Gly Val Leu Val Thr Thr Arg
1105                1110                1115                1120
Arg Arg Lys Lys Glu Gly Glu Tyr Asn Val Gln Gln Gln Cys Pro Gly
                1125                1130                1135
Tyr Tyr Gln Ser His Leu Asp Leu Glu Asp Leu Gln
            1140                1145

<210> 459
<211> 1156
<212> PRT
<213> Homo sapiens

<400> 459
   Glu Arg Val Leu Gln Gly Leu Leu Met Pro Leu Phe Lys Asn Thr Ser
    1               5                   10                  15
   Val Ser Ser Leu Tyr Ser Gly Cys Arg Leu Thr Leu Leu Arg Pro Glu
               20                  25                  30
   Lys Asp Gly Ala Ala Thr Arg Val Asp Ala Val Cys Thr His Arg Pro
           35                  40                  45
   Asp Pro Lys Ser Pro Gly Leu Asp Arg Glu Arg Leu Tyr Trp Lys Leu
       50                  55                  60
   Ser Gln Leu Thr His Gly Ile Thr Glu Leu Gly Pro Tyr Thr Leu Asp
   65                  70                  75                  80
   Arg His Ser Leu Tyr Val Asn Gly Phe Thr His Gln Ser Ser Met Thr
                   85                  90                  95
   Thr Thr Arg Thr Pro Asp Thr Ser Thr Met His Leu Ala Thr Ser Arg
               100                 105                 110
   Thr Pro Ala Ser Leu Ser Gly Pro Thr Thr Ala Ser Pro Leu Leu Val
           115                 120                 125
   Leu Phe Thr Ile Asn Phe Thr Ile Thr Asn Leu Arg Tyr Glu Glu Asn
       130                 135                 140
   Met His His Pro Gly Ser Arg Lys Phe Asn Thr Thr Glu Arg Val Leu
   145                 150                 155                 160
   Gln Gly Leu Leu Arg Pro Val Phe Lys Asn Thr Ser Val Gly Pro Leu
                   165                 170                 175
   Tyr Ser Gly Cys Arg Leu Thr Leu Leu Arg Pro Lys Lys Asp Gly Ala
               180                 185                 190
   Ala Thr Lys Val Asp Ala Ile Cys Thr Tyr Arg Pro Asp Pro Lys Ser
           195                 200                 205
   Pro Gly Leu Asp Arg Glu Gln Leu Tyr Trp Glu Leu Ser Gln Leu Thr
   210                 215                 220
   His Ser Ile Thr Glu Leu Gly Pro Tyr Thr Leu Asp Arg Asp Ser Leu
   225                 230                 235                 240
   Tyr Val Asn Gly Phe Thr Gln Arg Ser Ser Val Pro Thr Thr Ser Ile
                   245                 250                 255
   Pro Gly Thr Pro Thr Val Asp Leu Gly Thr Ser Gly Thr Pro Val Ser
                   260                 265                 270
   Lys Pro Gly Pro Ser Ala Ala Ser Pro Leu Leu Val Leu Phe Thr Leu
                   275                 280                 285
   Asn Phe Thr Ile Thr Asn Leu Arg Tyr Glu Glu Asn Met Gln His Pro
       290                 295                 300
   Gly Ser Arg Lys Phe Asn Thr Thr Glu Arg Val Leu Gln Gly Leu Leu
   305                 310                 315                 320
   Arg Ser Leu Phe Lys Ser Thr Ser Val Gly Pro Leu Tyr Ser Gly Cys
                   325                 330                 335
   Arg Leu Thr Leu Leu Arg Pro Glu Lys Asp Gly Thr Ala Thr Gly Val
                   340                 345                 350
   Asp Ala Ile Cys Thr His His Pro Asp Pro Lys Ser Pro Arg Leu Asp
           355                 360                 365
   Arg Glu Gln Leu Tyr Trp Glu Leu Ser Gln Leu Thr His Asn Ile Thr
       370                 375                 380
   Glu Leu Gly His Tyr Ala Leu Asp Asn Asp Ser Leu Phe Val Asn Gly
   385                 390                 395                 400
   Phe Thr His Arg Ser Ser Val Ser Thr Thr Ser Thr Pro Gly Thr Pro
                   405                 410                 415
   Thr Val Tyr Leu Gly Ala Ser Lys Thr Pro Ala Ser Ile Phe Gly Pro
                   420                 425                 430
   Ser Ala Ala Ser His Leu Leu Ile Leu Phe Thr Leu Asn Phe Thr Ile
           435                 440                 445
   Thr Asn Leu Arg Tyr Glu Glu Asn Met Trp Pro Gly Ser Arg Lys Phe
       450                 455                 460
   Asn Thr Thr Glu Arg Val Leu Gln Gly Leu Leu Arg Pro Leu Phe Lys
   465                 470                 475                 480
   Asn Thr Ser Val Gly Pro Leu Tyr Ser Gly Ser Arg Leu Thr Leu Leu
                   485                 490                 495
   Arg Pro Glu Lys Asp Gly Glu Ala Thr Gly Val Asp Ala Ile Cys Thr
                   500                 505                 510
   His Arg Pro Asp Pro Thr Gly Pro Gly Leu Asp Arg Glu Gln Leu Tyr
           515                 520                 525
   Leu Glu Leu Ser Gln Leu Thr His Ser Ile Thr Glu Leu Gly Pro Tyr
       530                 535                 540
   Thr Leu Asp Arg Asp Ser Leu Tyr Val Asn Gly Phe Thr His Arg Ser
   545                 550                 555                 560
   Ser Val Pro Thr Thr Ser Thr Gly Val Val Ser Glu Glu Pro Phe Thr
                   565                 570                 575
   Leu Asn Phe Thr Ile Asn Asn Leu Arg Tyr Met Ala Asp Met Gly Gln
                   580                 585                 590
   Pro Gly Ser Leu Lys Phe Asn Ile Thr Asp Asn Val Met Lys His Leu
           595                 600                 605
   Leu Ser Pro Leu Phe Gln Arg Ser Ser Leu Gly Ala Arg Tyr Thr Gly
       610                 615                 620
   Cys Arg Val Ile Ala Leu Arg Ser Val Lys Asn Gly Ala Glu Thr Arg
   625                 630                 635                 640
   Val Asp Leu Leu Cys Thr Tyr Leu Gln Pro Leu Ser Gly Pro Gly Leu
                   645                 650                 655
   Pro Ile Lys Gln Val Phe His Glu Leu Ser Gln Gln Thr His Gly Ile
                   660                 665                 670
   Thr Arg Leu Gly Pro Tyr Ser Leu Asp Lys Asp Ser Leu Tyr Leu Asn
           675                 680                 685
   Gly Tyr Asn Glu Pro Gly Leu Asp Glu Pro Pro Thr Thr Pro Lys Pro
       690                 695                 700
   Ala Thr Thr Phe Leu Pro Pro Leu Ser Glu Ala Thr Thr Ala Met Gly
   705                 710                 715                 720
   Tyr His Leu Lys Thr Leu Thr Leu Asn Phe Thr Ile Ser Asn Leu Gln
                   725                 730                 735
   Tyr Ser Pro Asp Met Gly Lys Gly Ser Ala Thr Phe Asn Ser Thr Glu
               740                 745                 750
   Gly Val Leu Gln His Leu Leu Arg Pro Leu Phe Gln Lys Ser Ser Met
           755                 760                 765
   Gly Pro Phe Tyr Leu Gly Cys Gln Leu Ile Ser Leu Arg Pro Glu Lys
       770                 775                 780
   Asp Gly Ala Ala Thr Gly Val Asp Thr Thr Cys Thr Tyr His Pro Asp
   785                 790                 795                 800
   Pro Val Gly Pro Gly Leu Asp Ile Gln Gln Leu Tyr Trp Glu Leu Ser
                   805                 810                 815
   Gln Leu Thr His Gly Val Thr Gln Leu Gly Phe Tyr Val Leu Asp Arg
               820                 825                 830
   Asp Ser Leu Phe Ile Asn Gly Tyr Ala Pro Gln Asn Leu Ser Ile Arg
           835                 840                 845
   Gly Glu Tyr Gln Ile Asn Phe His Ile Val Asn Trp Asn Leu Ser Asn
       850                 855                 860
   Pro Asp Pro Thr Ser Ser Glu Tyr Ile Thr Leu Leu Arg Asp Ile Gln
   865                 870                 875                 880
   Asp Lys Val Thr Thr Leu Tyr Lys Gly Ser Gln Leu His Asp Thr Phe
                   885                 890                 895
   Arg Phe Cys Leu Val Thr Asn Leu Thr Met Asp Ser Val Leu Val Thr
               900                 905                 910
   Val Lys Ala Leu Phe Ser Ser Asn Leu Asp Pro Ser Leu Val Glu Gln
           915                 920                 925
   Val Phe Leu Asp Lys Thr Leu Asn Ala Ser Phe His Trp Leu Gly Ser
       930                 935                 940
   Thr Tyr Gln Leu Val Asp Ile His Val Thr Glu Met Glu Ser Ser Val
   945                 950                 955                 960
   Tyr Gln Pro Thr Ser Ser Ser Ser Thr Gln His Phe Tyr Pro Asn Phe
                   965                 970                 975
   Thr Ile Thr Asn Leu Pro Tyr Ser Gln Asp Lys Ala Gln Pro Gly Thr
               980                 985                 990
   Thr Asn Tyr Gln Arg Asn Lys Arg Asn Ile Glu Asp Ala Leu Asn Gln
           995                 1000                1005
   Leu Phe Arg Asn Ser Ser Ile Lys Ser Tyr Phe Ser Asp Cys Gln Val
       1010                1015                1020
   Ser Thr Phe Arg Ser Val Pro Asn Arg His His Thr Gly Val Asp Ser
   1025                1030                1035                1040
   Leu Cys Asn Phe Ser Pro Leu Ala Arg Arg Val Asp Arg Val Ala Ile
                   1045                1050                1055
   Tyr Glu Glu Phe Leu Arg Met Thr Arg Asn Gly Thr Gln Leu Gln Asn
               1060                1065                1070
   Phe Thr Leu Asp Arg Ser Ser Val Leu Val Asp Gly Tyr Ser Pro Asn
           1075                1080                1085
   Arg Asn Glu Pro Leu Thr Gly Asn Ser Asp Leu Pro Phe Trp Ala Val
       1090                1095                1100
   Ile Phe Ile Gly Leu Ala Gly Leu Leu Gly Leu Ile Thr Cys Leu Ile
   1105                1110                1115                1120
   Cys Gly Val Leu Val Thr Thr Arg Arg Arg Lys Lys Glu Gly Glu Tyr
                   1125                1130                1135
   Asn Val Gln Gln Gln Cys Pro Gly Tyr Tyr Gln Ser His Leu Asp Leu
               1140                1145                1150
   Glu Asp Leu Gln
           1155

<210> 460
<211> 79
<212> PRT
<213> Homo sapiens

<400> 460
Met Ser Met Val Ser His Ser Gly Ala Leu Cys Pro Pro Leu Ala Phe
1               5                   10                  15
Leu Gly Pro Pro Gln Trp Thr Trp Glu His Leu Gly Leu Gln Phe Leu
            20                  25                  30
Asn Leu Val Pro Arg Leu Pro Ala Leu Ser Trp Cys Tyr Ser Leu Ser
        35                  40                  45
Thr Ser Pro Ser Pro Thr Cys Gly Met Arg Arg Thr Cys Ser Thr Leu
    50                  55                  60
Ala Pro Gly Ser Ser Thr Pro Arg Arg Gly Ser Phe Arg Ala Trp
65                  70                  75

<210> 461
<211> 313
<212> PRT
<213> Homo sapiens

<400> 461
Met Pro Leu Phe Lys Asn Thr Ser Val Ser Ser Leu Tyr Ser Gly Cys
1               5                   10                  15
Arg Leu Thr Leu Leu Arg Pro Glu Lys Asp Gly Ala Ala Thr Arg Val
            20                  25                  30
Asp Ala Val Cys Thr His Arg Pro Asp Pro Lys Ser Pro Gly Leu Asp
        35                  40                  45
Arg Glu Arg Leu Tyr Trp Lys Leu Ser Gln Leu Thr His Gly Ile Thr
    50                  55                  60
Glu Leu Gly Pro Tyr Thr Leu Asp Arg His Ser Leu Tyr Val Asn Gly
65                  70                  75                  80
Phe Thr His Gln Ser Ser Met Thr Thr Thr Arg Thr Pro Asp Thr Ser
                85                  90                  95
Thr Met His Leu Ala Thr Ser Arg Thr Pro Ala Ser Leu Ser Gly Pro
            100                 105                 110
Thr Thr Ala Ser Pro Leu Leu Val Leu Phe Thr Ile Asn Phe Thr Ile
        115                 120                 125
Thr Asn Leu Arg Tyr Glu Glu Asn Met His His Pro Gly Ser Arg Lys
    130                 135                 140
Phe Asn Thr Thr Glu Arg Val Leu Gln Gly Leu Leu Arg Pro Val Phe
145                 150                 155                 160
Lys Asn Thr Ser Val Gly Pro Leu Tyr Ser Gly Cys Arg Leu Thr Leu
                165                 170                 175
Leu Arg Pro Lys Lys Asp Gly Ala Ala Thr Lys Val Asp Ala Ile Cys
            180                 185                 190
Thr Tyr Arg Pro Asp Pro Lys Ser Pro Gly Leu Asp Arg Glu Gln Leu
        195                 200                 205

Tyr Trp Glu Leu Ser Gln Leu Thr His Ser Ile Thr Glu Leu Gly Pro
    210                 215                 220
Tyr Thr Leu Asp Arg Asp Ser Leu Tyr Val Asn Gly Phe Thr Gln Arg
225                 230                 235                 240
Ser Ser Val Pro Thr Thr Ser Ile Pro Gly Thr Pro Thr Val Asp Leu
                245                 250                 255
Gly Thr Ser Gly Thr Pro Val Ser Lys Pro Gly Pro Ser Ala Ala Ser
            260                 265                 270
Pro Leu Leu Val Leu Phe Thr Leu Asn Phe Thr Ile Thr Asn Leu Arg
        275                 280                 285
Tyr Glu Glu Asn Met Gln His Pro Gly Ser Arg Lys Phe Asn Thr Thr
    290                 295                 300
Glu Arg Val Leu Gln Gly Leu Leu Arg
305                 310

<210> 462
<211> 2996
<212> DNA
<213> Homo sapiens

<400> 462
cagccaccgg agtggatgcc atctgcaccc accgccctga ccccacaggc cctgggctgg 60
acagagagca gctgtatttg gagctgagcc agctgaccca cagcatcact gagctgggcc 120
cctacaccct ggacagggac agtctctatg tcaatggttt cacacagcgg agctctgtgc 180
ccaccactag cattcctggg acccccacag tggacctggg aacatctggg actccagttt 240
ctaaacctgg tccctcggct gccagccctc tcctggtgct attcactctc aacttcacca 300
tcaccaacct gcggtatgag gagaacatgc agcaccctgg ctccaggaag ttcaacacca 360
cggagagggt ccttcagggc ctggtccctg ttcaagagca ccagtgttgg ccctctgtac 420
tctggctgca gactgacttt gctcaggcct gaaaaggatg ggacagccac tggagtggat 480
gccatctgca cccaccaccc tgaccccaaa agccctaggc tggacagaga gcagctgtat 540
tgggagctga gccagctgac ccacaatatc actgagctgg gcccctatgc cctggacaac 600
gacagcctct ttgtcaatgg tttcactcat cggagctctg tgtccaccac cagcactcct 660
gggaccccca cagtgtatct gggagcatct aagactccag cctcgatatt tggcccttca 720
gctgccagcc atctcctgat actattcacc ctcaacttca ccatcactaa cctgcggtat 780
gaggagaaca tgtggcctgg ctccaggaag ttcaacacta cagagagggt ccttcagggc 840
ctgctaaggc ccttgttcaa gaacaccagt gttggccctc tgtactctgg ctgcaggctg 900
accttgctca ggccagagaa agatggggaa gccaccggag tggatgccat ctgcacccac 960
cgccctgacc ccacaggccc tgggctggac agagagcagc tgtatttgga gctgagccag 1020
ctgacccaca gcatcactga gctgggcccc tacacactgg acagggacag tctctatgtc 1080
aatggtttca cccatcggag ctctgtaccc accaccagca ccggggtggt cagcgaggag 1140
ccattcacac tgaacttcac catcaacaac ctgcgctaca tggcggacat gggccaaccc 1200
ggctccctca agttcaacat cacagacaac gtcatgaagc acctgctcag tcctttgttc 1260
cagaggagca gcctgggtgc acggtacaca ggctgcaggg tcatcgcact aaggtctgtg 1320
aagaacggtg ctgagacacg ggtggacctc ctctgcacct acctgcagcc cctcagcggc 1380
ccaggtctgc ctatcaagca ggtgttccat gagctgagcc agcagaccca tggcatcacc 1440
cggctgggcc cctactctct ggacaaagac agcctctacc ttaacggtta caatgaacct 1500
ggtccagatg agcctcctac aactcccaag ccagccacca cattcctgcc tcctctgtca 1560
gaagccacaa cagccatggg gtaccacctg aagaccctca cactcaactt caccatctcc 1620
aatctccagt attcaccaga tatgggcaag ggctcagcta cattcaactc caccgagggg 1680
gtccttcagc acctgctcag acccttgttc cagaagagca gcatgggccc cttctacttg 1740
ggttgccaac tgatctccct caggcctgag aaggatgggg cagccactgg tgtggacacc 1800
acctgcacct accaccctga ccctgtgggc cccgggctgg acatacagca gctttactgg 1860
gagctgagtc agctgaccca tggtgtcacc caactgggct tctatgtcct ggacagggat 1920
agcctcttca tcaatggcta tgcaccccag aatttatcaa tccggggcga gtaccagata 1980
aatttccaca ttgtcaactg gaacctcagt aatccagacc ccacatcctc agagtacatc 2040
accctgctga gggacatcca ggacaaggtc accacactct acaaaggcag tcaactacat 2100

gacacattcc gcttctgcct ggtcaccaac ttgacgatgg actccgtgtt ggtcactgtc 2160
aaggcattgt tctcctccaa tttggacccc agcctggtgg agcaagtctt tctagataag 2220
accctgaatg cctcattcca ttggctgggc tccacctacc agttggtgga catccatgtg 2280
acagaaatgg agtcatcagt ttatcaacca acaagcagct ccagcaccca gcacttctac 2340
ctgaatttca ccatcaccaa cctaccatat tcccaggaca aagcccagcc aggcaccacc 2400
aattaccaga ggaacaaaag gaatattgag gatgcgctca accaactctt ccgaaacagc 2460
agcatcaaga gttatttttc tgactgtcaa gtttcaacat tcaggtctgt ccccaacagg 2520
caccacaccg gggtggactc cctgtgtaac ttctcgccac tggctcggag agtagacaga 2580
gttgccatct atgaggaatt tctgcggatg acccggaatg gtacccagct gcagaacttc 2640
accctggaca ggagcagtgt ccttgtggat gggtattttc ccaacagaaa tgagccctta 2700
actgggaatt ctgaccttcc cttctgggct gtcatcctca tcggcttggc aggactcctg 2760
ggactcatca catgcctgat ctgcggtgtc ctggtgacca cccgccggcg gaagaaggaa 2820
ggagaataca acgtccagca acagtgccca ggctactacc agtcacacct agacctggag 2880
gatctgcaat gactggaact tgccggtgcc tggggtgcct ttcccccagc cagggtccaa 2940
agaagcttgg ctggggcaga aataaaccat attggtcgga cacaaaaaaa aaaaaa 2996

<210> 463
<211> 3557
<212> DNA
<213> Homo sapiens

<400> 463
gagagggtcc ttcagggtct gcttatgccc ttgttcaaga acaccagtgt cagctctctg 60
tactctggtt gcagactgac cttgctcagg cctgagaagg atggggcagc caccagagtg 120
gatgctgtct gcacccatcg tcctgacccc aaaagccctg gactggacag agagcggctg 180
tactggaagc tgagccagct gacccacggc atcactgagc tgggccccta caccctggac 240
aggcacagtc tctatgtcaa tggtttcacc catcagagct ctatgacgac caccagaact 300
cctgatacct ccacaatgca cctggcaacc tcgagaactc cagcctccct gtctggacct 360
acgaccgcca gccctctcct ggtgctattc acaattaact tcaccatcac taacctgcgg 420
tatgaggaga acatgcatca ccctggctct agaaagttta acaccacgga gagagtcctt 480
cagggtctgc tcaggcctgt gttcaagaac accagtgttg gccctctgta ctctggctgc 540
agactgacct tgctcaggcc caagaaggat ggggcagcca ccaaagtgga tgccatctgc 600
acctaccgcc ctgatcccaa aagccctgga ctggacagag agcagctata ctgggagctg 660
agccagctaa cccacagcat cactgagctg ggcccctaca ccctggacag ggacagtctc 720
tatgtcaatg gtttcacaca gcggagctct gtgcccacca ctagcattcc tgggaccccc 780
acagtggacc tgggaacatc tgggactcca gtttctaaac ctggtccctc ggctgccagc 840
cctctcctgg tgctattcac tctcaacttc accatcacca acctgcggta tgaggagaac 900
atgcagcacc ctggctccag gaagttcaac accacggaga gggtccttca gggcctgctc 960
aggtccctgt tcaagagcac cagtgttggc cctctgtact ctggctgcag actgactttg 1020
ctcaggcctg aaaaggatgg gacagccact ggagtggatg ccatctgcac ccaccaccct 1080
gaccccaaaa gccctaggct ggacagagag cagctgtatt gggagctgag ccagctgacc 1140
cacaatatca ctgagctggg ccactatgcc ctggacaacg acagcctctt tgtcaatggt 1200
ttcactcatc ggagctctgt gtccaccacc agcactcctg ggacccccac agtgtatctg 1260
ggagcatcta agactccagc ctcgatattt ggcccttcag ctgccagcca tctcctgata 1320
ctattcaccc tcaacttcac catcactaac ctgcggtatg aggagaacat gtggcctggc 1380
tccaggaagt tcaacactac agagagggtc cttcagggcc tgctaaggcc cttgttcaag 1440
aacaccagtg ttggccctct gtactctggc tccaggctga ccttgctcag gccagagaaa 1500
gatggggaag ccaccggagt ggatgccatc tgcacccacc gccctgaccc cacaggccct 1560
gggctggaca gagagcagct gtatttggag ctgagccagc tgacccacag catcactgag 1620
ctgggcccct acacactgga cagggacagt ctctatgtca atggtttcac ccatcggagc 1680
tctgtaccca ccaccagcac cggggtggtc agcgaggagc cattcacact gaacttcacc 1740
atcaacaacc tgcgctacat ggcggacatg ggccaacccg gctccctcaa gttcaacatc 1800
acagacaacg tcatgaagca cctgctcagt cctttgttcc agaggagcag cctgggtgca 1860
cggtacacag gctgcagggt catcgcacta aggtctgtga agaacggtgc tgagacacgg 1920
gtggacctcc tctgcaccta cctgcagccc ctcagcggcc caggtctgcc tatcaagcag 1980
gtgttccatg agctgagcca gcagacccat ggcatcaccc ggctgggccc ctactctctg 2040
gacaaagaca gcctctacct taacggttac aatgaacctg gtctagatga gcctcctaca 2100

actcccaagc cagccaccac attcctgcct cctctgtcag aagccacaac agccatgggg 2160
taccacctga agaccctcac actcaacttc accatctcca atctccagta ttcaccagat 2220
atgggcaagg gctcagctac attcaactcc accgaggggg tccttcagca cctgctcaga 2280
cccttgttcc agaagagcag catgggcccc ttctacttgg gttgccaact gatctccctc 2340
aggcctgaga aggatggggc agccactggt gtggacacca cctgcaccta ccaccctgac 2400
cctgtgggcc ccgggctgga catacagcag ctttactggg agctgagtca gctgacccat 2460
ggtgtcaccc aactgggctt ctatgtcctg gacagggata gcctcttcat caatggctat 2520
gcaccccaga atttatcaat ccggggcgag taccagataa atttccacat tgtcaactgg 2580
aacctcagta atccagaccc cacatcctca gagtacatca ccctgctgag ggacatccag 2640
gacaaggtca ccacactcta caaaggcagt caactacatg acacattccg cttctgcctg 2700
gtcaccaact tgacgatgga ctccgtgttg gtcactgtca aggcattgtt ctcctccaat 2760
ttggacccca gcctggtgga gcaagtcttt ctagataaga ccctgaatgc ctcattccat 2820
tggctgggct ccacctacca gttggtggac atccatgtga cagaaatgga gtcatcagtt 2880
tatcaaccaa caagcagctc cagcacccag cacttctacc cgaatttcac catcaccaac 2940
ctaccatatt cccaggacaa agcccagcca ggcaccacca attaccagag gaacaaaagg 3000
aatattgagg atgcgctcaa ccaactcttc cgaaacagca gcatcaagag ttatttttct 3060
gactgtcaag tttcaacatt caggtctgtc cccaacaggc accacaccgg ggtggactcc 3120
ctgtgtaact tctcgccact ggctcggaga gtagacagag ttgccatcta tgaggaattt 3180
ctgcggatga cccggaatgg tacccagctg cagaacttca ccctggacag gagcagtgtc 3240
cttgtggatg ggtattctcc caacagaaat gagcccttaa ctgggaattc tgaccttccc 3300
ttctgggctg tcatcttcat cggcttggca ggactcctgg gactcatcac atgcctgatc 3360
tgcggtgtcc tggtgaccac ccgccggcgg aagaaggaag gagaatacaa cgtccagcaa 3420
cagtgcccag gctactacca gtcacaccta gacctggagg atctgcaatg actggaactt 3480
gccggtgcct ggggtgcctt tcccccagcc agggtccaaa gaagcttggc tggggcagaa 3540
ataaaccata ttggtcg 3557

<210> 464
<211> 2712
<212> DNA
<213> Homo sapiens

<400> 464
aggacatgcg tcaccctggc tccaggaagt tcaacaccac agagagggtc ctgcagggtc 60
tgcttggtcc cttgttcaag aactccagtg tcggccctct gtactctggc tgcagactga 120
tctctctcag gtctgagaag gatggggcag ccactggagt ggatgccatc tgcacccacc 180
accttaaccc tcaaagcctg gactggacag ggagcagctg tactggcagc tgagccagat 240
gaccaatggc atcaaagagc tgggccccta caccctggac cggaacagtc tctacgtcaa 300
tggtttcacc catcggagct ctgggctcac caccagcact ccttggactt ccacagttga 360
ccttggaacc tcagggactc catcccccgt ccccagcccc acaactgctg gccctctcct 420
ggtgccattc accctaaact tcaccatcac caacctgcag tatgaggagg acatgcatcg 480
ccctggatct aggaagttca acgccacaga gagggtcctg cagggtctgc ttagtcccat 540
attcaagaac tccagtgttg gccctctgta ctctggctgc agactgacct ctctcaggcc 600
cgagaaggat ggggcagcaa ctggaatgga tgctgtctgc ctctaccacc ctaatcccaa 660
aagacctggg ctggacagag agcagctgta ctgggagcta agccagctga cccacaacat 720
cactgagctg ggcccctaca gcctggacag ggacagtctc tatgtcaatg gtttcaccca 780
tcagaactct gtgcccacca ccagtactcc tgggacctcc acagtgtact gggcaaccac 840
tgggactcca tcctccttcc ccggccacac agagcctggc cctctcctga taccattcac 900
attcaacttt accatcacca acctgcatta tgaggaaaac atgcaacacc ctggttccag 960
gaagttcaac gccacagaga gggtcctgca gggtctgctt agtcccatat tcaagaactc 1020
cagtgttggc cctctgtact ctggctgcag actgacctct ctcaggcccg agaaggatgg 1080
ggcagcaact ggaatggatg ctgtctgtct ctaccgaccc taatcccatc ggacctgggc 1140
tggacagaga gcagctgtac tgggagctga gccagctgac ccacgacatc actgagctgg 1200
gcccctacag ccctggacag ggacagtctc tatgtcaatg gtttcaccca tcagaactct 1260
gtgcccacca ccagtactcc tgggacctcc acagtgtact gggcaaccac tgggactcca 1320
tcctccttcc ccggccacac agagcctggc cctctcctga taccattcac tttcaacttt 1380
accatcacca acctgcatta tgaggaaaac atgcaacacc tggttccagg aagttcaaca 1440
ccacggagag ggttctgcag ggtctgctca cgcccttgtt caagaacacc agtgttggcc 1500

ctctgtactc tggctgcaga ctgaccttgc tcagacctga gaagcaggag gcagccactg 1560
gagtggacac catctgcact caccgccttg accctctaaa ccctggactg gacagagagc 1620
agctatactg ggagctgagc aaactgaccc gtggcatcat cgagctgggc ccctacctcc 1680
tggacagagg cagtctctat gtcaatggtt tcacccatcg gaactttgtg cccatcacca 1740
gcactcctgg gacctccaca gtacacctag gaacctctga aactccatcc tccctaccta 1800
gacccatagt gcctggccct ctcctggtgc cattcaccct caacttcacc atcaccaact 1860
tgcagtatga ggaggccatg cgacaccctg gctccaggaa gttcaatacc acggagaggg 1920
tcctacaggg tctgctcagg cccttgttca agaataccag tatcggccct ctgtactcca 1980
gctgcagact gaccttgctc aggccagaga aggacaaggc agccaccaga gtggatgcca 2040
tctgtaccca ccaccctgac cctcaaagcc ctggactgaa cagagagcag ctgtactggg 2100
agctgagcca gctgacccac ggcatcactg agctgggccc ctacaccctg gacaggcaca 2160
gtctctatgt caatggtttc acccatcaga gccccatacc aaccaccagc actcctgata 2220
cctccacaat gcacctggga acctcgagaa ctccagcctc cctgtctgga cctacgaccg 2280
ccagccctct cctggtgcta ttcacaatta acttcaccat cactaacctg cggtatgagg 2340
agaacatgca tcaccgctgg ctctagaaag tttaacacca cggagagagt ccttcagggt 2400
ctgctcaggc ctgtgttcaa agaacaccag tgttggccct ctgtactctg gctgcagact 2460
gaccttgctc aggcccgaga aggatggggc agccacgcaa agtggatgcc atctgcacct 2520
accgccctga tcccaaaagc cctggactgg acagagagca gctatactgg gagctgagcc 2580
agggtgatgc atgttctcct catatcgcag gttagtgatg gtgaagttaa ttgtgaatag 2640
caccaggaga gggctggcgg tcatgggtcc agacagggag cctggagttc tcgaggttgc 2700
caggtgcatg tc 2712

<210> 465
<211> 1175
<212> DNA
<213> Homo sapiens

<400> 465
gaggtatgct aactactact attatttagt aggctttgtt agaaacttct gttgttatag 60
tcaagggacg catggaaact tttatattta ttctctcttt aaatcctgtt gcatatgttt 120
agaagtaggc cttttggaaa tatataaagt tctccacttt tgaacatgtt gtttctttcc 180
cacctccacg acagctgcca gccctctcct ggtgctattc actctcaact tcaccatcac 240
caacctgcgg tatgaggaga acatgcagca ccctggctcc aggaagttca acactacaga 300
gagggtcctt cagggcctgc taaggccctt gttcaagaac accagtgttg gccctctgta 360
ctctggctgc aggctgacct tgctcaggcc agagaaagat ggggaagcca ccggagtgga 420
tgccatctgc acccaccgcc ctgaccccac aggccctggg ctggacagag agcagctgta 480
tttggagctg agccagctga cccacagcat cactgagctg ggcccctaca cactggacag 540
ggacagtctc tatgtcaatg gtttcaccca tcggagctct gtacccacca ccagcaccgg 600
ggtggtcagc gaggagccat tcacactgaa cttcaccatc aacaacctgc gctacatggc 660
ggacatgggc caacccggct ccctcaagtt caacatcaca gacaacgtca tgaagcacct 720
gctcagtcct ttgttccaga ggagcagcct gggtgcacgg tacacaggct gcagggtcat 780
cgcactaagg tctgtgaaga acggtgctga gacacgggtg gacctcctct gcacctacct 840
gcagcccctc agcggcccag gtctgcctat caagcaggtg ttccatgagc tgagccagca 900
gacccatggc atcacccggc tgggccccta ctctctggac aaagacagcc tctaccttaa 960
cggttacaat gaacctggtc cagatgagcc tcctacaact cccaagccag ccaccacatt 1020
cctgcctcct ctgtcagaag ccacaacagc catggggtac cacctgaaga ccctcacact 1080
caattcacat ctccaatctc cagtattcac cagatatggg caagggctca aggtacattc 1140
aatccaccga ggggggtcct tcagcaactg gtcag 1175

<210> 466
<211> 1959
<212> DNA
<213> Homo sapiens

<400> 466
catccccagc tcgaacagca gccacagtcc cattcatggt gccattcacc ctcaacttca 60
actcatcacc aacctgcagt acgaggagga catgcggcac ctggttccag gaagttcaac 120

gcgcacagag agagaactgc agggtcgtgc tcaaacccta gatcaggaat agcagtctgg 180
aatacctcta ttcaggctgc agactagcct cactcaggcc agagaaggat agctcagcca 240
cggcagtgga tgccatctgc acacatcgcc ctgaccctga agacctcgga ctggacagag 300
agcgactgta ctgggagctg agcaatctga caaatggcat ccaggagctg ggcccctaca 360
ccctggaccg gaacagtctc tatgtcaatg gtttcaccca tcgaagctct atgcccacca 420
ccagcactcc tgggacctcc acagtggatg tgggaacctc agggactcca tcctccagcc 480
ccagccccac gactgctggc cctctcctga tgccgttcac cctcaacttc accatcacca 540
acctgcagta cgaggaggac atgcgtcgca ctggctccag gaagttcaac accatggaga 600
gtgtcctgca gggtctgctc aagcccttgt tcaagaacac cagtgttggc cctctgtact 660
ctggctgcag attgaccttg ctcaggccca agaaagatgg ggcagccact ggagtggatg 720
ccatctgcac ccaccgcctt gaccccaaaa gccctggact caacagggag cagctgtact 780
gggagctaag caaactgacc aatgacattg aagagctggg cccctacacc ctggacagga 840
acagtctcta tgtcaatggt ttcacccatc agagctctgt gtccaccacc agcactcctg 900
ggacctccac agtggatctc agaacctcag tggactccat cctccctctc cagccccaca 960
attatggctg ctggccctct cctggtacca ttcaccctca acttcaccat caccaacctg 1020
cagtatgggg aggacatggg tcaccctggc tccaggaagt tcaacaccac agagagggtc 1080
ctgcagggtc tgcttggtcc catattcaag aacaccagtg ttggccctct gtactctggc 1140
tgcagactga cctctctcag gtccaagaag gatggagcag ccactggagt ggatgccatc 1200
tgcatccatc atcttgaccc caaaagccct ggactcaaca gagagcggct gtactgggag 1260
ctgagccaac tgaccaatgg catcaaagag ctgggcccct acaccctgga caggaacagt 1320
ctctatgtca atggtttcac ccatcggacc tctgtgccca ccaccagtac tcctgggacc 1380
tccacagtgt actgggcaac cactgggact ccatcctccc tccccgccac acagagcctg 1440
gccctctcct gataccattc acattcaact ttaccatcac ctacctgcat tatagaggaa 1500
aacatgcaac acccgtggtt ccaggaacga tgtcaacacc acaggagagg gttctgcagg 1560
gtcttcgctc acgcccattg ttacaagaac accagtagtt ggccctctgt actctggctg 1620
cagaatgacc ttgctcagac ctgagaagca ggaggcaaca cactggaatg gacaccatct 1680
gtatccacca gcgttagatc ccatcaggac ctggactgga cagagagcag gctatactgg 1740
gagctagagc cagctgaccc acagcatcac agagctggga ccctacagcc ctggataggg 1800
acagtctcta tgtcaatggc ttcaaccctt ggagctctgt gccaaccacc agcactcctg 1860
ggacctccac agtgcacctg gcaacctctg ggactccatc ctccctgcct ggccacacag 1920
cccctgtccc tctcttgata ccattcaccc tcaacttac 1959

<210> 467
<211> 1636
<212> DNA
<213> Homo sapiens

<400> 467
gacctcctct gcacctacct gcagcccctc agcggcccag gtctgcctat caagcaggtg 60
ttccatgagc tgagccagca gacccatggc atcacccggc tgggccccta ctctctggac 120
aaagacagcc tctaccttaa cggttacaat gaacctggtc cagatgagcc tcctacaact 180
cccaagccag ccaccacatt cctgcctcct ctgtcagaag ccacaacagc catggggtac 240
cacctgaaga ccctcacact caacttcacc atctccaatc tccagtattc accagatatg 300
ggcaagggct cagctacatt caactccacc gagggggtcc ttcagcacct gctcagaccc 360
ttgttccaga agagcagcat gggccccttc tacttgggtt gccaactgat ctccctcagg 420
cctgagaagg atggggcagc cactggtgtg gacaccacct gcacctacca ccctgaccct 480
gtgggccccg ggctggacat acagcagctt tactgggagc tgagtcagct gacccatggt 540
gtcacccaac tgggcttcta tgtcctggac agggatagcc tcttcatcaa tggctatgca 600
ccccagaatt tatcaatccg gggcgagtac cagataaatt tccacattgt caactggaac 660
ctcagtaatc cagaccccac atcctcagag tacatcaccc tgctgaggga catccaggac 720
aaggtcacca cactctacaa aggcagtcaa ctacatgaca cattccgctt ctgcctggtc 780
accaacttga cgatggactc cgtgttggtc actgtcaagg cattgttctc ctccaatttg 840
gaccccagcc tggtggagca agtctttcta gataagaccc tgaatgcctc attccattgg 900
ctgggctcca cctaccagtt ggtggacatc catgtgacag aaatggagtc atcagtttat 960
caaccaacaa gcagctccag cacccagcac ttctacctga atttcaccat caccaaccta 1020
ccatattccc aggacaaagc ccagccaggc accaccaatt accagaggaa caaaaggaat 1080
attgaggatg cgctcaacca actcttccga aacagcagca tcaagagtta tttttctgac 1140

tgtcaagttt caacattcag gtctgtcccc aacaggcacc acaccggggt ggactccctg 1200
tgtaacttct cgccactggc tcggagagta gacagagttg ccatctatga ggaatttctg 1260
cggatgaccc ggaatggtac ccagctgcag aacttcaccc tggacaggag cagtgtcctt 1320
gtggatgggt attctcccaa cagaaatgag cccttaactg ggaattctga ccttcccttc 1380
tgggctgtca tcctcatcgg cttggcagga ctcctgggac tcatcacatg cctgatctgc 1440
ggtgtcctgg tgaccacccg ccggcggaag aaggaaggag aatacaacgt ccagcaacag 1500
tgcccaggct actaccagtc acacctagac ctggaggatc tgcaatgact ggaacttgcc 1560
ggtgcctggg gtgcctttcc cccagccagg gtccaaagaa gcttggctgg ggcagaaata 1620
aaccatattg gtcgga 1636

<210> 468
<211> 231
<212> DNA
<213> Homo sapiens

<400> 468
actacatgac acattccgct tctgcctggt caccaacttg acaaatggag tcatcagttt 60
atcaaccaac aagcagctcc agcacccagc acttctacct gaatttcacc atcaccaacc 120
taccatattc ccaggacaaa gcccagccag gcaccaccaa ttaccagagg aacaaaagga 180
atattgagga tgcgctcaac caactcttcc gaaacagcag catcgagagt t 231

<210> 469
<211> 607
<212> DNA
<213> Homo sapiens

<400> 469
atgaagagct atcgctgtcc aggacataga agcccagttg ggtgacacca tgggtcagct 60
gactcagctc ccagtaaagc tgctgtatgt ccagcccggg gcccacaggg tcagggtggt 120
aggtgcaggt ggtgtccaca ccagtggctg ccccatcctt ctcaggccag gtgctgaagg 180
accccctcgg tggagttgaa tgtagctgag cccttgccca tatctggtga atactggaga 240
ttggagatgg tgaagttgag tgtgagggtc ttcaggtggt accccatggc tgttgtggct 300
tctgacagag gaggcaggaa tgtggtggct ggcttgggag ttgtaggagg ctcatctgga 360
ccaggttcat tgtaaccgtt aaggtagagg ctgtctttgt ccagagagta ggggcccagc 420
cgggtgatgc catgggtctg ctggctcagc tcatggaaca cctgcttgat aggcagacct 480
gggccgctga ggggctgcag gtaggtgcag aggaggtcca cccgtgtctc agcaccgttc 540
ttcacagacc ttagtgcgat gaccctgcag cctgtgtacc gtgcacccag gctgctcctc 600
tggaaca 607

<210> 470
<211> 981
<212> DNA
<213> Homo sapiens

<400> 470
ggtaaccaca gctgacccat ggcatcaaag agctgggccc ctacaccctg gacaggaaca 60
gtctctatgt caatggtttc acccatcgga gctctgtggc ccccaccagc actcctggga 120
cctccacagt ggaccttggg acctcaggga ctccatcctc cctccccagc cccacaacag 180
ctgttcctct cctggtgccg ttcaccctca actttaccat caccaatctg cagtatgggg 240
aggacatgcg tcaccctggc tccaggaagt tcaacaccac agagagggtc ctgcagggtc 300
tgcttggtcc cttgttcaag aactccagtg tcggccctct gtactctggc tgcagactga 360
tctctctcag gtctgagaag gatggggcag ccactggagt ggatgccatc tgcacccacc 420
accttaaccc tcaaagccct ggactggaca gggagcagct gtactggcag ctgagccaga 480
gaccacaacc tcatttatca cctattctga gacacacaca agttcagcca ttccaactct 540
ccctgtctcc ccctggtgca tcaaagatgc tgacctcact ggtcatcagt tctgggacag 600
acagcactac aactttccca acactgacgg agaccccata tgaaccagag acaacagcca 660
tacagctcat tcatcctgca gagaccaaca caatggttcc caggacaact cccaagtttt 720

cccatagtaa gtcagacacc acactcccag tagccatcac cagtcctggg ccagaagcca 780
gttcagctgt ttcaacgaca actatctcac ctgatatgtc agatctggtg acctcactgg 840
tccctagttc tgggacagac accagtacaa ccttcccaac attgagtgag accccatatg 900
aaccagagac tacagccacg tggctcactc atcctgcaga aaccagaaca acggtttctg 960
ggacaattcc caacttttcc c 981

<210> 471
<211> 959
<212> DNA
<213> Homo sapiens

<400> 471
cagatggcat ccactccggt ggcttcccca tctttctctg gcctgagcaa ggtcagcctg 60
cagccagagt acagagggcc aacactggtg ttcttgaaca agggccttag caggccctga 120
aggaccctct ctgtagtgtt gaacttcctg gagccaggcc acatgttctc ctcataccgc 180
aggttagtga tggtgaagtt gagggtgaat agtatcagga gatggctggc agctgaaggg 240
ccaaatatcg aggctggagt cttagatgct cccagataca ctgtgggggt cccaggagtg 300
ctggtggtgg acacagagct ccgatgagtg aaaccattga caaagaggct gtcgttgtcc 360
agggcatagg ggcccagctc agtgatattg tgggtcagct ggctcagctc ccaatacagc 420
tgctctctgt ccagcctagg gcttttgggg tcagggtggt gggtgcagat ggcatccact 480
ccagtggctg tcccatcctt ttcaggcctg agcaaagtca gtctgcagcc agagtacaga 540
gggccaacac tggtgctctt gaacagggac ctgagcaggc cctgaaggac cctctccgtg 600
gtgttgaact tcctggagcc agggtgctgc atgttctcct cataccgcag gttggtgatg 660
gtgaagttga gagtgaatag caccaggaga gggctggcag ccgagggacc aggtttagaa 720
actggagtcc cagatgttcc caggtccact gtgggggtcc caggaatgct agtggtgggc 780
acagagctcc gctgtgtgaa accattgaca tagagactgt ccctgtccag ggtgtagggg 840
cccagctcag tgatgctgtg ggttagctgg ctcagctccc agtatagctg ctctctgtcc 900
agtccagggc ttttgggatc agggcggtag gtgcagatgg catccacttt ggtggctgc 959

<210> 472
<211> 1315
<212> DNA
<213> Homo sapiens

<400> 472
ccaccgtctt gaccccaaaa gccctggagt ggacagggag cagctatact gggagctgag 60
ccagctgacc aatggcatca aagagctggg cccctacacc tggacaggaa cagtctctat 120
gtcaatggtt tcacccatcg gacctctgtg cccaccacca gcactcctgg gacctccaca 180
gtggaccttg gaacctcagg gactccattc tccctcccaa gccccgcaac tgctggccct 240
ctcctggtgc tgttcaccct caacttcacc atcaccaacc tgaagtatga ggaggacatg 300
catcgccctg gctccaggaa gttcaacacc actgagaggg tcctgcagac tctgcttggt 360
cctatgttca agaacaccag tgttggcctt ctgtactctg gctgcagact gaccttgctc 420
aggtccgaga aggatggagc agccactgga gtggatgcca tctgcaccca ccgtcttgac 480
cccaaaagcc ctggagtgga cagggagcag ctatactggg agctgagcca gctgaccaat 540
ggcatcaaag agctgggccc ctacaccctg gacaggaaca gtctctatgt caatggtttc 600
acccattgga tccctgtgcc caccagcagc actcctggga cctccacagt ggaccttggg 660
tcagggactc catcctccct ccccagcccc acaactgctg gccctctcct ggtgccgttc 720
accctcaact tcaccatcac caacctgaag tacgaggagg acatgcattg ccctggctcc 780
aggaagttca acaccacaga gagagtcctg cagagtctgc ttggtcccat gttcaagaac 840
accagtgttg gccctctgta ctctggctgc agactgacct tgctcaggtc cgagaaggat 900
ggagcagcca ctggagtgga tgccatctgc acccaccgtc ttgaccccaa aagcctggag 960
tggacaggga gcagctatac tgggagctga gccagctgac caatgccatc aaagagctgg 1020
gtccctacac cctggacagc aacagtcttc tatgtcaatg gtttcaccca tcagacctct 1080
gcgcccaaca ccagcactcc tgggacctcc acagtggacc ttgggacctc agggactcca 1140
tcctccctcc ccagccctac atctgctggc cctctcctgg tgccattcac cctcaacttc 1200
accatcacca acctgcagta cgaggaggac atgcatcacc caggctccag gaagttcaac 1260
accacggagc gggtcctgca gggtctgctt ggtcccatgt tcaagaacac tacga 1315

<210> 473
<211> 689
<212> DNA
<213> Homo sapiens

<400> 473
acggcatcag gagagggcca gcagtcgtgg ggctggggct ggaggatgga gtccctgagg 60
ttcccacatc cactgtggag gtcccaggag tgctggtggt gggcatagag cttcgatggg 120
tgaaaccatt gacatagaga ctgttccggt ccagggtgta ggggcccagc tcagtgatgc 180
cgtgggtcag ctggctcagc tcccagtaca gctgctctct gttcagtcca gggctttgag 240
ggtcagggtg gtgggtacag atggcatcca ctctggtggc tgccttgtcc ttctctggcc 300
ttgagcaagg tcagtctgca gcctgagagc taacagaggt ccgataactg gtattcttga 360
acaagggcct agagcagaac cctgtaggac catcgccgtg gtatatgaac ttcctagagc 420
caggatttcg cacggccatc actcatactg caacttgctg atggcaaagt tgaggataaa 480
cggcaccagg agagggccag ccacttatgg gtctaggtag ggaggatgga gtttcagagg 540
ttctcgagat ccactgtgga ggtcccagga gtgctggtgg tggacacaga gctctgatgg 600
gtgaaaccat tgacatagag actgttcctg tccagggtgt aggggcccag ctcttcaatg 660
tcattggtca gtttgcttag ctcccagta 689

<210> 474
<211> 495
<212> DNA
<213> Homo sapiens

<400> 474
gtggatatga gttgaatact cactgctggt ggtggacaca gagctctgat gggtgaaacc 60
tgcatagaga aggagggagg agagtgggta agagacaagg agaggtgggg gaccaaatgg 120
aggtcaatgc taccctggtg caatgaaccg agtttcatgg tacaggacaa attgaagatt 180
ttctatcagc atcctcacat caggaaagaa tgccctgagg gaacacagtc catgatggta 240
aggaaaccat gaagtccaga ccttagtcat cccatgtaga gcacatgaca gaattttcaa 300
aggccaggca gggagtgtga cctctagtta gagattagag gctgcccagc aagggggaag 360
agatttcaac cacatcacag ccactcacca ttgacataga gactgttcct gtccagggtg 420
taggggccca gctcttcaat gtcattggtc agtttgctta gctcccagta cagctgctcc 480
ctgttgagtc caggg 495

<210> 475
<211> 192
<212> DNA
<213> Homo sapiens

<400> 475
agtgcccagg ctactaccag tcacacctag acctggagga tctgcaatga ctggaacttg 60
ccggtgcctg gggatagcct cttcatcaat ggctatgcac cccagaattt atcaatccgg 120
ggcgagtacc agataaattt ccacattgtc aactggaacc tcagtaatcc agaccccaca 180
tcctcagagt ac 192

<210> 476
<211> 500
<212> DNA
<213> Homo sapiens

<400> 476
ccggtggctg ccccacgttt ttcaggcctg agcaaggtca gtctgcagcc agagtacaga 60
gggccaacac tggtgctctt gaacaagggc ttgagcagac cctgcaggac tctctccgtg 120
gtgttgaact tcctggaacc agggtgacgc atgtcctcct catactgcag gttggtgata 180
gtgaagttga gggtgaatgg caccaggaga gggccagggc tgtgtggcca gggaggaggg 240

ctggagtccc agaggtttcc aggtgcactg cagaggtccc aggaatactg gtggttggca 300
cagagctccg atgggtgaag ccattgacat agagactgtc cctgtccagg tgtaggggcc 360
cagctctgta acgctgttgg tcagctggct cagctcccag tatagccgct ctctgtccag 420
tccaggacca gtgggatcaa ggcggagggt gcagatggcg tccactccag tggctgcccc 480
atgtttctca ggtctgagca 500

<210> 477
<211> 191
<212> DNA
<213> Homo sapiens

<400> 477
gaggtatgct aactactact attatttagt aggctttgtt agaaacttct gttgttatag 60
tcaagggacg catggaaact ttttatatta ttctctcttt aaatcctgtt gcatatgttt 120
agaagtaggc cttttggaaa tatataaagt tctccacttt tgaacatgtt gtttctttcc 180
cacctccacg a 191

<210> 478
<211> 914
<212> PRT
<213> Homo sapiens

<400> 478
Met Ser Met Val Ser His Ser Gly Ala Leu Cys Pro Pro Leu Ala Phe
1               5                   10                  15
Leu Gly Pro Pro Gln Trp Thr Trp Glu His Leu Gly Leu Gln Phe Leu
            20                  25                  30
Asn Leu Val Pro Arg Leu Pro Ala Leu Ser Trp Cys Tyr Ser Leu Ser
        35                  40                  45
Thr Ser Pro Ser Pro Thr Cys Gly Met Arg Arg Thr Cys Ser Thr Leu
    50                  55                  60
Ala Pro Gly Ser Ser Thr Pro Arg Arg Gly Ser Phe Arg Ala Trp Ser
65                  70                  75                  80
Leu Phe Lys Ser Thr Ser Val Gly Pro Leu Tyr Ser Gly Cys Arg Leu
                85                  90                  95
Thr Leu Leu Arg Pro Glu Lys Asp Gly Thr Ala Thr Gly Val Asp Ala
            100                 105                 110
Ile Cys Thr His His Pro Asp Pro Lys Ser Pro Arg Leu Asp Arg Glu
        115                 120                 125
Gln Leu Tyr Trp Glu Leu Ser Gln Leu Thr His Asn Ile Thr Glu Leu
    130                 135                 140
Gly Pro Tyr Ala Leu Asp Asn Asp Ser Leu Phe Val Asn Gly Phe Thr
145                 150                 155                 160
His Arg Ser Ser Val Ser Thr Thr Ser Thr Pro Gly Thr Pro Thr Val
                165                 170                 175
Tyr Leu Gly Ala Ser Lys Thr Pro Ala Ser Ile Phe Gly Pro Ser Ala
            180                 185                 190
Ala Ser His Leu Leu Ile Leu Phe Thr Leu Asn Phe Thr Ile Thr Asn
        195                 200                 205
Leu Arg Tyr Glu Glu Asn Met Trp Pro Gly Ser Arg Lys Phe Asn Thr
    210                 215                 220
Thr Glu Arg Val Leu Gln Gly Leu Leu Arg Pro Leu Phe Lys Asn Thr
225                 230                 235                 240
Ser Val Gly Pro Leu Tyr Ser Gly Cys Arg Leu Thr Leu Leu Arg Pro
                245                 250                 255
Glu Lys Asp Gly Glu Ala Thr Gly Val Asp Ala Ile Cys Thr His Arg
            260                 265                 270

Pro Asp Pro Thr Gly Pro Gly Leu Asp Arg Glu Gln Leu Tyr Leu Glu
        275                 280                 285
Leu Ser Gln Leu Thr His Ser Ile Thr Glu Leu Gly Pro Tyr Thr Leu
    290                 295                 300
Asp Arg Asp Ser Leu Tyr Val Asn Gly Phe Thr His Arg Ser Ser Val
305                 310                 315                 320
Pro Thr Thr Ser Thr Gly Val Val Ser Glu Glu Pro Phe Thr Leu Asn
                325                 330                 335
Phe Thr Ile Asn Asn Leu Arg Tyr Met Ala Asp Met Gly Gln Pro Gly
            340                 345                 350
Ser Leu Lys Phe Asn Ile Thr Asp Asn Val Met Lys His Leu Leu Ser
        355                 360                 365
Pro Leu Phe Gln Arg Ser Ser Leu Gly Ala Arg Tyr Thr Gly Cys Arg
    370                 375                 380
Val Ile Ala Leu Arg Ser Val Lys Asn Gly Ala Glu Thr Arg Val Asp
385                 390                 395                 400
Leu Leu Cys Thr Tyr Leu Gln Pro Leu Ser Gly Pro Gly Leu Pro Ile
                405                 410                 415
Lys Gln Val Phe His Glu Leu Ser Gln Gln Thr His Gly Ile Thr Arg
            420                 425                 430
Leu Gly Pro Tyr Ser Leu Asp Lys Asp Ser Leu Tyr Leu Asn Gly Tyr
        435                 440                 445
Asn Glu Pro Gly Pro Asp Glu Pro Pro Thr Thr Pro Lys Pro Ala Thr
    450                 455                 460
Thr Phe Leu Pro Pro Leu Ser Glu Ala Thr Thr Ala Met Gly Tyr His
465                 470                 475                 480
Leu Lys Thr Leu Thr Leu Asn Phe Thr Ile Ser Asn Leu Gln Tyr Ser
                485                 490                 495
Pro Asp Met Gly Lys Gly Ser Ala Thr Phe Asn Ser Thr Glu Gly Val
            500                 505                 510
Leu Gln His Leu Leu Arg Pro Leu Phe Gln Lys Ser Ser Met Gly Pro
        515                 520                 525
Phe Tyr Leu Gly Cys Gln Leu Ile Ser Leu Arg Pro Glu Lys Asp Gly
    530                 535                 540
Ala Ala Thr Gly Val Asp Thr Thr Cys Thr Tyr His Pro Asp Pro Val
545                 550                 555                 560
Gly Pro Gly Leu Asp Ile Gln Gln Leu Tyr Trp Glu Leu Ser Gln Leu
                565                 570                 575
Thr His Gly Val Thr Gln Leu Gly Phe Tyr Val Leu Asp Arg Asp Ser
            580                 585                 590
Leu Phe Ile Asn Gly Tyr Ala Pro Gln Asn Leu Ser Ile Arg Gly Glu
        595                 600                 605
Tyr Gln Ile Asn Phe His Ile Val Asn Trp Asn Leu Ser Asn Pro Asp
    610                 615                 620
Pro Thr Ser Ser Glu Tyr Ile Thr Leu Leu Arg Asp Ile Gln Asp Lys
625                 630                 635                 640
Val Thr Thr Leu Tyr Lys Gly Ser Gln Leu His Asp Thr Phe Arg Phe
                645                 650                 655
Cys Leu Val Thr Asn Leu Thr Met Asp Ser Val Leu Val Thr Val Lys
            660                 665                 670
Ala Leu Phe Ser Ser Asn Leu Asp Pro Ser Leu Val Glu Gln Val Phe
        675                 680                 685
Leu Asp Lys Thr Leu Asn Ala Ser Phe His Trp Leu Gly Ser Thr Tyr
    690                 695                 700
Gln Leu Val Asp Ile His Val Thr Glu Met Glu Ser Ser Val Tyr Gln
705                 710                 715                 720
Pro Thr Ser Ser Ser Ser Thr Gln His Phe Tyr Leu Asn Phe Thr Ile

725                 730                 735
Thr Asn Leu Pro Tyr Ser Gln Asp Lys Ala Gln Pro Gly Thr Thr Asn
            740                 745                 750
Tyr Gln Arg Asn Lys Arg Asn Ile Glu Asp Ala Leu Asn Gln Leu Phe
        755                 760                 765
Arg Asn Ser Ser Ile Lys Ser Tyr Phe Ser Asp Cys Gln Val Ser Thr
    770                 775                 780
Phe Arg Ser Val Pro Asn Arg His His Thr Gly Val Asp Ser Leu Cys
785                 790                 795                 800
Asn Phe Ser Pro Leu Ala Arg Arg Val Asp Arg Val Ala Ile Tyr Glu
                805                 810                 815
Glu Phe Leu Arg Met Thr Arg Asn Gly Thr Gln Leu Gln Asn Phe Thr
            820                 825                 830
Leu Asp Arg Ser Ser Val Leu Val Asp Gly Tyr Phe Pro Asn Arg Asn
        835                 840                 845
Glu Pro Leu Thr Gly Asn Ser Asp Leu Pro Phe Trp Ala Val Ile Leu
    850                 855                 860
Ile Gly Leu Ala Gly Leu Leu Gly Leu Ile Thr Cys Leu Ile Cys Gly
865                 870                 875                 880
Val Leu Val Thr Thr Arg Arg Arg Lys Lys Glu Gly Glu Tyr Asn Val
                885                 890                 895
Gln Gln Gln Cys Pro Gly Tyr Tyr Gln Ser His Leu Asp Leu Glu Asp
            900                 905                 910
Leu Gln

<210> 479
<211> 1148
<212> PRT
<213> Homo sapiens

<400> 479
Met Pro Leu Phe Lys Asn Thr Ser Val Ser Ser Leu Tyr Ser Gly Cys
1               5                   10                  15
Arg Leu Thr Leu Leu Arg Pro Glu Lys Asp Gly Ala Ala Thr Arg Val
            20                  25                  30
Asp Ala Val Cys Thr His Arg Pro Asp Pro Lys Ser Pro Gly Leu Asp
        35                  40                  45
Arg Glu Arg Leu Tyr Trp Lys Leu Ser Gln Leu Thr His Gly Ile Thr
    50                  55                  60
Glu Leu Gly Pro Tyr Thr Leu Asp Arg His Ser Leu Tyr Val Asn Gly
65                  70                  75                  80
Phe Thr His Gln Ser Ser Met Thr Thr Thr Arg Thr Pro Asp Thr Ser
                85                  90                  95
Thr Met His Leu Ala Thr Ser Arg Thr Pro Ala Ser Leu Ser Gly Pro
            100                 105                 110
Thr Thr Ala Ser Pro Leu Leu Val Leu Phe Thr Ile Asn Phe Thr Ile
        115                 120                 125
Thr Asn Leu Arg Tyr Glu Glu Asn Met His His Pro Gly Ser Arg Lys
    130                 135                 140
Phe Asn Thr Thr Glu Arg Val Leu Gln Gly Leu Leu Arg Pro Val Phe
145                 150                 155                 160
Lys Asn Thr Ser Val Gly Pro Leu Tyr Ser Gly Cys Arg Leu Thr Leu
                165                 170                 175
Leu Arg Pro Lys Lys Asp Gly Ala Ala Thr Lys Val Asp Ala Ile Cys
            180                 185                 190

Thr Tyr Arg Pro Asp Pro Lys Ser Pro Gly Leu Asp Arg Glu Gln Leu
        195                 200                 205
Tyr Trp Glu Leu Ser Gln Leu Thr His Ser Ile Thr Glu Leu Gly Pro
    210                 215                 220
Tyr Thr Leu Asp Arg Asp Ser Leu Tyr Val Asn Gly Phe Thr Gln Arg
225                 230                 235                 240
Ser Ser Val Pro Thr Thr Ser Ile Pro Gly Thr Pro Thr Val Asp Leu
                245                 250                 255
Gly Thr Ser Gly Thr Pro Val Ser Lys Pro Gly Pro Ser Ala Ala Ser
            260                 265                 270
Pro Leu Leu Val Leu Phe Thr Leu Asn Phe Thr Ile Thr Asn Leu Arg
        275                 280                 285
Tyr Glu Glu Asn Met Gln His Pro Gly Ser Arg Lys Phe Asn Thr Thr
    290                 295                 300
Glu Arg Val Leu Gln Gly Leu Leu Arg Ser Leu Phe Lys Ser Thr Ser
305                 310                 315                 320
Val Gly Pro Leu Tyr Ser Gly Cys Arg Leu Thr Leu Leu Arg Pro Glu
                325                 330                 335
Lys Asp Gly Thr Ala Thr Gly Val Asp Ala Ile Cys Thr His His Pro
            340                 345                 350
Asp Pro Lys Ser Pro Arg Leu Asp Arg Glu Gln Leu Tyr Trp Glu Leu
        355                 360                 365
Ser Gln Leu Thr His Asn Ile Thr Glu Leu Gly His Tyr Ala Leu Asp
    370                 375                 380
Asn Asp Ser Leu Phe Val Asn Gly Phe Thr His Arg Ser Ser Val Ser
385                 390                 395                 400
Thr Thr Ser Thr Pro Gly Thr Pro Thr Val Tyr Leu Gly Ala Ser Lys
                405                 410                 415
Thr Pro Ala Ser Ile Phe Gly Pro Ser Ala Ala Ser His Leu Leu Ile
            420                 425                 430
Leu Phe Thr Leu Asn Phe Thr Ile Thr Asn Leu Arg Tyr Glu Glu Asn
        435                 440                 445
Met Trp Pro Gly Ser Arg Lys Phe Asn Thr Thr Glu Arg Val Leu Gln
    450                 455                 460
Gly Leu Leu Arg Pro Leu Phe Lys Asn Thr Ser Val Gly Pro Leu Tyr
465                 470                 475                 480
Ser Gly Ser Arg Leu Thr Leu Leu Arg Pro Glu Lys Asp Gly Glu Ala
                485                 490                 495
Thr Gly Val Asp Ala Ile Cys Thr His Arg Pro Asp Pro Thr Gly Pro
            500                 505                 510
Gly Leu Asp Arg Glu Gln Leu Tyr Leu Glu Leu Ser Gln Leu Thr His
        515                 520                 525
Ser Ile Thr Glu Leu Gly Pro Tyr Thr Leu Asp Arg Asp Ser Leu Tyr
    530                 535                 540
Val Asn Gly Phe Thr His Arg Ser Ser Val Pro Thr Thr Ser Thr Gly
545                 550                 555                 560
Val Val Ser Glu Glu Pro Phe Thr Leu Asn Phe Thr Ile Asn Asn Leu
                565                 570                 575
Arg Tyr Met Ala Asp Met Gly Gln Pro Gly Ser Leu Lys Phe Asn Ile
            580                 585                 590
Thr Asp Asn Val Met Lys His Leu Leu Ser Pro Leu Phe Gln Arg Ser
        595                 600                 605
Ser Leu Gly Ala Arg Tyr Thr Gly Cys Arg Val Ile Ala Leu Arg Ser
    610                 615                 620
Val Lys Asn Gly Ala Glu Thr Arg Val Asp Leu Leu Cys Thr Tyr Leu
625                 630                 635                 640
Gln Pro Leu Ser Gly Pro Gly Leu Pro Ile Lys Gln Val Phe His Glu

645                 650                 655
Leu Ser Gln Gln Thr His Gly Ile Thr Arg Leu Gly Pro Tyr Ser Leu
            660                 665                 670
Asp Lys Asp Ser Leu Tyr Leu Asn Gly Tyr Asn Glu Pro Gly Leu Asp
        675                 680                 685
Glu Pro Pro Thr Thr Pro Lys Pro Ala Thr Thr Phe Leu Pro Pro Leu
    690                 695                 700
Ser Glu Ala Thr Thr Ala Met Gly Tyr His Leu Lys Thr Leu Thr Leu
705                 710                 715                 720
Asn Phe Thr Ile Ser Asn Leu Gln Tyr Ser Pro Asp Met Gly Lys Gly
                725                 730                 735
Ser Ala Thr Phe Asn Ser Thr Glu Gly Val Leu Gln His Leu Leu Arg
            740                 745                 750
Pro Leu Phe Gln Lys Ser Ser Met Gly Pro Phe Tyr Leu Gly Cys Gln
        755                 760                 765
Leu Ile Ser Leu Arg Pro Glu Lys Asp Gly Ala Ala Thr Gly Val Asp
    770                 775                 780
Thr Thr Cys Thr Tyr His Pro Asp Pro Val Gly Pro Gly Leu Asp Ile
785                 790                 795                 800
Gln Gln Leu Tyr Trp Glu Leu Ser Gln Leu Thr His Gly Val Thr Gln
                805                 810                 815
Leu Gly Phe Tyr Val Leu Asp Arg Asp Ser Leu Phe Ile Asn Gly Tyr
            820                 825                 830
Ala Pro Gln Asn Leu Ser Ile Arg Gly Glu Tyr Gln Ile Asn Phe His
        835                 840                 845
Ile Val Asn Trp Asn Leu Ser Asn Pro Asp Pro Thr Ser Ser Glu Tyr
    850                 855                 860
Ile Thr Leu Leu Arg Asp Ile Gln Asp Lys Val Thr Thr Leu Tyr Lys
865                 870                 875                 880
Gly Ser Gln Leu His Asp Thr Phe Arg Phe Cys Leu Val Thr Asn Leu
                885                 890                 895
Thr Met Asp Ser Val Leu Val Thr Val Lys Ala Leu Phe Ser Ser Asn
            900                 905                 910
Leu Asp Pro Ser Leu Val Glu Gln Val Phe Leu Asp Lys Thr Leu Asn
        915                 920                 925
Ala Ser Phe His Trp Leu Gly Ser Thr Tyr Gln Leu Val Asp Ile His
    930                 935                 940
Val Thr Glu Met Glu Ser Ser Val Tyr Gln Pro Thr Ser Ser Ser Ser
945                 950                 955                 960
Thr Gln His Phe Tyr Pro Asn Phe Thr Ile Thr Asn Leu Pro Tyr Ser
                965                 970                 975
Gln Asp Lys Ala Gln Pro Gly Thr Thr Asn Tyr Gln Arg Asn Lys Arg
            980                 985                 990
Asn Ile Glu Asp Ala Leu Asn Gln Leu Phe Arg Asn Ser Ser Ile Lys
        995                 1000                1005
Ser Tyr Phe Ser Asp Cys Gln Val Ser Thr Phe Arg Ser Val Pro Asn
    1010                1015                1020
Arg His His Thr Gly Val Asp Ser Leu Cys Asn Phe Ser Pro Leu Ala
1025                1030                1035                1040
Arg Arg Val Asp Arg Val Ala Ile Tyr Glu Glu Phe Leu Arg Met Thr
                1045                1050                1055
Arg Asn Gly Thr Gln Leu Gln Asn Phe Thr Leu Asp Arg Ser Ser Val
            1060                1065                1070
Leu Val Asp Gly Tyr Ser Pro Asn Arg Asn Glu Pro Leu Thr Gly Asn
        1075                1080                1085
Ser Asp Leu Pro Phe Trp Ala Val Ile Phe Ile Gly Leu Ala Gly Leu
    1090                1095                1100

Leu Gly Leu Ile Thr Cys Leu Ile Cys Gly Val Leu Val Thr Thr Arg
1105                1110                1115                1120
Arg Arg Lys Lys Glu Gly Glu Tyr Asn Val Gln Gln Gln Cys Pro Gly
                1125                1130                1135
Tyr Tyr Gln Ser His Leu Asp Leu Glu Asp Leu Gln
            1140                1145

<210> 480
<211> 230
<212> PRT
<213> Homo sapiens

<400> 480
Met His Arg Pro Gly Ser Arg Lys Phe Asn Thr Thr Glu Arg Val Leu
 1               5                  10                  15
Gln Thr Leu Leu Gly Pro Met Phe Lys Asn Thr Ser Val Gly Leu Leu
            20                  25                  30
Tyr Ser Gly Cys Arg Leu Thr Leu Leu Arg Ser Glu Lys Asp Gly Ala
        35                  40                  45
Ala Thr Gly Val Asp Ala Ile Cys Thr His Arg Leu Asp Pro Lys Ser
    50                  55                  60
Pro Gly Val Asp Arg Glu Gln Leu Tyr Trp Glu Leu Ser Gln Leu Thr
65                  70                  75                  80
Asn Gly Ile Lys Glu Leu Gly Pro Tyr Thr Leu Asp Arg Asn Ser Leu
                85                  90                  95
Tyr Val Asn Gly Phe Thr His Trp Ile Pro Val Pro Thr Ser Ser Thr
            100                 105                 110
Pro Gly Thr Ser Thr Val Asp Leu Gly Ser Gly Thr Pro Ser Ser Leu
        115                 120                 125
Pro Ser Pro Thr Thr Ala Gly Pro Leu Leu Val Pro Phe Thr Leu Asn
    130                 135                 140
Phe Thr Ile Thr Asn Leu Lys Tyr Glu Glu Asp Met His Cys Pro Gly
145                 150                 155                 160
Ser Arg Lys Phe Asn Thr Thr Glu Arg Val Leu Gln Ser Leu Leu Gly
                165                 170                 175
Pro Met Phe Lys Asn Thr Ser Val Gly Pro Leu Tyr Ser Gly Cys Arg
            180                 185                 190
Leu Thr Leu Leu Arg Ser Glu Lys Asp Gly Ala Ala Thr Gly Val Asp
        195                 200                 205
Ala Ile Cys Thr His Arg Leu Asp Pro Lys Ser Leu Glu Trp Thr Gly
    210                 215                 220
Ser Ser Tyr Thr Gly Ser
225                 230

<210> 481
<211> 210
<212> PRT
<213> Homo sapiens

<400> 481
Met Gln His Pro Gly Ser Arg Lys Phe Asn Thr Thr Glu Arg Val Leu
 1               5                  10                  15
Gln Gly Leu Leu Arg Ser Leu Phe Lys Ser Thr Ser Val Gly Pro Leu
            20                  25                  30
Tyr Ser Gly Cys Arg Leu Thr Leu Leu Arg Pro Glu Lys Asp Gly Thr

35                  40                  45
Ala Thr Gly Val Asp Ala Ile Cys Thr His His Pro Asp Pro Lys Ser
    50                  55                  60
Pro Arg Leu Asp Arg Glu Gln Leu Tyr Trp Glu Leu Ser Gln Leu Thr
65                  70                  75                  80
His Asn Ile Thr Glu Leu Gly Pro Tyr Ala Leu Asp Asn Asp Ser Leu
                85                  90                  95
Phe Val Asn Gly Phe Thr His Arg Ser Ser Val Ser Thr Thr Ser Thr
            100                 105                 110
Pro Gly Thr Pro Thr Val Tyr Leu Gly Ala Ser Lys Thr Pro Ala Ser
        115                 120                 125
Ile Phe Gly Pro Ser Ala Ala Ser His Leu Leu Ile Leu Phe Thr Leu
    130                 135                 140
Asn Phe Thr Ile Thr Asn Leu Arg Tyr Glu Glu Asn Met Trp Pro Gly
145                 150                 155                 160
Ser Arg Lys Phe Asn Thr Thr Glu Arg Val Leu Gln Gly Leu Leu Arg
                165                 170                 175
Pro Leu Phe Lys Asn Thr Ser Val Gly Pro Leu Tyr Ser Gly Cys Arg
            180                 185                 190
Leu Thr Leu Leu Arg Pro Glu Lys Asp Gly Glu Ala Thr Gly Val Asp
        195                 200                 205
Ala Ile
    210

<210> 482
<211> 97
<212> PRT
<213> Homo sapiens

<400> 482
Met Ser Met Val Ser His Ser Gly Ala Leu Cys Pro Pro Leu Ala Phe
 1               5                  10                  15
Leu Gly Pro Pro Gln Trp Thr Trp Glu His Leu Gly Leu Gln Phe Leu
            20                  25                  30
Asn Leu Val Pro Arg Leu Pro Ala Leu Ser Trp Cys Tyr Ser Leu Ser
        35                  40                  45
Thr Ser Pro Ser Pro Thr Cys Gly Met Arg Arg Thr Cys Ser Thr Leu
    50                  55                  60
Ala Pro Gly Ser Ser Thr Pro Arg Arg Gly Ser Phe Arg Ala Cys Ser
65                  70                  75                  80
Gly Pro Cys Ser Arg Ala Pro Val Leu Ala Leu Cys Thr Leu Ala Ala
                85                  90                  95
Asp

<210> 483
<211> 438
<212> PRT
<213> Homo sapiens

<400> 483

<210> 484
<211> 216
<212> PRT

<213> Homo sapiens

<400> 484
Met Thr Leu Lys Ser Trp Ala Pro Thr Pro Trp Thr Gly Thr Val Ser
1               5                   10                  15
Met Ser Met Val Ser Pro Ile Arg Ala Leu Cys Pro Pro Pro Ala Leu
            20                  25                  30
Leu Gly Pro Pro Gln Trp Ile Ser Glu Pro Gln Trp Thr Pro Ser Ser
        35                  40                  45
Leu Ser Ser Pro Thr Ile Met Ala Ala Gly Pro Leu Leu Val Pro Phe
    50                  55                  60
Thr Leu Asn Phe Thr Ile Thr Asn Leu Gln Tyr Gly Glu Asp Met Gly
65                  70                  75                  80
His Pro Gly Ser Arg Lys Phe Asn Thr Thr Glu Arg Val Leu Gln Gly
                85                  90                  95
Leu Leu Gly Pro Ile Phe Lys Asn Thr Ser Val Gly Pro Leu Tyr Ser
            100                 105                 110
Gly Cys Arg Leu Thr Ser Leu Arg Ser Lys Lys Asp Gly Ala Ala Thr
        115                 120                 125
Gly Val Asp Ala Ile Cys Ile His His Leu Asp Pro Lys Ser Pro Gly
    130                 135                 140
Leu Asn Arg Glu Arg Leu Tyr Trp Glu Leu Ser Gln Leu Thr Asn Gly
145                 150                 155                 160
Ile Lys Glu Leu Gly Pro Tyr Thr Leu Asp Arg Asn Ser Leu Tyr Val
                165                 170                 175
Asn Gly Phe Thr His Arg Thr Ser Val Pro Thr Thr Ser Thr Pro Gly
            180                 185                 190
Thr Ser Thr Val Tyr Trp Ala Thr Thr Gly Thr Pro Ser Ser Leu Pro
        195                 200                 205
Ala Thr Gln Ser Leu Ala Leu Ser
    210                 215


<210> 485
<211> 268
<212> PRT
<213> Homo sapiens

<400> 485
Met Pro Thr Thr Ser Thr Pro Gly Thr Ser Thr Val Asp Val Gly Thr
1               5                   10                  15
Ser Gly Thr Pro Ser Ser Ser Pro Ser Pro Thr Thr Ala Gly Pro Leu
            20                  25                  30
Leu Met Pro Phe Thr Leu Asn Phe Thr Ile Thr Asn Leu Gln Tyr Glu
        35                  40                  45
Glu Asp Met Arg Arg Thr Gly Ser Arg Lys Phe Asn Thr Met Glu Ser
    50                  55                  60
Val Leu Gln Gly Leu Leu Lys Pro Leu Phe Lys Asn Thr Ser Val Gly
65                  70                  75                  80
Pro Leu Tyr Ser Gly Cys Arg Leu Thr Leu Leu Arg Pro Lys Lys Asp
                85                  90                  95
Gly Ala Ala Thr Gly Val Asp Ala Ile Cys Thr His Arg Leu Asp Pro
            100                 105                 110
Lys Ser Pro Gly Leu Asn Arg Glu Gln Leu Tyr Trp Glu Leu Ser Lys
        115                 120                 125
Leu Thr Asn Asp Ile Glu Glu Leu Gly Pro Tyr Thr Leu Asp Arg Asn
    130                 135                 140

Ser Leu Tyr Val Asn Gly Phe Thr His Gln Ser Ser Val Ser Thr Thr
145                 150                 155                 160
Ser Thr Pro Gly Thr Ser Thr Val Asp Leu Arg Thr Ser Val Asp Ser
                165                 170                 175
Ile Leu Pro Leu Gln Pro His Asn Tyr Gly Cys Trp Pro Ser Pro Gly
            180                 185                 190
Thr Ile His Pro Gln Leu His His His Gln Pro Ala Val Trp Gly Gly
        195                 200                 205
His Gly Ser Pro Trp Leu Gln Glu Val Gln His His Arg Glu Gly Pro
    210                 215                 220
Ala Gly Ser Ala Trp Ser His Ile Gln Glu His Gln Cys Trp Pro Ser
225                 230                 235                 240
Val Leu Trp Leu Gln Thr Asp Leu Ser Gln Val Gln Glu Gly Trp Ser
                245                 250                 255
Ser His Trp Ser Gly Cys His Leu His Pro Ser Ser
            260                 265

<210> 486
<211> 304
<212> PRT
<213> Homo sapiens

<400> 486
Met Gln His Pro Gly Ser Arg Lys Phe Asn Thr Thr Glu Arg Val Leu
 1               5                  10                  15
Gln Gly Leu Leu Arg Pro Leu Phe Lys Asn Thr Ser Val Gly Pro Leu
            20                  25                  30
Tyr Ser Gly Cys Arg Leu Thr Leu Leu Arg Pro Glu Lys Asp Gly Glu
        35                  40                  45
Ala Thr Gly Val Asp Ala Ile Cys Thr His Arg Pro Asp Pro Thr Gly
    50                  55                  60
Pro Gly Leu Asp Arg Glu Gln Leu Tyr Leu Glu Leu Ser Gln Leu Thr
65                  70                  75                  80
His Ser Ile Thr Glu Leu Gly Pro Tyr Thr Leu Asp Arg Asp Ser Leu
                85                  90                  95
Tyr Val Asn Gly Phe Thr His Arg Ser Ser Val Pro Thr Thr Ser Thr
            100                 105                 110
Gly Val Val Ser Glu Glu Pro Phe Thr Leu Asn Phe Thr Ile Asn Asn
        115                 120                 125
Leu Arg Tyr Met Ala Asp Met Gly Gln Pro Gly Ser Leu Lys Phe Asn
    130                 135                 140
Ile Thr Asp Asn Val Met Lys His Leu Leu Ser Pro Leu Phe Gln Arg
145                 150                 155                 160
Ser Ser Leu Gly Ala Arg Tyr Thr Gly Cys Arg Val Ile Ala Leu Arg
                165                 170                 175
Ser Val Lys Asn Gly Ala Glu Thr Arg Val Asp Leu Leu Cys Thr Tyr
            180                 185                 190
Leu Gln Pro Leu Ser Gly Pro Gly Leu Pro Ile Lys Gln Val Phe His
        195                 200                 205
Glu Leu Ser Gln Gln Thr His Gly Ile Thr Arg Leu Gly Pro Tyr Ser
    210                 215                 220
Leu Asp Lys Asp Ser Leu Tyr Leu Asn Gly Tyr Asn Glu Pro Gly Pro
225                 230                 235                 240
Asp Glu Pro Pro Thr Thr Pro Lys Pro Ala Thr Thr Phe Leu Pro Pro
                245                 250                 255
Leu Ser Glu Ala Thr Thr Ala Met Gly Tyr His Leu Lys Thr Leu Thr

260                 265                 270
Leu Asn Ser His Leu Gln Ser Pro Val Phe Thr Arg Tyr Gly Gln Gly
        275                 280                 285
Leu Lys Val His Ser Ile His Arg Gly Gly Ser Phe Ser Asn Trp Ser
    290                 295                 300


<210> 487
<211> 294
<212> PRT
<213> Homo sapiens

<400> 487
Met Thr Asn Gly Ile Lys Glu Leu Gly Pro Tyr Thr Leu Asp Arg Asn
 1               5                  10                  15
Ser Leu Tyr Val Asn Gly Phe Thr His Arg Ser Ser Gly Leu Thr Thr
            20                  25                  30
Ser Thr Pro Trp Thr Ser Thr Val Asp Leu Gly Thr Ser Gly Thr Pro
        35                  40                  45
Ser Pro Val Pro Ser Pro Thr Thr Ala Gly Pro Leu Leu Val Pro Phe
    50                  55                  60
Thr Leu Asn Phe Thr Ile Thr Asn Leu Gln Tyr Glu Glu Asp Met His
65                  70                  75                  80
Arg Pro Gly Ser Arg Lys Phe Asn Ala Thr Glu Arg Val Leu Gln Gly
                85                  90                  95
Leu Leu Ser Pro Ile Phe Lys Asn Ser Ser Val Gly Pro Leu Tyr Ser
            100                 105                 110
Gly Cys Arg Leu Thr Ser Leu Arg Pro Glu Lys Asp Gly Ala Ala Thr
        115                 120                 125
Gly Met Asp Ala Val Cys Leu Tyr His Pro Asn Pro Lys Arg Pro Gly
    130                 135                 140
Leu Asp Arg Glu Gln Leu Tyr Trp Glu Leu Ser Gln Leu Thr His Asn
145                 150                 155                 160
Ile Thr Glu Leu Gly Pro Tyr Ser Leu Asp Arg Asp Ser Leu Tyr Val
                165                 170                 175
Asn Gly Phe Thr His Gln Asn Ser Val Pro Thr Thr Ser Thr Pro Gly
            180                 185                 190
Thr Ser Thr Val Tyr Trp Ala Thr Thr Gly Thr Pro Ser Ser Phe Pro
        195                 200                 205
Gly His Thr Glu Pro Gly Pro Leu Leu Ile Pro Phe Thr Phe Asn Phe
    210                 215                 220
Thr Ile Thr Asn Leu His Tyr Glu Glu Asn Met Gln His Pro Gly Ser
225                 230                 235                 240
Arg Lys Phe Asn Ala Thr Glu Arg Val Leu Gln Gly Leu Leu Ser Pro
                245                 250                 255
Ile Phe Lys Asn Ser Ser Val Gly Pro Leu Tyr Ser Gly Cys Arg Leu
            260                 265                 270
Thr Ser Leu Arg Pro Glu Lys Asp Gly Ala Ala Thr Gly Met Asp Ala
        275                 280                 285
Val Cys Leu Tyr Arg Pro
    290


<210> 488
<211> 233
<212> PRT
<213> Homo sapiens

<400> 488
Ser Leu Val Glu Gln Val Phe Leu Asp Lys Thr Leu Asn Ala Ser Phe
1               5                   10                  15
His Trp Leu Gly Ser Thr Tyr Gln Leu Val Asp Ile His Val Thr Glu
            20                  25                  30
Met Glu Ser Ser Val Tyr Gln Pro Thr Ser Ser Ser Ser Thr Gln His
        35                  40                  45
Phe Tyr Leu Asn Phe Thr Ile Thr Asn Leu Pro Tyr Ser Gln Asp Lys
    50                  55                  60
Ala Gln Pro Gly Thr Thr Asn Tyr Gln Arg Asn Lys Arg Asn Ile Glu
65                  70                  75                  80
Asp Ala Leu Asn Gln Leu Phe Arg Asn Ser Ser Ile Lys Ser Tyr Phe
                85                  90                  95
Ser Asp Cys Gln Val Ser Thr Phe Arg Ser Val Pro Asn Arg His His
            100                 105                 110
Thr Gly Val Asp Ser Leu Cys Asn Phe Ser Pro Leu Ala Arg Arg Val
        115                 120                 125
Asp Arg Val Ala Ile Tyr Glu Glu Phe Leu Arg Met Thr Arg Asn Gly
    130                 135                 140
Thr Gln Leu Gln Asn Phe Thr Leu Asp Arg Ser Ser Val Leu Val Asp
145                 150                 155                 160
Gly Tyr Phe Pro Asn Arg Asn Glu Pro Leu Thr Gly Asn Ser Asp Leu
                165                 170                 175
Pro Phe Trp Ala Val Ile Leu Ile Gly Leu Ala Gly Leu Leu Gly Leu
            180                 185                 190
Ile Thr Cys Leu Ile Cys Gly Val Leu Val Thr Thr Arg Arg Arg Lys
        195                 200                 205
Lys Glu Gly Glu Tyr Asn Val Gln Gln Gln Cys Pro Gly Tyr Tyr Gln
    210                 215                 220
Ser His Leu Asp Leu Glu Asp Leu Gln
225                 230

<210> 489
<211> 178
<212> PRT
<213> Homo sapiens

<400> 489
Ser Leu Val Glu Gln Val Phe Leu Asp Lys Thr Leu Asn Ala Ser Phe
1               5                   10                  15
His Trp Leu Gly Ser Thr Tyr Gln Leu Val Asp Ile His Val Thr Glu
            20                  25                  30
Met Glu Ser Ser Val Tyr Gln Pro Thr Ser Ser Ser Ser Thr Gln His
        35                  40                  45
Phe Tyr Leu Asn Phe Thr Ile Thr Asn Leu Pro Tyr Ser Gln Asp Lys
    50                  55                  60
Ala Gln Pro Gly Thr Thr Asn Tyr Gln Arg Asn Lys Arg Asn Ile Glu
65                  70                  75                  80
Asp Ala Leu Asn Gln Leu Phe Arg Asn Ser Ser Ile Lys Ser Tyr Phe
                85                  90                  95
Ser Asp Cys Gln Val Ser Thr Phe Arg Ser Val Pro Asn Arg His His
            100                 105                 110
Thr Gly Val Asp Ser Leu Cys Asn Phe Ser Pro Leu Ala Arg Arg Val
        115                 120                 125
Asp Arg Val Ala Ile Tyr Glu Glu Phe Leu Arg Met Thr Arg Asn Gly

130                 135                 140
Thr Gln Leu Gln Asn Phe Thr Leu Asp Arg Ser Ser Val Leu Val Asp
145                 150                 155                 160
Gly Tyr Phe Pro Asn Arg Asn Glu Pro Leu Thr Gly Asn Ser Asp Leu
                165                 170                 175
Pro Phe

<210> 490
<211> 15
<212> PRT
<213> Homo sapiens

<400> 490
Thr Cys Gly Met Arg Arg Thr Cys Ser Thr Leu Ala Pro Gly Ser
1               5                   10                  15

<210> 491
<211> 15
<212> PRT
<213> Homo sapiens

<400> 491
Cys Arg Leu Thr Leu Leu Arg Pro Glu Lys Asp Gly Thr Ala Thr
1               5                   10                  15

<210> 492
<211> 15
<212> PRT
<213> Homo sapiens

<400> 492
Asp Gly Thr Ala Thr Gly Val Asp Ala Ile Cys Thr His His Pro
1               5                   10                  15

<210> 493
<211> 15
<212> PRT
<213> Homo sapiens

<400> 493
Cys Thr His His Pro Asp Pro Lys Ser Pro Arg Leu Asp Arg Glu
1               5                   10                  15

<210> 494
<211> 15
<212> PRT
<213> Homo sapiens

<400> 494
Arg Leu Asp Arg Glu Gln Leu Tyr Trp Glu Leu Ser Gln Leu Thr
1               5                   10                  15

<210> 495
<211> 15
<212> PRT
<213> Homo sapiens

<400> 495
Leu Gly Pro Tyr Ala Leu Asp Asn Asp Ser Leu Phe Val Asn Gly
1               5                   10                  15


<210> 496
<211> 15
<212> PRT
<213> Homo sapiens

<400> 496
Ser Val Ser Thr Thr Ser Thr Pro Gly Thr Pro Thr Tyr Val Leu
1               5                   10                  15


<210> 497
<211> 15
<212> PRT
<213> Homo sapiens

<400> 497
Leu Arg Pro Glu Lys Asp Gly Glu Ala Thr Gly Val Asp Ala Ile
1               5                   10                  15


<210> 498
<211> 15
<212> PRT
<213> Homo sapiens

<400> 498
Asp Pro Thr Gly Pro Gly Leu Asp Arg Glu Gln Leu Tyr Leu Glu
1               5                   10                  15


<210> 499
<211> 15
<212> PRT
<213> Homo sapiens

<400> 499
Leu Asp Arg Asp Ser Leu Tyr Val Asn Gly Phe Thr His Arg Ser
1               5                   10                  15


<210> 500
<211> 15
<212> PRT
<213> Homo sapiens

<400> 500
Gly Pro Tyr Ser Leu Asp Lys Asp Ser Leu Tyr Leu Asn Gly Tyr
1               5                   10                  15


<210> 501
<211> 15
<212> PRT
<213> Homo sapiens

<400> 501
Tyr Leu Asn Gly Tyr Asn Glu Pro Gly Pro Asp Glu Pro Pro Thr
1               5                   10                  15


<210> 502
<211> 15
<212> PRT
<213> Homo sapiens

<400> 502
Ala Thr Phe Asn Ser Thr Glu Gly Val Leu Gln His Leu Leu Arg
1               5                   10                  15


<210> 503
<211> 15
<212> PRT
<213> Homo sapiens

<400> 503
Gln Leu Ile Ser Leu Arg Pro Glu Lys Asp Gly Ala Ala Thr Gly
1               5                   10                  15


<210> 504
<211> 15
<212> PRT
<213> Homo sapiens

<400> 504
Gly Ala Ala Thr Gly Val Asp Thr Thr Cys Thr Tyr His Pro Asp
1               5                   10                  15


<210> 505
<211> 15
<212> PRT
<213> Homo sapiens

<400> 505
Thr Tyr His Pro Asp Pro Val Gly Pro Gly Leu Asp Ile Gln Gln
1               5                   10                  15


<210> 506
<211> 15

<212> PRT
<213> Homo sapiens

<400> 506
Leu Asp Ile Gln Gln Leu Tyr Trp Glu Leu Ser Gln Leu Thr His
1               5                   10                  15


<210> 507
<211> 15
<212> PRT
<213> Homo sapiens

<400> 507
His Ile Val Asn Trp Asn Leu Ser Asn Pro Asp Pro Thr Ser Ser
1               5                   10                  15


<210> 508
<211> 15
<212> PRT
<213> Homo sapiens

<400> 508
Asp Pro Thr Ser Ser Glu Tyr Ile Thr Leu Leu Arg Asp Ile Gln
1               5                   10                  15


<210> 509
<211> 15
<212> PRT
<213> Homo sapiens

<400> 509
Leu Arg Asp Ile Gln Asp Lys Val Thr Thr Leu Tyr Lys Gly Ser
1               5                   10                  15


<210> 510
<211> 15
<212> PRT
<213> Homo sapiens

<400> 510
Leu Tyr Lys Gly Ser Gln Leu His Asp Thr Phe Arg Phe Cys Leu
1               5                   10                  15


<210> 511
<211> 15
<212> PRT
<213> Homo sapiens

<400> 511
Asp Lys Ala Gln Pro Gly Thr Thr Asn Tyr Gln Arg Asn Lys Arg
1               5                   10                  15

<210> 512
<211> 450
<212> DNA
<213> Homo sapiens

<400> 512
gttacaatga acctggtcca gatgagcctc ctacaactcc caagccagcc accacattcc 60
tgcctcctct gtcagaagcc acaacagcca tggggtacca cctgaagacc ctcacactca 120
acttcaccat ctccaatctc cagtattcac cagatatggg caagggctca gctacattca 180
actccaccga gggggtcctt cagcacctgc tcagaccctt gttccagaag agcagcatgg 240
gccccttcta cttgggttgc caactgatct ccctcaggcc tgagaaggat ggggcagcca 300
ctggtgtgga caccacctgc acctaccacc ctgaccctgt gggccccggg ctggacatac 360
agcagcttta ctgggagctg agtcagctga cccatggtgt cacccaactg ggcttctatg 420
tcctggacag ggatagcctc ttcatcaatg 450

<210> 513
<211> 402
<212> DNA
<213> Homo sapiens

<400> 513
gtttcaccca tcggagctct gtacccacca ccagcaccgg ggtggtcagc gaggagccat 60
tcacactgaa cttcaccatc aacaacctgc gctacatggc ggacatgggc caacccggct 120
ccctcaagtt caacatcaca gacaacgtca tgaagcacct gctcagtcct ttgttccaga 180
ggagcagcct gggtgcacgg tacacaggct gcagggtcat cgcactaagg tctgtgaaga 240
acggtgctga gacacgggtg gacctcctct gcacctacct gcagcccctc agcggcccag 300
gtctgcctat caagcaggtg ttccatgagc tgagccagca gacccatggc atcacccggc 360
tgggccccta ctctctggac aaagacagcc tctaccttaa cg 402

<210> 514
<211> 465
<212> DNA
<213> Homo sapiens

<400> 514
gtttcactca tcggagctct gtgtccacca ccagcactcc tgggaccccc acagtgtatc 60
tgggagcatc taagactcca gcctcgatat ttggcccttc agctgccagc catctcctga 120
tactattcac cctcaacttc accatcacta acctgcggta tgaggagaac atgtggcctg 180
gctccaggaa gttcaacact acagagaggg tccttcaggg cctgctaagg cccttgttca 240
agaacaccag tgttggccct ctgtactctg gctgcaggct gaccttgctc aggccagaga 300
aagatgggga agccaccgga gtggatgcca tctgcaccca ccgccctgac cccacaggcc 360
ctgggctgga cagagagcag ctgtatttgg agctgagcca gctgacccac agcatcactg 420
agctgggccc ctacacactg gacagggaca gtctctatgt caatg 465

<210> 515
<211> 463
<212> DNA
<213> Homo sapiens

<400> 515
gtttcacaca gcggagctct gtgcccacca ctagcattcc tgggaccccc acagtggacc 60
tgggaacatc tgggactcca gtttctaaac ctggtccctc ggctgccagc cctctcctgg 120
tgctattcac tctcaacttc accatcacca acctgcggta tgaggagaac atgcagcacc 180
ctggctccag gaagttcaac accacggaga gggtccttca gggcctggtc cctgttcaag 240
agcaccagtg ttggccctct gtactctggc tgcagactga ctttgctcag gcctgaaaag 300
gatgggacag ccactggagt ggatgccatc tgcacccacc accctgaccc caaaagccct 360

aggctggaca gagagcagct gtattgggag ctgagccagc tgacccacaa tatcactgag 420
ctgggcccct atgccctgga caacgacagc ctctttgtca atg 463

<210> 516
<211> 156
<212> DNA
<213> Homo sapiens

<400> 516
cagccaccgg agtggatgcc atctgcaccc accgccctga ccccacaggc cctgggctgg 60
acagagagca gctgtatttg gagctgagcc agctgaccca cagcatcact gagctgggcc 120
cctacaccct ggacagggac agtctctatg tcaatg 156

<210> 517
<211> 450
<212> DNA
<213> Homo sapiens

<400> 517
gttacaatga acctggtcta gatgagcctc ctacaactcc caagccagcc accacattcc 60
tgcctcctct gtcagaagcc acaacagcca tggggtacca cctgaagacc ctcacactca 120
acttcaccat ctccaatctc cagtattcac cagatatggg caagggctca gctacattca 180
actccaccga gggggtcctt cagcacctgc tcagaccctt gttccagaag agcagcatgg 240
gccccttcta cttgggttgc caactgatct ccctcaggcc tgagaaggat ggggcagcca 300
ctggtgtgga caccacctgc acctaccacc ctgaccctgt gggccccggg ctggacatac 360
agcagcttta ctgggagctg agtcagctga cccatggtgt cacccaactg ggcttctatg 420
tcctggacag ggatagcctc ttcatcaatg 450

<210> 518
<211> 402
<212> DNA
<213> Homo sapiens

<400> 518
gtttcaccca tcggagctct gtacccacca ccagcaccgg ggtggtcagc gaggagccat 60
tcacactgaa cttcaccatc aacaacctgc gctacatggc ggacatgggc caacccggct 120
ccctcaagtt caacatcaca gacaacgtca tgaagcacct gctcagtcct ttgttccaga 180
ggagcagcct gggtgcacgg tacacaggct gcagggtcat cgcactaagg tctgtgaaga 240
acggtgctga gacacgggtg gacctcctct gcacctacct gcagcccctc agcggcccag 300
gtctgcctat caagcaggtg ttccatgagc tgagccagca gacccatggc atcacccggc 360
tgggccccta ctctctggac aaagacagcc tctaccttaa cg 402

<210> 519
<211> 465
<212> DNA
<213> Homo sapiens

<400> 519
gtttcactca tcggagctct gtgtccacca ccagcactcc tgggaccccc acagtgtatc 60
tgggagcatc taagactcca gcctcgatat ttggcccttc agctgccagc catctcctga 120
tactattcac cctcaacttc accatcacta acctgcggta tgaggagaac atgtggcctg 180
gctccaggaa gttcaacact acagagaggg tccttcaggg cctgctaagg cccttgttca 240
agaacaccag tgttggccct ctgtactctg gctccaggct gaccttgctc aggccagaga 300
aagatgggga agccaccgga gtggatgcca tctgcaccca ccgccctgac cccacaggcc 360
ctgggctgga cagagagcag ctgtatttgg agctgagcca gctgacccac agcatcactg 420
agctgggccc ctacacactg gacagggaca gtctctatgt caatg 465

<210> 520
<211> 468
<212> DNA
<213> Homo sapiens

<400> 520
gtttcacaca gcggagctct gtgcccacca ctagcattcc tgggaccccc acagtggacc 60
tgggaacatc tgggactcca gtttctaaac ctggtccctc ggctgccagc cctctcctgg 120
tgctattcac tctcaacttc accatcacca acctgcggta tgaggagaac atgcagcacc 180
ctggctccag gaagttcaac accacggaga gggtccttca gggcctgctc aggtccctgt 240
tcaagagcac cagtgttggc cctctgtact ctggctgcag actgactttg ctcaggcctg 300
aaaaggatgg gacagccact ggagtggatg ccatctgcac ccaccaccct gaccccaaaa 360
gccctaggct ggacagagag cagctgtatt gggagctgag ccagctgacc cacaatatca 420
ctgagctggg ccactatgcc ctggacaacg acagcctctt tgtcaatg 468

<210> 521
<211> 468
<212> DNA
<213> Homo sapiens

<400> 521
gtttcaccca tcagagctct atgacgacca ccagaactcc tgatacctcc acaatgcacc 60
tggcaacctc gagaactcca gcctccctgt ctggacctac gaccgccagc cctctcctgg 120
tgctattcac aattaacttc accatcacta acctgcggta tgaggagaac atgcatcacc 180
ctggctctag aaagtttaac accacggaga gagtccttca gggtctgctc aggcctgtgt 240
tcaagaacac cagtgttggc cctctgtact ctggctgcag actgaccttg ctcaggccca 300
agaaggatgg ggcagccacc aaagtggatg ccatctgcac ctaccgccct gatcccaaaa 360
gccctggact ggacagagag cagctatact gggagctgag ccagctaacc cacagcatca 420
ctgagctggg cccctacacc ctggacaggg acagtctcta tgtcaatg 468

<210> 522
<211> 262
<212> DNA
<213> Homo sapiens

<400> 522
gagagggtcc ttcagggtct gcttatgccc ttgttcaaga acaccagtgt cagctctctg 60
tactctggtt gcagactgac cttgctcagg cctgagaagg atggggcagc caccagagtg 120
gatgctgtct gcacccatcg tcctgacccc aaaagccctg gactggacag agagcggctg 180
tactggaagc tgagccagct gacccacggc atcactgagc tgggccccta caccctggac 240
aggcacagtc tctatgtcaa tg 262

<210> 523
<211> 302
<212> DNA
<213> Homo sapiens

<400> 523
aggacatgcg tcaccctggc tccaggaagt tcaacaccac agagagggtc ctgcagggtc 60
tgcttggtcc cttgttcaag aactccagtg tcggccctct gtactctggc tgcagactga 120
tctctctcag gtctgagaag gatggggcag ccactggagt ggatgccatc tgcacccacc 180
accttaaccc tcaaagcctg gactggacag ggagcagctg tactggcagc tgagccagat 240
gaccaatggc atcaaagagc tgggccccta caccctggac cggaacagtc tctacgtcaa 300
tg 302

<210> 524
<211> 468

<212> DNA
<213> Homo sapiens

<400> 524
gtttcaccca tcggagctct gggctcacca ccagcactcc ttggacttcc acagttgacc 60
ttggaacctc agggactcca tcccccgtcc ccagccccac aactgctggc cctctcctgg 120
tgccattcac cctaaacttc accatcacca acctgcagta tgaggaggac atgcatcgcc 180
ctggatctag gaagttcaac gccacagaga gggtcctgca gggtctgctt agtcccatat 240
tcaagaactc cagtgttggc cctctgtact ctggctgcag actgacctct ctcaggcccg 300
agaaggatgg ggcagcaact ggaatggatg ctgtctgcct ctaccaccct aatcccaaaa 360
gacctgggct ggacagagag cagctgtact gggagctaag ccagctgacc cacaacatca 420
ctgagctggg cccctacagc ctggacaggg acagtctcta tgtcaatg 468

<210> 525
<211> 470
<212> DNA
<213> Homo sapiens

<400> 525
gtttcaccca tcagaactct gtgcccacca ccagtactcc tgggacctcc acagtgtact 60
gggcaaccac tgggactcca tcctccttcc ccggccacac agagcctggc cctctcctga 120
taccattcac attcaacttt accatcacca acctgcatta tgaggaaaac atgcaacacc 180
ctggttccag gaagttcaac gccacagaga gggtcctgca gggtctgctt agtcccatat 240
tcaagaactc cagtgttggc cctctgtact ctggctgcag actgacctct ctcaggcccg 300
agaaggatgg ggcagcaact ggaatggatg ctgtctgtct ctaccgaccc taatcccatc 360
ggacctgggc tggacagaga gcagctgtac tgggagctga gccagctgac ccacgacatc 420
actgagctgg gcccctacag ccctggacag ggacagtctc tatgtcaatg 470

<210> 526
<211> 467
<212> DNA
<213> Homo sapiens

<400> 526
gtttcaccca tcagaactct gtgcccacca ccagtactcc tgggacctcc acagtgtact 60
gggcaaccac tgggactcca tcctccttcc ccggccacac agagcctggc cctctcctga 120
taccattcac tttcaacttt accatcacca acctgcatta tgaggaaaac atgcaacacc 180
tggttccagg aagttcaaca ccacggagag ggttctgcag ggtctgctca cgcccttgtt 240
caagaacacc agtgttggcc ctctgtactc tggctgcaga ctgaccttgc tcagacctga 300
gaagcaggag gcagccactg gagtggacac catctgcact caccgccttg accctctaaa 360
ccctggactg gacagagagc agctatactg ggagctgagc aaactgaccc gtggcatcat 420
cgagctgggc ccctacctcc tggacagagg cagtctctat gtcaatg 467

<210> 527
<211> 468
<212> DNA
<213> Homo sapiens

<400> 527
gtttcaccca tcggaacttt gtgcccatca ccagcactcc tgggacctcc acagtacacc 60
taggaacctc tgaaactcca tcctccctac ctagacccat agtgcctggc cctctcctgg 120
tgccattcac cctcaacttc accatcacca acttgcagta tgaggaggcc atgcgacacc 180
ctggctccag gaagttcaat accacggaga gggtcctaca gggtctgctc aggcccttgt 240
tcaagaatac cagtatcggc cctctgtact ccagctgcag actgaccttg ctcaggccag 300
agaaggacaa ggcagccacc agagtggatg ccatctgtac ccaccaccct gaccctcaaa 360
gccctggact gaacagagag cagctgtact gggagctgag ccagctgacc cacggcatca 420
ctgagctggg cccctacacc ctggacaggc acagtctcta tgtcaatg 468

<210> 528
<211> 537
<212> DNA
<213> Homo sapiens

<400> 528
gtttcaccca tcagagcccc ataccaacca ccagcactcc tgatacctcc acaatgcacc 60
tgggaacctc gagaactcca gcctccctgt ctggacctac gaccgccagc cctctcctgg 120
tgctattcac aattaacttc accatcacta acctgcggta tgaggagaac atgcatcacc 180
gctggctcta gaaagtttaa caccacggag agagtccttc agggtctgct caggcctgtg 240
ttcaaagaac accagtgttg gccctctgta ctctggctgc agactgacct tgctcaggcc 300
cgagaaggat ggggcagcca cgcaaagtgg atgccatctg cacctaccgc cctgatccca 360
aaagccctgg actggacaga gagcagctat actgggagct gagccagggt gatgcatgtt 420
ctcctcatat cgcaggttag tgatggtgaa gttaattgtg aatagcacca ggagagggct 480
ggcggtcatg ggtccagaca gggagcctgg agttctcgag gttgccaggt gcatgtc 537

<210> 529
<211> 231
<212> DNA
<213> Homo sapiens

<400> 529
tgttccagag gagcagcctg ggtgcacggt acacaggctg cagggtcatc gcactaaggt 60
ctgtgaagaa cggtgctgag acacgggtgg acctcctctg cacctacctg cagcccctca 120
gcggcccagg tctgcctatc aagcaggtgt tccatgagct gagccagcag acccatggca 180
tcacccggct gggcccctac tctctggaca aagacagcct ctaccttaac g 231

<210> 530
<211> 376
<212> DNA
<213> Homo sapiens

<400> 530
gttacaatga acctggtcca gatgagcctc ctacaactcc caagccagcc accacattcc 60
tgcctcctct gtcagaagcc acaacagcca tggggtacca cctgaagacc ctcacactca 120
acttcaccat ctccaatctc cagtattcac cagatatggg caagggctca gctacattca 180
actccaccga gggggtcctt cagcacctgg cctgagaagg atggggcagc cactggtgtg 240
gacaccacct gcacctacca ccctgaccct gtgggccccg ggctggacat acagcagctt 300
tactgggagc tgagtcagct gacccatggt gtcacccaac tgggcttcta tgtcctggac 360
agcgatagct cttcat 376

<210> 531
<211> 75
<212> DNA
<213> Homo sapiens

<400> 531
ggtaaccaca gctgacccat ggcatcaaag agctgggccc ctacaccctg gacaggaaca 60
gtctctatgt caatg 75

<210> 532
<211> 906
<212> DNA
<213> Homo sapiens

<400> 532

gtttcaccca tcggagctct gtggccccca ccagcactcc tgggacctcc acagtggacc 60
ttgggacctc agggactcca tcctccctcc ccagccccac aacagctgtt cctctcctgg 120
tgccgttcac cctcaacttt accatcacca atctgcagta tggggaggac atgcgtcacc 180
ctggctccag gaagttcaac accacagaga gggtcctgca gggtctgctt ggtcccttgt 240
tcaagaactc cagtgtcggc cctctgtact ctggctgcag actgatctct ctcaggtctg 300
agaaggatgg ggcagccact ggagtggatg ccatctgcac ccaccacctt aaccctcaaa 360
gccctggact ggacagggag cagctgtact ggcagctgag ccagagacca caacctcatt 420
tatcacctat tctgagacac acacaagttc agccattcca actctccctg tctccccctg 480
gtgcatcaaa gatgctgacc tcactggtca tcagttctgg gacagacagc actacaactt 540
tcccaacact gacggagacc ccatatgaac cagagacaac agccatacag ctcattcatc 600
ctgcagagac caacacaatg gttcccagga caactcccaa gttttcccat agtaagtcag 660
acaccacact cccagtagcc atcaccagtc ctgggccaga agccagttca gctgtttcaa 720
cgacaactat ctcacctgat atgtcagatc tggtgacctc actggtccct agttctggga 780
cagacaccag tacaaccttc ccaacattga gtgagacccc atatgaacca gagactacag 840
ccacgtggct cactcatcct gcagaaacca gaacaacggt ttctgggaca attcccaact 900
tttccc 906

<210> 533
<211> 404
<212> DNA
<213> Homo sapiens

<400> 533
gtttcaccca tcggagctct gtggccccca ccagcactcc tgggacctcc acagtggacc 60
ttgggacctc agggactcca tcctccctcc ccagccccac aacagctgtt cctctcctgg 120
tgccgttcac cctcaacttt accatcacca atctgcagta tggggaggac atgcgtcacc 180
ctggctccag gaagttcaac accacagaga gggtcctgca gggtctgctt ggtcccttgt 240
tcaagaactc cagtgtcggc cctctgtact ctggctgcag actgatctct ctcaggtctg 300
agaaggatgg ggcagccact ggagtggatg ccatctgcac ccaccacctt aaccctcaaa 360
gccctggact ggacagggag cagctgtact ggcagctgag ccag 404

<210> 534
<211> 157
<212> DNA
<213> Homo sapiens

<400> 534
gcagccacca aagtggatgc catctgcacc taccgccctg atcccaaaag ccctggactg 60
gacagagagc agctatactg ggagctgagc cagctaaccc acagcatcac tgagctgggc 120
ccctacaccc tggacaggga cagtctctat gtcaatg 157

<210> 535
<211> 468
<212> DNA
<213> Homo sapiens

<400> 535
gtttcacaca gcggagctct gtgcccacca ctagcattcc tgggaccccc acagtggacc 60
tgggaacatc tgggactcca gtttctaaac ctggtccctc ggctgccagc cctctcctgg 120
tgctattcac tctcaacttc accatcacca acctgcggta tgaggagaac atgcagcacc 180
ctggctccag gaagttcaac accacggaga gggtccttca gggcctgctc aggtccctgt 240
tcaagagcac cagtgttggc cctctgtact ctggctgcag actgactttg ctcaggcctg 300
aaaaggatgg gacagccact ggagtggatg ccatctgcac ccaccaccct gaccccaaaa 360
gccctaggct ggacagagag cagctgtatt gggagctgag ccagctgacc cacaatatca 420
ctgagctggg cccctatgcc ctggacaacg acagcctctt tgtcaatg 468

<210> 536

<211> 334
<212> DNA
<213> Homo sapiens

<400> 536
gtttcactca tcggagctct gtgtccacca ccagcactcc tgggaccccc acagtgtatc 60
tgggagcatc taagactcca gcctcgatat ttggcccttc agctgccagc catctcctga 120
tactattcac cctcaacttc accatcacta acctgcggta tgaggagaac atgtggcctg 180
gctccaggaa gttcaacact acagagaggg tccttcaggg cctgctaagg cccttgttca 240
agaacaccag tgttggccct ctgtactctg gctgcaggct gaccttgctc aggccagaga 300
aagatgggga agccaccgga gtggatgcca tctg 334

<210> 537
<211> 127
<212> DNA
<213> Homo sapiens

<400> 537
ccaccgtctt gaccccaaaa gccctggagt ggacagggag cagctatact gggagctgag 60
ccagctgacc aatggcatca aagagctggg cccctacacc tggacaggaa cagtctctat 120
gtcaatg 127

<210> 538
<211> 468
<212> DNA
<213> Homo sapiens

<400> 538
gtttcaccca tcggacctct gtgcccacca ccagcactcc tgggacctcc acagtggacc 60
ttggaacctc agggactcca ttctccctcc caagccccgc aactgctggc cctctcctgg 120
tgctgttcac cctcaacttc accatcacca acctgaagta tgaggaggac atgcatcgcc 180
ctggctccag gaagttcaac accactgaga gggtcctgca gactctgctt ggtcctatgt 240
tcaagaacac cagtgttggc cttctgtact ctggctgcag actgaccttg ctcaggtccg 300
agaaggatgg agcagccact ggagtggatg ccatctgcac ccaccgtctt gaccccaaaa 360
gccctggagt ggacagggag cagctatact gggagctgag ccagctgacc aatggcatca 420
aagagctggg cccctacacc ctggacagga acagtctcta tgtcaatg 468

<210> 539
<211> 465
<212> DNA
<213> Homo sapiens

<400> 539
gtttcaccca ttggatccct gtgcccacca gcagcactcc tgggacctcc acagtggacc 60
ttgggtcagg gactccatcc tccctcccca gccccacaac tgctggccct ctcctggtgc 120
cgttcaccct caacttcacc atcaccaacc tgaagtacga ggaggacatg cattgccctg 180
gctccaggaa gttcaacacc acagagagag tcctgcagag tctgcttggt cccatgttca 240
agaacaccag tgttggccct ctgtactctg gctgcagact gaccttgctc aggtccgaga 300
aggatggagc agccactgga gtggatgcca tctgcaccca ccgtcttgac cccaaaagcc 360
tggagtggac agggagcagc tatactggga gctgagccag ctgaccaatg ccatcaaaga 420
gctgggtccc tacaccctgg acagcaacag tcttctatgt caatg 465

<210> 540
<211> 255
<212> DNA
<213> Homo sapiens

<400> 540
gtttcaccca tcagacctct gcgcccaaca ccagcactcc tgggacctcc acagtggacc 60
ttgggacctc agggactcca tcctccctcc ccagccctac atctgctggc cctctcctgg 120
tgccattcac cctcaacttc accatcacca acctgcagta cgaggaggac atgcatcacc 180
caggctccag gaagttcaac accacggagc gggtcctgca gggtctgctt ggtcccatgt 240
tcaagaacac tacga 255

<210> 541
<211> 390
<212> DNA
<213> Homo sapiens

<400> 541
catccccagc tcgaacagca gccacagtcc cattcatggt gccattcacc ctcaacttca 60
actcatcacc aacctgcagt acgaggagga catgcggcac ctggttccag gaagttcaac 120
gcgcacagag agagaactgc agggtcgtgc tcaaacccta gatcaggaat agcagtctgg 180
aatacctcta ttcaggctgc agactagcct cactcaggcc agagaaggat agctcagcca 240
cggcagtgga tgccatctgc acacatcgcc ctgaccctga agacctcgga ctggacagag 300
agcgactgta ctgggagctg agcaatctga caaatggcat ccaggagctg ggcccctaca 360
ccctggaccg gaacagtctc tatgtcaatg 390

<210> 542
<211> 468
<212> DNA
<213> Homo sapiens

<400> 542
gtttcaccca tcgaagctct atgcccacca ccagcactcc tgggacctcc acagtggatg 60
tgggaacctc agggactcca tcctccagcc ccagccccac gactgctggc cctctcctga 120
tgccgttcac cctcaacttc accatcacca acctgcagta cgaggaggac atgcgtcgca 180
ctggctccag gaagttcaac accatggaga gtgtcctgca gggtctgctc aagcccttgt 240
tcaagaacac cagtgttggc cctctgtact ctggctgcag attgaccttg ctcaggccca 300
agaaagatgg ggcagccact ggagtggatg ccatctgcac ccaccgcctt gaccccaaaa 360
gccctggact caacagggag cagctgtact gggagctaag caaactgacc aatgacattg 420
aagagctggg cccctacacc ctggacagga acagtctcta tgtcaatg 468

<210> 543
<211> 475
<212> DNA
<213> Homo sapiens

<400> 543
gtttcaccca tcagagctct gtgtccacca ccagcactcc tgggacctcc acagtggatc 60
tcagaacctc agtggactcc atcctccctc tccagcccca caattatggc tgctggccct 120
ctcctggtac cattcaccct caacttcacc atcaccaacc tgcagtatgg ggaggacatg 180
ggtcaccctg gctccaggaa gttcaacacc acagagaggg tcctgcaggg tctgcttggt 240
cccatattca agaacaccag tgttggccct ctgtactctg gctgcagact gacctctctc 300
aggtccaaga aggatggagc agccactgga gtggatgcca tctgcatcca tcatcttgac 360
cccaaaagcc ctggactcaa cagagagcgg ctgtactggg agctgagcca actgaccaat 420
ggcatcaaag agctgggccc ctacaccctg gacaggaaca gtctctatgt caatg 475

<210> 544
<211> 485
<212> DNA
<213> Homo sapiens

<400> 544
   gtttcaccca tcggacctct gtgcccacca ccagtactcc tgggacctcc acagtgtact 60
   gggcaaccac tgggactcca tcctccctcc ccgccacaca gagcctggcc ctctcctgat 120
   accattcaca ttcaacttta ccatcaccta cctgcattat agaggaaaac atgcaacacc 180
   cgtggttcca ggaacgatgt caacaccaca ggagagggtt ctgcagggtc ttcgctcacg 240
   cccattgtta caagaacacc agtagttggc cctctgtact ctggctgcag aatgaccttg 300
   ctcagacctg agaagcagga ggcaacacac tggaatggac accatctgta tccaccagcg 360
   ttagatccca tcaggacctg gactggacag agagcaggct atactgggag ctagagccag 420
   ctgacccaca gcatcacaga gctgggaccc tacagccctg gatagggaca gtctctatgt 480
   caatg 485

<210> 545
<211> 141
<212> DNA
<213> Homo sapiens

<400> 545
gcttcaaccc ttggagctct gtgccaacca ccagcactcc tgggacctcc acagtgcacc 60
tggcaacctc tgggactcca tcctccctgc ctggccacac agcccctgtc cctctcttga 120
taccattcac cctcaactta c 141

<210> 546
<211> 142
<212> DNA
<213> Homo sapiens

<400> 546
gacctcctct gcacctacct gcagcccctc agcggcccag gtctgcctat caagcaggtg 60
ttccatgagc tgagccagca gacccatggc atcacccggc tgggccccta ctctctggac 120
aaagacagcc tctaccttaa cg 142

<210> 547
<211> 185
<212> DNA
<213> Homo sapiens

<400> 547
gttcttaccc aggccagaga aggatagctc agccacggca gtggatgcca tctgcacaca 60
tcgccctgac cctgaagacc tcggactgga cagagagcga ctgtactggg agctgagcaa 120
tctgacaaat ggcatccagg agctgggccc ctacaccctg gaccggaaca gtctctatgt 180
caatg 185

<210> 548
<211> 462
<212> DNA
<213> Homo sapiens

<400> 548
gtttcaccca tcgaagctct atgcccacca ccagcactcc tgggacctcc acagtggatg 60
tgggaacctc agggactcca tcctccagcc ccagccccac gactgctggc cctctcctga 120
tgccgttcac cctcaacttc accatcacca acctgcagta cgaggaggac atgcgtcgca 180
ctggctccag gaagttcaac accatggaga gtgtcctgca gggtctgccc ttgttcaaga 240
acaccagtgt tggccctctg tactctggct gcagattgac cttgctcagg cccgagaaag 300
atggggcagc cactggagtg gatgccatct gcacccaccg ccttgacccc aaaagccctg 360
gactcaacag ggagcagctg tactgggagc taagcaaact gaccaatgac attgaagagc 420
tgggccccta caccctggac aggaacagtc tctatgtcaa tg 462

<210> 549

<211> 400
<212> DNA
<213> Homo sapiens

<400> 549
actccatcct ccctctccag ccccacaatt atggctgctg gccctctcct ggtaccattc 60
accctcaact tcaccatcac caacctgcag tatggggagg acatgggtca ccctggctcc 120
aggaagttca acaccacaga gagggtcctg cagggtctgc ttggtcccat attcaagaac 180
accagtgttg gccctctgta ctctggctgc agactgacct ctctcaggtc tgagaaggat 240
ggagcagcca ctggagtgga tgccatctgc atccatcatc ttgaccccaa aagccctgga 300
ctcaacagag agcggctgta ctgggagctg agccaactga ccaatggcat caaagagctg 360
ggcccctaca ccctggacag gaacagtctc tatgtcaatg 400

<210> 550
<211> 468
<212> DNA
<213> Homo sapiens

<400> 550
gtttcaccca tcggacctct gtgcccacca gcagcactcc tgggacctcc acagtggacc 60
ttggaacctc agggactcca ttctccctcc caagccccgc aactgctggc cctctcctgg 120
tgctgttcac cctcaacttc accatcacca acctgaagta tgaggaggac atgcatcgcc 180
ctggctccag gaagttcaac accactgaga gggtcctgca gactctgctt ggtcctatgt 240
tcaagaacac cagtgttggc cttctgtact ctggctgcag actgaccttg ctcaggtccg 300
agaaggatgg agcagtcact ggagtggatg ccatctgcac ccaccgtctt gaccccaaaa 360
gccctggagt ggacagggag cagctatact gggagctgag ccagctgacc aatggcatca 420
aagagctggg cccctacacc ctggacaggc acagtctcta tgtcaatg 468

<210> 551
<211> 366
<212> DNA
<213> Homo sapiens

<400> 551
ctgctggccc tctcctggtg ctgttcaccc tcaacttcac catcaccaac ctgaagtatg 60
aggaggacat gcatcgccct ggctccagga agttcaacac cactgagagg gtcctgcaga 120
ctctgcgtgg tcctatgttc aagaacacca gtggtggcct tctgtactct ggctgcagac 180
tgaccttgct caggtccgag aaggatggag cagccactgg agtggatgcc atctgcaccc 240
accgtcttga ccccaaaagc cctggagtgg acagggagca gctatactgg gagctgagcc 300
agctgaccaa tggcatcaaa gagctgggcc cctacaccct ggacaggaac agtctctatg 360
tcaatg 366

<210> 552
<211> 465
<212> DNA
<213> Homo sapiens

<400> 552
gtttcaccca ttggatccct gtgcccacca gcagcactcc tgggacctcc acagtggacc 60
ttgggtcagg gactccatcc tccctcccca gccccacaac tgctggccct ctcctggtgc 120
cattcaccct caacttcacc atcaccaacc tgcagtacga ggaggacatg catcacccag 180
gctccaggaa gttcaacacc acggagcggg tcctgcaggg tctgcttggt cccatgttca 240
agaacaccag tgtcggcctt ctgtactctg gctgcagact gaccttgctc aggcctgaga 300
agaatggggc agccactgga atggatgcca tctgcagcca ccgtcttgac cccaaaagcc 360
ctggactcaa cagagagcag ctgtactggg agctgagcca gctgacccat ggcatcaaag 420
agctgggccc ctacaccctg gacaggcaca gtctctatgt caatg 465

<210> 553
<211> 401
<212> DNA
<213> Homo sapiens

<400> 553
aacgattcga ccttctcctg ggacctccac agtggacctt gggtcaggga ctccatcctc 60
cctccccagc cccacaactg ctggccctct cctggtgccg ttcaccctca acttcaccat 120
caccaacctg gagtacgagg aggacatgca ttgccctggc tccaggaagt tcaacaccac 180
agagagagtc ctgcagagtc tgcttggtcc catgttcaag aacaccagtg ttggccctct 240
gtactctggc tgcagactga ccttgctcag gtccgagaag gatggagcag ccactggagt 300
ggatgccatc tgcacccacc gtcttgaccc caaaagccct ggagtggaca gggagcagct 360
atactgggag ctgagccagc tgaccaatgg catcaaagaa a 401

<210> 554
<211> 385
<212> DNA
<213> Homo sapiens

<400> 554
tcctgggacc tccacagtgg accttgggtc ctcagggact ccatcctccc tccccagccc 60
tacatctgct ggccctctcc tggtgccatt caccctcaac ttcaccatca ccaacctgca 120
gtacgaggag gacatgcatc acccaggctc caggaagttc aacaccacgg agcgggtcct 180
gcagggtctg cttggtccca tgttcaagaa caccagtgtc ggccttctgt actctggctg 240
cagactgacc ttgctcaggc ctgagaagaa tggggcagcc actggaatgg atgccatctg 300
cagccaccgt cttgacccca aaagccctgg actcaacaga gagcagctgt actgggagct 360
gagccagctg acccatggca tcaaa 385

<210> 555
<211> 173
<212> DNA
<213> Homo sapiens

<400> 555
gtctgagaag gatggggcag ccactggagt ggatgccatc tgcacccacc accttaaccc 60
tcaaagccct ggactggaca gggagcagct gtactggcag ctgagccaga tgaccaatgg 120
catcaaagag ctgggcccct acaccctgga ccggaacagt ctctacgtca atg 173

<210> 556
<211> 468
<212> DNA
<213> Homo sapiens

<400> 556
gtttcaccca tcggagctct gggctcacca ccagcactcc ttggacttcc acagttgacc 60
ttggaacctc agggactcca tcccccgtcc ccagccccac aactgctggc cctctcctgg 120
tgccattcac cctaaacttc accatcacca acctgcagta tgaggaggac atgcatcgcc 180
ctggatctag gaagttcaac gccacagaga gggtcctgca gggtctgctt agtcccatat 240
tcaagaactc cagtgttggc cctctgtact ctggctgcag actgacctct ctcaggcccg 300
agaaggatgg ggcagcaact ggaatggatg ctgtctgcct ctaccaccct aatcccaaaa 360
gacctgggct ggacagagag cagctgtact gggagctaag ccagctgacc cacaacatca 420
ctgagctggg cccctacagc ctggacaggc acagtctcta tgtcaatg 468

<210> 557
<211> 468
<212> DNA
<213> Homo sapiens

<400> 557
gtttcaccca tcagaactct gtgcccacca ccagtactcc tgggacctcc acagtgtact 60
gggcaaccac tgggactcca tcctccttcc ccggccacac agagcctggc cctctcctga 120
taccattcac tttcaacttt accatcacca acctgcatta tgaggaaaac atgcaacacc 180
ctggttccag gaagttcaac accacggaga gggttctgca gggtctgctc acgcccttgt 240
tcaagaacac cagtgttggc cctctgtact ctggctgcag actgaccttg ctcagacctg 300
agaagcagga ggcagccact ggagtggaca ccatctgtac ccaccgcgtt gatcccatcg 360
gacctggact ggacagagag cggctatact gggagctgag ccagctgacc aacagcatca 420
cagagctggg accctacacc ctggataggg acagtctcta tgtcaatg 468

<210> 558
<211> 468
<212> DNA
<213> Homo sapiens

<400> 558
gcttcaaccc ttggagctct gtgccaacca ccagcactcc tgggacctcc acagtgcacc 60
tggcaacctc tgggactcca tcctccctgc ctggccacac agcccctgtc cctctcttga 120
taccattcac cctcaacttt accatcacca acctgcatta tgaagaaaac atgcaacacc 180
ctggttccag gaagttcaac accacggaga gggttctgca gggtctgctc aagcccttgt 240
tcaagagcac cagcgttggc cctctgtact ctggctgcag actgaccttg ctcagacctg 300
agaaacatgg ggcagccact ggagtggacg ccatctgcac cctccgcctt gatcccactg 360
gtcctggact ggacagagag cggctatact gggagctgag ccagctgacc aacagcgtta 420
cagagctggg cccctacacc ctggacaggg acagtctcta tgtcaatg 468

<210> 559
<211> 468
<212> DNA
<213> Homo sapiens

<400> 559
gcttcaccca tcggagctct gtgccaacca ccagtattcc tgggacctct gcagtgcacc 60
tggaaacctc tgggactcca gcctccctcc ctggccacac agcccctggc cctctcctgg 120
tgccattcac cctcaacttc actatcacca acctgcagta tgaggaggac atgcgtcacc 180
ctggttccag gaagttcaac accacggaga gagtcctgca gggtctgctc aagcccttgt 240
tcaagagcac cagtgttggc cctctgtact ctggctgcag actgaccttg ctcaggcctg 300
aaaaacgtgg ggcagccacc ggcgtggaca ccatctgcac tcaccgcctt gaccctctaa 360
accctggact ggacagagag cagctatact gggagctgag caaactgacc tgtggcatca 420
tcgagctggg cccctacctc ctggacagag gcagtctcta tgtcaatg 468

<210> 560
<211> 468
<212> DNA
<213> Homo sapiens

<400> 560
gtttcaccca tcggaacttt gtgcccatca ccagcactcc tgggacctcc acagtacacc 60
taggaacctc tgaaactcca tcctccctac ctagacccat agtgcctggc cctctcctgg 120
tgccattcac cctcaacttc accatcacca acttgcagta tgaggaggcc atgcgacacc 180
ctggctccag gaagttcaat accacggaga gggtcctaca gggtctgctc aggcccttgt 240
tcaagaatac cagtatcggc cctctgtact ccagctgcag actgaccttg ctcaggccag 300
agaaggacaa ggcagccacc agagtggatg ccatctgtac ccaccaccct gaccctcaaa 360
gccctggact gaacagagag cagctgtact gggagctgag ccagctgacc cacggcatca 420
ctgagctggg cccctacacc ctggacaggg acagtctcta tgtcgatg 468

<210> 561

<211> 468
<212> DNA
<213> Homo sapiens

<400> 561
gtttcactca ttggagcccc ataccaacca ccagcactcc tgggacctcc atagtgaacc 60
tgggaacctc tgggatccca ccttccctcc ctgaaactac agccaccggc cctctcctgg 120
tgccattcac actcaacttc accatcacta acctacagta tgaggagaac atgggtcacc 180
ctggctccag gaagttcaac atcacggaga gtgttctgca gggtctgctc aagcccttgt 240
tcaagagcac cagtgttggc cctctgtatt ctggctgcag actgaccttg ctcaggcctg 300
agaaggacgg agtagccacc agagtggacg ccatctgcac ccaccgccct gaccccaaaa 360
tccctgggct agacagacag cagctatact gggagctgag ccagctgacc cacagcatca 420
ctgagctggg accctacacc ctggataggg acagtctcta tgtcaatg 468

<210> 562
<211> 407
<212> DNA
<213> Homo sapiens

<400> 562
gtttcaccca gcggagctct gtgcccacca ccagcaccac tggccctgtc ctgctgccat 60
tcaccctcaa ttttaccatc attaacctgc agtatgagga ggacatgcat cgccctggct 120
ccaggaagtt caacaccacg gagagggtcc ttcagggtct gcttatgccc ttgttcaaga 180
acaccagtgt cagctctctg tactctggtt gcagactgac cttgctcagg cctgagaagg 240
atggggcagc caccagagtg gatgctgtct gcacccatcg tcctgacccc aaaagccctg 300
gactggacag agagcggctg tactggaagc tgagccagct gacccacggc atcactgagc 360
tgggccccta caccctggac aggcacagtc tctatgtcaa tggtttc 407

<210> 563
<211> 468
<212> DNA
<213> Homo sapiens

<400> 563
gtttcaccca tcagagctct atgacgacca ccagaactcc tgatacctcc acaatgcacc 60
tggcaacctc gagaactcca gcctccctgt ctggacctac gaccgccagc cctctcctgg 120
tgctattcac aattaacttc accatcacta acctgcggta tgaggagaac atgcatcacc 180
ctggctctag aaagtttaac accacggaga gagtccttca gggtctgctc aggcctgtgt 240
tcaagaacac cagtgttggc cctctgtact ctggctgcag actgaccttg ctcaggccca 300
agaaggatgg ggcagccacc aaagtggatg ccatctgcac ctaccgccct gatcccaaaa 360
gccctggact ggacagagag cagctatact gggagctgag ccagctaacc cacagcatca 420
ctgagctggg cccctacacc ctggacaggg acagtctcta tgtcaatg 468

<210> 564
<211> 468
<212> DNA
<213> Homo sapiens

<400> 564
gtttcacaca gcggagctct gtgcccacca ctagcattcc tgggaccccc acagtggacc 60
tgggaacatc tgggactcca gtttctaaac ctggtccctc ggctgccagc cctctcctgg 120
tgctattcac tctcaacttc accatcacca acctgcggta tgaggagaac atgcagcacc 180
ctggctccag gaagttcaac accacggaga gggtccttca gggcctgctc aggtccctgt 240
tcaagagcac cagtgttggc cctctgtact ctggctgcag actgactttg ctcaggcctg 300
aaaaggatgg gacagccact ggagtggatg ccatctgcac ccaccaccct gaccccaaaa 360
gccctaggct ggacagagag cagctgtatt gggagctgag ccagctgacc cacaatatca 420
ctgagctggg ccactatgcc ctggacaacg acagcctctt tgtcaatg 468

<210> 565
<211> 465
<212> DNA
<213> Homo sapiens

<400> 565
gtttcactca tcggagctct gtgtccacca ccagcactcc tgggaccccc acagtgtatc 60
tgggagcatc taagactcca gcctcgatat ttggcccttc agctgccagc catctcctga 120
tactattcac cctcaacttc accatcacta acctgcggta tgaggagaac atgtggcctg 180
gctccaggaa gttcaacact acagagaggg tccttcaggg cctgctaagg cccttgttca 240
agaacaccag tgttggccct ctgtactctg gctgcaggct gaccttgctc aggccagaga 300
aagatgggga agccaccgga gtggatgcca tctgcaccca ccgccctgac cccacaggcc 360
ctgggctgga cagagagcag ctgtatttgg agctgagcca gctgacccac agcatcactg 420
agctgggccc ctacacactg gacagggaca gtctctatgt caatg 465

<210> 566
<211> 402
<212> DNA
<213> Homo sapiens

<400> 566
gtttcaccca tcggagctct gtacccacca ccagcaccgg ggtggtcagc gaggagccat 60
tcacactgaa cttcaccatc aacaacctgc gctacatggc ggacatgggc caacccggct 120
ccctcaagtt caacatcaca gacaacgtca tgaagcacct gctcagtcct ttgttccaga 180
ggagcagcct gggtgcacgg tacacaggct gcagggtcat cgcactaagg tctgtgaaga 240
acggtgctga gacacgggtg gacctcctct gcacctacct gcagcccctc agcggcccag 300
gtctgcctat caagcaggtg ttccatgagc tgagccagca gacccatggc atcacccggc 360
tgggccccta ctctctggac aaagacagcc tctaccttaa cg 402

<210> 567
<211> 450
<212> DNA
<213> Homo sapiens

<400> 567
gttacaatga acctggtcta gatgagcctc ctacaactcc caagccagcc accacattcc 60
tgcctcctct gtcagaagcc acaacagcca tggggtacca cctgaagacc ctcacactca 120
acttcaccat ctccaatctc cagtattcac cagatatggg caagggctca gctacattca 180
actccaccga gggggtcctt cagcacctgc tcagaccctt gttccagaag agcagcatgg 240
gccccttcta cttgggttgc caactgatct ccctcaggcc tgagaaggat ggggcagcca 300
ctggtgtgga caccacctgc acctaccacc ctgaccctgt gggccccggg ctggacatac 360
agcagcttta ctgggagctg agtcagctga cccatggtgt cacccaactg ggcttctatg 420
tcctggacag ggatagcctc ttcatcaatg 450

<210> 568
<211> 1060
<212> DNA
<213> Homo sapiens

<220>
<221> misc_feature
<222> 406,742,801
<223> n = A,T,C or G

<400> 568
gctatgcacc ccagaattta tcaatccggg gcgagtacca gataaatttc cacattgtca 60

actggaacct cagtaatcca gaccccacat cctcagagta catcaccctg ctgagggaca 120
tccaggacaa ggtcaccaca ctctacaaag gcagtcaact acatgacaca ttccgcttct 180
gcctggtcac caacttgacg atggactccg tgttggtcac tgtcaaggca ttgttctcct 240
ccaatttgga ccccagcctg gtggagcaag tctttctaga taagaccctg aatgcctcat 300
tccattggct gggctccacc taccagttgg tggacatcca tgtgacagaa atggagtcat 360
cagtttatca accaacaagc agctccagca cccagcactt ctaccygaat ttcaccatca 420
ccaacctacc atattcccag gacaaagccc agccaggcac caccaattac cagaggaaca 480
aaaggaatat tgaggatgcg ctcaaccaac tcttccgaaa cagcagcatc aagagttatt 540
tttctgactg tcaagtttca acattcaggt ctgtccccaa caggcaccac accggggtgg 600
actccctgtg taacttctcg ccactggctc ggagagtaga cagagttgcc atctatgagg 660
aatttctgcg gatgacccgg aatggtaccc agctgcagaa cttcaccctg gacaggagca 720
gtgtccttgt ggatgggtat tytcccaaca gaaatgagcc cttaactggg aattctgacc 780
ttcccttctg ggctgtcatc ytcatcggct tggcaggact cctgggactc atcacatgcc 840
tgatctgcgg tgtcctggtg accacccgcc ggcggaagaa ggaaggagaa tacaacgtcc 900
agcaacagtg cccaggctac taccagtcac acctagacct ggaggatctg caatgactgg 960
aacttgccgg tgcctggggt gcctttcccc cagccagggt ccaaagaagc ttggctgggg 1020
cagaaataaa ccatattggt cggacacaaa aaaaaaaaaa 1060

<210> 569
<211> 10622
<212> DNA
<213> Homo sapiens

<220>
<221> misc_feature
<222> 1,691,1164,1165,1730,2015,2149,2785,3044,3163,
      4483,4632,4825,4841,4849,4883,4915,4932,4947,6355,
      6370,7716,8210,9131,9968,10304,10363
<223> n = A,T,C or G

<400> 569
ncatccccag ctcgaacagc agccacagtc ccattcatgg tgccattcac cctcaacttc 60
aactcatcac caacctgcag tacgaggagg acatgcggca cctggttcca ggaagttcaa 120
cgcgcacaga gagagaactg cagggtcgtg ctcaaaccct agatcaggaa tagcagtctg 180
gaatacctct attcaggctg cagactagcc tcactcaggc cagagaagga tagctcagcc 240
acggcagtgg atgccatctg cacacatcgc cctgaccctg aagacctcgg actggacaga 300
gagcgactgt actgggagct gagcaatctg acaaatggca tccaggagct gggcccctac 360
accctggacc ggaacagtct ctatgtcaat ggtttcaccc atcgaagctc tatgcccacc 420
accagcactc ctgggacctc cacagtggat gtgggaacct cagggactcc atcctccagc 480
cccagcccca cgactgctgg ccctctcctg atgccgttca ccctcaactt caccatcacc 540
aacctgcagt acgaggagga catgcgtcgc actggctcca ggaagttcaa caccatggag 600
agtgtcctgc agggtctgct caagcccttg ttcaagaaca ccagtgttgg ccctctgtac 660
tctggctgca gattgacctt gctcaggccc ragaaagatg gggcagccac tggagtggat 720
gccatctgca cccaccgcct tgaccccaaa agccctggac tcaacaggga gcagctgtac 780
tgggagctaa gcaaactgac caatgacatt gaagagctgg gcccctacac cctggacagg 840
aacagtctct atgtcaatgg tttcacccat cagagctctg tgtccaccac cagcactcct 900
gggacctcca cagtggatct cagaacctca gtgactccat cctccctctc cagccccaca 960
attatggctg ctggccctct cctggtacca ttcaccctca acttcaccat caccaacctg 1020
cagtatgggg aggacatggg tcaccctggc tccaggaagt tcaacaccac agagagggtc 1080
ctgcagggtc tgcttggtcc catattcaag aacaccagtg ttggccctct gtactctggc 1140
tgcagactga cctctctcag gtcyragaag gatggagcag ccactggagt ggatgccatc 1200
tgcatccatc atcttgaccc caaaagccct ggactcaaca gagagcggct gtactgggag 1260
ctgagccaac tgaccaatgg catcaaagag ctgggcccct acaccctgga caggaacagt 1320
ctctatgtca atgctgctgg ccctctcctg gtgctgttca ccctcaactt caccatcacc 1380
aacctgaagt atgaggagga catgcatcgc cctggctcca ggaagttcaa caccactgag 1440
agggtcctgc agactctgcg tggtcctatg ttcaagaaca ccagtggtgg ccttctgtac 1500

tctggctgca gactgacctt gctcaggtcc gagaaggatg gagcagccac tggagtggat 1560
gccatctgca cccaccgtct tgaccccaaa agccctggag tggacaggga gcagctatac 1620
tgggagctga gccagctgac caatggcatc aaagagctgg gcccctacac cctggacagg 1680
aacagtctct atgtcaatgg tttcacccat cggacctctg tgcccaccas cagcactcct 1740
gggacctcca cagtggacct tggaacctca gggactccat tctccctccc aagccccgca 1800
actgctggcc ctctcctggt gctgttcacc ctcaacttca ccatcaccaa cctgaagtat 1860
gaggaggaca tgcatcgccc tggctccagg aagttcaaca ccactgagag ggtcctgcag 1920
actctgcttg gtcctatgtt caagaacacc agtgttggcc ttctgtactc tggctgcaga 1980
ctgaccttgc tcaggtccga gaaggatgga gcagycactg gagtggatgc catctgcacc 2040
caccgtcttg accccaaaag ccctggagtg gacagggagc agctatactg ggagctgagc 2100
cagctgacca atggcatcaa agagctgggc ccctacaccc tggacaggma cagtctctat 2160
gtcaatggtt tcacccattg gatccctgtg cccaccagca gcactcctgg gacctccaca 2220
gtggaccttg ggtcagggac tccatcctcc ctccccagcc ccacaactgc tggccctctc 2280
ctggtgccat tcaccctcaa cttcaccatc accaacctgc agtacgagga ggacatgcat 2340
cacccaggct ccaggaagtt caacaccacg gagcgggtcc tgcagggtct gcttggtccc 2400
atgttcaaga acaccagtgt cggccttctg tactctggct gcagactgac cttgctcagg 2460
cctgagaaga atggggcagc cactggaatg gatgccatct gcagccaccg tcttgacccc 2520
aaaagccctg gactcaacag agagcagctg tactgggagc tgagccagct gacccatggc 2580
atcaaagagc tgggccccta caccctggac aggcacagtc tctatgtcaa tggtttcacc 2640
cattggatcc ctgtgcccac cagcagcact cctgggacct ccacagtgga ccttgggtca 2700
gggactccat cctccctccc cagccccaca actgctggcc ctctcctggt gccgttcacc 2760
ctcaacttca ccatcaccaa cctgragtac gaggaggaca tgcattgccc tggctccagg 2820
aagttcaaca ccacagagag agtcctgcag agtctgcttg gtcccatgtt caagaacacc 2880
agtgttggcc ctctgtactc tggctgcaga ctgaccttgc tcaggtccga gaaggatgga 2940
gcagccactg gagtggatgc catctgcacc caccgtcttg accccaaaag ccctggagtg 3000
gacagggagc agctatactg ggagctgagc cagctgacca atgscatcaa agagctgggt 3060
ccctacaccc tggacagcaa cagtctctat gtcaatggtt tcacccatca gacctctgcg 3120
cccaacacca gcactcctgg gacctccaca gtggaccttg ggwcctcagg gactccatcc 3180
tccctcccca gccctacatc tgctggccct ctcctggtgc cattcaccct caacttcacc 3240
atcaccaacc tgcagtacga ggaggacatg catcacccag gctccaggaa gttcaacacc 3300
acggagcggg tcctgcaggg tctgcttggt cccatgttca agaacaccag tgtcggcctt 3360
ctgtactctg gctgcagact gaccttgctc aggcctgaga agaatggggc agccactgga 3420
atggatgcca tctgcagcca ccgtcttgac cccaaaagcc ctggactcaa cagagagcag 3480
ctgtactggg agctgagcca gctgacccat ggcatcaaag agctgggccc ctacaccctg 3540
gacaggaaca gtctctatgt caatggtttc acccatcgga gctctgtggc ccccaccagc 3600
actcctggga cctccacagt ggaccttggg acctcaggga ctccatcctc cctccccagc 3660
cccacaacag ctgttcctct cctggtgccg ttcaccctca actttaccat caccaatctg 3720
cagtatgggg aggacatgcg tcaccctggc tccaggaagt tcaacaccac agagagggtc 3780
ctgcagggtc tgcttggtcc cttgttcaag aactccagtg tcggccctct gtactctggc 3840
tgcagactga tctctctcag gtctgagaag gatggggcag ccactggagt ggatgccatc 3900
tgcacccacc accttaaccc tcaaagccct ggactggaca gggagcagct gtactggcag 3960
ctgagccaga tgaccaatgg catcaaagag ctgggcccct acaccctgga ccggaacagt 4020
ctctacgtca atggtttcac ccatcggagc tctgggctca ccaccagcac tccttggact 4080
tccacagttg accttggaac ctcagggact ccatcccccg tccccagccc cacaactgct 4140
ggccctctcc tggtgccatt caccctaaac ttcaccatca ccaacctgca gtatgaggag 4200
gacatgcatc gccctggatc taggaagttc aacgccacag agagggtcct gcagggtctg 4260
cttagtccca tattcaagaa ctccagtgtt ggccctctgt actctggctg cagactgacc 4320
tctctcaggc ccgagaagga tggggcagca actggaatgg atgctgtctg cctctaccac 4380
cctaatccca aaagacctgg gctggacaga gagcagctgt actgggagct aagccagctg 4440
acccacaaca tcactgagct gggcccctac agcctggaca ggsacagtct ctatgtcaat 4500
ggtttcaccc atcagaactc tgtgcccacc accagtactc ctgggacctc cacagtgtac 4560
tgggcaacca ctgggactcc atcctccttc cccggccaca cagagcctgg ccctctcctg 4620
ataccattca cwttcaactt taccatcacc aacctgcatt atgaggaaaa catgcaacac 4680
cctggttcca ggaagttcaa caccacggag agggttctgc agggtctgct cacgcccttg 4740
ttcaagaaca ccagtgttgg ccctctgtac tctggctgca gactgacctt gctcagacct 4800
gagaagcagg aggcagccac tggartggac accatctgta yccaccgcst tgatcccatc 4860
ggacctggac tggacagaga gcrgctatac tgggagctga gccagctgac ccacrgcatc 4920

acagagctgg gmccctacac cctggayagg gacagtctct atgtcaatgg cttcaaccct 4980
tggagctctg tgccaaccac cagcactcct gggacctcca cagtgcacct ggcaacctct 5040
gggactccat cctccctgcc tggccacaca gcccctgtcc ctctcttgat accattcacc 5100
ctcaacttta ccatcaccaa cctgcattat gaagaaaaca tgcaacaccc tggttccagg 5160
aagttcaaca ccacggagag ggttctgcag ggtctgctca agcccttgtt caagagcacc 5220
agcgttggcc ctctgtactc tggctgcaga ctgaccttgc tcagacctga gaaacatggg 5280
gcagccactg gagtggacgc catctgcacc ctccgccttg atcccactgg tcctggactg 5340
gacagagagc ggctatactg ggagctgagc cagctgacca acagcgttac agagctgggc 5400
ccctacaccc tggacaggga cagtctctat gtcaatggct tcacccatcg gagctctgtg 5460
ccaaccacca gtattcctgg gacctctgca gtgcacctgg aaacctctgg gactccagcc 5520
tccctccctg gccacacagc ccctggccct ctcctggtgc cattcaccct caacttcact 5580
atcaccaacc tgcagtatga ggaggacatg cgtcaccctg gttccaggaa gttcaacacc 5640
acggagagag tcctgcaggg tctgctcaag cccttgttca agagcaccag tgttggccct 5700
ctgtactctg gctgcagact gaccttgctc aggcctgaaa aacgtggggc agccaccggc 5760
gtggacacca tctgcactca ccgccttgac cctctaaacc ctggactgga cagagagcag 5820
ctatactggg agctgagcaa actgacctgt ggcatcatcg agctgggccc ctacctcctg 5880
gacagaggca gtctctatgt caatggtttc acccatcgga actttgtgcc catcaccagc 5940
actcctggga cctccacagt acacctagga acctctgaaa ctccatcctc cctacctaga 6000
cccatagtgc ctggccctct cctggtgcca ttcaccctca acttcaccat caccaacttg 6060
cagtatgagg aggccatgcg acaccctggc tccaggaagt tcaataccac ggagagggtc 6120
ctacagggtc tgctcaggcc cttgttcaag aataccagta tcggccctct gtactccagc 6180
tgcagactga ccttgctcag gccagagaag gacaaggcag ccaccagagt ggatgccatc 6240
tgtacccacc accctgaccc tcaaagccct ggactgaaca gagagcagct gtactgggag 6300
ctgagccagc tgacccacgg catcactgag ctgggcccct acaccctgga caggsacagt 6360
ctctatgtcr atggtttcac tcattggagc cccataccaa ccaccagcac tcctgggacc 6420
tccatagtga acctgggaac ctctgggatc ccaccttccc tccctgaaac tacagccacc 6480
ggccctctcc tggtgccatt cacactcaac ttcaccatca ctaacctaca gtatgaggag 6540
aacatgggtc accctggctc caggaagttc aacatcacgg agagtgttct gcagggtctg 6600
ctcaagccct tgttcaagag caccagtgtt ggccctctgt attctggctg cagactgacc 6660
ttgctcaggc ctgagaagga cggagtagcc accagagtgg acgccatctg cacccaccgc 6720
cctgacccca aaatccctgg gctagacaga cagcagctat actgggagct gagccagctg 6780
acccacagca tcactgagct gggaccctac accctggata gggacagtct ctatgtcaat 6840
ggtttcaccc agcggagctc tgtgcccacc accagcactc ctgggacttt cacagtacag 6900
ccggaaacct ctgagactcc atcatccctc cctggcccca cagccactgg ccctgtcctg 6960
ctgccattca ccctcaattt taccatcatt aacctgcagt atgaggagga catgcatcgc 7020
cctggctcca ggaagttcaa caccacggag agggtccttc agggtctgct tatgcccttg 7080
ttcaagaaca ccagtgtcag ctctctgtac tctggttgca gactgacctt gctcaggcct 7140
gagaaggatg gggcagccac cagagtggat gctgtctgca cccatcgtcc tgaccccaaa 7200
agccctggac tggacagaga gcggctgtac tggaagctga gccagctgac ccacggcatc 7260
actgagctgg gcccctacac cctggacagg cacagtctct atgtcaatgg tttcacccat 7320
cagagctcta tgacgaccac cagaactcct gatacctcca caatgcacct ggcaacctcg 7380
agaactccag cctccctgtc tggacctacg accgccagcc ctctcctggt gctattcaca 7440
attaacttca ccatcactaa cctgcggtat gaggagaaca tgcatcaccc tggctctaga 7500
aagtttaaca ccacggagag agtccttcag ggtctgctca ggcctgtgtt caagaacacc 7560
agtgttggcc ctctgtactc tggctgcaga ctgaccttgc tcaggcccaa gaaggatggg 7620
gcagccacca aagtggatgc catctgcacc taccgccctg atcccaaaag ccctggactg 7680
gacagagagc agctatactg ggagctgagc cagctraccc acagcatcac tgagctgggc 7740
ccctacaccc tggacaggga cagtctctat gtcaatggtt tcacacagcg gagctctgtg 7800
cccaccacta gcattcctgg gacccccaca gtggacctgg gaacatctgg gactccagtt 7860
tctaaacctg gtccctcggc tgccagccct ctcctggtgc tattcactct caacttcacc 7920
atcaccaacc tgcggtatga ggagaacatg cagcaccctg gctccaggaa gttcaacacc 7980
acggagaggg tccttcaggg cctgctcagg tccctgttca agagcaccag tgttggccct 8040
ctgtactctg gctgcagact gactttgctc aggcctgaaa aggatggaac agccactgga 8100
gtggatgcca tctgcaccca ccaccctgac cccaaaagcc ctaggctgga cagagagcag 8160
ctgtattggg agctgagcca gctgacccac aatatcactg agctgggccm ctatgccctg 8220
gacaacgaca gcctctttgt caatggtttc actcatcgga gctctgtgtc caccaccagc 8280
actcctggga cccccacagt gtatctggga gcatctaaga ctccagcctc gatatttggc 8340

ccttcagctg ccagccatct cctgatacta ttcaccctca acttcaccat cactaacctg 8400
cggtatgagg agaacatgtg gcctggctcc aggaagttca acactacaga gagggtcctt 8460
cagggcctgc taaggccctt gttcaagaac accagtgttg gccctctgta ctctggctgc 8520
aggctgacct tgctcaggcc agagaaagat ggggaagcca ccggagtgga tgccatctgc 8580
acccaccgcc ctgaccccac aggccctggg ctggacagag agcagctgta tttggagctg 8640
agccagctga cccacagcat cactgagctg ggcccctaca cactggacag ggacagtctc 8700
tatgtcaatg gtttcaccca tcggagctct gtacccacca ccagcaccgg ggtggtcagc 8760
gaggagccat tcacactgaa cttcaccatc aacaacctgc gctacatggc ggacatgggc 8820
caacccggct ccctcaagtt caacatcaca gacaacgtca tgaagcacct gctcagtcct 8880
ttgttccaga ggagcagcct gggtgcacgg tacacaggct gcagggtcat cgcactaagg 8940
tctgtgaaga acggtgctga gacacgggtg gacctcctct gcacctacct gcagcccctc 9000
agcggcccag gtctgcctat caagcaggtg ttccatgagc tgagccagca gacccatggc 9060
atcacccggc tgggccccta ctctctggac aaagacagcc tctaccttaa cggttacaat 9120
gaacctggtc yagatgagcc tcctacaact cccaagccag ccaccacatt cctgcctcct 9180
ctgtcagaag ccacaacagc catggggtac cacctgaaga ccctcacact caacttcacc 9240
atctccaatc tccagtattc accagatatg ggcaagggct cagctacatt caactccacc 9300
gagggggtcc ttcagcacct gctcagaccc ttgttccaga agagcagcat gggccccttc 9360
tacttgggtt gccaactgat ctccctcagg cctgagaagg atggggcagc cactggtgtg 9420
gacaccacct gcacctacca ccctgaccct gtgggccccg ggctggacat acagcagctt 9480
tactgggagc tgagtcagct gacccatggt gtcacccaac tgggcttcta tgtcctggac 9540
agggatagcc tcttcatcaa tggctatgca ccccagaatt tatcaatccg gggcgagtac 9600
cagataaatt tccacattgt caactggaac ctcagtaatc cagaccccac atcctcagag 9660
tacatcaccc tgctgaggga catccaggac aaggtcacca cactctacaa aggcagtcaa 9720
ctacatgaca cattccgctt ctgcctggtc accaacttga cgatggactc cgtgttggtc 9780
actgtcaagg cattgttctc ctccaatttg gaccccagcc tggtggagca agtctttcta 9840
gataagaccc tgaatgcctc attccattgg ctgggctcca cctaccagtt ggtggacatc 9900
catgtgacag aaatggagtc atcagtttat caaccaacaa gcagctccag cacccagcac 9960
ttctaccyga atttcaccat caccaaccta ccatattccc aggacaaagc ccagccaggc 10020
accaccaatt accagaggaa caaaaggaat attgaggatg cgctcaacca actcttccga 10080
aacagcagca tcaagagtta tttttctgac tgtcaagttt caacattcag gtctgtcccc 10140
aacaggcacc acaccggggt ggactccctg tgtaacttct cgccactggc tcggagagta 10200
gacagagttg ccatctatga ggaatttctg cggatgaccc ggaatggtac ccagctgcag 10260
aacttcaccc tggacaggag cagtgtcctt gtggatgggt attytcccaa cagaaatgag 10320
cccttaactg ggaattctga ccttcccttc tgggctgtca tcytcatcgg cttggcagga 10380
ctcctgggac tcatcacatg cctgatctgc ggtgtcctgg tgaccacccg ccggcggaag 10440
aaggaaggag aatacaacgt ccagcaacag tgcccaggct actaccagtc acacctagac 10500
ctggaggatc tgcaatgact ggaacttgcc ggtgcctggg gtgcctttcc cccagccagg 10560
gtccaaagaa gcttggctgg ggcagaaata aaccatattg gtcggacaca aaaaaaaaaa 10620
aa 10622

<210> 570
<211> 469
<212> DNA
<213> Homo sapiens

<220>
<221> misc_feature
<222> 71,92,93,120,124,168,178,218,230,300,
321,350,387,412,414,415,422,423,451
<223> n = A,T,C or G

<400> 570
gtttcaccca tcggagctct gtgcccacca ccagcactcc tgggacctcc acagtggacc 60
tgggaacctc wgggactcca tcctccctcc cyrgccccac agctgctggc cctctcctgr 120
tgcyattcac cctcaacttc accatcacca acctgcagta tgaggagrac atgcatcrcc 180
ctggctccag gaagttcaac accacggaga gggtcctkca gggtctgcty aggtcccttg 240
ttcaagaaca ccagtgttgg ccctctgtac tctggctgca gactgacctt gctcaggccy 300

gagaaggatg gggcagccac yggagtggat gccatctgca cccaccgccy tgaccccaaa 360
agccctggac tggacagaga gcagctrtac tgggagctga gccagctgac cmayrgcatc 420
amwgagctgg gcccctacac cctggacagg racagtctct atgtcaatg 469

<210> 571
<211> 130
<212> PRT
<213> Homo sapiens

<220>
<221> variant
<222> 69,107,110
<223> Xaa = Any amino acid

<400> 571
His Pro Gln Leu Glu Gln Gln Pro Gln Ser His Ser Trp Cys His Ser
                5                   10                  15

Pro Ser Thr Ser Thr His His Gln Pro Ala Val Arg Gly Gly His Ala
            20                  25                  30

Ala Pro Gly Ser Arg Lys Phe Asn Ala His Arg Glu Arg Thr Ala Gly
        35                  40                  45

Ser Cys Ser Asn Pro Arg Ser Gly Ile Ala Val Trp Asn Thr Ser Ile
    50                  55                  60

Gln Ala Ala Asp Xaa Pro His Ser Gly Gln Arg Arg Ile Ala Gln Pro
65                  70                  75                  80

Arg Gln Trp Met Pro Ser Ala His Ile Ala Leu Thr Leu Lys Thr Ser
                85                  90                  95

Asp Trp Thr Glu Ser Asp Cys Thr Gly Ser Xaa Ala Ile Xaa Gln Met
            100                 105                 110

Ala Ser Arg Ser Trp Ala Pro Thr Pro Trp Thr Gly Thr Val Ser Met
        115                 120                 125

Ser Met
    130

<210> 572
<211> 130
<212> PRT
<213> Homo sapiens

<220>
<221> variant
<222> 1,58,78,92,94
<223> Xaa = Any amino acid

<400> 572
Xaa Ile Pro Ser Ser Asn Ser Ser His Ser Pro Ile His Gly Ala Ile
                5                   10                  15

His Pro Gln Leu Gln Leu Ile Thr Asn Leu Gln Tyr Glu Glu Asp Met
            20                  25                  30

Arg His Leu Val Pro Gly Ser Ser Thr Arg Thr Glu Arg Glu Leu Gln
        35                  40                  45

Gly Arg Ala Gln Thr Leu Asp Gln Glu Xaa Gln Ser Gly Ile Pro Leu
    50                  55                  60

Phe Arg Leu Gln Thr Ser Leu Thr Gln Ala Arg Glu Gly Xaa Leu Ser
65                  70                  75                  80

His Gly Ser Gly Cys His Leu His Thr Ser Pro Xaa Pro Xaa Arg Pro
                85                  90                  95

Arg Thr Gly Gln Arg Ala Thr Val Leu Gly Ala Glu Gln Ser Asp Lys
            100                 105                 110

Trp His Pro Gly Ala Gly Pro Leu His Pro Gly Pro Glu Gln Ser Leu
        115                 120                 125

Cys Gln
    130


<210> 573
<211> 130
<212> PRT
<213> Homo sapiens

<220>
<221> variant
<222> 1,54
<223> Xaa = Any amino acid

<400> 573
Xaa Ser Pro Ala Arg Thr Ala Ala Thr Val Pro Phe Met Val Pro Phe
                5                   10                  15

Thr Leu Asn Phe Asn Ser Ser Pro Thr Cys Ser Thr Arg Arg Thr Cys
            20                  25                  30

Gly Thr Trp Phe Gln Glu Val Gln Arg Ala Gln Arg Glu Asn Cys Arg
        35                  40                  45

Val Val Leu Lys Pro Xaa Ile Arg Asn Ser Ser Leu Glu Tyr Leu Tyr
    50                  55                  60

Ser Gly Cys Arg Leu Ala Ser Leu Arg Pro Glu Lys Asp Ser Ser Ala
65                  70                  75                  80

Thr Ala Val Asp Ala Ile Cys Thr His Arg Pro Asp Pro Glu Asp Leu
                85                  90                  95

Gly Leu Asp Arg Glu Arg Leu Tyr Trp Glu Leu Ser Asn Leu Thr Asn
            100                 105                 110

Gly Ile Gln Glu Leu Gly Pro Tyr Thr Leu Asp Arg Asn Ser Leu Tyr
        115                 120                 125

Val Asn
    130

<210> 574
<211> 156
<212> PRT
<213> Homo sapiens

<220>
<221> variant
<222> 101
<223> Xaa = Any amino acid

<400> 574
Gly Phe Thr His Arg Ser Ser Met Pro Thr Thr Ser Thr Pro Gly Thr
                5                   10                  15

Ser Thr Val Asp Val Gly Thr Ser Gly Thr Pro Ser Ser Ser Pro Ser
            20                  25                  30

Pro Thr Thr Ala Gly Pro Leu Leu Met Pro Phe Thr Leu Asn Phe Thr
        35                  40                  45

Ile Thr Asn Leu Gln Tyr Glu Glu Asp Met Arg Arg Thr Gly Ser Arg
    50                  55                  60

Lys Phe Asn Thr Met Glu Ser Val Leu Gln Gly Leu Leu Lys Pro Leu
65                  70                  75                  80

Phe Lys Asn Thr Ser Val Gly Pro Leu Tyr Ser Gly Cys Arg Leu Thr
                85                  90                  95

Leu Leu Arg Pro Xaa Lys Asp Gly Ala Ala Thr Gly Val Asp Ala Ile
            100                 105                 110

Cys Thr His Arg Leu Asp Pro Lys Ser Pro Gly Leu Asn Arg Glu Gln
        115                 120                 125

Leu Tyr Trp Glu Leu Ser Lys Leu Thr Asn Asp Ile Glu Glu Leu Gly
    130                 135                 140

Pro Tyr Thr Leu Asp Arg Asn Ser Leu Tyr Val Asn
145                 150                 155

<210> 575
<211> 158
<212> PRT
<213> Homo sapiens

<220>
<221> variant
<222> 103

<223> Xaa = Any amino acid

<400> 575
Gly Phe Thr His Gln Ser Ser Val Ser Thr Thr Ser Thr Pro Gly Thr
                5                   10                  15

Ser Thr Val Asp Leu Arg Thr Ser Val Thr Pro Ser Ser Leu Ser Ser
            20                  25                  30

Pro Thr Ile Met Ala Ala Gly Pro Leu Leu Val Pro Phe Thr Leu Asn
        35                  40                  45

Phe Thr Ile Thr Asn Leu Gln Tyr Gly Glu Asp Met Gly His Pro Gly
    50                  55                  60

Ser Arg Lys Phe Asn Thr Thr Glu Arg Val Leu Gln Gly Leu Leu Gly
65                  70                  75                  80

Pro Ile Phe Lys Asn Thr Ser Val Gly Pro Leu Tyr Ser Gly Cys Arg
                85                  90                  95

Leu Thr Ser Leu Arg Ser Xaa Lys Asp Gly Ala Ala Thr Gly Val Asp
            100                 105                 110

Ala Ile Cys Ile His His Leu Asp Pro Lys Ser Pro Gly Leu Asn Arg
        115                 120                 125

Glu Arg Leu Tyr Trp Glu Leu Ser Gln Leu Thr Asn Gly Ile Lys Glu
    130                 135                 140

Leu Gly Pro Tyr Thr Leu Asp Arg Asn Ser Leu Tyr Val Asn
145                 150                 155


<210> 576
<211> 122
<212> PRT
<213> Homo sapiens

<400> 576
Ala Ala Gly Pro Leu Leu Val Leu Phe Thr Leu Asn Phe Thr Ile Thr
                5                   10                  15

Asn Leu Lys Tyr Glu Glu Asp Met His Arg Pro Gly Ser Arg Lys Phe
            20                  25                  30

Asn Thr Thr Glu Arg Val Leu Gln Thr Leu Arg Gly Pro Met Phe Lys
        35                  40                  45

Asn Thr Ser Gly Gly Leu Leu Tyr Ser Gly Cys Arg Leu Thr Leu Leu
    50                  55                  60

Arg Ser Glu Lys Asp Gly Ala Ala Thr Gly Val Asp Ala Ile Cys Thr
65                  70                  75                  80

His Arg Leu Asp Pro Lys Ser Pro Gly Val Asp Arg Glu Gln Leu Tyr
                85                  90                  95

Trp Glu Leu Ser Gln Leu Thr Asn Gly Ile Lys Glu Leu Gly Pro Tyr
            100                 105                 110

Thr Leu Asp Arg Asn Ser Leu Tyr Val Asn
        115                 120


<210> 577
<211> 156
<212> PRT
<213> Homo sapiens

<220>
<221> variant
<222> 11,106,151
<223> Xaa = Any amino acid

<400> 577
Gly Phe Thr His Arg Thr Ser Val Pro Thr Xaa Ser Thr Pro Gly Thr
                  5                  10                  15

Ser Thr Val Asp Leu Gly Thr Ser Gly Thr Pro Phe Ser Leu Pro Ser
             20                  25                  30

Pro Ala Thr Ala Gly Pro Leu Leu Val Leu Phe Thr Leu Asn Phe Thr
         35                  40                  45

Ile Thr Asn Leu Lys Tyr Glu Glu Asp Met His Arg Pro Gly Ser Arg
     50                  55                  60

Lys Phe Asn Thr Thr Glu Arg Val Leu Gln Thr Leu Leu Gly Pro Met
 65                  70                  75                  80

Phe Lys Asn Thr Ser Val Gly Leu Leu Tyr Ser Gly Cys Arg Leu Thr
                 85                  90                  95

Leu Leu Arg Ser Glu Lys Asp Gly Ala Xaa Thr Gly Val Asp Ala Ile
            100                 105                 110

Cys Thr His Arg Leu Asp Pro Lys Ser Pro Gly Val Asp Arg Glu Gln
        115                 120                 125

Leu Tyr Trp Glu Leu Ser Gln Leu Thr Asn Gly Ile Lys Glu Leu Gly
    130                 135                 140

Pro Tyr Thr Leu Asp Arg Xaa Ser Leu Tyr Val Asn
145                 150                 155


<210> 578
<211> 155
<212> PRT
<213> Homo sapiens

<400> 578
Gly Phe Thr His Trp Ile Pro Val Pro Thr Ser Ser Thr Pro Gly Thr

                5                   10                  15

Ser Thr Val Asp Leu Gly Ser Gly Thr Pro Ser Ser Leu Pro Ser Pro
            20                  25                  30

Thr Thr Ala Gly Pro Leu Leu Val Pro Phe Thr Leu Asn Phe Thr Ile
        35                  40                  45

Thr Asn Leu Gln Tyr Glu Glu Asp Met His His Pro Gly Ser Arg Lys
    50                  55                  60

Phe Asn Thr Thr Glu Arg Val Leu Gln Gly Leu Leu Gly Pro Met Phe
65                  70                  75                  80

Lys Asn Thr Ser Val Gly Leu Leu Tyr Ser Gly Cys Arg Leu Thr Leu
                85                  90                  95

Leu Arg Pro Glu Lys Asn Gly Ala Ala Thr Gly Met Asp Ala Ile Cys
            100                 105                 110

Ser His Arg Leu Asp Pro Lys Ser Pro Gly Leu Asn Arg Glu Gln Leu
        115                 120                 125

Tyr Trp Glu Leu Ser Gln Leu Thr His Gly Ile Lys Glu Leu Gly Pro
    130                 135                 140

Tyr Thr Leu Asp Arg His Ser Leu Tyr Val Asn
145                 150                 155

<210> 579
<211> 155
<212> PRT
<213> Homo sapiens

<220>
<221> variant
<222> 52,138
<223> Xaa = Any amino acid

<400> 579
Gly Phe Thr His Trp Ile Pro Val Pro Thr Ser Ser Thr Pro Gly Thr
                5                   10                  15

Ser Thr Val Asp Leu Gly Ser Gly Thr Pro Ser Ser Leu Pro Ser Pro
            20                  25                  30

Thr Thr Ala Gly Pro Leu Leu Val Pro Phe Thr Leu Asn Phe Thr Ile
        35                  40                  45

Thr Asn Leu Xaa Tyr Glu Glu Asp Met His Cys Pro Gly Ser Arg Lys
    50                  55                  60

Phe Asn Thr Thr Glu Arg Val Leu Gln Ser Leu Leu Gly Pro Met Phe
65                  70                  75                  80

Lys Asn Thr Ser Val Gly Pro Leu Tyr Ser Gly Cys Arg Leu Thr Leu

85                  90                  95

Leu Arg Ser Glu Lys Asp Gly Ala Ala Thr Gly Val Asp Ala Ile Cys
            100                 105                 110

Thr His Arg Leu Asp Pro Lys Ser Pro Gly Val Asp Arg Glu Gln Leu
        115                 120                 125

Tyr Trp Glu Leu Ser Gln Leu Thr Asn Xaa Ile Lys Glu Leu Gly Pro
    130                 135                 140

Tyr Thr Leu Asp Ser Asn Ser Leu Tyr Val Asn
145                 150                 155


<210> 580
<211> 156
<212> PRT
<213> Homo sapiens

<220>
<221> variant
<222> 23
<223> Xaa = Any amino acid

<400> 580
Gly Phe Thr His Gln Thr Ser Ala Pro Asn Thr Ser Thr Pro Gly Thr
                5                   10                  15

Ser Thr Val Asp Leu Gly Xaa Ser Gly Thr Pro Ser Ser Leu Pro Ser
            20                  25                  30

Pro Thr Ser Ala Gly Pro Leu Leu Val Pro Phe Thr Leu Asn Phe Thr
        35                  40                  45

Ile Thr Asn Leu Gln Tyr Glu Glu Asp Met His His Pro Gly Ser Arg
    50                  55                  60

Lys Phe Asn Thr Thr Glu Arg Val Leu Gln Gly Leu Leu Gly Pro Met
65                  70                  75                  80

Phe Lys Asn Thr Ser Val Gly Leu Leu Tyr Ser Gly Cys Arg Leu Thr
                85                  90                  95

Leu Leu Arg Pro Glu Lys Asn Gly Ala Ala Thr Gly Met Asp Ala Ile
            100                 105                 110

Cys Ser His Arg Leu Asp Pro Lys Ser Pro Gly Leu Asn Arg Glu Gln
        115                 120                 125

Leu Tyr Trp Glu Leu Ser Gln Leu Thr His Gly Ile Lys Glu Leu Gly
    130                 135                 140

Pro Tyr Thr Leu Asp Arg Asn Ser Leu Tyr Val Asn
145                 150                 155

<210> 581
<211> 156
<212> PRT
<213> Homo sapiens

<400> 581
Gly Phe Thr His Arg Ser Ser Val Ala Pro Thr Ser Thr Pro Gly Thr
                5                   10                  15

Ser Thr Val Asp Leu Gly Thr Ser Gly Thr Pro Ser Ser Leu Pro Ser
            20                  25                  30

Pro Thr Thr Ala Val Pro Leu Leu Val Pro Phe Thr Leu Asn Phe Thr
        35                  40                  45

Ile Thr Asn Leu Gln Tyr Gly Glu Asp Met Arg His Pro Gly Ser Arg
    50                  55                  60

Lys Phe Asn Thr Thr Glu Arg Val Leu Gln Gly Leu Leu Gly Pro Leu
65                  70                  75                  80

Phe Lys Asn Ser Ser Val Gly Pro Leu Tyr Ser Gly Cys Arg Leu Ile
                85                  90                  95

Ser Leu Arg Ser Glu Lys Asp Gly Ala Ala Thr Gly Val Asp Ala Ile
            100                 105                 110

Cys Thr His His Leu Asn Pro Gln Ser Pro Gly Leu Asp Arg Glu Gln
        115                 120                 125

Leu Tyr Trp Gln Leu Ser Gln Met Thr Asn Gly Ile Lys Glu Leu Gly
    130                 135                 140

Pro Tyr Thr Leu Asp Arg Asn Ser Leu Tyr Val Asn
145                 150                 155


<210> 582
<211> 156
<212> PRT
<213> Homo sapiens

<220>
<221> variant
<222> 151
<223> Xaa = Any amino acid

<400> 582
Gly Phe Thr His Arg Ser Ser Gly Leu Thr Thr Ser Thr Pro Trp Thr
                5                   10                  15

Ser Thr Val Asp Leu Gly Thr Ser Gly Thr Pro Ser Pro Val Pro Ser
            20                  25                  30

Pro Thr Thr Ala Gly Pro Leu Leu Val Pro Phe Thr Leu Asn Phe Thr
        35                  40                  45

Ile Thr Asn Leu Gln Tyr Glu Glu Asp Met His Arg Pro Gly Ser Arg
    50                  55                  60

Lys Phe Asn Ala Thr Glu Arg Val Leu Gln Gly Leu Leu Ser Pro Ile
65                  70                  75                  80

Phe Lys Asn Ser Ser Val Gly Pro Leu Tyr Ser Gly Cys Arg Leu Thr
                85                  90                  95

Ser Leu Arg Pro Glu Lys Asp Gly Ala Ala Thr Gly Met Asp Ala Val
            100                 105                 110

Cys Leu Tyr His Pro Asn Pro Lys Arg Pro Gly Leu Asp Arg Glu Gln
        115                 120                 125

Leu Tyr Trp Glu Leu Ser Gln Leu Thr His Asn Ile Thr Glu Leu Gly
    130                 135                 140

Pro Tyr Ser Leu Asp Arg Xaa Ser Leu Tyr Val Asn
145                 150                 155


<210> 583
<211> 156
<212> PRT
<213> Homo sapiens

<220>
<221> variant
<222> 109,114,117,128,139
<223> Xaa = Any amino acid

<400> 583
Gly Phe Thr His Gln Asn Ser Val Pro Thr Thr Ser Thr Pro Gly Thr
                5                   10                  15

Ser Thr Val Tyr Trp Ala Thr Thr Gly Thr Pro Ser Ser Phe Pro Gly
            20                  25                  30

His Thr Glu Pro Gly Pro Leu Leu Ile Pro Phe Thr Phe Asn Phe Thr
        35                  40                  45

Ile Thr Asn Leu His Tyr Glu Glu Asn Met Gln His Pro Gly Ser Arg
    50                  55                  60

Lys Phe Asn Thr Thr Glu Arg Val Leu Gln Gly Leu Leu Thr Pro Leu
65                  70                  75                  80

Phe Lys Asn Thr Ser Val Gly Pro Leu Tyr Ser Gly Cys Arg Leu Thr
                85                  90                  95

Leu Leu Arg Pro Glu Lys Gln Glu Ala Ala Thr Gly Xaa Asp Thr Ile
            100                 105                 110

Cys Xaa His Arg Xaa Asp Pro Ile Gly Pro Gly Leu Asp Arg Glu Xaa
        115                 120                 125

Leu Tyr Trp Glu Leu Ser Gln Leu Thr His Xaa Ile Thr Glu Leu Gly
    130                 135                 140

Pro Tyr Thr Leu Asp Arg Asp Ser Leu Tyr Val Asn
145                 150                 155


<210> 584
<211> 156
<212> PRT
<213> Homo sapiens

<400> 584
Gly Phe Asn Pro Trp Ser Ser Val Pro Thr Thr Ser Thr Pro Gly Thr
                5                   10                  15

Ser Thr Val His Leu Ala Thr Ser Gly Thr Pro Ser Ser Leu Pro Gly
            20                  25                  30

His Thr Ala Pro Val Pro Leu Leu Ile Pro Phe Thr Leu Asn Phe Thr
        35                  40                  45

Ile Thr Asn Leu His Tyr Glu Glu Asn Met Gln His Pro Gly Ser Arg
    50                  55                  60

Lys Phe Asn Thr Thr Glu Arg Val Leu Gln Gly Leu Leu Lys Pro Leu
65                  70                  75                  80

Phe Lys Ser Thr Ser Val Gly Pro Leu Tyr Ser Gly Cys Arg Leu Thr
                85                  90                  95

Leu Leu Arg Pro Glu Lys His Gly Ala Ala Thr Gly Val Asp Ala Ile
            100                 105                 110

Cys Thr Leu Arg Leu Asp Pro Thr Gly Pro Gly Leu Asp Arg Glu Arg
        115                 120                 125

Leu Tyr Trp Glu Leu Ser Gln Leu Thr Asn Ser Val Thr Glu Leu Gly
    130                 135                 140

Pro Tyr Thr Leu Asp Arg Asp Ser Leu Tyr Val Asn
145                 150                 155


<210> 585
<211> 156
<212> PRT
<213> Homo sapiens

<400> 585
Gly Phe Thr His Arg Ser Ser Val Pro Thr Thr Ser Ile Pro Gly Thr
                5                   10                  15

Ser Ala Val His Leu Glu Thr Ser Gly Thr Pro Ala Ser Leu Pro Gly
            20                  25                  30

His Thr Ala Pro Gly Pro Leu Leu Val Pro Phe Thr Leu Asn Phe Thr

35                  40                  45

Ile Thr Asn Leu Gln Tyr Glu Glu Asp Met Arg His Pro Gly Ser Arg
    50                  55                  60

Lys Phe Asn Thr Thr Glu Arg Val Leu Gln Gly Leu Leu Lys Pro Leu
65                  70                  75                  80

Phe Lys Ser Thr Ser Val Gly Pro Leu Tyr Ser Gly Cys Arg Leu Thr
                85                  90                  95

Leu Leu Arg Pro Glu Lys Arg Gly Ala Ala Thr Gly Val Asp Thr Ile
            100                 105                 110

Cys Thr His Arg Leu Asp Pro Leu Asn Pro Gly Leu Asp Arg Glu Gln
        115                 120                 125

Leu Tyr Trp Glu Leu Ser Lys Leu Thr Cys Gly Ile Ile Glu Leu Gly
    130                 135                 140

Pro Tyr Leu Leu Asp Arg Gly Ser Leu Tyr Val Asn
145                 150                 155


<210> 586
<211> 156
<212> PRT
<213> Homo sapiens

<220>
<221> variant
<222> 151,156
<223> Xaa = Any amino acid

<400> 586
Gly Phe Thr His Arg Asn Phe Val Pro Ile Thr Ser Thr Pro Gly Thr
                5                   10                  15

Ser Thr Val His Leu Gly Thr Ser Glu Thr Pro Ser Ser Leu Pro Arg
            20                  25                  30

Pro Ile Val Pro Gly Pro Leu Leu Val Pro Phe Thr Leu Asn Phe Thr
        35                  40                  45

Ile Thr Asn Leu Gln Tyr Glu Glu Ala Met Arg His Pro Gly Ser Arg
    50                  55                  60

Lys Phe Asn Thr Thr Glu Arg Val Leu Gln Gly Leu Leu Arg Pro Leu
65                  70                  75                  80

Phe Lys Asn Thr Ser Ile Gly Pro Leu Tyr Ser Ser Cys Arg Leu Thr
                85                  90                  95

Leu Leu Arg Pro Glu Lys Asp Lys Ala Ala Thr Arg Val Asp Ala Ile
            100                 105                 110

Cys Thr His His Pro Asp Pro Gln Ser Pro Gly Leu Asn Arg Glu Gln

115                 120                 125

Leu Tyr Trp Glu Leu Ser Gln Leu Thr His Gly Ile Thr Glu Leu Gly
    130                 135                 140

Pro Tyr Thr Leu Asp Arg Xaa Ser Leu Tyr Val Xaa
145                 150                 155


<210> 587
<211> 156
<212> PRT
<213> Homo sapiens

<400> 587
Gly Phe Thr His Trp Ser Pro Ile Pro Thr Thr Ser Thr Pro Gly Thr
                5                   10                  15

Ser Ile Val Asn Leu Gly Thr Ser Gly Ile Pro Pro Ser Leu Pro Glu
            20                  25                  30

Thr Thr Ala Thr Gly Pro Leu Leu Val Pro Phe Thr Leu Asn Phe Thr
        35                  40                  45

Ile Thr Asn Leu Gln Tyr Glu Glu Asn Met Gly His Pro Gly Ser Arg
    50                  55                  60

Lys Phe Asn Ile Thr Glu Ser Val Leu Gln Gly Leu Leu Lys Pro Leu
65                  70                  75                  80

Phe Lys Ser Thr Ser Val Gly Pro Leu Tyr Ser Gly Cys Arg Leu Thr
                85                  90                  95

Leu Leu Arg Pro Glu Lys Asp Gly Val Ala Thr Arg Val Asp Ala Ile
            100                 105                 110

Cys Thr His Arg Pro Asp Pro Lys Ile Pro Gly Leu Asp Arg Gln Gln
        115                 120                 125

Leu Tyr Trp Glu Leu Ser Gln Leu Thr His Ser Ile Thr Glu Leu Gly
    130                 135                 140

Pro Tyr Thr Leu Asp Arg Asp Ser Leu Tyr Val Asn
145                 150                 155


<210> 588
<211> 156
<212> PRT
<213> Homo sapiens

<400> 588
Gly Phe Thr Gln Arg Ser Ser Val Pro Thr Thr Ser Thr Pro Gly Thr
                5                   10                  15

Phe Thr Val Gln Pro Glu Thr Ser Glu Thr Pro Ser Ser Leu Pro Gly
            20                  25                  30

Pro Thr Ala Thr Gly Pro Val Leu Leu Pro Phe Thr Leu Asn Phe Thr
            35                  40                  45

Ile Ile Asn Leu Gln Tyr Glu Glu Asp Met His Arg Pro Gly Ser Arg
    50                  55                  60

Lys Phe Asn Thr Thr Glu Arg Val Leu Gln Gly Leu Leu Met Pro Leu
65                  70                  75                  80

Phe Lys Asn Thr Ser Val Ser Ser Leu Tyr Ser Gly Cys Arg Leu Thr
                85                  90                  95

Leu Leu Arg Pro Glu Lys Asp Gly Ala Ala Thr Arg Val Asp Ala Val
            100                 105                 110

Cys Thr His Arg Pro Asp Pro Lys Ser Pro Gly Leu Asp Arg Glu Arg
        115                 120                 125

Leu Tyr Trp Lys Leu Ser Gln Leu Thr His Gly Ile Thr Glu Leu Gly
    130                 135                 140

Pro Tyr Thr Leu Asp Arg His Ser Leu Tyr Val Asn
145                 150                 155


<210> 589
<211> 156
<212> PRT
<213> Homo sapiens

<400> 589
Gly Phe Thr His Gln Ser Ser Met Thr Thr Thr Arg Thr Pro Asp Thr
                5                   10                  15

Ser Thr Met His Leu Ala Thr Ser Arg Thr Pro Ala Ser Leu Ser Gly
            20                  25                  30

Pro Thr Thr Ala Ser Pro Leu Leu Val Leu Phe Thr Ile Asn Phe Thr
        35                  40                  45

Ile Thr Asn Leu Arg Tyr Glu Glu Asn Met His His Pro Gly Ser Arg
    50                  55                  60

Lys Phe Asn Thr Thr Glu Arg Val Leu Gln Gly Leu Leu Arg Pro Val
65                  70                  75                  80

Phe Lys Asn Thr Ser Val Gly Pro Leu Tyr Ser Gly Cys Arg Leu Thr
                85                  90                  95

Leu Leu Arg Pro Lys Lys Asp Gly Ala Ala Thr Lys Val Asp Ala Ile
            100                 105                 110

Cys Thr Tyr Arg Pro Asp Pro Lys Ser Pro Gly Leu Asp Arg Glu Gln
        115                 120                 125

Leu Tyr Trp Glu Leu Ser Gln Leu Thr His Ser Ile Thr Glu Leu Gly

130                 135                 140

Pro Tyr Thr Leu Asp Arg Asp Ser Leu Tyr Val Asn
145                 150                 155


<210> 590
<211> 156
<212> PRT
<213> Homo sapiens

<220>
<221> variant
<222> 145
<223> Xaa = Any amino acid

<400> 590
Gly Phe Thr Gln Arg Ser Ser Val Pro Thr Thr Ser Ile Pro Gly Thr
                5                   10                  15

Pro Thr Val Asp Leu Gly Thr Ser Gly Thr Pro Val Ser Lys Pro Gly
            20                  25                  30

Pro Ser Ala Ala Ser Pro Leu Leu Val Leu Phe Thr Leu Asn Phe Thr
        35                  40                  45

Ile Thr Asn Leu Arg Tyr Glu Glu Asn Met Gln His Pro Gly Ser Arg
    50                  55                  60

Lys Phe Asn Thr Thr Glu Arg Val Leu Gln Gly Leu Leu Arg Ser Leu
65                  70                  75                  80

Phe Lys Ser Thr Ser Val Gly Pro Leu Tyr Ser Gly Cys Arg Leu Thr
                85                  90                  95

Leu Leu Arg Pro Glu Lys Asp Gly Thr Ala Thr Gly Val Asp Ala Ile
            100                 105                 110

Cys Thr His His Pro Asp Pro Lys Ser Pro Arg Leu Asp Arg Glu Gln
        115                 120                 125

Leu Tyr Trp Glu Leu Ser Gln Leu Thr His Asn Ile Thr Glu Leu Gly
    130                 135                 140

Xaa Tyr Ala Leu Asp Asn Asp Ser Leu Phe Val Asn
145                 150                 155


<210> 591
<211> 155
<212> PRT
<213> Homo sapiens

<400> 591
Gly Phe Thr His Arg Ser Ser Val Ser Thr Thr Ser Thr Pro Gly Thr
                5                   10                  15

Pro Thr Val Tyr Leu Gly Ala Ser Lys Thr Pro Ala Ser Ile Phe Gly
            20                  25                  30

Pro Ser Ala Ala Ser His Leu Leu Ile Leu Phe Thr Leu Asn Phe Thr
        35                  40                  45

Ile Thr Asn Leu Arg Tyr Glu Glu Asn Met Trp Pro Gly Ser Arg Lys
    50                  55                  60

Phe Asn Thr Thr Glu Arg Val Leu Gln Gly Leu Leu Arg Pro Leu Phe
65                  70                  75                  80

Lys Asn Thr Ser Val Gly Pro Leu Tyr Ser Gly Cys Arg Leu Thr Leu
                85                  90                  95

Leu Arg Pro Glu Lys Asp Gly Glu Ala Thr Gly Val Asp Ala Ile Cys
            100                 105                 110

Thr His Arg Pro Asp Pro Thr Gly Pro Gly Leu Asp Arg Glu Gln Leu
        115                 120                 125

Tyr Leu Glu Leu Ser Gln Leu Thr His Ser Ile Thr Glu Leu Gly Pro
    130                 135                 140

Tyr Thr Leu Asp Arg Asp Ser Leu Tyr Val Asn
145                 150                 155


<210> 592
<211> 134
<212> PRT
<213> Homo sapiens

<400> 592
Gly Phe Thr His Arg Ser Ser Val Pro Thr Thr Ser Thr Gly Val Val
                5                   10                  15

Ser Glu Glu Pro Phe Thr Leu Asn Phe Thr Ile Asn Asn Leu Arg Tyr
            20                  25                  30

Met Ala Asp Met Gly Gln Pro Gly Ser Leu Lys Phe Asn Ile Thr Asp
        35                  40                  45

Asn Val Met Lys His Leu Leu Ser Pro Leu Phe Gln Arg Ser Ser Leu
    50                  55                  60

Gly Ala Arg Tyr Thr Gly Cys Arg Val Ile Ala Leu Arg Ser Val Lys
65                  70                  75                  80

Asn Gly Ala Glu Thr Arg Val Asp Leu Leu Cys Thr Tyr Leu Gln Pro
                85                  90                  95

Leu Ser Gly Pro Gly Leu Pro Ile Lys Gln Val Phe His Glu Leu Ser
            100                 105                 110

Gln Gln Thr His Gly Ile Thr Arg Leu Gly Pro Tyr Ser Leu Asp Lys
        115                 120                 125

Asp Ser Leu Tyr Leu Asn
    130

<210> 593
<211> 150
<212> PRT
<213> Homo sapiens

<220>
<221> variant
<222> 7
<223> Xaa = Any amino acid

<400> 593
Gly Tyr Asn Glu Pro Gly Xaa Asp Glu Pro Pro Thr Thr Pro Lys Pro
                5                   10                  15

Ala Thr Thr Phe Leu Pro Pro Leu Ser Glu Ala Thr Thr Ala Met Gly
            20                  25                  30

Tyr His Leu Lys Thr Leu Thr Leu Asn Phe Thr Ile Ser Asn Leu Gln
        35                  40                  45

Tyr Ser Pro Asp Met Gly Lys Gly Ser Ala Thr Phe Asn Ser Thr Glu
    50                  55                  60

Gly Val Leu Gln His Leu Leu Arg Pro Leu Phe Gln Lys Ser Ser Met
65                  70                  75                  80

Gly Pro Phe Tyr Leu Gly Cys Gln Leu Ile Ser Leu Arg Pro Glu Lys
                85                  90                  95

Asp Gly Ala Ala Thr Gly Val Asp Thr Thr Cys Thr Tyr His Pro Asp
            100                 105                 110

Pro Val Gly Pro Gly Leu Asp Ile Gln Gln Leu Tyr Trp Glu Leu Ser
        115                 120                 125

Gln Leu Thr His Gly Val Thr Gln Leu Gly Phe Tyr Val Leu Asp Arg
    130                 135                 140

Asp Ser Leu Phe Ile Asn
145                 150

<210> 594
<211> 318
<212> PRT
<213> Homo sapiens

<220>
<221> variant
<222> 136,248,268
<223> Xaa = Any amino acid

<400> 594
Gly Tyr Ala Pro Gln Asn Leu Ser Ile Arg Gly Glu Tyr Gln Ile Asn
                5                   10                  15

Phe His Ile Val Asn Trp Asn Leu Ser Asn Pro Asp Pro Thr Ser Ser
            20                  25                  30

Glu Tyr Ile Thr Leu Leu Arg Asp Ile Gln Asp Lys Val Thr Thr Leu
        35                  40                  45

Tyr Lys Gly Ser Gln Leu His Asp Thr Phe Arg Phe Cys Leu Val Thr
    50                  55                  60

Asn Leu Thr Met Asp Ser Val Leu Val Thr Val Lys Ala Leu Phe Ser
65                  70                  75                  80

Ser Asn Leu Asp Pro Ser Leu Val Glu Gln Val Phe Leu Asp Lys Thr
                85                  90                  95

Leu Asn Ala Ser Phe His Trp Leu Gly Ser Thr Tyr Gln Leu Val Asp
            100                 105                 110

Ile His Val Thr Glu Met Glu Ser Ser Val Tyr Gln Pro Thr Ser Ser
        115                 120                 125

Ser Ser Thr Gln His Phe Tyr Xaa Asn Phe Thr Ile Thr Asn Leu Pro
    130                 135                 140

Tyr Ser Gln Asp Lys Ala Gln Pro Gly Thr Thr Asn Tyr Gln Arg Asn
145                 150                 155                 160

Lys Arg Asn Ile Glu Asp Ala Leu Asn Gln Leu Phe Arg Asn Ser Ser
                165                 170                 175

Ile Lys Ser Tyr Phe Ser Asp Cys Gln Val Ser Thr Phe Arg Ser Val
            180                 185                 190

Pro Asn Arg His His Thr Gly Val Asp Ser Leu Cys Asn Phe Ser Pro
        195                 200                 205

Leu Ala Arg Arg Val Asp Arg Val Ala Ile Tyr Glu Glu Phe Leu Arg
    210                 215                 220

Met Thr Arg Asn Gly Thr Gln Leu Gln Asn Phe Thr Leu Asp Arg Ser
225                 230                 235                 240

Ser Val Leu Val Asp Gly Tyr Xaa Pro Asn Arg Asn Glu Pro Leu Thr
                245                 250                 255

Gly Asn Ser Asp Leu Pro Phe Trp Ala Val Ile Xaa Ile Gly Leu Ala
            260                 265                 270

Gly Leu Leu Gly Leu Ile Thr Cys Leu Ile Cys Gly Val Leu Val Thr
        275                 280                 285

Thr Arg Arg Arg Lys Lys Glu Gly Glu Tyr Asn Val Gln Gln Gln Cys
    290                 295                 300

Pro Gly Tyr Tyr Gln Ser His Leu Asp Leu Glu Asp Leu Gln
305                 310                 315

<210> 595
<211> 3451
<212> PRT
<213> Homo sapiens

<220>
<221> VARIANT
<222> 177, 335, 523, 618, 663, 875, 961, 1001, 1441, 1555, 1560,
1563, 1574, 1585, 2065, 2070, 2683, 2990, 3269, 3381, 3401
<223> Xaa = Any Amino Acid

<400> 595
Ile Arg Asn Ser Ser Leu Glu Tyr Leu Tyr Ser Gly Cys Arg Leu Ala
 1               5                  10                  15
Ser Leu Arg Pro Glu Lys Asp Ser Ser Ala Thr Ala Val Asp Ala Ile
            20                  25                  30
Cys Thr His Arg Pro Asp Pro Glu Asp Leu Gly Leu Asp Arg Glu Arg
        35                  40                  45
Leu Tyr Trp Glu Leu Ser Asn Leu Thr Asn Gly Ile Gln Glu Leu Gly
    50                  55                  60
Pro Tyr Thr Leu Asp Arg Asn Ser Leu Tyr Val Asn Gly Phe Thr His
65                  70                  75                  80
Arg Ser Ser Met Pro Thr Thr Ser Thr Pro Gly Thr Ser Thr Val Asp
                85                  90                  95
Val Gly Thr Ser Gly Thr Pro Ser Ser Ser Pro Ser Pro Thr Thr Ala
            100                 105                 110
Gly Pro Leu Leu Met Pro Phe Thr Leu Asn Phe Thr Ile Thr Asn Leu
        115                 120                 125
Gln Tyr Glu Glu Asp Met Arg Arg Thr Gly Ser Arg Lys Phe Asn Thr
    130                 135                 140
Met Glu Ser Val Leu Gln Gly Leu Leu Lys Pro Leu Phe Lys Asn Thr
145                 150                 155                 160
Ser Val Gly Pro Leu Tyr Ser Gly Cys Arg Leu Thr Leu Leu Arg Pro
                165                 170                 175
Xaa Lys Asp Gly Ala Ala Thr Gly Val Asp Ala Ile Cys Thr His Arg
            180                 185                 190
Leu Asp Pro Lys Ser Pro Gly Leu Asn Arg Glu Gln Leu Tyr Trp Glu
        195                 200                 205
Leu Ser Lys Leu Thr Asn Asp Ile Glu Glu Leu Gly Pro Tyr Thr Leu
    210                 215                 220
Asp Arg Asn Ser Leu Tyr Val Asn Gly Phe Thr His Gln Ser Ser Val
225                 230                 235                 240
Ser Thr Thr Ser Thr Pro Gly Thr Ser Thr Val Asp Leu Arg Thr Ser
                245                 250                 255
Val Thr Pro Ser Ser Leu Ser Ser Pro Thr Ile Met Ala Ala Gly Pro
            260                 265                 270
Leu Leu Val Pro Phe Thr Leu Asn Phe Thr Ile Thr Asn Leu Gln Tyr
        275                 280                 285
Gly Glu Asp Met Gly His Pro Gly Ser Arg Lys Phe Asn Thr Thr Glu
    290                 295                 300
Arg Val Leu Gln Gly Leu Leu Gly Pro Ile Phe Lys Asn Thr Ser Val
305                 310                 315                 320

Gly Pro Leu Tyr Ser Gly Cys Arg Leu Thr Ser Leu Arg Ser Xaa Lys
                325                 330                 335
Asp Gly Ala Ala Thr Gly Val Asp Ala Ile Cys Ile His His Leu Asp
                340                 345                 350
Pro Lys Ser Pro Gly Leu Asn Arg Glu Arg Leu Tyr Trp Glu Leu Ser
                355                 360                 365
Gln Leu Thr Asn Gly Ile Lys Glu Leu Gly Pro Tyr Thr Leu Asp Arg
    370                 375                 380
Asn Ser Leu Tyr Val Asn Ala Ala Gly Pro Leu Leu Val Leu Phe Thr
385                 390                 395                 400
Leu Asn Phe Thr Ile Thr Asn Leu Lys Tyr Glu Glu Asp Met His Arg
                405                 410                 415
Pro Gly Ser Arg Lys Phe Asn Thr Thr Glu Arg Val Leu Gln Thr Leu
                420                 425                 430
Arg Gly Pro Met Phe Lys Asn Thr Ser Gly Gly Leu Leu Tyr Ser Gly
                435                 440                 445
Cys Arg Leu Thr Leu Leu Arg Ser Glu Lys Asp Gly Ala Ala Thr Gly
    450                 455                 460
Val Asp Ala Ile Cys Thr His Arg Leu Asp Pro Lys Ser Pro Gly Val
465                 470                 475                 480
Asp Arg Glu Gln Leu Tyr Trp Glu Leu Ser Gln Leu Thr Asn Gly Ile
                485                 490                 495
Lys Glu Leu Gly Pro Tyr Thr Leu Asp Arg Asn Ser Leu Tyr Val Asn
                500                 505                 510
Gly Phe Thr His Arg Thr Ser Val Pro Thr Xaa Ser Thr Pro Gly Thr
                515                 520                 525
Ser Thr Val Asp Leu Gly Thr Ser Gly Thr Pro Phe Ser Leu Pro Ser
    530                 535                 540
Pro Ala Thr Ala Gly Pro Leu Leu Val Leu Phe Thr Leu Asn Phe Thr
545                 550                 555                 560
Ile Thr Asn Leu Lys Tyr Glu Glu Asp Met His Arg Pro Gly Ser Arg
                565                 570                 575
Lys Phe Asn Thr Thr Glu Arg Val Leu Gln Thr Leu Leu Gly Pro Met
                580                 585                 590
Phe Lys Asn Thr Ser Val Gly Leu Leu Tyr Ser Gly Cys Arg Leu Thr
                595                 600                 605
Leu Leu Arg Ser Glu Lys Asp Gly Ala Xaa Thr Gly Val Asp Ala Ile
    610                 615                 620
Cys Thr His Arg Leu Asp Pro Lys Ser Pro Gly Val Asp Arg Glu Gln
625                 630                 635                 640
Leu Tyr Trp Glu Leu Ser Gln Leu Thr Asn Gly Ile Lys Glu Leu Gly
                645                 650                 655
Pro Tyr Thr Leu Asp Arg Xaa Ser Leu Tyr Val Asn Gly Phe Thr His
                660                 665                 670
Trp Ile Pro Val Pro Thr Ser Ser Thr Pro Gly Thr Ser Thr Val Asp
                675                 680                 685
Leu Gly Ser Gly Thr Pro Ser Ser Leu Pro Ser Pro Thr Thr Ala Gly
    690                 695                 700
Pro Leu Leu Val Pro Phe Thr Leu Asn Phe Thr Ile Thr Asn Leu Gln
705                 710                 715                 720
Tyr Glu Glu Asp Met His His Pro Gly Ser Arg Lys Phe Asn Thr Thr
                725                 730                 735
Glu Arg Val Leu Gln Gly Leu Leu Gly Pro Met Phe Lys Asn Thr Ser
                740                 745                 750
Val Gly Leu Leu Tyr Ser Gly Cys Arg Leu Thr Leu Leu Arg Pro Glu
                755                 760                 765
Lys Asn Gly Ala Ala Thr Gly Met Asp Ala Ile Cys Ser His Arg Leu

770                 775                 780
Asp Pro Lys Ser Pro Gly Leu Asn Arg Glu Gln Leu Tyr Trp Glu Leu
785                 790                 795                 800
Ser Gln Leu Thr His Gly Ile Lys Glu Leu Gly Pro Tyr Thr Leu Asp
                805                 810                 815
Arg His Ser Leu Tyr Val Asn Gly Phe Thr His Trp Ile Pro Val Pro
            820                 825                 830
Thr Ser Ser Thr Pro Gly Thr Ser Thr Val Asp Leu Gly Ser Gly Thr
        835                 840                 845
Pro Ser Ser Leu Pro Ser Pro Thr Thr Ala Gly Pro Leu Leu Val Pro
    850                 855                 860
Phe Thr Leu Asn Phe Thr Ile Thr Asn Leu Xaa Tyr Glu Glu Asp Met
865                 870                 875                 880
His Cys Pro Gly Ser Arg Lys Phe Asn Thr Thr Glu Arg Val Leu Gln
                885                 890                 895
Ser Leu Leu Gly Pro Met Phe Lys Asn Thr Ser Val Gly Pro Leu Tyr
            900                 905                 910
Ser Gly Cys Arg Leu Thr Leu Leu Arg Ser Glu Lys Asp Gly Ala Ala
        915                 920                 925
Thr Gly Val Asp Ala Ile Cys Thr His Arg Leu Asp Pro Lys Ser Pro
    930                 935                 940
Gly Val Asp Arg Glu Gln Leu Tyr Trp Glu Leu Ser Gln Leu Thr Asn
945                 950                 955                 960
Xaa Ile Lys Glu Leu Gly Pro Tyr Thr Leu Asp Ser Asn Ser Leu Tyr
                965                 970                 975
Val Asn Gly Phe Thr His Gln Thr Ser Ala Pro Asn Thr Ser Thr Pro
            980                 985                 990
Gly Thr Ser Thr Val Asp Leu Gly Xaa Ser Gly Thr Pro Ser Ser Leu
        995                 1000                1005
Pro Ser Pro Thr Ser Ala Gly Pro Leu Leu Val Pro Phe Thr Leu Asn
    1010                1015                1020
Phe Thr Ile Thr Asn Leu Gln Tyr Glu Glu Asp Met His His Pro Gly
1025                1030                1035                1040
Ser Arg Lys Phe Asn Thr Thr Glu Arg Val Leu Gln Gly Leu Leu Gly
                1045                1050                1055
Pro Met Phe Lys Asn Thr Ser Val Gly Leu Leu Tyr Ser Gly Cys Arg
            1060                1065                1070
Leu Thr Leu Leu Arg Pro Glu Lys Asn Gly Ala Ala Thr Gly Met Asp
        1075                1080                1085
Ala Ile Cys Ser His Arg Leu Asp Pro Lys Ser Pro Gly Leu Asn Arg
    1090                1095                1100
Glu Gln Leu Tyr Trp Glu Leu Ser Gln Leu Thr His Gly Ile Lys Glu
1105                1110                1115                1120
Leu Gly Pro Tyr Thr Leu Asp Arg Asn Ser Leu Tyr Val Asn Gly Phe
                1125                1130                1135
Thr His Arg Ser Ser Val Ala Pro Thr Ser Thr Pro Gly Thr Ser Thr
            1140                1145                1150
Val Asp Leu Gly Thr Ser Gly Thr Pro Ser Ser Leu Pro Ser Pro Thr
        1155                1160                1165
Thr Ala Val Pro Leu Leu Val Pro Phe Thr Leu Asn Phe Thr Ile Thr
    1170                1175                1180
Asn Leu Gln Tyr Gly Glu Asp Met Arg His Pro Gly Ser Arg Lys Phe
1185                1190                1195                1200
Asn Thr Thr Glu Arg Val Leu Gln Gly Leu Leu Gly Pro Leu Phe Lys
                1205                1210                1215
Asn Ser Ser Val Gly Pro Leu Tyr Ser Gly Cys Arg Leu Ile Ser Leu
            1220                1225                1230

Arg Ser Glu Lys Asp Gly Ala Ala Thr Gly Val Asp Ala Ile Cys Thr
        1235                1240                1245
His His Leu Asn Pro Gln Ser Pro Gly Leu Asp Arg Glu Gln Leu Tyr
    1250                1255                1260
Trp Gln Leu Ser Gln Met Thr Asn Gly Ile Lys Glu Leu Gly Pro Tyr
1265                1270                1275                1280
Thr Leu Asp Arg Asn Ser Leu Tyr Val Asn Gly Phe Thr His Arg Ser
                1285                1290                1295
Ser Gly Leu Thr Thr Ser Thr Pro Trp Thr Ser Thr Val Asp Leu Gly
            1300                1305                1310
Thr Ser Gly Thr Pro Ser Pro Val Pro Ser Pro Thr Thr Ala Gly Pro
        1315                1320                1325
Leu Leu Val Pro Phe Thr Leu Asn Phe Thr Ile Thr Asn Leu Gln Tyr
    1330                1335                1340
Glu Glu Asp Met His Arg Pro Gly Ser Arg Lys Phe Asn Ala Thr Glu
1345                1350                1355                1360
Arg Val Leu Gln Gly Leu Leu Ser Pro Ile Phe Lys Asn Ser Ser Val
                1365                1370                1375
Gly Pro Leu Tyr Ser Gly Cys Arg Leu Thr Ser Leu Arg Pro Glu Lys
            1380                1385                1390
Asp Gly Ala Ala Thr Gly Met Asp Ala Val Cys Leu Tyr His Pro Asn
        1395                1400                1405
Pro Lys Arg Pro Gly Leu Asp Arg Glu Gln Leu Tyr Trp Glu Leu Ser
    1410                1415                1420
Gln Leu Thr His Asn Ile Thr Glu Leu Gly Pro Tyr Ser Leu Asp Arg
1425                1430                1435                1440
Xaa Ser Leu Tyr Val Asn Gly Phe Thr His Gln Asn Ser Val Pro Thr
                1445                1450                1455
Thr Ser Thr Pro Gly Thr Ser Thr Val Tyr Trp Ala Thr Thr Gly Thr
            1460                1465                1470
Pro Ser Ser Phe Pro Gly His Thr Glu Pro Gly Pro Leu Leu Ile Pro
        1475                1480                1485
Phe Thr Phe Asn Phe Thr Ile Thr Asn Leu His Tyr Glu Glu Asn Met
    1490                1495                1500
Gln His Pro Gly Ser Arg Lys Phe Asn Thr Thr Glu Arg Val Leu Gln
1505                1510                1515                1520
Gly Leu Leu Thr Pro Leu Phe Lys Asn Thr Ser Val Gly Pro Leu Tyr
                1525                1530                1535
Ser Gly Cys Arg Leu Thr Leu Leu Arg Pro Glu Lys Gln Glu Ala Ala
            1540                1545                1550
Thr Gly Xaa Asp Thr Ile Cys Xaa His Arg Xaa Asp Pro Ile Gly Pro
        1555                1560                1565
Gly Leu Asp Arg Glu Xaa Leu Tyr Trp Glu Leu Ser Gln Leu Thr His
    1570                1575                1580
Xaa Ile Thr Glu Leu Gly Pro Tyr Thr Leu Asp Arg Asp Ser Leu Tyr
1585                1590                1595                1600
Val Asn Gly Phe Asn Pro Trp Ser Ser Val Pro Thr Thr Ser Thr Pro
                1605                1610                1615
Gly Thr Ser Thr Val His Leu Ala Thr Ser Gly Thr Pro Ser Ser Leu
            1620                1625                1630
Pro Gly His Thr Ala Pro Val Pro Leu Leu Ile Pro Phe Thr Leu Asn
        1635                1640                1645
Phe Thr Ile Thr Asn Leu His Tyr Glu Glu Asn Met Gln His Pro Gly
    1650                1655                1660
Ser Arg Lys Phe Asn Thr Thr Glu Arg Val Leu Gln Gly Leu Leu Lys
1665                1670                1675                1680
Pro Leu Phe Lys Ser Thr Ser Val Gly Pro Leu Tyr Ser Gly Cys Arg

1685                1690                1695
Leu Thr Leu Leu Arg Pro Glu Lys His Gly Ala Ala Thr Gly Val Asp
        1700                1705                1710
Ala Ile Cys Thr Leu Arg Leu Asp Pro Thr Gly Pro Gly Leu Asp Arg
    1715                1720                1725
Glu Arg Leu Tyr Trp Glu Leu Ser Gln Leu Thr Asn Ser Val Thr Glu
    1730                1735                1740
Leu Gly Pro Tyr Thr Leu Asp Arg Asp Ser Leu Tyr Val Asn Gly Phe
1745                1750                1755                1760
Thr His Arg Ser Ser Val Pro Thr Thr Ser Ile Pro Gly Thr Ser Ala
                1765                1770                1775
Val His Leu Glu Thr Ser Gly Thr Pro Ala Ser Leu Pro Gly His Thr
        1780                1785                1790
Ala Pro Gly Pro Leu Leu Val Pro Phe Thr Leu Asn Phe Thr Ile Thr
    1795                1800                1805
Asn Leu Gln Tyr Glu Glu Asp Met Arg His Pro Gly Ser Arg Lys Phe
    1810                1815                1820
Asn Thr Thr Glu Arg Val Leu Gln Gly Leu Leu Lys Pro Leu Phe Lys
1825                1830                1835                1840
Ser Thr Ser Val Gly Pro Leu Tyr Ser Gly Cys Arg Leu Thr Leu Leu
                1845                1850                1855
Arg Pro Glu Lys Arg Gly Ala Ala Thr Gly Val Asp Thr Ile Cys Thr
        1860                1865                1870
His Arg Leu Asp Pro Leu Asn Pro Gly Leu Asp Arg Glu Gln Leu Tyr
    1875                1880                1885
Trp Glu Leu Ser Lys Leu Thr Cys Gly Ile Ile Glu Leu Gly Pro Tyr
    1890                1895                1900
Leu Leu Asp Arg Gly Ser Leu Tyr Val Asn Gly Phe Thr His Arg Asn
1905                1910                1915                1920
Phe Val Pro Ile Thr Ser Thr Pro Gly Thr Ser Thr Val His Leu Gly
                1925                1930                1935
Thr Ser Glu Thr Pro Ser Ser Leu Pro Arg Pro Ile Val Pro Gly Pro
        1940                1945                1950
Leu Leu Val Pro Phe Thr Leu Asn Phe Thr Ile Thr Asn Leu Gln Tyr
    1955                1960                1965
Glu Glu Ala Met Arg His Pro Gly Ser Arg Lys Phe Asn Thr Thr Glu
    1970                1975                1980
Arg Val Leu Gln Gly Leu Leu Arg Pro Leu Phe Lys Asn Thr Ser Ile
1985                1990                1995                2000
Gly Pro Leu Tyr Ser Ser Cys Arg Leu Thr Leu Leu Arg Pro Glu Lys
                2005                2010                2015
Asp Lys Ala Ala Thr Arg Val Asp Ala Ile Cys Thr His His Pro Asp
        2020                2025                2030
Pro Gln Ser Pro Gly Leu Asn Arg Glu Gln Leu Tyr Trp Glu Leu Ser
    2035                2040                2045
Gln Leu Thr His Gly Ile Thr Glu Leu Gly Pro Tyr Thr Leu Asp Arg
    2050                2055                2060
Xaa Ser Leu Tyr Val Xaa Gly Phe Thr His Trp Ser Pro Ile Pro Thr
2065                2070                2075                2080
Thr Ser Thr Pro Gly Thr Ser Ile Val Asn Leu Gly Thr Ser Gly Ile
                2085                2090                2095
Pro Pro Ser Leu Pro Glu Thr Thr Ala Thr Gly Pro Leu Leu Val Pro
        2100                2105                2110
Phe Thr Leu Asn Phe Thr Ile Thr Asn Leu Gln Tyr Glu Glu Asn Met
    2115                2120                2125
Gly His Pro Gly Ser Arg Lys Phe Asn Ile Thr Glu Ser Val Leu Gln
    2130                2135                2140

Gly Leu Leu Lys Pro Leu Phe Lys Ser Thr Ser Val Gly Pro Leu Tyr
2145                2150                2155                2160
Ser Gly Cys Arg Leu Thr Leu Leu Arg Pro Glu Lys Asp Gly Val Ala
                2165                2170                2175
Thr Arg Val Asp Ala Ile Cys Thr His Arg Pro Asp Pro Lys Ile Pro
            2180                2185                2190
Gly Leu Asp Arg Gln Gln Leu Tyr Trp Glu Leu Ser Gln Leu Thr His
        2195                2200                2205
Ser Ile Thr Glu Leu Gly Pro Tyr Thr Leu Asp Arg Asp Ser Leu Tyr
    2210                2215                2220
Val Asn Gly Phe Thr Gln Arg Ser Ser Val Pro Thr Thr Ser Thr Pro
2225                2230                2235                2240
Gly Thr Phe Thr Val Gln Pro Glu Thr Ser Glu Thr Pro Ser Ser Leu
                2245                2250                2255
Pro Gly Pro Thr Ala Thr Gly Pro Val Leu Leu Pro Phe Thr Leu Asn
            2260                2265                2270
Phe Thr Ile Ile Asn Leu Gln Tyr Glu Glu Asp Met His Arg Pro Gly
        2275                2280                2285
Ser Arg Lys Phe Asn Thr Thr Glu Arg Val Leu Gln Gly Leu Leu Met
    2290                2295                2300
Pro Leu Phe Lys Asn Thr Ser Val Ser Ser Leu Tyr Ser Gly Cys Arg
2305                2310                2315                2320
Leu Thr Leu Leu Arg Pro Glu Lys Asp Gly Ala Ala Thr Arg Val Asp
                2325                2330                2335
Ala Val Cys Thr His Arg Pro Asp Pro Lys Ser Pro Gly Leu Asp Arg
            2340                2345                2350
Glu Arg Leu Tyr Trp Lys Leu Ser Gln Leu Thr His Gly Ile Thr Glu
        2355                2360                2365
Leu Gly Pro Tyr Thr Leu Asp Arg His Ser Leu Tyr Val Asn Gly Phe
    2370                2375                2380
Thr His Gln Ser Ser Met Thr Thr Thr Arg Thr Pro Asp Thr Ser Thr
2385                2390                2395                2400
Met His Leu Ala Thr Ser Arg Thr Pro Ala Ser Leu Ser Gly Pro Thr
                2405                2410                2415
Thr Ala Ser Pro Leu Leu Val Leu Phe Thr Ile Asn Phe Thr Ile Thr
            2420                2425                2430
Asn Leu Arg Tyr Glu Glu Asn Met His His Pro Gly Ser Arg Lys Phe
        2435                2440                2445
Asn Thr Thr Glu Arg Val Leu Gln Gly Leu Leu Arg Pro Val Phe Lys
    2450                2455                2460
Asn Thr Ser Val Gly Pro Leu Tyr Ser Gly Cys Arg Leu Thr Leu Leu
2465                2470                2475                2480
Arg Pro Lys Lys Asp Gly Ala Ala Thr Lys Val Asp Ala Ile Cys Thr
                2485                2490                2495
Tyr Arg Pro Asp Pro Lys Ser Pro Gly Leu Asp Arg Glu Gln Leu Tyr
            2500                2505                2510
Trp Glu Leu Ser Gln Leu Thr His Ser Ile Thr Glu Leu Gly Pro Tyr
        2515                2520                2525
Thr Leu Asp Arg Asp Ser Leu Tyr Val Asn Gly Phe Thr Gln Arg Ser
    2530                2535                2540
Ser Val Pro Thr Thr Ser Ile Pro Gly Thr Pro Thr Val Asp Leu Gly
2545                2550                2555                2560
Thr Ser Gly Thr Pro Val Ser Lys Pro Gly Pro Ser Ala Ala Ser Pro
                2565                2570                2575
Leu Leu Val Leu Phe Thr Leu Asn Phe Thr Ile Thr Asn Leu Arg Tyr
            2580                2585                2590
Glu Glu Asn Met Gln His Pro Gly Ser Arg Lys Phe Asn Thr Thr Glu

        2595                2600                2605
Arg Val Leu Gln Gly Leu Leu Arg Ser Leu Phe Lys Ser Thr Ser Val
    2610                2615                2620
Gly Pro Leu Tyr Ser Gly Cys Arg Leu Thr Leu Leu Arg Pro Glu Lys
2625                2630                2635                2640
Asp Gly Thr Ala Thr Gly Val Asp Ala Ile Cys Thr His His Pro Asp
                2645                2650                2655
Pro Lys Ser Pro Arg Leu Asp Arg Glu Gln Leu Tyr Trp Glu Leu Ser
            2660                2665                2670
Gln Leu Thr His Asn Ile Thr Glu Leu Gly Xaa Tyr Ala Leu Asp Asn
        2675                2680                2685
Asp Ser Leu Phe Val Asn Gly Phe Thr His Arg Ser Ser Val Ser Thr
    2690                2695                2700
Thr Ser Thr Pro Gly Thr Pro Thr Val Tyr Leu Gly Ala Ser Lys Thr
2705                2710                2715                2720
Pro Ala Ser Ile Phe Gly Pro Ser Ala Ala Ser His Leu Leu Ile Leu
                2725                2730                2735
Phe Thr Leu Asn Phe Thr Ile Thr Asn Leu Arg Tyr Glu Glu Asn Met
            2740                2745                2750
Trp Pro Gly Ser Arg Lys Phe Asn Thr Thr Glu Arg Val Leu Gln Gly
        2755                2760                2765
Leu Leu Arg Pro Leu Phe Lys Asn Thr Ser Val Gly Pro Leu Tyr Ser
    2770                2775                2780
Gly Cys Arg Leu Thr Leu Leu Arg Pro Glu Lys Asp Gly Glu Ala Thr
2785                2790                2795                2800
Gly Val Asp Ala Ile Cys Thr His Arg Pro Asp Pro Thr Gly Pro Gly
                2805                2810                2815
Leu Asp Arg Glu Gln Leu Tyr Leu Glu Leu Ser Gln Leu Thr His Ser
            2820                2825                2830
Ile Thr Glu Leu Gly Pro Tyr Thr Leu Asp Arg Asp Ser Leu Tyr Val
        2835                2840                2845
Asn Gly Phe Thr His Arg Ser Ser Val Pro Thr Thr Ser Thr Gly Val
    2850                2855                2860
Val Ser Glu Glu Pro Phe Thr Leu Asn Phe Thr Ile Asn Asn Leu Arg
2865                2870                2875                2880
Tyr Met Ala Asp Met Gly Gln Pro Gly Ser Leu Lys Phe Asn Ile Thr
                2885                2890                2895
Asp Asn Val Met Lys His Leu Leu Ser Pro Leu Phe Gln Arg Ser Ser
            2900                2905                2910
Leu Gly Ala Arg Tyr Thr Gly Cys Arg Val Ile Ala Leu Arg Ser Val
        2915                2920                2925
Lys Asn Gly Ala Glu Thr Arg Val Asp Leu Leu Cys Thr Tyr Leu Gln
    2930                2935                2940
Pro Leu Ser Gly Pro Gly Leu Pro Ile Lys Gln Val Phe His Glu Leu
2945                2950                2955                2960
Ser Gln Gln Thr His Gly Ile Thr Arg Leu Gly Pro Tyr Ser Leu Asp
                2965                2970                2975
Lys Asp Ser Leu Tyr Leu Asn Gly Tyr Asn Glu Pro Gly Xaa Asp Glu
            2980                2985                2990
Pro Pro Thr Thr Pro Lys Pro Ala Thr Thr Phe Leu Pro Pro Leu Ser
        2995                3000                3005
Glu Ala Thr Thr Ala Met Gly Tyr His Leu Lys Thr Leu Thr Leu Asn
    3010                3015                3020
Phe Thr Ile Ser Asn Leu Gln Tyr Ser Pro Asp Met Gly Lys Gly Ser
3025                3030                3035                3040
Ala Thr Phe Asn Ser Thr Glu Gly Val Leu Gln His Leu Leu Arg Pro
                3045                3050                3055

Leu Phe Gln Lys Ser Ser Met Gly Pro Phe Tyr Leu Gly Cys Gln Leu
            3060                3065                3070
Ile Ser Leu Arg Pro Glu Lys Asp Gly Ala Ala Thr Gly Val Asp Thr
        3075                3080                3085
Thr Cys Thr Tyr His Pro Asp Pro Val Gly Pro Gly Leu Asp Ile Gln
    3090                3095                3100
Gln Leu Tyr Trp Glu Leu Ser Gln Leu Thr His Gly Val Thr Gln Leu
3105                3110                3115                3120
Gly Phe Tyr Val Leu Asp Arg Asp Ser Leu Phe Ile Asn Gly Tyr Ala
                3125                3130                3135
Pro Gln Asn Leu Ser Ile Arg Gly Glu Tyr Gln Ile Asn Phe His Ile
            3140                3145                3150
Val Asn Trp Asn Leu Ser Asn Pro Asp Pro Thr Ser Ser Glu Tyr Ile
        3155                3160                3165
Thr Leu Leu Arg Asp Ile Gln Asp Lys Val Thr Thr Leu Tyr Lys Gly
    3170                3175                3180
Ser Gln Leu His Asp Thr Phe Arg Phe Cys Leu Val Thr Asn Leu Thr
3185                3190                3195                3200
Met Asp Ser Val Leu Val Thr Val Lys Ala Leu Phe Ser Ser Asn Leu
                3205                3210                3215
Asp Pro Ser Leu Val Glu Gln Val Phe Leu Asp Lys Thr Leu Asn Ala
            3220                3225                3230
Ser Phe His Trp Leu Gly Ser Thr Tyr Gln Leu Val Asp Ile His Val
        3235                3240                3245
Thr Glu Met Glu Ser Ser Val Tyr Gln Pro Thr Ser Ser Ser Ser Thr
    3250                3255                3260
Gln His Phe Tyr Xaa Asn Phe Thr Ile Thr Asn Leu Pro Tyr Ser Gln
3265                3270                3275                3280
Asp Lys Ala Gln Pro Gly Thr Thr Asn Tyr Gln Arg Asn Lys Arg Asn
                3285                3290                3295
Ile Glu Asp Ala Leu Asn Gln Leu Phe Arg Asn Ser Ser Ile Lys Ser
            3300                3305                3310
Tyr Phe Ser Asp Cys Gln Val Ser Thr Phe Arg Ser Val Pro Asn Arg
        3315                3320                3325
His His Thr Gly Val Asp Ser Leu Cys Asn Phe Ser Pro Leu Ala Arg
    3330                3335                3340
Arg Val Asp Arg Val Ala Ile Tyr Glu Glu Phe Leu Arg Met Thr Arg
3345                3350                3355                3360
Asn Gly Thr Gln Leu Gln Asn Phe Thr Leu Asp Arg Ser Ser Val Leu
                3365                3370                3375
Val Asp Gly Tyr Xaa Pro Asn Arg Asn Glu Pro Leu Thr Gly Asn Ser
            3380                3385                3390
Asp Leu Pro Phe Trp Ala Val Ile Xaa Ile Gly Leu Ala Gly Leu Leu
        3395                3400                3405
Gly Leu Ile Thr Cys Leu Ile Cys Gly Val Leu Val Thr Thr Arg Arg
    3410                3415                3420
Arg Lys Lys Glu Gly Glu Tyr Asn Val Gln Gln Gln Cys Pro Gly Tyr
3425                3430                3435                3440
Tyr Gln Ser His Leu Asp Leu Glu Asp Leu Gln
                3445                3450


<210> 596
<211> 156
<212> PRT
<213> Homo sapiens

<400> 596
Gly Phe Thr His Arg Ser Ser Val Pro Thr Thr Ser Thr Pro Gly Thr
                5                   10                  15

Ser Thr Val Asp Leu Gly Thr Ser Gly Thr Pro Ser Ser Leu Pro Ser
            20                  25                  30

Pro Thr Ala Ala Gly Pro Leu Leu Val Pro Phe Thr Leu Asn Phe Thr
        35                  40                  45

Ile Thr Asn Leu Gln Tyr Glu Glu Asp Met His His Pro Gly Ser Arg
    50                  55                  60

Lys Phe Asn Thr Thr Glu Arg Val Leu Gln Gly Leu Leu Gly Pro Leu
65                  70                  75                  80

Phe Lys Asn Thr Ser Val Gly Pro Leu Tyr Ser Gly Cys Arg Leu Thr
                85                  90                  95

Leu Leu Arg Pro Glu Lys Asp Gly Ala Ala Thr Gly Val Asp Ala Ile
            100                 105                 110

Cys Thr His Arg Leu Asp Pro Lys Ser Pro Gly Leu Asp Arg Glu Gln
        115                 120                 125

Leu Tyr Trp Glu Leu Ser Gln Leu Thr His Gly Ile Thr Glu Leu Gly
    130                 135                 140

Pro Tyr Thr Leu Asp Arg Asp Ser Leu Tyr Val Asn
145                 150                 155